# EUROPEAN PATENT APPLICATION

(11) **EP 2 660 325 A2**
(43) Date of publication of application: **06.11.2013**
(21) Application number: 13166332.0
(22) Date of filing: 02.05.2013
(51) Int. Cl.: C12N 15/864, A61K 48/00

(54) **AAV vectors and corresponding nucleotide sequences and methods**

(30) Priority: 02.05.2012 IN CH17142012; 04.06.2012 IN CH22312012
(71) Applicant: Christian Medical College, 632 002 Tamil Nadu (IN)
(72) Inventor: Hareendran, Sangeetha, 632 002 Tamil Nadu (IN); Gabriel, Nishanth, 632 004 Tamil Nadu (IN); Sen, Dwaipayan, 632 004 Tamil Nadu (IN); Gadkar, Rupali, 560 012 Karnataka (IN); Govindarajan, Sudha, 560 012 Karnataka (IN); Srinivasan, Narayana Swamy, 560 012 Karnataka (IN); Srivastava, Alok, 632 004 Tamil Nadu (IN); Jayandharan, Giridhara Rao, 632 004 Tamil Nadu (IN); Selot, Ruchita, 632 004 Tamil Nadu (IN); Balakrishnan, Balaji, 632 002 Tamil Nadu (IN); Krishnagopal, Akshaya, 632 002 Tamil Nadu (IN)
(74) Representative: Epping - Hermann - Fischer

(57) **Abstract**

The present disclosure relates to recombinant adeno-associated virus (AAV) vector serotype, wherein the capsid protein of AAV serotypes is mutated at single or multiple sites. The disclosure further relates to an improved transduction efficiency of these mutant AAV serotypes. The AAV serotypes disclosed are AAV1, AAV2, AAV3, AAV4, AAV5, AAV6, AAV7, AAV8, AAV9, AAV10. The instant disclosure relates to nucleotide sequences, recombinant vector, methods and kit thereof.

## Description

### TECHNICAL FIELD

The present disclosure relates to recombinant adeno-associated virus (AAV) vector serotype, wherein the capsid protein of AAV serotypes is mutated at single or multiple sites. The disclosure further relates to an improved transduction efficiency of these mutant AAV serotypes. The AAV serotypes disclosed are AAV1, AAV2, AAV3, AAV4, AAV5, AAV6, AAV7, AAV8, AAV9, AAV10. The instant disclosure thus relates to nucleotide sequences, recombinant vector, methods and kit thereof.

### BACKGROUND OF THE DISCLOSURE

Intracellular trafficking of virus from cytoplasm to nucleus is one of the crucial rate limiting events in determining the efficiency of gene transfer with AAV. However, their therapeutic efficiency when targeted to organ systems, such as during hepatic gene transfer in patients with hemophilia B, is suboptimal because of the CD8+ T cell response directed against the AAV capsid particularly at higher administered vector doses (≥2×10¹² viral genomes [VG]/kg) (Manno et al., 2006). A similar theme of vector dose-dependent immunotoxicity has emerged from the use of alternative AAV serotypes in other clinical trials as well (Stroes et al., 2008). More recently, in the recombinant AAV8- mediated gene transfer for hemophilia B (Nathwani et al., 2011), two patients who received the highest dose (2×10¹² VG/kg) of vector required glucocorticoid therapy to attenuate a capsid-specific T cell response developed against capsid. Further, a majority of AAV vectors are phosphorylated and degraded in the cytoplasm. The use of proteasomal inhibitors is known to result in a -2 fold increase in gene expression from AAV serotypes (Monahan *et al.,* 2010). However, systemic administration of these proteasomal inhibitors leads to severe side effe*cts* (Rajkumar *et al*.,2005). Therefore, irrespective of whether an alternative AAV serotype or an immune suppression protocol is used, it is important to develop novel AAV vectors that provide enhanced gene expression at significantly lower vector doses to achieve successful gene transfer in humans.

### STATEMENT OF THE DISCLOSURE

Accordingly, the present disclosure relates to a nucleotide sequence selected from a group comprising SEQ ID Nos. 139 to 148, having mutation at codon selected from a group comprising TCT, TCC, TCA, TCG, AGT, AGC, ACT, ACC, ACA, ACG, AAA and AAG or any combination thereof; a nucleotide sequence selected from a group comprising SEQ ID Nos. 70 to 138, having mutation at codon selected from a group comprising TCT, TCC, TCA, TCG, AGT, AGC, ACT, ACC, ACA, ACG, AAA and AAG or any combination thereof; a method of obtaining a nucleotide sequence selected from a group comprising SEQ ID Nos. 139 to 148, having mutation at codon selected from a group comprising TCT, TCC, TCA, TCG, AGT, AGC, ACT, ACC, ACA, ACG, AAA and AAG or any combination thereof, said method comprising act of introducing mutation in any of nucleotide sequence selected from a group comprising SEQ ID Nos. 139 to 148 through site directed mutagenesis by using the nucleotide sequence as mentioned above; a method of enhancing transduction efficiency, said method comprising act of expressing a target gene in presence of a nucleotide sequence selected from a group comprising SEQ ID Nos. 139 to 148, having mutation at codon selected from a group comprising TCT, TCC, TCA, TCG, AGT, AGC, ACT, ACC, ACA, ACG, AAA and AAG or any combination thereof; a recombinant cell comprising the nucleotide sequence as mentioned above; a method of obtaining the recombinant cell as mentioned above, said method comprising act of introducing the nucleotide sequence as mentioned above to a host cell, to obtain said recombinant cell; and a kit comprising a nucleotide sequence selected from a group comprising SEQ ID Nos. 70 to 138, having mutation at codon selected from a group comprising TCT, TCC, TCA, TCG, AGT, AGC, ACT, ACC, ACA, ACG, AAA and AAG or any combination thereof.

### BRIEF DESCRIPTION OF THE ACCOMPANYING FIGURES

In order that the disclosure may be readily understood and put into practical effect, reference will now be made to exemplary embodiments as illustrated with reference to the accompanying figures. The figure together with a detailed description below, are incorporated in and form part of the specification, and serve to further illustrate the embodiments and explain various principles and advantages, in accordance with the present disclosure wherein:

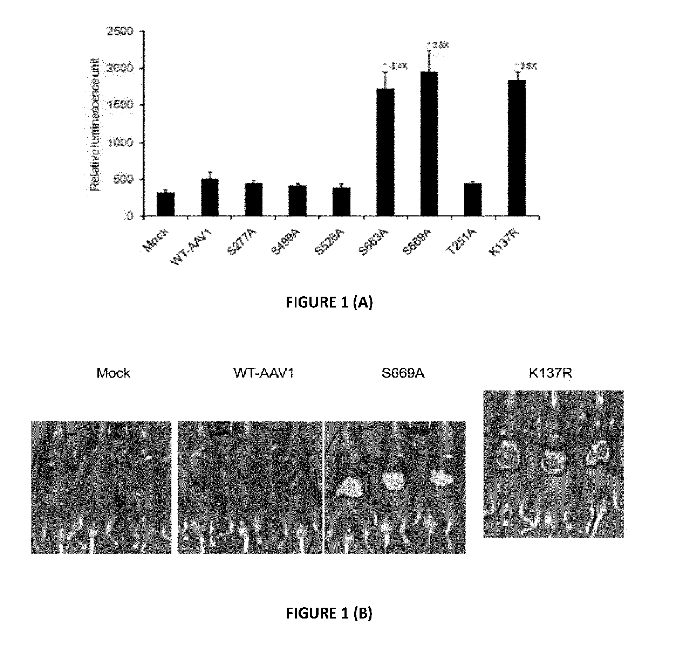

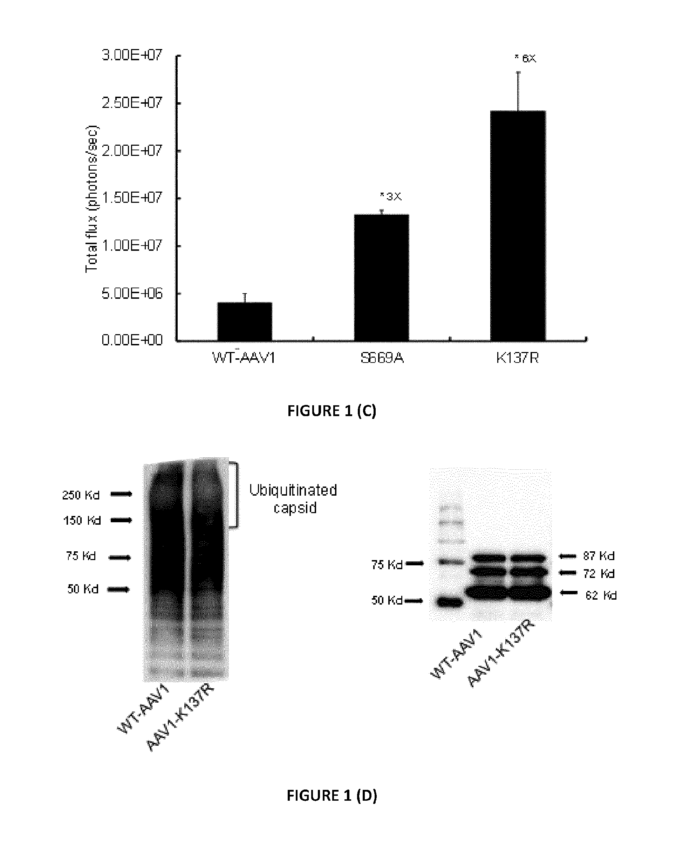

**Figure 1****:** illustrates increased transduction efficiency *in vitro* and *in vivo* of AAV1 serine/lysine mutant vectors. (a) *In vitro* transduction efficiency of AAV1 vectors. Equal number of CHO cells is mock-infected or infected with 5x10³ vgs/cell of the different AAV1 vectors. Forty eight hours later, the luciferase activity in the cell lysate is measured using a commercial kit (BioVision Inc, Milpitas, CA, USA) in a GlowMax^{™} 20/20 luminometer (Promega, WI, USA). The data depicted are mean of triplicate analysis. Fold increase in transduction in comparison to WT-AAV1 infected cells are shown. *p<0.05 *Vs* WT-AAV1 infected cells. (b) *In vivo* bio-luminescence imaging of AAA1 vector administered mice. Animals injected with 5x10¹⁰ vgs of AAV1-Luciferase vectors are imaged 2-weeks after gene transfer in an IVIS Spect-CT small animal imaging system (Perkin Elmer, Caliper Life Sciences). (c) The fold change in luciferase expression from animals injected with AAA1-K137R or AAV1-S669A vectors are shown in comparison to WT-AAV1 injected mice. ***p<0.05 *Vs* WT-AAV1 injected mice. (d) Approximately 3 x 10⁸ viral particles of WT-AAV1 and K137R-AAV1 vectors are denatured at 95°C for 5 minutes. The denatured viral particles are then used to perform the ubiquitin conjugation assay according to the manufacturer's protocol. The processed samples are electrophoresed on a 4-20% denaturing polyacrylamide gel and the ubiquitination pattern detected by immunoblotting. The mono-to-polyubiquitin conjugates are detected as a smear at molecular weight >150Kda. (e) VP1 (87Kda), VP2 (72 KDa) and VP3 (62Kda) capsid proteins are immunoblotted as loading controls.

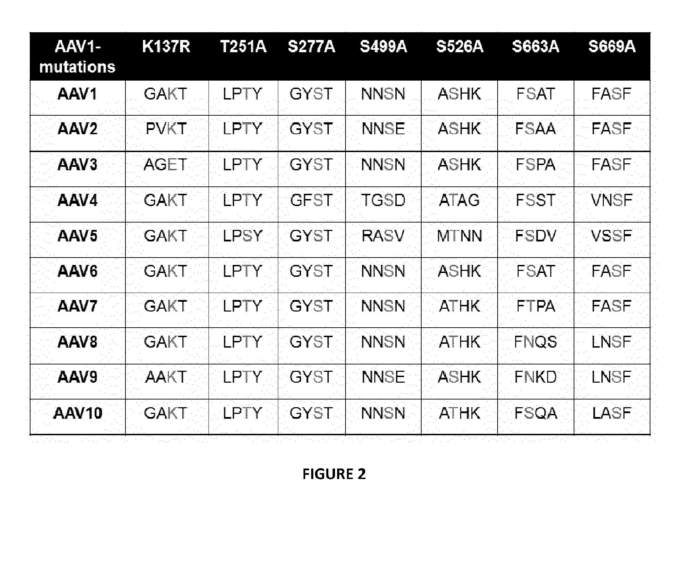

**Figure 2****:** illustrates the schematic representation of the serine (S), threonine (T) and lysine (K) residues mutated in AAV1 and their conservation status across other AAV serotypes. VP1 protein sequences from AAV serotypes 1 through 10 are aligned by ClustalW and the conservation status of the each of the target site for mutagenesis is shown in red.

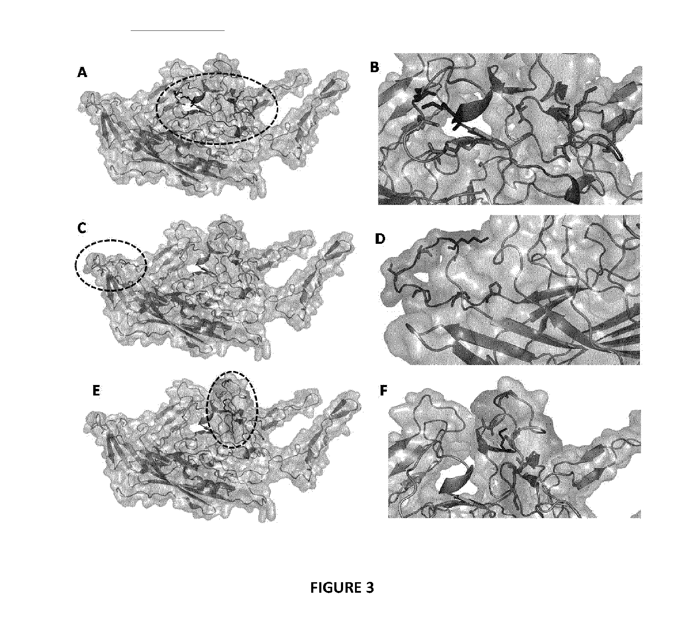

**Figure 3****:** illustrates the Structural analysis of phosphodegrons 1-3 in AAV2 capsid. In particular figures 1 a, c and e show phosphodegrons 1, 2 and 3 colored in green, respectively and the corresponding zoomed in regions of the three phosphodegrons are shown in b, d and f respectively. Phosphodegrons in the AAV2 capsid are largely present in the loop regions and are solvent exposed as shown in the figure. The phosphorylation and ubiquitination sites in the phosphodegrons are shown as green and blue spheres respectively. Receptor binding residues that have been also predicted as ubiquitination sites are shown as purple spheres. The acidic residues in phosphodegron 1 and 3 and prolines in phosphodegron 2 are colored red while rest of the protein structure is shown in grey. The images are generated using PYMOL software (DeLlano, 2002)

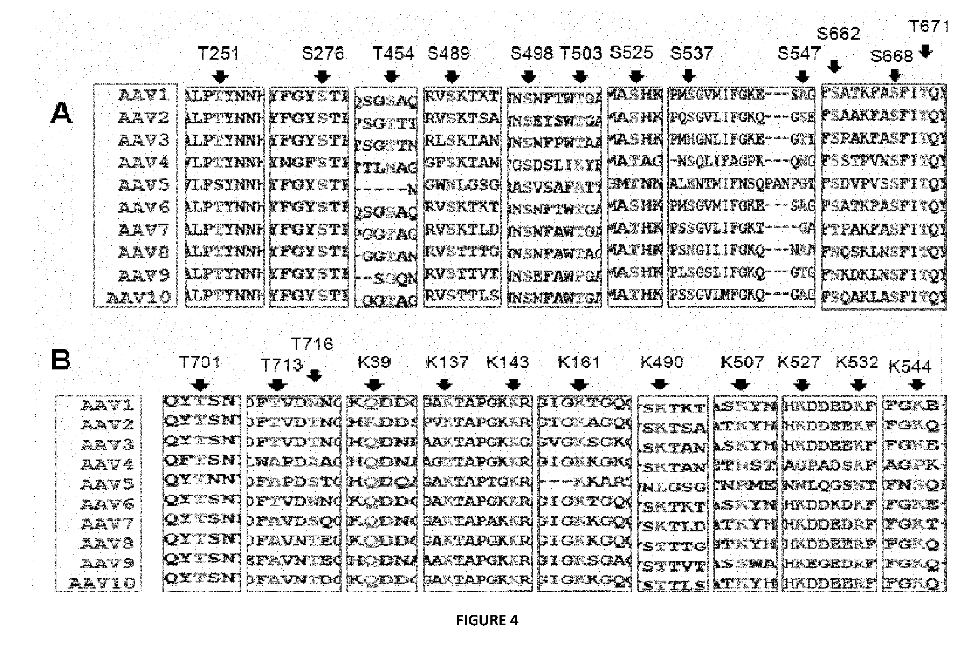

**Figure 4****:** illustrates the Schematic representation and conservation status of the various serine (S), threonine (T) and lysine (K) residues mutated in AAV2 capsid. VP1 protein sequences from AAV serotypes 1 through 10 are aligned by ClustalW and the conservation status of the each of the mutated site is given. S/T residues are shown in panel A while lysine residues in panel B. S/T/K residues within phosphodegrons 1, 2 and 3 are shown in red font while those chosen based on evolutionary conservation is shown in green font. Those residues which are chosen based on either *in silico* prediction to be a part of a phosphosite or high ubiquitination score on UbPred tool are shown in blue. A control threonine mutation shown in brown font is chosen as a negative control for the mutation experiments.

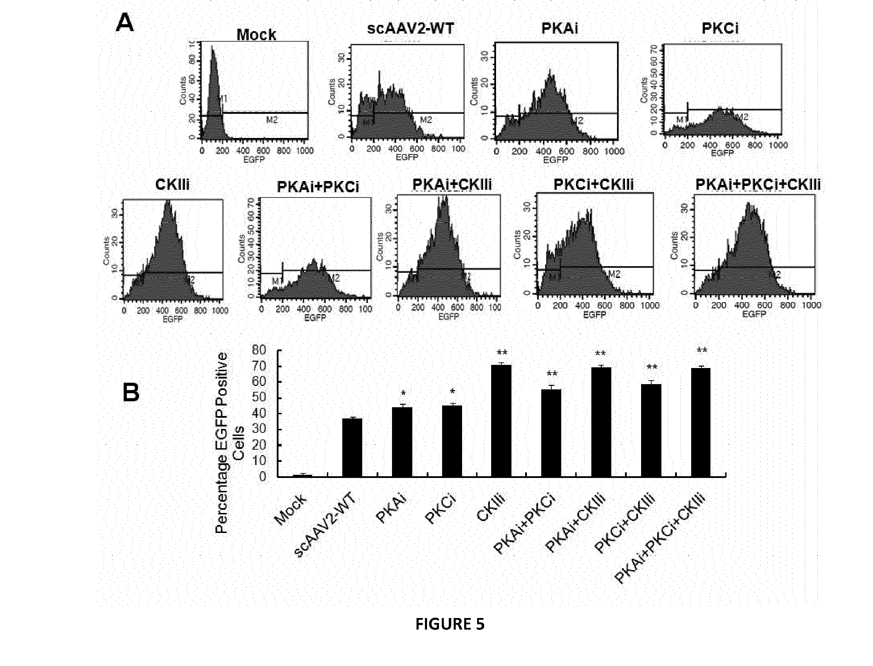

**Figure 5****:** illustrates the effect of pharmacological inhibition of host cellular serine/threonine kinases on AAV2 mediated gene expression. (A) HeLa cells are mock-(PBS) treated or pretreated with the protein kinase A (PKA), protein kinase C (PKC), casein kinase (CK)-II inhibitors (i) either alone or in combinations shown, 24hrs prior to transduction with AAV2-EGFP vectors. Twenty four hours post-transduction, cell suspensions are analyzed for EGFP expression by flow cytometry (B) Quantitative representation of the data from (A). One way analysis of variance (ANOVA) test is used for statistical analysis. *p<0.05; **p<0.01 *Vs* AAV2-WT infected cells.

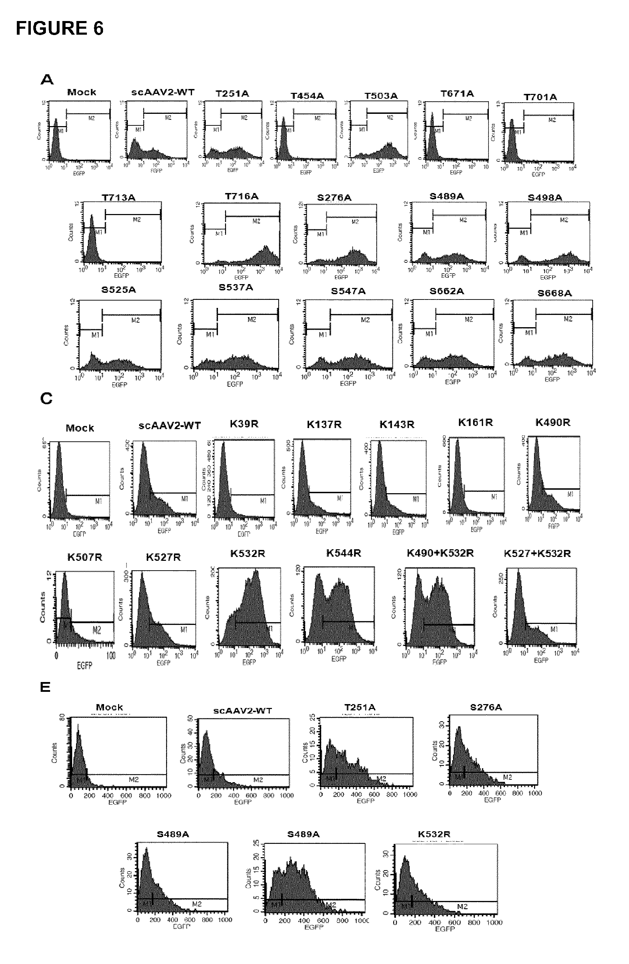

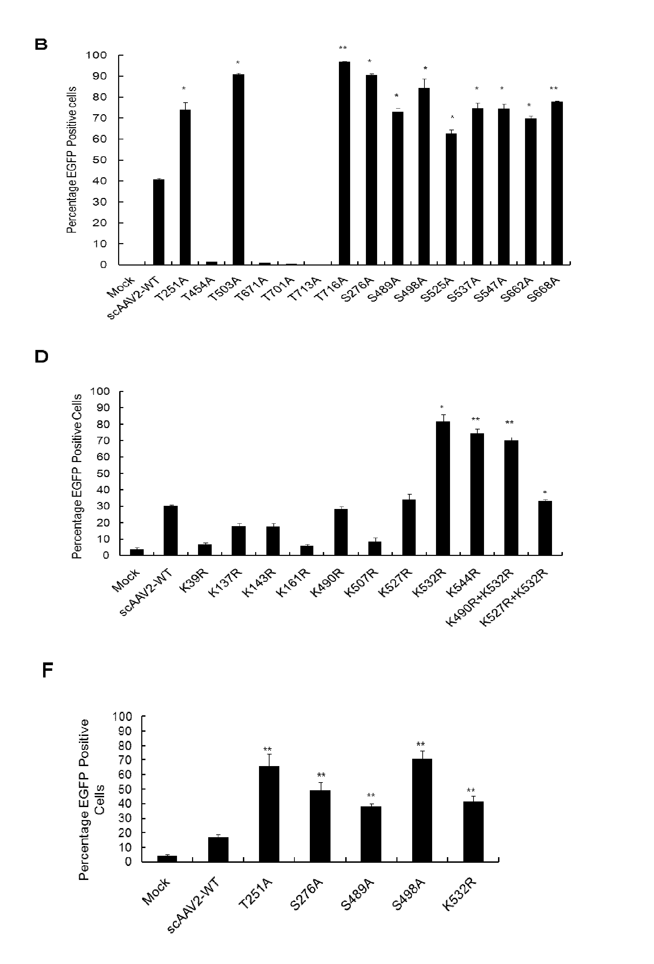

**Figure 6****:** illustrates the increased transduction efficiency of AAV2 serine/threonine/lysine mutant vectors *in vitro.* HeLa cells are either mock-infected or infected with 2x10³ vgs/cell of AAV2-WT or AAV2-S/T→A (A) or AAV2- K→R (C) mutant vectors and cells analyzed for EGFP expression 48hrs later by flow cytometry. The percentage EGFP positive cells post-transduction with either serine/threonine mutants (B) or lysine mutants (D) are shown. Similar experiments are carried out in HEK 293 cells with an MOI of 2x10³ vgs/cell of AAV2-WT or AAV2 S/T/K mutant vectors (E). Quantitative analysis of this data by flow cytometric analysis is shown in F. The data depicted in A, C, and E are representative histograms while the data in B, D, F are mean of triplicate analysis. One way analysis of variance (ANOVA) test is used for statistical analysis.*p<0.05, **p<0.01 *Vs* AAV2-WT infected cells.

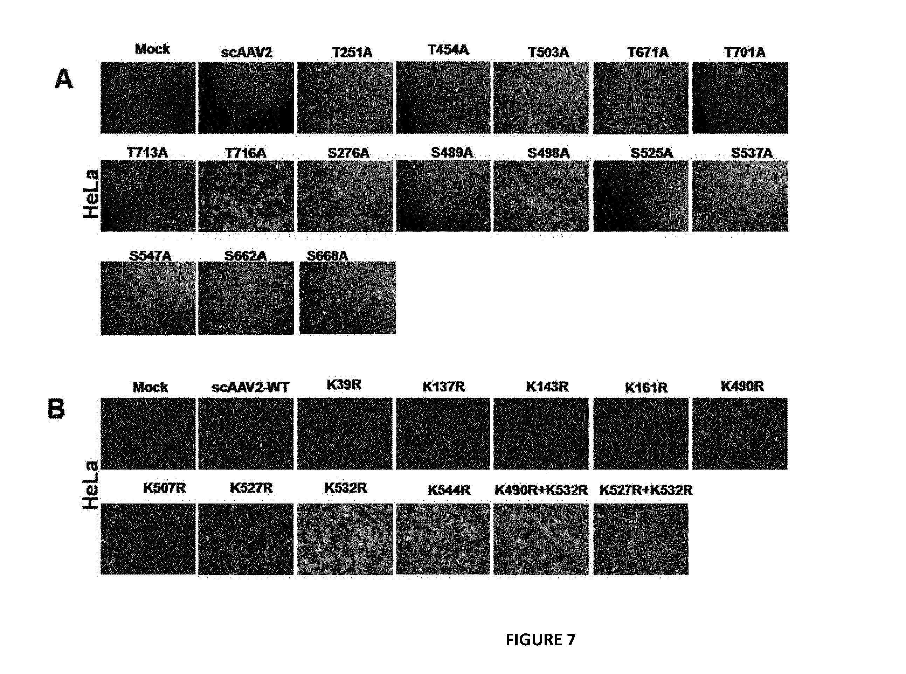

**Figure 7****:** illustrates the fluorescence imaging of HeLa cells infected with AAV2 wild-type or S/T/K mutant vectors. HeLa cells are either mock-infected or infected with 2x10³ vgs/cell of AAV2-WT or AAV2-S/T/K mutant vectors. Forty eight hours later, the cells are analyzed by fluorescence microscopy (A) Visual comparison of AAV2 S/T→A mutants compared to AAV2-WT vectors. (B) Visual comparison of AAV2 K→R mutants compared to AAV2-WT vectors.

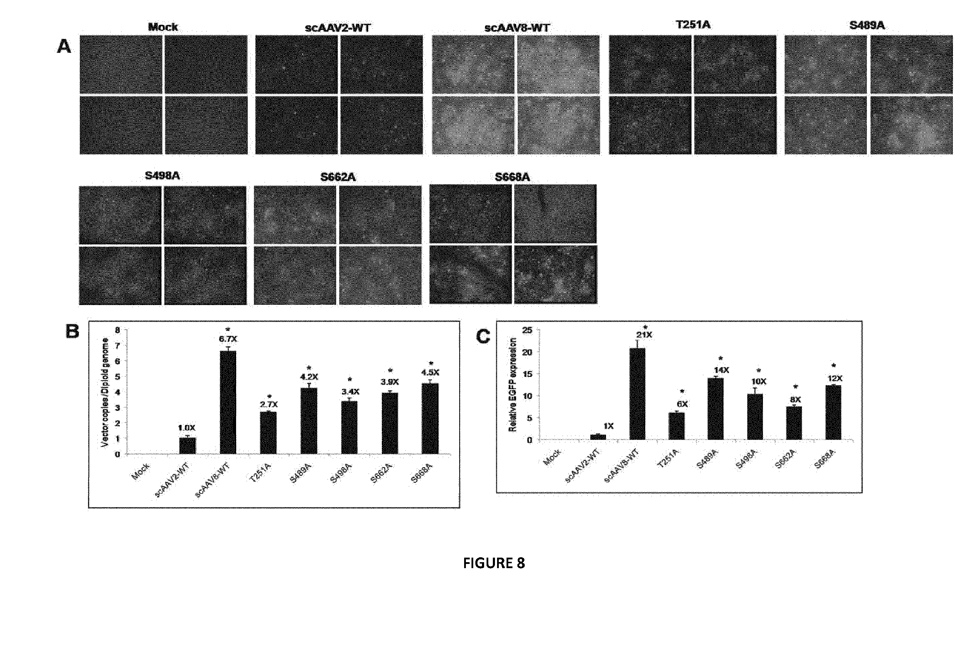

**Figure 8****:** illustrates enhanced transduction upon hepatic gene transfer of AAV2 serine/threonine mutant *vectors in vivo.* (A) Transgene expression is detected by fluorescence microscopy 4-weeks post-injection with 5x10¹⁰ vector particles/animal of scAAV2-EGFP, control scAAV8-EGFP or AAV2 mutant S/T vectors. Representative images of hepatic tissues from four different animals in each group are shown. (B) Estimation of vector genome copies in the liver after AAV mediated gene transfer. Genomic DNA is isolated from liver tissue of C57BL/6 mice 4-weeks post vector administration and the viral copy numbers estimated by a quantitative PCR (C) Analysis of EGFP transcript levels by real-time quantitative PCR. Hepatic RNA isolated from animals injected with AAV2-WT, AAV8-WT or AAV2 S/T vectors are analyzed for EGFP expression and the data normalized to the GAPDH reference gene. One way analysis of variance (ANOVA) test is used for the statistical comparisons. *p<0.05 *Vs* AAV2-WT injected animals.

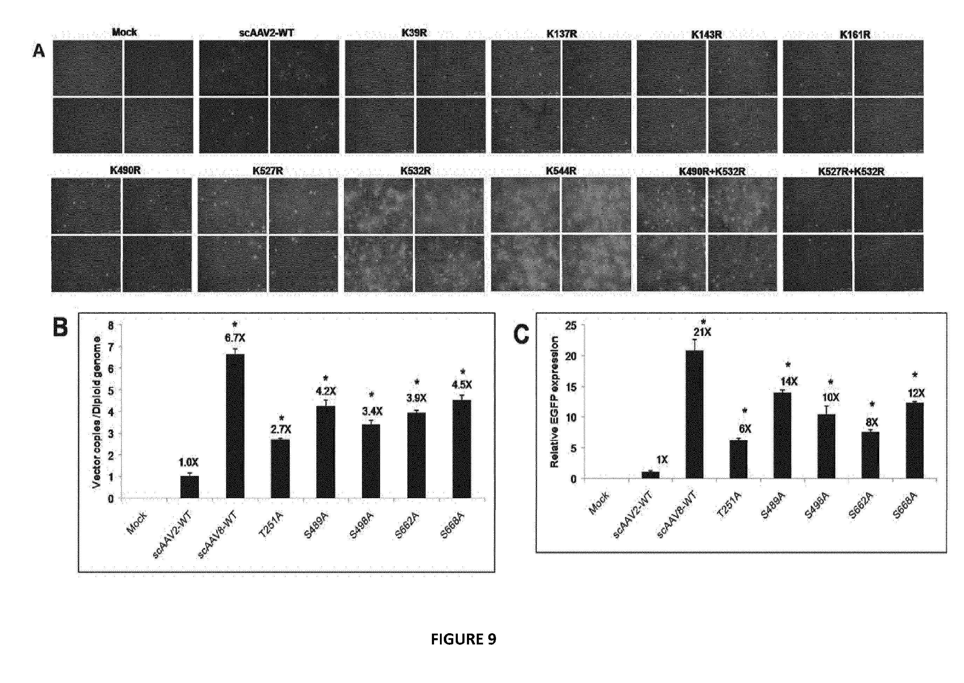

**Figure 9****:** illustrates the analysis of AAV2 lysine mutant vector-mediated EGFP expression in hepatocytes of normal C57BL/6 mice in comparison to wild-type AAV2 vectors. (A) Transgene expression is detected by fluorescence microscopy 4-weeks post-injection of 5x10¹⁰ vector particles/animal of scAAV2-EGFP or AAV2 K→R mutant vectors. Representative images of hepatic tissues from four different animals in each group are shown. (B) Estimation of vector genome copies in the liver after AAV mediated gene transfer. Genomic DNA is isolated from liver tissue of C57BL/6 mice 4-weeks post vector administration and the viral copy numbers estimated by a quantitative PCR as described in the "materials and methods". (C) Analysis of EGFP transcript levels by real-time quantitative PCR. Hepatic RNA isolated from animals injected with AAV2-WT or K→R mutant vectors are analyzed for EGFP expression and the data normalized to the GAPDH reference gene. One way analysis of variance (ANOVA) test is used for the statistical comparisons. *p<0.05 Vs AAV2-WT injected animals.

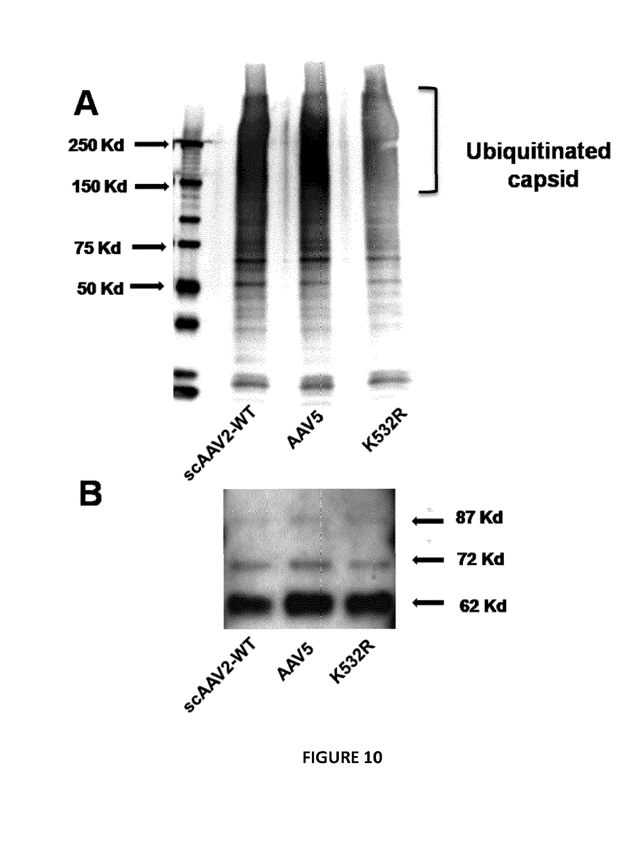

**Figure 10****:** illustrates reduced ubiquitination of AAV2 lysine mutant K532R in comparison to AAV2-WT or AAV5-WT vectors. (A) Approximately 3 x 10⁸ viral particles of AAV2-WT, AAV5-WT and AAV2 K532R vectors are denatured at 95°C for 5 minutes. The denatured viral particles are then used to perform the ubiquitin conjugation assay. The processed samples are electrophoresed on a 5-20% denaturing polyacrylamide gel and the ubiquitination pattern detected by immunoblotting using an anti-ubiquitin antibody. The mono-to-polyubiquitin conjugates are detected as a smear at molecular weight >150Kda. **(B)** AAV Capsid VP1, VP2 and VP3 proteins are used as loading control.

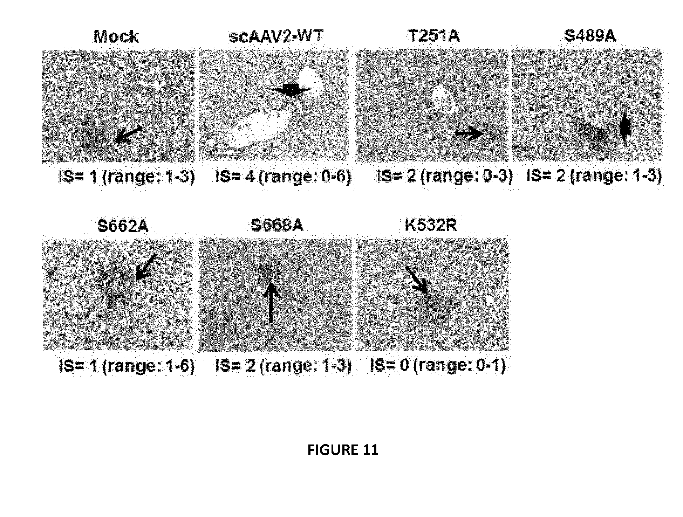

**Figure 11****:** illustrates the histological examination of C57BL/6 liver samples 4-weeks post-injection of AAV2-WT or mutant vectors. Hepatic sections are fixed in 10% buffered formalin and stained with hematoxylin-eosin. The median inflammation score (IS) for each group is indicated below the images (Magnification-40X) with the range of values given within brackets. Arrowhead and thin arrow denote portal and focal lobular inflammation, respectively. Representative image of one animal liver from each group (n=3) is shown.

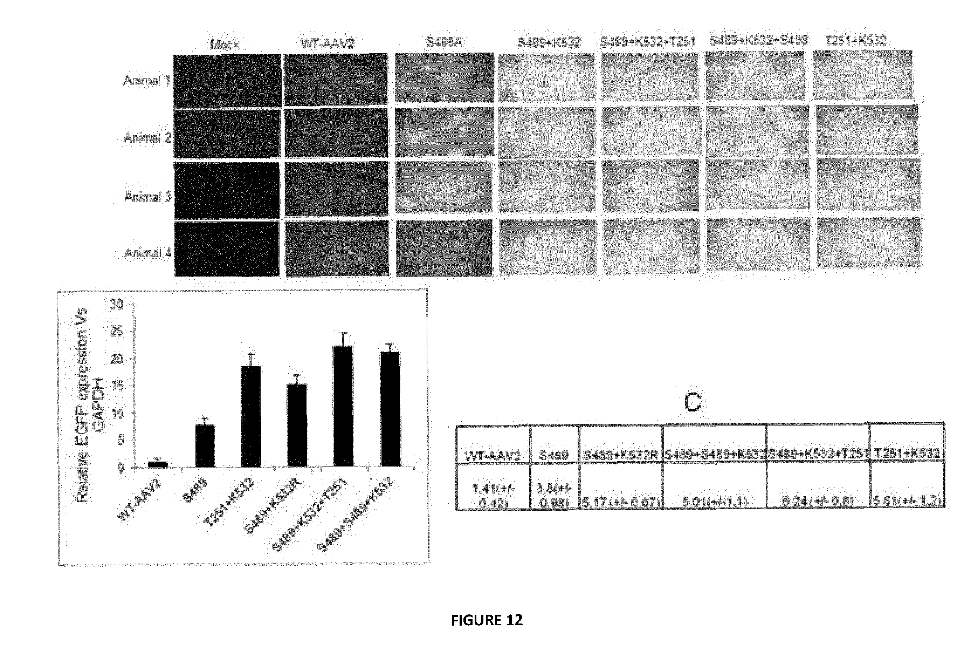

**Figure 12****:** illustrates enhanced transduction upon hepatic gene transfer of AAV2 serine/threonine/Lysine multiple-mutant *vectors in vivo.* (A) Transgene expression is detected by fluorescence microscopy 4-weeks post-injection with 5x10¹⁰ vector particles/animal of scAAV2-EGFP or multiple AAV2 mutant S/T/K vectors. Representative images of hepatic tissues from four different animals in each group are shown. (B) Analysis of EGFP transcript levels by real-time quantitative PCR. Hepatic RNA isolated from animals injected with AAV2-WT or AAV2 S/T/K vectors are analyzed for EGFP expression and the data normalized to the GAPDH reference gene. (C)Estimation of vector genome copies in the liver after AAV mediated gene transfer. Genomic DNA is isolated from liver tissue of C57BL/6 mice 4-weeks post vector administration and the viral copy numbers estimated by a quantitative PCR. One way analysis of variance (ANOVA) test is used for the statistical comparisons. *p<0.05 *Vs* AAV2-WT injected animals.
**Figure 13:** illustrates reduced cross-neutralizing antibody formation for AAV2 triple mutant [S489A+T251A+K532R] vector by Neutralization antibody assay.

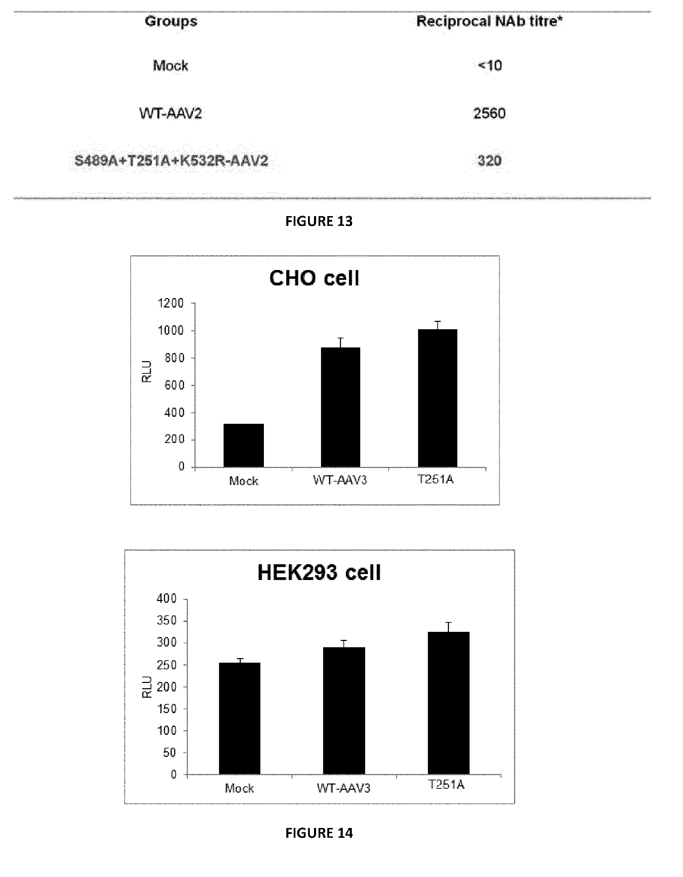

**Figure 14****:** illustrates *In vitro* transduction efficiency of T251A-AAV3 mutant vector as compared to WT-AAV3. Equal number of (A) CHO cells, (B) HEK 293 cells are mock-infected or infected with 5x10³ vgs/cell of the AAV3 vectors. Forty eight hours later, the luciferase activity in the cell lysate is measured using a commercial kit (BioVision Inc, Milpitas, CA, USA) in a GlowMax^{™} 20/20 luminometer (Promega, WI, USA). Data is mean of 3 wells for each condition. RLU= Relative luciferase unit.

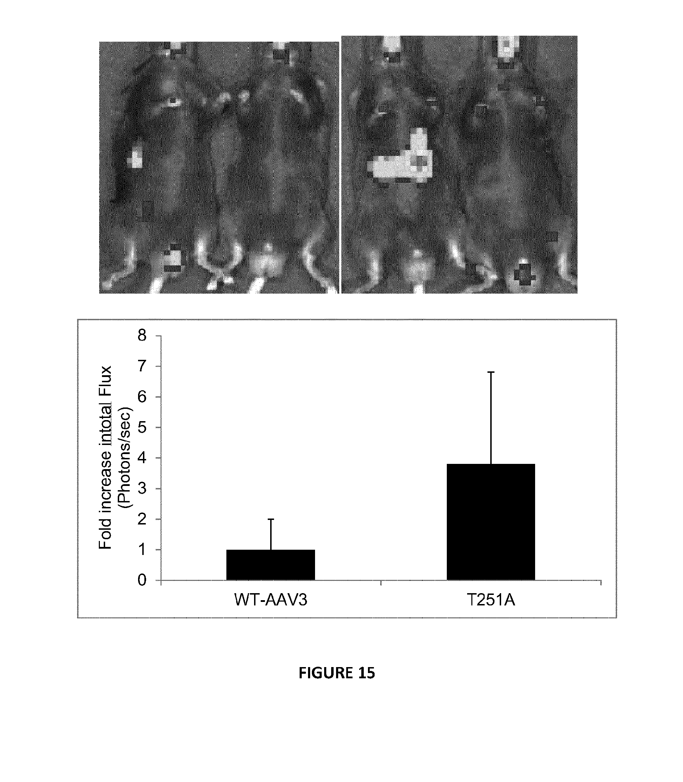

**Figure 15****:** illustrates increased transduction efficiencyof AAV3 T251A mutant vector *in vivo*. *In vivo* bio-luminescence imaging of AAV3 vector administered mice. Animals injected with 5x10¹⁰ vgs of AAV3 Luciferase vectors are imaged 1-week after gene transfer in an IVIS Spect-CT small animal imaging system (Perkin Elmer, Caliper Life Sciences).

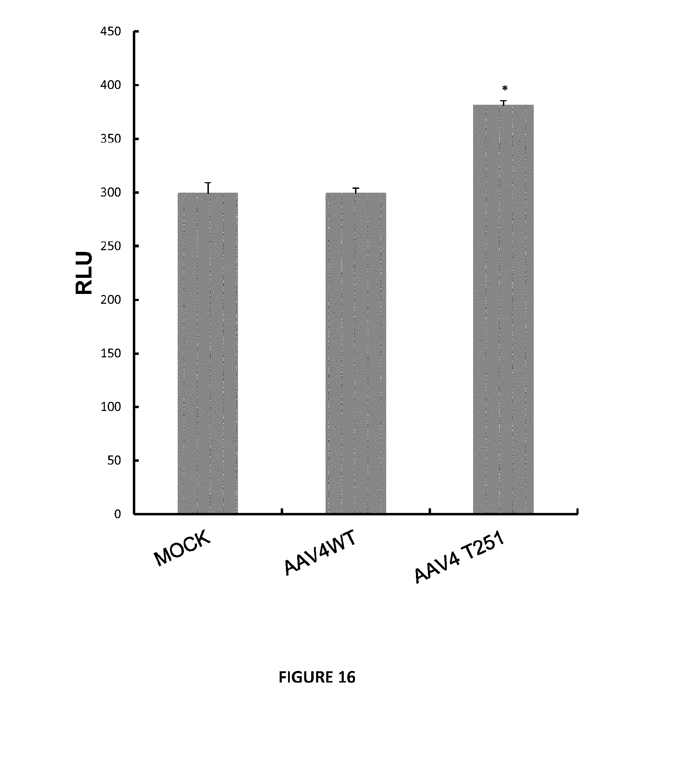

**Figure 16****:** illustrates *In vitro* transduction efficiency of T251A-AAV4 mutant vector as compared to WT-AAV4. Equal number of CHO cells are mock-infected or infected with 2x10³ vgs/cell of the AAV4 vectors. Forty eight hours later, the luciferase activity in the cell lysate is measured using a commercial kit (BioVision Inc, Milpitas, CA, USA) in a GlowMax^{™} 20/20 luminometer (Promega, WI, USA). Data is mean of 3 wells for each condition. RLU= Relative luciferase unit. *p<0.05

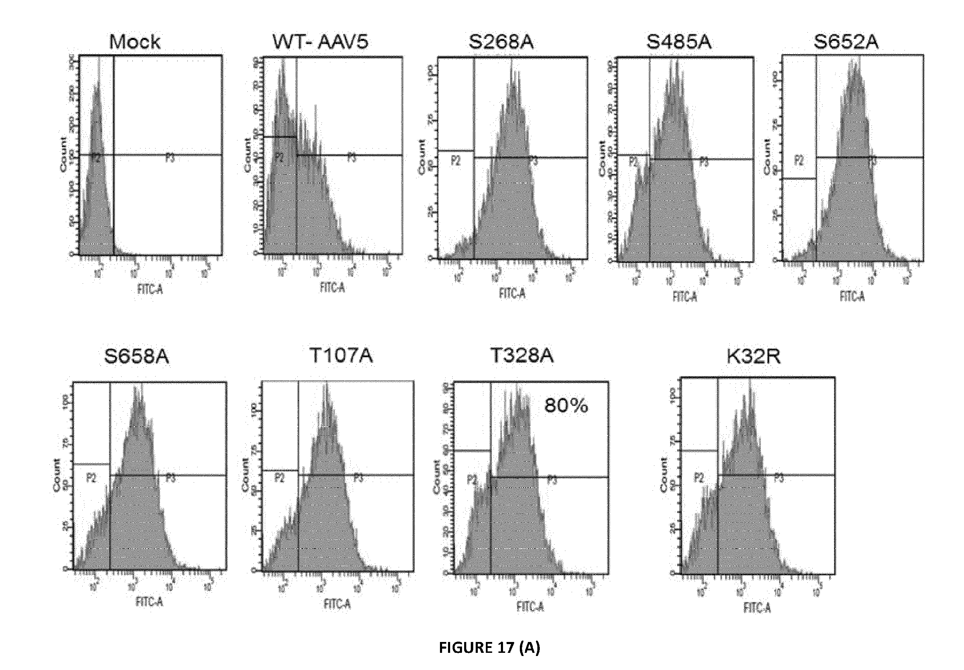

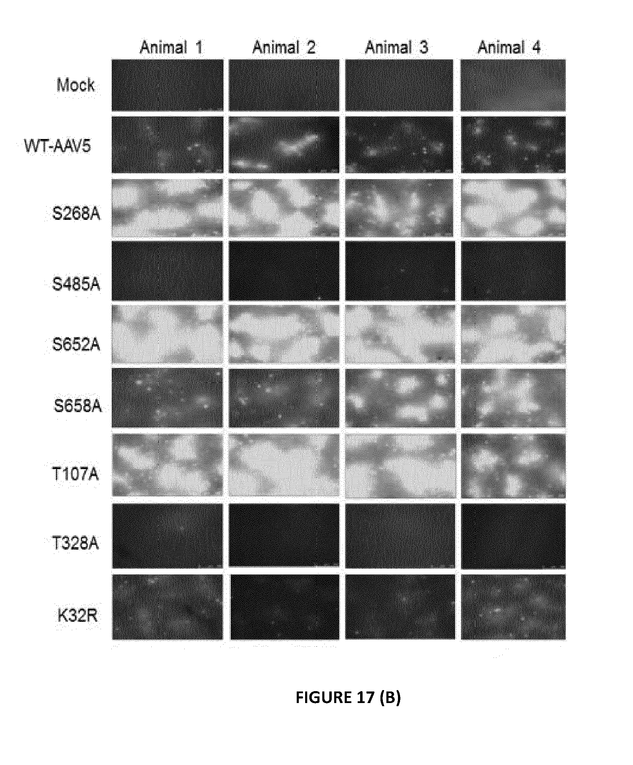

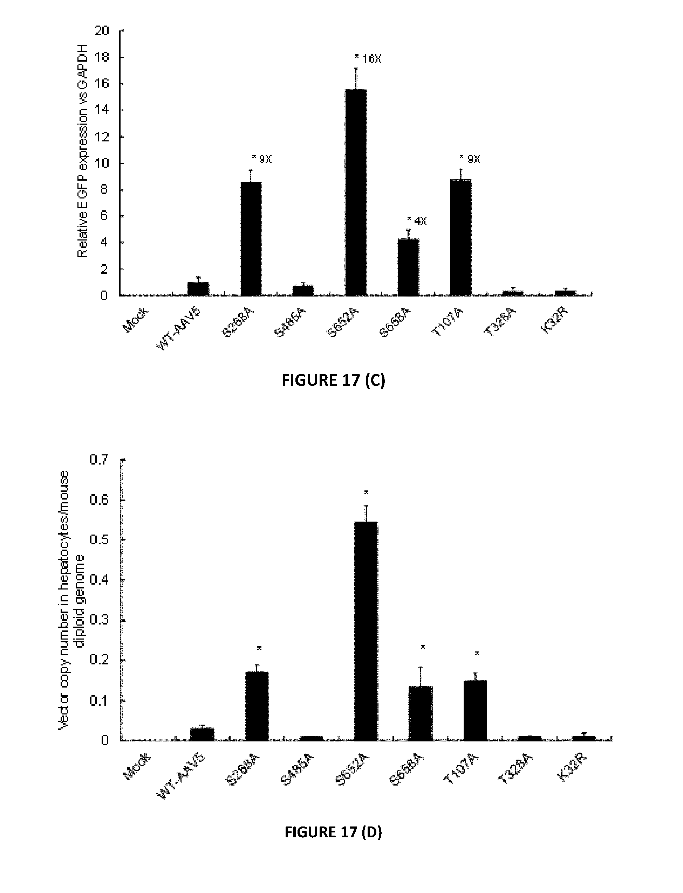

**Figure 17****:** illustrates increased transduction efficiency of AAV5 serine/threonine mutant vectors *in vitro* and *in vivo.* (**A**) *In vitro* transduction efficiency of AAV5 vectors. CHO cells are either mock-infected or infected with 5x10³ vgs/cell of the different AAV5 vectors. Forty-eight hours post-transduction, cell suspensions are analyzed for EGFP expression by flow cytometry. Representative histograms are shown. The data generated is from mean of triplicate analyses from two independent experiments. **(B)** Visual comparison of AAV5 S/T/K mutants in comparison to WT-AAV5 vectors. EGFP expression is detected by fluorescence microscopy 4-weeks post-administration of 5x10¹⁰ vector particles/animal of WT-AAV5 or mutant vectors. Representative images of hepatic tissues from four different animals in each group are shown. **(C)** Analysis of EGFP transcript levels by real-time quantitative PCR. Hepatic RNA isolated from animals injected with WT-AAV5 or AAV5 mutant vectors are analyzed for EGFP expression and the data normalized to the GAPDH reference gene. **(D)** Estimation of vector genome copies in the liver after AAV5 mediated gene transfer. Genomic DNA is isolated from liver tissue of C57BL/6 mice 4-weeks post vector administration and the viral copy numbers estimated by a quantitative PCR.*p<0.05 Vs WT-AAV5 injected animals.

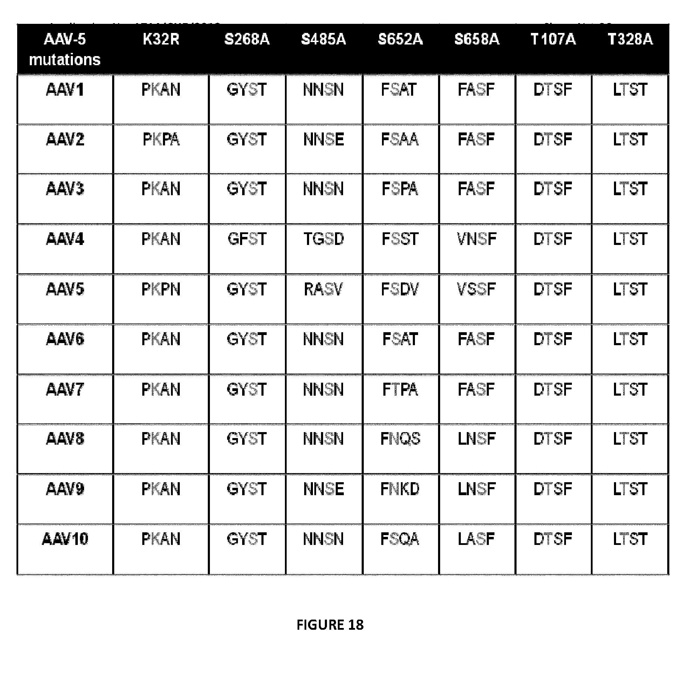

**Figure 18****:** illustrates the schematic representation of the serine (S), threonine (T) and lysine (K) residues mutated in AAV5 and their conservation status across other AAV serotypes. VP1 protein sequences from AAV serotypes 1 through 10 are aligned by ClustalW and the conservation status of the each of the target site for mutagenesis is shown in red.

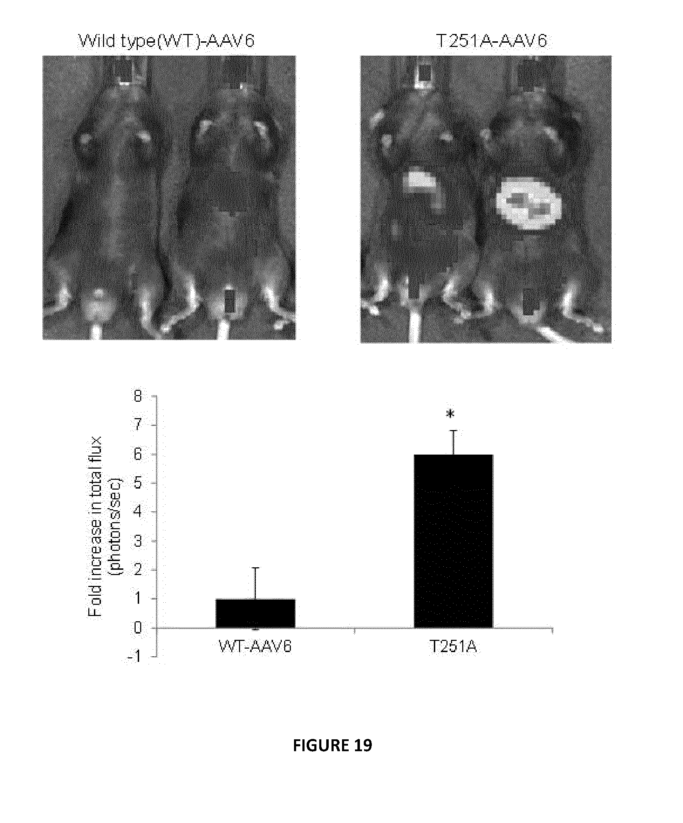

**Figure 19****:** illustrates increased transduction efficiency of AAV6 T251A mutant vector *in vivo*. *In vivo* bio-luminescence imaging of AAV6 vector administered mice. Animals injected with 1x10¹⁰ vgs of AAV6-Luciferase vectors are imaged 1 week after gene transfer in an IVIS Spect-CT small animal imaging system (Perkin Elmer, Caliper Life Sciences). *p<0.05

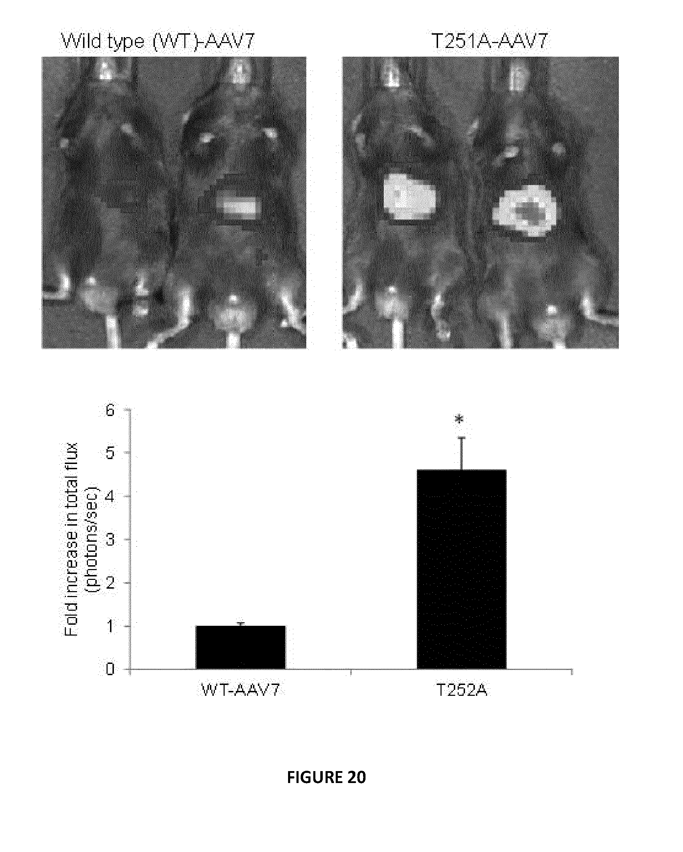

**Figure 20****:** illustrates increased transduction efficiency of AAV7 T252A mutant vector *in vivo. In vivo* bio-luminescence imaging of AAV7 vector administered mice. Animals injected with 5x10¹⁰ vgs of AAV7-Luciferase vectors are imaged 1 week after gene transfer in an IVIS Spect-CT small animal imaging system (Perkin Elmer, Caliper Life Sciences). *p<0.05.

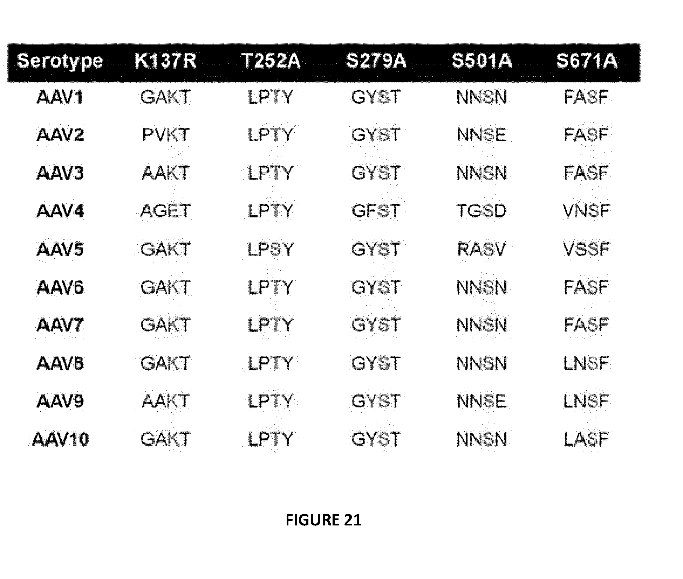

**Figure 21****:** illustrates the schematic representation of the serine (S), threonine (T) and lysine (K) residues mutated in AAV8 and their conservation status across other AAV serotypes. VP1 protein sequences from AAV serotypes 1 through 10 are aligned by ClustalW and the conservation status of the each of the target site for mutagenesis is shown in red.

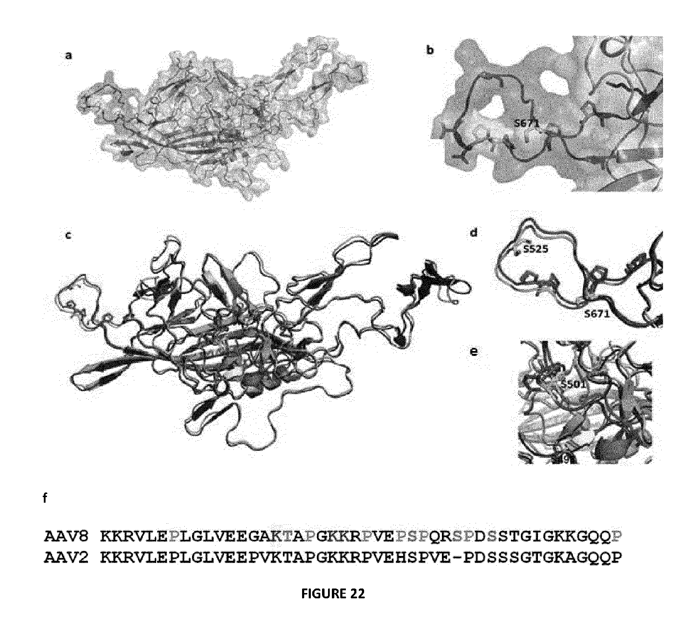

**Figure 22****:** illustrates the phosphodegron in AAV8 capsid structure. (A) The figure shows the capsid protein from AAV8 (PDB id: 2qa0) which is coloured in yellow. S671 (Coloured in cyan) lies in phosphodegron region (652-674) which is coloured in red. The phosphodegron is rich in prolines which are coloured green. The predicted phosphosites (serines and threonine) are shown in red while the predicted ubiquitination sites are in blue. **(B)** The zoomed in view of phosphodegron region (652-674) containing S671. **(C)** Comparison of phosphodegrons in AAV8 and AAV2. The figure shows structural superimposition of AAV8 (PDB id: 2qa0) and AAV2 (PDB id: 11p3 coloured in yellow and grey respectively. Phosphodegron in AAV8 coloured in red is equivalent to phosphodegron2 in AAV2 (515-528) which is coloured in green. Phosphodegrons in both AAV2 and 8 are rich in proline residues. The residue S525 in AAV2 (coloured in cyan) lies in the phosphodegron and has been shown to increase the transduction efficiency (Gabriel *et al.,* 2013). **(D)** The zoomed in view of the phosphodegron2 in AAV2 and AAV8. **(E)** The zoomed in view of phosphodegron3 of AAV2 in comparison with that in AAV8. The presence of another phosphodegron region in AAV2 (Phosphodegron 3: residues 489-507) which is rich in acidic residues is coloured in purple. S489 and S498 in this region of AAV2 have shown to increase the transduction efficiency *in vivo.* Whereas, the equivalent region in AAV8 coloured in pink lacks the acidic residues. **(F)** The figure shows the predicted phosphodegron stretch (122-137) in AAV8 which contains K137 highlighted in yellow. The phosphodegron is rich in proline and are coloured in green. The predicted phosphosites (T138, S149, S153 and S156) are coloured in magenta while the predicted ubiquitination sites (K137, K142 and K143) are coloured in blue.

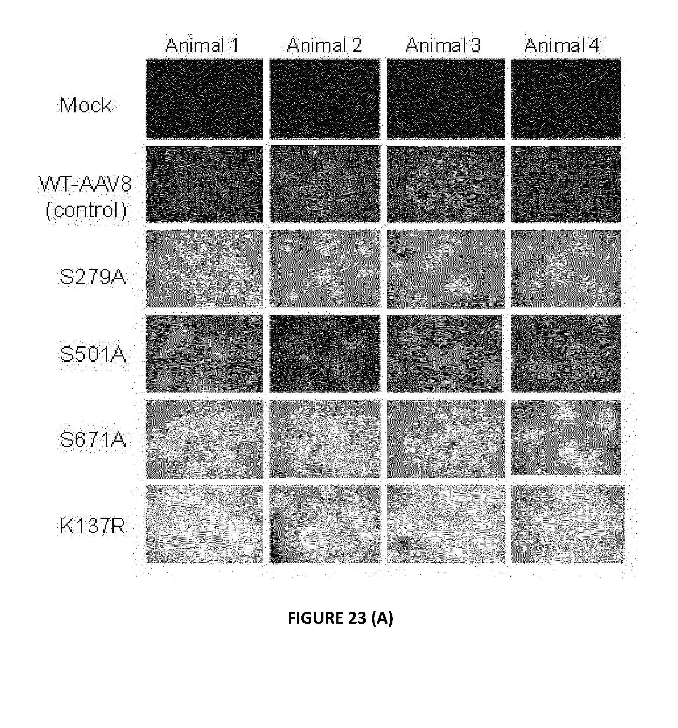

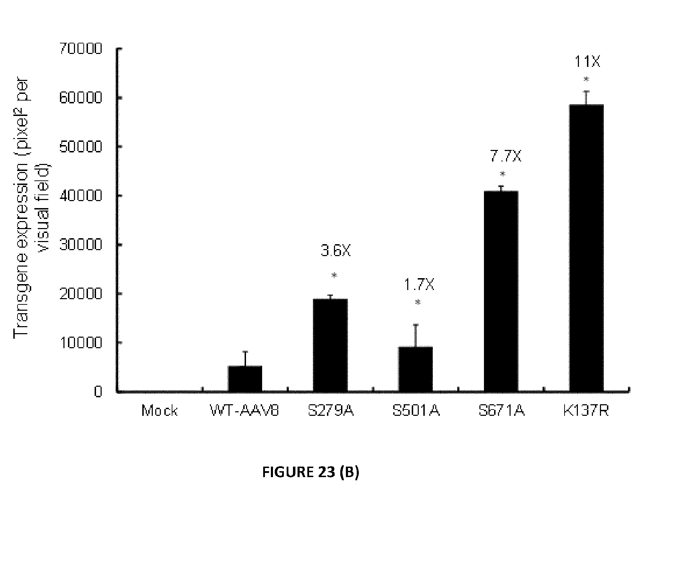

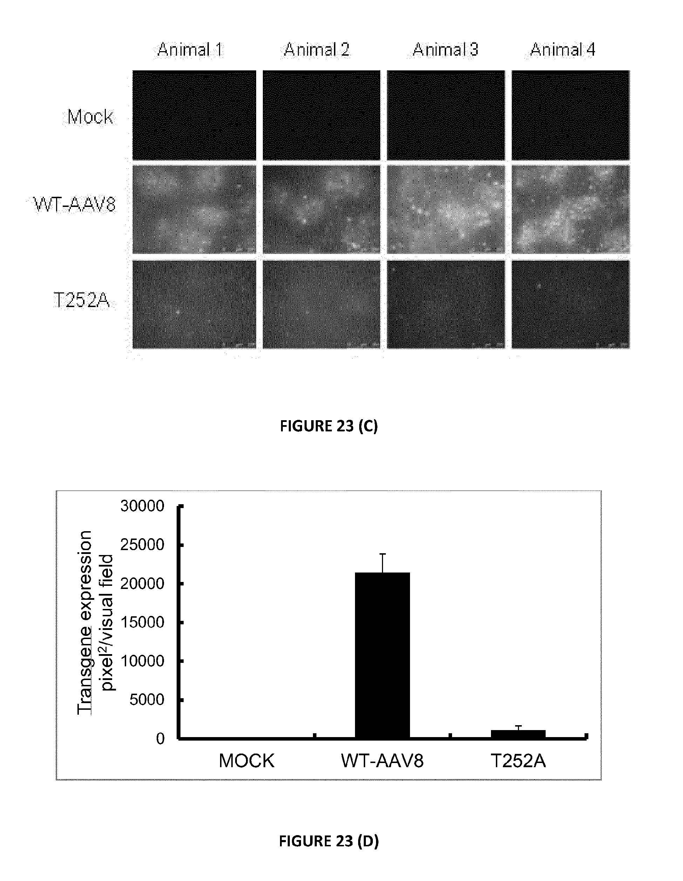

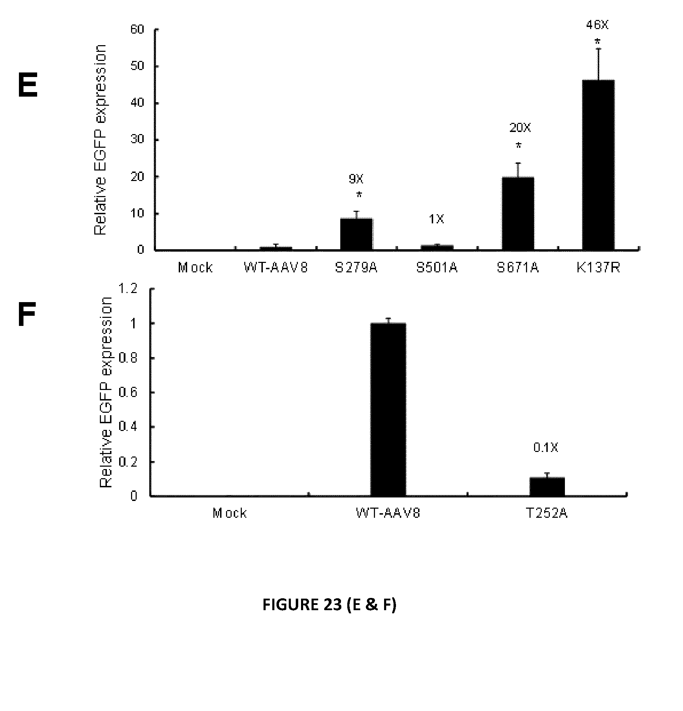

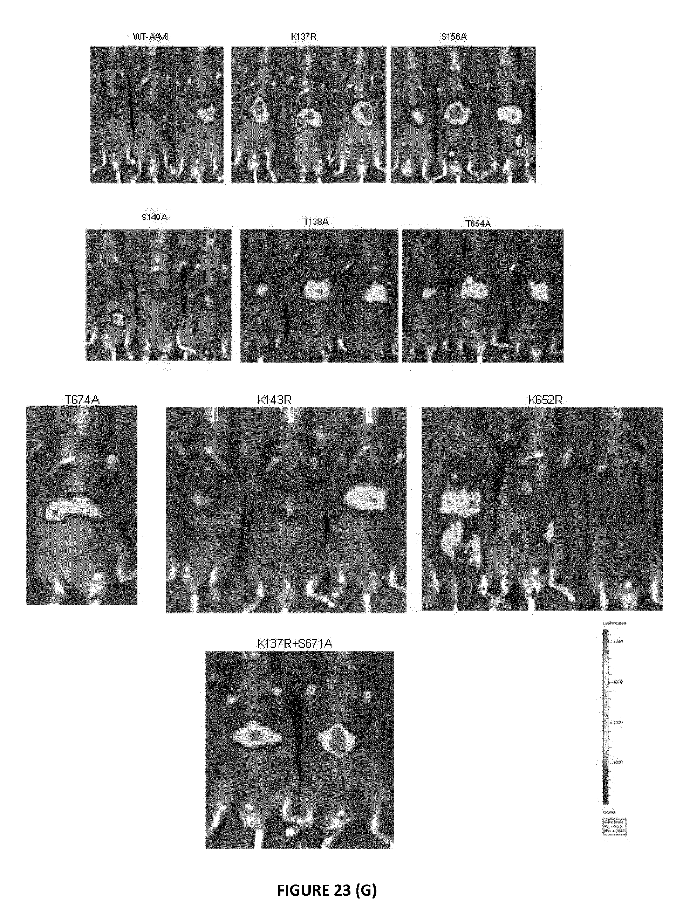

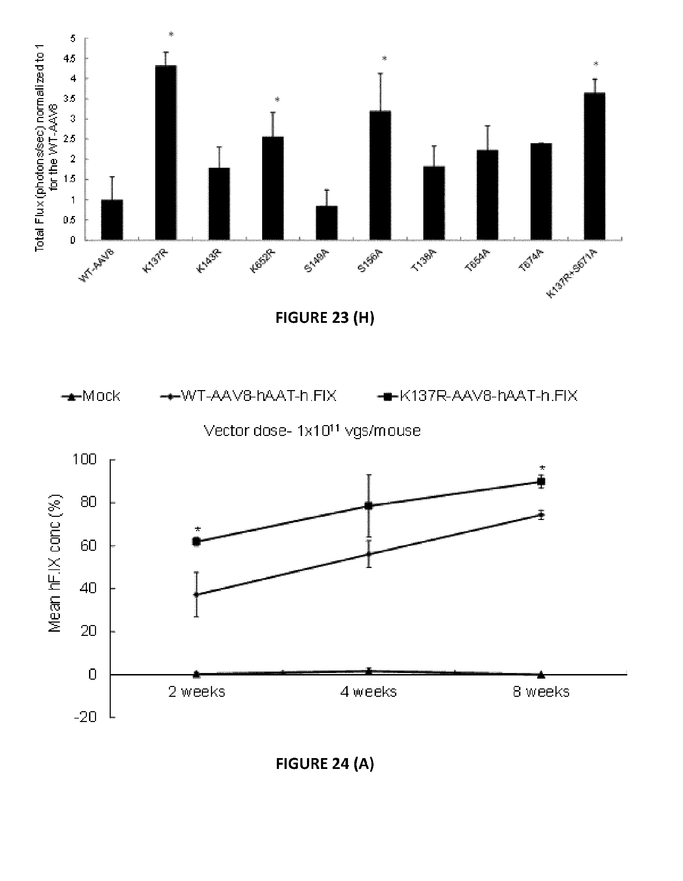

**Figure 23****:** illustrates enhanced transduction upon hepatic gene transfer of AAV8 serine and lysine mutant vectors *in vivo.* **(A)** Representative images from each animal (n=4 for mock, S279A, S501A and S671A, n=8 for WT and K137R) showing the transgene expression as detected by fluorescence microscopy 4-weeks post-injection with 5x10¹⁰ particles/animal of wild-type or S→A and K→R mutant AAV8 *via* the tail vein. **(B)** Quantitative analyses of the data from (A). **(C)** Representative images from each animal (n=4) showing the transgene expression as detected by fluorescence microscopy 2-weeks post-injection with 5x10¹⁰ particles of wild-type or T→A mutant AAV8 vectors. **(D)** Quantitative analyses of the data from (C). All the images are taken at the same exposure within each group (A or C) tested (207 milli-secs for A and 1.2 sec for C), gain (n=2) and intensity (n=4) in a fluorescence microscope (Leica CTR6000, GmbH, Germany). **(E)** EGFP transcript levels normalized to GAPDH expression in murine hepatocytes 4 weeks or **(F)** 2 weeks post vector administration as measured by quantitative PCR. **(G)** Comparison of the best performing AAV8 K137R mutant with luciferase as the transgene construct 2 weeks post hepatic gene transfer, with other AAV8 mutants and their quantitation data is shown in **H.** Animals are injected with 5x10¹⁰ vgs of different AAV8-Luciferase vectors are imaged 2-weeks after gene transfer in an IVIS Spect-CT small animal imaging system (Perkin Elmer, Caliper Life Sciences). Analysis of Variance (ANOVA) is used to compare gene expression between WT or mutant AAV8 treated groups.*p<0.05.

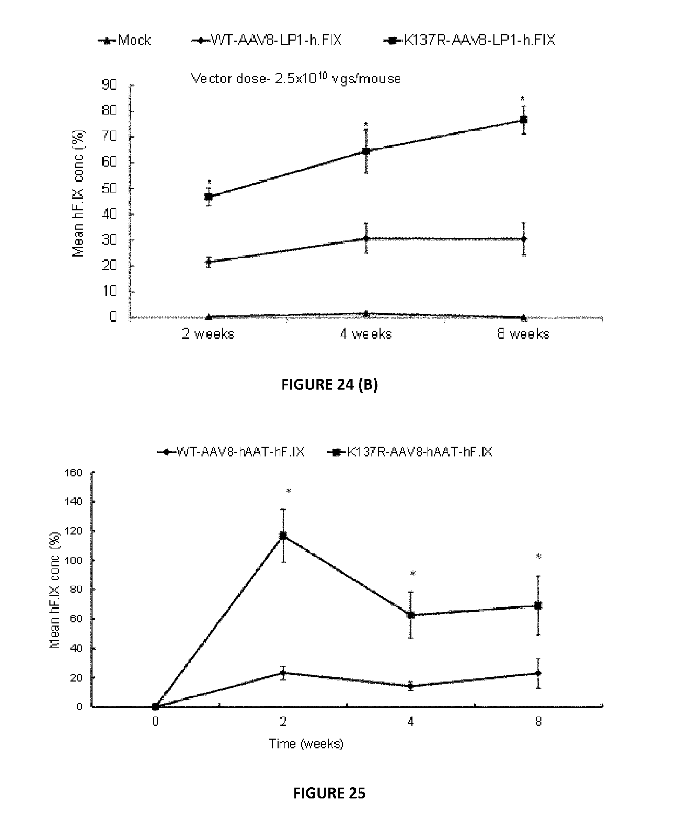

**Figure 24****:** illustrates superior hF.IX expression of K137R-AAV8 vector in comparison to WT-AAV8 vectors in C57BL/6 mice. Increased h.FIX expression from transgene constructs driven by either hAAT **(A)** or LP1 **(B)** promoters from animals injected with K137R-AAV8 vector and compared to the WT-AAV8 vector up to 8 weeks after hepatic gene transfer. *p<0.05 Vs. WT-AAV8 injected mice.
**Figure 25****:** illustrates superior hF.IX expression of K137R-AAV8 vector in comparison to WT-AAV8 vectors in hemophilia B mice. Increased h.FIX expression is seen from animals injected with K137R-AAV8-FIX vector when compared to the WT-AAV8-FIX vector up to 8 weeks after hepatic gene transfer. *p<0.05 Vs. WT-AAV8-FIX injected mice.

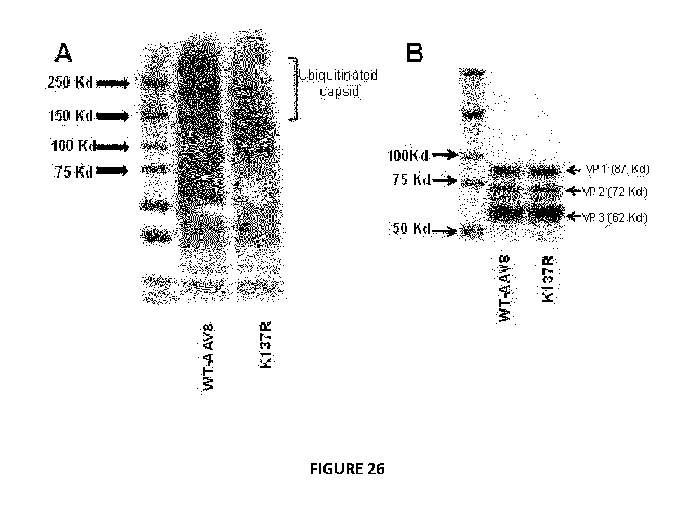

**Figure 26****:** illustrates reduced ubiquitination of K137R-AAV8 lysine mutant vector in comparison to WT-AAV8 vector. **(A)** Approximately 3 x 10⁸ viral particles of WT-AAV8 and K137R-AAV8 vectors are denatured at 95°C for 5 minutes. The denatured viral particles are then used to perform the ubiquitin conjugation assay according to the manufacturer's protocol. The processed samples are electrophoresed on a 4-20% denaturing polyacrylamide gel and the ubiquitination pattern detected by immunoblotting using an anti-ubiquitin antibody. The mono-to-polyubiquitin conjugates are detected as a smear at molecular weight >150Kda. **(B)** Approximately 3 x 10⁸ viral particles of WT-AAV8 and K137R-AAV8 vectors are denatured with RIPA buffer containing protease inhibitor cocktail at 95°C for 10 minutes. The samples are resolved in a 4-20% denaturing polyacrylamide gel and the VP1 (87Kda), VP2 (72 KDa) and VP3 (62Kda) capsid proteins are detected with AAV clone B1 antibody

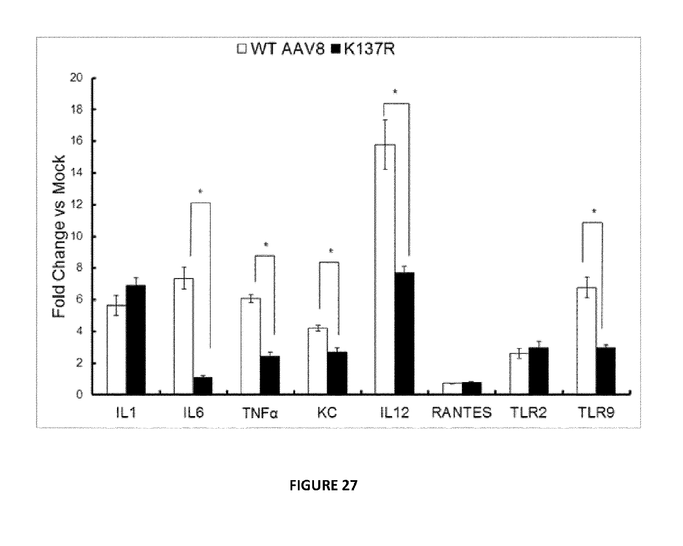

**Figure 27****:** illustrates reduced inflammatory cytokine response of K137R-AAV8 vector in comparison to WT-AAV8 vectors *in vivo.* Quantitative PCR is used to profile the hepatic expression of key pro-inflammatory cytokines and other markers of innate immune response. The relative fold change in the target gene expression from mice injected with WT-AAV8 and K137R-AAV8 vector in comparison to mock-injected animals are shown. *p<0.05 denotes statistical significance as compared to the WT-AAV8 injected mice.

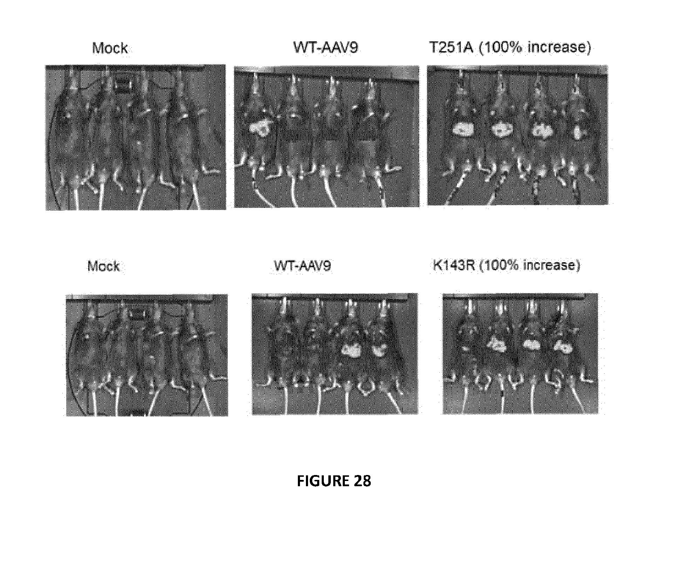

**Figure 28****:** illustrates the transduction efficiency of AAV9 vectors *in vivo.* C57BL6 mice are infected with the WT-AAV9 and the mutant vectors at Sx10¹⁰vgs per animal. Thirty (T251A) or forty-five (K143R) days post administration the luciferase activity is determined in a small animal imaging system.

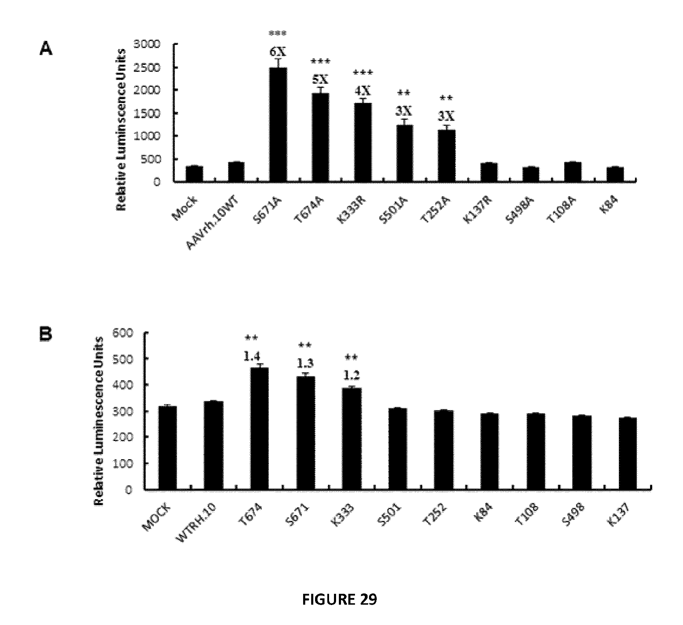

**Figure 29****:** illustrates the comparison of AAVrh.10 wild-type and mutant vectors *in vitro* in HeLa **(A)** and AAV293 cells **(B).** Fold changes in relative luminescence units observed in cells 48-hrs post-infection with WT and the nine AAVrh.10 mutants as analyzed by the luciferase detection assay.

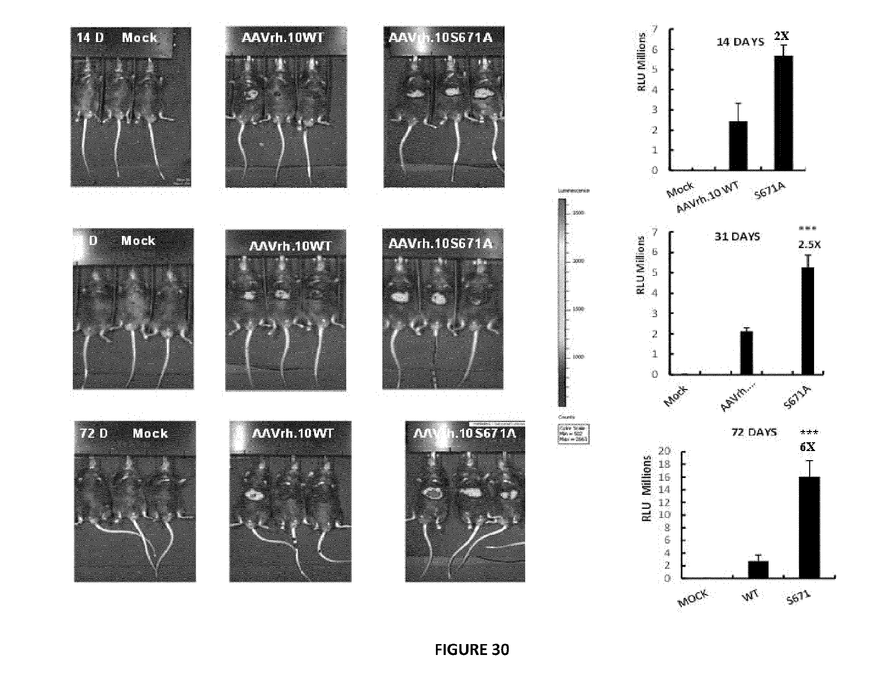

**Figure 30****:** illustrates the improved transduction efficiency of AAVrh.10-S671A vectors in C57BL/6 mice in comparison to Wild type AAVrh.10 (WT) vectors as demonstrated by bio-luminescence imaging in an IVIS Spect-CT imaging system.

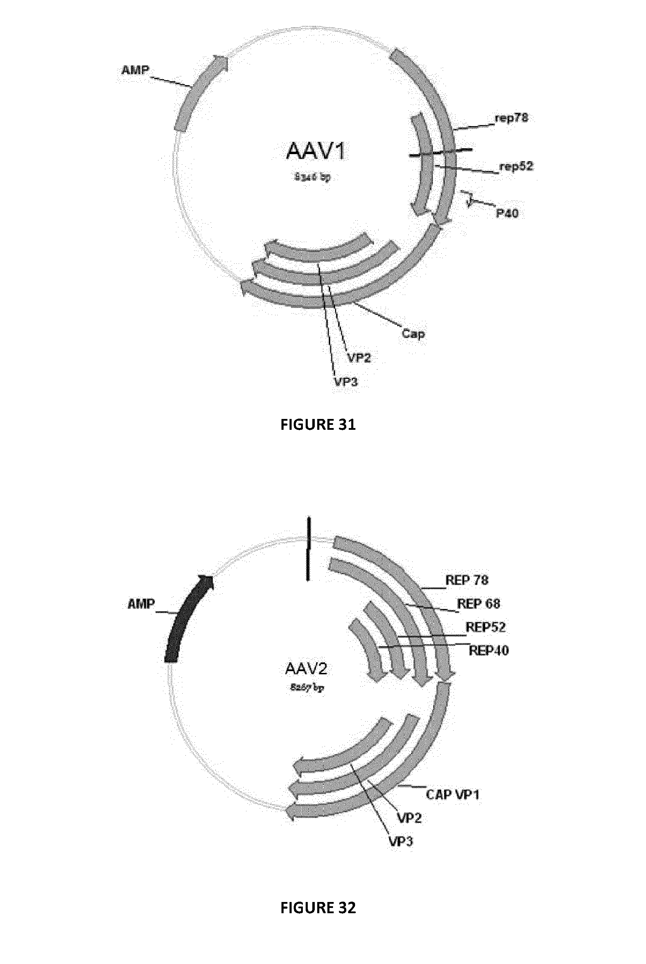

**Figure 31****:** illustrates the wild type vector construct of AAV1
**Figure 32****:** illustrates the wild type vector construct of AAV2

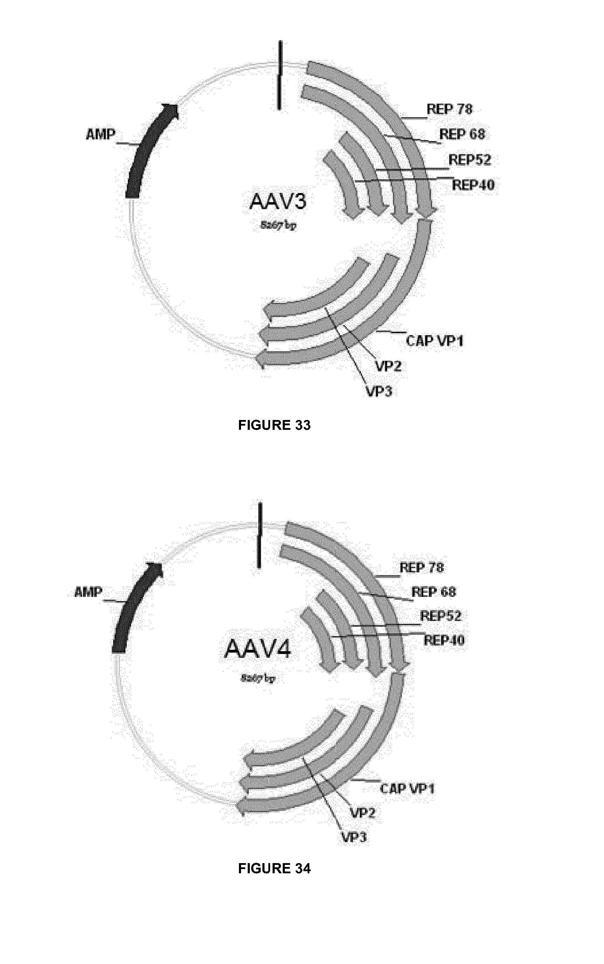

**Figure 33****:** illustrates the wild type vector construct of AAV3
**Figure 34****:** illustrates the wild type vector construct of AAV4

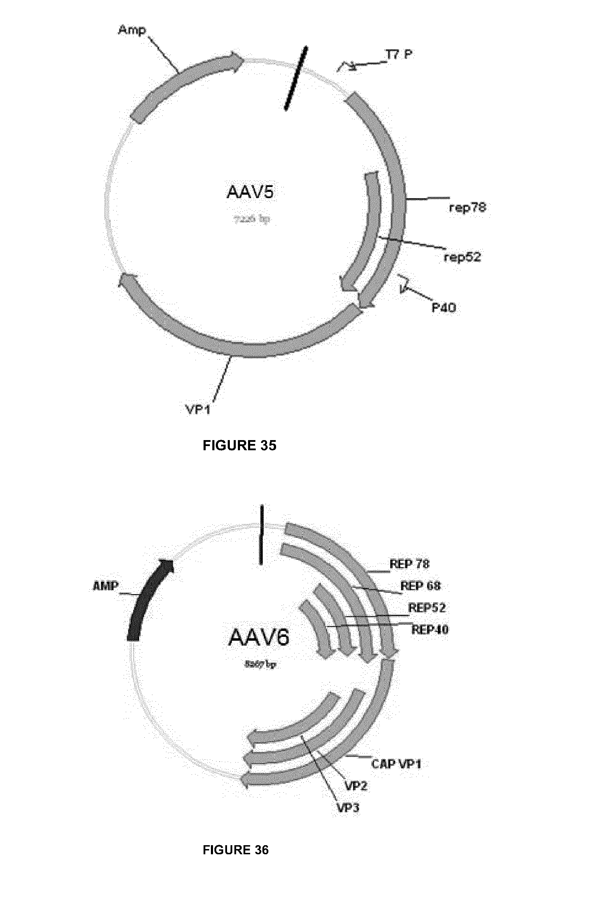

**Figure 35****:** illustrates the wild type vector construct of AAV5
**Figure 36****:** illustrates the wild type vector construct of AAV6

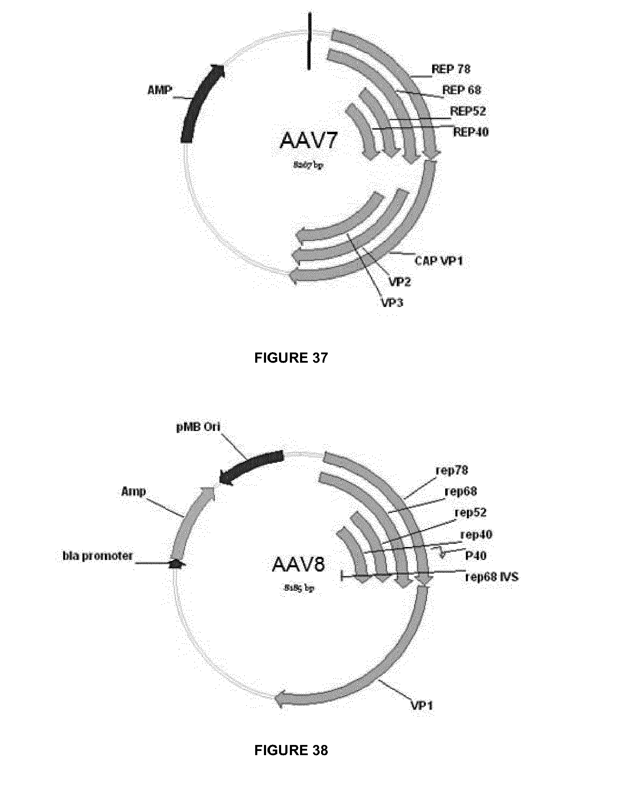

**Figure 37****:** illustrates the wild type vector construct of AAV7
**Figure 38****:** illustrates the wild type vector construct of AAV8

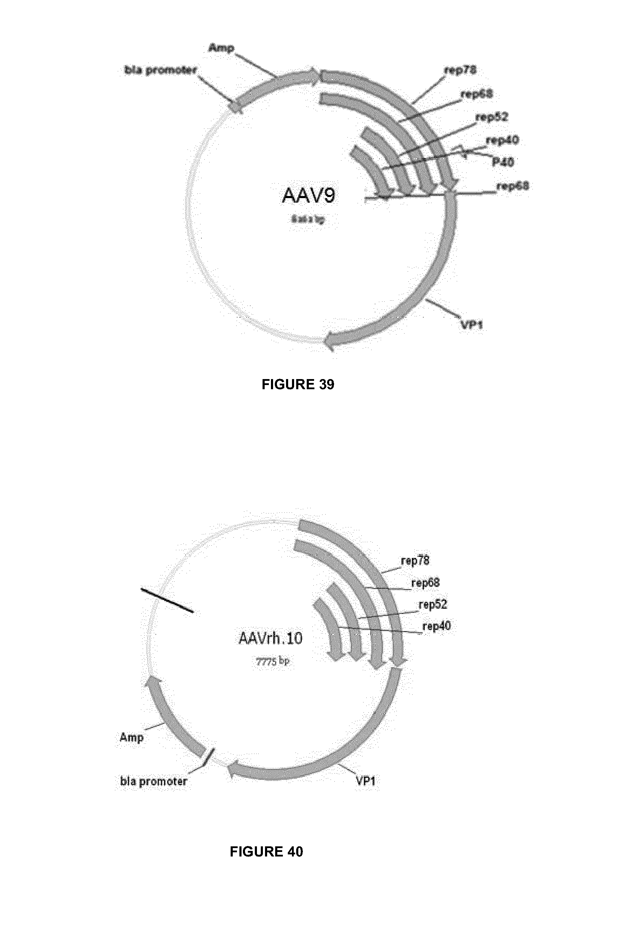

**Figure 39****:** illustrates the wild type vector construct of AAV9
**Figure 40****:** illustrates the wild type vector construct of AAV10

### DETAILED DESCRIPTION OF THE DISCLOSURE

The present disclosure relates to a nucleotide sequence selected from a group comprising SEQ ID Nos. 139 to 148, having mutation at codon selected from a group comprising TCT, TCC, TCA, TCG, AGT, AGC, ACT, ACC, ACA, ACG, AAA and AAG or any combination thereof.

In an embodiment of the present disclosure, the sequence selected from a group comprising SEQ ID Nos. 139 to 148, corresponds to serotypes 1 to 10 respectively, of wild type adeno-associated virus vector.

In another embodiment of the present disclosure, the sequence after mutation is represented by SEQ ID Nos. 149 to 158, respectively with respect to the wild type SEQ ID Nos. 139 to 148.

In yet another embodiment of the present disclosure, the sequence having SEQ ID Nos. 149 to 158, corresponds to mutated serotypes 1 to 10 respectively, of adeno-associated virus vector. In still another embodiment of the present disclosure, the codon TCT, TCC, TCA, TCG, AGT or AGC code for amino acid serine; codon ACT, ACC, ACA or ACG code for amino acid threonine; and the codon AAA or AAG code for amino acid lysine.

In still another embodiment of the present disclosure, the codon TCT, TCC, TCA, TCG, AGT, AGC, ACT, ACC, ACA or ACG is mutated to any codon selected from a group comprising GCT, GCC, GCA or GCG.

In still another embodiment of the present disclosure, the codon AAA or AAG is mutated to any codon selected from a group comprising CGT, CGC, CGA, CGG, AGA or AGG.

In still another embodiment of the present disclosure, the codon GCT, GCC, GCA or GCG code for amino acid alanine; and the codon CGT, CGC, CGA, CGG, AGA or AGG code for amino acid arginine.

In still another embodiment of the present disclosure, mutation in the codon coding for serine or threonine results in replacement of said serine or threonine amino acid with alanine amino acid.

In still another embodiment of the present disclosure, mutation in the codon coding for lysine results in replacement of said lysine amino acid with arginine amino acid.

In still another embodiment of the present disclosure, the mutation in codon TCT, TCC, TCA, TCG, AGT or AGC in any of the SEQ ID Nos. 139 to 148, occurs at position of the corresponding amino acid sequence, said position selected from a group comprising 149, 156, 268, 276, 277, 278, 279, 485, 489, 490, 492, 498, 499, 501, 525, 526, 537, 547, 652, 658, 662, 663, 668, 669 and 671 or any combination thereof.

In still another embodiment of the present disclosure, the mutation in codon ACT, ACC, ACA or ACG in any of the SEQ ID Nos. 139 to 148, occurs at position of the corresponding amino acid sequence, said position selected from a group comprising 107, 108, 138, 245, 251, 252, 328, 454, 503, 654, 671, 674, 701, 713 and 716 or any combination thereof.

In still another embodiment of the present disclosure, the mutation in codon AAA or AAG in any of the SEQ ID Nos. 139 to 148, occurs at position of the corresponding amino acid sequence, said position selected from a group comprising 32, 39, 84, 90, 137, 143, 161, 333, 490, 507, 527, 532, 544 and 652 or any combination thereof.

The present disclosure further relates to a nucleotide sequence selected from a group comprising SEQ ID Nos. 70 to 138, having mutation at codon selected from a group comprising TCT, TCC, TCA, TCG, AGT, AGC, ACT, ACC, ACA, ACG, AAA and AAG or any combination thereof.

In another embodiment of the present disclosure, the sequence selected from a group comprising SEQ ID Nos. 70 to 138, represents wild type primer sequence capable of amplifying nucleotide sequence corresponding to serotypes 1 to 10, of wild type adeno-associated virus vector.

In yet another embodiment of the present disclosure, the codon TCT, TCC, TCA, TCG, AGT, AGC, ACT, ACC, ACA or ACG is mutated to any codon selected from a group comprising GCT, GCC, GCA or GCG.

In still another embodiment of the present disclosure, the codon AAA or AAG is mutated to any codon selected from a group comprising CGT, CGC, CGA, CGG, AGA or AGG.

In still another embodiment of the present disclosure, the sequence having said mutation is selected from a sequence having SEQ ID Nos. 1 to 69.

In still another embodiment of the present disclosure, the mutated sequence act as primer for carrying out site directed mutagenesis for obtaining the mutated nucleotide sequence as mentioned above.

The present disclosure further relates to a method of obtaining a nucleotide sequence selected from a group comprising SEQ ID Nos. 139 to 148, having mutation at codon selected from a group comprising TCT, TCC, TCA, TCG, AGT, AGC, ACT, ACC, ACA, ACG, AAA and AAG or any combination thereof, said method comprising act of introducing mutation in any of nucleotide sequence selected from a group comprising SEQ ID Nos. 139 to 148 through site directed mutagenesis by using the nucleotide sequence as mentioned above.

In another embodiment of the present disclosure, the sequence selected from a group comprising SEQ ID Nos. 139 to 148, corresponds to serotypes 1 to 10 respectively, of wild type adeno-associated virus vector.

The present disclosure also relates to a method of enhancing transduction efficiency, said method comprising act of expressing a target gene in presence of a nucleotide sequence selected from a group comprising SEQ ID Nos. 139 to 148, having mutation at codon selected from a group comprising TCT, TCC, TCA, TCG, AGT, AGC, ACT, ACC, ACA, ACG, AAA and AAG or any combination thereof.

In another embodiment of the present disclosure, the sequence selected from a group comprising SEQ ID Nos. 139 to 148, corresponds to serotypes 1 to 10 respectively, of wild type adeno-associated virus vector.

In still another embodiment of the present disclosure, the transduction efficiency is enhanced with minimizing immunological response when compared with transduction carried out in presence of wild type adeno-associated virus vector having sequence selected from a group comprising SEQ ID Nos. 139 to 148.

The present disclosure also relates to a recombinant cell comprising the nucleotide sequence as mentioned above.

The present disclosure also relates to a method of obtaining the recombinant cell as mentioned above, said method comprising act of introducing the nucleotide sequence as mentioned above to a host cell, to obtain said recombinant cell.

The present disclosure also relates to a kit comprising a nucleotide sequence selected from a group comprising SEQ ID Nos. 70 to 138, having mutation at codon selected from a group comprising TCT, TCC, TCA, TCG, AGT, AGC, ACT, ACC, ACA, ACG, AAA and AAG or any combination thereof.

In another embodiment of the present disclosure, the sequence selected from a group comprising SEQ ID Nos. 70 to 138, represents wild type primer sequence capable of amplifying nucleotide sequence corresponding to serotypes 1 to 10, of wild type adeno-associated virus vector.

The instant disclosure selects specific serine, threonine, lysine residues for modification since a majority these residues are conserved among various known AAV serotypes [AAV1-10].. Further, based on *in silico* structural prediction, it is identified that certain Ser/Thr/Lys residues are close to or within the "phosphodegrons" which are sites predicted to be targets for host cellular kinase/ubiquitination machinery. Thus, on studying the compatibility of these modifications on AAV2 capsid the residues are specifically mutated as: Serine/Threonine to Alanine and Lysine to Arginine. Further, additional lysine residues are mutated to Arginine on the AAV2 capsid as predicted by UBPred software that calculates the likelihood of an amino acid sequence likely to be ubiquitinated. Upon experimental analysis, it is found that various serine, threonine and lysine mutants of AAV serotypes show an increase in transgene expression *in vitro* as well as higher transduction efficiency when administered to murine models *in vivo.* This demonstrates a superior AAV vector system for efficacious gene transfer *in vivo.* It is found that combining the various serine/threonine and lysine as multiple mutants further augments the efficiency of AAV mediated gene therapy.

In an embodiment, the instant disclosure presents that AAV serotypes which are generally targeted for destruction in the cytoplasm by the host-cellular kinase/ubiquitination/proteasomal degradation machinery, are modified at the serine/threonine kinase targets or ubiquitination targets (lysine) on AAV capsid, which improves its transduction efficiency.

In an embodiment of the present disclosure, *in-silico* structural analysis of the AAV2 capsid enables identification of three protein motifs (phosphodegrons) which are the phosphorylation sites recognized as degradation signals by ubiquitin ligases (Table 1).

**Table 1 describes the location and amino acid sequence of the three phosphodegrons in the AAV2 capsid. The predicted phosphorylation and ubiquitination sites (shown in bold font) that are highly conserved among all the serotypes of AAV within the phosphodegron region (shown enlarged) are listed. All the three phosphodegrons are solvent accessible as shown by its high average solvent accessibility.**

| **Phosphode gron** | **Amino acid position (NCBI numbering)** | **Amino acid sequence (N-C terminus)** | **Average solvent accessibility (%)** |
|---|---|---|---|
| 1 | 525-564 | SHKDDEEKFFPQSGVLIFGKQGSE KTNVDIEKVMITDEEE | 23.6 |
| 2 | 652-665 | _{PVPANPST}**T**_{F}**S**_{AA}**K** | 35.0 |
| 3 | 489-507 | **SK**_{TSADNNN}**S**_{EYSW}**T**_{GAT}**K** | 24.5 |

Point-mutations are generated in each of the serine/threonine residues to Alanine residues within or around the three phosphodegrons' residues on AAV2 capsid protein viz. S276A, S489A, S498A, S525A, S537A, S547A, S662A, S668A, T251A, T454A, T503A, T671A, T701A, T713A, and T716A (illustrated in figure 4).

In an embodiment of the present disclosure, the lysine residues are modified to arginine residues viz. K39R, K137R, K143R, K161R, K490R, K507R, K527R, K532R, and K544R.

In an embodiment of the present disclosure, the mutant AAV2 vectors have multiple combinations of AAV2 serine/threonine/arginine mutants as illustrated in Table 2 below.

**Table 2: Mutant AAV2 vectors having multiple combinations of AAV2serine/threonine/arginine mutations.**

| **DOUBLE MUTANTS** | |
|---|---|
| 1 | AAV2 K490R + K532R |
| 2 | AAV2 K527R+ K532R |
| 3 | AAV2 K532R + S489A |
| 4 | AAV2 T251A+K532R |
| | |

| **TRIPLE MUTANT** | |
|---|---|
| 1 | AAV2 T251A + K532R+ S489A |
| 2 | AAV2 S489A+S498A+K532R |

In an embodiment of the present disclosure, increased transduction efficiency of the AAV2 mutant vectors translates into enhanced therapeutic benefit in patients undergoing AAV-mediated gene-therapy. As a result, this dramatically reduces the requirement of multiple-vector administration or attenuates the host immune response due to lower mutant AAV vector doses administered, thus potentially improving the safety and efficacy of gene-therapy in humans.

**Wide-Applicability:** A majority of serine/threonine/lysine residues targeted for mutation in phosphodegrons (1-3) of AAV serotype-2 are conserved in other AAV serotypes (AAV1-10). A similar serine/threonine→alanine or lysine→arginine mutations in AAA-1, 3, 4, 5, 6, 7, 8, 9, 10 serotypes improves the transduction efficiency from these serotypes as well, translating into broad applicability in the gene therapy field for various diseases.

In an embodiment of the present disclosure, the mutant AAV vectors thus offer the following competitive advantages:
(i) improve efficiency of gene delivery;
(ii) lower the costs of gene therapy as low doses of vectors will be administered; and
(iii) promote safety of the AAV vectors by limiting dose-dependent immune-toxicity seen with conventional WT-AAV vectors.

In another embodiment of the present disclosure, each of the S/T/K residues identified in the vicinity of phosphodegron is mutated either as a single mutant, double mutant or multiple mutants.

In another embodiment of the present disclosure, vast majority of S/T/K mutant capsids will not affect the vector packaging efficiency.

In another embodiment of the present disclosure, about 15 S/T→ A mutant AAV vectors tested for their transduction efficiency at a multiplicity of infection (MOI) in HeLa cells, out of which, 11 had a significantly higher increase in EGFP-positive cells (63-97%) compared with AAV-WT vector-infected cells (41%) by FACS analysis.

In another embodiment of the present disclosure, conservation of a residue across AAV serotypes is considered an added advantage in selection for mutation of the capsid protein, which is illustrated in figures 2, 4, 18 and 21.

In another embodiment of the present disclosure, the S/T/K mutant AAV vectors have an ability to bypass natural neutralizing antibodies to WT-AAV2, and combined with its low seroprevalence in humans.

In another embodiment of the present disclosure, S/T/K residues are about 19.2% on the capsid protein of AAV2 capsid and most S/T/K mutations within AAV2 or other AAV1-10 serotypes are likely to increase transduction efficiency.

In another embodiment of the present disclosure, mutation of S/K residues enhanced the liver-directed transgene expression across all AAV serotypes.

In an embodiment, the methodology employed to arrive at Ser/Thr/Lys mutants across all serotypes is Site-directed mutagenesis as provided by Example 1 herein. Details of primers used for site-directed mutagenesis of specific Serine/threonine to Alanine and Lysine to

**Table 3: Details of primers used for site-directed mutagenesis of specific Serine/threonine to Alanine and Lysine to Arginine residues in AAV1 capsid**

| **RESIDUE** | **SEQUENCE (5' to 3')** | **NUCLEOTIDE CHANGE** | **CHANGE IN RESTRICTION ENZYME** |
|---|---|---|---|
| AAV1-S277A | Wild Type Primer Sequence: | AGC→GCC | C→A NcoI appears |
| | CAACCACTACTTCGGCTACAGCACCCCCTGGGGG TATTTTG (SEQ ID 70) | | |
| | Mutant Primer Sequence:- | | |
| | CAACCACTACTTCGGCTACGCCACCCCATGGGGG TATTTTG (SEQ ID 1) | | |
| AAV1-S499A | Wild Type Primer Sequence:- | AGC→GCC | No silent mutation |
| | CAAAAACAGACAACAACAACAGCAATTTTACCTG GACTGGTG (SEQ ID 71) | | |
| | Mutant Primer Sequence:- | | |
| | CAAAAACAGACAACAACAACGCCAATTTTACCTG GACTGGTG (SEQ ID 2) | | |
| AAV1-S526A | Wild Type Primer Sequence:- | TCA→GCA | T→C NcoI appears |
| | GCACTGCTATGGCCTCACACAAAGACGAC (SEQ ID 72) | | |
| | Mutant Primer Sequence:- | | |
| | GCACTGCCATGGCCGCACACAAAGACGAC(SEQ ID 3) | | |
| AAV1-S663A | Wild Type Primer Sequence:- | TCA→GCA | G→C SacII appears |
| | CGAATCCTCCGGCGGAGTTTTCAGCTACAAAGTTT GCTTC (SEQ ID 73) | | |
| | Mutant Primer Sequence:- | | |
| | CGAATCCTCCCGCGGAGTTTGCAGCTACAAAGTTT GCTTC (SEQ ID 4) | | |
| AAV1-S669A | Wild Type Primer Sequence:- | TCA→GCA | BsmI appears |
| | TTTCAGCTACAAAGTTTGCTTCATTCATCACCCAA TACTCC (SEQ ID 74) | | |
| | Mutant Primer Sequence:- | | |
| | TTTCAGCTACAAAGTTTGCTGCATTCATCACCCAA TACTCC (SEQ ID 5) | | |
| AAV1-T251A | Wild Type Primer Sequence:- | ACC→GCC | C→G NaeI appears |
| | CCTGGGCCTTGCCCACCTACAATAACCACC(SEQ ID 75) | | |
| | Mutant Primer Sequence:- | | |
| | CCTGGGCCTTGCCGGCCTACAATAACCACC(SEQ ID 6) | | |
| AAV1-K137R | Wild Type Primer Sequence:- | AAG→AGA | T→G, Bp1I appears |
| | CTGGTTGAGGAAGGCGCTAAGACGGCTCCTGGAA AGAAAC (SEQ ID 76) | | |
| | Mutant Primer Sequence:- | | |
| | CTGGTTGAGGAAGGCGCGAGAACGGCTCCTGGAA | | |
| | AGAAAC (SEQ ID 7) | | |

**Table 4: describes the details of primers used for site directed mutagenesis in AAV2 and the change in nucleotide and restriction pattern of silent mutations incorporated within the primers.**

| **Residue** | **Sequence (5' to 3')** | **Nucleotide change** | **CHANGE IN RESTRICTION ENZYME** |
|---|---|---|---|
| S276A | Wild type Primer sequence: | AGC→GCC | Pf1M1 appears |
| | CACTACTTTGGCTACAGCACCCCTTGGGGGTATTTTG ACTTC (SEQ ID 77) | | |
| | Mutant Primer sequence: | | |
| | CACTACTTTGGCTACGCCACCCCTTGGGGGTATTTG ACTTC (SEQ ID 8) | | |
| S489A | Wild type Primer sequence: | TCA→GCT | Alu1 appears |
| | TACCGCCAGCAGCGAGTATCAAAGACATCTGCGG (SEQ ID 78) | | |
| | Mutant Primer sequence | | |
| | TACCGCCAGCAGCGAGTAGCTAAGACATCTGCGG (SEQ ID 9) | | |
| S498A | Wild type Primer sequence: | AGT→GCT | G→A Pst1 appears |
| | AAGACATCTGCGGATAACAACAACAGTGAATACTCG TGGACT (SEQ ID 79) | | |
| | Mutant Primer sequence | | |
| | AAGACATCTGCAGATAACAACAACGCTGAATACTCG TGGACT (SEQ ID 10) | | |
| S525A | Wild type primer sequence: | AGC→GCC | C→G NcoI disappears |
| | CCGGGCCCGGCCATGGCAAGCCACAAGGACG(SEQ ID 80) | | |
| | Mutant primer sequence: | | |
| | CCGGGCCCGGCGATGGCAGCCCACAAGGACG(SEQ ID 11) | | |
| S537A | Wild type primer sequence: | AGC→GCC | BsrBI disappears |
| | AGAAAAGTTTTTCCTCAGAGCGGGGTTCTCATCTTT GGG (SEQ ID 81) | | |
| | Mutant primer sequence: | | |
| | AGAAAAGTTTTTCCTCAGGCCGGGGTTCTCATCTTT GGG (SEQ ID 12) | | |
| S547A | Wild type primer sequence: | TCA→GCC | Sfol appears |
| | CATCTTTGGGAAGCAAGGCTCAGAGAAAACAAATGT GGACA (SEQ ID 82) | | |
| | Mutant primer sequence: | | |
| | CATCTTTGGGAAGCAAGGCGCCGAGAAAACAAATGT GGACA (SEQ ID 13) | | |
| S662A | Wild type primer sequence: | AGT→GCT | AcuI disappears |
| | AATCCTTCGACCACCTTCAGTGCGGCAAAGTTTGCTT C (SEQ ID 83) | | |
| | Mutant primer sequence: | | |
| | AATCCTTCGACCACCTTCGCTGCGGCAAAGTTTGCTT C (SEQ ID 14) | | |
| S668A | Wild type primer sequence: | TCC→GCC | T→G AccI appears |
| | GCGAATCCTTCGACCACCTTCAGTGCGGCAAAGTTT GCTTCCTTCATCACA (SEQ ID 84) | | |
| | Mutant primer sequence: | | |
| | GCGAATCCGTCGACCACCTTCAGTGCGGCAAAGTTT GCTGCCTTCATCACA (SEQ ID 15) | | |
| T251A | Wild type primer sequence: | ACC→GCC | C→G Nael appears |
| | ACCCGAACCTGGGCCCTGCCCACCTACAACAACCAC CTCTAC (SEQ ID 85) | | |
| | Mutant primer sequence: | | |
| | ACCCGAACCTGGGCCCTGCCGGCCTACAACAACCAC CTCTAC (SEQ ID 16) | | |
| T454A | Wild type primer sequence: | ACC→GCC | G→A Af1 II appears |
| | TATTACTTGAGCAGAACAAACACTCCAAGTGGAACC ACCACGCAGTCAAGG (SEQ ID 86) | | |
| | Mutant primer sequence: | | |
| | TATTACTTAAGCAGAACAAACACTCCAAGTGGAGCC ACCACGCAGTCAAGG (SEQ ID 17) | | |
| T503A | Wild type primer sequence: | ACT→GCT | G→A PstI appears |
| | ACATCTGCGGATAACAACAACAGTGAATACTCGTGG ACTGGAGCTACCAAG (SEQ ID 87) | | |
| | Mutant primer sequence: | | |
| | ACATCTGCAGATAACAACAACAGTGAATACTCGTGG GCTGGAGCTACCAAG (SEQ ID 18) | | |
| T671A | Wild type primer sequence: | ACA→GCA | A→C ApaBI disappears |
| | TTCAGTGCGGCAAAGTTTGCTTCCTTCATCACACAGT ACTCCACGGGA (SEQ ID 88) | | |
| | Mutant primer sequence: | | |
| | TTCAGTGCGGCCAAGTTTGCTTCCTTCATCGCACAGT ACTCCACGGGA (SEQ ID 19) | | |
| T701A | Wild type primer sequence: | ACT→GCT | TatI disappears |
| | CGCTGGAATCCCGAAATTCAGTACACTTCCAACTAC AACAAGTCTGTT (SEQ ID 89) | | |
| | Mutant primer sequence: | | |
| | CGCTGGAATCCCGAAATTCAGTACGCTTCCAACTAC AACAAGTCTGTT (SEQ ID 20) | | |
| T713A | Wild type primer sequence: | ACT→GCT | G→C SalI appears |
| | GTTAATGTGGACTTTACTGTGGACACTAATGGCGTG TATTCAGAG (SEQ ID 90) | | |
| | Mutant primer sequence: | | |
| | GTTAATGTGGACTTTGCTGTCGACACTAATGGCGTG TATTCAGAG (SEQ ID 21) | | |
| T716A | Wild type primer sequence: | ACT→GCT | G→A AccI appears |
| | AAGTCTGTTAATGTGGACTTTACTGTGGACACTAAT GGCGTGTATTCA (SEQ ID 91) | | |
| | Mutant primer sequence: | | |
| | AAGTCTGTTAATGTAGACTTTACTGTGGACGCTAAT GGCGTGTATTCA (SEQ ID 22) | | |
| K39R | Wild type primer sequence: | AAG→AGG | G→A BsrBI appears |
| | CCAAAGCCCGCAGAGCGGCATAAGGACGACAGCAG GGGTCTTGTG (SEQ ID 92) | | |
| | Mutant primer sequence: | | |
| | CCAAAGCCCGCAGAACGGCATAGGGACGACAGCAG GGGTCTTGTG (SEQ ID 23) | | |
| K137R | Wild type primer sequence: | AAG→AGG | T→C SmaI appears |
| | GGCCTGGTTGAGGAACCTGTTAAGACGGCTCCGGGA AAAAAGAGG (SEQ ID 93) | | |
| | Mutant primer sequence: | | |
| | GGCCTGGTTGAGGAACCTGTTAGGACGGCCCCGGGA AAAAAGAGG (SEQ ID 24) | | |
| K143R | Wild type primer sequence: | AAG→AGG | T→C SmaI appears |
| | CCTGTTAAGACGGCTCCGGGAAAAAGAGGCCGGT AGAGCAC (SEQ ID 94) | | |
| | Mutant primer sequence: | | |
| | CCTGTTAAGACGGCCCCGGGAAAAGGAGGCCGGT | | |
| | AGAGCAC (SEQ ID 25) | | |
| K161R | Wild type primer sequence: | AAG→AGG | G→C NaeI appears |
| | GACTCCTCCTCGGGAACCGGAAGGCGGGCCAGCA GCCTGCAAGAAAAAGATTG (SEQ ID 95) | | |
| | Mutant primer sequence: | | |
| | GACTCCTCCTCGGGAACCGGAAGGGCCGGCCAGCAG CCTGCAAGAAAAAGATTG (SEQ ID 26) | | |
| K490R | Wild type primer sequence: | AAG→AGG | G→A PstI disappears |
| | CAGCAGCGAGTATCAAAGACATCTGCGGATAACAAC AAC (SEQ ID 96) | | |
| | Mutant primer sequence: | | |
| | CAGCAGCGAGTATCAAGGACATCTGCAGATAACAAC AAC (SEQ ID 27) | | |
| K507R | Wild type primer sequence: | AAG→AGA | Acc65I disappears |
| | TGGACTGGAGCTACCAAGTACCACCTCAATGGCAGA GAC (SEQ ID 97) | | |
| | Mutant primer sequence: | | |
| | TGGACTGGAGCTACCAGATACCACCTCAATGGCAGA GAC (SEQ ID 28) | | |
| K527R | Wild type primer sequence: | AAG→AGG | C→A NcoI disappears |
| | CCGGGCCCGGCCATGGCAAGCCACAAGGACGATGA AGAAAAG (SEQ ID 98) | | |
| | Mutant primer sequence: | | |
| | CCGGGCCCGGCAATGGCAAGCCACAGGGACGATGA AGAAAAG (SEQ ID 29) | | |
| K532R | Wild type primer sequence: | AAG→AGG | G→A BsrBI disappears |
| | CACAAGGACGATGAAGAAAAGTTTTTTCCTCAGAGC GGGGTTCTC (SEQ ID 99) | | |
| | Mutant primer sequence: | | |
| | CACAAGGACGATGAAGAAGGTTTTTCCTCAAAGC GGGGTTCTC (SEQ ID 30) | | |
| K544R | Wild type primer sequence: | AAG→AGA | G→T AccI appears |
| | GTTCTCATCTTTGGGAAGCAAGGCTCAGAGAAAACA AATGTG (SEQ ID 100) | | |
| | Mutant primer sequence: | | |
| | GTTCTCATCTTTGGTAGACAAGGCTCAGAGAAAACA AATGTG (SEQ ID 31) | | |

**Table 5: describes the details of primers used for site-directed mutagenesis of specific threonine to alanine residue in AAV3 capsid**

| **Residue** | **Sequence (5'→3')** | **Nucleotide Change** | **Change in Restriction Enzyme site** |
|---|---|---|---|
| T251A | Wild type primer Sequence | ACT→GCT | No change in Restriction Enzyme site |
| | CCTGGGCCCTGCCCACTTACAACAACCATC | | |
| | Mutated primer Sequnce (SEQ ID 101) | | |
| | CCTGGGCCCTGCCCGCTTACAACAACCATC (SEQ ID 32) | | |

**Table 6: Details of primers used for site-directed mutagenesis of specific threonine to alanine residue in AAV4 capsid**

| **Residue** | **Sequence (5'→3')** | **Nucleotide Change** | **Change in Restriction Enzyme Site** |
|---|---|---|---|
| T245A | Wild type primer Sequence | ACC→GCC | No Change in Restriction Enzyme site |
| | CCTGGGTCTTGCCCACCTACAACAACCAC (SEQ ID 102) | | |
| | Mutated primer Sequnce | | |
| | CCTGGGTCTTGCCCGCCTACAACAACCAC (SEQ ID 33) | | |

**Table 7: describes the details of primers used for site-directed mutagenesis of specific Serine/threonine to Alanine and Lysine to Arginine residues in AAV5**

| **RESIDUE** | **SEQUENCE (5' to 3')** | **NUCLEOTID ECHANGE** | **CHANGE IN RESTRICTIO N ENZYME** |
|---|---|---|---|
| AAV5-S268A | Wild Type Primer Sequence:- | AGC→GCC | No silent mutations |
| | AACGCCTACTTTGGATACAGCACCCCCTGGGGT AC (SEQ ID 103) | | |
| | Mutant Primer Sequence:- | | |
| | GCCTACTTTGGATACGCCACCCCCTGGGGG(SEQ ID 34) | | |
| AAV5-S485A | Wild Type Primer Sequence:- | AGT→GCT | TSPR I disappears |
| | CGGGGTCAACCGCGCCAGTGTCAGCGCCTTCGCC (SEQ ID 104) | | |
| | Mutant Primer Sequence:- | | |
| | GGTCAACCGCGCCGCTGTCAGCGCCTTC (SEQ ID 35) | | |
| AAV5-S652A | Wild Type Primer Sequence:- | TCG→GCG | HPYI 881 disappears |
| | GAAATATCACCAGCTTCTCGGACGTGCCCGTCAGC (SEQ ID 105) | | |
| | Mutant Primer Sequence:- | | |
| | ATATCACCAGCTTCGCGGACGTGCCCGTC (SEQ ID 36) | | |
| AAV5-S658A | Wild Type Primer Sequence:- | AGC→GCC | ALU I disappears |
| | TCGGACGTGCCCGTCAGCAGCTTCATCACCCAGTA CAG (SEQ ID 106) | | |
| | Mutant Primer Sequence:- | | |
| | GACGTGCCCGTCAGCGCCTTCATCACCCAGTA (SEQ ID 37) | | |
| AAV5-K32R | Wild Type Primer Sequence:- | AAA→AGA | No silent mutation |
| | AAGCGGGCCCACCGAAACCAAAACCCAATCAGCA G (SEQ ID 107) | | |
| | Mutant Primer Sequence:- | | |
| | CGGGCCCACCGAAACCAAGACCCAATCAG(SEQ ID 38) | | |
| AAV5-T107A | Wild Type Primer Sequence:- | ACA→GCA | MWO I appears |
| | AAGCTCGCCGACGACACATCCTTCGGGGAA(SEQ ID 108) | | |
| | Mutant Primer Sequence:- | | |
| | CTCGCCGACGACGCATCCTTCGGGG (SEQ ID 39) | | |
| AAV5-T328A | Wild Type Primer Sequence:- | ACC→GCC | HPH I disappears |
| | CGCCAACAACCTCACCTCCACCGTCCAAGT (SEQ ID 109) | | |
| | Mutant Primer Sequence:- | | |
| | CGCCAACAACCTCGCCTCCACCGTCCA (SEQ ID 40) | | |

**Table 8: describes the details of primers used for site-directed mutagenesis of specific threonine to alanine residue in AAV6 capsid**

| **Residue** | **Sequence (5'→3')** | **Nucleotide Change** | **Change in Restriction Enzyme site** |
|---|---|---|---|
| T251A | Wild type primer Sequence | ACC→GCC | C→G, Nae I appears |
| | CATGGGCCTTGCCCACCTATAACAACCAC (SEQ ID 110) | | |
| | Mutated primer Sequence | | |
| | CATGGGCCTTGCCGGCCTATAACAACCA (SEQ ID 41) | | |

**Table 9: describes the details of primers used for site-directed mutagenesis of specific serine to alanine residue in AAV7 capsid**

| **Residue** | **Sequence (5'→3')** | **Nucleotide Change** | **Change in Restriction Enzyme site** |
|---|---|---|---|
| T252A | Wild type primer Sequence | ACC→GCC | No Change in Restriction Enzyme site |
| | CTGGGCCCTGCCCACCTACAACAACCA (SEQ ID 111) | | |
| | Mutated primer Sequence | | |
| | CTGGGCCCTGCCCGCCTACAACAACCA (SEQ ID 42) | | |

**Table 10: describes the details primers used for site directed mutagenesis in AAV8 capsid and the change in nucleotide and restriction pattern of silent mutation obtained with the primers.**

| **RESIDUE** | **SEQUENCE (5' to 3')** | **NUCLEOTIDE CHANGE** | **CHANGE IN RESTRICTION ENZYME DUE TO** SILENT MUTATION |
|---|---|---|---|
| S149A | Wild Type Primer Sequence:- | TCA →GCA | No silent mutation |
| | GAGACCGGTAGAGCCATCACCCCAGCGT TCTCC (SEQ ID 112) | | |
| | Mutant Primer Sequence:- | | |
| | GAGACCGGTAGAGCCAGCACCCCAGCG TTCTCC (SEQ ID 43) | | |
| | Wild Type Primer Sequence:- | | |
| S156A | CCCAGCGTTCTCCAGACTCCTCTACGGG CATCGGC (SEQ ID 113) | TCC→GCC | No silent mutation |
| | Mutant Primer Sequence:- | | |
| | CCCAGCGTTCTCCAGACGCCTCTACGGG CATCGGC (SEQ ID 44) | | |
| S279A | Wild Type Primer Sequence: | AGC→GCC | SfcI disappears |
| | CTACTTCGGCTACAGCACCCCCTGGGGG (SEQ ID 114) | | |
| | Mutant Primer Sequence:- | | |
| | CTACTTCGGCTACGCCACCCCCTGGGGG (SEQ ID 45) | | |
| S501A | Wild Type Primer Sequence: | AGC→GCC | G→T AgeI appears |
| | GACAACCGGGCAAAACAACAATAGCAA CTTTGCCTGGACT (SEQ ID 115) | | |
| | Mutant Primer Sequence:- | | |
| | GACAACCGGTCAAAACAACAATGCCAA CTTTGCCTGGACT (SEQ ID 46) | | |
| S671A | Wild Type Primer Sequence:- | TCT→GCT | T→A AluI disappears |
| | CCTTCAACCAGTCAAAGCTGAACTCTTT CATCACGCAATACA (SEQ ID 116) | | |
| | Mutant Primer Sequence:- | | |
| | CCTTCAACCAGTCAAAGCAGAACGCTTT CATCACGCAATACA (SEQ ID 47) | | |
| | Wild Type Primer Sequence:- | | |
| T138A | TTGAGGAAGGCGCTAAGACGGCTCCTGG AAAGAAG (SEQ ID 117) | | |
| | Mutant Primer Sequence:- | | |
| | TTGAGGAAGGCGCTAAGGCGGCTCCTG GAAAGAAG (SEQ ID 48) | ACG→GCG | No silent mutation |
| T252A | Wild type primer sequence: | ACC→GCC | C→G NaeI appears |
| | ACCCGAACCTGGGCCCTGCCCACCTACA ACAACCACCTCTAC (SEQ ID 118) | | |
| | Mutant primer sequence: | | |
| | ACCCGAACCTGGGCCCTGCCGGCCTACA ACAACCACCTCTAC (SEQ ID 49) | | |
| | Wild Type Primer Sequence:- | | |
| | AGATCCTGATCAAGAACACGCCTGTACC TGCGGATCCTCC (SEQ ID 119) | ACG→GCG | T→A appearance of BstUI,HinplI, HhaI, BsrI |
| T654A | Mutant Primer Sequence:- | | |
| | AGATCCTGATCAAGAACGCGCCAGTACC TGCGGATCCTCC (SEQ ID 50) | | |
| | Wild Type Primer Sequence:- | | |
| T674A | AAAGCTGAACTCTTTCATCACGCAATAC AGCACCGGACA (SEQ ID 120) | | No silent mutation |
| | Mutant Primer Sequence:- | | |
| | AAAGCTGAACTCTTTCATCGCGCAATAC AGCACCGGACA (SEQ ID 51) | ACG→GCG | |
| K137R | Wild Type Primer Sequence:- | AAG→AGG | C→G HhaI disappears |
| | GGTTGAGGAAGGCGCTAAGACGGCTCCT GG (SEQ ID 121) | | |
| | Mutant Primer Sequence:- | | |
| | GGTTGAGGAAGGGGCTAGGACGGCTCCT GG (SEQ ID 52) | | |
| | Wild Type Primer Sequence:- | | |
| K143R | AGGCGCTAAGACGGCTCCTGGAAAGAA GAGACCGGTAGAGCCA (SEQ ID 122) | AAG→AGA | No silent mutation |
| | Mutant Primer Sequence:- | | |
| | AGGCGCTAAGACGGCTCCTGGAAAGAG AAGACCGGTAGAGCCA (SEQ ID 53) | | |
| | Wild Type Primer Sequence:- | AAG→CGA | |
| K652R | TGGCCTGAAACATCCTCCGCCTCAGATC CTGATCAAGAACACGCCTGTACCTGCGG (SEQ ID 123) | | C→T disappearance of BclI, DpnI, Hpy188I |
| | Mutant Primer Sequence:- | | |
| | TGGCCTGAAACATCCTCCGCCTCAGATC CTGATTCGAAACACGCCTGTACCTGCGG (SEQ ID 54) (SEQ ID 54) | | |

**Table 11: describes the details of primers used for site-directed mutagenesis of specific Serine/threonine to Alanine and Lysine to Arginine residues in AAV9 capsid**

| **RESIDUE** | **SEQUENCE (5' to 3')** | **NUCLEOTIDE CHANGE** | **CHANGE IN RESTRICTION ENZYME DUE TO** SILENT MUTATION |
|---|---|---|---|
| AAV9-S278A | Wild Type Primer Sequence:- | AGC→GCA | No silent mutation |
| | CAACGCCTACTTCGGCTACAGCACCCCCTGGGG GTATTTTG (SEQ ID 124) | | |
| | Mutant Primer Sequence:- | | |
| | CAACGCCTACTTCGGCTACGCAACCCCCTGGGG GTATTTTG (SEQ ID 55) | | |
| AAV9-S490A | Wild Type Primer Sequence:- | TCA→GCG | No silent mutation |
| | CAGCTACCGACAACAACGTGTCTCAACCACTGT GACTCAAAACAACA (SEQ ID 125) | | |
| | Mutant Primer Sequence:- | | |
| | CAGCTACCGACAACAACGTGTCGCGACCACTGT GACTCAAAACAACA (SEQ ID 56) | | |
| AAV9-S669A | Wild Type Primer Sequence:- | TCT→GCC | |
| | GCCTTCAACAAGGACAAGCTGAACTCTTTCATC ACCCAGTATTCTACTGGCCAA (SEQ ID 126) | | |
| | Mutant Primer Sequence:- | | C→T HypaV disappears |
| | GCCTTCAACAAGGACAAGCTGAACGCCTTTATC ACCCAGTATTCTACTGGCCAA (SEQ ID 57) | | |
| AAV9-S499A | Wild Type Primer Sequence:- | AGC→GCC | A→G Tsp509I, ApoI disappears |
| | CCACTGTGACTCAAAACAACAACAGCGAATTTG CTTGGCCTGGAGCTTC (SEQ ID 127) | | |
| | Mutant Primer Sequence:- | | |
| | CCACTGTGACTCAAAACAACAACGCCGAGTTTG CTTGGCCTGGAGCTTC (SEQ ID 58) | | |
| AAV9-T251A | Wild Type Primer Sequence:- | ACC→GCC | disappears |
| | CCCGAACCTGGGCCCTGCCCACCTACAACAATC ACCTCTA (SEQ ID 128) | | |
| | Mutant Primer Sequence:- | | C→A AciI |
| | CCCGAACCTGGGCCCTGCCAGCCTACAACAATC ACCTCTA (SEQ ID 59) | | |
| AAV9-K143R | Wild Type Primer Sequence:- | AAG→AGA | MboII disappears |
| | AGCGGCTAAGACGGCTCCTGGAAAGAAGAGGC CTGTAGAGCAG (SEQ ID 129) | | |
| | Mutant Primer Sequence:- | | |
| | AGCGGCTAAGACGGCTCCTGGAAAGAGAAGGC CTGTAGAGCAG (SEQ ID 60) | | |

**Table 12: describes the details of primers used for site-directed mutagenesis of specific Serine/Threonine to Alanine and Lysine to Arginine residues in AAVrh.10**

| **RESIDUE** | **SEQUENCE (5' to 3')** | **NUCLEOTIDE CHANGE** |
|---|---|---|
| K84R | Wild Type Primer Sequence:- | AAA→AGA |
| | GCCTACGACCAGCAGCTCAAAGCGGGTGAC (SEQ ID 130) | |
| | Mutant Primer Sequence:- | |
| | GCCTACGACCAGCAGCTCAGAGCGGGTGAC (SEQ ID 61) | |
| K137R | Wild Type Primer Sequence:- | AAG→AGG |
| | TGGTTGAGGAAGGCGCTAAGACGGCTCCT (SEQ ID 131) | |
| | Mutant Primer Sequence:- | |
| | TGGTTGAGGAAGGCGCTAGGACGGCTCCT (SEQ ID 62) | |
| K333R | Wild Type Primer Sequence:- | AAG→AGG |
| | GCAGAATGAAGGCACCAAGACCATCGCCAATAACC ID 132) | |
| | Mutant Primer Sequence:- | |
| | GCAGAATGAAGGCACCAGGACCATCGCCAATAACC (SEQ ID 63) | |
| S492A | Wild Type Primer Sequence:- | TCC→GCC |
| | GCAGCAACGCGTCTCCACGACACTGTC (SEQ ID 133) | |
| | Mutant Primer Sequence:- | |
| | GCAGCAACGCGTCGCCACGACACTGTC (SEQ ID 64) | |
| S501A | Wild Type Primer Sequence:- | AGC→GCC |
| | CTGTCGCAAAATAACAACAGCAACTTTGCCTGGACCGG(SEQ ID 134) | |
| | Mutant Primer Sequence:- | |
| | CTGTCGCAAAATAACAACGCCAACTTTGCCTGGACCGG(SEQ ID 65) | |
| S671A | Wild Type Primer Sequence:- | TCG→GCG |
| | CAAGCTAAGCTGGCGTCGTTCATCACGCAGT (SEQ ID 135) | |
| | Mutant Primer Sequence:- | |
| | CAAGCTAAGCTGGCGGCGTTCATCACGCAGT (SEQ ID 66) | |
| T108A | Wild Type Primer Sequence:- | ACG→GCG |
| | GCGTCTGCAAGAAGATACGTCTTTTGGGGGC (SEQ ID 136) | |
| | Mutant Primer Sequence:- | |
| | GCGTCTGCAAGAAGATGCGTCTTTTGGGGGC (SEQ ID 67) | |
| T252A | Wild Type Primer Sequence:- | ACC→GCC |
| | CTGGGCCCTCCCCACCTACAACAACCA (SEQ ID 137) | |
| | Mutant Primer Sequence:- | |
| | CTGGGCCCTCCCCGCCTACAACAACCA (SEQ ID 68) | |
| T674A | Wild Type Primer Sequence:- | ACG→GCG |
| | TGGCGTCGTTCATCACGCAGTACAGCACC (SEQ ID 138) | |
| | Mutant Primer Sequence:- | |
| | TGGCGTCGTTCATCGCGCAGTACAGCACC (SEQ ID 69) | |

In an embodiment, the aim of the present invention is to arrive at AAV vectors having mutations in their capsid protein. Such mutated AAV vectors comprise mutations in either Serine, Threonine or Lysine amino acids, or any combination of mutations thereof. Further, within a AAV vector sequence, such mutations may occur at one or multiple places and any combination of such mutations fall within the purview of this invention. Sequences provided by SEQ ID Nos. 148 to 157 only represent examples of such vector sequences having all the mutations in each serotype, and should not be construed to limit the instant invention to only these sequences. The aim of the invention is to cover AAV vector sequences which may comprise either one, either few or either all of the mutations provided by SEQ ID Nos. 148 to 157.

The disclosure is further illustrated by the following Examples. The following examples are included to demonstrate preferred embodiments of the disclosure. It should be appreciated by those of skill in the art that the techniques disclosed in the examples which follow represent techniques discovered by the inventors to function well in the practice of the disclosure, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the disclosure. The present disclosure is not to be limited in scope by the specific embodiments described herein, which are intended as illustrations of particular aspects of the disclosure, and functionally equivalent methods and components are within the scope of the disclosure. Indeed, various modifications of the disclosure, in addition to those shown and described herein, will become apparent to those skilled in the art from the foregoing description. Such modifications are intended to fall within the scope of the appended claims.

### EXAMPLES:

### Structural analysis of AAV Capsid:

### Structural analysis of AAV2 capsid:

The three-dimensional structure of the AAV2 capsid from the protein Data bank (PDB accession number 1LP3) is analyzed extensively. Protein-protein interaction interface residues on the capsid proteins are determined by accessibility-based method. Solvent accessibility values of the residues are determined with the NACCESS program. Phosphorylation sites in capsid protein are predicted with NetPhosK, kinasePhos and Scansite, prediction of k-spaced Amino Acid Pairs and prediction of Ubiquitination sites with Bayesian discriminant Method.

Structures are visualized with the PyMOL software package. To assess the conservation of the predicted phosphorylation as well as ubiquitination sites, multiple sequence alignment of the VP1 sequence across the 10 serotypes is generated with ClustalW.

### Structural analysis of AAV8 capsid:

The three dimentsional structure of the AAV8 capsid (PDB code-2qa0) is analyzed extensively to determine interaction interfaces of capsid protein chains. Accessibility-based method is employed to determine the residues participating in the protein-protein interactions between capsid proteins. Solvent accessibility of every residue is computed using NACCESS tool and the residues are grouped as solvent-exposed if the solvent accessibility values are more than 7%, while those with lesser accessibility are called buries residues. The residues are called interface residues if they are buried (accessibility <7%) in protein complex while being solvent-exposed (accessibility .10%) in isolated chains.

The structure of the viral capsid is visualized using PYMOL software and compared to the structure of AAV2 capsid using DALIlite tool for structure-based comparison.

The above mentioned structural analysis is similarly performed for other serotypes (AAA1, AAV3-AAV7 and AAV9-AAV10). From the above explanation the person skilled in the art will be able to perform the structural analysis for AAV serotypes without any undue experimentation.

### Methodology to arrive at Ser/Thr/Lys mutants across all serotypes by Site-directed mutasenesis:

Site directed mutagenesis is performed on wild type rep-cap plasmid pACG2/R2C by Quik Change II XL Site-Directed Mutagenesis Kit (Stratagene, CA, USA) as per the manufacturer's protocol. Briefly, a one step PCR is performed for 18 cycles with the mutation containing primers followed by DpnI digestion for 1h. Primers are designed to introduce amino acid change from serine/threonine to alanine or lysine to arginine (refer table 4). Transformation of XL10-Gold Ultracompetent Cells is carried out with 2µL of DPN1 digested DNA followed by plating in agar plates containing ampicillin according to the manufacturer's protocol (Stratagene). Plasmids isolated from colonies are confirmed for the presence of the desired point mutation by restriction digestion and DNA sequencing, prior to using them for packaging viral vectors.

### Production of recombinant AAV2 vectors:

Highly purified stocks of self-complementary (sc) AAV2- WT or 26 capsid mutants of AAV2 vectors or AAV8-WT vector carrying the enhanced green fluorescent protein (EGFP) gene driven by the chicken b-actin promoter are generated by polyethyleneimine-based triple transfection of AAV-293 cells. Briefly, forty 150-mm² dishes 80% confluent with AAV-293 cells are transfected with AAV2 rep/cap (p.ACG2), transgene (dsAAV2-EGFP), and AAV-helper free (p.helper) plasmids. Cells are collected 72hr post transfection, lysed, and treated with Benzonase nuclease (25 units/ml; Sigma-Aldrich). Subsequently, the vectors are purified by iodixanol gradient ultracentrifugation (OptiPrep; Sigma-Aldrich) followed by column chromatography (HiTrap SP column; GE Healthcare Life Sciences, Pittsburgh, PA). The vectors are finally concentrated to a final volume of 0.5 ml in phosphate-buffered saline (PBS), using Amicon Ultra 10K centrifugal filters (Millipore, Bedford, MA). The physical particle titers of the vectors are quantified by slot-blot analysis and expressed as vector genomes per millilitre.

### Production of recombinant AAV8 vectors:

Highly purified stocks of self-complementary wild-type (WT) AAV8, or the mutant AAV8 vectors encoding the enhanced green fluorescent protein (EGFP) gene driven by the chicken b-actin promoter containing the cytomegalovirus (CMV) enhancer and SV40 poly A signal or the human coagulation factor IX (h.FIX) under the control of liver-specific promoters, human alpha-1-antitrypsin (hAAT) or LP1 promoter (consisting of core liver specific elements from human apolipoprotein hepatic control region) are generated by polyethyleneimine-based triple transfection of AAV-293 cells (Stratagene). Briefly, forty numbers of 150-mm² dishes 80% confluent with AAV 293 cells are transfected with AAV8 rep-cap, transgene-containing and AAV-helper free (p.helper) plasmids. Cells are collected 72hr post-transfection, lysed, and treated with 25 units/ml of benzonase nuclease (Sigma Aldrich, St Louis, MO). Subsequently, the vectors are purified by iodixanol gradient ultracentrifugation (Optiprep, Sigma Aldrich) followed by column chromatography (HiTrap Q column, GE Healthcare, Pittsburgh, PA). The vectors are finally concentrated to a final volume of 0.5 ml in phosphate buffered saline (PBS) using Amicon Ultra 10K centrifugal filters (Millipore, Bedford, MA). The physical particle titers of the vectors are quantified by slot blot analysis and expressed as viral genomes (vgs)/ml.

### Recombinant AAV vector transduction assays in vitro

To assess the effect of pharmacological inhibition of cellular serine/threonine kinases on AAV transduction, approximately 1.6×10⁵ HeLa cells are mock (PBS)-treated or pretreated with optimal concentrations of PKA inhibitor (25 nM), PKC inhibitor (70 nM), or CKII inhibitor (1 1M), or with a combination of each of these inhibitors overnight and transduced with AAV-WT vector at 2×10³ VG/cell. The safe and effective concentration of kinase inhibitors used is determined by 3-(4,5-dimethylthiazol-2-yl)-2,5- diphenyltetrazolium bromide (MTT) assay, performed with three 10-fold dilutions around the median inhibition constant (IC₅₀) values for these small-molecule inhibitors. Twenty-four hours later, transgene expression is measured by flow cytometry (FACS Calibur; BD Biosciences, San Jose, CA). A total of 1×10⁴ events are analyzed for each sample. Mean values of percent EGFP positivity from three replicate samples are used for comparison between treatment groups. To assess the efficacy of the novel mutant vectors generated, HeLa or HEK-293 cells are mock-infected or infected with either AAV-WT or AAV S/T/K mutant vector (2103 VG/cell). Forty-eight hours post-transduction, transgene expression is quantitated by flow cytometry (FACSCalibur; BD Biosciences) or captured by EGFP imaging. For flow cytometric analysis, HeLa or HEK-293 cells are trypsinized (0.05% trypsin; Sigma-Aldrich) and rinsed twice with PBS (pH 7.4). A total of 1×10⁴ events are analyzed for each sample. In total, three independent experiments are performed including three intraassay replicates in each of the experiment. Mean values of percent GFP positivity from these nine replicate samples are used for comparison between AAV-WT- and AAV S/T/K-infected cells.

### Result and conclusion:

*In silico* analysis of the AAV2 capsid structure using various phosphorylation prediction tools identified PKA, PKC and CKII kinases as major binding partners of phosphodegrons of AAV2 capsid. Since these enzymes are primarily serine/threonine kinases with an ability to phosphorylate S/T residues, the kinase activity is inhibited by specific small molecule inhibitors and then infected the HeLa cells with scAAV2-EGFP vector. As described in Fig. 5A/B, a significantly higher gene expression of the AAV2-WT vector is observed when HeLa cells are pre-treated with these kinase inhibitors, with a maximal 90% increase seen in cells treated with the CKII inhibitor. This demonstrates that one or more surface-exposed serine and/or threonine amino acid in the AAV2 capsid is phosphorylated within the host cell by PKA, PKC and CKII serine/threonine kinases and specific inhibition of this process improves the gene expression from AAV vectors. Since, systemic administration of serine/threonine kinase inhibitors in an in vivo setting is likely to be toxic (Force and Kolaja, 2011), we instead chose to modify the kinase target substrates in the AAV2 capsid to further improve the transduction efficiency of AAV2 vectors with negligible or no toxicity/side-effects. The transduction potential of nine single-mutant and two double mutant AAV2 K→R vectors in HeLa cells at an MOI of 2000. The K532R and K544R single mutants and one double mutant (K490+532R) showed transduction efficacy of about 70% to about 82% when compared 30% efficacy in AAV2-WT vector. Similar results are observed for the mutant vectors- T251A, S276A, S489A, S498A, and K532R in HEK-293 cells.

A mutant is deduced to have an improved transduction profile, if it shows an increase in GFP gene expression over and above the wild-type AAV vectors, by either FACS analysis or by fluorescence imaging. Figures 5, 6 and 17 A demonstrates a significant increase in EGFP gene expression for the various Ser/Thr/Lys mutants tested by either FACS or by fluorescence microscopy. A maximal increase can be seen in vitro with S668A (7 fold), T251A (5.5 fold) or K532R (13.5 fold) mutants. This increase is further shown qualitatively for the Ser/Thr>Ala mutants as illustrated in figures 7A, 8, or Lys>Arg mutants as illustrated in figures 7B, 9 in HeLa cells by fluorescence imaging. Further, figure 12 illustrates the fluorescence imaging of single and double or triple mutant vectors in AAV2, while Fig 23G/H shows luciferase imaging of AAV8 double mutant vector.

### RECOMBINANT AAV VECTOR TRANSDUCTION STUDIES IN VIVO

C57BL/6 mice are purchased from Jackson Laboratory (Bar Harbor, ME). All animal experiments are approved and carried out according to the institutional guidelines for animal care (Christian Medical College, Vellore, India). Groups (n = 4 per group) of 8 to 12 week old C57BL/6 mice are mock-injected or injected with 5×10¹⁰ VG each of scAAV-WT or scAAV S/T/K mutant vector carrying the EGFP transgene, via the tail vein. Mice are euthanized 4 weeks after vector administration. Cross-sections from three hepatic lobes of the mock-injected and vector-injected groups are assessed for EGFP expression by fluorescence microscopy.

### Estimation of AAV vector genome copies and EGFP expression in murine hepatocytes by Quantitative PCR analysis:

Groups of 8- to 12-week-old C57BL/6 mice (n = 4-8 per group) are mock-injected or injected with 5×10¹⁰ vgs each of WT-AAV or AAV mutant vectors, containing EGFP as the transgene, via the tail vein. Mice are euthanized 2 or 4 weeks after vector administration. Cross-sections from three hepatic lobes of the mock-injected and vector-injected groups are assessed for EGFP expression by a fluorescence microscope (Leica CTR6000; GmbH, Stuttgart, Germany). Images from five visual fields of mock-infected and vector infected cells are analyzed by ImageJ analysis software (NIH, Bethesda, MD). Transgene expression (mean value) is assessed as total area of green fluorescence and expressed as mean pixels per visual field (mean - SD). The best performing AAV capsid mutant, along with the WT AAV vector containing h.FIX as the transgene (under LP1 and hAAT promoter), are administered into 8 to 12 week-old male C57BL/6 mice (n = 3-4 per group) intravenously at different doses (2.5×10¹⁰ and 1×10¹¹ vgs per mouse). Blood is collected retro-orbitally 2, 4, and 8 weeks post-vector administration. The antigenic activity of hF.IX (FIX:Ag) is measured using a commercial kit (Asserachrom, Diagnostica Stago, Asniers, France).

Result: The circulating levels of h.FIX are higher in all the K137R AAVB-treated groups as compared to the WT-AAV8- treated groups either at 2 weeks (62% vs. 37% for hAAT constructs and 47% vs. 21% for LP1 constructs), 4 weeks (78% vs. 56% for hAAT constructs and 64% vs. 30% for LP1 constructs) or 8 weeks (90% vs. 74% for hAAT constructs and 77% vs. 31% for LP1 constructs) post-hepatic gene transfer. These expression levels further corroborate the potential of the K137R mutant for hepatic gene therapy of hemophilia B The K137R mutant has an increased level of h.FIX expression for up to 2 months post hepatic gene transfer as described in figures 24 and 25.

### Biodistribution studies:

Liver, spleen, lung, heart, kidney, and muscle tissue are collected from each of the mice administered with either WT-AAV or AAV S/T/K mutant vectors, 2 or 4 weeks after gene transfer. Genomic DNA is isolated using the QIAamp DNA Mini Kit (Qiagen, Valencia, CA) according to the manufacturer's protocol. Quantitative polymerase chain reaction (PCR) is carried out to estimate the vector copy numbers in 100 ng of template genomic DNA by amplifying the viral inverted terminal repeats (ITRs) with specific probes/primers as described using a low ROX qPCR mastermix according to manufacturer's protocol (Eurogentec, Seraing, Belgium). Data is captured and normalized to mouse glyceraldehyde-3-phosphate dehydrogenase (GAPDH) housekeeping control gene and analyzed in an ABI Prism 7500 Sequence Detection System Version 1.1 Software (Life Technologies, Applied Biosystems).

### Real-time PCR assay:

Groups (n = 4 per group) of 8- to 12-week-old C57BL/6 mice are mock-injected or injected with 1×10¹¹ vgs each of WT-AAV or AAV S/T/K mutant vectors intravenously. Two hours later, mice are euthanized. Total RNA is isolated from liver sections of each mouse using the NucleoSpin_RNA isolation kit (Machery-Nagel, Du" ren, Germany). Approximately 2µg of RNA is reverse transcribed using the first-strand RT kit (Qiagen, SABiosciences). The transcript levels of interleukin (IL) 1; IL6; tumor necrosis factor (TNF) α; Kupffer cells (KC); regulated on activation, normal T cell expressed and secreted (RANTES); IL12; toll-like receptor (TLR) 2; and TLR9 is measured using primers given below with a PCR master mix (MESA GREEN Mastermix plus, Eurogentec).

| **Primer Name** | **Oligo sequence (5' to 3')** | **SEQ ID No.** |
|---|---|---|
| Mouse IL1 alpha - F | TTTCCACAGCCACGAAGCT | 159 |
| Mouse IL1 alpha - R | TGATGAAAGGGCTCCCAAGT | 160 |
| Mouse IL6 - F | TGTTCTCTGGGAAATCGTGGA | 161 |
| Mouse IL6 - R | TCAGAATTGCCATTCGACAAC | 162 |
| Mouse TNF alpha - F | CCAGAACTCCAGGCGGT | 163 |
| Mouse TNF alpha - R | TGGGAACTTCTCATCCCTTTG | 164 |
| Mouse KC-F | GCCTATCGCCAATGAGCTG | 165 |
| Mouse KC-R | TTCGGTTTGGGTGCAGTG | 166 |
| Mouse RANTES-F | CTTTGCCTACCTCTCCCTCG | 167 |
| Mouse RANTES-R | CACACTTGGCGGTTCCTTC | 168 |
| Mouse IL12 alpha - F | TGCCACCTACTCCCTTGGAT | 169 |
| Mouse IL12 alpha - R | GGCTGTTGGAACGCTGAC | 170 |
| Mouse TLR-2-F | CAGCTTAAAGGGCGGGTCAGAG | 171 |
| Mouse TLR-2-R | TGGAGACGCCAGCTCTGGCTCA | 172 |
| Mouse TLR-9-F | CCAGACGCTCTTCGAGAACC | 173 |
| Mouse TLR-9-R | GTTATAGAAGTGGCGGTTGT | 174 |

Total RNA is isolated from the murine liver samples 2 or 4 weeks post-vector administration (5×10¹⁰ vgs per mouse) using TRIZOL_ reagent (Sigma Aldrich) to measure EGFP transcript levels. Approximately 1µg of RNA is reverse-transcribed using Verso^{™} Reverse Transcriptase according to the manufacturer's protocol (Thermo Scientific, Surrey, United Kingdom). TAQMAN_ PCR is carried out using primers/probe against EGFP gene (Forward Primer: CTTCAAGATCCGCCACAACATC; Reverse Primer: ACC ATGTGATCGCGCTTCTC; Probe: FAM-CGCCGACCACTACCAGCAGAACACC-TAMRA) and according to the manufacturer's protocol (Eurogentec). GAPDH is used as the housekeeping control gene. Data is captured and analysed using the ABI Prism 7500 Sequence Detection (Life Technologies, Applied Biosystems).

Result: Consistent with the in vitro studies, liver tissues of mice administered the four AAV2 S/A mutants (S489A, S498A, S662A, and S668A) and the T251A mutant showed higher levels of EGFP reporter when compared with animals injected with AAV2-WT vector and analyzed by fluorescence microscopy (Figure. 8A). A similar increase in EGFP levels is noted after hepatic gene transfer with the AAV2 lysine mutants K532R, K544R, and K490R + K532R, as described in figure 9A and further figure 12 describes serine/threonine/Lysine multiple-mutant vectors, K489R + K532R, S489+S489+K532, S489+K532+T251 and T251+K532 exhibiting enhanced transduction upon hepatic gene transfer *in vivo.* To confirm this phenomenon, we then measured AAV vector genome copy numbers in the liver tissue of vector- or mock-injected mice. As shown in Figures. 8B and 9B, a significant increase in vector copies per diploid genome (up to 4.9-fold) is observed in animals injected with S/T/K mutant vectors in comparison with animals that received the AAV2-WT vector alone. To further corroborate these data, we then measured the transcript levels of EGFP in hepatic RNA isolated from these mice. The studies demonstrate higher levels of transgene transcript expression (up to 14-fold) after hepatic gene transfer, in AAV2 S/T/K mutant administered mice in comparison with AAV2-WT vector injected animals. In all these studies, AAV8-injected animals are used as a control group for hepatic gene transfer. From the above result it is very evident that the aforementioned data clearly suggests that select S/T→A and K→R mutations can augment the transduction efficiency of AAV2 vectors in vivo.

Two of the AAV8 S→A mutants (S279A and S671A) and the K137R mutant tested had a 3.6-to 11-fold higher EGFP expression by fluorescence imaging (Figures. 23A and 23B) and a 9-to 46-fold higher EGFP transcript level as analyzed by quantitative PCR (Figures. 23E). The T252A mutant had lower levels of EGFP expression when compared to the WT-AAV8 vector, 2 weeks postadministration (Figures. 23C, 23D, and 23F). When the transduction efficiency of the K137R mutant is assessed in a more immunogenic BALB/c strain of mice (Michou et al., 1997; Breous et al., 2010), a similar increase in transduction is noted for the K137R mutant (14- fold by fluorescence imaging, data not shown) as compared to the WT-AAV8 vector. To further corroborate this data, the biodistribution of AAV8 vectors in recipient mice is analysed by quantitative PCR measurement of vector genomes in various tissues. Table 13 demonstrates that the K137R mutant had the best tropism for liver as compared to the WT AAV8 (106 vs. 7.7 vector copies/mouse diploid genome). Interestingly, a preferential transduction of the lungs (0.13 vs. 0.03 vector copies/mouse diploid genome), and muscle tissue (1.38 vs. 0.45 vector copies/mouse diploid genome) is also noted for the K137R mutant suggesting that this vector has a better systemic transduction profile when compared to the other vectors tested. Similarly, the S/A mutant vectors (S279A, S501A, and S671A)\ generally showed increased transduction of the liver and muscle compared to WT-AAV8 vectors. However, the T252A mutant is considerably retargeted to other tissues such as the lungs, which may explain the low levels of EGFP expression seen in the liver (Figures. 23C, 22D, and 22F). These results clearly indicate that the S/A and K/R mutant capsids augment the hepatic transduction of AAV8 vectors, with K137R being the most effective. Hence, this mutant in particular is subjected to further analysis to gain insights into mechanisms by which it exhibits its effects on transduction of the vector.

**Table 13: Vector biodistribution in various organs in C57BL/6 mice 2-or 4-weeks post gene transfer with the WT-AAV8 and S/T/K mutant AAV8 vectors. Values are shown as mean (± SD) vector copy numbers per mouse diploid genome.**

| **Vector type** | **Muscle** | **Liver** | **Kidney** | **Lung** | **Spleen** | **Heart** |
|---|---|---|---|---|---|---|
| **WT-AAV8 (4 weeks)** | 0.45 ±0.2 | 7.77± 0.8 | 0.0016 ±0.00045 | 0.03± 0.07 | 0.004 ± 0.0009 | 0.0003 ± 0.00006 |
| **K137R-AAV8 (4 weeks)** | 1.38±0.4 | 106.8 ± 11 | 0.0011 ±0.0003 | 0.13± 0.09 | 0.004 ± 0.0006 | 0.0006 ± 0.00004 |
| **S279A-AAV8 (4 weeks)** | 2.84±0.5 | 30.3± 3.2 | 0.0029 ±0.0007 | 0.01± 0.03 | 0.002 ± 0.0005 | 0.0007 ± 0.00001 |
| **S501A-AAV8 (4 weeks)** | 3.07±0.6 | 15 ± 1.9 | 0.003± 0.0008 | 0.11 ± 0.08 | 0.0009 ± 0.0008 | 0.003± 0.0002 |
| **S671A-AAV8 (4 weeks)** | 1.89±0.3 | 65.89± 5.6 | 0.0006 ± 0.00012 | 0.051 ± 0.01 | 0.001 ± 0.0006 | 0.001 ± 0.0008 |
| **WT-AAV8 (2 weeks)** | 0.008 ±0.08 | 0.51± 0.1 | 0.0006 ±0.0002 | 0.0008± 0.0002 | 0.022 ± 0.009 | 0.0001 ± 0.00005 |
| **T252A (2 weeks)** | 0.01 ± 0.005 | 0.01 ±0.002 | 0.0001 ± 0.00006 | 0.01 ± 0.004 | 0.0005 ±0.00009 | 0.0006 ± 0.00009 |

### Estimation of neutralizing antibodies against S/T/K AAV vectors

Heat-inactivated serum samples from AAV-WT-injected or S→A and K→R mutant AAV injected C57BL/6 mice are assayed for neutralizing antibody (NAb) titers as described previously (Calcedo et al., 2009). Briefly, groups of mice (n = 4) are administered 5×1010 VG of AAV-WT and AAV S/T/K mutant vectors via tail vein injections. Four weeks after vector delivery, animals are killed and serum is collected. The pooled serum is snap frozen to -80°C. Heat inactivated samples are assayed for the neutralizing antibody (NAb) titres as described previously (Calcedo et al., 2009). Dilutions of serum samples [1:5 to 1:81920] from animals are pre-incubated for 1hr with AAV vectors and the mixture added to Huh7 cells. The NAb titer is reported as the highest plasma dilution that inhibited AAV transduction of Huh7 cells by 50% or more compared with that for the naive serum control. Limit of detection of the assay was 1/5 dilution. These values provide reciprocal titers of antibodies present in the sample analyzed i.e higher the values of dilution that are required to cross-neutralize WT-AAV, the greater the concentration of antibodies that were originally present in the serum samples.

Result with AAV2: AAV2 S489A vector demonstrates lower neutralization antibody titres compared to the WT-AAV2 vector. Pooled serum samples from WT-AAV2 or AAV2 mutant injected mice (n=4 per group) are analyzed for neutralizing antibodies 4-weeks after vector administration. Values are the reciprocal of the serum dilution at which relative luminescence units (RLUs) is reduced 50% compared to virus control wells (as described in the below table 14).

**Table: 14**

| **SL. No.** | **Groups** | **Reciprocal NAb Titre** |
|---|---|---|
| 1 | scAAV2-WT | 5120 |
| 2 | S489A | 640 |
| 3 | S525A | 5120 |
| 4 | S537A | 5120 |
| 5 | S547A | 5120 |
| 6 | S662A | 5120 |
| 7 | Anti AAV2 Rabbit Control Serum | 81920 |

Result with AAV8: The mutant K137R vector is significantly less immunogenic when compared to WT-AAV8 vectors, which is demonstrated by measuring the neutralizing antibodies against the various mutants which demonstrated a 2-fold reduction in the neutralizing antibody titre for the K137R-AAV8 vector (as describd in the below table 15).

**Table: 15**

| **S. No.** | **Groups** | **Reciprocal NAb titer** |
|---|---|---|
| 1 | Mock | 0 |
| 2 | WT-AAV8 | 320 |
| 3 | S279A | 320 |
| 4 | S501A | 640 |
| 5 | S671A | 320 |
| 6 | K137R | 160 |
| 7 | Anti-AAV8 rabbit control serum | 20480 |

### Recombinant AAV2 vector transduction studies in vivo

Groups (n=4, per group) of 8-12 weeks-old C57BL/6 are injected with ~5 X 10¹⁰ viral genome particles (vg) of wild type (wt) scAAV2 or mutant scAAV2 vectors containing EGFP as the transgene, *via* the tail vein. Mock injection is done with PBS. Mice are euthanized 4 weeks after vector administration. Cross-sections from three hepatic lobes of the mock-injected and vector-injected groups assessed for EGFP expression by fluorescence microscopy. Images from five visual fields are analysed quantitatively by Adobe Photoshop CS2 software/Image-J and expressed as mean pixels per visual field (mean ±SE).

Result: In vivo studies are conducted in C57BL/6 mice, wherein - 2 to 22 fold increase in transduction efficiency is observed upon hepatic gene transfer of ∼5x10¹⁰ vgs of AAV2 mutant vectors (Serine residues: S489A, S498A, S525A, S537A, S547A, S662A, S668A; threonine residue: T251A and lysine residue: K532R ). The feasibility of the use of serine/threonine→alanine or lysine→7arginine vectors either as single mutants (K532R, S668A, S662A, S489A, and T251A) or multiple mutants (K532R+ S668A+ S662A+ S489A+ T251A) for potential gene therapy of human diseases is illustrated in table 16 below.

**Table 16: Transduction efficacy of S/T/K Mutants of AAV2**

| **S.No.** | **AAV2 Mutants** | ***In vitro* Analysis : Fold Change** | | ***In vivo* Analysis : Fold Change** | | |
|---|---|---|---|---|---|---|
| | | **Fluorescent Microscopy** | **Flow Cytometry *** | **Fluorescent Microscopy** | **EGFP expression by QPCR** | **Vector copy no by QPCR** |
| **SINGE MUTANTS** | | | | | | |
| | AAV2WT | 1 | 1.0 | 1 | 1 | 1 |
| 1 | AAV2 S276A | 5 | 2.3 | - | - | - |
| 2 | AAV2 S489A | 4.25 | 1.9 | 21.8 | 14 | 4.2 |
| 3 | AAV2 S498A | 5.8 | 2.1 | 4 | 10 | 3.4 |
| 4 | AAV2 S525A | 0.76 | 1.5 | 1.7 | - | - |
| 5 | AAV2 S537A | 0.65 | 1.8 | 7.3 | - | - |
| 6 | AAV2 S547A | 2.45 | 1.8 | 2.1 | - | - |
| 7 | AAV2 S662A | 1.7 | 1.7 | 17.4 | 8 | 3.9 |
| 8 | AAV2 S668A | 7.44 | 2.1 | 4 | 12 | 4.5 |
| 9 | AAV2 T251A | 5.54 | 1.9 | 4 | 6 | 2.7 |
| 10 | AAV2 T503A | 17 | 2.3 | | | |
| 11 | AAV2 T716A | 36 | 2.5 | | | |
| 12 | AAV2 K527R | 1.3 | 1.5 | 2 | 4.4 | 3.2 |
| 13 | AAV2 K532R | 18 | 2.7 | 4 | 11.8 | 4.5 |
| 14 | AAV2 K544R | 15 | 2.5 | 7 | 12.3 | 4.9 |

| **DOUBLE MUTANTS** | | | | | | |
|---|---|---|---|---|---|---|
| | | | | | | |
| 1 | AAV2 K490R + K532R | 15 | 2.4 | 4 | 18 | 2.5 |
| 2 | AAV2 K527R+ K532R | 3 | 1.1 | 0.2 | 0.7 | 1.9 |
| 3 | AAV2 T251A+ | - | 3.4 | - | 20 | 5.1 |
| | K532R | | | | | |
| 4 | AAV2 S489+K532R | - | 3.6 | - | 15 | 4.9 |

| **TRIPLE MUTANT** | | | | | | |
|---|---|---|---|---|---|---|
| 1 | AAV2 T251A + K532R+ S489A | - | 3.4 | - | 22 | 6.2 |
| 2 | AAV2 S489A+S498A+K532 R | - | 3 | - | 22 | 5.8 |

Further, quantitative analysis of mutant AAV2 and AAV8 depicting the transduction efficacy was also studied for the mutants mentioned herein. The data is provided in figure 17 below.

### Wild Type and Mutant Sequences of AAV serotypes 1 to 10, having mutations at specific locations.

| | |
|---|---|
| SEQUENCE IN RED | REP SEQUENCE |
| SEQUENCE IN BLUE | CAP SEQUENCE |
| **SEQUENCE IN BLACK** | **BACKBONE SEQUENCE** |
| SEQUENCE IN GREEN | AMP SEQUENCE |

### AAV1-Wild Type ; SEQ ID No. 139

CACCTAAATTGTAAGCGTTAATATTTTGTTAAAATTCGCGTTAAATTTTTGTTAAATC
AGCTCATTTTTTAACCAATAGGCCGAAATCGGCAAAATCCCTTATAAATCAAAAGAA
TAGACCGAGATAGGGTTGAGTGTTGTTCCAGTTTGGAACAAGAGTCCACTATTAAAG
AACGTGGACTCCAACGTCAAAGGGCGAAAAACCGTCTATCAGGGCGATGGCCCACT
ACGTGAACCATCACCCTAATCAAGTTTTTTGGGGTCGAGGTGCCGTAAAGCACTAAA
TCGGAACCCTAAAGGGAGCCCCCGATTTAGAGCTTGACGGGGAAAGCCGGCGAACG
TGGCGAGAAAGGAAGGGAAGAAAGCGAAAGGAGCGGGCGCTAGGGCGCTGGCAAG
TGTAGCGGTCACGCTGCGCGTAACCACCACACCCGCCGCGCTTAATGCGCCGCTACA
GGGCGCGTCCCATTCGCCATTCAGGCTGCGCAACTGTTGGGAAGGGCGATCGGTGC
GGGCCTCTTCGCTATTACGCCAGCTGGCGAAAGGGGGATGTGCTGCAAGGCGATTA
AGTTGGGTAACGCCAGGGTTTTCCCAGTCACGACGTTGTAAAACGACGGCCAGTGA
ATTGTAATACGACTCACTATAGGGCGAATTGGAGCTCCACCGCGGTGGCGGCCGCTC
TAGAGTCCTGTATTAGAGGTCACGTGAGTGTTTTGCGACATTTTGCGACACCATGTG
GTCACGCTGGGTATTTAAGCCCGAGTGAGCACGCAGGGTCTCCATTTTGAAGCGGGA
GGTTTGAACGCGCAGCACGGCGGGGTTTTACGAGATTGTGATTAAGGTCCCCAGCG
ACCTTGACGGGCATCTGCCCGGCATTTCTGACAGCTTTGTGAACTGGGTGGCCGAGA
AGGAATGGGAGTTGCCGCCAGATTCTGACATGGATCTGAATCTGATTGAGCAGGCA
CCCCTGACCGTGGCCGAGAAGCTGCAGCGCGACTTTCTGACGGAATGGCGCCGTGT
GAGTAAGGCCCCGGAGGCCCTTTTCTTTGTGCAATTTGAGAAGGGAGAGAGCTACTT
CCACATGCACGTGCTCGTGGAAACCACCGGGGTGAAATCCATGGTTTTGGGACGTTT

CCTGAGTCAGATTCGCGAAAAACTGATTCAGAGAATTTACCGCGGATCGAGCCGA
CTTTGCCAAACTGGTTCGCGGTCACAAAGACCAGAAATGGCGCCGGAGGCGGAAC
AAGGTGGTGGATGAGTGCTACATCCCCAATTACTTGCTCCCCAAAACCCAGCCTGAG
CTCCAGTGGGCGTGGACTAATATGGAACAGTATTTAAGCGCCTGTTTGAATCTCACG
GAGCGTAAACGGTTGGTGGCGCAGCATCTGACGCACGTGTCGCAGACGCAGGAGCA
GAACAAAGAGAATCAGAATCCCAATTCTGATGCGCCGGTGATCAGATCAAAAACTT
CAGCCAGGTACATGGAGCTGGTCGGGTGGCTCGTGGACAAGGGGATTACCTCGGAG
AAGCAGTGGATCCAGGAGGACCAGGCCTCATACATCTCCTTCAATGCGGCCTCCAA
CTCGCGGTCCCAAATCAAGGCTGCCTTGGACAATGCGGGAAAGATTATGAGCCTGA
CTAAAACCGCCCCCGACTACCTGGTGGGCCAGCAGCCCGTGGAGGACATTTCCAGC
AATCGGATTTATAAAATTTTGGAACTAAACGGGTACGATCCCCAATATGCGGCTTCC
GTCTTTCTGGGATGGGCCACGAAAAAGTTCGGCAAGAGGAACACCATCTGGCTGTTT
GGGCCTGCAACTACCGGGAAGACCAACATCGCGGAGGCCATAGCCCACACTGTGCC
CTTCTACGGGTGCGTAAACTGGACCAATGAGAACTTTCCCTTCAACGACTGTGTCGA
CAAGATGGTGATCTGGTGGGAGGAGGGGAAGATGACCGCCAAGGTCGTGGAGTCGG
CCAAAGCCATTCTCGGAGGAAGCAAGGTGCGCGTGGACCAGAAATGCAAGTCCTCG
GCCCAGATAGACCCGACTCCCGTGATCGTCACCTCCAACACCAACATGTGCGCCGTG
ATTGACGGGAACTCAACGACCTTCGAACACCAGCAGCCGTTGCAAGACCGGATGTT
CAAATTTGAACTCACCCGCCGTCTGGATCATGACTTTGGGAAGGTCACCAAGCAGGA
AGTCAAAGACTTTTTCCGGTGGGCAAAGGATCACGTGGTTGAGGTGGAGCATGAAT
TCTACGTCAAAAAGGGTGGAGCCAAGAAAAGACCCGCCCCCAGTGACGCAGATATA
AGTGAGCCCAAACGGGTGCGCGAGTCAGTTGCGCAGCCATCGACGTCAGACGCGGA
AGCTTCGATCAACTACGCAGACAGGTACCAAAACAAATGTTCTCGTCACGTGGGCAT
GAATCTGATGCTGTTTCCCTGCAGACAATGCGAGAGAATGAATCAGAATTCAAATAT
CTGCTTCACTCACGGACAGAAAGACTGTTTAGAGTGCTTTCCCGTGTCAGAATCTCA
ACCCGTTTCTGTCGTCAAAAAGGCGTATCAGAAACTGTGCTACATTCATCATATCAT
GGGAAAGGTGCCAGACGCTTGCACTGCCTGCGATCTGGTCAATGTGGATTTGGATGA
CTGCATCTTTGAACAATAAATGATTTAAACCAGGTATGGCTGCCGATGGTTATCTTC
CAGATTGGCTCGAGGACAACCTCTCTGAGGGCATTCGCGAGTGGTGGGACTTGAAA
CCTGGAGCCCCGAAGCCCAAAGCCAACCAGCAAAAGCAGGACGACGGCCGGGGTC
TGGTGCTTCCTGGCTACAAGTACCTCGGACCCTTCAACGGACTCGACAAGGGGGAG

CCCGTCAACGCGGCGGACGCAGCGGCCCTCGAGCACGACAAGGCCTACGACCAGCA
GCTCAAAGCGGGTGACAATCCGTACCTGCGGTATAACCACGCCGACGCCGAGTTTC
AGGAGCGTCTGCAAGAAGATACGTCTTTTGGGGGCAACCTCGGGCGAGCAGTCTTC
CAGGCCAAGAAGCGGGTTCTCGAACCTCTCGGTCTGGTTGAGGAAGGCGCTAAGAC
GGCTCCTGGAAAGAAACGTCCGGTAGAGCAGTCGCCACAAGAGCCAGACTCCTCCT
CGGGCATCGGCAAGACAGGCCAGCAGCCCGCTAAAAAGAGACTCAATTTTGGTCAG
ACTGGCGACTCAGAGTCAGTCCCCGATCCACAACCTCTCGGAGAACCTCCAGCAAC
CCCCGCTGCTGTGGGACCTACTACAATGGCTTCAGGCGGTGGCGCACCAATGGCAG
ACAATAACGAAGGCGCCGACGGAGTGGGTAATGCCTCAGGAAATTGGCATTGCGAT
TCCACATGGCTGGGCGACAGAGTCATCACCACCAGCACCCGCACCTGGGCCTTGCCC
ACCTACAATAACCACCTCTACAAGCAAATCTCCAGTGCTTCAACGGGGGCCAGCAA
CGACAACCACTACTTCGGCTACAGCCCCCTGGGGGTATTTTGATTTCAACAGATTCC
ACTGCCACTTTTCACCACGTGACTGGCAGCGACTCATCAACAACAATTGGGGATTCC
GGCCCAAGAGACTCAACTTCAAACTCTTCAACATCCAAGTCAAGGAGGTCACGACG
AATGATGGCGTCACAACCATCGCTAATAACCTTACCAGCACGGTTCAAGTCTTCTCG
GACTCGGAGTACCAGCTTCCGTACGTCCTCGGCTCTGCGCACCAGGGCTGCCTCCCT
CCGTTCCCGGCGGACGTGTTCATGATTCCGCAATACGGCTACCTGACGCTCAACAAT
GGCAGCCAAGCCGTGGGACGTTCATCCTTTTACTGCCTGGAATATTTCCCTTCTCAG
ATGCTGAGAACGGGCAACAACTTTACCTTCAGCTACACCTTTGAGGAAGTGCCTTTC
CACAGCAGCTACGCGCACAGCCAGAGCCTGGACCGGCTGATGAATCCTCTCATCGA
CCAATACCTGTATTACCTGAACAGAACTCAAAATCAGTCCGGAAGTGCCCAAAACA
AGGACTTGCTGTTTAGCCGTGGGTCTCCAGCTGGCATGTCTGTTCAGCCCAAAAACT
GGCTACCTGGACCCTGTTATCGGCAGCAGCGTTTCTAAAACAAAAACAGACAAC
AACAACAGCAATTTTACCTGGACTGGTGCTTCAAAATATAACCTCAATGGGCGTGAA
TCCATCATCAACCCTGGCACTGCTATGGCCTCACACAAAGACGACGAAGACAAGTT
CTTTCCCATGAGCGGTGTCATGATTTTTGGAAAAGAGAGCGCCGGAGCTTCAAACAC
TGCATTGGACAATGTCATGATTACAGACGAAGAGGAAATTAAAGCCACTAACCCTG
TGGCCACCGAAAGATTTGGGACCGTGGCAGTCAATTTCCAGAGCAGCAGCACAGAC
CCTGCGACCGGAGATGTGCATGCTATGGGAGCATTACCTGGCATGGTGTGGCAAGA
TAGAGACGTGTACCTGCAGGGTCCCATTTGGGCCAAAATTCCTCACACAGATGGACA
CTTTCACCCGTCTCCTCTTATGGGCGGCTTTGGACTCAAGAACCCGCCTCCTCAGATC

CTCATCAAAAACACGCCTGTTCCTGCGAATCCTCCGGCGGAGTTTTCAGCTACAAAG
TTTGCTTCATTCATCACCCAATACTCCACAGGACAAGTGAGTGTGGAAATTGAATGG
GAGCTGCAGAAAGAAAACAGCAAGCGCTGGAATCCCGAAGTGCAGTACACATCCA
ATTATGCAAAATCTGCCAACGTTGATTTTACTGTGGACAACAATGGACTTTATACTG
AGCCTCGCCCCATTGGCACCCGTTACCTTACCCGTCCCCTGTAATTTACGTGTTAATC
AATAAACCGGTTGATTCGTTTCAGTTGAACTTTGGTCTCGCGGCCGCTCGATAAGCT
TTTGTTCCCTTTAGTGAGGGTTAATTTCGAGCTTGGCGTAATCATGGTCATAGCTGTT
TCCTGTGTGAAATTGTTATCCGCTCACAATTCCACACAACATACGAGCCGGAAGCAT
AAAGTGTAAAGCCTGGGGTGCCTAATGAGTGAGCTAACTCACATTAATTGCGTTGCG
CTCACTGCCCGCTTTCCAGTCGGGAAACCTGTCGTGCCAGCTGCATTAATGAATCGG
CCAACGCGCGGGGAGAGGCGGTTTGCGTATTGGGCGCTCTTCCGCTTCCTCGCTCAC
TGACTCGCTGCGCTCGGTCGTTCGGCTGCGCGAGCGGTATCAGCTCCCTCAAAGCGT
AATACCGGTTATCCACAGATCAGGGGATACGCAGAAAGACATGTGAGTAAGGCAG
CCAAAGTCCAGGAACCGGTAAAAGGCCCCGCGCTTAGTGCTTTACGGCACCTCG
ACCCCAAAAAACTTGATTAGGGTGATGGTTCACGTAGTGGGCCATCGCCCTGATAGA
CGGTTTTTCGCCCTTTGACGTTGGAGTCCACGTTCTTTAATAGTGGACTCTTGTTCCA
AACTGGAACAACACTCAACCCTATCTCGGTCTATTCTTTTGATTTATAAGGGATTTTG
CCGATTTCGGCCTATTGGTTAAAAAATGAGCTGATTTAACAAAAATTTAACGCGAAT
TTTAACAAAATATTAACGTTTACAATTTAAATATTTGCTTATACAATCTTCCTGTTTT
TGGGGCTTTTCTGATTATCAACCGGGGTACATATGATTGACATGCTAGTTTTACGATT
ACCGTTCATCGATTCTCTTGTTTGCTCCAGACTCTCAGGCAATGACCTGATAGCCTTT
GTAGAGACCTCTCAAAAATAGCTACCCTCTCCGGCATGAATTTATCAGCTAGAACGG
TTGAATATCATATTGATGGTGATTTGACTGTCTCCGGCCTTTCTCACCCGTTTGAATC
TTTACCTACACATTACTCAGGCATTGCATTTAAAATATATGAGGGTTCTAAAAATTTT
TATCCTTGCGTTGAAATAAAGGCTTCTCCCGCAAAAGTATTACAGGGTCATAATGTT
TTTGGTACAACCGATTTAGCTTTATGCTCTGAGGCTTTATTGCTTAATTTTGCTAATT
CTTTGCCTTGCCTGTATGATTTATTGGATGTTGGAATTCCTGATGCGGTATTTTCTCCT
TACGCATCTGTGCGGTATTTCACACCGCATATGGTGCACTCTCAGTACAATCTGCTCT
GATGCCGCATAGTTAAGCCAGCCCCGACACCCGCCAACACCCGCTGACGCGCCCTG
ACGGGCTTGTCTGCTCCCGGCATCCGCTTACAGACAAGCTGTGACCGTCTCCGGGAG
CTGCATGTGTCAGAGGTTTTCACCGTCATCACCGAAACGCGCGAGACGAAAGGGCC

TCGTGATACGCCTATTTTTATAGGTTAATGTCATGATAATAATGGTTTCTTAGACGTC
AGGTGGCACTTTTCGGGGAAATGTGCGCGGAACCCCTATTTGTTTATTTTTCTAAATA
CATTCAAATATGTATCCGCTCATGAGACAATAACCCTGATAAATGCTTCAATAATAT
TGAAAAAGGAAGAGTATGAGTATTCAACATTTCCGTGTCGCCCTTATTCCCTTTTTTG
CGGCATTTTGCCTTCCTGTTTTTGCTCACCCAGAAACGCTGGTGAAAGTAAAAGATG
CTGAAGATCAGTTGGGTGCACGAGTGGGTTACATCGAACTGGATCTCAACAGCGGT
AAGATCCTTGAGAGTTTTCGCCCCGAAGAACGTTTTCCAATGATGAGCACTTTTAAA
GTTCTGCTATGTGGCGCGGTATTATCCCGTATTGACGCCGGGCAAGAGCAACTCGGT
CGCCGCATACACTATTCTCAGAATGACTTGGTTGAGTACTCACCAGTCACAGAAAAG
CATCTTACGGATGGCATGACAGTAAGAGAATTATGCAGTGCTGCCATAACCATGAGT
GATAACACTGCGGCCAACTTACTTCTGACAACGATCGGAGGACCGAAGGAGCTAAC
CGCTTTTTTGCACAACATGGGGGATCATGTAACTCGCCTTGATCGTTGGGAACCGGA
GCTGAATGAAGCCATACCAAACGACGAGCGTGACACCACGATGCCTGTAGCAATGG
CAACAACGTTGCGCAAACTATTAACTGGCGAACTACTTACTCTAGCTTCCCGGCAAC
AATTAATAGACTGGATGGAGGCGGATAAAGTTGCAGGACCACTTCTGCGCTCGGCC
CTTCCGGCTGGCTGGTTTATTGCTGATAAATCTGGAGCCGGTGAGCGTGGGTCTCGC
GGTATCATTGCAGCACTGGGGCCAGATGGTAAGCCCTCCCGTATCGTAGTTATCTAC
ACGACGGGGAGTCAGGCAACTATGGATGAACGAAATAGACAGATCGCTGAGATAG
GTGCCTCACTGATTAAGCATTGGTAACTGTCAGACCAAGTTTACTCATATATACTTTA
GATTGATTTAAAACTTCATTTTTAATTTAAAAGGATCTAGGTGAAGATCCTCACCTA
AATTGTAAGCGTTAATATTTTGTTAAAATTCGCGTTAAATTTTTGTTAAATCAGCTCA
TTTTTTAACCAATAGGCCGAAATCGGCAAAATCCCTTATAAATCAAAAGAATAGACC
GAGATAGGGTTGAGTGTTGTTCCAGTTTGGAACAAGAGTCCACTATTAAAGAACGTG
GACTCCAACGTCAAAGGGCGAAAAACCGTCTATCAGGGCGATGGCCCACTACGTGA
ACCATCACCCTAATCAAGTTTTTTGGGGTCGAGGTGCCGTAAAGCACTAAATCGGAA
CCCTAAAGGGAGCCCCCGATTTAGAGCTTGACGGGGAAAGCCGGCGAACGTGGCGA
GAAAGGAAGGGAAGAAAGCGAAAGGAGCGGGCGCTAGGGCGCTGGCAAGTGTAGC
GGTCACGCTGCGCGTAACCACCACACCCGCCGCGCTTAATGCGCCGCTACAGGGCG
CGTCCCATTCGCCATTCAGGCTGCGCAACTGTTGGGAAGGGCGATCGGTGCGGGCCT
CTTCGCTATTACGCCAGCTGGCGAAAGGGGGATGTGCTGCAAGGCGATTAAGTTGG
GTAACGCCAGGGTTTTCCCAGTCACGACGTTGTAAAACGACGGCCAGTGAATTGTAA

TACGACTCACTATAGGGCGAATTGGAGCTCCACCGCGGTGGCGGCCGCTCTAGAGT
CCTGTATTAGAGGTCACGTGAGTGTTTTGCGACATTTTGCGACACCATGTGGTCACG
CTGGGTATTTAAGCCCGAGTGAGCACGCAGGGTCTCCATTTTGAAGCGGGAGGTTTG
AACGCGCAGCC

### AAV1-Mutant ; SEQ ID No. 149

CACCTAAATTGTAAGCGTTAATATTTTGTTAAAATTCGCGTTAAATTTTTGTTAAATC
AGCTCATTTTTTAACCAATAGGCCGAAATCGGCAAAATCCCTTATAAATCAAAAGAA
TAGACCGAGATAGGGTTGAGTGTTGTTCCAGTTTGGAACAAGAGTCCACTATTAAAG
AACGTGGACTCCAACGTCAAAGGGCGAAAAACCGTCTATCAGGGCGATGGCCCACT
ACGTGAACCATCACCCTAATCAAGTTTTTTGGGGTCGAGGTGCCGTAAAGCACTAAA
TCGGAACCCTAAAGGGAGCCCCCGATTTAGAGCTTGACGGGGAAAGCCGGCGAACG
TGGCGAGAAAGGAAGGGAAGAAAGCGAAAGGAGCGGGCGCTAGGGCGCTGGCAAG
TGTAGCGGTCACGCTGCGCGTAACCACCACACCCGCCGCGCTTAATGCGCCGCTACA
GGGCGCGTCCCATTCGCCATTCAGGCTGCGCAACTGTTGGGAAGGGCGATCGGTGC
GGGCCTCTTCGCTATTACGCCAGCTGGCGAAAGGGGGATGTGCTGCAAGGCGATTA
AGTTGGGTAACGCCAGGGTTTTCCCAGTCACGACGTTGTAAAACGACGGCCAGTGA
ATTGTAATACGACTCACTATAGGGCGAATTGGAGCTCCACCGCGGTGGCGGCCGCTC
TAGAGTCCTGTATTAGAGGTCACGTGAGTGTTTTGCGACATTTTGCGACACCATGTG
GTCACGCTGGGTATTTAAGCCCGAGTGAGCACGCAGGGTCTCCATTTTGAAGCGGGA
GGTTTGAACGCGCAGCACGGCGGGTTTTACGAGATTGTGATTAAGGTCCCCAGCG
ACCTTGACGGGCATCTGCCCGGCATTTCTGACAGCTTTGTGAACTGGGTGGCCGAGA
AGGAATGGGAGTTGCCGCCAGATTCTGACATGGATCTGAATCTGATTGAGCAGGCA
CCCCTGACCGTGGCCGAGAAGCTGCAGCGCGACTTTCTGACGGAATGGCGCCGTGT
GAGTAAGGCCCCGGAGGCCCTTTTCTTTGTGCAATTTGAGAAGGGAGAGAGCTACTT
CCACATGCACGTGCTCGTGGAAACCACCGGGGTGAAATCCATGGTTTTGGGACGTTT
CCTGAGTCAGATTCGCGAAAAACTGATTCAGAGAATTTACCGCGGGATCGAGCCGA
CTTTGCCAAACTGGTTCGCGGTCACAAAGACCAGAAATGGCGCCGGAGGCGGGAAC
AAGGTGGTGGATGAGTGCTACATCCCCAATTACTTGCTCCCCAAAACCCAGCCTGAG
CTCCAGTGGGCGTGGACTAATATGGAACAGTATTTAAGCGCCTGTTTGAATCTCACG

GAGCGTAAACGGTTGGTGGCGCAGCATCTGACGCACGTGTCGCAGACGCAGGAGCA
GAACAAAGAGAATCAGAATCCCAATTCTGATGCGCCGGTGATCAGATCAAAAACTT
CAGCCAGGTACATGGAGCTGGTCGGGTGGCTCGTGGACAAGGGGATTACCTCGGAG
AAGCAGTGGATCCAGGAGGACCAGGCCTCATACATCTCCTTCAATGCGGCCTCCAA
CTCGCGGTCCCAAATCAAGGCTGCCTTGGACAATGCGGGAAAGATTATGAGCCTGA
CTAAAACCGCCCCCGACTACCTGGTGGGCCAGCAGCCCGTGGAGGACATTTCCAGC
AATCGGATTTATAAAATTTTGGAACTAAACGGGTACGATCCCCAATATGCGGCTTCC
GTCTTTCTGGGATGGGCCACGAAAAAGTTCGGCAAGAGGAACACCATCTGGCTGTTT
GGGCCTGCAACTACCGGGAAGACCAACATCGCGGAGGCCATAGCCCACACTGTGCC
CTTCTACGGGTGCGTAAACTGGACCAATGAGAACTTTCCCTTCAACGACTGTGTCGA
CAAGATGGTGATCTGGTGGGAGGAGGGGAAGATGACCGCCAAGGTCGTGGAGTCGG
CCAAAGCCATTCTCGGAGGAAGCAAGGTGCGCGTGGACCAGAAATGCAAGTCCTCG
GCCCAGATAGACCCGACTCCCGTGATCGTCACCTCCAACACCAACATGTGCGCCGTG
ATTGACGGGAACTCAACGACCTTCGAACACCAGCAGCCGTTGCAAGACCGGATGTT
CAAATTTGAACTCACCCGCCGTCTGGATCATGACTTTGGGAAGGTCACCAAGCAGGA
AGTCAAAGACTTTTTCCGGTGGGCAAAGGATCACGTGGTTGAGGTGGAGCATGAAT
TCTACGTCAAAAAGGGTGGAGCCAAGAAAAGACCCGCCCCCAGTGACGCAGATATA
AGTGAGCCCAAACGGGTGCGCGAGTCAGTTGCGCAGCCATCGACGTCAGACGCGGA
AGCTTCGATCAACTACGCAGACAGGTACCAAAACAAATGTTCTCGTCACGTGGGCAT
GAATCTGATGCTGTTTCCCTGCAGACAATGCGAGAGAATGAATCAGAATTCAAATAT
CTGCTTCACTCACGGACAGAAAGACTGTTTAGAGTGCTTTCCCGTGTCAGAATCTCA
ACCCGTTTCTGTCGTCAAAAAGGCGTATCAGAAACTGTGCTACATTCATCATATCAT
GGGAAAGGTGCCAGACGCTTGCACTGCCTGCGATCTGGTCAATGTGGATTTGGATGA
CTGCATCTTTGAACAATAAATGATTTAAACCAGGTATGGCTGCCGATGGTTATCTTC
CAGATTGGCTCGAGGACAACCTCTCTGAGGGCATTCGCGAGTGGTGGGACTTGAAA
CCTGGAGCCCCGAAGCCCAAAGCCAACCAGCAAAAGCAGGACGACGGCCGGGGTC
TGGTGCTTCCTGGCTACAAGTACCTCGGACCCTTCAACGGACTCGACAAGGGGGAG
CCCGTCAACGCGGCGGACGCAGCGGCCCTCGAGCACGACAAGGCCTACGACCAGCA
GCTCAAAGCGGGTGACAATCCGTACCTGCGGTATAACCACGCCGACGCCGAGTTTC
AGGAGCGTCTGCAAGAAGATACGTCTTTTGGGGGCAACCTCGGGCGAGCAGTCTTC
CAGGCCAAGAAGCGGGTTCTCGAACCTC

K137R

TCGGTCTGGTTGAGGAAGGCGCTAGAACGGCTCCTGGAAAGAAACGTCCGGTAGAG
CAGTCGCCACAAGAGCCAGACTCCTCCTCGGGCATCGGCAAGACAGGCCAGCAGCC
CGCTAAAAAGAGACTCAATTTTGGTCAGACTGGCGACTCAGAGTCAGTCCCCGATCC
ACAACCTCTCGGAGAACCTCCAGCAACCCCCGCTGCTGTGGGACCTACTACAATGGC
TTCAGGCGGTGGCGCACCAATGGCAGACAATAACGAAGGCGCCGACGGAGTGGGTA
ATGCCTCAGGAAATTGGCATTGCGATTCCACATGGCTGGGCGACAGAGTCATCACCA
CCAGCACCCGCACCTGGGCCTTGCCCGCCTACAAT

T251A

AACCACCTCTACAAGCAAATCTCCAGTGCTTCAACGGGGGCCAGCAACGACAACCA
CTACTTCGGCTACGCC

S277A

CCCCTGGGGGTATTTTGATTTCAACAGATTCCACTGCCACTTTTCACCACGTGACTGG
CAGCGACTCATCAACAACAATTGGGGATTCCGGCCCAAGAGACTCAACTTCAAACT
CTTCAACATCCAAGTCAAGGAGGTCACGACGAATGATGGCGTCACAACCATCGCTA
ATAACCTTACCAGCACGGTTCAAGTCTTCTCGGACTCGGAGTACCAGCTTCCGTACG
TCCTCGGCTCTGCGCACCAGGGCTGCCTCCCTCCGTTCCCGGCGGACGTGTTCATGA
TTCCGCAATACGGCTACCTGACGCTCAACAATGGCAGCCAAGCCGTGGGACGTTCAT
CCTTTTACTGCCTGGAATATTTCCCTTCTCAGATGCTGAGAACGGGCAACAACTTTAC
CTTCAGCTACACCTTTGAGGAAGTGCCTTTCCACAGCAGCTACGCGCACAGCCAGAG
CCTGGACCGGCTGATGAATCCTCTCATCGACCAATACCTGTATTACCTGAACAGAAC
TCAAAATCAGTCCGGAAGTGCCCAAAACAAGGACTTGCTGTTTAGCCGTGGGTCTCC
AGCTGGCATGTCTGTTCAGCCCAAAAACTGGCTA

CCTGGACCCTGTTATCGGCAGCAGCGCGTTTCTAAAACAAAAACAGACAACAACAA
CGCCAATTTTACCTGGAC

S499A

TGGTGCTTCAAAATATAACCTCAATGGGCGTGAATCCATCATCAACCCTGGCACTGC
TATGGCCGCACACAAAGACGACGAAGACAAGTTCTTTCCCATGAGCGGTGTCATGA
S526A
TTTTTGGAAAAGAGAGCGCCGGAGCTTCAAACACTGCATTGGACAATGTCATGATTA
CAGACGAAGAGGAAATTAAAGCCACTAACCCTGTGGCCACCGAAAGATTTGGGACC
GTGGCAGTCAATTTCCAGAGCAGCAGCACAGACCCTGCGACCGGAGATGTGCATGC
TATGGGAGCATTACCTGGCATGGTGTGGCAAGATAGAGACGTGTACCTGCAGGGTC
CCATTTGGGCCAAAATTCCTCACACAGATGGACACTTTCACCCGTCTCCTCTTATGG
GCGGCTTTGGACTCAAGAACCCGCCTCCTCAGATCCTCATCAAAAACACGCCTGT
S663A S669A
TCCTGCGAATCCTCCGGCGGAGTTTGCAGCTACAAAGTTTGCTGCATTCATCACCCA
ATACTCCACAGGACAAGTGAGTGTGGAAATTGAATGGGAGCTGCAGAAAGAAAACA
GCAAGCGCTGGAATCCCGAAGTGCAGTACACATCCAATTATGCAAAATCTGCCAAC
GTTGATTTTACTGTGGACAACAATGGACTTTATACTGAGCCTCGCCCCATTGGCACC
CGTTACCTTACCCGTCCCCTGTAATTTACGTGTTAATCAATAAACCGGTTGATTCGTT
TCAGTTGAACTTTGGTCTCGCGGCCGCTCGATAAGCTTTGTTCCCTTTAGTGAGGGT
TAATTTCGAGCTTGGCGTAATCATGGTCATAGCTGTTTCCTGTGTGAAATTGTTATCC
GCTCACAATTCCACACAACATACGAGCCGGAAGCATAAAGTGTAAAGCCTGGGGTG
CCTAATGAGTGAGCTAACTCACATTAATTGCGTTGCGCTCACTGCCCGCTTTCCAGTC
GGGAAACCTGTCGTGCCAGCTGCATTAATGAATCGGCCAACGCGCGGGGAGAGGCG
GTTTGCGTATTGGGCGCTCTTCCGCTTCCTCGCTCACTGACTCGCTGCGCTCGGTCGT
TCGGCTGCGGCGAGCGGTATCAGCTCACTCAAAGGCGGTAATACGGTTATCCACAGAAT
CAGGGGATAACGCAGGAAAGAACATGTGAGCAAAAGGCCAGCAAAAGGCCAGGAACCGTA
AAAAGGCCGCGTTGCTGGCGTTTTTCCATAGGCTCCGCCCCCCTGACGAGCATCACAAAAATCGACGCTCAAGTCAGAGG

GTGATGGTTCACGTAGTGGGCCATCGCCCTGATAGACGGTTTTTCGCCCTTTGACGTT
GGAGTCCACGTTCTTTAATAGTGGACTCTTGTTCCAAACTGGAACAACACTCAACCC
TATCTCGGTCTATTCTTTTGATTTATAAGGGATTTTGCCGATTTCGGCCTATTGGTTA
AAAAATGAGCTGATTTAACAAAAATTTAACGCGAATTTTAACAAAATATTAACGTTT
ACAATTTAAATATTTGCTTATACAATCTTCCTGTTTTTGGGGCTTTTCTGATTATCAA
CCGGGGTACATATGATTGACATGCTAGTTTTACGATTACCGTTCATCGATTCTCTTGT
TTGCTCCAGACTCTCAGGCAATGACCTGATAGCCTTTGTAGAGACCTCTCAAAAATA
GCTACCCTCTCCGGCATGAATTTATCAGCTAGAACGGTTGAATATCATATTGATGGT
GATTTGACTGTCTCCGGCCTTTCTCACCCGTTTGAATCTTTACCTACACATTACTCAG
GCATTGCATTTAAAATATATGAGGGTTCTAAAAATTTTTATCCTTGCGTTGAAATAA
AGGCTTCTCCCGCAAAAGTATTACAGGGTCATAATGTTTTTGGTACAACCGATTTAG
CTTTATGCTCTGAGGCTTTATTGCTTAATTTTGCTAATTCTTTGCCTTGCCTGTATGAT
TTATTGGATGTTGGAATTCCTGATGCGGTATTTTCTCCTTACGCATCTGTGCGGTATT
TCACACCGCATATGGTGCACTCTCAGTACAATCTGCTCTGATGCCGCATAGTTAAGC
CAGCCCCGACACCCGCCAACACCCGCTGACGCGCCCTGACGGGCTTGTCTGCTCCCG
GCATCCGCTTACAGACAAGCTGTGACCGTCTCCGGGAGCTGCATGTGTCAGAGGTTT
TCACCGTCATCACCGAAACGCGCGAGACGAAAGGGCCTCGTGATACGCCTATTTTTA
TAGGTTAATGTCATGATAATAATGGTTTCTTAGACGTCAGGTGGCACTTTTCGGGGA
AATGTGCGCGGAACCCCTATTTGTTTATTTTTCTAAATACATTCAAATATGTATCCGC
TCATGAGACAATAACCCTGATAAATGCTTCAATAATATTGAAAAAGGAAGAGTATG
AGTATTCAACATTTCCGTGTCGCCCTTATTCCCTTTTTTGCGGCATTTTGCCTTCCTGT
TTTTGCTCACCCAGAAACGCTGGTGAAAGTAAAAGATGCTGAAGATCAGTTGGGTG
CACGAGTGGGTTACATCGAACTGGATCTCAACAGCGGTAAGATCCTTGAGAGTTTTC
GCCCCGAAGAACGTTTTCCAATGATGAGCACTTTTAAAGTTCTGCTATGTGGCGCG
TATTATCCCGTATTGACGCCGGGCAAGAGCAACTCGGTCGCCGCATACACTATTCTC
AGAATGACTTGGTTGAGTACTCACCAGTCACAGAAAAGCATCTTACGGATGGCATG
ACAGTAAGAGAATTATGCAGTGCTGCCATAACCATGAGTGATAACACTGCGGCCAA
CTTACTTCTGACAACGATCGGAGGACCGAAGGAGCTAACCGCTTTTTTGCACAACAT
GGGGGATCATGTAACTCGCCTTGATCGTTGGGAACCGGAGCTGAATGAAGCCATAC
CAAACGACGAGCGTGACACCACGATGCCTGTAGCAATGGCAACAACGTTGCGCAAA
CTATTAACTGGCGAACTACTTACTCTAGCTTCCCGGCAACAATTAATAGACTGGATG

GAGGCGGATAAAGTTGCAGGACCACTTCTGCGCTCGGCCCTTCCGGCTGGCTGGTTT
ATTGCTGATAAATCTGGAGCCGGTGAGCGTGGGTCTCGCGGTATCATTGCAGCACTG
GGGCCAGATGGTAAGCCCTCCCGTATCGTAGTTATCTACACGACGGGGAGTCAGGC
AACTATGGATGAACGAAATAGACAGATCGCTGAGATAGGTGCCTCACTGATTAAGC
ATTGGTAACTGTCAGACCAAGTTTACTCATATATACTTTAGATTGATTTAAAACTTCA
TTTTTAATTTAAAAGGATCTAGGTGAAGATCCTCACCTAAATTGTAAGCGTTAATAT
TTTGTTAAAATTCGCGTTAAATTTTTGTTAAATCAGCTCATTTTTTAACCAATAGGCC
GAAATCGGCAAAATCCCTTATAAATCAAAAGAATAGACCGAGATAGGGTTGAGTGT
TGTTCCAGTTTGGAACAAGAGTCCACTATTAAAGAACGTGGACTCCAACGTCAAAG
GGCGAAAAACCGTCTATCAGGGCGATGGCCCACTACGTGAACCATCACCCTAATCA
AGTTTTTTGGGGTCGAGGTGCCGTAAAGCACTAAATCGGAACCCTAAAGGGAGCCC
CCGATTTAGAGCTTGACGGGGAAAGCCGGCGAACGTGGCGAGAAAGGAAGGGAAG
AAAGCGAAAGGAGCGGGCGCTAGGGCGCTGGCAAGTGTAGCGGTCACGCTGCGCGT
AACCACCACACCCGCCGCGCTTAATGCGCCGCTACAGGGCGCGTCCCATTCGCCATT
CAGGCTGCGCAACTGTTGGGAAGGGCGATCGGTGCGGGCCTCTTCGCTATTACGCCA
GCTGGCGAAAGGGGGATGTGCTGCAAGGCGATTAAGTTGGGTAACGCCAGGGTTTT
CCCAGTCACGACGTTGTAAAACGACGGCCAGTGAATTGTAATACGACTCACTATAG
GGCGAATTGGAGCTCCACCGCGGTGGCGGCCGCTCTAGAGTCCTGTATTAGAGGTCA
CGTGAGTGTTTTGCGACATTTTGCGACACCATGTGGTCACGCTGGGTATTTAAGCCC
GAGTGAGCACGCAGGGTCTCCATTTTGAAGCGGGAGGTTTGAACGCGCAGCC

| AAV1- RESIDUE | CODON CHANGE |
|---|---|
| AAV1- S277A | AGC→GCC |
| AAV1- S499A | AGC→GCC |
| AAV1- S526A | TCA→GCA |
| AAV1- S663A | TCA→GCA |
| AAV1- S669A | TCA→GCA |
| AAV1- T251A | ACC→GCC |
| AAV1- K137R | AAG→AGA |

### AAV2-Wild Type; SEQ ID No. 140

AATTCCCATCATCAATAATATACCTTATTTGGATTGAAGCCAATATGATAATGAGG
GGGTGGAGTTTGTGACGTGGCGCGGGGCGTGGGAACGGGGCGGGTGACGTAGTAGT
CTCTAGAGTCCTGTATTAGAGGTCACGTGAGTGTTTTGCGACATTTTGCGACACCAT
GTGGTCACGCTGGGTATTTAAGCCCGAGTGAGCACGCAGGGTCTCCATTTTGAAGCG
GGAGGTTTGAACGCGCAGCCACCAACGGCGGGGTTTTACGAGATTGTGATTAAGGT
CCCCAGCGACCTTGACGGGCATCTGCCCGGCATTTCTGACAGCTTTGTGAACTGGGT
GGCCGAGAAGGAATGGGAGTTGCCGCCAGATTCTGACATGGATCTGAATCTGATTG
AGCAGGCACCCCTGACCGTGGCCGAGAAGCTGCAGCGCGACTTTCTGACGGAATGG
CGCCGTGTGAGTAAGGCCCCGGAGGCCCTTTTCTTTGTGCAATTTGAGAAGGAGAG
AGCTACTTCCACATGCACGTGCTCGTGGAAACCACCGGGGTGAAATCCATGGTTTTG
GGACGTTTCCTGAGTCAGATTCGCGAAAAACTGATTCAGAGAATTTACCGCGGGATC
GAGCCGACTTTGCCAAACTGGTTCGCGGTCACAAAGACCAGAAATGGCGCCGGAGG
CGGGAACAAGGTGGTGGATGAGTGCTACATCCCCAATTACTTGCTCCCCAAAACCC
AGCCTGAGCTCCAGTGGGCGTGGACTAATATGGAACAGTATTTAAGCGCCTGTTTGA
ATCTCACGGAGCGTAAACGGTTGGTGGCGCAGCATCTGACGCACGTGTCGCAGACG
CAGGAGCAGAACAAAGAGAATCAGAATCCCAATTCTGATGCGCCGGTGATCAGATC
AAAAACTTCAGCCAGGTACATGGAGCTGGTCGGGTGGCTCGTGGACAAGGGGATTA
CCTCGGAGAAGCAGTGGATCCAGGAGGACCAGGCCTCATACATCTCCTTCAATGCG
GCCTCCAACTCGCGGTCCCAAATCAAGGCTGCCTTGGACAATGCGGGAAAGATTAT
GAGCCTGACTAAAACCGCCCCCGACTACCTGGTGGGCCAGCAGCCCGTGGAGGACA
TTTCCAGCAATCGGATTTATAAAATTTTGGAACTAAACGGGTACGATCCCCAATATG
CGGCTTCCGTCTTTCTGGGATGGGCCACGAAAAAGTTCGGCAAGAGGAACACCATCT
GGCTGTTTGGGCCTGCAACTACCGGGAAGACCAACATCGCGGAGGCCATAGCCCAC
ACTGTGCCCTTCTACGGGTGCGTAAACTGGACCAATGAGAACTTTCCCTTCAACGAC
TGTGTCGACAAGATGGTGATCTGGTGGGAGGAGGGGAAGATGACCGCCAAGGTCGT
GGAGTCGGCCAAAGCCATTCTCGGAGGAAGCAAGGTGCGCGTGGACCAGAAATGCA
AGTCCTCGGCCCAGATAGACCCGACTCCCGTGATCGTCACCTCCAACACCAACATGT
GCGCCGTGATTGACGGGAACTCAACGACCTTCGAACACCAGCAGCCGTTGCAAGAC
CGGATGTTCAAATTTGAACTCACCCGCCGTCTGGATCATGACTTTGGGAAGGTCACC
AAGCAGGAAGTCAAAGACTTTTTCCGGTGGGCAAAGGATCACGTGGTTGAGGTGGA
GCATGAATTCTACGTCAAAAAGGGTGGAGCCAAGAAAAGACCCGCCCCCAGTGACG
CAGATATAAGTGAGCCCAAACGGGTGCGCGAGTCAGTTGCGCAGCCATCGACGTCA
GACGCGGAAGCTTCGATCAACTACGCAGACAGGTACCAAAACAAATGTTCTCGTCA
CGTGGGCATGAATCTGATGCTGTTTCCCTGCAGACAATGCGAGAGAATGAATCAGA
ATTCAAATATCTGCTTCACTCACGGACAGAAAGACTGTTTAGAGTGCTTTCCCGTGT
CAGAATCTCAACCCGTTTCTGTCGTCAAAAAGGCGTATCAGAAACTGTGCTACATTC
ATCATATCATGGGAAAGGTGCCAGACGCTTGCACTGCCTGCGATCTGGTCAATGTGG
ATTTGGATGACTGCATCTTTGAACAATAAATGATTTAAATCAGGTATGGCTGCCGAT
GGTTATCTTCCAGATTGGCTCGAGGACACTCTCTCTGAAGGAATAAGACAGTGGTGG

AAGCTCAAACCTGGCCCACCACCACCAAAGCCCGCAGAGCGGCATAAGGACGACAG
CAGGGGTCTTGTGCTTCCTGGGTACAAGTACCTCGGACCCTTCAACGGACTCGACAA
GGGAGAGCCGGTCAACGAGGCAGACGCCGCGGCCCTCGAGCACGACAAAGCCTAC
GACCGGCAGCTCGACAGCGGAGACAACCCGTACCTCAAGTACAACCACGCCGACGC
GGAGTTTCAGGAGCGCCTTAAAGAAGATACGTCTTTTGGGGGCAACCTCGGACGAG
CAGTCTTCCAGGCGAAAAAGAGGGTTCTTGAACCTCTGGGCCTGGTTGAGGAACCTG
TTAAGACGGCTCCGGGAAAAAAGAGGCCGGTAGAGCACTCTCCTGTGGAGCCAGAC
TCCTCCTCGGGAACCGGAAAGGCGGGCCAGCAGCCTGCAAGAAAAAGATTGAATTT
TGGTCAGACTGGAGACGCAGACTCAGTACCTGACCCCCAGCCTCTCGGACAGCCAC
CAGCAGCCCCCTCTGGTCTGGGAACTAATACGATGGCTACAGGCAGTGGCGCACCA
ATGGCAGACAATAACGAGGGCGCCGACGGAGTGGGTAATTCCTCCGGAAATTGGCA
TTGCGATTCCACATGGATGGGCGACAGAGTCATCACCACCAGCACCCGAACCTGGG
CCCTGCCCACCTACAACAACCACCTCTACAAACAAATTTCCAGCCAATCAGGAGCCT
CGAACGACAATCACTACTTTGGCTACAGCACCCCTTGGGGGTATTTTGACTTCAACA
GATTCCACTGCCACTTTTCACCACGTGACTGGCAAAGACTCATCAACAACAACTGGG
GATTCCGACCCAAGAGACTCAACTTCAAGCTCTTTAACATTCAAGTCAAAGAGGTCA
CGCAGAATGACGGTACGACGACGATTGCCAATAACCTTACCAGCACGGTTCAGGTG
TTTACTGACTCGGAGTACCAGCTCCCGTACGTCCTCGGCTCGGCGCATCAAGGATGC
CTCCCGCCGTTCCCAGCAGACGTCTTCATGGTGCCACAGTATGGATACCTCACCCTG
AACAACGGGAGTCAGGCAGTAGGACGCTCTTCATTTTACTGCCTGGAGTACTTTCCT
TCTCAGATGCTGCGTACCGGAAACAACTTTACCTTCAGCTACACTTTTGAGGACGTT
CCTTTCCACAGCAGCTACGCTCACAGCCAGAGTCTGGACCGTCTCATGAATCCTCTC
ATCGACCAGTACCTGTATTACTTGAGCAGAACAAACACTCCAAGTGGAACCACCAC
GCAGTCAAGGCTTCAGTTTTCTCAGGCCGGAGCGAGTGACATTCGGGACCAGTCTAG
GAACTGGCTTCCTGGACCCTGTTACCGCCAGCAGCGAGTATCAAAGACATCTGCGG
ATAACAACAACAGTGAATACTCGTGGACTGGAGCTACCAAGTACCACCTCAATGGC
AGAGACTCTCTGGTGAATCCGGGCCCGGCCATGGCAAGCCACAAGGACGATGAAGA
AAAGTTTTTCCTCAGAGCGGGGTTCTCATCTTTGGGAAGCAAGGCTCAGAGAAAAC
AAATGTGGACATTGAAAAGGTCATGATTACAGACGAAGAGGAAATCAGGACAACCA
ATCCCGTGGCTACGGAGCAGTATGGTTCTGTATCTACCAACCTCCAGAGAGGCAACA
GACAAGCAGCTACCGCAGATGTCAACACACAAGGCGTTCTTCCAGGCATGGTCTGG
CAGGACAGAGATGTGTACCTTCAGGGGCCCATCTGGGCAAAGATTCCACACACGGA
CGGACATTTTCACCCCTCTCCCCTCATGGGTGGATTCGGACTTAAACACCCTCCTCCA
CAGATTCTCATCAAGAACACCCCGGTACCTGCGAATCCTTCGACCACCTTCAGTGCG
GCAAAGTTTGCTTCCTTCATCACACAGTACTCCACGGGACAGGTCAGCGTGGAGATC
GAGTGGGAGCTGCAGAAGGAAAACAGCAAACGCTGGAATCCCGAAATTCAGTACAC
TTCCAACTACAACAAGTCTGTTAATGTGGACTTTACTGTGGACACTAATGGCGTGTA
TTCAGAGCCTCGCCCCATTGGCACCAGATACCTGACTCGTAATCTGTAATTGCTTGTT
AATCAATAAACCGTTTAATTCGTTTCAGTTGAACTTTGGTCTCTGCGTATTTCTTTCTT
ATCTAGTTTCCATGCTCTAGAGAAACCTACGTCACCCGCCCCCGTTCCCACGCCCCGC

GCCACGTCACAAACTCCACCCCCTCATTATCATATTGGCTTCAATCCAAAATAAGGT
ATATTATTGATGATGCATCGCTGGCGTAATAGCGAAGAGGCCCGCACCGATCGCCCT
TCCCAACAGTTGCGCAGCCTGAATGGCGAATGGAATTCCAGACGATTGAGCGTCAA
AATGTAGGTATTTCCATGAGCGTTTTTCCTGTTGCAATGGCTGGCGGTAATATTGTTC
TGGATATTACCAGCAAGGCCGATAGTTTGAGTTCTTCTACTCAGGCAAGTGATGTTA
TTACTAATCAAAGAAGTATTGCGACAACGGTTAATTTGCGTGATGGACAGACTCTTT
TACTCGGTGGCCTCACTGATTATAAAAACACTTCTCAGGATTCTGGCGTACCGTTCCT
GTCTAAAATCCCTTTAATCGGCCTCCTGTTTAGCTCCCGCTCTGATTCTAACGAGGAA
AGCACGTTATACGTGCTCGTCAAAGCAACCATAGTACGCGCCCTGTAGCGGCGCATT
AAGCGCGGCGGGTGTGGTGGTTACGCGCAGCGTGACCGCTACACTTGCCAGCGCCC
TAGCGCCCGCTCCTTTCGCTTTCTTCCCTTCCTTTCTCGCCACGTTCGCCGGCTTTCCC
CGTCAAGCTCTAAATCGGGGGCTCCCTTTAGGGTTCCGATTTAGTGCTTTACGGCAC
CTCGACCCCAAAAAACTTGATTAGGGTGATGGTTCACGTAGTGGGCCATCGCCCTGA
TAGACGGTTTTTCGCCCTTTGACGTTGGAGTCCACGTTCTTTAATAGTGGACTCTTGT
TCCAAACTGGAACAACACTCAACCCTATCTCGGTCTATTCTTTTGATTTATAAGGGAT
TTTGCCGATTTCGGCCTATTGGTTAAAAAATGAGCTGATTTAACAAAAATTTAACGC
GAATTTTAACAAAATATTAACGCTTACAATTTAAATATTTGCTTATACAATCTTCCTG
TTTTTGGGGCTTTTCTGATTATCAACCGGGGTACATATGATTGACATGCTAGTTTTAC
GATTACCGTTCATCGATTCTCTTGTTTGCTCCAGACTCTCAGGCAATGACCTGATAGC
CTTTGTAGAGACCTCTCAAAAATAGCTACCCTCTCCGGCATGAATTTATCAGCTAGA
ACGGTTGAATATCATATTGATGGTGATTTGACTGTCTCCGGCCTTTCTCACCCGTTTG
AATCTTTACCTACACATTACTCAGGCATTGCATTTAAAATATATGAGGGTTCTAAAA
ATTTTTATCCTTGCGTTGAAATAAAGGCTTCTCCCGCAAAAGTATTACAGGGTCATA
ATGTTTTTGGTACAACCGATTTAGCTTTATGCTCTGAGGCTTTATTGCTTAATTTTGCT
AATTCTTTGCCTTGCCTGTATGATTTATTGGATGTTGGAATCGCCTGATGCGGTATTT
TCTCCTTACGCATCTGTGCGGTATTTCACACCGCATATGGTGCACTCTCAGTACAATC
TGCTCTGATGCCGCATAGTTAAGCCAGCCCCGACACCCGCCAACACCCGCTGACGCG
CCCTGACGGGCTTGTCTGCTCCCGGCATCCGCTTACAGACAAGCTGTGACCGTCTCC
GGGAGCTGCATGTGTCAGAGGTTTTCACCGTCATCACCGAAACGCGCGAGACGAAA
GGGCCTCGTGATACGCCTATTTTTATAGGTTAATGTCATGATAATAATGGTTTCTTAG
ACGTCAGGTGGCACTTTTCGGGGAAATGTGCGCGGAACCCCTATTTGTTTATTTTTCT
AAATACATTCAAATATGTATCCGCTCATGAGACAATAACCCTGATAAATGCTTCAAT
AATATTGAAAAAGGAAGAGTATGAGTATTCAACATTTCCGTGTCGCCCTTATTCCCT
TTTTTGCGGCATTTTGCCTTCCTGTTTTTGCTCACCCAGAAACGCTGGTGAAAGTAAA
AGATGCTGAAGATCAGTTGGGTGCACGAGTGGGTTACATCGAACTGGATCTCAACA
GCGGTAAGATCCTTGAGAGTTTTCGCCCCGAAGAACGTTTTCCAATGATGAGCACTT
TTAAAGTTCTGCTATGTGGCGCGGTATTATCCCGTATTGACGCCGGGCAAGAGCAAC
TCGGTCGCCGCATACACTATTCTCAGAATGACTTGGTTGAGTACTCACCAGTTCCCG
TATTGACGCCGGGCAAGAGCAACTCGGTCGCCGCATACACTATTCTCAGAATGACTT
GGTTGAGTACTCACCAGTCACAGAAAAGCATCTTACGGATGGCATGACAGTAAGAG

AATTATGCAGTGCTGCCATAACCATGAGTGATAACACTGCGGCCAACTTACTTCTGA
CAACGATCGGAGGACCGAAGGAGCTAACCGCTTTTTTGCACAACATGGGGGATCAT
GTAACTCGCCTTGATCGTTGGGAACCGGAGCTGAATGAAGCCATACCAAACGACGA
GCGTGACACCACGATGCCTGTAGCAATGGCAACAACGTTGCGCAAACTATTAACTG
GCGAACTACTTACTCTAGCTTCCCGGCAACAATTAATAGACTGGATGGAGGCGGATA
AAGTTGCAGGACCACTTCTGCGCTCGGCCCTTCCGGCTGGCTGGTTTATTGCTGATA
AATCTGGAGCCGGTGAGCGTGGGTCTCGCGGTATCATTGCAGCACTGGGGCCAGAT
GGTAAGCCCTCCCGTATCGTAGTTATCTACACGACGGGGAGTCAGGCAACTATGGAT
GAACGAAATAGACAGATCGCTGAGATAGGTGCCTCACTGATTAAGCATTGGTAACT
GTCAGACCAAGTTTACTCATATATACTTTAGATTGATTTAAAACTTCATTTTTAATTT
AAAAGGATCTAGGTGAAGATCCTTTTTGATAATCTCATGACCAAAATCCCTTAACGT
GAGTTTTCGTTCCACTGAGCGTCAGACCCCGTAGAAAAGATCAAAGGATCTTCTTGA
GATCCTTTTTTTCTGCGCGTAATCTGCTGCTTGCAAACAAAAAAACCACCGCTACCA
GCGGTGGTTTGTTTGCCGGATCAAGAGCTACCAACTCTTTTTCCGAAGGTAACTGGC
TTCAGCAGAGCGCAGATACCAAATACTGTTCCTTCTAGTGTAGCCGTAGTTAG
GCCACCACTTCAAGAACTCTGTAGCACCGCCTACATACCTCGCTCTGCTAATCCTGT
TACCAGTGGCTGCTGCCAGTGGCGATAAGTCGTGTCTTACCGGGTTGGACTCAAGAC
GATAGTTACCGGATAAGGCGCAGCGGTCGGGCTGAACGGGGGGTTCGTGCACACAG
CCCAGCTTGGAGCGAACGACCTACACCGAACTGATATTTTTTTTTCGTGAGCTATG
AGAAAGCGCCACGCTTCCCGAAGGGAGAAAGGCGGACAGGTATCCGGTAAGCGGC
AGGGTCGGAACAGGAGAGCGCACGAGGGAGCTTCCAGGGGGAAACGCCTGGTATCT
TTATAGTCCTGTCGGGTTTCGCCACCTCTGACTTGAGCGTCGATTTTTGTGATGCTCG
TCAGGGGGGCGGAGCCTATGGAAAAACGCCAGCAACGCGGCCTTTTTACGGTTCCT
GGCCTTTTGCTGGCCTTTTGCTCACATGTTCTTTCCTGCGTTATCCCCTGATTCTGTGG
ATAACCGTATTACCGCCTTTGAGTGAGCTGATACCGCTCGCCGCAGCCGAACGACCG
AGCGCAGCGAGTCAGTGAGCGAGGAAGCGGAAGAGCGCCCAATACGCAAACCGCC
TCTCCCCGCGCGTTGGCCGATTCATTAATGCAG

### AAV2-Mutant ; SEQ ID No. 150

AATTCCCATCATCAATAATATACCTTATTTTGGATTGAAGCCAATATGATAATGAGG
GGGTGGAGTTTGTGACGTGGCGCGGGGCGTGGGAACGGGGCGGGTGACGTAGTAGT
CTCTAGAGTCCTGTATTAGAGGTCACGTGAGTGTTTTGCGACATTTTGCGACACCAT
GTGGTCACGCTGGGTATTTAAGCCCGAGTGAGCACGCAGGGTCTCCATTTTGAAGCG
GGAGGTTTGAACGCGCAGCCACCAACGGCGGGGTTTTACGAGATTGTGATTAAGGT

CCCCAGCGACCTTGACGGGCATCTGCCCGGCATTTCTGACAGCTTTGTGAACTGGGT
GGCCGAGAAGGAATGGGAGTTGCCGCCAGATTCTGACATGGATCTGAATCTGATTG
AGCAGGCACCCCTGACCGTGGCCGAGAAGCTGCAGCGCGACTTTCTGACGGAATGG
CGCCGTGTGAGTAAGGCCCCGGAGGCCCTTTTCTTTGTGCAATTTGAGAAGGGAGAG
AGCTACTTCCACATGCACGTGCTCGTGGAAACCACCGGGGTGAAATCCATGGTTTTG
GGACGTTTCCTGAGTCAGATTCGCGAAAAACTGATTCAGAGAATTTACCGCGGGATC
GAGCCGACTTTGCCAAACTGGTTCGCGGTCACAAAGACCAGAAATGGCGCCGGAGG
CGGGAACAAGGTGGTGGATGAGTGCTACATCCCCAATTACTTGCTCCCCAAAACCC
AGCCTGAGCTCCAGTGGGCGTGGACTAATATGGAACAGTATTTAAGCGCCTGTTTGA
ATCTCACGGAGCGTAAACGGTTGGTGGCGCAGCATCTGACGCACGTGTCGCAGACG
CAGGAGCAGAACAAAGAGAATCAGAATCCCAATTCTGATGCGCCGGTGATCAGATC
AAAAACTTCAGCCAGGTACATGGAGCTGGTCGGGTGGCTCGTGGACAAGGGGATTA
CCTCGGAGAAGCAGTGGATCCAGGAGGACCAGGCCTCATACATCTCCTTCAATGCG
GCCTCCAACTCGCGGTCCCAAATCAAGGCTGCCTTGGACAATGCGGGAAAGATTAT
GAGCCTGACTAAAACCGCCCCCGACTACCTGGTGGGCCAGCAGCCCGTGGAGGACA
TTTCCAGCAATCGGATTTATAAAATTTTGGAACTAAACGGGTACGATCCCCAATATG
CGGCTTCCGTCTTTCTGGGATGGGCCACGAAAAAGTTCGGCAAGAGGAACACCATCT
GGCTGTTTGGGCCTGCAACTACCGGGAAGACCAACATCGCGGAGGCCATAGCCCAC
ACTGTGCCCTTCTACGGGTGCGTAAACTGGACCAATGAGAACTTTCCCTTCAACGAC
TGTGTCGACAAGATGGTGATCTGGTGGGAGGAGGGGAAGATGACCGCCAAGGTCGT
GGAGTCGGCCAAAGCCATTCTCGGAGGAAGCAAGGTGCGCGTGGACCAGAAATGCA
AGTCCTCGGCCCAGATAGACCCGACTCCCGTGATCGTCACCTCCAACACCAACATGT
GCGCCGTGATTGACGGGAACTCAACGACCTTCGAACACCAGCAGCCGTTGCAAGAC

CGGATGTTCAAATTTGAACTCACCCGCCGTCTGGATCATGACTTTGGGAAGGTCACC
AAGCAGGAAGTCAAAGACTTTTTCCGGTGGGCAAAGGATCACGTGGTTGAGGTGGA
GCATGAATTCTACGTCAAAAAGGGTGGAGCCAAGAAAAGACCCGCCCCCAGTGACG
CAGATATAAGTGAGCCCAAACGGGTGCGCGAGTCAGTTGCGCAGCCATCGACGTCA
GACGCGGAAGCTTCGATCAACTACGCAGACAGGTACCAAAACAAATGTTCTCGTCA
CGTGGGCATGAATCTGATGCTGTTTCCCTGCAGACAATGCGAGAGAATGAATCAGA
ATTCAAATATCTGCTTCACTCACGGACAGAAAGACTGTTTAGAGTGCTTTCCCGTGT
CAGAATCTCAACCCGTTTCTGTCGTCAAAAAGGCGTATCAGAAACTGTGCTACATTC
ATCATATCATGGGAAAGGTGCCAGACGCTTGCACTGCCTGCGATCTGGTCAATGTGG
ATTTGGATGACTGCATCTTTGAACAATAAATGATTTAAATCAGGTATGGCTGCCGAT
GGTTATCTTCCAGATTGGCTCGAGGACACTCTCTCTGAAGGAATAAGACAGTGGTGG
K39R
AAGCTCAAACCTGGCCCACCACCACCAAAGCCCGCAGAACGGCATAGGGACGACAG
CAGGGGTCTTGTGCTTCCTGGGTACAAGTACCTCGGACCCTTCAACGGACTCGACAA
GGGAGAGCCGGTCAACGAGGCAGACGCCGCGGCCCTCGAGCACGACAAAGCCTAC
GACCGGCAGCTCGACAGCGGAGACAACCCGTACCTCAAGTACAACCACGCCGACGC
GGAGTTTCAGGAGCGCCTTAAAGAAGATACGTCTTTTGGGGGCAACCTCGGACGAG
CAGTCTTCCAGGCGAAAAAGAGGGTTCTTGAACCTCTGGGCCTGGTTGAGGAACCTG
K137R K143R
TTAGGACGGCCCCGGGAAAAAGGAGGCCGGTAGAGCACTCTCCTGTGGAGCCAGAC
K161R
TCCTCCTCGGGAACCGGAAGGGCCGGCCAGCAGCCTGCAAGAAAAAGATTGAATTT
TGGTCAGACTGGAGACGCAGACTCAGTACCTGACCCCCAGCCTCTCGGACAGCCAC
CAGCAGCCCCCTCTGGTCTGGGAACTAATACGATGGCTACAGGCAGTGGCGCACCA
ATGGCAGACAATAACGAGGGCGCCGACGGAGTGGGTAATTCCTCCGGAAATTGGCA
TTGCGATTCCACATGGATGGGCGACAGAGTCATCACCACCAGCACCCGAACCTGGG

T251A
CCCTGCCGGCCTACAACAACCACCTCTACAAACAAATTTCCAGCCAATCAGGAGCCT
S276A
CGAACGACAATCACTACTTTGGCTACGCCACCCCTTGGGGGTATTTTGACTTCAACA
GATTCCACTGCCACTTTTCACCACGTGACTGGCAAAGACTCATCAACAACAACTGGG
GATTCCGACCCAAGAGACTCAACTTCAAGCTCTTTAACATTCAAGTCAAAGAGGTCA
CGCAGAATGACGGTACGACGACGATTGCCAATAACCTTACCAGCACGGTTCAGGTG
TTTACTGACTCGGAGTACCAGCTCCCGTACGTCCTCGGCTCGGCGCATCAAGGATGC
CTCCCGCCGTTCCCAGCAGACGTCTTCATGGTGCCACAGTATGGATACCTCACCCTG
AACAACGGGAGTCAGGCAGTAGGACGCTCTTCATTTTACTGCCTGGAGTACTTTCCT
TCTCAGATGCTGCGTACCGGAAACAACTTTACCTTCAGCTACACTTTTGAGGACGTT
CCTTTCCACAGCAGCTACGCTCACAGCCAGAGTCTGGACCGTCTCATGAATCCTCTC
T454A
ATCGACCAGTACCTGTATTACTTAAGCAGAACAAACACTCCAAGTGGAGCCACCAC
GCAGTCAAGGCTTCAGTTTTCTCAGGCCGGAGCGAGTGACATTCGGGACCAGTCTAG
S489A K490R
GAACTGGCTTCCTGGACCCTGTTACCGCCAGCAGCGAGTAGCTAAGACATCTGCAG
S498A T503A K507R
ATAACAACAACGCTGAATACTCGTGGGCTGGAGCTACCAGATACCACCTCAATGGC
S525A K527R
AGAGACTCTCTGGTGAATCCGGGCCCGGCGATGGCAGCCCACAGGGACGATGAAGA
K532R S537A K544R S547A
AAGGTTTTTCCTCAGGCCGGGGTTCTCATCTTTGGTAGACAAGGCGCCGAGAAAAC
AAATGTGGACATTGAAAAGGTCATGATTACAGACGAAGAGGAAATCAGGACAACCA
ATCCCGTGGCTACGGAGCAGTATGGTTCTGTATCTACCAACCTCCAGAGAGGCAACA
GACAAGCAGCTACCGCAGATGTCAACACACAAGGCGTTCTTCCAGGCATGGTCTGG
CAGGACAGAGATGTGTACCTTCAGGGGCCCATCTGGGCAAAGATTCCACACACGGA
CGGACATTTTCACCCCTCTCCCCTCATGGGTGGATTCGGACTTAAACACCCTCCTCCA
S662A
CAGATTCTCATCAAGAACACCCCGGTACCTGCGAATCCGTCGACCACCTTCAGTGCG
S668A T671A
GCCAAGTTTGCTGCCTTCATCGCACAGTACTCCACGGGACAGGTCAGCGTGGAGATC

T701A
GAGTGGGAGCTGCAGAAGGAAAACAGCAAACGCTGGAATCCCGAAATTCAGTACGC
T713A T716A
TTCCAACTACAACAAGTCTGTTAATGTAGACTTTGCTGTGGACGCTAATGGCGTGTA
TTCAGAGCCTCGCCCCATTGGCACCAGATACCTGACTCGTAATCTGTAATTGCTTGTT
AATCAATAAACCGTTTAATTCGTTTCAGTTGAACTTTGGTCTCTGCGTATTTCTTTCTT
ATCTAGTTTCCATGCTCTAGAGAAACCTACGTCACCCGCCCCGTTCCCACGCCCCGC
GCCACGTCACAAACTCCACCCCCTCATTATCATATTGGCTTCAATCCAAAATAAGGT
ATATTATTGATGATGCATCGCTGGCGTAATAGCGAAGAGGCCCGCACCGATCGCCCT
TCCCAACAGTTGCGCAGCCTGAATGGCGAATGGAATTCCAGACGATTGAGCGTCAA
AATGTAGGTATTTCCATGAGCGTTTTTCCTGTTGCAATGGCTGGCGGTAATATTGTTC
TGGATATTACCAGCAAGGCCGATAGTTTGAGTTCTTCTACTCAGGCAAGTGATGTTA
TTACTAATCAAAGAAGTATTGCGACAACGGTTAATTTGCGTGATGGACAGACTCTTT
TACTCGGTGGCCTCACTGATTATAAAAACACTTCTCAGGATTCTGGCGTACCGTTCCT
GTCTAAAATCCCTTTAATCGGCCTCCTGTTTAGCTCCCGCTCTGATTCTAACGAGGAA
AGCACGTTATACGTGCTCGTCAAAGCAACCATAGTACGCGCCCTGTAGCGGCGCATT
AAGCGCGGCGGGTGTGGTGGTTACGCGCAGCGTGACCGCTACACTTGCCAGCGCCC
TAGCGCCCGCTCCTTTCGCTTTCTTCCCTTCCTTTCTCGCCACGTTCGCCGGCTTTCCC
CGTCAAGCTCTAAATCGGGGGCTCCCTTTAGGGTTCCGATTTAGTGCTTTACGGCAC
CTCGACCCCAAAAAACTTGATTAGGGTGATGGTTCACGTAGTGGGCCATCGCCCTGA
TAGACGGTTTTTCGCCCTTTGACGTTGGAGTCCACGTTCTTTAATAGTGGACTCTTGT
TCCAAACTGGAACAACACTCAACCCTATCTCGGTCTATTCTTTTGATTTATAAGGGAT
TTTGCCGATTTCGGCCTATTGGTTAAAAAATGAGCTGATTTAACAAAAATTTAACGC
GAATTTTAACAAAATATTAACGCTTACAATTTAAATATTTGCTTATACAATCTTCCTG
TTTTTGGGGCTTTTCTGATTATCAACCGGGGTACATATGATTGACATGCTAGTTTTAC

GATTACCGTTCATCGATTCTCTTGTTTGCTCCAGACTCTCAGGCAATGACCTGATAGC
CTTTGTAGAGACCTCTCAAAAATAGCTACCCTCTCCGGCATGAATTTATCAGCTAGA
ACGGTTGAATATCATATTGATGGTGATTTGACTGTCTCCGGCCTTTCTCACCCGTTTG
AATCTTTACCTACACATTACTCAGGCATTGCATTTAAAATATATGAGGGTTCTAAAA
ATTTTTATCCTTGCGTTGAAATAAAGGCTTCTCCCGCAAAAGTATTACAGGGTCATA
ATGTTTTTGGTACAACCGATTTAGCTTTATGCTCTGAGGCTTTATTGCTTAATTTTGCT
AATTCTTTGCCTTGCCTGTATGATTTATTGGATGTTGGAATCGCCTGATGCGGTATTT
TCTCCTTACGCATCTGTGCGGTATTTCACACCGCATATGGTGCACTCTCAGTACAATC
TGCTCTGATGCCGCATAGTTAAGCCAGCCCCGACACCCGCCAACACCCGCTGACGCG
CCCTGACGGGCTTGTCTGCTCCCGGCATCCGCTTACAGACAAGCTGTGACCGTCTCC
GGGAGCTGCATGTGTCAGAGGTTTTCACCGTCATCACCGAAACGCGCGAGACGAAA
GGGCCTCGTGATACGCCTATTTTTATAGGTTAATGTCATGATAATAATGGTTTCTTAG
ACGTCAGGTGGCACTTTTCGGGGAAATGTGCGCGGAACCCCTATTTGTTTATTTTTCT
AAATACATTCAAATATGTATCCGCTCATGAGACAATAACCCTGATAAATGCTTCAAT
AATATTGAAAAAGGAAGAGTATGAGTATTCAACATTTCCGTGTCGCCCTTATTCCCT
TTTTTGCGGCATTTTGCCTTCCTGTTTTTGCTCACCCAGAAACGCTGGTGAAAGTAAA
AGATGCTGAAGATCAGTTGGGTGCACGAGTGGGTTACATCGAACTGGATCTCAACA
GCGGTAAGATCCTTGAGAGTTTTCGCCCCGAAGAACGTTTTCCAATGATGAGCACTT
TTAAAGTTCTGCTATGTGGCGCGGTATTATCCCGTATTGACGCCGGGCAAGAGCAAC
TCGGTCGCCGCATACACTATTCTCAGAATGACTTGGTTGAGTACTCACCAGTTCCCG
TATTGACGCCGGGCAAGAGCAACTCGGTCGCCGCATACACTATTCTCAGAATGACTT
GGTTGAGTACTCACCAGTCACAGAAAAGCATCTTACGGATGGCATGACAGTAAGAG
AATTATGCAGTGCTGCCATAACCATGAGTGATAACACTGCGGCCAACTTACTTCTGA

CAACGATCGGAGGACCGAAGGAGCTAACCGCTTTTTTGCACAACATGGGGGATCAT
GTAACTCGCCTTGATCGTTGGGAACCGGAGCTGAATGAAGCCATACCAAACGACGA
GCGTGACACCACGATGCCTGTAGCAATGGCAACAACGTTGCGCAAACTATTAACTG
GCGAACTACTTACTCTAGCTTCCCGGCAACAATTAATAGACTGGATGGAGGCGGATA
AAGTTGCAGGACCACTTCTGCGCTCGGCCCTTCCGGCTGGCTGGTTTATTGCTGATA
AATCTGGAGCCGGTGAGCGTGGGTCTCGCGGTATCATTGCAGCACTGGGGCCAGAT
GGTAAGCCCTCCCGTATCGTAGTTATCTACACGACGGGGAGTCAGGCAACTATGGAT
GAACGAAATAGACAGATCGCTGAGATAGGTGCCTCACTGATTAAGCATTGGTAACT
GTCAGACCAAGTTTACTCATATATACTTTAGATTGATTTAAAACTTCATTTTTAATTT
AAAAGGATCTAGGTGAAGATCCTTTTTGATAATCTCATGACCAAAATCCCTTAACGT
GAGTTTTCGTTCCACTGAGCGTCAGACCCCGTAGAAAAGATCAAAGGATCTTCTTGA
GATCCTTTTTTTCTGCGCGTAATCTGCTGCTTGCAAACAAAAAAACCACCGCTACCA
GCGGTGGTTTGTTTGCCGGATCAAGAGCTACCAACTCTTTTTCCGAAGGTAACTGGC
TTCAGCAGAGCGCAGATACCAAATACTGTTCCTTCTAGTGTAGCCGTAGTTAG
GCCACCACTTCAAGAACTCTGTAGCACCGCCTACATACCTCGCTCTGCTAATCCTGT
TACCAGTGGCTGCTGCCAGTGGCGATAAGTCGTGTCTTACCGGGTTGGACTCAAGAC
GATAGTTACCGGATAAGGCGCAGCGGTCGGGCTGAACGGGGGGTTCGTGCACACAG
CCCAGCTTGGAGCGAACGACCTACACCGAACTGATATTTTTTTTTCGTGAGCTATG
AGAAAGCGCCACGCTTCCCGAAGGGAGAAAGGCGGACAGGTATCCGGTAAGCGGC
AGGGTCGGAACAGGAGAGCGCACGAGGGAGCTTCCAGGGGGAAACGCCTGGTATCT
TTATAGTCCTGTCGGGTTTCGCCACCTCTGACTTGAGCGTCGATTTTTGTGATGCTCG
TCAGGGGGGCGGAGCCTATGGAAAAACGCCAGCAACGCGGCCTTTTTACGGTTCCT
GGCCTTTTGCTGGCCTTTTGCTCACATGTTCTTTCCTGCGTTATCCCCTGATTCTGTGG

ATAACCGTATTACCGCCTTTGAGTGAGCTGATACCGCTCGCCGCAGCCGAACGACCG

AGCGCAGCGAGTCAGTGAGCGAGGAAGCGGAAGAGCGCCCAATACGCAAACCGCC

TCTCCCCGCGCGTTGGCCGATTCATTAATGCAG

| **AAV2 RESIDUE** | **NUCLEOTIDE CHANGE** |
|---|---|
| AAV2-S276A | AGC→GCC |
| AAV2-S489A | TCA→GCT |
| AAV2-S498A | AGT→GCT |
| AAV2-S525A | AGC→GCC |
| AAV2-S537A | AGC→GCC |
| AAV2-S547A | TCA→GCC |
| AAV2-S662A | AGT→GCT |
| AAV2-S668A | TCC→GCC |
| AAV2-T251A | ACC→GCC |
| AAV2-T454A | ACC→GCC |
| AAV2-T503A | ACT→GCT |
| AAV2-T671A | ACA→GCA |
| AAV2-T701A | ACT→GCT |
| AAV2-T713A | ACT→GCT |
| AAV2-T716A | ACT→GCT |
| AAV2-K39R | AAG→AGG |
| AAV2-K137R | AAG→AGG |
| AAV2-K143R | AAG→AGG |
| AAV2-K161R | AAG→AGG |
| AAV2-K490R | AAG→AGG |
| AAV2-K507R | AAG→AGA |
| AAV2-K527R | AAG→AGG |
| AAV2-K532R | AAG→AGG |
| AAV2-K544R | AAG→AGA |

### AAV3-Wild Type; SEQ ID No. 141

AATTCCCATCATCAATAATATACCTTATTTAGGATTGAAGCCAATATGATAATGAGC
CGGTGGAGTTTGTGACGTGGCGCGGGGCGTGGGAACGGGGCGGGTGACGTAGTAGT
CTCTAGAGTCCTGTATTAGAGGTCACGTGAGTGTTTTGCGACTTTTGCGACACCATG
TGGTCACGCTGGGTATTTAAGCCCGAGTGAGCACGCAGGGTCTCCATTTTGAAGCGG
GAGGTTTGAACGCGCAGCCACCACGGGGGGTTTTACGAGATTGTGATTAAGGTCC
CCAGCGACCTTGACGGGCATCTGCCCGGCATTTCTGACAGCTTTGTGAACTGGGTGG
CCGAGAAGGAATGGGAGTTGCCGCCAGATTCTGACATGGATCTGAATCTGATTGAG
CAGGCACCCCTGACCGTGGCCGAGAAGCTGCAGCGCGACTTTCTGACGGAATGGCG
CCGTGTGAGTAAGGCCCCGGAGGCCCTTTTCTTTGTGCAATTTGAGAAGGAGAGAG
CTACTTCCACATGCACGTGCTCGTGGAAACCACCGGGGTGAAATCCATGGTTTTGGG
ACGTTTCCTGAGTCAGATTCGCGAAAAACTGATTCAGAGAATTTACCGCGGGATCGA
GCCGACTTTGCCAAACTGGTTCGCGGTCACAAAGACCAGAAATGGCGCCGGAGGCG
GGAACAAGGTGGTGGATGAGTGCTACATCCCCAATTACTTGCTCCCCAAAACCCAG
CCTGAGCTCCAGTGGGCGTGGACTAATATGGAACAGTATTTAAGCGCCTGTTTGAAT
CTCACGGAGCGTAAACGGTTGGTGGCGCAGCATCTGACGCACGTGTCGCAGACGCA
GGAGCAGAACAAAGAGAATCAGAATCCCAATTCTGATGCGCCGGTGATCAGATCAA
AAACTTCAGCCAGGTACATGGAGCTGGTCGGGTGGCTCGTGGACAAGGGGATTACC
TCGGAGAAGCAGTGGATCCAGGAGGACCAGGCCTCATACATCTCCTTCAATGCGGC
CTCCAACTCGCGGTCCCAAATCAAGGCTGCCTTGGACAATGCGGGAAAGATTATGA
GCCTGACTAAAACCGCCCCCGACTACCTGGTGGGCCAGCAGCCCGTGGAGGACATT
TCCAGCAATCGGATTTATAAAATTTTGGAACTAAACGGGTACGATCCCCAATATGCG
GCTTCCGTCTTTCTGGGATGGGCCACGAAAAAGTTCGGCAAGAGGAACACCATCTG
GCTGTTTGGGCCTGCAACTACCGGGAAGACCAACATCGCGGAGGCCATAGCCCACA
CTGTGCCCTTCTACGGGTGCGTAAACTGGACCAATGAGAACTTTCCCTTCAACGACT
GTGTCGACAAGATGGTGATCTGGTGGGAGGAGGGGAAGATGACCGCCAAGGTCGTG
GAGTCGGCCAAAGCCATTCTCGGAGGAAGCAAGGTGCGCGTGGACCAGAAATGCAA
GTCCTCGGCCCAGATAGACCCGACTCCCGTGATCGTCACCTCCAACACCAACATGTG
CGCCGTGATTGACGGGAACTCAACGACCTTCGAACACCAGCAGCCGTTGCAAGACC
GGATGTTCAAATTTGAACTCACCCGCCGTCTGGATCATGACTTTGGGAAGGTCACCA
AGCAGGAAGTCAAAGACTTTTTCCGGTGGGCAAAGGATCACGTGGTTGAGGTGGAG
CATGAATTCTACGTCAAAAAGGGTGGAGCCAAGAAAAGACCCGCCCCCAGTGACGC
AGATATAAGTGAGCCCAAACGGGTGCGCGAGTCAGTTGCGCAGCCATCGACGTCAG
ACGCGGAAGCTTCGATCAACTACGCAGACAGGTACCAAAACAAATGTTCTCGTCAC
GTGGGCATGAATCTGATGCTGTTTCCCTGCAGACAATGCGAGAGAATGAATCAGAA
TTCAAATATCTGCTTCACTCACGGACAGAAAGACTGTTTAGAGTGCTTTCCCGTGTC
AGAATCTCAACCCGTTTCTGTCGTCAAAAAGGCGTATCAGAAACTGTGCTACATTCA
TCATATCATGGGAAAGGTGCCAGACGCTTGCACTGCCTGCGATCTGGTCAATGTGGA
TTTGGATGACTGCATCTTTGAACAATAAATGATTTAAATCAGGTATGGCTGCTGACG
GTTATCTTCCAGATTGGCTCGAGGACAACCTTTCTGAAGGCATTCGTGAGTGGTGGG

CTCTGAAACCTGGAGTCCCTCAACCCAAAGCGAACCAACAACACCAGGACAACCGT
CGGGGTCTTGTGCTTCCGGGTTACAAATACCTCGGACCCGGTAACGGACTCGACAAA
GGAGAGCCGGTCAACGAGGCGGACGCGGCAGCCCTCGAACACGACAAAGCTTACG
ACCAGCAGCTCAAGGCCGGTGACAACCCGTACCTCAAGTACAACCACGCCGACGCC
GAGTTTCAGGAGCGTCTTCAAGAAGATACGTCTTTTGGGGGCAACCTTGGCAGAGCA
GTCTTCCAGGCCAAAAAGAGGATCCTTGAGCCTCTTGGTCTGGTTGAGGAAGCAGCT
AAAACGGCTCCTGGAAAGAAGGGGGCTGTAGATCAGTCTCCTCAGGAACCGGACTC
ATCATCTGGTGTTGGCAAATCGGGCAAACAGCCTGCCAGAAAAAGACTAAATTTCG
GTCAGACTGGAGACTCAGAGTCAGTCCCAGACCCTCAACCTCTCGGAGAACCACCA
GCAGCCCCCACAAGTTTGGGATCTAATACAATGGCTTCAGGCGGTGGCGCACCAAT
GGCAGACAATAACGAGGGTGCCGATGGAGTGGGTAATTCCTCAGGAAATTGGCATT
GCGATTCCCAATGGCTGGGCGACAGAGTCATCACCACCAGCACCAGAACCTGGGCC
CTGCCCACTTACAACAACCATCTCTACAAGCAAATCTCCAGCCAATCAGGAGCTTCA
AACGACAACCACTACTTTGGCTACAGCACCCCTTGGGGGTATTTTGACTTTAACAGA
TTCCACTGCCACTTCTCACCACGTGACTGGCAGCGACTCATTAACAACAACTGGGGA
TTCCGGCCCAAGAAACTCAGCTTCAAGCTCTTCAACATCCAAGTTAGAGGGGTCACG
CAGAACGATGGCACGACGACTATTGCCAATAACCTTACCAGCACGGTTCAAGTGTTT
ACGGACTCGGAGTATCAGCTCCCGTACGTGCTCGGGTCGGCGCACCAAGGCTGTCTC
CCGCCGTTTCCAGCGGACGTCTTCATGGTCCCTCAGTATGGATACCTCACCCTGAAC
AACGGAAGTCAAGCGGTGGGACGCTCATCCTTTTACTGCCTGGAGTACTTCCCTTCG
CAGATGCTAAGGACTGGAAATAACTTCCAATTCAGCTATACCTTCGAGGATGTACCT
TTTCACAGCAGCTACGCTCACAGCCAGAGTTTGGATCGCTTGATGAATCCTCTTATT
GATCAGTATCTGTACTACCTGAACAGAACGCAAGGAACAACCTCTGGAACAACCAA
CCAATCACGGCTGCTTTTTAGCCAGGCTGGGCCTCAGTCTATGTCTTTGCAGGCCAG
AAATTGGCTACCTGGGCCCTGCTACCGGCAACAGAGACTTTCAAAGACTGCTAACG
ACAACAACAACAGTAACTTTCCTTGGACAGCGGCCAGCAAATATCATCTCAATGGC
CGCGACTCGCTGGTGAATCCAGGACCAGCTATGGCCAGTCACAAGGACGATGAAGA
AAAATTTTTCCCTATGCACGGCAATCTAATATTTGGCAAAGAAGGGACAACGGCAA
GTAACGCAGAATTAGATAATGTAATGATTACGGATGAAGAAGAGATTCGTACCACC
AATCCTGTGGCAACAGAGCAGTATGGAACTGTGGCAAATAACTTGCAGAGCTCAAA
TACAGCTCCCACGACTGGAACTGTCAATCATCAGGGGGCCTTACCTGGCATGGTGTG
GCAAGATCGTGACGTGTACCTTCAAGGACCTATCTGGGCAAAGATTCCTCACACGGA
TGGACACTTTCATCCTTCTCCTCTGATGGGAGGCTTTGGACTGAAACATCCGCCTCCT
CAAATCATGATCAAAAATACTCCGGTACCGGCAAATCCTCCGACGACTTTCAGCCCG
GCCAAGTTTGCTTCATTTATCACTCAGTACTCCACTGGACAGGTCAGCGTGGAAATT
GAGTGGGAGCTACAGAAAGAAAACAGCAAACGTTGGAATCCAGAGATTCAGTACAC
TTCCAACTACAACAAGTCTGTTAATGTGGACTTTACTGTAGACACTAATGGTGTTTAT
AGTGAACCTCGCCCTATTGGAACCCGGTATCTCACACGAAACTTGTGATTGCTTGTT
AATCAATAAACCGTTTAATTCGTTTCAGTTGAACTTTGGTCTCTGCGTATTTCTTTCTT
ATCTAGTTTCCATGCTCTAGACTACTACGTCACCCGCCCCGTTCCCACGCCCCGCGCC
ACGTCACAAACTCCACCCCCTCATTATCATATTGGCTTCAATCCAAAATAAGGTATA

TTATTGATGATGCATCGCTGGCGTAATAGCGAAGAGGCCCGCACCGATCGCCCTTCC
CAACAGTTGCGCAGCCTGAATGGCGAATGGAATTCCAGACGATTGAGCGTCAAAAT
GTAGGTATTTCCATGAGCGTTTTTCCTGTTGCAATGGCTGGCGGTAATATTGTTCTGG
ATATTACCAGCAAGGCCGATAGTTTGAGTTCTTCTACTCAGGCAAGTGATGTTATTA
CTAATCAAAGAAGTATTGCGACAACGGTTAATTTGCGTGATGGACAGACTCTTTTAC
TCGGTGGCCTCACTGATTATAAAAACACTTCTCAGGATTCTGGCGTACCGTTCCTGTC
TAAAATCCCTTTAATCGGCCTCCTGTTTAGCTCCCGCTCTGATTCTAACGAGGAAAG
CACGTTATACGTGCTCGTCAAAGCAACCATAGTACGCGCCCTGTAGCGGCGCATTAA
GCGCGGCGGGTGTGGTGGTTACGCGCAGCGTGACCGCTACACTTGCCAGCGCCCTA
GCGCCCGCTCCTTTCGCTTTCTTCCCTTCCTTTCTCGCCACGTTCGCCGGCTTTCCCCG
TCAAGCTCTAAATCGGGGGCTCCCTTTAGGGTTCCGATTTAGTGCTTTACGGCACCTC
GACCCCAAAAAACTTGATTAGGGTGATGGTTCACGTAGTGGGCCATCGCCCTGATAG
ACGGTTTTTCGCCCTTTGACGTTGGAGTCCACGTTCTTTAATAGTGGACTCTTGTTCC
AAACTGGAACAACACTCAACCCTATCTCGGTCTATTCTTTTGATTTATAAGGGATTTT
GCCGATTTCGGCCTATTGGTTAAAAAATGAGCTGATTTAACAAAAATTTAACGCGAA
TTTTAACAAAATATTAACGTTTACAATTTAAATATTTGCTTATACAATCTTCCTGTTT
TTGGGGCTTTTCTGATTATCAACCGGGGTACATATGATTGACATGCTAGTTTTACGAT
TACCGTTCATCGATTCTCTTGTTTGCTCCAGACTCTCAGGCAATGACCTGATAGCCTT
TGTAGAGACCTCTCAAAAATAGCTACCCTCTCCGGCATGAATTTATCAGCTAGAACG
GTTGAATATCATATTGATGGTGATTTGACTGTCTCCGGCCTTTCTCACCCGTTTGAAT
CTTTACCTACACATTACTCAGGCATTGCATTTAAAATATATGAGGGTTCTAAAAATTT
TTATCCTTGCGTTGAAATAAAGGCTTCTCCCGCAAAAGTATTACAGGGTCATAATGT
TTTTGGTACAACCGATTTAGCTTTATGCTCTGAGGCTTTATTGCTTAATTTTGCTAATT
CTTTGCCTTGCCTGTATGATTTATTGGATGTTGGAATTCCTGATGCGGTATTTTCTCCT
TACGCATCTGTGCGGTATTTCACACCGCATATGGTGCACTCTCAGTACAATCTGCTCT
GATGCCGCATAGTTAAGCCAGCCCCGACACCCGCCAACACCCGCTGACGCGCCCTG
ACGGGCTTGTCTGCTCCCGGCATCCGCTTACAGACAAGCTGTGACCGTCTCCGGGAG
CTGCATGTGTCAGAGGTTTTCACCGTCATCACCGAAACGCGCGAGACGAAAGGGCC
TCGTGATACGCCTATTTTTATAGGTTAATGTCATGATAATAATGGTTTCTTAGACGTC
AGGTGGCACTTTTCGGGGAAATGTGCGCGGAACCCCTATTTGTTTATTTTTCTAAATA
CATTCAAATATGTATCCGCTCATGAGACAATAACCCTGATAAATGCTTCAATAATAT
TGAAAAAGGAAGAGTATGAGTATTCAACATTTCCGTGTCGCCCTTATTCCCTTTTTTG
CGGCATTTTGCCTTCCTGTTTTTGCTCACCCAGAAACGCTGGTGAAAGTAAAAGATG
CTGAAGATCAGTTGGGTGCACGAGTGGGTTACATCGAACTGGATCTCAACAGCGGT
AAGATCCTTGAGAGTTTTCGCCCCGAAGAACGTTTTCCAATGATGAGCACTTTTAAA
GTTCTGCTATGTGGCGCGGTATTATCCCGTATTGACGCCGGGCAAGAGCAACTCGGT
CGCCGCATACACTATTCTCAGAATGACTTGGTTGAGTACTCACCAGTCACAGAAAAG
CATCTTACGGATGGCATGACAGTAAGAGAATTATGCAGTGCTGCCATAACCATGAGT
GATAACACTGCGGCCAACTTACTTCTGACAACGATCGGAGGACCGAAGGAGCTAAC
CGCTTTTTTGCACAACATGGGGGATCATGTAACTCGCCTTGATCGTTGGGAACCGGA
GCTGAATGAAGCCATACCAAACGACGAGCGTGACACCACGATGCCTGTAGCAATGG

CAACAACGTTGCGCAAACTATTAACTGGCGAACTACTTACTCTAGCTTCCCGGCAAC
AATTAATAGACTGGATGGAGGCGGATAAAGTTGCAGGACCACTTCTGCGCTCGGCC
CTTCCGGCTGGCTGGTTTATTGCTGATAAATCTGGAGCCGGTGAGCGTGGGTCTCGC
GGTATCATTGCAGCACTGGGGCCAGATGGTAAGCCCTCCCGTATCGTAGTTATCTAC
ACGACGGGGAGTCAGGCAACTATGGATGAACGAAATAGACAGATCGCTGAGATAG
GTGCCTCACTGATTAAGCATTGGTAACTGTCAGACCAAGTTTACTCATATATACTTTA
GATTGATTTAAAACTTCATTTTTAATTTAAAAGGATCTAGGTGAAGATCCTTTTTGAT
AATCTCATGACCAAAATCCCTTAACGTGAGTTTTCGTTCCACTGAGCGTCAGACCCC
GTAGAAAAGATCAAAGGATCTTCTTGAGATCCTTTTTTTCTGCGCGTAATCTGCTGCT
TGCAAACAAAAAAACCACCGCTACCAGCGGTGGTTTGTTTGCCGGATCAAGAGCTA
CCAACTCTTTTTCCGAAGGTAACTGGCTTCAGCAGAGCGCAGATACCAAATACTGTC
CTTCTAGTGTAGCCGTAGTTAGGCCACCACTTCAAGAACTCTGTAGCACCGCCTACA
TACCTCGCTCTGCTAATCCTGTTACCAGTGGCTGCTGCCAGTGGCGATAAGTCGTGT
CTTACCGGGTTGGACTCAAGACGATAGTTACCGGATAAGGCGCAGCGGTCGGGCTG
AACGGGGGGTTCGTGCACACAGCCCAGCTTGGAGCGAACGACCTACACCGAACTGA
GATACCTACAGCGTGAGCTATGAGAAAGCGCCACGCTTCCCGAAGGGAGAAAGGCG
GACAGGTATCCGGTAAGCGGCAGGGTCGGAACAGGAGAGCGCACGAGGGAGCTTC
CAGGGGGAAACGCCTGGTATCTTTATAGTCCTGTCGGGTTTCGCCACCTCTGACTTG
AGCGTCGATTTTTGTGATGCTCGTCAGGGGGGCGGAGCCTATGGAAAAACGCCAGC
AACGCGGCCTTTTTACGGTTCCTGGCCTTTTGCTGGCCTTTTGCTCACATGTTCTTTCC
TGCGTTATCCCCTGATTCTGTGGATAACCGTATTACCGCCTTTGAGTGAGCTGATACC
GCTCGCCGCAGCCGAACGACCGAGCGCAGCGAGTCAGTGAGCGAGGAAGCGGAAG
AGCGCCCAATACGCAAACCGCCTCTCCCCGCGCGTTGGCCGATTCATTAATGCAG

### AAV3-Mutant; SEQ ID No. 151

AATTCCCATCATCAATAATATACCTTATTTAGGATTGAAGCCAATATGATAATGAGC
CGGTGGAGTTTGTGACGTGGCGCGGGGCGTGGGAACGGGGCGGGTGACGTAGTAGT
CTCTAGAGTCCTGTATTAGAGGTCACGTGAGTGTTTTGCGACTTTTGCGACACCATG
TGGTCACGCTGGGTATTTAAGCCCGAGTGAGCACGCAGGGTCTCCATTTTGAAGCGG
GAGGTTTGAACGCGCAGCCACCACGGGGGGTTTTACGAGATTGTGATTAAGGTCC
CCAGCGACCTTGACGGGCATCTGCCCGGCATTTCTGACAGCTTTGTGAACTGGGTGG
CCGAGAAGGAATGGGAGTTGCCGCCAGATTCTGACATGGATCTGAATCTGATTGAG
CAGGCACCCCTGACCGTGGCCGAGAAGCTGCAGCGCGACTTTCTGACGGAATGGCG
CCGTGTGAGTAAGGCCCCGGAGGCCCTTTTCTTTGTGCAATTTGAGAAGGGAGAGAG
CTACTTCCACATGCACGTGCTCGTGGAAACCACCGGGGTGAAATCCATGGTTTTGGG
ACGTTTCCTGAGTCAGATTCGCGAAAAACTGATTCAGAGAATTTACCGCGGGATCGA
GCCGACTTTGCCAAACTGGTTCGCGGTCACAAAGACCAGAAATGGCGCCGGAGGCG
GGAACAAGGTGGTGGATGAGTGCTACATCCCCAATTACTTGCTCCCCAAAACCCAG

CCTGAGCTCCAGTGGCGTGGACTAATATGGAACAGTATTTAAGCGCCTGTTTGAAT
CTCACGGAGCGTAAACGGTTGGTGGCGCAGCATCTGACGCACGTGTCGCAGACGCA
GGAGCAGAACAAAGAGAATCAGAATCCCAATTCTGATGCGCCGGTGATCAGATCAA
AAACTTCAGCCAGGTACATGGAGCTGGTCGGGTGGCTCGTGGACAAGGGGATTACC
TCGGAGAAGCAGTGGATCCAGGAGGACCAGGCCTCATACATCTCCTTCAATGCGGC
CTCCAACTCGCGGTCCCAAATCAAGGCTGCCTTGGACAATGCGGGAAAGATTATGA
GCCTGACTAAAACCGCCCCCGACTACCTGGTGGGCCAGCAGCCCGTGGAGGACATT
TCCAGCAATCGGATTTATAAAATTTTGGAACTAAACGGGTACGATCCCCAATATGCG
GCTTCCGTCTTTCTGGGATGGGCCACGAAAAAGTTCGGCAAGAGGAACACCATCTG
GCTGTTTGGGCCTGCAACTACCGGGAAGACCAACATCGCGGAGGCCATAGCCCACA
CTGTGCCCTTCTACGGGTGCGTAAACTGGACCAATGAGAACTTTCCCTTCAACGACT
GTGTCGACAAGATGGTGATCTGGTGGGAGGAGGGGAAGATGACCGCCAAGGTCGTG
GAGTCGGCCAAAGCCATTCTCGGAGGAAGCAAGGTGCGCGTGGACCAGAAATGCAA
GTCCTCGGCCCAGATAGACCCGACTCCCGTGATCGTCACCTCCAACACCAACATGTG
CGCCGTGATTGACGGGAACTCAACGACCTTCGAACACCAGCAGCCGTTGCAAGACC
GGATGTTCAAATTTGAACTCACCCGCCGTCTGGATCATGACTTTGGGAAGGTCACCA
AGCAGGAAGTCAAAGACTTTTTCCGGTGGGCAAAGGATCACGTGGTTGAGGTGGAG
CATGAATTCTACGTCAAAAAGGGTGGAGCCAAGAAAAGACCCGCCCCCAGTGACGC
AGATATAAGTGAGCCCAAACGGGTGCGCGAGTCAGTTGCGCAGCCATCGACGTCAG
ACGCGGAAGCTTCGATCAACTACGCAGACAGGTACCAAAACAAATGTTCTCGTCAC
GTGGGCATGAATCTGATGCTGTTTCCCTGCAGACAATGCGAGAGAATGAATCAGAA
TTCAAATATCTGCTTCACTCACGGACAGAAAGACTGTTTAGAGTGCTTTCCCGTGTC
AGAATCTCAACCCGTTTCTGTCGTCAAAAAGGCGTATCAGAAACTGTGCTACATTCA
TCATATCATGGGAAAGGTGCCAGACGCTTGCACTGCCTGCGATCTGGTCAATGTGGA
TTTGGATGACTGCATCTTTGAACAATAAATGATTTAAATCAGGTATGGCTGCTGACG
GTTATCTTCCAGATTGGCTCGAGGACAACCTTTCTGAAGGCATTCGTGAGTGGTGG
CTCTGAAACCTGGAGTCCCTCAACCCAAAGCGAACCAACAACACCAGGACAACCGT
CGGGGTCTTGTGCTTCCGGGTTACAAATACCTCGGACCCGGTAACGGACTCGACAAA
GGAGAGCCGGTCAACGAGGCGGACGCGGCAGCCCTCGAACACGACAAAGCTTACG
ACCAGCAGCTCAAGGCCGGTGACAACCCGTACCTCAAGTACAACCACGCCGACGCC
GAGTTTCAGGAGCGTCTTCAAGAAGATACGTCTTTTGGGGGCAACCTTGGCAGAGC

AGTCTTCCAGGCCAAAAAGAGGATCCTTGAGCCTCTTGGTCTGGTTGAGGAAGCAGC
TAGAACGGCTCCTGGAAAGAAGGGGGCTGTAGATCAGTCTCCTCAGGAACCGGACT
CATCATCTGGTGTTGGCAAATCGGGCAAACAGCCTGCCAGAAAAAGACTAAATTTC
GGTCAGACTGGAGACTCAGAGTCAGTCCCAGACCCTCAACCTCTCGGAGAACCACC
AGCAGCCCCCACAAGTTTGGGATCTAATACAATGGCTTCAGGCGGTGGCGCACCA
ATGGCAGACAATAACGAGGGTGCCGATGGAGTGGGTAATTCCTCAGGAAATTGGCA

TTGCGATTCCCAATGGCTGGGCGACAGAGTCATCACCACCAGCACCAGAACCTGGG
CCCTGCCCGCTTACAACAACCATCTCTA
T251A

CAAGCAAATCTCCAGCCAATCAGGAGCTTCAAACGACAACCACTACTTTGGCTACA
GCACCCCTTGGGGGTATTTTGACTTTAACAGATTCCACTGCCACTTCTCACCACGTGA
CTGGCAGCGACTCATTAACAACAACTGGGGATTCCGGCCCAAGAAACTCAGCTTCA
AGCTCTTCAACATCCAAGTTAGAGGGGTCACGCAGAACGATGGCACGACGACTATT
GCCAATAACCTTACCAGCACGGTTCAAGTGTTTACGGACTCGGAGTATCAGCTCCCG
TACGTGCTCGGGTCGGCGCACCAAGGCTGTCTCCCGCCGTTTCCAGCGGACGTCTTC
ATGGTCCCTCAGTATGGATACCTCACCCTGAACAACGGAAGTCAAGCGGTGGGACG
CTCATCCTTTTACTGCCTGGAGTACTTCCCTTCGCAGATGCTAAGGACTGGAAATAA
CTTCCAATTCAGCTATACCTTCGAGGATGTACCTTTTCACAGCAGCTACGCTCACAG
CCAGAGTTTGGATCGCTTGATGAATCCTCTTATTGATCAGTATCTGTACTACCTGAAC
AGAACGCAAGGAACAACCTCTGGAACAACCAACCAATCACGGCTGCTTTTTAGCCA
GGCTGGGCCTCAGTCTATGTCTTTGCAGGCCAGAAATTGGCTACCTGGGCCCTGCTA
CCGGCAACAGAGACTTGCAAAGACTGCTAACGACAACAACAACAGTAACTTTCCTT
GGACAGCGGCCAGCAAATATCATCTCAATGGCCGCGACTCGCTGGTGAATCCAGGA
CCAGCTATGGCCAGTCACAAGGACGATGAAGAAAATTTTTCCCTATGCACGGCAA
TCTAATATTTGGCAAAGAAGGGACAACGGCAAGTAACGCAGAATTAGATAATGTAA
TGATTACGGATGAAGAAGAGATTCGTACCACCAATCCTGTGGCAACAGAGCAGTAT
GGAACTGTGGCAAATAACTTGCAGAGCTCAAATACAGCTCCCACGACTGGAACTGT
CAATCATCAGGGGGCCTTACCTGGCATGGTGTGGCAAGATCGTGACGTGTACCTTCA
AGGACCTATCTGGGCAAAGATTCCTCACACGGATGGACACTTTCATCCTTCTCCTCT
GATGGGAGGCTTTGGACTGAAACATCCGCCTCCTCAAATCATGATCAAAAATACTCC
GGTACCGGCAAATCCTCCGACGACTTTCAGCCCGGCCAAGTTTGCTGCATTTATCAC
TCAGTACTCCACTGGACAGGTCAGCGTGGAAATTGAGTGGGAGCTACAGAAAGAAA
ACAGCAAACGTTGGAATCCAGAGATTCAGTACACTTCCAACTACAACAAGTCTGTTA
ATGTGGACTTTACTGTAGACACTAATGGTGTTTATAGTGAACCTCGCCCTATTGGAA
CCCGGTATCTCACACGAAACTTGTGATTGCTTGTTAATCAATAAACCGTTTAATTCGT
TTCAGTTGAACTTTGGTCTCTGCGTATTTCTTTCTTATCTAGTTTCCATGCTCTAGACT
ACTACGTCACCCGCCCCGTTCCCACGCCCCGCGCCACGTCACAAACTCCACCCCCTC
ATTATCATATTGGCTTCAATCCAAAATAAGGTATATTATTGATGATGCATCGCTGGC
GTAATAGCGAAGAGGCCCGCACCGATCGCCCTTCCCAACAGTTGCGCAGCCTGAAT
GGCGAATGGAATTCCAGACGATTGAGCGTCAAAATGTAGGTATTTCCATGAGCGTTT
TTCCTGTTGCAATGGCTGGCGGTAATATTGTTCTGGATATTACCAGCAAGGCCGATA
GTTTGAGTTCTTCTACTCAGGCAAGTGATGTTATTACTAATCAAAGAAGTATTGCGA
CAACGGTTAATTTGCGTGATGGACAGACTCTTTTACTCGGTGGCCTCACTGATTATA
AAAACACTTCTCAGGATTCTGGCGTACCGTTCCTGTCTAAAATCCCTTTAATCGGCCT
CCTGTTTAGCTCCCGCTCTGATTCTAACGAGGAAAGCACGTTATACGTGCTCGTCAA
AGCAACCATAGTACGCGCCCTGTAGCGGCGCATTAAGCGCGGCGGGTGTGGTGGTT

ACGCGCAGCGTGACCGCTACACTTGCCAGCGCCCTAGCGCCCGCTCCTTTCGCTTTC
TTCCCTTCCTTTCTCGCCACGTTCGCCGGCTTTCCCCGTCAAGCTCTAAATCGGGGGC
TCCCTTTAGGGTTCCGATTTAGTGCTTTACGGCACCTCGACCCCAAAAAACTTGATTA
GGGTGATGGTTCACGTAGTGGGCCATCGCCCTGATAGACGGTTTTTCGCCCTTTGAC
GTTGGAGTCCACGTTCTTTAATAGTGGACTCTTGTTCCAAACTGGAACAACACTCAA
CCCTATCTCGGTCTATTCTTTTGATTTATAAGGGATTTTGCCGATTTCGGCCTATTGG
TTAAAAAATGAGCTGATTTAACAAAAATTTAACGCGAATTTTAACAAAATATTAACG
TTTACAATTTAAATATTTGCTTATACAATCTTCCTGTTTTTGGGGCTTTTCTGATTATC
AACCGGGGTACATATGATTGACATGCTAGTTTTACGATTACCGTTCATCGATTCTCTT
GTTTGCTCCAGACTCTCAGGCAATGACCTGATAGCCTTTGTAGAGACCTCTCAAAAA
TAGCTACCCTCTCCGGCATGAATTTATCAGCTAGAACGGTTGAATATCATATTGATG
GTGATTTGACTGTCTCCGGCCTTTCTCACCCGTTTGAATCTTTACCTACACATTACTC
AGGCATTGCATTTAAAATATATGAGGGTTCTAAAAATTTTTATCCTTGCGTTGAAAT
AAAGGCTTCTCCCGCAAAAGTATTACAGGGTCATAATGTTTTTGGTACAACCGATTT
AGCTTTATGCTCTGAGGCTTTATTGCTTAATTTTGCTAATTCTTTGCCTTGCCTGTATG
ATTTATTGGATGTTGGAATTCCTGATGCGGTATTTTCTCCTTACGCATCTGTGCGGTA
TTTCACACCGCATATGGTGCACTCTCAGTACAATCTGCTCTGATGCCGCATAGTTAA
GCCAGCCCCGACACCCGCCAACACCCGCTGACGCGCCCTGACGGGCTTGTCTGCTCC
CGGCATCCGCTTACAGACAAGCTGTGACCGTCTCCGGGAGCTGCATGTGTCAGAGGT
TTTCACCGTCATCACCGAAACGCGCGAGACGAAAGGGCCTCGTGATACGCCTATTTT
TATAGGTTAATGTCATGATAATAATGGTTTCTTAGACGTCAGGTGGCACTTTTCGGG
GAAATGTGCGCGGAACCCCTATTTGTTTATTTTTCTAAATACATTCAAATATGTATCC
GCTCATGAGACAATAACCCTGATAAATGCTTCAATAATATTGAAAAAGGAAGAGTA
TGAGTATTCAACATTTCCGTGTCGCCCTTATTCCCTTTTTTGCGGCATTTTGCCTTCCT
GTTTTTGCTCACCCAGAAACGCTGGTGAAAGTAAAAGATGCTGAAGATCAGTTGGGT
GCACGAGTGGGTTACATCGAACTGGATCTCAACAGCGGTAAGATCCTTGAGAGTTTT
CGCCCCGAAGAACGTTTTCCAATGATGAGCACTTTTAAAGTTCTGCTATGTGGCGCG
GTATTATCCCGTATTGACGCCGGGCAAGAGCAACTCGGTCGCCGCATACACTATTCT
CAGAATGACTTGGTTGAGTACTCACCAGTCACAGAAAAGCATCTTACGGATGGCAT
GACAGTAAGAGAATTATGCAGTGCTGCCATAACCATGAGTGATAACACTGCGGCCA
ACTTACTTCTGACAACGATCGGAGGACCGAAGGAGCTAACCGCTTTTTTGCACAACA
TGGGGGATCATGTAACTCGCCTTGATCGTTGGGAACCGGAGCTGAATGAAGCCATA
CCAAACGACGAGCGTGACACCACGATGCCTGTAGCAATGGCAACAACGTTGCGCAA
ACTATTAACTGGCGAACTACTTACTCTAGCTTCCCGGCAACAATTAATAGACTGGAT
GGAGGCGGATAAAGTTGCAGGACCACTTCTGCGCTCGGCCCTTCCGGCTGGCTGGTT
TATTGCTGATAAATCTGGAGCCGGTGAGCGTGGGTCTCGCGGTATCATTGCAGCACT
GGGGCCAGATGGTAAGCCCTCCCGTATCGTAGTTATCTACACGACGGGGAGTCAGG
CAACTATGGATGAACGAAATAGACAGATCGCTGAGATAGGTGCCTCACTGATTAAG
CATTGGTAACTGTCAGACCAAGTTTACTCATATATACTTTAGATTGATTTAAAACTTC
ATTTTTAATTTAAAAGGATCTAGGTGAAGATCCTTTTTGATAATCTCATGACCAAAA
TCCCTTAACGTGAGTTTTCGTTCCACTGAGCGTCAGACCCCGTAGAAAAGATCAAAG

GATCTTCTTGAGATCCTTTTTTTCTGCGCGTAATCTGCTGCTTGCAAACAAAAAAACC
ACCGCTACCAGCGGTGGTTTGTTTGCCGGATCAAGAGCTACCAACTCTTTTTCCGAA
GGTAACTGGCTTCAGCAGAGCGCAGATACCAAATACTGTCCTTCTAGTGTAGCCGTA
GTTAGGCCACCACTTCAAGAACTCTGTAGCACCGCCTACATACCTCGCTCTGCTAAT
CCTGTTACCAGTGGCTGCTGCCAGTGGCGATAAGTCGTGTCTTACCGGGTTGGACTC
AAGACGATAGTTACCGGATAAGGCGCAGCGGTCGGGCTGAACGGGGGGTTCGTGCA
CACAGCCCAGCTTGGAGCGAACGACCTACACCGAACTGAGATACCTACAGCGTGAG
CTATGAGAAAGCGCCACGCTTCCCGAAGGGAGAAAGGCGGACAGGTATCCGGTAAG
CGGCAGGGTCGGAACAGGAGAGCGCACGAGGGAGCTTCCAGGGGGAAACGCCTGG
TATCTTTATAGTCCTGTCGGGTTTCGCCACCTCTGACTTGAGCGTCGATTTTTGTGAT
GCTCGTCAGGGGGGCGGAGCCTATGGAAAAACGCCAGCAACGCGGCCTTTTTACGG
TTCCTGGCCTTTTGCTGGCCTTTTGCTCACATGTTCTTTCCTGCGTTATCCCCTGATTC
TGTGGATAACCGTATTACCGCCTTTGAGTGAGCTGATACCGCTCGCCGCAGCCGAAC
GACCGAGCGCAGCGAGTCAGTGAGCGAGGAAGCGGAAGAGCGCCCAATACGCAAA
CCGCCTCTCCCCGCGCGTTGGCCGATTCATTAATGCAG

| **AAV3 RESIDUE** | **NUCLEOTIDE CHANGE** |
|---|---|
| AAV3-T251A | ACT→GCT |

### AAV4-Wild Type; SEQ ID No. 142

AATTCCCATCATCAATAATATACCTTATTTAGGATTGAAGCCAATATGATAATGAGC
CGGTGGAGTTTGTGACGTGGCGCGGGGCGTGGGAACGGGGCGGGTGACGTAGTAGT
CTCTAGAGTCCTGTATTAGAGGTCACGTGAGTGTTTTGCGACTTTTGCGACACCATG
TGGTCACGCTGGGTATTTAAGCCCGAGTGAGCACGCAGGGTCTCCATTTTGAAGCGG
GAGGTTTGAACGCGCAGCCACCACGGGGGGTTTTACGAGATTGTGATTAAGGTCC
CCAGCGACCTTGACGGGCATCTGCCCGGCATTTCTGACAGCTTTGTGAACTGGGTGG
CCGAGAAGGAATGGGAGTTGCCGCCAGATTCTGACATGGATCTGAATCTGATTGAG
CAGGCACCCCTGACCGTGGCCGAGAAGCTGCAGCGCGACTTTCTGACGGAATGGCG
CCGTGTGAGTAAGGCCCCGGAGGCCCTTTTCTTTGTGCAATTTGAGAAGGGAGAGAG
CTACTTCCACATGCACGTGCTCGTGGAAACCACCGGGGTGAAATCCATGGTTTTGGG
ACGTTTCCTGAGTCAGATTCGCGAAAAACTGATTCAGAGAATTTACCGCGGGATCGA
GCCGACTTTGCCAAACTGGTTCGCGGTCACAAAGACCAGAAATGGCGCCGGAGGCG
GGAACAAGGTGGTGGATGAGTGCTACATCCCCAATTACTTGCTCCCCAAAACCCAG
CCTGAGCTCCAGTGGGCGTGGACTAATATGGAACAGTATTTAAGCGCCTGTTTGAAT
CTCACGGAGCGTAAACGGTTGGTGGCGCAGCATCTGACGCACGTGTCGCAGACGCA

GGAGCAGAACAAAGAGAATCAGAATCCCAATTCTGATGCGCCGGTGATCAGATCAA
AAACTTCAGCCAGGTACATGGAGCTGGTCGGTGGCTCGTGGACAAGGGATTACC
TCGGAGAAGCAGTGGATCCAGGAGGACCAGGCCTCATACATCTCCTTCAATGCGGC
CTCCAACTCGCGGTCCCAAATCAAGGCTGCCTTGGACAATGCGGAAAGATTATGA
GCCTGACTAAAACCGCCCCCGACTACCTGGTGGCCAGCAGCCCGTGGAGGACATT
TCCAGCAATCGGATTTATAAAATTTTGGAACTAAACGGGTACGATCCCAATATGCG
GCTTCCGTCTTTCTGGGATGGCCACGAAAAGTTCGGCAAGAGGAACACCATCTG
GCTGTTTGGGCCTGCAACTACCGGGAAGACCAACATCGCGGAGGCCATAGCCCACA
CTGTGCCCTTCTACGGGTGCGTAAACTGGACCAATGAGAACTTTCCCTTCAACGACT
GTGTCGACAAGATGGTGATCTGGTGGAGGAGGGGAAGATGACCGCCAAGGTCGTG
GAGTCGGCCAAAGCCATTCTCGGAGGAAGCAAGGTGCGCGTGGACCAGAAATGCAA
GTCCTCGGCCCAGATAGACCCGACTCCCGTGATCGTCACCTCCAACACCAACATGTG
CGCCGTGATTGACGGGAACTCAACGACCTTCGAACACCAGCAGCCGTTGCAAGACC
GGATGTTCAAATTTGAACTCACCCGCCGTCTGGATCATGACTTTGGGAAGGTCACCA
AGCAGGAAGTCAAAGACTTTTTCCGGTGGGCAAAGGATCACGTGGTTGAGGTGGAG
CATGAATTCTACGTCAAAAAGGGTGGAGCCAAGAAAAGACCCGCCCCCAGTGACGC
AGATATAAGTGAGCCCAAACGGGTGCGCGAGTCAGTTGCGCAGCCATCGACGTCAG
ACGCGGAAGCTTCGATCAACTACGCAGACAGGTACCAAAACAAATGTTCTCGTCAC
GTGGGCATGAATCTGATGCTGTTTCCCTGCAGACAATGCGAGAGAATGAATCAGAA
TTCAAATATCTGCTTCACTCACGGACAGAAAGACTGTTTAGAGTGCTTTCCCGTGTC
AGAATCTCAACCCGTTTCTGTCGTCAAAAAGGCGTATCAGAAACTGTGCTACATTCA
TCATATCATGGGAAAGGTGCCAGACGCTTGCACTGCCTGCGATCTGGTCAATGTGGA
TTTGGATGACTGCATCTTTGAACAATAAATGATTTAAATCAGGTATGACTGACGGTT
ACCTTCCAGATTGGCTAGAGGACAACCTCTCTGAAGGCGTTCGAGAGTGGTGGGCG
CTGCAACCTGGAGCCCCTAAACCCAAGGCAAATCAACAACATCAGGACAACGCTCG
GGGTCTTGTGCTTCCGGGTTACAAATACCTCGGACCCGGCAACGGACTCGACAAGG
GGGAACCCGTCAACGCAGCGGACGCGGCAGCCCTCGAGCACGACAAGGCCTACGAC
CAGCAGCTCAAGGCCGGTGACAACCCCTACCTCAAGTACAACCACGCCGACGCGGA
GTTCCAGCAGCGGCTTCAGGGCGACACATCGTTTGGGGGCAACCTCGGCAGAGCAG
TCTTCCAGGCCAAAAAGAGGGTTCTTGAACCTCTTGGTCTGGTTGAGCAAGCGGGTG
AGACGGCTCCTGGAAAGAAGAGACCGTTGATTGAATCCCCCCAGCAGCCCGACTCC
TCCACGGGTATCGGCAAAAAAGGCAAGCAGCCGGCTAAAAAGAAGCTCGTTTTCGA
AGACGAAACTGGAGCAGGCGACGGACCCCCTGAGGGATCAACTTCCGGAGCCATGT
CTGATGACAGTGAGATGCGTGCAGCAGCTGGCGGAGCTGCAGTCGAGGGCGGACAA
GGTGCCGATGGAGTGGGTAATGCCTCGGGTGATTGGCATTGCGATTCCACCTGGTCT
GAGGGCCACGTCACGACCACCAGCACCAGAACCTGGGTCTTGCCCACCTACAACAA
CCACCTCTACAAGCGACTCGGAGAGAGCCTGCAGTCCAACACCTACAACGGATTCT
CCACCCCCTGGGGATACTTTGACTTCAACCGCTTCCACTGCCACTTCTCACCACGTG
ACTGGCAGCGACTCATCAACAACAACTGGGGCATGCGACCCAAAGCCATGCGGGTC
AAAATCTTCAACATCCAGGTCAAGGAGGTCACGACGTCGAACGGCGAGACAACGGT
GGCTAATAACCTTACCAGCACGGTTCAGATCTTTGCGGACTCGTCGTACGAACTGCC

GTACGTGATGGATGCGGGTCAAGAGGCAGCCTGCCTCCTTTTCCCAACGACGTCTT
TATGGTGCCCCAGTACGGCTACTGTGGACTGGTGACCGGCAACACTTCGCAGCAAC
AGACTGACAGAAATGCCTTCTACTGCCTGGAGTACTTTCCTTCGCAGATGCTGCGGA
CTGGCAACAACTTTGAAATTACGTACAGTTTTGAGAAGGTGCCTTTCCACTCGATGT
ACGCGCACAGCCAGAGCCTGGACCGGCTGATGAACCCTCTCATCGACCAGTACCTG
TGGGGACTGCAATCGACCACCACCGGAACCACCCTGAATGCCGGGACTGCCACCAC
CAACTTTACCAAGCTGCGGCCTACCAACTTTTCCAACTTTAAAAAGAACTGGCTGCC
CGGGCCTTCAATCAAGCAGCAGGGCTTCTCAAAGACTGCCAATCAAAACTACAAGA
TCCCTGCCACCGGGTCAGACAGTCTCATCAAATACGAGACGCACAGCACTCTGGAC
GGAAGATGGAGTGCCCTGACCCCCGGACCTCCAATGGCCACGGCTGGACCTGCGGA
CAGCAAGTTCAGCAACAGCCAGCTCATCTTTGCGGGGCCTAAACAGAACGGCAACA
CGGCCACCGTACCCGGGACTCTGATCTTCACCTCTGAGGAGGAGCTGGCAGCCACC
AACGCCACCGATACGGACATGTGGGGCAACCTACCTGGCGGTGACCAGAGCAACAG
CAACCTGCCGACCGTGGACAGACTGACAGCCTTGGGAGCCGTGCCTGGAATGGTCT
GGCAAAACAGAGACATTTACTACCAGGGTCCCATTTGGGCCAAGATTCCTCATACCG
ATGGACACTTTCACCCCTCACCGCTGATTGGTGGGTTTGGGCTGAAACACCCGCCTC
CTCAAATTTTTATCAAGAACACCCCGGTACCTGCGAATCCTGCAACGACCTTCAGCT
CTACTCCGGTAAACTCCTTCATTACTCAGTACAGCACTGGCCAGGTGTCGGTGCAGA
TTGACTGGGAGATCCAGAAGGAGCGGTCCAAACGCTGGAACCCCGAGGTCCAGTTT
ACCTCCAACTACGGACAGCAAAACTCTCTGTTGTGGGCTCCCGATGCGGCTGGAA
ATACACTGAGCCTAGGGCTATCGGTACCCGCTACCTCACCCACCACCTGTAATTGCT
TGTTAATCAATAAACCGTTTAATTCGTTTCAGTTGAACTTTGGTCTCTGCGTATTTCT
TTCTTATCTAGTTTCCATGCTCTAGACTACTACGTCACCCGCCCCGTTCCCACGCCCC
GCGCCACGTCACAAACTCCACCCCCTCATTATCATATTGGCTTCAATCCAAAATAAG
GTATATTATTGATGATGCATCGCTGGCGTAATAGCGAAGAGGCCCGCACCGATCGCC
CTTCCCAACAGTTGCGCAGCCTGAATGGCGAATGGAATTCCAGACGATTGAGCGTCA
AAATGTAGGTATTTCCATGAGCGTTTTTCCTGTTGCAATGGCTGGCGGTAATATTGTT
CTGGATATTACCAGCAAGGCCGATAGTTTGAGTTCTTCTACTCAGGCAAGTGATGTT
ATTACTAATCAAAGAAGTATTGCGACAACGGTTAATTTGCGTGATGGACAGACTCTT
TTACTCGGTGGCCTCACTGATTATAAAAACACTTCTCAGGATTCTGGCGTACCGTTCC
TGTCTAAAATCCCTTTAATCGGCCTCCTGTTTAGCTCCCGCTCTGATTCTAACGAGGA
AAGCACGTTATACGTGCTCGTCAAAGCAACCATAGTACGCGCCCTGTAGCGGCGCA
TTAAGCGCGGCGGGTGTGGTGGTTACGCGCAGCGTGACCGCTACACTTGCCAGCGC
CCTAGCGCCCGCTCCTTTCGCTTTCTTCCCTTCCTTTCTCGCCACGTTCGCCGGCTTTC
CCCGTCAAGCTCTAAATCGGGGGCTCCCTTTAGGGTTCCGATTTAGTGCTTTACGGC
ACCTCGACCCCAAAAAACTTGATTAGGGTGATGGTTCACGTAGTGGGCCATCGCCCT
GATAGACGGTTTTTCGCCCTTTGACGTTGGAGTCCACGTTCTTTAATAGTGGACTCTT
GTTCCAAACTGGAACAACACTCAACCCTATCTCGGTCTATTCTTTTGATTTATAAGGG
ATTTTGCCGATTTCGGCCTATTGGTTAAAAAATGAGCTGATTTAACAAAAATTTAAC
GCGAATTTTAACAAAATATTAACGTTTACAATTTAAATATTTGCTTATACAATCTTCC
TGTTTTTGGGGCTTTTCTGATTATCAACCGGGGTACATATGATTGACATGCTAGTTTT

ACGATTACCGTTCATCGATTCTCTTGTTTGCTCCAGACTCTCAGGCAATGACCTGATA
GCCTTTGTAGAGACCTCTCAAAAATAGCTACCCTCTCCGGCATGAATTTATCAGCTA
GAACGGTTGAATATCATATTGATGGTGATTTGACTGTCTCCGGCCTTTCTCACCCGTT
TGAATCTTTACCTACACATTACTCAGGCATTGCATTTAAAATATATGAGGGTTCTAA
AAATTTTTATCCTTGCGTTGAAATAAAGGCTTCTCCCGCAAAAGTATTACAGGGTCA
TAATGTTTTTGGTACAACCGATTTAGCTTTATGCTCTGAGGCTTTATTGCTTAATTTT
GCTAATTCTTTGCCTTGCCTGTATGATTTATTGGATGTTGGAATTCCTGATGCGGTAT
TTTCTCCTTACGCATCTGTGCGGTATTTCACACCGCATATGGTGCACTCTCAGTACAA
TCTGCTCTGATGCCGCATAGTTAAGCCAGCCCCGACACCCGCCAACACCCGCTGACG
CGCCCTGACGGGCTTGTCTGCTCCCGGCATCCGCTTACAGACAAGCTGTGACCGTCT
CCGGGAGCTGCATGTGTCAGAGGTTTTCACCGTCATCACCGAAACGCGCGAGACGA
AAGGGCCTCGTGATACGCCTATTTTTATAGGTTAATGTCATGATAATAATGGTTTCTT
AGACGTCAGGTGGCACTTTTCGGGGAAATGTGCGCGGAACCCCTATTTGTTTATTTT
TCTAAATACATTCAAATATGTATCCGCTCATGAGACAATAACCCTGATAAATGCTTC
AATAATATTGAAAAAGGAAGAGTATGAGTATTCAACATTTCCGTGTCGCCCTTATTC
CCTTTTTTGCGGCATTTTGCCTTCCTGTTTTTGCTCACCCAGAAACGCTGGTGAAAGT
AAAAGATGCTGAAGATCAGTTGGGTGCACGAGTGGGTTACATCGAACTGGATCTCA
ACAGCGGTAAGATCCTTGAGAGTTTTCGCCCCGAAGAACGTTTTCCAATGATGAGCA
CTTTTAAAGTTCTGCTATGTGGCGCGGTATTATCCCGTATTGACGCCGGGCAAGAGC
AACTCGGTCGCCGCATACACTATTCTCAGAATGACTTGGTTGAGTACTCACCAGTCA
CAGAAAAGCATCTTACGGATGGCATGACAGTAAGAGAATTATGCAGTGCTGCCATA
ACCATGAGTGATAACACTGCGGCCAACTTACTTCTGACAACGATCGGAGGACCGAA
GGAGCTAACCGCTTTTTTGCACAACATGGGGGATCATGTAACTCGCCTTGATCGTTG
GGAACCGGAGCTGAATGAAGCCATACCAAACGACGAGCGTGACACCACGATGCCTG
TAGCAATGGCAACAACGTTGCGCAAACTATTAACTGGCGAACTACTTACTCTAGCTT
CCCGGCAACAATTAATAGACTGGATGGAGGCGGATAAAGTTGCAGGACCACTTCTG
CGCTCGGCCCTTCCGGCTGGCTGGTTTATTGCTGATAAATCTGGAGCCGGTGAGCGT
GGGTCTCGCGGTATCATTGCAGCACTGGGGCCAGATGGTAAGCCCTCCCGTATCGTA
GTTATCTACACGACGGGGAGTCAGGCAACTATGGATGAACGAAATAGACAGATCGC
TGAGATAGGTGCCTCACTGATTAAGCATTGGTAACTGTCAGACCAAGTTTACTCATA
TATACTTTAGATTGATTTAAAACTTCATTTTTAATTTAAAAGGATCTAGGTGAAGATC
CTTTTTGATAATCTCATGACCAAAATCCCTTAACGTGAGTTTTCGTTCCACTGAGCGT
CAGACCCCGTAGAAAAGATCAAAGGATCTTCTTGAGATCCTTTTTTTCTGCGCGTAA
TCTGCTGCTTGCAAACAAAAAAACCACCGCTACCAGCGGTGGTTTGTTTGCCGGATC
AAGAGCTACCAACTCTTTTTCCGAAGGTAACTGGCTTCAGCAGAGCGCAGATACCAA
ATACTGTCCTTCTAGTGTAGCCGTAGTTAGGCCACCACTTCAAGAACTCTGTAGCAC
CGCCTACATACCTCGCTCTGCTAATCCTGTTACCAGTGGCTGCTGCCAGTGGCGATA
AGTCGTGTCTTACCGGGTTGGACTCAAGACGATAGTTACCGGATAAGGCGCAGCGG
TCGGGCTGAACGGGGGGTTCGTGCACACAGCCCAGCTTGGAGCGAACGACCTACAC
CGAACTGAGATACCTACAGCGTGAGCTATGAGAAAGCGCCACGCTTCCCGAAGGGA
GAAAGGCGGACAGGTATCCGGTAAGCGGCAGGGTCGGAACAGGAGAGCGCACGAG

GGAGCTTCCAGGGGGAAACGCCTGGTATCTTTATAGTCCTGTCGGGTTTCGCCACCT
CTGACTTGAGCGTCGATTTTTGTGATGCTCGTCAGGGGGGCGGAGCCTATGGAAAA
CGCCAGCAACGCGGCCTTTTTACGGTTCCTGGCCTTTTGCTGGCCTTTTGCTCACATG
TTCTTTCCTGCGTTATCCCCTGATTCTGTGGATAACCGTATTACCGCCTTTGAGTGAG
CTGATACCGCTCGCCGCAGCCGAACGACCGAGCGCAGCGAGTCAGTGAGCGAGGAA
GCGGAAGAGCGCCCAATACGCAAACCGCCTCTCCCCGCGCGTTGGCCGATTCATTA
ATGCAG

### AAV4-Mutant ; SEQ ID No. 152

AATTCCCATCATCAATAATATACCTTATTTAGGATTGAAGCCAATATGATAATGAGC
CGGTGGAGTTTGTGACGTGGCGCGGGGCGTGGGAACGGGGCGGGTGACGTAGTAGT
CTCTAGAGTCCTGTATTAGAGGTCACGTGAGTGTTTTGCGACTTTTTGCGACACCATG
TGGTCACGCTGGGTATTTAAGCCCGAGTGAGCACGCAGGGTCTCCATTTTGAAGCGG
GAGGTTTGAACGCGCAGCCACCACGGGGGGTTTTACGAGATTGTGATTAAGGTCC
CCAGCGACCTTGACGGGCATCTGCCCGGCATTTCTGACAGCTTTGTGAACTGGGTGG
CCGAGAAGGAATGGGAGTTGCCGCCAGATTCTGACATGGATCTGAATCTGATTGAG
CAGGCACCCCTGACCGTGGCCGAGAAGCTGCAGCGCGACTTTCTGACGGAATGGCG
CCGTGTGAGTAAGGCCCCGGAGGCCCTTTTCTTTGTGCAATTTGAGAAGGGAGAGAG
CTACTTCCACATGCACGTGCTCGTGGAAACCACCGGGGTGAAATCCATGGTTTTGGG
ACGTTTCCTGAGTCAGATTCGCGAAAAACTGATTCAGAGAATTTACCGCGGGATCGA
GCCGACTTTGCCAAACTGGTTCGCGGTCACAAAGACCAGAAATGGCGCCGGAGGCG
GGAACAAGGTGGTGGATGAGTGCTACATCCCCAATTACTTGCTCCCCAAAACCCAG
CCTGAGCTCCAGTGGGCGTGGACTAATATGGAACAGTATTTAAGCGCCTGTTTGAAT
CTCACGGAGCGTAAACGGTTGGTGGCGCAGCATCTGACGCACGTGTCGCAGACGCA
GGAGCAGAACAAAGAGAATCAGAATCCCAATTCTGATGCGCCGGTGATCAGATCAA
AAACTTCAGCCAGGTACATGGAGCTGGTCGGGTGGCTCGTGGACAAGGGGATTACC
TCGGAGAAGCAGTGGATCCAGGAGGACCAGGCCTCATACATCTCCTTCAATGCGGC
CTCCAACTCGCGGTCCCAAATCAAGGCTGCCTTGGACAATGCGGGAAAGATTATGA
GCCTGACTAAAACCGCCCCCGACTACCTGGTGGGCCAGCAGCCCGTGGAGGACATT
TCCAGCAATCGGATTTATAAAATTTTGGAACTAAACGGGTACGATCCCCAATATGCG
GCTTCCGTCTTTCTGGGATGGGCCACGAAAAAGTTCGGCAAGAGGAACACCATCTG
GCTGTTTGGGCCTGCAACTACCGGGAAGACCAACATCGCGGAGGCCATAGCCCACA
CTGTGCCCTTCTACGGGTGCGTAAACTGGACCAATGAGAACTTTCCCTTCAACGACT
GTGTCGACAAGATGGTGATCTGGTGGGAGGAGGGGAAGATGACCGCCAAGGTCGTG
GAGTCGGCCAAAGCCATTCTCGGAGGAAGCAAGGTGCGCGTGGACCAGAAATGCAA
GTCCTCGGCCCAGATAGACCCGACTCCCGTGATCGTCACCTCCAACACCAACATGTG
CGCCGTGATTGACGGGAACTCAACGACCTTCGAACACCAGCAGCCGTTGCAAGACC
GGATGTTCAAATTTGAACTCACCCGCCGTCTGGATCATGACTTTGGGAAGGTCACCA
AGCAGGAAGTCAAAGACTTTTTCCGGTGGGCAAAGGATCACGTGGTTGAGGTGGAG

CATGAATTCTACGTCAAAAAGGGTGGAGCCAAGAAAAGACCCGCCCCCAGTGACGC
AGATATAAGTGAGCCCAAACGGGTGCGCGAGTCAGTTGCGCAGCCATCGACGTCAG
ACGCGGAAGCTTCGATCAACTACGCAGACAGGTACCAAAACAAATGTTCTCGTCAC
GTGGGCATGAATCTGATGCTGTTTCCCTGCAGACAATGCGAGAGAATGAATCAGAA
TTCAAATATCTGCTTCACTCACGGACAGAAAGACTGTTTAGAGTGCTTTCCCGTGTC
AGAATCTCAACCCGTTTCTGTCGTCAAAAAGGCGTATCAGAAACTGTGCTACATTCA
TCATATCATGGGAAAGGTGCCAGACGCTTGCACTGCCTGCGATCTGGTCAATGTGGA
TTTGGATGACTGCATCTTTGAACAATAAATGATTTAAATCAGGT
ATGACTGACGGTTACCTTCCAGATTGGCTAGAGGACAACCTCTCTGAAGGCGTTCGA
GAGTGGTGGGCGCTGCAACCTGGAGCCCCTAAACCCAAGGCAAATCAACAACATCA
GGACAACGCTCGGGGTCTTGTGCTTCCGGGTTACAAATACCTCGGACCCGGCAACG
GACTCGACAAGGGGGAACCCGTCAACGCAGCGGACGCGGCAGCCCTCGAGCACGA
CAAGGCCTACGACCAGCAGCTCAAGGCCGGTGACAACCCCTACCTCAAGTACAACC
ACGCCGACGCGGAGTTCCAGCAGCGGCTTCAGGGCGACACATCGTTTGGGGGCAAC
CTCGGCAGAGCAGTCTTCCAGGCCAAAAAGAGGGTTCTTGAACCTCTTGGTCTGGTT
GAGCAAGCGGGTGAGACGGCTCCTGGAAAGAGGAGACCGTTGATTGAATCCCCCCA
GCAGCCCGACTCCTCCACGGGTATCGGCAAAAAAGGCAAGCAGCCGGCTAAAAAGA
AGCTCGTTTTCGAAGACGAAACTGGAGCAGGCGACGGACCCCCTGAGGGATCAAC
TTCCGGAGCCATGTCTGATGACAGTGAGATGCGTGCAGCAGCTGGCGGAGCTGCAG
TCGAGGGCGGACAAGGTGCCGATGGAGTGGGTAATGCCTCGGGTGATTGGCATTGC
GATTCCACCTGGTCTGAGGGCCACGTCA

T245
CGACCACCAGCACCAGAACCTGGGTCTTGCCCGCCTACAACAACCACCTCTACAAG
CGACTCGGAGAGAGCCTGCAGTCCAACACCTACAACGGATTCTCCACCCCCTGGGG
ATACTTTGACTTCAACCGCTTCCACTGCCACTTCTCACCACGTGACTGGCAGCGACT
CATCAACAACAACTGGGGCATGCGACCCAAAGCCATGCGGGTCAAAATCTTCAACA
TCCAGGTCAAGGAGGTCACGACGTCGAACGGCGAGACAACGGTGGCTAATAACCT
TACCAGCACGGTTCAGATCTTTGCGGACTCGTCGTACGAACTGCCGTACGTGATGGA
TGCGGGTCAAGAGGGCAGCCTGCCTCCTTTTCCCAACGACGTCTTTATGGTGCCCCA
GTACGGCTACTGTGGACTGGTGACCGGCAACACTTCGCAGCAACAGACTGACAGAA
ATGCCTTCTACTGCCTGGAGTACTTTCCTTCGCAGATGCTGCGGACTGGCAACAACT
TTGAAATTACGTACAGTTTTGAGAAGGTGCCTTTCCACTCGATGTACGCGCACAGCC
AGAGCCTGGACCGGCTGATGAACCCTCTCATCGACCAGTACCTGTGGGGACTGCAA
TCGACCACCACCGGAACCACCCTGAATGCCGGGACTGCCACCACCAACTTTACCAA
GCTGCGGCCTACCAACTTTTCCAACTTTAAAAAGAACTGGCTGCCCGGGCCTTCAAT
CAAGCAGCAGGGCTTCGCAAAGACTGCCAATCAAAACTACAAGATCCCTGCCACCG
GGTCAGACAGTCTCATCAAATACGAGACGCACAGCACTCTGGACGGAAGATGGAGT
GCCCTGACCCCCGGACCTCCAATGGCCACGGCTGGACCTGCGGACAGCAAGTTCAG
CAACAGCCAGCTCATCTTTGCGGGGCCTAAACAGAACGGCAACACGGCCACCGTAC
CCGGGACTCTGATCTTCACCTCTGAGGAGGAGCTGGCAGCCACCAACGCCACCGAT

ACGGACATGTGGGGCAACCTACCTGGCGGTGACCAGAGCAACAGCAACCTGCCGAC
CGTGGACAGACTGACAGCCTTGGGAGCCGTGCCTGGAATGGTCTGGCAAAACAGAG
ACATTTACTACCAGGGTCCCATTTGGCCAAGATTCCTCATACCGATGGACACTTTC
ACCCCTCACCGCTGATTGGTGGGTTTGGCTGAAACACCCGCCTCCTCAAATTTTTAT
CAAGAACACCCCGGTACCTGCGAATCCTGCAACGACCTTCAGCTCTACTCCGGTAAA
CGCCTTCATTACTCAGTACAGCACTGGCCAGGTGTCGGTGCAGATTGACTGGGAGAT
CCAGAAGGAGCGGTCCAAACGCTGGAACCCCGAGGTCCAGTTTACCTCCAACTACG
GACAGCAAAACTCTCTGTTGTGGGCTCCCGATGCGGCTGGGAAATACACTGAGCCTA
GGGCTATCGGTACCCGCTACCTCACCCACCACCTGTAATTGCTTGTTAATCAATAAA
CCGTTTAATTCGTTTCAGTTGAACTTTGGTCTCTGCGTATTTCTTTCTTATCTAGTTTC
CATGCTCTAGACTACTACGTCACCCGCCCCGTTCCCACGCCCCGCGCCACGTCACAA
ACTCCACCCCCTCATTATCATATTGGCTTCAATCCAAAATAAGGTATATTATTGATGA
TGCATCGCTGGCGTAATAGCGAAGAGGCCCGCACCGATCGCCCTTCCCAACAGTTGC
GCAGCCTGAATGGCGAATGGAATTCCAGACGATTGAGCGTCAAAATGTAGGTATTT
CCATGAGCGTTTTTCCTGTTGCAATGGCTGGCGGTAATATTGTTCTGGATATTACCAG
CAAGGCCGATAGTTTGAGTTCTTCTACTCAGGCAAGTGATGTTATTACTAATCAAAG
AAGTATTGCGACAACGGTTAATTTGCGTGATGGACAGACTCTTTTACTCGGTGGCCT
CACTGATTATAAAAACACTTCTCAGGATTCTGGCGTACCGTTCCTGTCTAAAATCCCT
TTAATCGGCCTCCTGTTTAGCTCCCGCTCTGATTCTAACGAGGAAAGCACGTTATAC
GTGCTCGTCAAAGCAACCATAGTACGCGCCCTGTAGCGGCGCATTAAGCGCGGCGG
GTGTGGTGGTTACGCGCAGCGTGACCGCTACACTTGCCAGCGCCCTAGCGCCCGCTC
CTTTCGCTTTCTTCCCTTCCTTTCTCGCCACGTTCGCCGGCTTTCCCCGTCAAGCTCTA
AATCGGGGGCTCCCTTTAGGGTTCCGATTTAGTGCTTTACGGCACCTCGACCCCAAA
AAACTTGATTAGGGTGATGGTTCACGTAGTGGGCCATCGCCCTGATAGACGGTTTTT
CGCCCTTTGACGTTGGAGTCCACGTTCTTTAATAGTGGACTCTTGTTCCAAACTGGAA
CAACACTCAACCCTATCTCGGTCTATTCTTTTGATTTATAAGGGATTTTGCCGATTTC
GGCCTATTGGTTAAAAAATGAGCTGATTTAACAAAAATTTAACGCGAATTTTAACAA
AATATTAACGTTTACAATTTAAATATTTGCTTATACAATCTTCCTGTTTTTGGGGCTT
TTCTGATTATCAACCGGGGTACATATGATTGACATGCTAGTTTTACGATTACCGTTCA
TCGATTCTCTTGTTTGCTCCAGACTCTCAGGCAATGACCTGATAGCCTTTGTAGAGAC
CTCTCAAAAATAGCTACCCTCTCCGGCATGAATTTATCAGCTAGAACGGTTGAATAT
CATATTGATGGTGATTTGACTGTCTCCGGCCTTTCTCACCCGTTTGAATCTTTACCTA
CACATTACTCAGGCATTGCATTTAAAATATATGAGGGTTCTAAAAATTTTTATCCTTG
CGTTGAAATAAAGGCTTCTCCCGCAAAAGTATTACAGGGTCATAATGTTTTTGGTAC
AACCGATTTAGCTTTATGCTCTGAGGCTTTATTGCTTAATTTTGCTAATTCTTTGCCTT
GCCTGTATGATTTATTGGATGTTGGAATTCCTGATGCGGTATTTTCTCCTTACGCATC
TGTGCGGTATTTCACACCGCATATGGTGCACTCTCAGTACAATCTGCTCTGATGCCG
CATAGTTAAGCCAGCCCCGACACCCGCCAACACCCGCTGACGCGCCCTGACGGGCT
TGTCTGCTCCCGGCATCCGCTTACAGACAAGCTGTGACCGTCTCCGGGAGCTGCATG
TGTCAGAGGTTTTCACCGTCATCACCGAAACGCGCGAGACGAAAGGGCCTCGTGAT
ACGCCTATTTTTATAGGTTAATGTCATGATAATAATGGTTTCTTAGACGTCAGGTGGC

ACTTTTCGGGGAAATGTGCGCGGAACCCCTATTTGTTTATTTTTCTAAATACATTCAA
ATATGTATCCGCTCATGAGACAATAACCCTGATAAATGCTTCAATAATATTGAAAAA
GGAAGAGTATGAGTATTCAACATTTCCGTGTCGCCCTTATTCCCTTTTTTGCGGCATT
TTGCCTTCCTGTTTTTGCTCACCCAGAAACGCTGGTGAAAGTAAAAGATGCTGAAGA
TCAGTTGGGTGCACGAGTGGGTTACATCGAACTGGATCTCAACAGCGGTAAGATCCT
TGAGAGTTTTCGCCCCGAAGAACGTTTTCCAATGATGAGCACTTTTAAAGTTCTGCT
ATGTGGCGCGGTATTATCCCGTATTGACGCCGGGCAAGAGCAACTCGGTCGCCGCAT
ACACTATTCTCAGAATGACTTGGTTGAGTACTCACCAGTCACAGAAAAGCATCTTAC
GGATGGCATGACAGTAAGAGAATTATGCAGTGCTGCCATAACCATGAGTGATAACA
CTGCGGCCAACTTACTTCTGACAACGATCGGAGGACCGAAGGAGCTAACCGCTTTTT
TGCACAACATGGGGGATCATGTAACTCGCCTTGATCGTTGGGAACCGGAGCTGAAT
GAAGCCATACCAAACGACGAGCGTGACACCACGATGCCTGTAGCAATGGCAACAAC
GTTGCGCAAACTATTAACTGGCGAACTACTTACTCTAGCTTCCCGGCAACAATTAAT
AGACTGGATGGAGGCGGATAAAGTTGCAGGACCACTTCTGCGCTCGGCCCTTCCGG
CTGGCTGGTTTATTGCTGATAAATCTGGAGCCGGTGAGCGTGGGTCTCGCGGTATCA
TTGCAGCACTGGGGCCAGATGGTAAGCCCTCCCGTATCGTAGTTATCTACACGACGG
GGAGTCAGGCAACTATGGATGAACGAAATAGACAGATCGCTGAGATAGGTGCCTCA
CTGATTAAGCATTGGTAACTGTCAGACCAAGTTTACTCATATATACTTTAGATTGATT
TAAAACTTCATTTTTAATTTAAAAGGATCTAGGTGAAGATCCTTTTTGATAATCTCAT
GACCAAAATCCCTTAACGTGAGTTTTCGTTCCACTGAGCGTCAGACCCCGTAGAAAA
GATCAAAGGATCTTCTTGAGATCCTTTTTTTCTGCGCGTAATCTGCTGCTTGCAAACA
AAAAAACCACCGCTACCAGCGGTGGTTTGTTTGCCGGATCAAGAGCTACCAACTCTT
TTTCCGAAGGTAACTGGCTTCAGCAGAGCGCAGATACCAAATACTGTCCTTCTAGTG
TAGCCGTAGTTAGGCCACCACTTCAAGAACTCTGTAGCACCGCCTACATACCTCGCT
CTGCTAATCCTGTTACCAGTGGCTGCTGCCAGTGGCGATAAGTCGTGTCTTACCGGG
TTGGACTCAAGACGATAGTTACCGGATAAGGCGCAGCGGTCGGGCTGAACGGGGGG
TTCGTGCACACAGCCCAGCTTGGAGCGAACGACCTACACCGAACTGAGATACCTAC
AGCGTGAGCTATGAGAAAGCGCCACGCTTCCCGAAGGGAGAAAGGCGGACAGGTAT
CCGGTAAGCGGCAGGGTCGGAACAGGAGAGCGCACGAGGGAGCTTCCAGGGGGAA
ACGCCTGGTATCTTTATAGTCCTGTCGGGTTTCGCCACCTCTGACTTGAGCGTCGATT
TTTGTGATGCTCGTCAGGGGGGCGGAGCCTATGGAAAAACGCCAGCAACGCGGCCT
TTTTACGGTTCCTGGCCTTTTGCTGGCCTTTTGCTCACATGTTCTTTCCTGCGTTATCC
CCTGATTCTGTGGATAACCGTATTACCGCCTTTGAGTGAGCTGATACCGCTCGCCGC
AGCCGAACGACCGAGCGCAGCGAGTCAGTGAGCGAGGAAGCGGAAGAGCGCCCAA
TACGCAAACCGCCTCTCCCCGCGCGTTGGCCGATTCATTAATGCA

| **R2C4 RESIDUE** | **NUCLEOTIDE CHANGE** |
|---|---|
| AAV4-T245A | ACC→GCC |

### AAV5-Wild Type; SEQ ID No. 143

CACCTAAATTGTAAGCGTTAATATTTTGTTAAAATTCGCGTTAAATTTTTGTTAAATC
AGCTCATTTTTTAACCAATAGGCCGAAATCGGCAAAATCCCTTATAAATCAAAAGAA
TAGACCGAGATAGGGTTGAGTGTTGTTCCAGTTTGGAACAAGAGTCCACTATTAAAG
AACGTGGACTCCAACGTCAAAGGGCGAAAAACCGTCTATCAGGGCGATGGCCCACT
ACGTGAACCATCACCCTAATCAAGTTTTTTGGGGTCGAGGTGCCGTAAAGCACTAAA
TCGGAACCCTAAAGGGAGCCCCCGATTTAGAGCTTGACGGGGAAAGCCGGCGAACG
TGGCGAGAAAGGAAGGGAAGAAAGCGAAAGGAGCGGGCGCTAGGGCGCTGGCAAG
TGTAGCGGTCACGCTGCGCGTAACCACCACACCCGCCGCGCTTAATGCGCCGCTACA
GGGCGCGTCCCATTCGCCATTCAGGCTGCGCAACTGTTGGGAAGGGCGATCGGTGC
GGGCCTCTTCGCTATTACGCCAGCTGGCGAAAGGGGGATGTGCTGCAAGGCGATTA
AGTTGGGTAACGCCAGGGTTTTCCCAGTCACGACGTTGTAAAACGACGGCCAGTGA
ATTGTAATACGACTCACTATAGGGCGAATTGGAGCTCCACCGCGGTGGCGGCCGCTC
TAGAGTCCTGTATTAGAGGTCACGTGAGTGTTTTGCGACATTTTGCGACACCATGTG
GTCACGCTGGGTATTTAAGCCCGAGTGAGCACGCAGGGTCTCCATTTTGAAGCGGGA
GGTTTGAACGCGCAGCCACCACGGCGGGGTTTTACGAGATTGTGATTAAGGTCCCCA
GCGACCTTGACGGGCATCTGCCCGGCATTTCTGACAGCTTTGTGAACTGGGTGGCCG
AGAAGGAATGGGAGTTGCCGCCAGATTCTGACATGGATCTGAATCTGATTGAGCAG
GCACCCCTGACCGTGGCCGAGAAGCTGCAGCGCGACTTTCTGACGGAATGGCGCCG
TGTGAGTAAGGCCCCGGAGGCCCTTTTCTTTGTGCAATTTGAGAAGGGAGAGAGCTA
CTTCCACATGCACGTGCTCGTGGAAACCACCGGGGTGAAATCCATGGTTTTGGGACG
TTTCCTGAGTCAGATTCGCGAAAAACTGATTCAGAGAATTTACCGCGGGATCGAGCC
GACTTTGCCAAACTGGTTCGCGGTCACAAAGACCAGAAATGGCGCCGGAGGCGGGA
ACAAGGTGGTGGATGAGTGCTACATCCCCAATTACTTGCTCCCCAAAACCCAGCCTG
AGCTCCAGTGGGCGTGGACTAATATGGAACAGTATTTAAGCGCCTGTTTGAATCTCA
CGGAGCGTAAACGGTTGGTGGCGCAGCATCTGACGCACGTGTCGCAGACGCAGGAG
CAGAACAAAGAGAATCAGAATCCCAATTCTGATGCGCCGGTGATCAGATCAAAAAC
TTCAGCCAGGTACATGGAGCTGGTCGGGTGGCTCGTGGACAAGGGGATTACCTCGG
AGAAGCAGTGGATCCAGGAGGACCAGGCCTCATACATCTCCTTCAATGCGGCCTCC
AACTCGCGGTCCCAAATCAAGGCTGCCTTGGACAATGCGGGAAAGATTATGAGCCT
GACTAAAACCGCCCCCGACTACCTGGTGGGCCAGCAGCCCGTGGAGGACATTTCCA
GCAATCGGATTTATAAAATTTTGGAACTAAACGGGTACGATCCCCAATATGCGGCTT
CCGTCTTTCTGGGATGGGCCACGAAAAAGTTCGGCAAGAGGAACACCATCTGGCTG
TTTGGGCCTGCAACTACCGGGAAGACCAACATCGCGGAGGCCATAGCCCACACTGT
GCCCTTCTACGGGTGCGTAAACTGGACCAATGAGAACTTTCCCTTCAACGACTGTGT
CGACAAGATGGTGATCTGGTGGGAGGAGGGGAAGATGACCGCCAAGGTCGTGGAGT
CGGCCAAAGCCATTCTCGGAGGAAGCAAGGTGCGCGTGGACCAGAAATGCAAGTCC
TCGGCCCAGATAGACCCGACTCCCGTGATCGTCACCTCCAACACCAACATGTGCGCC
GTGATTGACGGGAACTCAACGACCTTCGAACACCAGCAGCCGTTGCAAGACCGGAT
GTTCAAATTTGAACTCACCCGCCGTCTGGATCATGACTTTGGGAAGGTCACCAAGCA

GGAAGTCAAAGACTTTTTCCGGTGGGCAAAGGATCACGTGGTTGAGGTGGAGCATG
AATTCTACGTCAAAAAGGGTGGAGCCAAGAAAAGACCCGCCCCCAGTGACGCAGAT
ATAAGTGAGCCCAAACGGGTGCGCGAGTCAGTTGCGCAGCCATCGACGTCAGACGC
GGAAGCTTCGATCAACTACGCAGACAGGTACCAAAACAAATGTTCTCGTCACGTGG
GCATGAATCTGATGCTGTTTCCCTGCAGACAATGCGAGAGAATGAATCAGAATTCAA
ATATCTGCTTCACTCACGGACAGAAAGACTGTTTAGAGTGCTTTCCCGTGTCAGAAT
CTCAACCCGTTTCTGTCGTCAAAAAGGCGTATCAGAAACTGTGCTACATTCATCATA
TCATGGGAAAGGTGCCAGACGCTTGCACTGCCTGCGATCTGGTCAATGTGGATTTGG
ATGACTGCATCTTTGAACAATAAATGATTTAAATAGTCATGTCTTTTGTTGATCACCC
TCCAGATTGGTTGGAAGAAGTTGGTGAAGGTCTTCGCGAGTTTTTGGGCCTTGAAGC
GGGCCCACCGAAACCAAAACCCAATCAGCAGCATCAAGATCAAGCCCGTGGTCTTG
TGCTGCCTGGTTATAACTATCTCGGACCCGGAAACGGTCTCGATCGAGGAGAGCCTG
TCAACAGGGCAGACGAGGTCGCGCGAGAGCACGACATCTCGTACAACGAGCAGCTT
GAGGCGGGAGACAACCCCTACCTCAAGTACAACCACGCGGACGCCGAGTTTCAGGA
GAAGCTCGCCGACGACACATCCTTCGGGGGAAACCTCGGAAAGGCAGTCTTTCAGG
CCAAGAAAAGGGTTCTCGAACCTTTTGGCCTGGTTGAAGAGGGTGCTAAGACGGCC
CCTACCGGAAAGCGGATAGACGACCACTTTCCAAAAAGAAAGAAGGCTCGGACCGA
AGAGGACTCCAAGCCTTCCACCTCGTCAGACGCCGAAGCTGGACCCAGCGGATCCC
AGCAGCTGCAAATCCCAGCCCAACCAGCCTCAAGTTTGGGAGCTGATACAATGTCT
GCGGGAGGTGGCGGCCCATTGGGCGACAATAACCAAGGTGCCGATGGAGTGGGCAA
TGCCTCGGGAGATTGGCATTGCGATTCCACGTGGATGGGGGACAGAGTCGTCACCA
AGTCCACCCGAACCTGGGTGCTGCCCAGCTACAACAACCACCAGTACCGAGAGATC
AAAAGCGGCTCCGTCGACGGAAGCAACGCCAACGCCTACTTTGGATACAGCACCCC
CTGGGGGTACTTTGACTTTAACCGCTTCCACAGCCACTGGAGCCCCCGAGACTGGCA
AAGACTCATCAACAACTACTGGGGCTTCAGACCCCGGTCCCTCAGAGTCAAAATCTT
CAACATTCAAGTCAAAGAGGTCACGGTGCAGGACTCCACCACCACCATCGCCAACA
ACCTCACCTCCACCGTCCAAGTGTTTACGGACGACGACTACCAGCTGCCCTACGTCG
TCGGCAACGGGACCGAGGGATGCCTGCCGGCCTTCCCTCCGCAGGTCTTTACGCTGC
CGCAGTACGGTTACGCGACGCTGAACCGCGACAACACAGAAAATCCCACCGAGAGG
AGCAGCTTCTTCTGCCTAGAGTACTTTCCCAGCAAGATGCTGAGAACGGGCAACAAC
TTTGAGTTTACCTACAACTTTGAGGAGGTGCCCTTCCACTCCAGCTTCGCTCCCAGTC
AGAACCTGTTCAAGCTGGCCAACCCGCTGGTGGACCAGTACTTGTACCGCTTCGTGA
GCACAAATAACACTGGCGGAGTCCAGTTCAACAAGAACCTGGCCGGGAGATACGCC
AACACCTACAAAAACTGGTTCCCGGGGCCCATGGGCCGAACCCAGGGCTGGAACCT
GGGCTCCGGGGTCAACCGCGCCAGTGTCAGCGCCTTCGCCACGACCAATAGGATGG
AGCTCGAGGGCGCGAGTTACCAGGTGCCCCCGCAGCCGAACGGCATGACCAACAAC
CTCCAGGGCAGCAACACCTATGCCCTGGAGAACACTATGATCTTCAACAGCCAGCC
GGCGAACCCGGGCACCACCGCCACGTACCTCGAGGGCAACATGCTCATCACCAGCG
AGAGCGAGACGCAGCCGGTGAACCGCGTGGCGTACAACGTCGGCGGGCAGATGGC
CACCAACAACCAGAGCTCCACCACTGCCCCCGCGACCGGCACGTACAACCTCCAGG

AAATCGTGCCCGGCAGCGTGTGGATGGAGAGGGACGTGTACCTCCAAGGACCCATC
TGGGCCAAGATCCCAGAGACGGGGGCGCACTTTCACCCCTCTCCGGCCATGGGCGG
ATTCGGACTCAAACACCCACCGCCCATGATGCTCATCAAGAACACGCCTGTGCCCGG
AAATATCACCAGCTTCTCGGACGTGCCCGTCAGCAGCTTCATCACCCAGTACAGCAC
CGGGCAGGTCACCGTGGAGATGGAGTGGGAGCTCAAGAAGGAAAACTCCAAGAGG
TGGAACCCAGAGATCCAGTACACAAACAACTACAACGACCCCCAGTTTGTGGACTT
TGCCCCGGACAGCACCGGGGAATACAGAACCACCAGACCTATCGGAACCCGATACC
TTACCCGACCCCTTTAACCCATTCATGTCGCATACCCTCAATAAACCGTGTATTCGTG
TCAGTAAAATACTGCCTCTTGTGGTCATTTCTAGAACTAGAACTAGTGGATCCCCCG
GGCTGCAGGAATTCGATATCAAGCTTATCGATACCGTCGACCTCGAGGGGGGCCC
GGTACCCAGCTTTTGTTCCCTTTAGTGAGGGTTAATTTCGAGCTTGGCGTAATCATGG
TCATAGCTGTTTCCTGTGTGAAATTGTTATCCGCTCACAATTCCACACAACATACGA
GCCGGAAGCATAAAGTGTAAAGCCTGGGGTGCCTAATGAGTGAGCTAACTCACATT
AATTGCGTTGCGCTCACTGCCCGCTTTCCAGTCGGGAAACCTGTCGTGCCAGCTGCA
TTAATGAATCGGCCAACGCGCGGGGAGAGGCGGTTTGCGTATTGGGCGCTCTTCCGC
TTCCTCGCTCACTGACTCGCTGCGCTCGGTCGTTCGGCTGCGGCGAGCGGTATCAGC
TCACTCAAAGGCGGTAATACGGTTATCCACAGAATCAGGGGATAACGCAGGAAAGA
ACATGTGAGCAAAAGGCCAGCAAAAGGCCAGGAACCGTAAAAAGGCCGCGTTGCT
GGCGTTTTTCCATAGGCTCCGCCCCCCTGACGAGCATCACAAAAATCGACGCTCAAG
TCAGAGGTGGCGAAACCCGACAGGACTATAAAGATACCAGGCGTTTCCCCCTGGAA
GCTCCCTCGTGCGCTCTCCTGTTCCGACCCTGCCGCTTACCGGATACCTGTCCGCCTT
TCTCCCTTCGGGAAGCGTGGCGCTTTCTCATAGCTCACGCTGTAGGTATCTCAGTTCG
GTGTAGGTCGTTCGCTCCAAGCTGGGCTGTGTGCACGAACCCCCCGTTCAGCCCGAC
CGCTGCGCCTTATCCGGTAACTATCGTCTTGAGTCCAACCCGGTAAGACACGACTTA
TCGCCACTGGCAGCAGCCACTGGTAACAGGATTAGCAGAGCGAGGTATGTAGGCGG
TGCTACAGAGTTCTTGAAGTGGTGGCCTAACTACGGCTACACTAGAAGGACAGTATT
TGGTATCTGCGCTCTGCTGAAGCCAGTTACCTTCGGAAAAAGAGTTGGTAGCTCTTG
ATCCGGCAAACAAACCACCGCTGGTAGCGGTGGTTTTTTTGTTTGCAAGCAGCAGAT
TACGCGCAGAAAAAAAGGATCTCAAGAAGATCCTTTGATCTTTTCTACGGGGTCTGA
CGCTCAGTGGAACGAAAACTCACGTTAAGGGATTTTGGTCATGAGATTATCAAAAA
GGATCTTCACCTAGATCCTTTTAAATTAAAAATGAAGTTTTAAATCAATCTAAAGTA
TATATGAGTAAACTTGGTCTGACAGTTACCAATGCTTAATCAGTGAGGCACCTATCT
CAGCGATCTGTCTATTTCGTTCATCCATAGTTGCCTGACTCCCCGTCGTGTAGATAAC
TACGATACGGGAGGGCTTACCATCTGGCCCCAGTGCTGCAATGATACCGCGAGACC
CACGCTCACCGGCTCCAGATTTATCAGCAATAAACCAGCCAGCCGGAAGGGCCGAG
CGCAGAAGTGGTCCTGCAACTTTATCCGCCTCCATCCAGTCTATTAATTGTTGCCGG
GAAGCTAGAGTAAGTAGTTCGCCAGTTAATAGTTTGCGCAACGTTGTTGCCATTGCT
ACAGGCATCGTGGTGTCACGCTCGTCGTTTGGTATGGCTTCATTCAGCTCCGGTTCCC
AACGATCAAGGCGAGTTACATGATCCCCCATGTTGTGCAAAAAAGCGGTTAGCTCCT
TCGGTCCTCCGATCGTTGTCAGAAGTAAGTTGGCCGCAGTGTTATCACTCATGGTTA

TGGCAGCACTGCATAATTCTCTTACTGTCATGCCATCCGTAAGATGCTTTTCTGTGAC
TGGTGAGTACTCAACCAAGTCATTCTGAGAATAGTGTATGCGGCGACCGAGTTGCTC
TTGCCCGGCGTCAATACGGGATAATACCGCGCCACATAGCAGAACTTTAAAAGTGCT
CATCATTGGAAAACGTTCTTCGGGGCGAAAACTCTCAAGGATCTTACCGCTGTTGAG
ATCCAGTTCGATGTAACCCACTCGTGCACCCAACTGATCTTCAGCATCTTTTACTTTC
ACCAGCGTTTCTGGGTGAGCAAAAACAGGAAGGCAAAATGCCGCAAAAAAGGGAA
TAAGGGCGACACGGAAATGTTGAATACTCATACTCTTCCTTTTTCAATATTATTGAA
GCATTTATCAGGGTTATTGTCTCATGAGCGGATACATATTTGAATGTATTTAGAAAA
ATAAACAAATAGGGGTTCCGCGCACATTTCCCCGAAAAGTGC

### AAV5-Mutant; SEQ ID No. 153

CACCTAAATTGTAAGCGTTAATATTTTGTTAAAATTCGCGTTAAATTTTTGTTAAATC
AGCTCATTTTTTAACCAATAGGCCGAAATCGGCAAAATCCCTTATAAATCAAAAGAA
TAGACCGAGATAGGGTTGAGTGTTGTTCCAGTTTGGAACAAGAGTCCACTATTAAAG
AACGTGGACTCCAACGTCAAAGGGCGAAAAACCGTCTATCAGGGCGATGGCCCACT
ACGTGAACCATCACCCTAATCAAGTTTTTTGGGGTCGAGGTGCCGTAAAGCACTAAA
TCGGAACCCTAAAGGGAGCCCCCGATTTAGAGCTTGACGGGGAAAGCCGGCGAACG
TGGCGAGAAAGGAAGGGAAGAAAGCGAAAGGAGCGGGCGCTAGGGCGCTGGCAAG
TGTAGCGGTCACGCTGCGCGTAACCACCACACCCGCCGCGCTTAATGCGCCGCTACA
GGGCGCGTCCCATTCGCCATTCAGGCTGCGCAACTGTTGGGAAGGGCGATCGGTGC
GGGCCTCTTCGCTATTACGCCAGCTGGCGAAAGGGGGATGTGCTGCAAGGCGATTA
AGTTGGGTAACGCCAGGGTTTTCCCAGTCACGACGTTGTAAAACGACGGCCAGTGA
ATTGTAATACGACTCACTATAGGGCGAATTGGAGCTCCACCGCGGTGGCGGCCGCTC
TAGAGTCCTGTATTAGAGGTCACGTGAGTGTTTTGCGACATTTTGCGACACCATGTG
GTCACGCTGGGTATTTAAGCCCGAGTGAGCACGCAGGGTCTCCATTTTGAAGCGGGA
GGTTTGAACGCGCAGCCACCACGGCGGGGTTTTACGAGATTGTGATTAAGGTCCCCA
GCGACCTTGACGGGCATCTGCCCGGCATTTCTGACAGCTTTGTGAACTGGGTGGCCG
AGAAGGAATGGGAGTTGCCGCCAGATTCTGACATGGATCTGAATCTGATTGAGCAG
GCACCCCTGACCGTGGCCGAGAAGCTGCAGCGCGACTTTCTGACGGAATGGCGCCG
TGTGAGTAAGGCCCCGGAGGCCCTTTTCTTTGTGCAATTTGAGAAGGGAGAGAGCTA
CTTCCACATGCACGTGCTCGTGGAAACCACCGGGGTGAAATCCATGGTTTTGGGACG
TTTCCTGAGTCAGATTCGCGAAAAACTGATTCAGAGAATTTACCGCGGGATCGAGCC
GACTTTGCCAAACTGGTTCGCGGTCACAAAGACCAGAAATGGCGCCGGAGGCGGGA
ACAAGGTGGTGGATGAGTGCTACATCCCCAATTACTTGCTCCCCAAAACCCAGCCTG
AGCTCCAGTGGGCGTGGACTAATATGGAACAGTATTTAAGCGCCTGTTTGAATCTCA
CGGAGCGTAAACGGTTGGTGGCGCAGCATCTGACGCACGTGTCGCAGACGCAGGAG
CAGAACAAAGAGAATCAGAATCCCAATTCTGATGCGCCGGTGATCAGATCAAAAAC
TTCAGCCAGGTACATGGAGCTGGTCGGGTGGCTCGTGGACAAGGGGATTACCTCGG

AGAAGCAGTGGATCCAGGAGGACCAGGCCTCATACATCTCCTTCAATGCGGCCTCC
AACTCGCGGTCCCAAATCAAGGCTGCCTTGGACAATGCGGGAAAGATTATGAGCCT
GACTAAAACCGCCCCCGACTACCTGGTGGGCCAGCAGCCCGTGGAGGACATTTCCA
GCAATCGGATTTATAAAATTTTGGAACTAAACGGGTACGATCCCCAATATGCGGCTT
CCGTCTTTCTGGGATGGGCCACGAAAAAGTTCGGCAAGAGGAACACCATCTGGCTG
TTTGGGCCTGCAACTACCGGGAAGACCAACATCGCGGAGGCCATAGCCCACACTGT
GCCCTTCTACGGGTGCGTAAACTGGACCAATGAGAACTTTCCCTTCAACGACTGTGT
CGACAAGATGGTGATCTGGTGGGAGGAGGGGAAGATGACCGCCAAGGTCGTGGAGT
CGGCCAAAGCCATTCTCGGAGGAAGCAAGGTGCGCGTGGACCAGAAATGCAAGTCC
TCGGCCCAGATAGACCCGACTCCCGTGATCGTCACCTCCAACACCAACATGTGCGCC
GTGATTGACGGGAACTCAACGACCTTCGAACACCAGCAGCCGTTGCAAGACCGGAT
GTTCAAATTTGAACTCACCCGCCGTCTGGATCATGACTTTGGGAAGGTCACCAAGCA
GGAAGTCAAAGACTTTTTCCGGTGGGCAAAGGATCACGTGGTTGAGGTGGAGCATG
AATTCTACGTCAAAAAGGGTGGAGCCAAGAAAAGACCCGCCCCCAGTGACGCAGAT
ATAAGTGAGCCCAAACGGGTGCGCGAGTCAGTTGCGCAGCCATCGACGTCAGACGC
GGAAGCTTCGATCAACTACGCAGACAGGTACCAAAACAAATGTTCTCGTCACGTGG
GCATGAATCTGATGCTGTTTCCCTGCAGACAATGCGAGAGAATGAATCAGAATTCAA
ATATCTGCTTCACTCACGGACAGAAAGACTGTTTAGAGTGCTTTCCCGTGTCAGAAT
CTCAACCCGTTTCTGTCGTCAAAAAGGCGTATCAGAAACTGTGCTACATTCATCATA
TCATGGGAAAGGTGCCAGACGCTTGCACTGCCTGCGATCTGGTCAATGTGGATTTGG
ATGACTGCATCTTTGAACAATAAATGATTTAAATAGTCATGTCTTTTGTTGATCACCC
TCCAGATTGGTTGGAAGAAGTTGGTGAAGGTCTTCGCGAGTTTTTGGGCCTTGAAGC

K32R

GGGCCCACCGAAACCAGAACCCAATCAGCAGCATCAAGATCAAGCCCGTGGTCTTG
TGCTGCCTGGTTATAACTATCTCGGACCCGGAAACGGTCTCGATCGAGGAGAGCCTG
TCAACAGGGCAGACGAGGTCGCGCGAGAGCACGACATCTCGTACAACGAGCAGCTT
GAGGCGGGAGACAACCCCTACCTCAAGTACAACCACGCGGACGCCGAGTTTCAGGA

T107A

GAAGCTCGCCGACGACGCATCCTTCGGGGGAAACCTCGGAAAGGCAGTCTTTCAGG
CCAAGAAAAGGGTTCTCGAACCTTTTGGCCTGGTTGAAGAGGGTGCTAAGACGGCC
CCTACCGGAAAGCGGATAGACGACCACTTTCCAAAAAGAAAGAAGGCTCGGACCGA
AGAGGACTCCAAGCCTTCCACCTCGTCAGACGCCGAAGCTGGACCCAGCGGATCCC
AGCAGCTGCAAATCCCAGCCCAACCAGCCTCAAGTTTGGGAGCTGATACAATGTCT
GCGGGAGGTGGCGGCCCATTGGGCGACAATAACCAAGGTGCCGATGGAGTGGGCAA
TGCCTCGGGAGATTGGCATTGCGATTCCACGTGGATGGGGGACAGAGTCGTCACCA
AGTCCACCCGAACCTGGGTGCTGCCCAGCTACAACAACCACCAGTACCGAGAGATC
AAAAGCGGCTCCGTCGACG

S268A

GAAGCAACGCCAACGCCTACTTTGGATACGCCACCCCCTGGGGGTACTTTGACTTTA
ACCGCTTCCACAGCCACTGGAGCCCCCGAGACTGGCAAAGACTCATCAACAACTAC
TGGGGCTTCAGACCCCGGTCCCTCAGAGTCAAAATCTTCAACATTCAAGTCAAAGAG

T328A

GTCACGGTGCAGGACTCCACCACCACCATCGCCAACAACCTCGCCTCCACCGTCCAA
GTGTTTACGGACGACGACTACCAGCTGCCCTACGTCGTCGGCAACGGGACCGAGGG
ATGCCTGCCGGCCTTCCCTCCGCAGGTCTTTACGCTGCCGCAGTACGGTTACGCGAC
GCTGAACCGCGACAACACAGAAAATCCCACCGAGAGGAGCAGCTTCTTCTGCCTAG
AGTACTTTCCCAGCAAGATGCTGAGAACGGGCAACAACTTTGAGTTTACCTACAACT
TTGAGGAGGTGCCCTTCCACTCCAGCTTCGCTCCCAGTCAGAACCTGTTCAAGCTGG
CCAACCCGCTGGTGGACCAGTACTTGTACCGCTTCGTGAGCACAAATAACACTGGCG
GAGTCCAGTTCAACAAGAACCTGGCCGGGAGATACGCCAACACCTACAAAAACTGG
TTCCCGGGGCCCATGGGCCGAACCCAGGGCTGGAACCTGG

S485A

GCTCCGGGGTCAACCGCGCCGCTGTCAGCGCCTTCGCCACGACCAATAGGATGGAG
CTCGAGGGCGCGAGTTACCAGGTGCCCCCGCAGCCGAACGGCATGACCAACAACCT
CCAGGGCAGCAACACCTATGCCCTGGAGAACACTATGATCTTCAACAGCCAGCCGG
CGAACCCGGGCACCACCGCCACGTACCTCGAGGGCAACATGCTCATCACCAGCGAG
AGCGAGACGCAGCCGGTGAACCGCGTGGCGTACAACGTCGGCGGGCAGATGGCCAC
CAACAACCAGAGCTCCACCACTGCCCCCGCGACCGGCACGTACAACCTCCAGGAAA
TCGTGCCCGGCAGCGTGTGGATGGAGAGGGACGTGTACCTCCAAGGACCCATCTGG
GCCAAGATCCCAGAGACGGGGGCGCACTTTCACCCCTCTCCGGCCATGGGCGGATT
CGGACTCAAACACCCACCGCCCATGATGCTCATCAAGAACACGCCTGTGCCCGGAA
ATATC

S652A S658A

ACCAGCTTCGCGGACGTGCCCGTCAGCGCCTTCATCACCCAGTACAGCACCGGGCA
GGTCACCGTGGAGATGGAGTGGGAGCTCAAGAAGGAAAACTCCAAGAGGTGGAAC
CCAGAGATCCAGTACACAAACAACTACAACGACCCCCAGTTTGTGGACTTTGCCCCG
GACAGCACCGGGGAATACAGAACCACCAGACCTATCGGAACCCGATACCTTACCCG
ACCCCTTTAACCCATTCATGTCGCATACCCTCAATAAACCGTGTATTCGTGTCAGTAA
AATACTGCCTCTTGTGGTCATTTCTAGAACTAGAACTAGTGGATCCCCCGGGCTGCA
GGAATTCGATATCAAGCTTATCGATACCGTCGACCTCGAGGGGGGGCCCGGTACCC
AGCTTTTGTTCCCTTTAGTGAGGGTTAATTTCGAGCTTGGCGTAATCATGGTCATAGC
TGTTTCCTGTGTGAAATTGTTATCCGCTCACAATTCCACACAACATACGAGCCGGAA

GCATAAAGTGTAAAGCCTGGGGTGCCTAATGAGTGAGCTAACTCACATTAATTGCGT
TGCGCTCACTGCCCGCTTTCCAGTCGGGAAACCTGTCGTGCCAGCTGCATTAATGAA
TCGGCCAACGCGCGGGGAGAGGCGGTTTGCGTATTGGGCGCTCTTCCGCTTCCTCGC
TCACTGACTCGCTGCGCTCGGTCGTTCGGCTGCGGCGAGCGGTATCAGCTCACTCAA
AGGCGGTAATACGGTTATCCACAGAATCAGGGGATAACGCAGGAAAGAACATGTGA
GCAAAAGGCCAGCAAAAGGCCAGGAACCGTAAAAAGGCCGCGTTGCTGGCGTTTTT
CCATAGGCTCCGCCCCCCTGACGAGCATCACAAAAATCGACGCTCAAGTCAGAGGT
GGCGAAACCCGACAGGACTATAAAGATACCAGGCGTTTCCCCCTGGAAGCTCCCTC
GTGCGCTCTCCTGTTCCGACCCTGCCGCTTACCGGATACCTGTCCGCCTTTCTCCCTT
CGGGAAGCGTGGCGCTTTCTCATAGCTCACGCTGTAGGTATCTCAGTTCGGTGTAGG
TCGTTCGCTCCAAGCTGGGCTGTGTGCACGAACCCCCCGTTCAGCCCGACCGCTGCG
CCTTATCCGGTAACTATCGTCTTGAGTCCAACCCGGTAAGACACGACTTATCGCCAC
TGGCAGCAGCCACTGGTAACAGGATTAGCAGAGCGAGGTATGTAGGCGGTGCTACA
GAGTTCTTGAAGTGGTGGCCTAACTACGGCTACACTAGAAGGACAGTATTTGGTATC
TGCGCTCTGCTGAAGCCAGTTACCTTCGGAAAAAGAGTTGGTAGCTCTTGATCCGGC
AAACAAACCACCGCTGGTAGCGGTGGTTTTTTTGTTTGCAAGCAGCAGATTACGCGC
AGAAAAAAAGGATCTCAAGAAGATCCTTTGATCTTTTCTACGGGGTCTGACGCTCAG
TGGAACGAAAACTCACGTTAAGGGATTTTGGTCATGAGATTATCAAAAAGGATCTTC
ACCTAGATCCTTTTAAATTAAAAATGAAGTTTTAAATCAATCTAAAGTATATATGAG
TAAACTTGGTCTGACAGTTACCAATGCTTAATCAGTGAGGCACCTATCTCAGCGATC
TGTCTATTTCGTTCATCCATAGTTGCCTGACTCCCCGTCGTGTAGATAACTACGATAC
GGGAGGGCTTACCATCTGGCCCCAGTGCTGCAATGATACCGCGAGACCCACGCTCA
CCGGCTCCAGATTTATCAGCAATAAACCAGCCAGCCGGAAGGGCCGAGCGCAGAAG
TGGTCCTGCAACTTTATCCGCCTCCATCCAGTCTATTAATTGTTGCCGGGAAGCTAGA
GTAAGTAGTTCGCCAGTTAATAGTTTGCGCAACGTTGTTGCCATTGCTACAGGCATC
GTGGTGTCACGCTCGTCGTTTGGTATGGCTTCATTCAGCTCCGGTTCCCAACGATCAA
GGCGAGTTACATGATCCCCCATGTTGTGCAAAAAAGCGGTTAGCTCCTTCGGTCCTC
CGATCGTTGTCAGAAGTAAGTTGGCCGCAGTGTTATCACTCATGGTTATGGCAGCAC
TGCATAATTCTCTTACTGTCATGCCATCCGTAAGATGCTTTTCTGTGACTGGTGAGTA
CTCAACCAAGTCATTCTGAGAATAGTGTATGCGGCGACCGAGTTGCTCTTGCCCGGC
GTCAATACGGGATAATACCGCGCCACATAGCAGAACTTTAAAAGTGCTCATCATTGG
AAAACGTTCTTCGGGGCGAAAACTCTCAAGGATCTTACCGCTGTTGAGATCCAGTTC
GATGTAACCCACTCGTGCACCCAACTGATCTTCAGCATCTTTTACTTTCACCAGCGTT
TCTGGGTGAGCAAAAACAGGAAGGCAAAATGCCGCAAAAAAGGGAATAAGGGCGA
CACGGAAATGTTGAATACTCATACTCTTCCTTTTTCAATATTATTGAAGCATTTATCA
GGGTTATTGTCTCATGAGCGGATACATATTTGAATGTATTTAGAAAAATAAACAAAT
AGGGGTTCCGCGCACATTTCCCCGAAAAGTGC

| AAV5 RESIDUE | NUCLEOTIDE CHANGE |
|---|---|
| AAV5-S268A | AGC→GCC |
| AAV5-S485A | AGT→GCT |
| AAV5-S652A | TCG→GCG |
| AAV5-S658A | AGC→GCC |
| AAV5-K32R | AAA→AGA |
| AAV5-T107A | ACA→GCA |
| AAV5-T328A | ACC→GCC |

### AAV6-Wild Type; SEQ ID No. 144

AATTCCCATCATCAATAATATACCTTATTTAGGATTGAAGCCAATATGATAATGAGC
CGGTGGAGTTTGTGACGTGGCGCGGGGCGTGGGAACGGGGCGGGTGACGTAGTAGT
CTCTAGAGTCCTGTATTAGAGGTCACGTGAGTGTTTTGCGACTTTTGCGACACCATG
TGGTCACGCTGGGTATTTAAGCCCGAGTGAGCACGCAGGGTCTCCATTTTGAAGCGG
GAGGTTTGAACGCGCAGCCACCACGGGGGGTTTTACGAGATTGTGATTAAGGTCC
CCAGCGACCTTGACGGGCATCTGCCCGGCATTTCTGACAGCTTTGTGAACTGGGTGG
CCGAGAAGGAATGGGAGTTGCCGCCAGATTCTGACATGGATCTGAATCTGATTGAG
CAGGCACCCCTGACCGTGGCCGAGAAGCTGCAGCGCGACTTTCTGACGGAATGGCG
CCGTGTGAGTAAGGCCCCGGAGGCCCTTTTCTTTGTGCAATTTGAGAAGGGAGAGAG
CTACTTCCACATGCACGTGCTCGTGGAAACCACCGGGGTGAAATCCATGGTTTTGGG
ACGTTTCCTGAGTCAGATTCGCGAAAAACTGATTCAGAGAATTTACCGCGGGATCGA
GCCGACTTTGCCAAACTGGTTCGCGGTCACAAAGACCAGAAATGGCGCCGGAGGCG
GGAACAAGGTGGTGGATGAGTGCTACATCCCCAATTACTTGCTCCCCAAAACCCAG
CCTGAGCTCCAGTGGGCGTGGACTAATATGGAACAGTATTTAAGCGCCTGTTTGAAT
CTCACGGAGCGTAAACGGTTGGTGGCGCAGCATCTGACGCACGTGTCGCAGACGCA
GGAGCAGAACAAAGAGAATCAGAATCCCAATTCTGATGCGCCGGTGATCAGATCAA
AAACTTCAGCCAGGTACATGGAGCTGGTCGGGTGGCTCGTGGACAAGGGGATTACC
TCGGAGAAGCAGTGGATCCAGGAGGACCAGGCCTCATACATCTCCTTCAATGCGGC
CTCCAACTCGCGGTCCCAAATCAAGGCTGCCTTGGACAATGCGGGAAAGATTATGA
GCCTGACTAAAACCGCCCCCGACTACCTGGTGGGCCAGCAGCCCGTGGAGGACATT

TCCAGCAATCGGATTTATAAAATTTTGGAACTAAACGGGTACGATCCCCAATATGCG
GCTTCCGTCTTTCTGGGATGGCCACGAAAAAGTTCGGCAAGAGGAACACCATCTG
GCTGTTTGGGCCTGCAACTACCGGAAGACCAACATCGCGGAGGCCATAGCCCACA
CTGTGCCCTTCTACGGGTGCGTAAACTGGACCAATGAGAACTTTCCCTTCAACGACT
GTGTCGACAAGATGGTGATCTGGTGGAGGAGGGGAAGATGACCGCCAAGGTCGTG
GAGTCGGCCAAAGCCATTCTCGGAGGAAGCAAGGTGCGCGTGGACCAGAAATGCAA
GTCCTCGGCCCAGATAGACCCGACTCCCGTGATCGTCACCTCCAACACCAACATGTG
CGCCGTGATTGACGGGAACTCAACGACCTTCGAACACCAGCAGCCGTTGCAAGACC
GGATGTTCAAATTTGAACTCACCCGCCGTCTGGATCATGACTTTGGGAAGGTCACCA
AGCAGGAAGTCAAAGACTTTTTCCGGTGGGCAAAGGATCACGTGGTTGAGGTGGAG
CATGAATTCTACGTCAAAAAGGGTGGAGCCAAGAAAAGACCCGCCCCCAGTGACGC
AGATATAAGTGAGCCCAAACGGGTGCGCGAGTCAGTTGCGCAGCCATCGACGTCAG
ACGCGGAAGCTTCGATCAACTACGCAGACAGGTACCAAAACAAATGTTCTCGTCAC
GTGGGCATGAATCTGATGCTGTTTCCCTGCAGACAATGCGAGAGAATGAATCAGAA
TTCAAATATCTGCTTCACTCACGGACAGAAAGACTGTTTAGAGTGCTTTCCCGTGTC
AGAATCTCAACCCGTTTCTGTCGTCAAAAAGGCGTATCAGAAACTGTGCTACATTCA
TCATATCATGGGAAAGGTGCCAGACGCTTGCACTGCCTGCGATCTGGTCAATGTGGA
TTTGGATGACTGCATCTTTGAACAATAAATGATTTAAATCAGGTATGGCTGCCGATG
GTTATCTTCCAGATTGGCTCGAGGACAACCTCTCTGAGGGCATTCGCGAGTGGTGGG
ACTTGAAACCTGGAGCCCCGAAACCCAAAGCCAACCAGCAAAAGCAGGACGACGG
CCGGGGTCTGGTGCTTCCTGGCTACAAGTACCTCGGACCCTTCAACGGACTCGACAA
GGGGGAGCCCGTCAACGCGGCGGATGCAGCGGCCCTCGAGCACGACAAGGCCTACG
ACCAGCAGCTCAAAGCGGGTGACAATCCGTACCTGCGGTATAACCACGCCGACGCC
GAGTTTCAGGAGCGTCTGCAAGAAGATACGTCTTTGGGGGCAACCTCGGGCGAGC
AGTCTTCCAGGCCAAGAAGAGGGTTCTCGAACCTTTTGGTCTGGTTGAGGAAGGTGC
TAAGACGGCTCCTGGAAAGAAACGTCCGGTAGAGCAGTCGCCACAAGAGCCAGACT
CCTCCTCGGGCATTGGCAAGACAGGCCAGCAGCCCGCTAAAAAGAGACTCAATTTT
GGTCAGACTGGCGACTCAGAGTCAGTCCCCGACCCACAACCTCTCGGAGAACCTCC
AGCAACCCCCGCTGCTGTGGGACCTACTACAATGGCTTCAGGCGGTGGCGCACCAA
TGGCAGACAATAACGAAGGCGCCGACGGAGTGGGTAATGCCTCAGGAAATTGGCAT
TGCGATTCCACATGGCTGGGCGACAGAGTCATCACCACCAGCACCCGAACATGGGC
CTTGCCCACCTATAACAACCACCTCTACAAGCAAATCTCCAGTGCTTCAACGGGGGC
CAGCAACGACAACCACTACTTCGGCTACAGCACCCCCTGGGGGTATTTTGATTTCAA
CAGATTCCACTGCCATTTCTCACCACGTGACTGGCAGCGACTCATCAACAACAATTG
GGGATTCCGGCCCAAGAGACTCAACTTCAAGCTCTTCAACATCCAAGTCAAGGAGG
TCACGACGAATGATGGCGTCACGACCATCGCTAATAACCTTACCAGCACGGTTCAA
GTCTTCTCGGACTCGGAGTACCAGTTGCCGTACGTCCTCGGCTCTGCGCACCAGGGC
TGCCTCCCTCCGTTCCCGGCGGACGTGTTCATGATTCCGCAGTACGGCTACCTAACG
CTCAACAATGGCAGCCAGGCAGTGGGACGGTCATCCTTTTACTGCCTGGAATATTTC
CCATCGCAGATGCTGAGAACGGGCAATAACTTTACCTTCAGCTACACCTTCGAGGAC
GTGCCTTTCCACAGCAGCTACGCGCACAGCCAGAGCCTGGACCGGCTGATGAATCCT

CTCATCGACCAGTACCTGTATTACCTGAACAGAACTCAGAATCAGTCCGGAAGTGCC
CAAAACAAGGACTTGCTGTTTAGCCGGGGTCTCCAGCTGGCATGTCTGTTCAGCCC
AAAAACTGGCTACCTGGACCCTGTTACCGGCAGCAGCGTTTCTAAACAAAAAC
AGACAACAACAACAGCAACTTTACCTGGACTGGTGCTTCAAAATATAACCTTAATGG
GCGTGAATCTATAATCAACCCTGGCACTGCTATGGCCTCACACAAAGACGACAAAG
ACAAGTTCTTTCCCATGAGCGGTGTCATGATTTTTGGAAAGGAGAGCGCCGGAGCTT
CAAACACTGCATTGGACAATGTCATGATCACAGACGAAGAGGAAATCAAAGCCACT
AACCCCGTGGCCACCGAAAGATTTGGGACTGTGGCAGTCAATCTCCAGAGCAGCAG
CACAGACCCTGCGACCGGAGATGTGCATGTTATGGGAGCCTTACCTGGAATGGTGTG
GCAAGACAGAGACGTATACCTGCAGGGTCCTATTTGGGCCAAAATTCCTCACACGG
ATGGACACTTTCACCCGTCTCCTCTCATGGGCGGCTTTGGACTTAAGCACCCGCCTC
CTCAGATCCTCATCAAAAACACGCCTGTTCCTGCGAATCCTCCGGCAGAGTTTCGG
CTACAAAGTTTGCTTCATTCATCACCCAGTATTCCACAGGACAAGTGAGCGTGGAGA
TTGAATGGGAGCTGCAGAAAGAAAACAGCAAACGCTGGAATCCCGAAGTGCAGTAT
ACATCTAACTATGCAAAATCTGCCAACGTTGATTTCACTGTGGACAACAATGGACTT
TATACTGAGCCTCGCCCCATTGGCACCCGTTACCTCACCCGTCCCCTGTAATTGCTTG
TTAATCAATAAACCGTTTAATTCGTTTCAGTTGAACTTTGGTCTCTGCGTATTTCTTTC
TTATCTAGTTTCCATGCTCTAGACTACTACGTCACCCGCCCCGTTCCCACGCCCCGCG
CCACGTCACAAACTCCACCCCCTCATTATCATATTGGCTTCAATCCAAAATAAGGTA
TATTATTGATGATGCATCGCTGGCGTAATAGCGAAGAGGCCCGCACCGATCGCCCTT
CCCAACAGTTGCGCAGCCTGAATGGCGAATGGAATTCCAGACGATTGAGCGTCAAA
ATGTAGGTATTTCCATGAGCGTTTTTCCTGTTGCAATGGCTGGCGGTAATATTGTTCT
GGATATTACCAGCAAGGCCGATAGTTTGAGTTCTTCTACTCAGGCAAGTGATGTTAT
TACTAATCAAAGAAGTATTGCGACAACGGTTAATTTGCGTGATGGACAGACTCTTTT
ACTCGGTGGCCTCACTGATTATAAAAACACTTCTCAGGATTCTGGCGTACCGTTCCT
GTCTAAAATCCCTTTAATCGGCCTCCTGTTTAGCTCCCGCTCTGATTCTAACGAGGAA
AGCACGTTATACGTGCTCGTCAAAGCAACCATAGTACGCGCCCTGTAGCGGCGCATT
AAGCGCGGCGGGTGTGGTGGTTACGCGCAGCGTGACCGCTACACTTGCCAGCGCCC
TAGCGCCCGCTCCTTTCGCTTTCTTCCCTTCCTTTCTCGCCACGTTCGCCGGCTTTCCC
CGTCAAGCTCTAAATCGGGGGCTCCCTTTAGGGTTCCGATTTAGTGCTTTACGGCAC
CTCGACCCCAAAAAACTTGATTAGGGTGATGGTTCACGTAGTGGGCCATCGCCCTGA
TAGACGGTTTTTCGCCCTTTGACGTTGGAGTCCACGTTCTTTAATAGTGGACTCTTGT
TCCAAACTGGAACAACACTCAACCCTATCTCGGTCTATTCTTTTGATTTATAAGGGAT
TTTGCCGATTTCGGCCTATTGGTTAAAAAATGAGCTGATTTAACAAAAATTTAACGC
GAATTTTAACAAAATATTAACGTTTACAATTTAAATATTTGCTTATACAATCTTCCTG
TTTTTGGGGCTTTTCTGATTATCAACCGGGGTACATATGATTGACATGCTAGTTTTAC
GATTACCGTTCATCGATTCTCTTGTTTGCTCCAGACTCTCAGGCAATGACCTGATAGC
CTTTGTAGAGACCTCTCAAAAATAGCTACCCTCTCCGGCATGAATTTATCAGCTAGA
ACGGTTGAATATCATATTGATGGTGATTTGACTGTCTCCGGCCTTTCTCACCCGTTTG
AATCTTTACCTACACATTACTCAGGCATTGCATTTAAAATATATGAGGGTTCTAAAA
ATTTTTATCCTTGCGTTGAAATAAAGGCTTCTCCCGCAAAAGTATTACAGGGTCATA

ATGTTTTTGGTACAACCGATTTAGCTTTATGCTCTGAGGCTTTATTGCTTAATTTTGCT
AATTCTTTGCCTTGCCTGTATGATTTATTGGATGTTGGAATTCCTGATGCGGTATTTT
CTCCTTACGCATCTGTGCGGTATTTCACACCGCATATGGTGCACTCTCAGTACAATCT
GCTCTGATGCCGCATAGTTAAGCCAGCCCCGACACCCGCCAACACCCGCTGACGCG
CCCTGACGGGCTTGTCTGCTCCCGGCATCCGCTTACAGACAAGCTGTGACCGTCTCC
GGGAGCTGCATGTGTCAGAGGTTTTCACCGTCATCACCGAAACGCGCGAGACGAAA
GGGCCTCGTGATACGCCTATTTTTATAGGTTAATGTCATGATAATAATGGTTTCTTAG
ACGTCAGGTGGCACTTTTCGGGGAAATGTGCGCGGAACCCCTATTTGTTTATTTTTCT
AAATACATTCAAATATGTATCCGCTCATGAGACAATAACCCTGATAAATGCTTCAAT
AATATTGAAAAAGGAAGAGTATGAGTATTCAACATTTCCGTGTCGCCCTTATTCCCT
TTTTTGCGGCATTTTGCCTTCCTGTTTTTGCTCACCCAGAAACGCTGGTGAAAGTAAA
AGATGCTGAAGATCAGTTGGGTGCACGAGTGGGTTACATCGAACTGGATCTCAACA
GCGGTAAGATCCTTGAGAGTTTTCGCCCCGAAGAACGTTTTCCAATGATGAGCACTT
TTAAAGTTCTGCTATGTGGCGCGGTATTATCCCGTATTGACGCCGGGCAAGAGCAAC
TCGGTCGCCGCATACACTATTCTCAGAATGACTTGGTTGAGTACTCACCAGTCACAG
AAAAGCATCTTACGGATGGCATGACAGTAAGAGAATTATGCAGTGCTGCCATAACC
ATGAGTGATAACACTGCGGCCAACTTACTTCTGACAACGATCGGAGGACCGAAGGA
GCTAACCGCTTTTTTGCACAACATGGGGGATCATGTAACTCGCCTTGATCGTTGGGA
ACCGGAGCTGAATGAAGCCATACCAAACGACGAGCGTGACACCACGATGCCTGTAG
CAATGGCAACAACGTTGCGCAAACTATTAACTGGCGAACTACTTACTCTAGCTTCCC
GGCAACAATTAATAGACTGGATGGAGGCGGATAAAGTTGCAGGACCACTTCTGCGC
TCGGCCCTTCCGGCTGGCTGGTTTATTGCTGATAAATCTGGAGCCGGTGAGCGTGGG
TCTCGCGGTATCATTGCAGCACTGGGGCCAGATGGTAAGCCCTCCCGTATCGTAGTT
ATCTACACGACGGGGAGTCAGGCAACTATGGATGAACGAAATAGACAGATCGCTGA
GATAGGTGCCTCACTGATTAAGCATTGGTAACTGTCAGACCAAGTTTACTCATATAT
ACTTTAGATTGATTTAAAACTTCATTTTTAATTTAAAAGGATCTAGGTGAAGATCCTT
TTTGATAATCTCATGACCAAAATCCCTTAACGTGAGTTTTCGTTCCACTGAGCGTCAG
ACCCCGTAGAAAAGATCAAAGGATCTTCTTGAGATCCTTTTTTTCTGCGCGTAATCT
GCTGCTTGCAAACAAAAAAACCACCGCTACCAGCGGTGGTTTGTTTGCCGGATCAAG
AGCTACCAACTCTTTTTCCGAAGGTAACTGGCTTCAGCAGAGCGCAGATACCAAATA
CTGTCCTTCTAGTGTAGCCGTAGTTAGGCCACCACTTCAAGAACTCTGTAGCACCGC
CTACATACCTCGCTCTGCTAATCCTGTTACCAGTGGCTGCTGCCAGTGGCGATAAGT
CGTGTCTTACCGGGTTGGACTCAAGACGATAGTTACCGGATAAGGCGCAGCGGTCG
GGCTGAACGGGGGGTTCGTGCACACAGCCCAGCTTGGAGCGAACGACCTACACCGA
ACTGAGATACCTACAGCGTGAGCTATGAGAAAGCGCCACGCTTCCCGAAGGGAGAA
AGGCGGACAGGTATCCGGTAAGCGGCAGGGTCGGAACAGGAGAGCGCACGAGGGA
GCTTCCAGGGGGAAACGCCTGGTATCTTTATAGTCCTGTCGGGTTTCGCCACCTCTG
ACTTGAGCGTCGATTTTTGTGATGCTCGTCAGGGGGGCGGAGCCTATGGAAAAACGC
CAGCAACGCGGCCTTTTTACGGTTCCTGGCCTTTTGCTGGCCTTTTGCTCACATGTTC
TTTCCTGCGTTATCCCCTGATTCTGTGGATAACCGTATTACCGCCTTTGAGTGAGCTG
ATACCGCTCGCCGCAGCCGAACGACCGAGCGCAGCGAGTCAGTGAGCGAGGAAGC

GGAAGAGCGCCCAATACGCAAACCGCCTCTCCCCGCGCGTTGGCCGATTCATTAATG
CAG

### AAV6-Mutant ; SEQ ID No. 154

AATTCCCATCATCAATAATATACCTTATTTAGGATTGAAGCCAATATGATAATGAGC
CGGTGGAGTTTGTGACGTGGCGCGGGGCGTGGGAACGGGGCGGGTGACGTAGTAGT
CTCTAGAGTCCTGTATTAGAGGTCACGTGAGTGTTTTGCGACTTTTGCGACACCATG
TGGTCACGCTGGGTATTTAAGCCCGAGTGAGCACGCAGGGTCTCCATTTTGAAGCGG
GAGGTTTGAACGCGCAGCCACCACGGGGGGTTTTACGAGATTGTGATTAAGGTCC
CCAGCGACCTTGACGGGCATCTGCCCGGCATTTCTGACAGCTTTGTGAACTGGGTGG
CCGAGAAGGAATGGGAGTTGCCGCCAGATTCTGACATGGATCTGAATCTGATTGAG
CAGGCACCCCTGACCGTGGCCGAGAAGCTGCAGCGCGACTTTCTGACGGAATGGCG
CCGTGTGAGTAAGGCCCCGGAGGCCCTTTTCTTTGTGCAATTTGAGAAGGGAGAGAG
CTACTTCCACATGCACGTGCTCGTGGAAACCACCGGGGTGAAATCCATGGTTTTGGG
ACGTTTCCTGAGTCAGATTCGCGAAAAACTGATTCAGAGAATTTACCGCGGGATCGA
GCCGACTTTGCCAAACTGGTTCGCGGTCACAAAGACCAGAAATGGCGCCGGAGGCG
GGAACAAGGTGGTGGATGAGTGCTACATCCCCAATTACTTGCTCCCCAAAACCCAG
CCTGAGCTCCAGTGGGCGTGGACTAATATGGAACAGTATTTAAGCGCCTGTTTGAAT
CTCACGGAGCGTAAACGGTTGGTGGCGCAGCATCTGACGCACGTGTCGCAGACGCA
GGAGCAGAACAAAGAGAATCAGAATCCCAATTCTGATGCGCCGGTGATCAGATCAA
AAACTTCAGCCAGGTACATGGAGCTGGTCGGGTGGCTCGTGGACAAGGGGATTACC
TCGGAGAAGCAGTGGATCCAGGAGGACCAGGCCTCATACATCTCCTTCAATGCGGC
CTCCAACTCGCGGTCCCAAATCAAGGCTGCCTTGGACAATGCGGGAAAGATTATGA
GCCTGACTAAAACCGCCCCCGACTACCTGGTGGGCCAGCAGCCCGTGGAGGACATT
TCCAGCAATCGGATTTATAAAATTTTGGAACTAAACGGGTACGATCCCCAATATGCG
GCTTCCGTCTTTCTGGGATGGGCCACGAAAAAGTTCGGCAAGAGGAACACCATCTG
GCTGTTTGGGCCTGCAACTACCGGGAAGACCAACATCGCGGAGGCCATAGCCCACA
CTGTGCCCTTCTACGGGTGCGTAAACTGGACCAATGAGAACTTTCCCTTCAACGACT
GTGTCGACAAGATGGTGATCTGGTGGGAGGAGGGGAAGATGACCGCCAAGGTCGTG
GAGTCGGCCAAAGCCATTCTCGGAGGAAGCAAGGTGCGCGTGGACCAGAAATGCAA
GTCCTCGGCCCAGATAGACCCGACTCCCGTGATCGTCACCTCCAACACCAACATGTG
CGCCGTGATTGACGGGAACTCAACGACCTTCGAACACCAGCAGCCGTTGCAAGACC
GGATGTTCAAATTTGAACTCACCCGCCGTCTGGATCATGACTTTGGGAAGGTCACCA
AGCAGGAAGTCAAAGACTTTTTCCGGTGGGCAAAGGATCACGTGGTTGAGGTGGAG
CATGAATTCTACGTCAAAAAGGGTGGAGCCAAGAAAAGACCCGCCCCCAGTGACGC
AGATATAAGTGAGCCCAAACGGGTGCGCGAGTCAGTTGCGCAGCCATCGACGTCAG
ACGCGGAAGCTTCGATCAACTACGCAGACAGGTACCAAAACAAATGTTCTCGTCAC
GTGGGCATGAATCTGATGCTGTTTCCCTGCAGACAATGCGAGAGAATGAATCAGAA
TTCAAATATCTGCTTCACTCACGGACAGAAAGACTGTTTAGAGTGCTTTCCCGTGTC

AGAATCTCAACCCGTTTCTGTCGTCAAAAAGGCGTATCAGAAACTGTGCTACATTCA
TCATATCATGGAAAGGTGCCAGACGCTTGCACTGCCTGCGATCTGGTCAATGTGGA
TTTGGATGACTGCATCTTTGAACAATAAATGATTTAAATCAGGTATGGCTGCCGATG
GTTATCTTCCAGATTGGCTCGAGGACAACCTCTCTGAGGGCATTCGCGAGTGGTGG
ACTTGAAACCTGGAGCCCCGAAACCCAAAGCCAACCAGCAAAAGCAGGACGACGG
CCGGGGTCTGGTGCTTCCTGGCTACAAGTACCTCGGACCCTTCAACGGACTCGACAA
GGGGGAGCCCGTCAACGCGGCGGATGCAGCGGCCCTCGAGCACGACAAGGCCTACG
ACCAGCAGCTCAAAGCGGGTGACAATCCGTACCTGCGGTATAACCACGCCGACGCC
GAGTTTCAGGAGCGTCTGCAAGAAGATACGTCTTTTGGGGGCAACCTCGGGCGAGC
AGTCTTCCAGGCCAAGAAGAGGGTTCTCGAACCTTTTGGTCTGGTTGAGGAAGGTGC
TAGGACGGCTCCTGGAAAGAAACGTCCGGTAGAGCAGTCGCCACAAGAGCCAGACT
CCTCCTCGGGCATTGGCAAGACAGGCCAGCAGCCCGCTAAAAAGAGACTCAATTTT
GGTCAGACTGGCGACTCAGAGTCAGTCCCCGACCCACAACCTCTCGGAGAACCTCC
AGCAACCCCCGCTGCTGTGGGACCTACTACAATGGCTTCAGGCGGTGGCGCACCAA
TGGCAGACAATAACGAAGGCGCCGACGGAGTGGGTAATGCCTCAGGAAATTGGCAT
TGCGATTCCACAT

T251A
GGCTGGGCGACAGAGTCATCACCACCAGCACCCGAACATGGGCCTTGCCCGCCTAT
AACAACCACCTCTACAAGCAAATCTCCAGTGCTTCAACGGGGGCCAGCAACGACAA
CCACTACTTCGGCTACAGCACCCCCTGGGGGTATTTTGATTTCAACAGATTCCACTG
CCATTTCTCACCACGTGACTGGCAGCGACTCATCAACAACAATTGGGGATTCCGGCC
CAAGAGACTCAACTTCAAGCTCTTCAACATCCAAGTCAAGGAGGTCACGACGAATG
ATGGCGTCACGACCATCGCTAATAACCTTACCAGCACGGTTCAAGTCTTCTCGGACT
CGGAGTACCAGTTGCCGTACGTCCTCGGCTCTGCGCACCAGGGCTGCCTCCCTCCGT
TCCCGGCGGACGTGTTCATGATTCCGCAGTACGGCTACCTAACGCTCAACAATGGCA
GCCAGGCAGTGGGACGGTCATCCTTTTACTGCCTGGAATATTTCCCATCGCAGATGC
TGAGAACGGGCAATAACTTTACCTTCAGCTACACCTTCGAGGACGTGCCTTTCCACA
GCAGCTACGCGCACAGCCAGAGCCTGGACCGGCTGATGAATCCTCTCATCGACCAG
TACCTGTATTACCTGAACAGAACTCAGAATCAGTCCGGAAGTGCCCAAAACAAGGA
CTTGCTGTTTAGCCGGGGGTCTCCAGCTGGCATGTCTGTTCAGCCCAAAAACTGGCT
ACCTGGACCCTGTTACCGGCAGCAGCGCGTTGCTAAAACAAAAACAGACAACAACA
ACAGCAACTTTACCTGGACTGGTGCTTCAAAATATAACCTTAATGGGCGTGAATCTA
TAATCAACCCTGGCACTGCTATGGCCTCACACAAAGACGACAAAGACAAGTTCTTTC
CCATGAGCGGTGTCATGATTTTTGGAAAGGAGAGCGCCGGAGCTTCAAACACTGCA
TTGGACAATGTCATGATCACAGACGAAGAGGAAATCAAAGCCACTAACCCCGTGGC
CACCGAAAGATTTGGGACTGTGGCAGTCAATCTCCAGAGCAGCAGCACAGACCCTG
CGACCGGAGATGTGCATGTTATGGGAGCCTTACCTGGAATGGTGTGGCAAGACAGA
GACGTATACCTGCAGGGTCCTATTTGGGCCAAAATTCCTCACACGGATGGACACTTT
CACCCGTCTCCTCTCATGGGCGGCTTTGGACTTAAGCACCCGCCTCCTCAGATCCTC
ATCAAAAACACGCCTGTTCCTGCGAATCCTCCGGCAGAGTTTTCGGCTACAAAGTTT
GCTGCATTCATCACCCAGTATTCCACAGGACAAGTGAGCGTGGAGATTGAATGGGA

GCTGCAGAAAGAAAACAGCAAACGCTGGAATCCCGAAGTGCAGTATACATCTAACT
ATGCAAAATCTGCCAACGTTGATTTCACTGTGGACAACAATGGACTTTATACTGAGC
CTCGCCCCATTGGCACCCGTTACCTCACCCGTCCCCTGTAATTGCTTGTTAATCAATA
AACCGTTTAATTCGTTTCAGTTGAACTTTGGTCTCTGCGTATTTCTTTCTTATCTAGTT
TCCATGCTCTAGACTACTACGTCACCCGCCCCGTTCCCACGCCCCGCGCCACGTCAC
AAACTCCACCCCCTCATTATCATATTGGCTTCAATCCAAAATAAGGTATATTATTGAT
GATGCATCGCTGGCGTAATAGCGAAGAGGCCCGCACCGATCGCCCTTCCCAACAGT
TGCGCAGCCTGAATGGCGAATGGAATTCCAGACGATTGAGCGTCAAAATGTAGGTA
TTTCCATGAGCGTTTTTCCTGTTGCAATGGCTGGCGGTAATATTGTTCTGGATATTAC
CAGCAAGGCCGATAGTTTGAGTTCTTCTACTCAGGCAAGTGATGTTATTACTAATCA
AAGAAGTATTGCGACAACGGTTAATTTGCGTGATGGACAGACTCTTTTACTCGGTGG
CCTCACTGATTATAAAAACACTTCTCAGGATTCTGGCGTACCGTTCCTGTCTAAAATC
CCTTTAATCGGCCTCCTGTTTAGCTCCCGCTCTGATTCTAACGAGGAAAGCACGTTAT
ACGTGCTCGTCAAAGCAACCATAGTACGCGCCCTGTAGCGGCGCATTAAGCGCGGC
GGGTGTGGTGGTTACGCGCAGCGTGACCGCTACACTTGCCAGCGCCCTAGCGCCCGC
TCCTTTCGCTTTCTTCCCTTCCTTTCTCGCCACGTTCGCCGGCTTTCCCCGTCAAGCTC
TAAATCGGGGGCTCCCTTTAGGGTTCCGATTTAGTGCTTTACGGCACCTCGACCCCA
AAAAACTTGATTAGGGTGATGGTTCACGTAGTGGGCCATCGCCCTGATAGACGGTTT
TTCGCCCTTTGACGTTGGAGTCCACGTTCTTTAATAGTGGACTCTTGTTCCAAACTGG
AACAACACTCAACCCTATCTCGGTCTATTCTTTTGATTTATAAGGGATTTTGCCGATT
TCGGCCTATTGGTTAAAAAATGAGCTGATTTAACAAAAATTTAACGCGAATTTTAAC
AAAATATTAACGTTTACAATTTAAATATTTGCTTATACAATCTTCCTGTTTTTGGGGC
TTTTCTGATTATCAACCGGGGTACATATGATTGACATGCTAGTTTTACGATTACCGTT
CATCGATTCTCTTGTTTGCTCCAGACTCTCAGGCAATGACCTGATAGCCTTTGTAGAG
ACCTCTCAAAAATAGCTACCCTCTCCGGCATGAATTTATCAGCTAGAACGGTTGAAT
ATCATATTGATGGTGATTTGACTGTCTCCGGCCTTTCTCACCCGTTTGAATCTTTACC
TACACATTACTCAGGCATTGCATTTAAAATATATGAGGGTTCTAAAAATTTTTATCCT
TGCGTTGAAATAAAGGCTTCTCCCGCAAAAGTATTACAGGGTCATAATGTTTTTGGT
ACAACCGATTTAGCTTTATGCTCTGAGGCTTTATTGCTTAATTTTGCTAATTCTTTGC
CTTGCCTGTATGATTTATTGGATGTTGGAATTCCTGATGCGGTATTTTCTCCTTACGC
ATCTGTGCGGTATTTCACACCGCATATGGTGCACTCTCAGTACAATCTGCTCTGATGC
CGCATAGTTAAGCCAGCCCCGACACCCGCCAACACCCGCTGACGCGCCCTGACGGG
CTTGTCTGCTCCCGGCATCCGCTTACAGACAAGCTGTGACCGTCTCCGGGAGCTGCA
TGTGTCAGAGGTTTTCACCGTCATCACCGAAACGCGCGAGACGAAAGGGCCTCGTG
ATACGCCTATTTTTATAGGTTAATGTCATGATAATAATGGTTTCTTAGACGTCAGGTG
GCACTTTTCGGGGAAATGTGCGCGGAACCCCTATTTGTTTATTTTTCTAAATACATTC
AAATATGTATCCGCTCATGAGACAATAACCCTGATAAATGCTTCAATAATATTGAAA
AAGGAAGAGTATGAGTATTCAACATTTCCGTGTCGCCCTTATTCCCTTTTTTGCGGCA
TTTTGCCTTCCTGTTTTTGCTCACCCAGAAACGCTGGTGAAAGTAAAAGATGCTGAA
GATCAGTTGGGTGCACGAGTGGGTTACATCGAACTGGATCTCAACAGCGGTAAGAT
CCTTGAGAGTTTTCGCCCCGAAGAACGTTTTCCAATGATGAGCACTTTTAAAGTTCT

GCTATGTGGCGCGGTATTATCCCGTATTGACGCCGGGCAAGAGCAACTCGGTCGCCG
CATACACTATTCTCAGAATGACTTGGTTGAGTACTCACCAGTCACAGAAAAGCATCT
TACGGATGGCATGACAGTAAGAGAATTATGCAGTGCTGCCATAACCATGAGTGATA
ACACTGCGGCCAACTTACTTCTGACAACGATCGGAGGACCGAAGGAGCTAACCGCT
TTTTTGCACAACATGGGGGATCATGTAACTCGCCTTGATCGTTGGGAACCGGAGCTG
AATGAAGCCATACCAAACGACGAGCGTGACACCACGATGCCTGTAGCAATGGCAAC
AACGTTGCGCAAACTATTAACTGGCGAACTACTTACTCTAGCTTCCCGGCAACAATT
AATAGACTGGATGGAGGCGGATAAAGTTGCAGGACCACTTCTGCGCTCGGCCCTTCC
GGCTGGCTGGTTTATTGCTGATAAATCTGGAGCCGGTGAGCGTGGGTCTCGCGGTAT
CATTGCAGCACTGGGGCCAGATGGTAAGCCCTCCCGTATCGTAGTTATCTACACGAC
GGGGAGTCAGGCAACTATGGATGAACGAAATAGACAGATCGCTGAGATAGGTGCCT
CACTGATTAAGCATTGGTAACTGTCAGACCAAGTTTACTCATATATACTTTAGATTG
ATTTAAAACTTCATTTTTAATTTAAAAGGATCTAGGTGAAGATCCTTTTTGATAATCT
CATGACCAAAATCCCTTAACGTGAGTTTTCGTTCCACTGAGCGTCAGACCCCGTAGA
AAAGATCAAAGGATCTTCTTGAGATCCTTTTTTTCTGCGCGTAATCTGCTGCTTGCAA
ACAAAAAAACCACCGCTACCAGCGGTGGTTTGTTTGCCGGATCAAGAGCTACCAAC
TCTTTTTCCGAAGGTAACTGGCTTCAGCAGAGCGCAGATACCAAATACTGTCCTTCT
AGTGTAGCCGTAGTTAGGCCACCACTTCAAGAACTCTGTAGCACCGCCTACATACCT
CGCTCTGCTAATCCTGTTACCAGTGGCTGCTGCCAGTGGCGATAAGTCGTGTCTTAC
CGGGTTGGACTCAAGACGATAGTTACCGGATAAGGCGCAGCGGTCGGGCTGAACGG
GGGGTTCGTGCACACAGCCCAGCTTGGAGCGAACGACCTACACCGAACTGAGATAC
CTACAGCGTGAGCTATGAGAAAGCGCCACGCTTCCCGAAGGGAGAAAGGCGGACA
GGTATCCGGTAAGCGGCAGGGTCGGAACAGGAGAGCGCACGAGGGAGCTTCCAGG
GGGAAACGCCTGGTATCTTTATAGTCCTGTCGGGTTTCGCCACCTCTGACTTGAGCG
TCGATTTTTGTGATGCTCGTCAGGGGGGCGGAGCCTATGGAAAAACGCCAGCAACG
CGGCCTTTTTACGGTTCCTGGCCTTTTGCTGGCCTTTTGCTCACATGTTCTTTCCTGCG
TTATCCCCTGATTCTGTGGATAACCGTATTACCGCCTTTGAGTGAGCTGATACCGCTC
GCCGCAGCCGAACGACCGAGCGCAGCGAGTCAGTGAGCGAGGAAGCGGAAGAGCG
CCCAATACGCAAACCGCCTCTCCCCGCGCGTTGGCCGATTCATTAATGCAG

| **AAV6 RESIDUE** | **NUCLEOTIDE CHANGE** |
|---|---|
| AAV6-T251A | ACC→GCC |

### AAV7-Wild Type; SEQ ID No. 145

AATTCCCATCATCAATAATATACCTTATTTAGGATTGAAGCCAATATGATAATGAGC
CGGTGGAGTTTGTGACGTGGCGCGGGGCGTGGGAACGGGGCGGGTGACGTAGTAGT

CTCTAGAGTCCTGTATTAGAGGTCACGTGAGTGTTTTGCGACTTTTGCGACACCATG
TGGTCACGCTGGGTATTTAAGCCCGAGTGAGCACGCAGGGTCTCCATTTTGAAGCGG
GAGGTTTGAACGCGCAGCCACCACGGGGGGTTTTACGAGATTGTGATTAAGGTCC
CCAGCGACCTTGACGGCATCTGCCCGGCATTTCTGACAGCTTTGTGAACTGGGTGG
CCGAGAAGGAATGGGAGTTGCCGCCAGATTCTGACATGGATCTGAATCTGATTGAG
CAGGCACCCCTGACCGTGGCCGAGAAGCTGCAGCGCGACTTTCTGACGGAATGGCG
CCGTGTGAGTAAGGCCCCGGAGGCCCTTTTCTTTGTGCAATTTGAGAAGGGAGAGAG
CTACTTCCACATGCACGTGCTCGTGGAAACCACCGGGGTGAAATCCATGGTTTTGGG
ACGTTTCCTGAGTCAGATTCGCGAAAAACTGATTCAGAGAATTTACCGCGGGATCGA
GCCGACTTTGCCAAACTGGTTCGCGGTCACAAAGACCAGAAATGGCGCCGGAGGCG
GGAACAAGGTGGTGGATGAGTGCTACATCCCCAATTACTTGCTCCCCAAAACCCAG
CCTGAGCTCCAGTGGGCGTGGACTAATATGGAACAGTATTTAAGCGCCTGTTTGAAT
CTCACGGAGCGTAAACGGTTGGTGGCGCAGCATCTGACGCACGTGTCGCAGACGCA
GGAGCAGAACAAAGAGAATCAGAATCCCAATTCTGATGCGCCGGTGATCAGATCAA
AAACTTCAGCCAGGTACATGGAGCTGGTCGGGTGGCTCGTGGACAAGGGGATTACC
TCGGAGAAGCAGTGGATCCAGGAGGACCAGGCCTCATACATCTCCTTCAATGCGGC
CTCCAACTCGCGGTCCCAAATCAAGGCTGCCTTGGACAATGCGGGAAAGATTATGA
GCCTGACTAAAACCGCCCCCGACTACCTGGTGGGCCAGCAGCCCGTGGAGGACATT
TCCAGCAATCGGATTTATAAAATTTTGGAACTAAACGGGTACGATCCCCAATATGCG
GCTTCCGTCTTTCTGGGATGGGCCACGAAAAAGTTCGGCAAGAGGAACACCATCTG
GCTGTTTGGGCCTGCAACTACCGGGAAGACCAACATCGCGGAGGCCATAGCCCACA
CTGTGCCCTTCTACGGGTGCGTAAACTGGACCAATGAGAACTTTCCCTTCAACGACT
GTGTCGACAAGATGGTGATCTGGTGGGAGGAGGGGAAGATGACCGCCAAGGTCGTG
GAGTCGGCCAAAGCCATTCTCGGAGGAAGCAAGGTGCGCGTGGACCAGAAATGCAA
GTCCTCGGCCCAGATAGACCCGACTCCCGTGATCGTCACCTCCAACACCAACATGTG
CGCCGTGATTGACGGGAACTCAACGACCTTCGAACACCAGCAGCCGTTGCAAGACC
GGATGTTCAAATTTGAACTCACCCGCCGTCTGGATCATGACTTTGGGAAGGTCACCA
AGCAGGAAGTCAAAGACTTTTTCCGGTGGGCAAAGGATCACGTGGTTGAGGTGGAG
CATGAATTCTACGTCAAAAAGGGTGGAGCCAAGAAAAGACCCGCCCCCAGTGACGC
AGATATAAGTGAGCCCAAACGGGTGCGCGAGTCAGTTGCGCAGCCATCGACGTCAG
ACGCGGAAGCTTCGATCAACTACGCAGACAGGTACCAAAACAAATGTTCTCGTCAC
GTGGGCATGAATCTGATGCTGTTTCCCTGCAGACAATGCGAGAGAATGAATCAGAA
TTCAAATATCTGCTTCACTCACGGACAGAAAGACTGTTTAGAGTGCTTTCCCGTGTC
AGAATCTCAACCCGTTTCTGTCGTCAAAAAGGCGTATCAGAAACTGTGCTACATTCA
TCATATCATGGGAAAGGTGCCAGACGCTTGCACTGCCTGCGATCTGGTCAATGTGGA
TTTGGATGACTGCATCTTTGAACAATAAATGATTTAAATCAGGTATGGCTGCCGATG
GTTATCTTCCAGATTGGCTCGAGGACAACCTCTCTGAGGGCATTCGCGAGTGGTGGG
ACCTGAAACCTGGAGCCCCGAAACCCAAAGCCAACCAGCAAAAGCAGGACAACGG
CCGGGGTCTGGTGCT
TCCTGGCTACAAGTACCTCGGACCCTTCAACGGACTCGACAAGGGGGAGCCCGTCA
ACGCGGCGGACGCA

GCGGCCCTCGAGCACGACAAGGCCTACGACCAGCAGCTCAAAGCGGGTGACAATCC
GTACCTGCGGTATA
ACCACGCCGACGCCGAGTTTCAGGAGCGTCTGCAAGAAGATACGTCATTTGGGGGC
AACCTCGGGCGAGC
AGTCTTCCAGGCCAAGAAGCGGGTTCTCGAACCTCTCGGTCTGGTTGAGGAAGGCGC
TAAGACGGCTCCT
GCAAAGAAGAGACCGGTAGAGCCGTCACCTCAGCGTTCCCCCGACTCCTCCACGGG
CATCGGCAAGAAAG
GCCAGCAGCCCGCCAGAAAGAGACTCAATTTCGGTCAGACTGGCGACTCAGAGTCA
GTCCCCGACCCTCA
ACCTCTCGGAGAACCTCCAGCAGCGCCCTCTAGTGTGGGATCTGGTACAGTGGCTGC
AGGCGGTGGCGCA
CCAATGGCAGACAATAACGAAGGTGCCGACGGAGTGGGTAATGCCTCAGGAAATTG
GCATTGCGATTCCA
CATGGCTGGGCGACAGAGTCATTACCACCAGCACCCGAACCTGGGCCCTGCCCACC
TACAACAACCACCT
CTACAAGCAAATCTCCAGTGAAACTGCAGGTAGTACCAACGACAACACCTACTTCG
GCTACAGCACCCCC
TGGGGGTATTTTGACTTTAACAGATTCCACTGCCACTTCTCACCACGTGACTGGCAG
CGACTCATCAACA
ACAACTGGGGATTCCGGCCCAAGAAGCTGCGGTTCAAGCTCTTCAACATCCAGGTC
AAGGAGGTCACGAC
GAATGACGGCGTTACGACCATCGCTAATAACCTTACCAGCACGATTCAGGTATTCTC
GGACTCGGAATAC
CAGCTGCCGTACGTCCTCGGCTCTGCGCACCAGGGCTGCCTGCCTCCGTTCCCGGCG
GACGTCTTCATGA
TTCCTCAGTACGGCTACCTGACTCTCAACAATGGCAGTCAGTCTGTGGGACGTTCCT
CCTTCTACTGCCT
GGAGTACTTCCCCTCTCAGATGCTGAGAACGGGCAACAACTTTGAGTTCAGCTACAG
CTTCGAGGACGTG
CCTTTCCACAGCAGCTACGCACACAGCCAGAGCCTGGACCGGCTGATGAATCCCCTC
ATCGACCAGTACT
TGTACTACCTGGCCAGAACACAGAGTAACCCAGGAGGCACAGCTGGCAATCGGGAA
CTGCAGTTTTACCA
GGGCGGGCCTTCAACTATGGCCGAACAAGCCAAGAATTGGTTACCTGGACCTTGCTT
CCGGCAACAAAGA
GTCTCCAAAACGCTGGATCAAAACAACAACAGCAACTTTGCTTGGACTGGTGCCAC
CAAATATCACCTGA
ACGGCAGAAACTCGTTGGTTAATCCCGGCGTCGCCATGGCAACTCACAAGGACGAC
GAGGACCGCTTTTT

CCCATCCAGCGGAGTCCTGATTTTTGGAAAAACTGGAGCAACTAACAAAACTACATT
GGAAAATGTGTTA
ATGACAAATGAAGAAGAAATTCGTCCTACTAATCCTGTAGCCACGGAAGAATACGG
GATAGTCAGCAGCA
ACTTACAAGCGGCTAATACTGCAGCCCAGACACAAGTTGTCAACAACCAGGGAGCC
TTACCTGGCATGGT
CTGGCAGAACCGGGACGTGTACCTGCAGGGTCCCATCTGGGCCAAGATTCCTCACA
CGGATGGCAACTTT
CACCCGTCTCCTTTGATGGGCGGCTTTGGACTTAAACATCCGCCTCCTCAGATCCTG
ATCAAGAACACTC
CCGTTCCCGCTAATCCTCCGGAGGTGTTTACTCCTGCCAAGTTTGCTTCGTTCATCAC
ACAGTACAGCAC
CGGACAAGTCAGCGTGGAAATCGAGTGGGAGCTGCAGAAGGAAAACAGCAAGCGC
TGGAACCCGGAGATT
CAGTACACCTCCAACTTTGAAAAGCAGACTGGTGTGGACTTTGCCGTTGACAGCCAG
GGTGTTTACTCTG
AGCCTCGCCCTATTGGCACTCGTTACCTCACCCGTAATCTGTAATTGCTTGTTAATCA
ATAAACCGTTTAATTCGTTTCAGTTGAACTTTGGTCTCTGCGTATTTCTTTCTTATCTA
GTTTCCATGCTCTAGACTACTACGTCACCCGCCCCGTTCCCACGCCCCGCGCCACGT
CACAAACTCCACCCCCTCATTATCATATTGGCTTCAATCCAAAATAAGGTATATTATT
GATGATGCATCGCTGGCGTAATAGCGAAGAGGCCCGCACCGATCGCCCTTCCCAAC
AGTTGCGCAGCCTGAATGGCGAATGGAATTCCAGACGATTGAGCGTCAAAATGTAG
GTATTTCCATGAGCGTTTTTCCTGTTGCAATGGCTGGCGGTAATATTGTTCTGGATAT
TACCAGCAAGGCCGATAGTTTGAGTTCTTCTACTCAGGCAAGTGATGTTATTACTAA
TCAAAGAAGTATTGCGACAACGGTTAATTTGCGTGATGGACAGACTCTTTTACTCGG
TGGCCTCACTGATTATAAAAACACTTCTCAGGATTCTGGCGTACCGTTCCTGTCTAA
AATCCCTTTAATCGGCCTCCTGTTTAGCTCCCGCTCTGATTCTAACGAGGAAAGCAC
GTTATACGTGCTCGTCAAAGCAACCATAGTACGCGCCCTGTAGCGGCGCATTAAGCG
CGGCGGGTGTGGTGGTTACGCGCAGCGTGACCGCTACACTTGCCAGCGCCCTAGCG
CCCGCTCCTTTCGCTTTCTTCCCTTCCTTTCTCGCCACGTTCGCCGGCTTTCCCCGTCA
AGCTCTAAATCGGGGGCTCCCTTTAGGGTTCCGATTTAGTGCTTTACGGCACCTCGA
CCCCAAAAAACTTGATTAGGGTGATGGTTCACGTAGTGGGCCATCGCCCTGATAGAC
GGTTTTTCGCCCTTTGACGTTGGAGTCCACGTTCTTTAATAGTGGACTCTTGTTCCAA
ACTGGAACAACACTCAACCCTATCTCGGTCTATTCTTTTGATTTATAAGGGATTTTGC
CGATTTCGGCCTATTGGTTAAAAAATGAGCTGATTTAACAAAAATTTAACGCGAATT
TTAACAAAATATTAACGTTTACAATTTAAATATTTGCTTATACAATCTTCCTGTTTTT
GGGGCTTTTCTGATTATCAACCGGGGTACATATGATTGACATGCTAGTTTTACGATT
ACCGTTCATCGATTCTCTTGTTTGCTCCAGACTCTCAGGCAATGACCTGATAGCCTTT
GTAGAGACCTCTCAAAAATAGCTACCCTCTCCGGCATGAATTTATCAGCTAGAACGG
TTGAATATCATATTGATGGTGATTTGACTGTCTCCGGCCTTTCTCACCCGTTTGAATC
TTTACCTACACATTACTCAGGCATTGCATTTAAAATATATGAGGGTTCTAAAAATTTT

TATCCTTGCGTTGAAATAAAGGCTTCTCCCGCAAAAGTATTACAGGGTCATAATGTT
TTTGGTACAACCGATTTAGCTTTATGCTCTGAGGCTTTATTGCTTAATTTTGCTAATT
CTTTGCCTTGCCTGTATGATTTATTGGATGTTGGAATTCCTGATGCGGTATTTTCTCCT
TACGCATCTGTGCGGTATTTCACACCGCATATGGTGCACTCTCAGTACAATCTGCTCT
GATGCCGCATAGTTAAGCCAGCCCCGACACCCGCCAACACCCGCTGACGCGCCCTG
ACGGGCTTGTCTGCTCCCGGCATCCGCTTACAGACAAGCTGTGACCGTCTCCGGGAG
CTGCATGTGTCAGAGGTTTTCACCGTCATCACCGAAACGCGCGAGACGAAAGGGCC
TCGTGATACGCCTATTTTTATAGGTTAATGTCATGATAATAATGGTTTCTTAGACGTC
AGGTGGCACTTTTCGGGGAAATGTGCGCGGAACCCCTATTTGTTTATTTTTCTAAATA
CATTCAAATATGTATCCGCTCATGAGACAATAACCCTGATAAATGCTTCAATAATAT
TGAAAAAGGAAGAGTATGAGTATTCAACATTTCCGTGTCGCCCTTATTCCCTTTTTTG
CGGCATTTTGCCTTCCTGTTTTTGCTCACCCAGAAACGCTGGTGAAAGTAAAAGATG
CTGAAGATCAGTTGGGTGCACGAGTGGGTTACATCGAACTGGATCTCAACAGCGGT
AAGATCCTTGAGAGTTTTCGCCCCGAAGAACGTTTTCCAATGATGAGCACTTTTAAA
GTTCTGCTATGTGGCGCGGTATTATCCCGTATTGACGCCGGGCAAGAGCAACTCGGT
CGCCGCATACACTATTCTCAGAATGACTTGGTTGAGTACTCACCAGTCACAGAAAAG
CATCTTACGGATGGCATGACAGTAAGAGAATTATGCAGTGCTGCCATAACCATGAGT
GATAACACTGCGGCCAACTTACTTCTGACAACGATCGGAGGACCGAAGGAGCTAAC
CGCTTTTTTGCACAACATGGGGGATCATGTAACTCGCCTTGATCGTTGGGAACCGGA
GCTGAATGAAGCCATACCAAACGACGAGCGTGACACCACGATGCCTGTAGCAATGG
CAACAACGTTGCGCAAACTATTAACTGGCGAACTACTTACTCTAGCTTCCCGGCAAC
AATTAATAGACTGGATGGAGGCGGATAAAGTTGCAGGACCACTTCTGCGCTCGGCC
CTTCCGGCTGGCTGGTTTATTGCTGATAAATCTGGAGCCGGTGAGCGTGGGTCTCGC
GGTATCATTGCAGCACTGGGGCCAGATGGTAAGCCCTCCCGTATCGTAGTTATCTAC
ACGACGGGGAGTCAGGCAACTATGGATGAACGAAATAGACAGATCGCTGAGATAG
GTGCCTCACTGATTAAGCATTGGTAACTGTCAGACCAAGTTTACTCATATATACTTTA
GATTGATTTAAAACTTCATTTTTAATTTAAAAGGATCTAGGTGAAGATCCTTTTTGAT
AATCTCATGACCAAAATCCCTTAACGTGAGTTTTCGTTCCACTGAGCGTCAGACCCC
GTAGAAAAGATCAAAGGATCTTCTTGAGATCCTTTTTTTCTGCGCGTAATCTGCTGCT
TGCAAACAAAAAAACCACCGCTACCAGCGGTGGTTTGTTTGCCGGATCAAGAGCTA
CCAACTCTTTTTCCGAAGGTAACTGGCTTCAGCAGAGCGCAGATACCAAATACTGTC
CTTCTAGTGTAGCCGTAGTTAGGCCACCACTTCAAGAACTCTGTAGCACCGCCTACA
TACCTCGCTCTGCTAATCCTGTTACCAGTGGCTGCTGCCAGTGGCGATAAGTCGTGT
CTTACCGGGTTGGACTCAAGACGATAGTTACCGGATAAGGCGCAGCGGTCGGGCTG
AACGGGGGGTTCGTGCACACAGCCCAGCTTGGAGCGAACGACCTACACCGAACTGA
GATACCTACAGCGTGAGCTATGAGAAAGCGCCACGCTTCCCGAAGGGAGAAAGGCG
GACAGGTATCCGGTAAGCGGCAGGGTCGGAACAGGAGAGCGCACGAGGGAGCTTC
CAGGGGGAAACGCCTGGTATCTTTATAGTCCTGTCGGGTTTCGCCACCTCTGACTTG
AGCGTCGATTTTTGTGATGCTCGTCAGGGGGGCGGAGCCTATGGAAAAACGCCAGC
AACGCGGCCTTTTTACGGTTCCTGGCCTTTTGCTGGCCTTTTGCTCACATGTTCTTTCC
TGCGTTATCCCCTGATTCTGTGGATAACCGTATTACCGCCTTTGAGTGAGCTGATACC

GCTCGCCGCAGCCGAACGACCGAGCGCAGCGAGTCAGTGAGCGAGGAAGCGGAAG
AGCGCCCAATACGCAAACCGCCTCTCCCCGCGCGTTGGCCGATTCATTAATGCAG

### AAV7-Mutant ; SEQ ID No. 155

AATTCCCATCATCAATAATATACCTTATTTAGGATTGAAGCCAATATGATAATGAGC
CGGTGGAGTTTGTGACGTGGCGCGGGGCGTGGAACGGGGCGGGTGACGTAGTAGT
CTCTAGAGTCCTGTATTAGAGGTCACGTGAGTGTTTTGCGACTTTTTGCGACACCATG
TGGTCACGCTGGGTATTTAAGCCCGAGTGAGCACGCAGGGTCTCCATTTTGAAGCGG
GAGGTTTGAACGCGCAGCCACCACGGGGGGTTTTACGAGATTGTGATTAAGGTCC
CCAGCGACCTTGACGGGCATCTGCCCGGCATTTCTGACAGCTTTGTGAACTGGGTGG
CCGAGAAGGAATGGGAGTTGCCGCCAGATTCTGACATGGATCTGAATCTGATTGAG
CAGGCACCCCTGACCGTGGCCGAGAAGCTGCAGCGCGACTTTCTGACGGAATGGCG
CCGTGTGAGTAAGGCCCCGGAGGCCCTTTTCTTTGTGCAATTTGAGAAGGAGAGAG
CTACTTCCACATGCACGTGCTCGTGGAAACCACCGGGGTGAAATCCATGGTTTTGGG
ACGTTTCCTGAGTCAGATTCGCGAAAAACTGATTCAGAGAATTTACCGCGGGATCGA
GCCGACTTTGCCAAACTGGTTCGCGGTCACAAAGACCAGAAATGGCGCCGGAGGCG
GGAACAAGGTGGTGGATGAGTGCTACATCCCCAATTACTTGCTCCCCAAAACCCAG
CCTGAGCTCCAGTGGGCGTGGACTAATATGGAACAGTATTTAAGCGCCTGTTTGAAT
CTCACGGAGCGTAAACGGTTGGTGGCGCAGCATCTGACGCACGTGTCGCAGACGCA
GGAGCAGAACAAAGAGAATCAGAATCCCAATTCTGATGCGCCGGTGATCAGATCAA
AAACTTCAGCCAGGTACATGGAGCTGGTCGGGTGGCTCGTGGACAAGGGGATTACC
TCGGAGAAGCAGTGGATCCAGGAGGACCAGGCCTCATACATCTCCTTCAATGCGGC
CTCCAACTCGCGGTCCCAAATCAAGGCTGCCTTGGACAATGCGGGAAAGATTATGA
GCCTGACTAAAACCGCCCCCGACTACCTGGTGGCCAGCAGCCCGTGGAGGACATT
TCCAGCAATCGGATTTATAAAATTTTGGAACTAAACGGGTACGATCCCCAATATGCG
GCTTCCGTCTTTCTGGGATGGGCCACGAAAAAGTTCGGCAAGAGGAACACCATCTG
GCTGTTTGGGCCTGCAACTACCGGGAAGACCAACATCGCGGAGGCCATAGCCCACA
CTGTGCCCTTCTACGGGTGCGTAAACTGGACCAATGAGAACTTTCCCTTCAACGACT
GTGTCGACAAGATGGTGATCTGGTGGGAGGAGGGGAAGATGACCGCCAAGGTCGTG
GAGTCGGCCAAAGCCATTCTCGGAGGAAGCAAGGTGCGCGTGGACCAGAAATGCAA
GTCCTCGGCCCAGATAGACCCGACTCCCGTGATCGTCACCTCCAACACCAACATGTG
CGCCGTGATTGACGGGAACTCAACGACCTTCGAACACCAGCAGCCGTTGCAAGACC
GGATGTTCAAATTTGAACTCACCCGCCGTCTGGATCATGACTTTGGGAAGGTCACCA
AGCAGGAAGTCAAAGACTTTTTCCGGTGGGCAAAGGATCACGTGGTTGAGGTGGAG
CATGAATTCTACGTCAAAAAGGGTGGAGCCAAGAAAAGACCCGCCCCCAGTGACGC
AGATATAAGTGAGCCCAAACGGGTGCGCGAGTCAGTTGCGCAGCCATCGACGTCAG
ACGCGGAAGCTTCGATCAACTACGCAGACAGGTACCAAAACAAATGTTCTCGTCAC
GTGGGCATGAATCTGATGCTGTTTCCCTGCAGACAATGCGAGAGAATGAATCAGAA
TTCAAATATCTGCTTCACTCACGGACAGAAAGACTGTTTAGAGTGCTTTCCCGTGTC

AGAATCTCAACCCGTTTCTGTCGTCAAAAAGGCGTATCAGAAACTGTGCTACATTCA
TCATATCATGGAAAGGTGCCAGACGCTTGCACTGCCTGCGATCTGGTCAATGTGGA
TTTGGATGACTGCATCTTTGAACAATAAATGATTTAAATCAGGTATGGCTGCCGATG
GTTATCTTCCAGATTGGCTCGAGGACAACCTCTCTGAGGGCATTCGCGAGTGGTGG
ACCTGAAACCTGGAGCCCCGAAACCCAAAGCCAACCAGCAAAAGCAGGACAACGG
CCGGGGTCTGGTGCTTCCTGGCTACAAGTACCTCGGACCCTTCAACGGACTCGACAA
GGGGGAGCCCGTCAACGCGGCGGACGCAGCGGCCCTCGAGCACGACAAGGCCTAC
GACCAGCAGCTCAAAGCGGGTGACAATCCGTACCTGCGGTATAACCACGCCGACGC
CGAGTTTCAGGAGCGTCTGCAAGAAGATACGTCATTTGGGGGCAACCTCGGGCGAG
CAGTCTTCCAGGCCAAGAAGCGGGTTCTCGAACCTCTCGGTCTGGTTGAGGAAGGCG
CTAGGACGGCTCCTGCAAAGAAGAGACCGGTAGAGCCGTCACCTCAGCGTTCCCCC
GACTCCTCCACGGGCATCGGCAAGAAAGGCCAGCAGCCCGCCAGAAAGAGACTCAA
TTTCGGTCAGACTGGCGACTCAGAGTCAGTCCCCGACCCTCAACCTCTCGGAGAACC
TCCAGCAGCGCCCTCTAGTGTGGGATCTGGTACAGTGGCTGCAGGCGGTGGCGCAC
CAATGGCAGACAATAACGAAGGTGCCGACGGAGTGGGTAATGCCTCAGGAAATTGG
CATTGCGATTCCA

T252A
CATGGCTGGGCGACAGAGTCATTACCACCAGCACCCGAACCTGGGCCCTGCCCGCC
TACAACAACCACCTCTACAAGCAAATCTCCAGTGAAACTGCAGGTAGTACCAACGA
CAACACCTACTTCGGCTACAGCACCCCCTGGGGGTATTTTGACTTTAACAGATTCCA
CTGCCACTTCTCACCACGTGACTGGCAGCGACTCATCAACAACAACTGGGGATTCCG
GCCCAAGAAGCTGCGGTTCAAGCTCTTCAACATCCAGGTCAAGGAGGTCACGACGA
ATGACGGCGTTACGACCATCGCTAATAACCTTACCAGCACGATTCAGGTATTCTCGG
ACTCGGAATACCAGCTGCCGTACGTCCTCGGCTCTGCGCACCAGGGCTGCCTGCCTC
CGTTCCCGGCGGACGTCTTCATGATTCCTCAGTACGGCTACCTGACTCTCAACAATG
GCAGTCAGTCTGTGGGACGTTCCTCCTTCTACTGCCTGGAGTACTTCCCCTCTCAGAT
GCTGAGAACGGGCAACAACTTTGAGTTCAGCTACAGCTTCGAGGACGTGCCTTTCCA
CAGCAGCTACGCACACAGCCAGAGCCTGGACCGGCTGATGAATCCCCTCATCGACC
AGTACTTGTACTACCTGGCCAGAACACAGAGTAACCCAGGAGGCACAGCTGGCAAT
CGGGAACTGCAGTTTTACCAGGGCGGGCCTTCAACTATGGCCGAACAAGCCAAGAA
TTGGTTACCTGGACCTTGCTTCCGGCAACAAAGAGTCGCCAAAACGCTGGATCAAAA
CAACAACAGCAACTTTGCTTGGACTGGTGCCACCAAATATCACCTGAACGGCAGAA
ACTCGTTGGTTAATCCCGGCGTCGCCATGGCAACTCACAAGGACGACGAGGACCGC
TTTTTCCCATCCAGCGGAGTCCTGATTTTTGGAAAAACTGGAGCAACTAACAAAACT
ACATTGGAAAATGTGTTAATGACAAATGAAGAAGAAATTCGTCCTACTAATCCTGTA
GCCACGGAAGAATACGGGATAGTCAGCAGCAACTTACAAGCGGCTAATACTGCAGC
CCAGACACAAGTTGTCAACAACCAGGGAGCCTTACCTGGCATGGTCTGGCAGAACC
GGGACGTGTACCTGCAGGGTCCCATCTGGGCCAAGATTCCTCACACGGATGGCAAC
TTTCACCCGTCTCCTTTGATGGGCGGCTTTGGACTTAAACATCCGCCTCCTCAGATCC
TGATCAAGAACACTCCCGTTCCCGCTAATCCTCCGGAGGTGTTTACTCCTGCCAAGT
TTGCTGCGTTCATCACACAGTACAGCACCGGACAAGTCAGCGTGGAAATCGAGTGG

GAGCTGCAGAAGGAAAACAGCAAGCGCTGGAACCCGGAGATTCAGTACACCTCCAA
CTTTGAAAAGCAGACTGGTGTGGACTTTGCCGTTGACAGCCAGGGTGTTTACTCTG
AGCCTCGCCCTATTGGCACTCGTTACCTCACCCGTAATCTGTAATTGCTTGTTAATCA
ATAAACCGTTTAATTCGTTTCAGTTGAACTTTGGTCTCTGCGTATTTCTTTCTTATCTA
GTTTCCATGCTCTAGACTACTACGTCACCCGCCCCGTTCCCACGCCCCGCGCCACGT
CACAAACTCCACCCCCTCATTATCATATTGGCTTCAATCCAAAATAAGGTATATTATT
GATGATGCATCGCTGGCGTAATAGCGAAGAGGCCCGCACCGATCGCCCTTCCCAAC
AGTTGCGCAGCCTGAATGGCGAATGGAATTCCAGACGATTGAGCGTCAAAATGTAG
GTATTTCCATGAGCGTTTTTCCTGTTGCAATGGCTGGCGGTAATATTGTTCTGGATAT
TACCAGCAAGGCCGATAGTTTGAGTTCTTCTACTCAGGCAAGTGATGTTATTACTAA
TCAAAGAAGTATTGCGACAACGGTTAATTTGCGTGATGGACAGACTCTTTTACTCGG
TGGCCTCACTGATTATAAAAACACTTCTCAGGATTCTGGCGTACCGTTCCTGTCTAA
AATCCCTTTAATCGGCCTCCTGTTTAGCTCCCGCTCTGATTCTAACGAGGAAAGCAC
GTTATACGTGCTCGTCAAAGCAACCATAGTACGCGCCCTGTAGCGGCGCATTAAGCG
CGGCGGGTGTGGTGGTTACGCGCAGCGTGACCGCTACACTTGCCAGCGCCCTAGCG
CCCGCTCCTTTCGCTTTCTTCCCTTCCTTTCTCGCCACGTTCGCCGGCTTTCCCCGTCA
AGCTCTAAATCGGGGGCTCCCTTTAGGGTTCCGATTTAGTGCTTTACGGCACCTCGA
CCCCAAAAAACTTGATTAGGGTGATGGTTCACGTAGTGGGCCATCGCCCTGATAGAC
GGTTTTTCGCCCTTTGACGTTGGAGTCCACGTTCTTTAATAGTGGACTCTTGTTCCAA
ACTGGAACAACACTCAACCCTATCTCGGTCTATTCTTTTGATTTATAAGGGATTTTGC
CGATTTCGGCCTATTGGTTAAAAAATGAGCTGATTTAACAAAAATTTAACGCGAATT
TTAACAAAATATTAACGTTTACAATTTAAATATTTGCTTATACAATCTTCCTGTTTTT
GGGGCTTTTCTGATTATCAACCGGGGTACATATGATTGACATGCTAGTTTTACGATT
ACCGTTCATCGATTCTCTTGTTTGCTCCAGACTCTCAGGCAATGACCTGATAGCCTTT
GTAGAGACCTCTCAAAAATAGCTACCCTCTCCGGCATGAATTTATCAGCTAGAACGG
TTGAATATCATATTGATGGTGATTTGACTGTCTCCGGCCTTTCTCACCCGTTTGAATC
TTTACCTACACATTACTCAGGCATTGCATTTAAAATATATGAGGGTTCTAAAAATTTT
TATCCTTGCGTTGAAATAAAGGCTTCTCCCGCAAAAGTATTACAGGGTCATAATGTT
TTTGGTACAACCGATTTAGCTTTATGCTCTGAGGCTTTATTGCTTAATTTTGCTAATT
CTTTGCCTTGCCTGTATGATTTATTGGATGTTGGAATTCCTGATGCGGTATTTTCTCCT
TACGCATCTGTGCGGTATTTCACACCGCATATGGTGCACTCTCAGTACAATCTGCTCT
GATGCCGCATAGTTAAGCCAGCCCCGACACCCGCCAACACCCGCTGACGCGCCCTG
ACGGGCTTGTCTGCTCCCGGCATCCGCTTACAGACAAGCTGTGACCGTCTCCGGGAG
CTGCATGTGTCAGAGGTTTTCACCGTCATCACCGAAACGCGCGAGACGAAAGGGCC
TCGTGATACGCCTATTTTTATAGGTTAATGTCATGATAATAATGGTTTCTTAGACGTC
AGGTGGCACTTTTCGGGGAAATGTGCGCGGAACCCCTATTTGTTTATTTTTCTAAATA
CATTCAAATATGTATCCGCTCATGAGACAATAACCCTGATAAATGCTTCAATAATAT
TGAAAAAGGAAGAGTATGAGTATTCAACATTTCCGTGTCGCCCTTATTCCCTTTTTTG
CGGCATTTTGCCTTCCTGTTTTTGCTCACCCAGAAACGCTGGTGAAAGTAAAAGATG
CTGAAGATCAGTTGGGTGCACGAGTGGGTTACATCGAACTGGATCTCAACAGCGGT
AAGATCCTTGAGAGTTTTCGCCCCGAAGAACGTTTTCCAATGATGAGCACTTTTAAA

GTTCTGCTATGTGGCGCGGTATTATCCCGTATTGACGCCGGGCAAGAGCAACTCGGT
CGCCGCATACACTATTCTCAGAATGACTTGGTTGAGTACTCACCAGTCACAGAAAAG
CATCTTACGGATGGCATGACAGTAAGAGAATTATGCAGTGCTGCCATAACCATGAGT
GATAACACTGCGGCCAACTTACTTCTGACAACGATCGGAGGACCGAAGGAGCTAAC
CGCTTTTTTGCACAACATGGGGGATCATGTAACTCGCCTTGATCGTTGGGAACCGGA
GCTGAATGAAGCCATACCAAACGACGAGCGTGACACCACGATGCCTGTAGCAATGG
CAACAACGTTGCGCAAACTATTAACTGGCGAACTACTTACTCTAGCTTCCCGGCAAC
AATTAATAGACTGGATGGAGGCGGATAAAGTTGCAGGACCACTTCTGCGCTCGGCC
CTTCCGGCTGGCTGGTTTATTGCTGATAAATCTGGAGCCGGTGAGCGTGGGTCTCGC
GGTATCATTGCAGCACTGGGGCCAGATGGTAAGCCCTCCCGTATCGTAGTTATCTAC
ACGACGGGGAGTCAGGCAACTATGGATGAACGAAATAGACAGATCGCTGAGATAG
GTGCCTCACTGATTAAGCATTGGTAACTGTCAGACCAAGTTTACTCATATATACTTTA
GATTGATTTAAAACTTCATTTTTAATTTAAAAGGATCTAGGTGAAGATCCTTTTTGAT
AATCTCATGACCAAAATCCCTTAACGTGAGTTTTCGTTCCACTGAGCGTCAGACCCC
GTAGAAAAGATCAAAGGATCTTCTTGAGATCCTTTTTTTCTGCGCGTAATCTGCTGCT
TGCAAACAAAAAAACCACCGCTACCAGCGGTGGTTTGTTTGCCGGATCAAGAGCTA
CCAACTCTTTTTCCGAAGGTAACTGGCTTCAGCAGAGCGCAGATACCAAATACTGTC
CTTCTAGTGTAGCCGTAGTTAGGCCACCACTTCAAGAACTCTGTAGCACCGCCTACA
TACCTCGCTCTGCTAATCCTGTTACCAGTGGCTGCTGCCAGTGGCGATAAGTCGTGT
CTTACCGGGTTGGACTCAAGACGATAGTTACCGGATAAGGCGCAGCGGTCGGGCTG
AACGGGGGGTTCGTGCACACAGCCCAGCTTGGAGCGAACGACCTACACCGAACTGA
GATACCTACAGCGTGAGCTATGAGAAAGCGCCACGCTTCCCGAAGGGAGAAAGGCG
GACAGGTATCCGGTAAGCGGCAGGGTCGGAACAGGAGAGCGCACGAGGGAGCTTC
CAGGGGGAAACGCCTGGTATCTTTATAGTCCTGTCGGGTTTCGCCACCTCTGACTTG
AGCGTCGATTTTTGTGATGCTCGTCAGGGGGGCGGAGCCTATGGAAAAACGCCAGC
AACGCGGCCTTTTTACGGTTCCTGGCCTTTTGCTGGCCTTTTGCTCACATGTTCTTTCC
TGCGTTATCCCCTGATTCTGTGGATAACCGTATTACCGCCTTTGAGTGAGCTGATACC
GCTCGCCGCAGCCGAACGACCGAGCGCAGCGAGTCAGTGAGCGAGGAAGCGGAAG
AGCGCCCAATACGCAAACCGCCTCTCCCCGCGCGTTGGCCGATTCATTAATGCAG

| **AAV7 RESIDUE** | **NUCLEOTIDE CHANGE** |
|---|---|
| AAV7-T252A | ACC→GCC |

### AAV8-Wild Type; SEQ ID No. 146

AATTCCCATCATCAATAATATACCTTATTTAGGATTGAAGCCAATATGATAATGAGC
CGGTGGAGTTTGTGACGTGGCGCGGGCGTGGGAACGGGGCGGGTGACGTAGTAGT
CTCTAGAGTCCTGTATTAGAGGTCACGTGAGTGTTTTGCGACTTTTGCGACACCATG
TGGTCACGCTGGGTATTTAAGCCCGAGTGAGCACGCAGGGTCTCCATTTTGAAGCGG
GAGGTTTGAACGCGCAGCCACCACGGGGGGTTTTACGAGATTGTGATTAAGGTCC
CCAGCGACCTTGACGGGCATCTGCCCGGCATTTCTGACAGCTTTGTGAACTGGGTGG
CCGAGAAGGAATGGGAGTTGCCGCCAGATTCTGACATGGATCTGAATCTGATTGAG
CAGGCACCCCTGACCGTGGCCGAGAAGCTGCAGCGCGACTTTCTGACGGAATGGCG
CCGTGTGAGTAAGGCCCCGGAGGCCCTTTTCTTTGTGCAATTTGAGAAGGGAGAGAG
CTACTTCCACATGCACGTGCTCGTGGAAACCACCGGGGTGAAATCCATGGTTTTGGG
ACGTTTCCTGAGTCAGATTCGCGAAAAACTGATTCAGAGAATTTACCGCGGGATCGA
GCCGACTTTGCCAAACTGGTTCGCGGTCACAAAGACCAGAAATGGCGCCGGAGGCG
GGAACAAGGTGGTGGATGAGTGCTACATCCCCAATTACTTGCTCCCCAAAACCCAG
CCTGAGCTCCAGTGGGCGTGGACTAATATGGAACAGTATTTAAGCGCCTGTTTGAAT
CTCACGGAGCGTAAACGGTTGGTGGCGCAGCATCTGACGCACGTGTCGCAGACGCA
GGAGCAGAACAAAGAGAATCAGAATCCCAATTCTGATGCGCCGGTGATCAGATCAA
AAACTTCAGCCAGGTACATGGAGCTGGTCGGGTGGCTCGTGGACAAGGGGATTACC
TCGGAGAAGCAGTGGATCCAGGAGGACCAGGCCTCATACATCTCCTTCAATGCGGC
CTCCAACTCGCGGTCCCAAATCAAGGCTGCCTTGGACAATGCGGGAAAGATTATGA
GCCTGACTAAAACCGCCCCCGACTACCTGGTGGGCCAGCAGCCCGTGGAGGACATT
TCCAGCAATCGGATTTATAAAATTTTGGAACTAAACGGGTACGATCCCCAATATGCG
GCTTCCGTCTTTCTGGGATGGGCCACGAAAAAGTTCGGCAAGAGGAACACCATCTG
GCTGTTTGGGCCTGCAACTACCGGGAAGACCAACATCGCGGAGGCCATAGCCCACA
CTGTGCCCTTCTACGGGTGCGTAAACTGGACCAATGAGAACTTTCCCTTCAACGACT
GTGTCGACAAGATGGTGATCTGGTGGGAGGAGGGGAAGATGACCGCCAAGGTCGTG
GAGTCGGCCAAAGCCATTCTCGGAGGAAGCAAGGTGCGCGTGGACCAGAAATGCAA
GTCCTCGGCCCAGATAGACCCGACTCCCGTGATCGTCACCTCCAACACCAACATGTG
CGCCGTGATTGACGGGAACTCAACGACCTTCGAACACCAGCAGCCGTTGCAAGACC
GGATGTTCAAATTTGAACTCACCCGCCGTCTGGATCATGACTTTGGGAAGGTCACCA
AGCAGGAAGTCAAAGACTTTTTCCGGTGGGCAAAGGATCACGTGGTTGAGGTGGAG
CATGAATTCTACGTCAAAAAGGGTGGAGCCAAGAAAAGACCCGCCCCCAGTGACGC
AGATATAAGTGAGCCCAAACGGGTGCGCGAGTCAGTTGCGCAGCCATCGACGTCAG
ACGCGGAAGCTTCGATCAACTACGCAGACAGGTACCAAAACAAATGTTCTCGTCAC
GTGGGCATGAATCTGATGCTGTTTCCCTGCAGACAATGCGAGAGAATGAATCAGAA
TTCAAATATCTGCTTCACTCACGGACAGAAAGACTGTTTAGAGTGCTTTCCCGTGTC
AGAATCTCAACCCGTTTCTGTCGTCAAAAAGGCGTATCAGAAACTGTGCTACATTCA
TCATATCATGGGAAAGGTGCCAGACGCTTGCACTGCCTGCGATCTGGTCAATGTGGA
TTTGGATGACTGCATCTTTGAACAATAAATGATTTAAATCAGGTATGGCTGCCGATG
GTTATCTTCCAGATTGGCTCGAGGACAACCTCTCTGAGGGCATTCGCGAGTGGTGGG

CGCTGAAACCTGGAGCCCCGAAGCCAAAGCCAACCAGCAAAAGCAGGACGACGG
CCGGGGTCTGGTGCTTCCTGGCTACAAGTACCTCGGACCCTTCAACGGACTCGACAA
GGGGGAGCCCGTCAACGCGGCGGACGCAGCGGCCCTGGAGCACGACAAGGCCTAC
GACCAGCAGCTGCAGGCGGGTGACAATCCGTACCTGCGGTATAACCACGCCGACGC
CGAGTTTCAGGAGCGTCTGCAAGAAGATACGTCTTTTGGGGGCAACCTCGGGCGAG
CAGTCTTCCAGGCCAAGAAGCGGGTTCTCGAACCTCTCGGTCTGGTTGAGGAAGGCG
CTAAGACGGCTCCTGGAAAGAAGAGACCGGTAGAGCCATCACCCCAGCGTTCTCCA
GACTCCTCTACGGGCATCGGCAAGAAAGGCCAACAGCCCGCCAGAAAAAGACTCAA
TTTTGGTCAGACTGGCGACTCAGAGTCAGTTCCAGACCCTCAACCTCTCGGAGAACC
TCCAGCAGCGCCCTCTGGTGTGGGACCTAATACAATGGCTGCAGGCGGTGGCGCAC
CAATGGCAGACAATAACGAAGGCGCCGACGGAGTGGGTAGTTCCTCGGGAAATTGG
CATTGCGATTCCACATGGCTGGGCGACAGAGTCATCACCACCAGCACCCGAACCTG
GGCCCTGCCCACCTACAACAACCACCTCTACAAGCAAATCTCCAACGGACATCGG
GAGGAGCCACCAACGACAACACCTACTTCGGCTACAGCACCCCCTGGGGGTATTTT
GACTTTAACAGATTCCACTGCCACTTTTCACCACGTGACTGGCAGCGACTCATCAAC
AACAACTGGGGATTCCGGCCCAAGAGACTCAGCTTCAAGCTCTTCAACATCCAGGTC
AAGGAGGTCACGCAGAATGAAGGCACCAAGACCATCGCCAATAACCTCACCAGCAC
CATCCAGGTGTTTACGGACTCGGAGTACCAGCTGCCGTACGTTCTCGGCTCTGCCCA
CCAGGGCTGCCTGCCTCCGTTCCCGGCGGACGTGTTCATGATTCCCCAGTACGGCTA
CCTAACACTCAACAACGGTAGTCAGGCCGTGGGACGCTCCTCCTTCTACTGCCTGGA
ATACTTTCCTTCGCAGATGCTGAGAACCGGCAACAACTTCCAGTTTACTTACACCTTC
GAGGACGTGCCTTTCCACAGCAGCTACGCCCACAGCCAGAGCTTGGACCGGCTGAT
GAATCCTCTGATTGACCAGTACCTGTACTACTTGTCTCGGACTCAAACAACAGGAGG
CACGGCAAATACGCAGACTCTGGGCTTCAGCCAAGGTGGGCCTAATACAATGGCCA
ATCAGGCAAAGAACTGGCTGCCAGGACCCTGTTACCGCCAACAACGCGTCTCAACG
ACAACCGGGCAAAACAACAATAGCAACTTTGCCTGGACTGCTGGGACCAAATACCA
TCTGAATGGAAGAAATTCATTGGCTAATCCTGGCATCGCTATGGCAACACACAAAG
ACGACGAGGAGCGTTTTTTCCCAGTAACGGGATCCTGATTTTTGGCAAACAAAATG
CTGCCAGAGACAATGCGGATTACAGCGATGTCATGCTCACCAGCGAGGAAGAAATC
AAAACCACTAACCCTGTGGCTACAGAGGAATACGGTATCGTGGCAGATAACTTGCA
GCAGCAAAACACGGCTCCTCAAATTGGAACTGTCAACAGCCAGGGGGCCTTACCCG
GTATGGTCTGGCAGAACCGGGACGTGTACCTGCAGGGTCCCATCTGGGCCAAGATTC
CTCACACGGACGGCAACTTCCACCCGTCTCCGCTGATGGGCGGCTTTGGCCTGAAAC
ATCCTCCGCCTCAGATCCTGATCAAGAACACGCCTGTACCTGCGGATCCTCCGACCA
CCTTCAACCAGTCAAAGCTGAACTCTTTCATCACGCAATACAGCACCGGACAGGTCA
GCGTGGAAATTGAATGGGAGCTGCAGAAGGAAAACAGCAAGCGCTGGAACCCCGA
GATCCAGTACACCTCCAACTACTACAAATCTACAAGTGTGGACTTTGCTGTTAATAC
AGAAGGCGTGTACTCTGAACCCCGCCCCATTGGCACCCGTTACCTCACCCGTAATCT
GTAATTGCTTGTTAATCAATAAACCGTTTAATTCGTTTCAGTTGAACTTTGGTCTCTG
CGTATTTCTTTCTTATCTAGTTTCCATGCTCTAGACTACTACGTCACCCGCCCCGTTCC

CACGCCCCGCGCCACGTCACAAACTCCACCCCCTCATTATCATATTGGCTTCAATCC
AAAATAAGGTATATTATTGATGATGCATCGCTGGCGTAATAGCGAAGAGGCCCGCA
CCGATCGCCCTTCCCAACAGTTGCGCAGCCTGAATGGCGAATGGAATTCCAGACGAT
TGAGCGTCAAAATGTAGGTATTTCCATGAGCGTTTTTCCTGTTGCAATGGCTGGCGG
TAATATTGTTCTGGATATTACCAGCAAGGCCGATAGTTTGAGTTCTTCTACTCAGGC
AAGTGATGTTATTACTAATCAAAGAAGTATTGCGACAACGGTTAATTTGCGTGATGG
ACAGACTCTTTTACTCGGTGGCCTCACTGATTATAAAAACACTTCTCAGGATTCTGG
CGTACCGTTCCTGTCTAAAATCCCTTTAATCGGCCTCCTGTTTAGCTCCCGCTCTGAT
TCTAACGAGGAAAGCACGTTATACGTGCTCGTCAAAGCAACCATAGTACGCGCCCT
GTAGCGGCGCATTAAGCGCGGCGGGTGTGGTGGTTACGCGCAGCGTGACCGCTACA
CTTGCCAGCGCCCTAGCGCCCGCTCCTTTCGCTTTCTTCCCTTCCTTTCTCGCCACGTT
CGCCGGCTTTCCCCGTCAAGCTCTAAATCGGGGGCTCCCTTTAGGGTTCCGATTTAGT
GCTTTACGGCACCTCGACCCCAAAAAACTTGATTAGGGTGATGGTTCACGTAGTGGG
CCATCGCCCTGATAGACGGTTTTTCGCCCTTTGACGTTGGAGTCCACGTTCTTTAATA
GTGGACTCTTGTTCCAAACTGGAACAACACTCAACCCTATCTCGGTCTATTCTTTTGA
TTTATAAGGGATTTTGCCGATTTCGGCCTATTGGTTAAAAAATGAGCTGATTTAACA
AAAATTTAACGCGAATTTTAACAAAATATTAACGTTTACAATTTAAATATTTGCTTAT
ACAATCTTCCTGTTTTTGGGGCTTTTCTGATTATCAACCGGGGTACATATGATTGACA
TGCTAGTTTTACGATTACCGTTCATCGATTCTCTTGTTTGCTCCAGACTCTCAGGCAA
TGACCTGATAGCCTTTGTAGAGACCTCTCAAAAATAGCTACCCTCTCCGGCATGAAT
TTATCAGCTAGAACGGTTGAATATCATATTGATGGTGATTTGACTGTCTCCGGCCTTT
CTCACCCGTTTGAATCTTTACCTACACATTACTCAGGCATTGCATTTAAAATATATGA
GGGTTCTAAAAATTTTTATCCTTGCGTTGAAATAAAGGCTTCTCCCGCAAAAGTATT
ACAGGGTCATAATGTTTTTGGTACAACCGATTTAGCTTTATGCTCTGAGGCTTTATTG
CTTAATTTTGCTAATTCTTTGCCTTGCCTGTATGATTTATTGGATGTTGGAATTCCTGA
TGCGGTATTTTCTCCTTACGCATCTGTGCGGTATTTCACACCGCATATGGTGCACTCT
CAGTACAATCTGCTCTGATGCCGCATAGTTAAGCCAGCCCCGACACCCGCCAACACC
CGCTGACGCGCCCTGACGGGCTTGTCTGCTCCCGGCATCCGCTTACAGACAAGCTGT
GACCGTCTCCGGGAGCTGCATGTGTCAGAGGTTTTCACCGTCATCACCGAAACGCGC
GAGACGAAAGGGCCTCGTGATACGCCTATTTTTATAGGTTAATGTCATGATAATAAT
GGTTTCTTAGACGTCAGGTGGCACTTTTCGGGGAAATGTGCGCGGAACCCCTATTTG
TTTATTTTTCTAAATACATTCAAATATGTATCCGCTCATGAGACAATAACCCTGATAA
ATGCTTCAATAATATTGAAAAAGGAAGAGTATGAGTATTCAACATTTCCGTGTCGCC
CTTATTCCCTTTTTTGCGGCATTTTGCCTTCCTGTTTTTGCTCACCCAGAAACGCTGGT
GAAAGTAAAAGATGCTGAAGATCAGTTGGGTGCACGAGTGGGTTACATCGAACTGG
ATCTCAACAGCGGTAAGATCCTTGAGAGTTTTCGCCCCGAAGAACGTTTTCCAATGA
TGAGCACTTTTAAAGTTCTGCTATGTGGCGCGGTATTATCCCGTATTGACGCCGGGC
AAGAGCAACTCGGTCGCCGCATACACTATTCTCAGAATGACTTGGTTGAGTACTCAC
CAGTCACAGAAAAGCATCTTACGGATGGCATGACAGTAAGAGAATTATGCAGTGCT
GCCATAACCATGAGTGATAACACTGCGGCCAACTTACTTCTGACAACGATCGGAGG

ACCGAAGGAGCTAACCGCTTTTTTGCACAACATGGGGGATCATGTAACTCGCCTTGA
TCGTTGGGAACCGGAGCTGAATGAAGCCATACCAAACGACGAGCGTGACACCACGA
TGCCTGTAGCAATGGCAACAACGTTGCGCAAACTATTAACTGGCGAACTACTTACTC
TAGCTTCCCGGCAACAATTAATAGACTGGATGGAGGCGGATAAAGTTGCAGGACCA
CTTCTGCGCTCGGCCCTTCCGGCTGGCTGGTTTATTGCTGATAAATCTGGAGCCGGTG
AGCGTGGGTCTCGCGGTATCATTGCAGCACTGGGGCCAGATGGTAAGCCCTCCCGTA
TCGTAGTTATCTACACGACGGGGAGTCAGGCAACTATGGATGAACGAAATAGACAG
ATCGCTGAGATAGGTGCCTCACTGATTAAGCATTGGTAACTGTCAGACCAAGTTTAC
TCATATATACTTTAGATTGATTTAAAACTTCATTTTTAATTTAAAAGGATCTAGGTGA
AGATCCTTTTTGATAATCTCATGACCAAAATCCCTTAACGTGAGTTTTCGTTCCACTG
AGCGTCAGACCCCGTAGAAAAGATCAAAGGATCTTCTTGAGATCCTTTTTTTCTGCG
CGTAATCTGCTGCTTGCAAACAAAAAAACCACCGCTACCAGCGGTGGTTTGTTTGCC
GGATCAAGAGCTACCAACTCTTTTTCCGAAGGTAACTGGCTTCAGCAGAGCGCAGAT
ACCAAATACTGTCCTTCTAGTGTAGCCGTAGTTAGGCCACCACTTCAAGAACTCTGT
AGCACCGCCTACATACCTCGCTCTGCTAATCCTGTTACCAGTGGCTGCTGCCAGTGG
CGATAAGTCGTGTCTTACCGGGTTGGACTCAAGACGATAGTTACCGGATAAGGCGC
AGCGGTCGGGCTGAACGGGGGGTTCGTGCACACAGCCCAGCTTGGAGCGAACGACC
TACACCGAACTGAGATACCTACAGCGTGAGCTATGAGAAAGCGCCACGCTTCCCGA
AGGGAGAAAGGCGGACAGGTATCCGGTAAGCGGCAGGGTCGGAACAGGAGAGCGC
ACGAGGGAGCTTCCAGGGGGAAACGCCTGGTATCTTTATAGTCCTGTCGGGTTTCGC
CACCTCTGACTTGAGCGTCGATTTTTGTGATGCTCGTCAGGGGGGCGGAGCCTATGG
AAAAACGCCAGCAACGCGGCCTTTTTACGGTTCCTGGCCTTTTGCTGGCCTTTTGCTC
ACATGTTCTTTCCTGCGTTATCCCCTGATTCTGTGGATAACCGTATTACCGCCTTTGA
GTGAGCTGATACCGCTCGCCGCAGCCGAACGACCGAGCGCAGCGAGTCAGTGAGCG
AGGAAGCGGAAGAGCGCCCAATACGCAAACCGCCTCTCCCCGCGCGTTGGCCGATT
CATTAATGCAG

### AAV8-Mutant ; SEQ ID No. 156

AATTCCCATCATCAATAATATACCTTATTTAGGATTGAAGCCAATATGATAATGAGC
CGGTGGAGTTTGTGACGTGGCGCGGGGCGTGGGAACGGGGCGGGTGACGTAGTAGT
CTCTAGAGTCCTGTATTAGAGGTCACGTGAGTGTTTTGCGACTTTTGCGACACCATG
TGGTCACGCTGGGTATTTAAGCCCGAGTGAGCACGCAGGGTCTCCATTTTGAAGCGG
GAGGTTTGAACGCGCAGCCACCACGGGGGGTTTTACGAGATTGTGATTAAGGTCC
CCAGCGACCTTGACGGGCATCTGCCCGGCATTTCTGACAGCTTTGTGAACTGGGTGG
CCGAGAAGGAATGGGAGTTGCCGCCAGATTCTGACATGGATCTGAATCTGATTGAG
CAGGCACCCCTGACCGTGGCCGAGAAGCTGCAGCGCGACTTTCTGACGGAATGGCG
CCGTGTGAGTAAGGCCCCGGAGGCCCTTTTCTTTGTGCAATTTGAGAAGGGAGAGAG
CTACTTCCACATGCACGTGCTCGTGGAAACCACCGGGGTGAAATCCATGGTTTTGGG
ACGTTTCCTGAGTCAGATTCGCGAAAAACTGATTCAGAGAATTTACCGCGGGATCGA
GCCGACTTTGCCAAACTGGTTCGCGGTCACAAAGACCAGAAATGGCGCCGGAGGCG

GGAACAAGGTGGTGGATGAGTGCTACATCCCCAATTACTTGCTCCCCAAAACCCAG
CCTGAGCTCCAGTGGGCGTGGACTAATATGGAACAGTATTTAAGCGCCTGTTTGAAT
CTCACGGAGCGTAAACGGTTGGTGGCGCAGCATCTGACGCACGTGTCGCAGACGCA
GGAGCAGAACAAAGAGAATCAGAATCCCAATTCTGATGCGCCGGTGATCAGATCAA
AAACTTCAGCCAGGTACATGGAGCTGGTCGGGTGGCTCGTGGACAAGGGGATTACC
TCGGAGAAGCAGTGGATCCAGGAGGACCAGGCCTCATACATCTCCTTCAATGCGGC
CTCCAACTCGCGGTCCCAAATCAAGGCTGCCTTGGACAATGCGGGAAAGATTATGA
GCCTGACTAAAACCGCCCCCGACTACCTGGTGGCCAGCAGCCCGTGGAGGACATT
TCCAGCAATCGGATTTATAAAATTTGGAACTAAACGGGTACGATCCCCAATATGCG
GCTTCCGTCTTTCTGGGATGGCCACGAAAAAGTTCGGCAAGAGGAACACCATCTG
GCTGTTTGGGCCTGCAACTACCGGGAAGACCAACATCGCGGAGGCCATAGCCCACA
CTGTGCCCTTCTACGGGTGCGTAAACTGGACCAATGAGAACTTTCCCTTCAACGACT
GTGTCGACAAGATGGTGATCTGGTGGAGGAGGGGAAGATGACCGCCAAGGTCGTG
GAGTCGGCCAAAGCCATTCTCGGAGGAAGCAAGGTGCGCGTGGACCAGAAATGCAA
GTCCTCGGCCCAGATAGACCCGACTCCCGTGATCGTCACCTCCAACACCAACATGTG
CGCCGTGATTGACGGGAACTCAACGACCTTCGAACACCAGCAGCCGTTGCAAGACC
GGATGTTCAAATTTGAACTCACCCGCCGTCTGGATCATGACTTTGGGAAGGTCACCA
AGCAGGAAGTCAAAGACTTTTTCCGGTGGGCAAAGGATCACGTGGTTGAGGTGGAG
CATGAATTCTACGTCAAAAAGGGTGGAGCCAAGAAAAGACCCGCCCCCAGTGACGC
AGATATAAGTGAGCCCAAACGGGTGCGCGAGTCAGTTGCGCAGCCATCGACGTCAG
ACGCGGAAGCTTCGATCAACTACGCAGACAGGTACCAAAACAAATGTTCTCGTCAC
GTGGGCATGAATCTGATGCTGTTTCCCTGCAGACAATGCGAGAGAATGAATCAGAA
TTCAAATATCTGCTTCACTCACGGACAGAAAGACTGTTTAGAGTGCTTTCCCGTGTC
AGAATCTCAACCCGTTTCTGTCGTCAAAAAGGCGTATCAGAAACTGTGCTACATTCA
TCATATCATGGGAAAGGTGCCAGACGCTTGCACTGCCTGCGATCTGGTCAATGTGGA
TTTGGATGACTGCATCTTTGAACAATAAATGATTTAAATCAGGTATGGCTGCCGATG
GTTATCTTCCAGATTGGCTCGAGGACAACCTCTCTGAGGGCATTCGCGAGTGGTGGG
CGCTGAAACCTGGAGCCCCGAAGCCCAAAGCCAACCAGCAAAAGCAGGACGACGG
CCGGGGTCTGGTGCTTCCTGGCTACAAGTACCTCGGACCCTTCAACGGACTCGACAA
GGGGGAGCCCGTCAACGCGGCGGACGCAGCGGCCCTGGAGCACGACAAGGCCTAC
GACCAGCAGCTGCAGGCGGGTGACAATCCGTACCTGCGGTATAACCACGCCGACGC
CGAGTTTCAGGAGCGTCTGCAAGAAGATACGTCTTTTGGGGGCAACCTCGGGCGAG
CAGTCTTCCAGGCCAAGAAGCGGGTTCTCGAACCTCTCGGTCTGGTTGAGGAAGGG
GCT

K137 T138 K143 S149

AGGGCGGCTCCTGGAAGGAGAAGACCGGTAGAGCCAGCACCCCAGCGTGCTCCAGA

S156

CGCCTCTACGGGCATCGGCAAGAAAGGCCAACAGCCCGCCAGAAAAAGACTCAATT
TTGGTCAGACTGGCGACTCAGAGTCAGTTCCAGACCCTCAACCTCTCGGAGAACCTC
CAGCAGCGCCCTCTGGTGTGGGACCTAATACAATGGCTGCAGGCGGTGGCGCACCA

ATGGCAGACAATAACGAAGGCGCCGACGGAGTGGGTAGTTCCTCGGGAAATT
GGCATTGCGATTCCACATGGCTGGGCGACAGAGTCATCACCACCAGCACCCGAACC
TGGGCCCTGCCGGCCTACAACAACCACCTCTACAAGCAAATCTCCAACGGGAC
T252A

S279
ATCGGGAGGAGCCACCAACGACAACACCTACTTCGGCTACGCCACCCCCTGGGGGT
ATTTTGACTTTAACAGATTCCACTGCCACTTTTCACCACGTGACTGGCAGCGACTCAT
CAACAACAACTGGGGATTCCGGCCCAAGAGACTCAGCTTCAAGCTCTTCAACATCC
AGGTCAAGGAGGTCACGCAGAATGAAGGCACCAAGACCATCGCCAATAACCTCACC
AGCACCATCCAGGTGTTTACGGACTCGGAGTACCAGCTGCCGTACGTTCTCGGCTCT
GCCCACCAGGGCTGCCTGCCTCCGTTCCCGGCGGACGTGTTCATGATTCCCCAGTAC
GGCTACCTAACACTCAACAACGGTAGTCAGGCCGTGGGACGCTCCTCCTTCTACTGC
CTGGAATACTTTCCTTCGCAGATGCTGAGAACCGGCAACAACTTCCAGTTTACTTAC
ACCTTCGAGGACGTGCCTTTCCACAGCAGCTACGCCCACAGCCAGAGCTTGGACCG
GCTGATGAATCCTCTGATTGACCAGTACCTGTACTACTTGTCTCGGACTCAAACAAC
AGGAGGCACGGCAAATACGCAGACTCTGGGCTTCAGCCAAGGTGGGCCTAATACAA
TGGCCAATCAGGCAAAGAACTGGCTGCCAGGACCCTGTTACCGCCAACAAC

S501
GCGTCTCAACGACAACCGGGCAAAACAACAATGCCAACTTTGCCTGGACTGCTGGG
ACCAAATACCATCTGAATGGAAGAAATTCATTGGCTAATCCTGGCATCGCTATGGCA
ACACACAAAGACGACGAGGAGCGTTTTTTCCCAGTAACGGGATCCTGATTTTTGGC
AAACAAAATGCTGCCAGAGACAATGCGGATTACAGCGATGTCATGCTCACCAGCGA
GGAAGAAATCAAAACCACTAACCCTGTGGCTACAGAGGAATACGGTATCGTGGCAG
ATAACTTGCAGCAGCAAAACACGGCTCCTCAAATTGGAACTGTCAACAGCCAGGGG
GCCTTACCCGGTATGGTCTGGCAGAACCGGGACGTGTACCTGCAGGGTCCCATCT
GGGCCAAGATTCCTCACACGGACGGCAACTTCCACCCGTCTCCGCTGATGGGCGGCT
TTGGCCTGAAACATCCTCCGCCTCAGATCCTGATCCGA
K652R

T654 S671
AACGCGCCTGTACCTGCGGATCCTCCGGCTACCTTCAACCAGGCAAAGCTGAACGCT

T674
TTCATCGCGCAATACAGCACCGGACAGGTCAGCGTGGAAATTGAATGGGAGCTGCA
GAAGGAAAACAGCAAGCGCTGGAACCCCGAGATCCAGTACACCTCCAACTACTACA
AATCTACAAGTGTGGACTTTGCTGTTAATACAGAAGGCGTGTACTCTGAACCCCGCC
CCATTGGCACCCGTTACCTCACCCGTAATCTGTAATTGCTTGTTAATCAATAAACCGT
TTAATTCGTTTCAGTTGAACTTTGGTCTCTGCGTATTTCTTTCTTATCTAGTTTCCATG
CTCTAGACTACTACGTCACCCGCCCCGTTCCCACGCCCCGCGCCACGTCACAAACTC
CACCCCCCTCATTATCATATTGGCTTCAATCCAAAATAAGGTATATTATTGATGATGC

ATCGCTGGCGTAATAGCGAAGAGGCCCGCACCGATCGCCCTTCCCAACAGTTGCGC
AGCCTGAATGGCGAATGGAATTCCAGACGATTGAGCGTCAAAATGTAGGTATTTCC
ATGAGCGTTTTTCCTGTTGCAATGGCTGGCGGTAATATTGTTCTGGATATTACCAGCA
AGGCCGATAGTTTGAGTTCTTCTACTCAGGCAAGTGATGTTATTACTAATCAAAGAA
GTATTGCGACAACGGTTAATTTGCGTGATGGACAGACTCTTTTACTCGGTGGCCTCA
CTGATTATAAAAACACTTCTCAGGATTCTGGCGTACCGTTCCTGTCTAAAATCCCTTT
AATCGGCCTCCTGTTTAGCTCCCGCTCTGATTCTAACGAGGAAAGCACGTTATACGT
GCTCGTCAAAGCAACCATAGTACGCGCCCTGTAGCGGCGCATTAAGCGCGGCGGGT
GTGGTGGTTACGCGCAGCGTGACCGCTACACTTGCCAGCGCCCTAGCGCCCGCTCCT
TTCGCTTTCTTCCCTTCCTTTCTCGCCACGTTCGCCGGCTTTCCCCGTCAAGCTCTAAA
TCGGGGGCTCCCTTTAGGGTTCCGATTTAGTGCTTTACGGCACCTCGACCCCAAAAA
ACTTGATTAGGGTGATGGTTCACGTAGTGGGCCATCGCCCTGATAGACGGTTTTTCG
CCCTTTGACGTTGGAGTCCACGTTCTTTAATAGTGGACTCTTGTTCCAAACTGGAACA
ACACTCAACCCTATCTCGGTCTATTCTTTTGATTTATAAGGGATTTTGCCGATTTCGG
CCTATTGGTTAAAAAATGAGCTGATTTAACAAAAATTTAACGCGAATTTTAACAAAA
TATTAACGTTTACAATTTAAATATTTGCTTATACAATCTTCCTGTTTTTGGGGCTTTTC
TGATTATCAACCGGGGTACATATGATTGACATGCTAGTTTTACGATTACCGTTCATC
GATTCTCTTGTTTGCTCCAGACTCTCAGGCAATGACCTGATAGCCTTTGTAGAGACCT
CTCAAAAATAGCTACCCTCTCCGGCATGAATTTATCAGCTAGAACGGTTGAATATCA
TATTGATGGTGATTTGACTGTCTCCGGCCTTTCTCACCCGTTTGAATCTTTACCTACA
CATTACTCAGGCATTGCATTTAAAATATATGAGGGTTCTAAAAATTTTTATCCTTGCG
TTGAAATAAAGGCTTCTCCCGCAAAAGTATTACAGGGTCATAATGTTTTTGGTACAA
CCGATTTAGCTTTATGCTCTGAGGCTTTATTGCTTAATTTTGCTAATTCTTTGCCTTGC
CTGTATGATTTATTGGATGTTGGAATTCCTGATGCGGTATTTTCTCCTTACGCATCTG
TGCGGTATTTCACACCGCATATGGTGCACTCTCAGTACAATCTGCTCTGATGCCGCA
TAGTTAAGCCAGCCCCGACACCCGCCAACACCCGCTGACGCGCCCTGACGGGCTTGT
CTGCTCCCGGCATCCGCTTACAGACAAGCTGTGACCGTCTCCGGGAGCTGCATGTGT
CAGAGGTTTTCACCGTCATCACCGAAACGCGCGAGACGAAAGGGCCTCGTGATACG
CCTATTTTTATAGGTTAATGTCATGATAATAATGGTTTCTTAGACGTCAGGTGGCACT
TTTCGGGGAAATGTGCGCGGAACCCCTATTTGTTTATTTTTCTAAATACATTCAAATA
TGTATCCGCTCATGAGACAATAACCCTGATAAATGCTTCAATAATATTGAAAAAGGA
AGAGTATGAGTATTCAACATTTCCGTGTCGCCCTTATTCCCTTTTTTGCGGCATTTTG
CCTTCCTGTTTTTGCTCACCCAGAAACGCTGGTGAAAGTAAAAGATGCTGAAGATCA
GTTGGGTGCACGAGTGGGTTACATCGAACTGGATCTCAACAGCGGTAAGATCCTTGA
GAGTTTTCGCCCCGAAGAACGTTTTCCAATGATGAGCACTTTTAAAGTTCTGCTATGT
GGCGCGGTATTATCCCGTATTGACGCCGGGCAAGAGCAACTCGGTCGCCGCATACA
CTATTCTCAGAATGACTTGGTTGAGTACTCACCAGTCACAGAAAAGCATCTTACGGA
TGGCATGACAGTAAGAGAATTATGCAGTGCTGCCATAACCATGAGTGATAACACTG
CGGCCAACTTACTTCTGACAACGATCGGAGGACCGAAGGAGCTAACCGCTTTTTTGC
ACAACATGGGGGATCATGTAACTCGCCTTGATCGTTGGGAACCGGAGCTGAATGAA

GCCATACCAAACGACGAGCGTGACACCACGATGCCTGTAGCAATGGCAACAACGTT
GCGCAAACTATTAACTGGCGAACTACTTACTCTAGCTTCCCGGCAACAATTAATAGA
CTGGATGGAGGCGGATAAAGTTGCAGGACCACTTCTGCGCTCGGCCCTTCCGGCTGG
CTGGTTTATTGCTGATAAATCTGGAGCCGGTGAGCGTGGGTCTCGCGGTATCATTGC
AGCACTGGGGCCAGATGGTAAGCCCTCCCGTATCGTAGTTATCTACACGACGGGGA
GTCAGGCAACTATGGATGAACGAAATAGACAGATCGCTGAGATAGGTGCCTCACTG
ATTAAGCATTGGTAACTGTCAGACCAAGTTTACTCATATATACTTTAGATTGATTTAA
AACTTCATTTTTAATTTAAAAGGATCTAGGTGAAGATCCTTTTTGATAATCTCATGAC
CAAAATCCCTTAACGTGAGTTTTCGTTCCACTGAGCGTCAGACCCCGTAGAAAAGAT
CAAAGGATCTTCTTGAGATCCTTTTTTTCTGCGCGTAATCTGCTGCTTGCAAACAAAA
AAACCACCGCTACCAGCGGTGGTTTGTTTGCCGGATCAAGAGCTACCAACTCTTTTT
CCGAAGGTAACTGGCTTCAGCAGAGCGCAGATACCAAATACTGTCCTTCTAGTGTAG
CCGTAGTTAGGCCACCACTTCAAGAACTCTGTAGCACCGCCTACATACCTCGCTCTG
CTAATCCTGTTACCAGTGGCTGCTGCCAGTGGCGATAAGTCGTGTCTTACCGGGTTG
GACTCAAGACGATAGTTACCGGATAAGGCGCAGCGGTCGGGCTGAACGGGGGGTTC
GTGCACACAGCCCAGCTTGGAGCGAACGACCTACACCGAACTGAGATACCTACAGC
GTGAGCTATGAGAAAGCGCCACGCTTCCCGAAGGGAGAAAGGCGGACAGGTATCCG
GTAAGCGGCAGGGTCGGAACAGGAGAGCGCACGAGGGAGCTTCCAGGGGGAAACG
CCTGGTATCTTTATAGTCCTGTCGGGTTTCGCCACCTCTGACTTGAGCGTCGATTTTT
GTGATGCTCGTCAGGGGGGCGGAGCCTATGGAAAAACGCCAGCAACGCGGCCTTTT
TACGGTTCCTGGCCTTTTGCTGGCCTTTTGCTCACATGTTCTTTCCTGCGTTATCCCCT
GATTCTGTGGATAACCGTATTACCGCCTTTGAGTGAGCTGATACCGCTCGCCGCAGC
CGAACGACCGAGCGCAGCGAGTCAGTGAGCGAGGAAGCGGAAGAGCGCCCAATAC
GCAAACCGCCTCTCCCCGCGCGTTGGCCGATTCATTAATGCAG

| **AAV8 RESIDUE** | **NUCLEOTIDE CHANGE** |
|---|---|
| AAV8-S149A | TCA→GCA |
| AAV8-S156A | TCC→GCC |
| AAV8-S279A | AGC→GCC |
| AAV8-S501A | AGC→GCC |
| AAV8-S671A | TCT→GCT |
| AAV8-T138A | ACG→GCG |
| AAA8- T252A | ACC→GCC |
| AAV8-T654A | ACG→GCG |
| AAV8-T674A | ACG→GCG |
| AAV8-K137R | AAG→AGG |
| AAV8-K143R | AAG→AGA |
| AAV8-K652R | AAG→CGA |
| AAV8-K137R+S671A | AAC→AGG, TCT→GCT |

### AAV9-Wild Type; SEQ ID No. 147

ACGGCGGGGTTTTACGAGATTGTGATTAAGGTCCCCAGCGACCTTGACGGGCATCTG
CCCGGCATTTCTGACAGCTTTGTGAACTGGGTGGCCGAGAAGGAATGGGAGTTGCC
GCCAGATTCTGACATGGATCTGAATCTGATTGAGCAGGCACCCCTGACCGTGGCCGA
GAAGCTGCAGCGCGACTTTCTGACGGAATGGCGCCGTGTGAGTAAGGCCCCGGAGG
CCCTTTTCTTTGTGCAATTTGAGAAGGAGAGAGCTACTTCCACATGCACGTGCTCG
TGGAAACCACCGGGGTGAAATCCATGGTTTTGGGACGTTTCCTGAGTCAGATTCGCG
AAAAACTGATTCAGAGAATTTACCGCGGGATCGAGCCGACTTTGCCAAACTGGTTCG
CGGTCACAAAGACCAGAAATGGCGCCGGAGGCGGGAACAAGGTGGTGGATGAGTG
CTACATCCCCAATTACTTGCTCCCCAAAACCCAGCCTGAGCTCCAGTGGGCGTGGAC
TAATATGGAACAGTATTTAAGCGCCTGTTTGAATCTCACGGAGCGTAAACGGTTGGT
GGCGCAGCATCTGACGCACGTGTCGCAGACGCAGGAGCAGAACAAAGAGAATCAG
AATCCCAATTCTGATGCGCCGGTGATCAGATCAAAAACTTCAGCCAGGTACATGGA
GCTGGTCGGGTGGCTCGTGGACAAGGGGATTACCTCGGAGAAGCAGTGGATCCAGG
AGGACCAGGCCTCATACATCTCCTTCAATGCGGCCTCCAACTCGCGGTCCCAAATCA
AGGCTGCCTTGGACAATGCGGGAAAGATTATGAGCCTGACTAAAACCGCCCCCGAC
TACCTGGTGGGCCAGCAGCCCGTGGAGGACATTTCCAGCAATCGGATTTATAAAATT
TTGGAACTAAACGGGTACGATCCCCAATATGCGGCTTCCGTCTTTCTGGGATGGGCC
ACGAAAAAGTTCGGCAAGAGGAACACCATCTGGCTGTTTGGGCCTGCAACTACCGG
GAAGACCAACATCGCGGAGGCCATAGCCCACACTGTGCCCTTCTACGGGTGCGTAA
ACTGGACCAATGAGAACTTTCCCTTCAACGACTGTGTCGACAAGATGGTGATCTGGT
GGGAGGAGGGGAAGATGACCGCCAAGGTCGTGGAGTCGGCCAAAGCCATTCTCGGA
GGAAGCAAGGTGCGCGTGGACCAGAAATGCAAGTCCTCGGCCCAGATAGACCCGAC
TCCCGTGATCGTCACCTCCAACACCAACATGTGCGCCGTGATTGACGGGAACTCAAC
GACCTTCGAACACCAGCAGCCGTTGCAAGACCGGATGTTCAAATTTGAACTCACCCG
CCGTCTGGATCATGACTTTGGGAAGGTCACCAAGCAGGAAGTCAAAGACTTTTTCCG
GTGGGCAAAGGATCACGTGGTTGAGGTGGAGCATGAATTCTACGTCAAAAAGGGTG
GAGCCAAGAAAAGACCCGCCCCCAGTGACGCAGATATAAGTGAGCCCAAACGGGT
GCGCGAGTCAGTTGCGCAGCCATCGACGTCAGACGCGGAAGCTTCGATCAACTACG
CAGACAGGTACCAAAACAAATGTTCTCGTCACGTGGGCATGAATCTGATGCTGTTTC

CCTGCAGACAATGCGAGAGAATGAATCAGAATTCAAATATCTGCTTCACTCACGGA
CAGAAAGACTGTTTAGAGTGCTTTCCCGTGTCAGAATCTCAACCCGTTTCTGTCGTC
AAAAAGGCGTATCAGAAACTGTGCTACATTCATCATATCATGGAAAGGTGCCAGA
CGCTTGCACTGCCTGCGATCTGGTCAATGTGGATTTGGATGACTGCATCTTTGAACA
ATAAATGATTTAAATCAGGTATGGCTGCCGATGGTTATCTTCCAGATTGGCTCGAGG
ACAACCTTAGTGAAGGAATTCGCGAGTGGTGGGCTTTGAAACCTGGAGCCCCTCAA
CCCAAGGCAAATCAACAACATCAAGACAACGCTCGAGGTCTTGTGCTTCCGGGTTA
CAAATACCTTGGACCCGGCAACGGACTCGACAAGGGGGAGCCGGTCAACGCAGCAG
ACGCGGCGGCCCTCGAGCACGACAAGGCCTACGACCAGCAGCTCAAGGCCGGAGAC
AACCCGTACCTCAAGTACAACCACGCCGACGCCGAGTTCCAGGAGCGGCTCAAAGA
AGATACGTCTTTTGGGGGCAACCTCGGGCGAGCAGTCTTCCAGGCCAAAAAGAGGC
TTCTTGAACCTCTTGGTCTGGTTGAGGAAGCGGCTAAGACGGCTCCTGGAAAGAAGA
GGCCTGTAGAGCAGTCTCCTCAGGAACCGGACTCCTCCGCGGGTATTGGCAAATCG
GGTGCACAGCCCGCTAAAAAGAGACTCAATTTCGGTCAGACTGGCGACACAGAGTC
AGTCCCAGACCCTCAACCAATCGGAGAACCTCCCGCAGCCCCCTCAGGTGTGGGAT
CTCTTACAATGGCTTCAGGTGGTGGCGCACCAGTGGCAGACAATAACGAAGGTGCC
GATGGAGTGGGTAGTTCCTCGGGAAATTGGCATTGCGATTCCCAATGGCTGGGGGA
CAGAGTCATCACCACCAGCACCCGAACCTGGGCCCTGCCCACCTACAACAATCACCT
CTACAAGCAAATCTCCAACAGCACATCTGGAGGATCTTCAAATGACAACGCCTACTT
CGGCTACAGCACCCCCTGGGGGTATTTTGACTTCAACAGATTCCACTGCCACTTCTC
ACCACGTGACTGGCAGCGACTCATCAACAACAACTGGGGATTCCGGCCTAAGCGAC
TCAACTTCAAGCTCTTCAACATTCAGGTCAAAGAGGTTACGGACAACAATGGAGTCA
AGACCATCGCCAATAACCTTACCAGCACGGTCCAGGTCTTCACGGACTCAGACTATC
AGCTCCCGTACGTGCTCGGGTCGGCTCACGAGGGCTGCCTCCCGCCGTTCCCAGCGG
ACGTTTTCATGATTCCTCAGTACGGGTATCTGACGCTTAATGATGGAAGCCAGGCCG
TGGGTCGTTCGTCCTTTTACTGCCTGGAATATTTCCCGTCGCAAATGCTAAGAACGG
GTAACAACTTCCAGTTCAGCTACGAGTTTGAGAACGTACCTTTCCATAGCAGCTACG
CTCACAGCCAAAGCCTGGACCGACTAATGAATCCACTCATCGACCAATACTTGTACT
ATCTCTCAAAGACTATTAACGGTTCTGGACAGAATCAACAAACGCTAAAATTCAGTG
TGGCCGGACCCAGCAACATGGCTGTCCAGGGAAGAAACTACATACCTGGACCCAGC
TACCGACAACAACGTGTCTCAACCACTGTGACTCAAAACAACAACAGCGAATTTGC
TTGGCCTGGAGCTTCTTCTTGGGCTCTCAATGGACGTAATAGCTTGATGAATCCTGG
ACCTGCTATGGCCAGCCACAAAGAAGGAGAGGACCGTTTCTTTCCTTTGTCTGGATC
TTTAATTTTTGGCAAACAAGGAACTGGAAGAGACAACGTGGATGCGGACAAAGTCA
TGATAACCAACGAAGAAGAAATTAAAACTACTAACCCGGTAGCAACGGAGTCCTAT
GGACAAGTGGCCACAAACCACCAGAGTGCCCAAGCACAGGCGCAGACCGGCTGGG
TTCAAAACCAAGGAATACTTCCGGGTATGGTTTGGCAGGACAGAGATGTGTACCTGC
AAGGACCCATTTGGGCCAAAATTCCTCACACGGACGGCAACTTTCACCCTTCTCCGC
TGATGGGAGGGTTTGGAATGAAGCACCCGCCTCCTCAGATCCTCATCAAAAACACA
CCTGTACCTGCGGATCCTCCAACGGCCTTCAACAAGGACAAGCTGAACTCTTTCATC

ACCCAGTATTCTACTGGCCAAGTCAGCGTGGAGATCGAGTGGGAGCTGCAGAAGGA
AAACAGCAAGCGCTGGAACCCGGAGATCCAGTACACTTCCAACTATTACAAGTCTA
ATAATGTTGAATTTGCTGTTAATACTGAAGGTGTATATAGTGAACCCCGCCCCATTG
GCACCAGATACCTGACTCGTAATCTGTAATTGCTTGTTTCGGATCTCAACGGCCTTCA
CAGGTACAAGTGAACGCCTTTATCACCCAGTATTCTACTGGAAGGTTTTCCTCGAG
TGGGAGCTGCAGAAGGAAAAAGGGCGCTCCGAACCCGGAGATCCAGTACACTTCC
AACTATTACAAGTCTAATAATGTTGAATTTGTTGTTAATACTGAAGGTGTATATAGT
GAACCCCGCCCCATTGGCACCAGATACCTGACTCGTAATCTGTAATTGCTTGTTAAT
CAATAAACCGTTTAATTCGTTTCAGTTGAACTTTGGTCTCTGCGAAGGGCGAATTCG
TTTAAACCTGCAGGACTAGAGGTCCTGTATTAGAGGTCACGTGAGTGTTTTGCGACA
TTTTGCGACACCAAGAGGTCACGCTGGGTATTTAAGCCCGAGTGAGCACGCAGGGT
CTCCATTTTGAAGCGGGAGGTTTGAACGCGCAGCCGCCAAGCCGAATTCTGCAGATA
TCCATCACACTGGCGGAAATGGGGAAGTAAAGAGCTGAGATCAGTTGGGTGCACGA
GTGGGTTACATCGAACTGGATCTCAACAGCGGTAAGATCCTTGAGAGTTTTCGCCCC
GAAGAACGTTTTCCAATGATGAGCACTTTTAAAGTTCTGCTATGTGGCGCGGTATTA
TCCCGTATTGACGCCGGGCAAGAGCAACTCGGTCGCCGCATACACTATTCTCAGAAT
GACTTGGTTGAGTACTCACCAGTCACAGAAAAGCATCTTACGGATGGCATGACAGT
AAGAGAATTATGCAGTGCTGCCATAACCATGAGTGATAACACTGCGGCCAACTTACT
TCTGACAACGATCGGAGGACCGAAGGAGCTAACCGCTTTTTTGCACAACATGGGGG
ATCATGTAACTCGCCTTGATCGTTGGGAACCGGAGCTGAATGAAGCCATACCAAAC
GACGAGCGTGACACCACGATGCCTGTAGCAATGGCAACAACGTTGCGCAAACTATT
AAAAGTCCGGCAACTATGGATGACGAAATAGAACGATCGCTGAAATAGTGCCTCAC
TGATAGCATGGTAACTGTCGACCAGTTTACCTCATATATACTTAGATGATTAACTCA
TTTATCAAGATTCTGTAAGTGAAGAATCCGCTTTTGATATCTCATGACAATTCCTTAA
CGTGATTTCGTCCAACTGAACGCTCGCATCTCGTAAGATCAGGACTCTCTGAATCTTT
CAGCGATTCGCGTTGCACCGTACAGACGGGTAGTCAGTGCATGGCACACGTTGCGC
AACTATTACTGGCGAACTACTTACTCTAGCTTCCCGGCAACAATTAATAGACTGGAT
GGAGGCGGATAAAGTTGCAGGACCACTTCTGCGCTCGGCCCTTCCGGCTGGCTGGTT
TATTGCTGATAAATCTGGAGCCGGTGAGCGTGGGTCTCGCGGTATCATTGCAGCACT
GGGGCCAGATGGTAAGCCCTCCCGTATCGTAGTTATCTACACGACGGGGAGTCA
GGGCACTATGGATGGACCAAATAGACAGATCCCCGAATAGGTGCCTCCTGATTAGC
TTGGTAATGGCCGACCAGTTACTCTATATACTTTATTGGGCGTCTCACGTCAGACCC
GTAGAAGATCAAGGATCTTCTTGAGATCCTTTTTTTCTGCGCGTAATCTGCTGCTTGC
AAACAAAAAAACCACCGCTACCAGCGGTGGTTTGTTTGCCGGATCAAGAGCTACCA
ACTCTTTTTCCGAAGGTAACTGGCTTCAGCAGAGCGCAGATACCAAATACTGTTCTT
CTAGTGTAGCCGTAGTTAGGCCACCACTTCAAGAACTCTGTAGCACCGCCTACATAC
CTCGCTCTGCTAATCCTGTTACCAGTGGCTGCTGCCAGTGGCGATAAGTCGTGTCTTA
CCGGGTTGGACTCAAGACGATAGTTACCGGATAAGGCGCAGCGGTCGGGCTGAACG
GGGGGTTCGTGCACACAGCCCAGCTTGGAGCGAACGTCCTAACACCGAATCTGAG
ATTACCTACAGCGTGAGTTACACGTGACTTGATAGCGCCACGCTTCCGAGGGAGAA

AGGCGGACAGGTATCCGGTAAGCGGCAGGGTCGGAACAGGAGAGCGCACGAGGGA
GCTTCCAGGGGGAAACGCCTGGTATCTTTATAGTCCTGTCGGGTTTCGCCACCTCTG
ACTTGAGCGTCGATTTTTGTGATGCTCGTCAGGGGGGCGGAGCCTATGGAAAAACGC
CAGCAACGCGGCCTTTTTACGGTTCCTGGCCTTTTTGATGGCCTTGGGCTACGATTCC
GACTGGAAGCGGGCAGTGAGCGCAACGCAATTAATGTGAGTTAGCTCACTCATTAG
GCACCCCAGGCTTTACACTTTATGCTTCCGGCTCGTATGTTGTGTGGAATTGTGAGCG
GATAACAATTTCACACAGGAAACAGCTATGACCATGATTACGCCAAGCGCGCAATT
AACCCTCACTAAAGGGAACAAAAGCTGGAGCTCCACCGCGGTGGAAACCTTATAAC
AAAAAGAAGGGCGGGGTGGCCTCACTGATTAAAATAACACTTTCTCAGACATTCA
GGCGTACCGTTCCTGTCTAAAATCTCTTTAATCGGCCTCCTGTTTAACTCCCGCTCTG
ATTCTAACGAGGAAACCCTTCCGATTTACGACTGGTAGCGGGCAGTGAGCGCAACG
CAATTAATGTGAGTTAGCTCACTCATTAGGCACCCCAGGCTTTACACTTTATGCTTCC
GGCTCGTATGTTGTGTGGAATTGTGAGCGGATAACAATTTCACACAGGAAACAGCTA
TGACCATGATTACGCCAAGCGCGCAATTAACCCTCACTAAAGGGAACAAAAGCTGG
AGCTCCACCGCGGTGGCGATTTTTAGGATCGATTTAGTGCTTTACGGCACCTCGAC
CCCAAAAACTTGATTAGGGTGATGGTTCACGTATTGGGCCATCGCCCTGATAGACGG
TTTTTCGCCCTTTGACGTTGGAGTCCACGTTCTTTAATAGTGGACTCTTGTTCCAAAC
TGGAACAACACTCAACCCTATCTCGGTCTATTCTTTTGATTTATAAGGGATTTTGCCG
ATTTCGGCCTATTGGTTAAAAAATGAGCTGATTTAACAAAAATTTAACGCGAATTTT
AACAAAATATTAACGCTTACAATTTAGGTGGCACTTTTCGGGGAAATGTGCGCGGAA
CCCCTATTTGTTTATTTTTCTAAATACATTCAAATATGTATCCGCTCATGAGACAATA
ACCCTGATAAATGCTTCAATAATATTGAAAAAGGAAGAGTGAGTATGAGTATTCAA
CATTTCCGTGTCGCCCTTATTCCCTTTTTTGCGGCATTTTGCCTTCCTGTTTTTGCTCA
CCCAGAAACGCTGGTGAAAGTAAAAGATGCTGAAGATCAGTTGGGTGCACGAGTGG
GTTACATCGAACTGGATCTCAACAGCGGTAAGATCCTTGAGAGTTTTCGCCCCGAAG
AACGTTTTCCAATGATGAGCACTTTTAAAGTTCTGCTATGTGGCGCGGTATTATCCCG
TATTGACGCCGGGCAAGAGCAACTCGGTCGCCGCATACACTATTCTCAGAATGACTT
GGTTGAGTACTCACCAGTCACAGAAAAGCATCTTACGGATGGCATGACAGTAAGAG
AATTATGCAGTGCTGCCATAACCATGAGTGATAACACTGCGGCCAACTTACTTCTGA
CAACGATCGGAGGACCGAAGGAGCTAACCGCTTTTTTGCACAACATGGGGGATCAT
GTAACTCGCCTTGATCGTTGGGAACCGGAGCTGAATGAAGCCATACCAAACGACGA
GCGTGACACCACGATGCCTGTAGCAATGGCAACAACGTTGCGCAAACTATTAACTG
GCGAACTACTTACTCTAGCTTCCCGGCAACAATTAATAGACTGGATGGAGGCGGATA
AAGTTGCAGGACCACTTCTGCGCTCGGCCCTTCCGGCTGGCTGGTTTATTGCTGATA
AATCTGGAGCCGGTGAGCGTGGGTCTCGCGGTATCATTGCAGCACTGGGGCCAGAT
GGTAAGCCCTCCCGTATCGTAGTTACTACACGACGGGGAGTCAGGCAACTATGGAT
GAACGAAATAGACAGATCGCTGAGATAGGTGCCTCACTGATTAAGCATTGG

### AAV9-Mutant ; SEQ ID No. 157

ACGGCGGGGTTTACGAGATTGTGATTAAGGTCCCCAGCGACCTTGACGGGCATCTG
CCCGGCATTTCTGACAGCTTTGTGAACTGGGTGGCCGAGAAGGAATGGGAGTTGCC
GCCAGATTCTGACATGGATCTGAATCTGATTGAGCAGGCACCCCTGACCGTGGCCGA
GAAGCTGCAGCGCGACTTTCTGACGGAATGGCGCCGTGTGAGTAAGGCCCCGGAGG
CCCTTTTCTTTGTGCAATTTGAGAAGGGAGAGAGCTACTTCCACATGCACGTGCTCG
TGGAAACCACCGGGGTGAAATCCATGGTTTTGGGACGTTTCCTGAGTCAGATTCGCG
AAAAACTGATTCAGAGAATTTACCGCGGGATCGAGCCGACTTTGCCAAACTGGTTCG
CGGTCACAAAGACCAGAAATGGCGCCGGAGGCGGGAACAAGGTGGTGGATGAGTG
CTACATCCCCAATTACTTGCTCCCCAAAACCCAGCCTGAGCTCCAGTGGGCGTGGAC
TAATATGGAACAGTATTTAAGCGCCTGTTTGAATCTCACGGAGCGTAAACGGTTGGT
GGCGCAGCATCTGACGCACGTGTCGCAGACGCAGGAGCAGAACAAAGAGAATCAG
AATCCCAATTCTGATGCGCCGGTGATCAGATCAAAAACTTCAGCCAGGTACATGGA
GCTGGTCGGGTGGCTCGTGGACAAGGGGATTACCTCGGAGAAGCAGTGGATCCAGG
AGGACCAGGCCTCATACATCTCCTTCAATGCGGCCTCCAACTCGCGGTCCCAAATCA
AGGCTGCCTTGGACAATGCGGGAAAGATTATGAGCCTGACTAAAACCGCCCCCGAC
TACCTGGTGGGCCAGCAGCCCGTGGAGGACATTTCCAGCAATCGGATTTATAAAATT
TTGGAACTAAACGGGTACGATCCCCAATATGCGGCTTCCGTCTTTCTGGGATGGGCC
ACGAAAAAGTTCGGCAAGAGGAACACCATCTGGCTGTTTGGGCCTGCAACTACCGG
GAAGACCAACATCGCGGAGGCCATAGCCCACACTGTGCCCTTCTACGGGTGCGTAA
ACTGGACCAATGAGAACTTTCCCTTCAACGACTGTGTCGACAAGATGGTGATCTGGT
GGGAGGAGGGGAAGATGACCGCCAAGGTCGTGGAGTCGGCCAAAGCCATTCTCGGA
GGAAGCAAGGTGCGCGTGGACCAGAAATGCAAGTCCTCGGCCCAGATAGACCCGAC
TCCCGTGATCGTCACCTCCAACACCAACATGTGCGCCGTGATTGACGGGAACTCAAC
GACCTTCGAACACCAGCAGCCGTTGCAAGACCGGATGTTCAAATTTGAACTCACCCG
CCGTCTGGATCATGACTTTGGGAAGGTCACCAAGCAGGAAGTCAAAGACTTTTTCCG
GTGGGCAAAGGATCACGTGGTTGAGGTGGAGCATGAATTCTACGTCAAAAAGGGTG
GAGCCAAGAAAAGACCCGCCCCCAGTGACGCAGATATAAGTGAGCCCAAACGGGT
GCGCGAGTCAGTTGCGCAGCCATCGACGTCAGACGCGGAAGCTTCGATCAACTACG
CAGACAGGTACCAAAACAAATGTTCTCGTCACGTGGGCATGAATCTGATGCTGTTTC
CCTGCAGACAATGCGAGAGAATGAATCAGAATTCAAATATCTGCTTCACTCACGGA
CAGAAAGACTGTTTAGAGTGCTTTCCCGTGTCAGAATCTCAACCCGTTTCTGTCGTC
AAAAAGGCGTATCAGAAACTGTGCTACATTCATCATATCATGGGAAAGGTGCCAGA
CGCTTGCACTGCCTGCGATCTGGTCAATGTGGATTTGGATGACTGCATCTTTGAACA
ATAAATGATTTAAATCAGGTATGGCTGCCGATGGTTATCTTCCAGATTGGCTCGAGG
ACAACCTTAGTGAAGGAATTCGCGAGTGGTGGGCTTTGAAACCTGGAGCCCCTCAA
CCCAAGGCAAATCAACAACATCAAGACAACGCTCGAGGTCTTGTGCTTCCGGGTTA
CAAATACCTTGGACCCGGCAACGGACTCGACAAGGGGGAGCCGGTCAACGCAGCAG
ACGCGGCGGCCCTCGAGCACGACAAGGCCTACGACCAGCAGCTCAAGGCCGGAGAC
AACCCGTACCTCAAGTACAACCACGCCGACGCCGAGTTCCAGGAGCGGCTCAAAGA
AGATACGTCTTTTGGGGGCAACCTCGGGCGAGCAGTCTTCCAGGCCAAAAAGAGGC
TTCTT

K143R
GAACCTCTTGGTCTGGTTGAGGAAGCGGCTAAGACGGCTCCTGGAAAGAGAAGGCC
TGTAGAGCAGTCTCCTCAGGAACCGGACTCCTCCGCGGGTATTGGCAAATCGGGTGC
ACAGCCCGCTAAAAAGAGACTCAATTTCGGTCAGACTGGCGACACAGAGTCAGTCC
CAGACCCTCAACCAATCGGAGAACCTCCCGCAGCCCCCTCAGGTGTGGGATCTCTTA
CAATGGCTTCAGGTGGTGGCGCACCAGTGGCAGACAATAACGAAGGTGCCGATGG

AGTGGGTAGTTCCTCGGGAAATTGGCATTGCGATTCCCAATGGCTGGGGGACAGAG
TCATCACCACCAGCACCCGAACCTGGGCCCTGCCCGCCTACAACAATCACCTCTACA
T251A

AGCAAATCTCCAACAGCACATCTGGAGGATCTTCAAATGACAACGCCTACTTCGGC

S-278A
TACGCAACCCCCTGGGGGTATTTTGACTTCAACAGATTCCACTGCCACTTCTCACCAC
GTGACTGGCAGCGACTCATCAACAACAACTGGGGATTCCGGCCTAAGCGACTCAAC
TTCAAGCTCTTCAACATTCAGGTCAAAGAGGTTACGGACAACAATGGAGTCAAGAC
CATCGCCAATAACCTTACCAGCACGGTCCAGGTCTTCACGGACTCAGACTATCAGCT
CCCGTACGTGCTCGGGTCGGCTCACGAGGGCTGCCTCCCGCCGTTCCCAGCGGACGT
TTTCATGATTCCTCAGTACGGGTATCTGACGCTTAATGATGGAAGCCAGGCCGTGGG
TCGTTCGTCCTTTTACTGCCTGGAATATTTCCCGTCGCAAATGCTAAGAACGGGTAA
CAACTTCCAGTTCAGCTACGAGTTTGAGAACGTACCTTTCCATAGCAGCTACGCTCA
CAGCCAAAGCCTGGACCGACTAATGAATCCACTCATCGACCAATACTTGTACTATCT
CTCAAAGACTATTAACGGTTCTGGACAGAATCAACAAACGCTAAAATTCAGTGTGG
CCGGACCCAGCAACATGGCTGTC

S-490
CAGGGAAGAAACTACATACCTGGACCCAGCTACCGACAACAACGTGTCGCGACCAC
TGTGACTCAAAACAACAACGCCGAATTTGCTTGGCCTGGAGCTTCTTCTTGGGCTC
S-499

TCAATGGACGTAATAGCTTGATGAATCCTGGACCTGCTATGGCCAGCCACAAAGAA
GGAGAGGACCGTTTCTTTCCTTTGTCTGGATCTTTAATTTTTGGCAAACAAGGAACTG
GAAGAGACAACGTGGATGCGGACAAAGTCATGATAACCAACGAAGAAGAAATTAA
AACTACTAACCCGGTAGCAACGGAGTCCTATGGACAAGTGGCCACAAACCACCAGA
GTGCCCAAGCACAGGCGCAGACCGGCTGGGTTCAAAACCAAGGAATACTTCCGGGT
ATGGTTTGGCAGGACAGAGATGTGTACCTGCAAGGACCCATTTGGGCCAAAATTCCT
CACACGGACGGCAACTTTCACCCTTCTCCGCTGATGGGAGGGTTTGGAATGAAGCAC
CCGCCTCCTCAGATCCTCATCAAAAACACACCTGTACCTGCGGATCCTCCAACGGCC

S-669
TTCAACAAGGACAAGCTGAACGCCTTCATCACCCAGTATTCTACTGGCCAAGTCAGC
GTGGAGATCGAGTGGGAGCTGCAGAAGGAAAACAGCAAGCGCTGGAACCCGGAGA
TCCAGTACACTTCCAACTATTACAAGTCTAATAATGTTGAATTTGCTGTTAATACTGA

AGGTGTATATAGTGAACCCCGCCCCATTGGCACCAGATACCTGACTCGTAATCTGTA
ATTGCTTGTTTCGGATCTCAACGGCCTTCACAGGTACAAGTGAACGCCTTTATCACC
CAGTATTCTACTGGAAGGTTTTCCTCGAGTGGGAGCTGCAGAAGGAAAAAGGGC
GCTCCGAACCCGGAGATCCAGTACACTTCCAACTATTACAAGTCTAATAATGTTGAA
TTTGTTGTTAATACTGAAGGTGTATATAGTGAACCCCGCCCCATTGGCACCAGATAC
CTGACTCGTAATCTGTAATTGCTTGTTAATCAATAAACCGTTTAATTCGTTTCAGTTG
AACTTTGGTCTCTGCGAAGGGCGAATTCGTTTAAACCTGCAGGACTAGAGGTCCTGT
ATTAGAGGTCACGTGAGTGTTTTGCGACATTTTGCGACACCAAGAGGTCACGCTGGG
TATTTAAGCCCGAGTGAGCACGCAGGGTCTCCATTTTGAAGCGGGAGGTTTGAACGC
GCAGCCGCCAAGCCGAATTCTGCAGATATCCATCACACTGGCGGAAATGGGGAAGT
AAAGAGCTGAGATCAGTTGGGTGCACGAGTGGGTTACATCGAACTGGATCTCAACA
GCGGTAAGATCCTTGAGAGTTTTCGCCCCGAAGAACGTTTTCCAATGATGAGCACTT
TTAAAGTTCTGCTATGTGGCGCGGTATTATCCCGTATTGACGCCGGGCAAGAGCAAC
TCGGTCGCCGCATACACTATTCTCAGAATGACTTGGTTGAGTACTCACCAGTCACAG
AAAAGCATCTTACGGATGGCATGACAGTAAGAGAATTATGCAGTGCTGCCATAACC
ATGAGTGATAACACTGCGGCCAACTTACTTCTGACAACGATCGGAGGACCGAAGGA
GCTAACCGCTTTTTTGCACAACATGGGGGATCATGTAACTCGCCTTGATCGTTGGGA
ACCGGAGCTGAATGAAGCCATACCAAACGACGAGCGTGACACCACGATGCCTGTAG
CAATGGCAACAACGTTGCGCAAACTATTAAAAGTCCGGCAACTATGGATGACGAAA
TAGAACGATCGCTGAAATAGTGCCTCACTGATAGCATGGTAACTGTCGACCAGTTTA
CCTCATATATACTTAGATGATTAACTCATTTATCAAGATTCTGTAAGTGAAGAATCC
GCTTTTGATATCTCATGACAATTCCTTAACGTGATTTCGTCCAACTGAACGCTCGCAT
CTCGTAAGATCAGGACTCTCTGAATCTTTCAGCGATTCGCGTTGCACCGTACAGACG
GGTAGTCAGTGCATGGCACACGTTGCGCAACTATTACTGGCGAACTACTTACTCTAG
CTTCCCGGCAACAATTAATAGACTGGATGGAGGCGGATAAAGTTGCAGGACCACTT
CTGCGCTCGGCCCTTCCGGCTGGCTGGTTTATTGCTGATAAATCTGGAGCCGGTGAG
CGTGGGTCTCGCGGTATCATTGCAGCACTGGGGCCAGATGGTAAGCCCTCCCGTAT
CGTAGTTATCTACACGACGGGGAGTCAGGCACTATGGATGGACCAAATAGACAG
ATCCCCGAATAGGTGCCTCCTGATTAGCTTGGTAATGGCCGACCAGTTACTCTATAT
ACTTTTATTGGGCGTCTCACGTCAGACCCGTAGAAGATCAAGGATCTTCTTGAGATC
CTTTTTTTCTGCGCGTAATCTGCTGCTTGCAAACAAAAAAACCACCGCTACCAGCGG
TGGTTTGTTTGCCGGATCAAGAGCTACCAACTCTTTTTCCGAAGGTAACTGGCTTCA
GCAGAGCGCAGATACCAAATACTGTTCTTCTAGTGTAGCCGTAGTTAGGCCACCACT
TCAAGAACTCTGTAGCACCGCCTACATACCTCGCTCTGCTAATCCTGTTACCAGTGG
CTGCTGCCAGTGGCGATAAGTCGTGTCTTACCGGGTTGGACTCAAGACGATAGTTAC
CGGATAAGGCGCAGCGGTCGGGCTGAACGGGGGGTTCGTGCACACAGCCCAGCTT
GGAGCGAACGTCCTAACACCGAATCTGAGATTACCTACAGCGTGAGTTACACGTGA
CTTGATAGCGCCACGCTTCCGAGGGAGAAAGGCGGACAGGTATCCGGTAAGCGGCA
GGGTCGGAACAGGAGAGCGCACGAGGGAGCTTCCAGGGGGAAACGCCTGGTATCTT
TATAGTCCTGTCGGGTTTCGCCACCTCTGACTTGAGCGTCGATTTTTGTGATGCTCGT
CAGGGGGGCGGAGCCTATGGAAAAACGCCAGCAACGCGGCCTTTTTACGGTTCCTG
GCCTTTTTGATGGCCTTGGGCTACGATTCCGACTGGAAGCGGGCAGTGAGCGCAACG
CAATTAATGTGAGTTAGCTCACTCATTAGGCACCCCAGGCTTTACACTTTATGCTTCC
GGCTCGTATGTTGTGTGGAATTGTGAGCGGATAACAATTTCACACAGGAAACAGCTA
TGACCATGATTACGCCAAGCGCGCAATTAACCCTCACTAAAGGGAACAAAAGCTGG
AGCTCCACCGCGGTGGAAACCTTATAACAAAAAGAAGGGCGGGGGTGGCCTCACTG

ATTAAAATAACACTTTCTCAGACATTCAGGCGTACCGTTCCTGTCTAAAATCTCTTTA
ATCGGCCTCCTGTTTAACTCCCGCTCTGATTCTAACGAGGAAACCCTTCCGATTTACG
ACTGGTAGCGGGCAGTGAGCGCAACGCAATTAATGTGAGTTAGCTCACTCATTAGG
CACCCCAGGCTTTACACTTTATGCTTCCGGCTCGTATGTTGTGTGGAATTGTGAGCGG
ATAACAATTTCACACAGGAAACAGCTATGACCATGATTACGCCAAGCGCGCAATTA
ACCCTCACTAAAGGGAACAAAAGCTGGAGCTCCACCGCGGTGGCGATTTTTAGGA
TCGATTTAGTGCTTTACGGCACCTCGACCCCAAAAACTTGATTAGGGTGATGGTTCA
CGTATTGGGCCATCGCCCTGATAGACGGTTTTTCGCCCTTTGACGTTGGAGTCCACGT
TCTTTAATAGTGGACTCTTGTTCCAAACTGGAACAACACTCAACCCTATCTCGGTCTA
TTCTTTTGATTTATAAGGGATTTTGCCGATTTCGGCCTATTGGTTAAAAAATGAGCTG
ATTTAACAAAAATTTAACGCGAATTTTAACAAAATATTAACGCTTACAATTTAGGTG
GCACTTTTCGGGGAAATGTGCGCGGAACCCCTATTTGTTTATTTTTCTAAATACATTC
AAATATGTATCCGCTCATGAGACAATAACCCTGATAAATGCTTCAATAATATTGAAA
AAGGAAGAGTGAGTATGAGTATTCAACATTTCCGTGTCGCCCTTATTCCCTTTTTTGC
GGCATTTTGCCTTCCTGTTTTTGCTCACCCAGAAACGCTGGTGAAAGTAAAAGATGC
TGAAGATCAGTTGGGTGCACGAGTGGGTTACATCGAACTGGATCTCAACAGCGGTA
AGATCCTTGAGAGTTTTCGCCCCGAAGAACGTTTTCCAATGATGAGCACTTTTAAAG
TTCTGCTATGTGGCGCGGTATTATCCCGTATTGACGCCGGGCAAGAGCAACTCGGTC
GCCGCATACACTATTCTCAGAATGACTTGGTTGAGTACTCACCAGTCACAGAAAAGC
ATCTTACGGATGGCATGACAGTAAGAGAATTATGCAGTGCTGCCATAACCATGAGT
GATAACACTGCGGCCAACTTACTTCTGACAACGATCGGAGGACCGAAGGAGCTAAC
CGCTTTTTTGCACAACATGGGGGATCATGTAACTCGCCTTGATCGTTGGGAACCGGA
GCTGAATGAAGCCATACCAAACGACGAGCGTGACACCACGATGCCTGTAGCAATGG
CAACAACGTTGCGCAAACTATTAACTGGCGAACTACTTACTCTAGCTTCCCGGCAAC
AATTAATAGACTGGATGGAGGCGGATAAAGTTGCAGGACCACTTCTGCGCTCGGCC
CTTCCGGCTGGCTGGTTTATTGCTGATAAATCTGGAGCCGGTGAGCGTGGGTCTCGC
GGTATCATTGCAGCACTGGGGCCAGATGGTAAGCCCTCCCGTATCGTAGTTATCTAC
ACGACGGGGAGTCAGGCAACTATGGATGAACGAAATAGACAGATCGCTGAGATAG
GTGCCTCACTGATTAAGCATTGG

| AAV9 RESIDUE | NUCLEOTIDE CHANGE |
|---|---|
| AAV9-S278A | AGC→GCA |
| AAV9-S490A | TCA→GCG |
| AAV9-S669A | TCT→GCC |
| AAV9-S499A | AGC→GCC |
| AAV9-T251A | ACC→GCC |
| AAV9-K143R | AAG→AGA |

### AAV10-Wild Type; SEQ ID No. 148

GGCATTTCTGACAGCTTTGTGAACTGGGTGGCCGAGAAGGAATGGGAGTTGCCGCC
AGATTCTGACATGGATCTGAATCTGATTGAGCAGGCACCCCTGACCGTGGCCGAGA
AGCTGCAGCGCGACTTTCTGACGGAATGGCGCCGTGTGAGTAAGGCCCCGGAGGCT
CTTTTCTTTGTGCAATTTGAGAAGGGAGAGAGCTACTTCCACATGCACGTGCTCGTG
GAAACCACCGGGGTGAAATCCATGGTTTTGGGACGTTTCCTGAGTCAGATTCGCGAA
AAACTGATTCAGAGAATTTACCGCGGGATCGAGCCGACTTTGCCAAACTGGTTCGCG
GTCACAAAGACCAGAAATGGCGCCGGAGGCGGGAACAAGGTGGTGGATGAGTGCT
ACATCCCCAATTACTTGCTCCCCAAAACCCAGCCTGAGCTCCAGTGGGCGTGGACTA
ATATGGAACAGTATTTAAGCGCCTGTTTGAATCTCACGGAGCGTAACGGGTGGTGGC
GCAGCATCTGACGCACGTGTCGCAGACGCAAAACCGCCCCCGACTACCTGGTGGGC
CAGCAGCCCGTGGAGGACATTTCCAGCAATCGGATTTATAAAATTTTGGAACTAAAC
GGGTACGATCCCCAATATGCGGCTTCCGTCTTTCTGGGATGGGCCACGAAAAAGTTC
GGCAAGAGGAACACCATCTGGCTGTTTGGGCCTGCAACTACCGGGAAGACCAACAT
CGCGGAGGCCATAGCCCACACTGTGCCCTTCTACGGGTGCGTAAACTGGACCAATG
AGAACTTTCCCTTCAACGACTGTGTCGACAAGATGGTGATCTGGTGGGAGGAGGGG
AAGATGACCGCCAAGGTCGTGGAGTCGGCCAAAGCCATTCTCGGAGGAAAGCAAA
GGTGCGCGTGGACCAGAAATGCAAGTCCTCTCACTCACGGACAGAAAGACTGTTTA
GAGTGCTTTCCCGTGTCAGAATCTCAACCCGTTTCTGTCGTCAAAAAGGCGTATCAA
AAACTGTGCTACATTCATCATATCATGGAAAGGTGCCAGACGCTTGCACTGCCTG
CGATCTGGTCAATGTGGATTTGGATGACTGCATCTTTGAACAATAAATGATTTAAAT
CAGGTATGGCTGCCGATGGTTATCGCACTGCCTGCGATCTGGTCAATGTGGATTTGG
ATGACTGCATCTTTGAACAATAAATGATTTAAATCAGGTATGGCTGCCGATGGTTAT
CTTCCAGATTGGCTCGAGGACAACCTCTCTGAGGGCATTCGCGAGTGGTGGGACTTG
AAACCTGGAGCCCCGAAACCCAAAGCCAACCAGCAAAAGCAGGACGACGGCCGGG
GTCTGGTGCTTCCTGGCTACAAGTACCTCGGACCCTTCAACGGACTCGACAAGGGGG
AGCCCGTCAACGCGGCGGACGCAGCGGCCCTCGAGCACGACAAGGCCTACGACCAG
CAGCTCAAAGCGGGTGACAATCCGTACCTGCGGTATAACCACGCCGACGCCGAGTT
TCAGGAGCGTCTGCAAGAAGATACGTCTTTTGGGGGCAACCTCGGGCGAGCAGTCTT
CCAGGCCAAGAAGCGGGTTCTCGAACCTCTCGGTCTGGTTGAGGAAGGCGCTAAGA
CGGCTCCTGGAAAGAAGAGACCGGTAGAGCCATCACCCCAGCGTTCTCCAGACTCC
TCAATGTGGATTTGGATGACTGCATCTTTGAACAATATATGATTTAAATCAGGTATG
GCTGCCGATGGTTATCTTCCAGATTGGCTCGAGGACAACCTCTCTGAGGGCATTCGC
GAGTGGTGGGACTTGAAACCTGGAGCCCCGAAACCCAAAGCCAACCAGCAAAAGC
AGGACGACGGCCGGGGTCTGGTGCTTCCTGGCTACAAGTACCTCGGACCCTTCAACG
GACTCGACAAGGGGGAGCCCGTCAACGCGGCGGACGCAGCGGCCCTCGAGCACGA
CAAGGCCTACGACCAGCAGCTCAAAGCGGGTGACAATCCGTACCTGCGGTATAACC
ACGCCGACGCCGAGTTTCAGGAGCGTCTGCAAGAAGATACGTCTTTTGGGGGCAAC
CTCGGGCGAGCAGTCTTCCAGGCCAAGAAGCGGGTTCTCGAACCTCTCGGTCTGGTT
GAGGAAGGCGCTAAGACGGCTCCTGGAAAGAAGAGACCGGTAGAGCCATCACCCC
AGCGTTCTCCAGACTCCTCTACGGGCATCGGCAAGAAAGGCCAGCAGCCCGCGAAA
AAGAGACTCAACTTTGGGCAGACTGGCGACTCAGAGTCAGTGCCCGACCCTCAACC
AATCGGAGAACCCCCCGCAGCCCCTCTGGTCTGGGATCTGGTACAATGGCTGCAGG
CGGTGGCGCTCCAATGGCAGACAATCCCAACCCCAATTTTCAGAACCTCCGGAAA
AAAAATCCTCTTTTGGGGGCCACCCCCGGCCAACCATCCTTCCAGCCCAAAAACCGG

TTCCCCAACCCTTTCGTCTTGGTTGAGAAAGGCCCTAAAACGCTCCCGGAAAAAAAA
AAACCGGAAAACCCTCCCCCCACCTTCCCCAAATCCTCCAACGGCCTCCGCAAAA
AAAGGCCACCACCCCCCAAAAAAAAAATCCACTTTGGGCAAAACGGCCAATCCAAA
TCCGTGGCCCAACCTCCACCCATCCGAAAACCCCCCCCAGCCCCTCTGGCCGGGA
TTTGGTAACATGGCTGGCGGGGGGGCCCCCCCATGGAACCCTCCCCACCTACAA
CAACCACCAAAATAAACAAAGCCCCCAACGGAGGGGTTATTCCCCCAGAAATTGGG
GAAGCACCAACGACAACACCTACTTCGGCTACAGCACCCCCTGGGGGTATTTTGACT
TTAACAGATTCCACTGCCACTTCTCACCACGTGACTGGCAGCGACTCATCAACAACA
ACTGGGGATTCCGGCCCAAAAAACTCAACTTCAAGCTCTTCAACATCCACGTCAAGG
AGGTCACGCAAAATGAAGGCACCAAGACCATCGCCAATAACCTTACCAGCACGAAT
CAGGTCTTTACCGACTCGGAATACCAGCTCCCGTACCTCCTCGGCTCTGCGCACCAG
GGCTGCCTGCCTGATGAACCCCCTCATCGACCAGTACCTGTACTACCTGTCTCGGAC
TCAGTCCACGGGAGGTACCGCAGGAACTCAGCAGTTGCTATTTTCTCAGGCCGGGCC
TAATAACATGTCGGCTCAGGCCAAAAACTGGCTACCCGGGCCCTGCTACCGGCAGC
AACGCGTCTCCACGACACTGTCGCAAAATAACAACAGCAACTTTGCCTGGACCGGT
GCCACCAAGTATCATCTGAATGGCAGAAACTCTCTGGTAAATCCCGGTGTCGCTATG
GCAACCCACAAGGACGACGAAGAGCGATTTTTTCCGTCCAGCGGAGTCTTAATGTTT
GGGAAACAGGGAGCTGGAAAAGACAACGTGGACTATAGCATCGTTATGCTAACCAG
TGAGGAAGAAATTAAAACCACCAACCCAGTGGCCACAGAACAGTACGGCGTGGTGG
CCGATAACCTGCAACAGCAAAACGCCGCTCCTATTGTAGGGGCCGTCAACAGTCAA
GGAGCCTTACCTGTCATGGTCTGGCAGAACCGGGACGTGTACCTGCAGGGTCCTATC
TGGGCCAAGATTCCTCACACGGACGGAAACTTTCATCCCTCGCCGCTGATGGGAGGC
TTTGGACTGAAACACCCGCCTCCTCCATCCTGATTAAAAATACACCTGTTCCCGTGG
ATCCTCCAACTACCTTCAGTCCAGCTAAGCTGGCGTCGTTCATCACGCAGTACAGCA
CCGGACAGGTCAGCGTGGAAATTGAATGGGAGCTGCAAAAAAAAAACAGCAAACG
CTGGAACCCAAAAATTCAATACTCTTCCAACTACTACAAATCTACAAATGTGGACTT
TGCTGTTAACACAGATGGCACTTATTGGTACGATCAGACTGGAGCGGGCAGTGAGC
GCACGCAATTAATGTGAGTTAGCTCACTCATTAGGCACCCCAGGCTTTACACTTTAT
GCTTCCGGCTCGTATGTTGTGTGGAATTGTGAGCGGATAACAATTTCACACAGGAAA
CAGCTATGACCATGATTACGCCAAGCGCGCAATTAACCCTCACTAAAGGGAACAAA
AGCTGGAGCTCCACCGCGGTGGTGCTTTACGGCACCTCGACCCCAAAAAACTTGATT
AGGGTGATGGTTCACGAAAAGGGCCATCGCCCTGATAGACGGTTTTTCGCCCTTTGA
CGTTGGAGTCCACGTTCTTTAATAGTGGACTCTTGTTCCAAACTGGAACAACACTCA
ACCCTATCTCGGTCTATTCTTTTGATTTATAAGGGATTTTGCCGATTTCGGCCTATTG
GTTAAAAAATGAGCTGATTTAACAAAAATTTAACGCGAATTTTAACAAAATATTAAC
GCTTACAATTTAGGTGGCACTTTTCGGGGAAATGTGCGCGGAACCCCTATTTGTTTA
TTTTTCTAAATACATTCAAATATGTATCCGCTCATGAGACAATAACCCTGATAAATG
CTTCAATAATATTGAAAAAGGAAGAGTATGAGTATTCAACATTTCCGTGTCGCCCTT
ATTCCCTTTTTTGCGGCATTTTGCCTTCCTGTTTTTGCTCACCCAGAAACGCTGGTGA
AAGTAAAAGATGCTGAAGATCAGTTGGGTGCACGAGTGGGTTACATCGAACTGGAT
CTCAACAGCGGTAAGATCCTTGAGAGTTTTCGCCCCGAAGAACGTTTTCCAATGATG
AGCACTTTTAAAGTTCTGCTATGTGGCGCGGTATTATCCCGTATTGACGCCGGGCA
AGAGCAACTCGGTCGCCGCATACACTATTCTCAGATGACTTGGTTGAGTACTCCCCA
GTCACAGAAAAGCATCTTACGATGGCATGACAGTAAGGAGATTATGCATGCTGCCA
TACCATGATGAATAACACTGCGCACTTACTTCTGACACGATCGGAGGACCGAAGGA
GCTCGGCGCTTCTGGGGCTTTTTTGAAGGCTAAAAAGGGCCCAAGGGGGGGGG

GGGGGGCAAGCACAACCCCCCGTACGTCGGGGGGGGAGAAGGAGATTTCCTTTA
GCAAAGAGAACGGTGCCCCCCCGGCGAGGGAACGGGCCCCTGGTTTCCCCCGCC
CAACGGAGAAACCATACACCCACTACTATGCACAACATCTATATGTAATCATAAAA
CCATAGATCATCGCTATAGCGCGACGCGCCCTCTCTGCATACTGTGAGGTAAGACG
ATCTGTGGCTCCGATTCTCGACATATTGACGCTAGACTGCGTTGCTCAGTCTTGCACG
AGTGGGTTACATCGAACTGGATCTCAACAGCGGTAAGATCCTTGAGAGTTTTCGCCC
CGAAGAACGTTTTCCAATGATGAGCACTTTTAAAGTTCTGCTATGTGGCGCGGTATT
ATCCCGTATTGACGCCGGGCAAGAGCAACTCGGTCGCCGCATACACTATTCTCAGAA
TGACTTGGTTGAGTACTCACCAGTCACAGAAAAGCATCTTACGGATGGCATGACAGT
AAGAGAATTATGCAGTGCTGCCATAACCATGAGTGATAACACTGCGGCCAACTTACT
TCTGACAACGATCGGAGGACCGAAGGAGCTAACCGCTTTTTTGCACAACATGGGGG
ATCATGTAACTCGCCTTGATCGTTGGGAACCGGAGCTGAATGAAGCCATACCAAAC
GACGAGCGTGACACCACGATGCCTGTAGCAATGGCAACAACGTTGCGCAAACTATT
AACTGGCGAACTACTTACTCTAGCTTCCCGGCAACAATTAATAGACTGGATGGAGGC
GGATAAAGTTGCAGGACCACTTCTGCGCTCGGCCCTTCCGGCTGGCTGGTTTATTGC
TGATAAATCTGGAGCCGGTGAGCGTGGGTCTCGCGGTATCATTGCAGCACTGGGGCC
AGATGGTAAGCCCTCCCGTATCGTAGTTATCTACACGACGGGGAGTCAGGCAACTAT
GGATCAACAATTAATAGACTGGATGGAGGCGGATAAAGTTGCAGGACCACTTCTGC
GCTCGGCCCTTCCGGCTGGCTGGTTTATTGCTGATAAATCTGGAGCCGGTGAGCGTG
GGTCTCGCGGTATCATTGCAGCACTGGGGCCAGATGGTAAGCCCTCCCGTATCGTAG
TTATCTACACGACGGGGAGTCAGGCAACTATGGATGAACGAAATAGACAGATCGCT
GAGATAGGTGCCTCACTGATTAAGCATTGGTAACTGTCAGACCAAGTTTACTCATAT
ATACTTTAGATTGATTTAAAACTTCATTTTTAATTTAAAAGGATCTAGGTGAAGATCC
TTTTTGATAATCTCATGACCAAAATCCCTTAACGTGAGTTTTCGTTCCACTGAGCGTC
AGACCCCGTAGAAAAGATCAAAGGATCTTCTTGAGATCCTTTTTTTCTGCGCGTAAT
CTGCTGCTTGCAAACAAAAAAACCACCGCTACCAGCGGTGGTTTGTTTGCCGGATCA
AGAGCTACCAACTCTTTTTCCGAAGGTAACTGGCTTCAGCAGAGCGCAGATACCAAA
TACTGTTCTTCTAGTGTAGCCGTAGTTAGGCCACCACTTCAAGAACTCTGTAGCACC
GCCTACATCCGAAGGTAACTGGCTTCAGCAGAGCGCAGATACCAAATACTGTTCTTC
TAGTGTAGCCGTAGTTAGGCCACCACTTCAAGAACTCTGTAGCACCGCCTACATACC
TCGCTCTGCTAATCCTGTTACCAGTGGCTGCTGCCAGTGGCGATAAGTCGTGTCTTAC
CGGGTTGGACTCAAGACGATAGTTACCGGATAAGGCGCAGCGGTCGGGCTGAACGG
GGGGTTCGTGCACACAGCCCAGCTTGGAGCGAACGACCTACACCGAACTGAGATAC
CTACAGCGTGAGCTATGAGAAAGCGCCACGCTTCCCGAAGGGAGAAAGGCGGACA
GGTATCCGGTAAGCGGCAGGGTCGGAACAGGAGAGCGCACGAGGGAGCTTCCAGG
GGGAAACGCCTGGTATCTTTATAGTCCTGTCGGGTTTCGCCACCTCTGACTTGAGCG
TCGATTTTTGTGATGCTCGTCAGGGGGGCGGAGCCTATGGAAAAACGCCAGCAAC
GCGGCCTTTTTACGGTTGAACAGGAGAGCGCACGAGGGAGCTTCCAGGGGGAAACG
CCTGGTATCTTTATAGTCCTGTCGGGTTTCGCCACCTCTGACTTGAGCGTCGATTTTT
GTGATGCTCGTCAGGGGGGCGGAGCCTATGGAAAAACGCCAGCAACGCGGCCTTTT
TACGGTTCCTGGCCTTTTGCTGGCCTTTTGCTGGCCTTTTGCTCACATGTTCTTTCCTG
CGTTATCCCCTGATTCTGTGGATAACCGTATTACCGCCTTTGAGTGAGCTGATACCGC
TCGCCGCAGCCGAACGACCGAGCGCAGCGAGTCAGTGAGCGAGGAAGCGGAAGAG
CGCCCAATACGCAAACCGCCTCTCCCCGCGCGTTGGCCGATTCATTAATGCAG
CGCAACGCAATTAATGTGAGTTAGCTCACTCATTAGGCACCCCAGGCTTTACACTTT
ATGCTTCCGGCTCGTATGTTGTGTGGAATTGTGAGCGGATAACAATTTCACACAGGA

AACAGCTATGACCATGATTACGCCAAGCGCGCAATTAACCCTCACTAAAGGGAACA
AAAGCTGGAGCTCCACCGCGGTG

### AAV10-Mutant ; SEQ ID No. 158

GGCATTTCTGACAGCTTTGTGAACTGGGTGGCCGAGAAGGAATGGGAGTTGCCGCC
AGATTCTGACATGGATCTGAATCTGATTGAGCAGGCACCCCTGACCGTGGCCGAGA
AGCTGCAGCGCGACTTTCTGACGGAATGGCGCCGTGTGAGTAAGGCCCCGGAGGCT
CTTTTCTTTGTGCAATTTGAGAAGGGAGAGAGCTACTTCCACATGCACGTGCTCGTG
GAAACCACCGGGGTGAAATCCATGG
TTTTGGGACGTTTCCTGAGTCAGATTCGCGAAAAACTGATTCAGAGAATTTACCGCG
GGATCGAGCCGACTTTGCCAAACTGGTTCGCGGTCACAAAGACCAGAAATGGCGCC
GGAGGCGGGAACAAGGTGGTGGATGAGTGCTACATCCCCAATTACTTGCTCCCCAA
AACCCAGCCTGAGCTCCAGTGGGCGTGGACTAATATGGAACAGTATTTAAGCGCCT
GTTTGAATCTCACGGAGCGTAACGGGTGGTGGCGCAGCATCTGACGCACGTGTCGC
AGACGCAAAACCGCCCCCGACTACCTGGTGGGCCAGCAGCCCGTGGAGGACATTTC
CAGCAATCGGATTTATAAAATTTGGAACTAAACGGGTACGATCCCCAATATGCGGC
TTCCGTCTTTCTGGGATGGGCCACGAAAAAGTTCGGCAAGAGGAACACCATCTGGCT
GTTTGGGCCTGCAACTACCGGGAAGACCAACATCGCGGAGGCCATAGCCCACACTG
TGCCCTTCTACGGGTGCGTAAACTGGACCAATGAGAACTTTCCCTTCAACGACTGTG
TCGACAAGATGGTGATCTGGTGGGAGGAGGGGAAGATGACCGCCAAGGTCGTGGAG
TCGGCCAAAGCCATTTCTCGGAGGAAAGCAAAGGTGCGCGTGGACCAGAAATGCAA
GTCCTCTCACTCACGGACAGAAAGACTGTTTAGAGTGCTTTCCCGTGTCAGAATCTC
AACCCGTTTCTGTCGTCAAAAAGGCGTATCAAAAAACTGTGCTACATTCATCATATC
ATGGGAAAGGTGCCAGACGCTTGCACTGCCTGCGATCTGGTCAATGTGGATTTGGAT
GACTGCATCTTTGAACAATAAATGATTTAAATCAGGTATGGCTGCCGATGGTTATCG
CACTGCCTGCGATCTGGTCAATGTGGATTTGGATGACTGCATCTTTGAACAATAAAT
GATTTAAATCAGGTATGGCTGCCGATGGTTATCTTCCAGATTGGCTCGAGGACAACC
TCTCTGAGGGCATTCGCGAGTGGTGGGACTTGAAACCTGGAGCCCCGAAACCCAAA
GCCAACCAGCAAAAGCAGGACGACGGCCGGGGTCTGGTGCTTCCTGGCTACAAGTA
CCTCGGACCCTTCAACGGACTCGACAAGGGGGAGCCCGTCAACGCGGCGGACGCAG
CGGCCCTCGAGCACGACAAGGCCTACGACCAGCAGCTCAAAGCGGGTGACAATCCG
TACCTGCGGTATAACCACGCCGACGCCGAGTTTCAGGAGCGTCTGCAAGAAGATAC
GTCTTTTGGGGGCAACCTCGGGCGAGCAGTCTTCCAGGCCAAGAAGCGGGTTCTCGA
ACCTCTCGGTCTGGTTGAGGAAGGCGCTAAGACGGCTCCTGGAAAGAAGAGACCGG
TAGAGCCATCACCCCAGCGTTCTCCAGACTCCTCAATGTGGATTTGGATGACTGCAT
CTTTGAACAATATATGATTTAAATCAGGTATGGCTGCCGATGGTTATCTTCCAGATT
GGCTCGAGGACAACCTCTCTGAGGGCATTCGCGAGTGGTGGGACTTGAAACCTGGA
GCCCCGAAACCCAAAGCCAACCAGCAAAAGCAGGACGACGGCCGGGGTCTGGTGCT
TCCTGGCTACAAGTACCTCGGACCCTTCAACGGACTCGACAAGGGGGAGCCCGTCA
ACGCGGCGGACGCAGCGGCCCTCGAGCACGACAAGGCCTACGACCAGCAGCTC

K84R
AGAGCGGGTGACAATCCGTACCTGCGGTATAACCACGCCGACGCCGAGTTTCAGGA
GCGTCTGCAAGAAGATGCGTCTTTTTCTGGTTGAGGAAGGCGCTAGGACGGCTCCTG
T108A K137R

GAAAGAAGAGACCGGTAGAGCCATCACCCCAGCGTTCTCCAGACTCCTCTACGGGC
ATCGGCAAGAAAGGCCAGCAGCCCGCGAAAAAGAGACTCAACTTTGGGCAGACTG
GCGACTCAGAGTCAGTGCCCGACCCTCAACCAATCGGAGAACCCCCCGCAGCCCCT
CTGGTCTGGGATCTGGTACAATGGCTGCAGGCGGTGGCGCTCCAATGGCAGACAAT
CCCCAACCCCAATTTTCAGAACCTCCGGAAAAAAATCCTCTTTTGGGGGCCACCCC
CGGCCAACCATCCTTCCAGCCCAAAAACCGGTTCCCCAACCCTTTCGTCTTGGTTGA
GAAAGGCCCTAAAACGCTCCCGGAAAAAAAAAAACCGGAAAACCCTCCCCCCCACC
TTCCCCCAAATCCTCCAACGGCCTCCGCAAAAAAAGGCCACCACCCCCCAAAAAAA
AAATCCACTTTGGGCAAAACGGCCAATCCAAATCCGTGGCCCAACCTCCACCCATCC
GAAAACCCCCCCCCAGCCCCTCTGGGCCGGGATTTGGTAACATGGCTGGCGGGG

T252A
GGGGCCCCCCCATGGGAACCCTCCCCGCCTACAACAACCACCAAAATAAACAAAGC
CCCCAACGGAGGGGTTATTCCCCCAGAAATTGGGGAAGCACCAACGACAACACCTA
CTTCGGCTACAGCACCCCCTGGGGGTATTTTGACTTTAACAGATTCCACTGCCACTTC
TCACCACGTGACTGGCAGCGACTCATCAACAACAACTGGGGATTCCGGCCCAAAAA
ACTCAACTTCAAGCTCTTCAACATCCACGTC

K333R
AAGGAGGTCACGCAAAATGAAGGCACCAGGACCATCGCCAATAACCTTACCAGCAC
GAATCAGGTCTTTACCGACTCGGAATACCAGCTCCCGTACCTCCTCGGCTCTGCGCA
CCAGGGCTGCCTGCCTGATGAACCCCCTCATCGACCAGTACCTGTACTACCTGTCTC
GGACTCAGTCCACGGGAGGTACCGCAGGAACTCAGCAGTTGCTATTTTCTCAGGCCG
GGCCTAATAACATGTCGGCTCAGGCCAAAAACT

S492A
GGCTACCCGGGCCCTGCTACCGGCAGCAACGCGTCGCCACGACACTGTCGCAAAAT
AACAACGCCAACTTTGCCTGGACCGGTGCCACCAAGTATCATCTGAATGGCAGAAA
S501A

CTCTCTGGTAAATCCCGGTGTCGCTATGGCAACCCACAAGGACGACGAAGAGCGAT
TTTTTCCGTCCAGCGGAGTCTTAATGTTTGGGAAACAGGGAGCTGGAAAAGACAAC
GTGGACTATAGCATCGTTATGCTAACCAGTGAGGAAGAAATTAAAACCACCAACCC
AGTGGCCACAGAACAGTACGGCGTGGTGGCCGATAACCTGCAACAGCAAAACGCCG
CTCCTATTGTAGGGGCCGTCAACAGTCAAGGAGCCTTACCTGTCATGGTCTGGCAGA
ACCGGGACGTGTACCTGCAGGGTCCTATCTGGGCCAAGATTCCTCACACGGACGGA
AACTTTCATCCCTCGCCGCTGATGGGAGGCTTTGGACTGAAACACCCGCCTCCTCCA
TCCTGATTAAAAATACACCTGTTCCCGTGGATCCTCCAACTACCTTCAGTCC
AGCTAAGCTGGCGGCGTT
S671A

T674A
CATCGCGCAGTACAGCACCGGACAGGTCAGCGTGGAAATTGAATGGAGCTGCAAA
AAAAAAACAGCAAACGCTGGAACCCAAAAATTCAATACTCTTCCAACTACTACAAA
TCTACAAATGTGGACTTTGCTGTTAACACAGATGGCACTTATTGGTACGATCAGACT
GGAGCGGGCAGTGAGCGCACGCAATTAATGTGAGTTAGCTCACTCATTAGGCACCC
CAGGCTTTACACTTTATGCTTCCGGCTCGTATGTTGTGTGGAATTGTGAGCGGATAA
CAATTTCACACAGGAAACAGCTATGACCATGATTACGCCAAGCGCGCAATTAACCC
TCACTAAAGGGAACAAAAGCTGGAGCTCCACCGCGGTGGTGCTTTACGGCACCTCG
ACCCCAAAAAACTTGATTAGGGTGATGGTTCACGAAAAGGGCCATCGCCCTGATAG
ACGGTTTTTCGCCCTTTGACGTTGGAGTCCACGTTCTTTAATAGTGGACTCTTGTTCC
AAACTGGAACAACACTCAACCCTATCTCGGTCTATTCTTTTGATTTATAAGGGATTTT
GCCGATTTCGGCCTATTGGTTAAAAAATGAGCTGATTTAACAAAAATTTAACGCGAA
TTTTAACAAAATATTAACGCTTACAATTTAGGTGGCACTTTTCGGGGAAATGTGCGC
GGAACCCCTATTTGTTTATTTTTCTAAATACATTCAAATATGTATCCGCTCATGAGAC
AATAACCCTGATAAATGCTTCAATAATATTGAAAAAGGAAGAGTATGAGTATTCAA
CATTTCCGTGTCGCCCTTATTCCCTTTTTTGCGGCATTTTGCCTTCCTGTTTTTGCTCA
CCCAGAAACGCTGGTGAAAGTAAAAGATGCTGAAGATCAGTTGGGTGCACGAGTGG
GTTACATCGAACTGGATCTCAACAGCGGTAAGATCCTTGAGAGTTTTCGCCCCGAAG
AACGTTTTCCAATGATGAGCACTTTTAAAGTTCTGCTATGTGGCGCGGTATTATCCCG
TATTGACGCCGGGCAAGAGCAACTCGGTCGCCGCATACACTATTCTCAGATGACTT
GGTTGAGTACTCCCCAGTCACAGAAAAGCATCTTACGATGGCATGACAGTAAGGAG
ATTATGCATGCTGCCATACCATGATGAATAACACTGCGCACTTACTTCTGACACGAT
CGGAGGACCGAAGGAGCTCGGCGCTTCTGGGGCTTTTTTGAAGGCTAAAAAGGG
CCCAAGGGGGGGGGGGGGGGCAAGCACAACCCCCCGTACGTCGGGGGGGGGAGA
AGGAGATTTTCCTTTAGCAAAGAGAACGGTGCCCCCCCCGGCGAGGGAACGGGCC
CCTGGTTTCCCCCGCCCAACGGAGAAACCATACACCCACTACTATGCACAACATCTA
TATGTAATCATAAAACCATAGATCATCGCTATAGCGCGACGCGCCCCTCTCTGCATA
CTGTGAGGTAAGACGATCTGTGGCTCCGATTCTCGACATATTGACGCTAGACTGCGT
TGCTCAGTCTTGCACGAGTGGGTTACATCGAACTGGATCTCAACAGCGGTAAGATCC
TTGAGAGTTTTCGCCCCGAAGAACGTTTTCCAATGATGAGCACTTTTAAAGTTCTGCT
ATGTGGCGCGGTATTATCCCGTATTGACGCCGGGCAAGAGCAACTCGGTCGCCGCAT
ACACTATTCTCAGAATGACTTGGTTGAGTACTCACCAGTCACAGAAAAGCATCTTAC
GGATGGCATGACAGTAAGAGAATTATGCAGTGCTGCCATAACCATGAGTGATAACA
CTGCGGCCAACTTACTTCTGACAACGATCGGAGGACCGAAGGAGCTAACCGCTTTTT
TGCACAACATGGGGGATCATGTAACTCGCCTTGATCGTTGGGAACCGGAGCTGAAT
GAAGCCATACCAAACGACGAGCGTGACACCACGATGCCTGTAGCAATGGCAACAAC
GTTGCGCAAACTATTAACTGGCGAACTACTTACTCTAGCTTCCCGGCAACAATTAAT
AGACTGGATGGAGGCGGATAAAGTTGCAGGACCACTTCTGCGCTCGGCCCTTCCGG
CTGGCTGGTTTATTGCTGATAAATCTGGAGCCGGTGAGCGTGGGTCTCGCGGTATCA
TTGCAGCACTGGGGCCAGATGGTAAGCCCTCCCGTATCGTAGTTATCTACACGACGG
GGAGTCAGGCAACTATGGATCAACAATTAATAGACTGGATGGAGGCGGATAAAGTT
GCAGGACCACTTCTGCGCTCGGCCCTTCCGGCTGGCTGGTTTATTGCTGATAAATCT
GGAGCCGGTGAGCGTGGGTCTCGCGGTATCATTGCAGCACTGGGGCCAGATGGTAA
GCCCTCCCGTATCGTAGTTATCTACACGACGGGGAGTCAGGCAACTATGGATGAACG
AAATAGACAGATCGCTGAGATAGGTGCCTCACTGATTAAGCATTGGTAACTGTCAG

ACCAAGTTTACTCATATATACTTTAGATTGATTTAAAACTTCATTTTTAATTTAAAAG
GATCTAGGTGAAGATCCTTTTTGATAATCTCATGACCAAAATCCCTTAACGTGAGTT
TTCGTTCCACTGAGCGTCAGACCCCGTAGAAAAGATCAAAGGATCTTCTTGAGATCC
TTTTTTTCTGCGCGTAATCTGCTGCTTGCAAACAAAAAAACCACCGCTACCAGCGGT
GGTTTGTTTGCCGGATCAAGAGCTACCAACTCTTTTTCCGAAGGTAACTGGCTTCAG
CAGAGCGCAGATACCAAATACTGTTCTTCTAGTGTAGCCGTAGTTAGGCCACCACTT
CAAGAACTCTGTAGCACCGCCTACATCCGAAGGTAACTGGCTTCAGCAGAGCGCAG
ATACCAAATACTGTTCTTCTAGTGTAGCCGTAGTTAGGCCACCACTTCAAGAACTCT
GTAGCACCGCCTACATACCTCGCTCTGCTAATCCTGTTACCAGTGGCTGCTGCCAGT
GGCGATAAGTCGTGTCTTACCGGGTTGGACTCAAGACGATAGTTACCGGATAAGGC
GCAGCGGTCGGGCTGAACGGGGGGTTCGTGCACACAGCCCAGCTTGGAGCGAACGA
CCTACACCGAACTGAGATACCTACAGCGTGAGCTATGAGAAAGCGCCACGCTTCCC
GAAGGGAGAAAGGCGGACAGGTATCCGGTAAGCGGCAGGGTCGGAACAGGAGAGC
GCACGAGGGAGCTTCCAGGGGGAAACGCCTGGTATCTTTATAGTCCTGTCGGGTTTC
GCCACCTCTGACTTGAGCGTCGATTTTTGTGATGCTCGTCAGGGGGGCGGAGCCTA
TGGAAAAACGCCAGCAACGCGGCCTTTTTACGGTTGAACAGGAGAGCGCACGAGGG
AGCTTCCAGGGGGAAACGCCTGGTATCTTTATAGTCCTGTCGGGTTTCGCCACCTCT
GACTTGAGCGTCGATTTTTGTGATGCTCGTCAGGGGGGCGGAGCCTATGGAAAAAC
GCCAGCAACGCGGCCTTTTTACGGTTCCTGGCCTTTTGCTGGCCTTTTGCTGGCCTTT
TGCTCACATGTTCTTTCCTGCGTTATCCCCTGATTCTGTGGATAACCGTATTACCGCC
TTTGAGTGAGCTGATACCGCTCGCCGCAGCCGAACGACCGAGCGCAGCGAGTCAGT
GAGCGAGGAAGCGGAAGAGCGCCCAATACGCAAACCGCCTCTCCCCGCAAGATAC
AGACTGGAGCGGGCAGTGAGCGCAACGCAATTAATGTGAGTTAGCTCACTCATTAG
GCACCCCAGGCTTTACACTTTATGCTTCCGGCTCGTATGTTGTGTGGAATTGTGAGCG
GATAACAATTTCACACAGGAAACAGCTATGACCATGATTACGCCAAGCGCGCAATT
AACCCTCACTAAAGGGAACAAAAGCTGGAGCTCCACCGCGGTG

| AAV10 RESIDUE | NUCLEOTIDE CHANGE |
|---|---|
| AAV10-K84R | AAG→AGG |
| AAV10-K137R | AAG→AGG |
| AAV10-K333R | AAG→AGG |
| AAV10-S492A | TCC→GCC |
| AAV10-S501A | AGC→GCC |
| AAV10-S671A | TCG→GCG |
| AAV10-T108A | ACG→GCG |
| AAV10-T252A | ACC→GCC |
| AAV10-T674A | ACG→GCG |

**Table 17: Quantitative analysis of mutant AAV2 and AAV8 depicting the transduction efficacy.**

| **S.No.** | **AAV8 Mutants** | ***In vivo* Analysis : Fold Change** | | |
|---|---|---|---|---|
| | | **Vector copy no by QPCR** | **EGFP expression by QPCR** | **Luciferase expression** |
| **SINGLE MUTANTS** | | | | |
| 1 | AAV8 WT | 1 | 1 | - |
| 2 | AAV8 S279A | 3.9 | 9 | - |
| 3 | AAV2 S501 | 2 | 1 | 4.2 |
| 4 | AAV2 S671A | 9.2 | 20 | 3.4 |
| 5 | AAV2 S 149A | - | - | 2 |
| 6 | AAV2 S156A | - | - | 1.8 |
| 7 | AAV2 K137R | 15 | 46 | 3.8 |
| 8 | AAV2 K143R | - | - | 1.5 |
| 9 | AAV2 K652R | - | - | 2.4 |
| 10 | AAV2 T252A | 0.1 | 0.02 | 2.7 |
| 11 | AAV2 T138A | - | - | 1 |
| 12 | AAV2 T654A | - | - | 1.8 |

| **DOUBLE MUTANTS** | | | | |
|---|---|---|---|---|
| 1 | AAV8 K137R + S671A | - | - | 2.4 |

### Ubiquitin conjugation assay and immunoblotting:

A ubiquitination assay of viral capsids is carried out with a ubiquitin-protein conjugation kit according to the protocol of the manufacturer (Boston Biochem, Cambridge, MA). Briefly, 10× energy solution, conjugation fraction A, conjugation fraction B, and ubiquitin are mixed to a final reaction volume of 100 µl. The conjugation reaction is then initiated by adding 3×10⁸ heat-denatured AAV-WT, AAV S/ T/K mutant vector and incubated at 37°C for 4hr. Equal volumes of sodium dodecyl sulfate (SDS)-denatured ubiquitinated samples are then resolved on a 4-20% gradient gel. The ubiquitination pattern for the various viral particles is detected by immunoblotting of the samples with mouse antiubiquitin monoclonal antibody (P4D1) and horseradish peroxidase (HRP)-conjugated anti-mouse IgG1 secondary (Cell Signaling Technology, Boston, MA). VP1, VP2, and VP3 capsid proteins are detected with AAV clone B1 antibody (Fitzgerald, North Acton, MA) and HRP-conjugated anti-mouse IgG1 secondary antibody (Cell Signaling Technology).

Result: As can be seen in Figure. 10, the AAV2 K532R mutant vector demonstrated significantly reduced ubiquitination compared with either the AAV2-WT or AAV5-WT vector. Interestingly, AAV5 capsid had higher ubiquitination than did AAV2-WT capsid, a phenomenon that has been reported previously. These data provide direct evidence that the superior transduction achieved with the AAV2 K532R mutant vector is due to reduced ubiquitination of the viral capsid, which possibly results in rapid intracellular trafficking of the virus and improved gene expression, as has been suggested previously for the AAV2 tyrosine mutant vectors.

AAV8 K137R mutant vector has significantly reduced ubiquitination pattern compared to WT-AAV8 vector. AAV8 capsid proteins VP1 (87kDa), VP2 (72 kDa), and VP3 (62 kDa) are probed as gel-loading controls, which showed similar levels of these proteins across the samples tested (Figure 26B). These data provide direct evidence that the superior transduction achieved with the K137R mutant vector is due to the reduced ubiquitination of the viral capsid, which possibly results in rapid intracellular trafficking of the virus and improved gene expression.

## Claims

1. A nucleotide sequence selected from a group comprising SEQ ID Nos. 139 to 148, having mutation at codon selected from a group comprising TCT, TCC, TCA, TCG, AGT, AGC, ACT, ACC, ACA, ACG, AAA and AAG or any combination thereof.

2. The nucleotide sequence as claimed in claim 1, wherein the sequence selected from a group comprising SEQ ID Nos. 139 to 148, corresponds to serotypes to 10 respectively, of wild type adeno-associated virus vector; and wherein the sequence after mutation is represented by SEQ ID Nos. 149 to 158, respectively with respect to the wild type SEQ ID Nos. 139 to 148.

3. The nucleotide sequence as claimed in claim 1, wherein the sequence having SEQ ID Nos. 149 to 158, corresponds to mutated serotypes to 10 respectively, of adeno-associated virus vector.

4. The nucleotide sequence as claimed in claim 1, wherein the codon TCT, TCC, TCA, TCG, AGT or AGC code for amino acid serine; codon ACT, ACC, ACA or ACG code for amino acid threonine; and the codon AAA or AAG code for amino acid lysine.

5. The nucleotide sequence as claimed in claim 1, wherein the codon TCT, TCC, TCA, TCG, AGT, AGC, ACT, ACC, ACA or ACG is mutated to any codon selected from a group comprising GCT, GCC, GCA or GCG; and wherein the codon AAA or AAG is mutated to any codon selected from a group comprising CGT, CGC, CGA, CGG, AGA or AGG.

6. The nucleotide sequence as claimed in claim 5, wherein the codon GCT, GCC, GCA or GCG code for amino acid alanine; and the codon CGT, CGC, CGA, CGG, AGA or AGG code for amino acid arginine.

7. The nucleotide sequence as claimed in any of the claims to 6, wherein mutation in the codon coding for serine or threonine results in replacement of said serine or threonine amino acid with alanine amino acid; and wherein mutation in the codon coding for lysine results in replacement of said lysine amino acid with arginine amino acid.

8. The nucleotide sequence as claimed in 5, wherein the mutation in codon TCT, TCC, TCA, TCG, AGT or AGC in any of the SEQ ID Nos. 139 to 148, occurs at position of the corresponding amino acid sequence, said position selected from a group comprising 149, 156, 268, 276, 277, 278, 279, 485, 489, 490, 492, 498, 499, 501, 525, 526, 537, 547, 652, 658, 662, 663, 668, 669 and 671 or any combination thereof; and wherein the mutation in codon ACT, ACC, ACA or ACG in any of the SEQ ID Nos. 139 to 148, occurs at position of the corresponding amino acid sequence, said position selected from a group comprising 107, 108, 138, 245, 251, 252, 328, 454, 503, 654, 671, 674, 701, 713 and 716 or any combination thereof; and wherein the mutation in codon AAA or AAG in any of the SEQ ID Nos. 139 to 148, occurs at position of the corresponding amino acid sequence, said position selected from a group comprising 32, 39, 84, 90, 137, 143, 161, 333, 490, 507, 527, 532, 544 and 652 or any combination thereof.

9. A nucleotide sequence selected from a group comprising SEQ ID Nos. 70 to 138, having mutation at codon selected from a group comprising TCT, TCC, TCA, TCG, AGT, AGC, ACT, ACC, ACA, ACG, AAA and AAG or any combination thereof.

10. The nucleotide sequence as claimed in claim 9, wherein the sequence selected from a group comprising SEQ ID Nos. 70 to 138, represents wild type primer sequence capable of amplifying nucleotide sequence corresponding to serotypes 1 to 10, of wild type adeno-associated virus vector.

11. The nucleotide sequence as claimed in claim 9, wherein the codon TCT, TCC, TCA, TCG, AGT, AGC, ACT, ACC, ACA or ACG is mutated to any codon selected from a group comprising GCT, GCC, GCA or GCG; and wherein the codon AAA or AAG is mutated to any codon selected from a group comprising CGT, CGC, CGA, CGG, AGA or AGG; and wherein the sequence having said mutation is selected from a sequence having SEQ ID Nos. to 69; and wherein the mutated sequence act as primer for carrying out site directed mutagenesis for obtaining the mutated nucleotide sequence as claimed in claim 1.

12. A method of obtaining a nucleotide sequence selected from a group comprising SEQ ID Nos. 139 to 148, having mutation at codon selected from a group comprising TCT, TCC, TCA, TCG, AGT, AGC, ACT, ACC, ACA, ACG, AAA and AAG or any combination thereof, said method comprising act of introducing mutation in any of nucleotide sequence selected from a group comprising SEQ ID Nos. 139 to 148 through site directed mutagenesis by using the nucleotide sequence of claim 9.

13. A method of enhancing transduction efficiency, said method comprising act of expressing a target gene in presence of a nucleotide sequence selected from a group comprising SEQ ID Nos. 139 to 148, having mutation at codon selected from a group comprising TCT, TCC, TCA, TCG, AGT, AGC, ACT, ACC, ACA, ACG, AAA and AAG or any combination thereof.

14. The method as claimed in claim 13, wherein the transduction efficiency is enhanced with minimizing immunological response when compared with transduction carried out in presence of wild type adeno-associated virus vector having sequence selected from a group comprising SEQ ID Nos. 139 to 148.

15. A recombinant cell comprising the nucleotide sequence of claim 1.

16. A method of obtaining the recombinant cell as claimed in claim 25, said method comprising act of introducing the nucleotide sequence of claim to a host cell, to obtain said recombinant cell.

17. A kit comprising a nucleotide sequence selected from a group comprising SEQ ID Nos. 70 to 138, having mutation at codon selected from a group comprising TCT, TCC, TCA, TCG, AGT, AGC, ACT, ACC, ACA, ACG, AAA and AAG or any combination thereof.


SEQUENCE LISTING

<110>  CHRISTIAN MEDICAL COLLEGE

<120>  "NUCLEOTIDE SEQUENCES, METHODS, RECOMBINANT CELL, AND A KIT
       THEREOF"

<130>  IP20287

<150>  1714/CHE/2012 and 2231/CHE/2012
<151>  2012-05-02

<160>  174

<170>  PatentIn version 3.5

<210>  1
<211>  41
<212>  DNA
<213>  Adeno-Associated Virus

<220>
<221>  primer_bind
<222>  (1)..(41)

<400>  1
caaccactac ttcggctacg ccaccccatg ggggtatttt g                    41

<210>  2
<211>  42
<212>  DNA
<213>  Adeno-Associated Virus

<220>
<221>  primer_bind
<222>  (1)..(42)

<400>  2
caaaaacaga caacaacaac gccaatttta cctggactgg tg                    42

<210>  3
<211>  29
<212>  DNA
<213>  Adeno-Associated Virus

<220>
<221>  primer_bind
<222>  (1)..(29)

<400>  3
gcactgccat ggccgcacac aaagacgac                                   29

<210>  4
<211>  40
<212>  DNA
<213>  Adeno-Associated Virus

<220>
<221>  primer_bind
<222>  (1)..(40)

<400>  4
cgaatcctcc cgcggagttt gcagctacaa agtttgcttc                              40


<210>  5
<211>  41
<212>  DNA
<213>  Adeno-Associated Virus


<220>
<221>  primer_bind
<222>  (1)..(41)

<400>  5
tttcagctac aaagtttgct gcattcatca cccaatactc c                           41


<210>  6
<211>  30
<212>  DNA
<213>  Adeno-Associated Virus


<220>
<221>  primer_bind
<222>  (1)..(30)

<400>  6
cctgggcctt gccggcctac aataaccacc                                        30


<210>  7
<211>  40
<212>  DNA
<213>  Adeno-Associated Virus


<220>
<221>  primer_bind
<222>  (1)..(40)

<400>  7
ctggttgagg aaggcgcgag aacggctcct ggaaagaaac                              40


<210>  8
<211>  42
<212>  DNA
<213>  Adeno-Associated Virus


<220>
<221>  primer_bind
<222>  (1)..(42)

<400>  8
cactactttg gctacgccac cccttggggg tattttgact tc                          42

<210>  9
<211>  34
<212>  DNA
<213>  Adeno-Associated Virus

<220>
<221>  primer_bind
<222>  (1)..(34)

<400>  9
taccgccagc agcgagtagc taagacatct gcgg                                    34

<210>  10
<211>  42
<212>  DNA
<213>  Adeno-Associated Virus

<220>
<221>  primer_bind
<222>  (1)..(42)

<400>  10
aagacatctg cagataacaa caacgctgaa tactcgtgga ct                           42

<210>  11
<211>  31
<212>  DNA
<213>  Adeno-Associated Virus

<220>
<221>  primer_bind
<222>  (1)..(31)

<400>  11
ccgggcccgg cgatggcagc ccacaaggac g                                       31

<210>  12
<211>  40
<212>  DNA
<213>  Adeno-Associated Virus

<220>
<221>  primer_bind
<222>  (1)..(40)

<400>  12
agaaaagttt tttcctcagg ccggggttct catctttggg                              40

<210>  13
<211>  41
<212>  DNA
<213>  Adeno-Associated Virus

<220>
<221>  primer_bind
<222>  (1)..(41)

<400>  13
catctttggg aagcaaggcg ccgagaaaac aaatgtggac a                          41


<210>  14
<211>  38
<212>  DNA
<213>  Adeno-Associated Virus


<220>
<221>  primer_bind
<222>  (1)..(38)

<400>  14
aatccttcga ccaccttcgc tgcggcaaag tttgcttc                              38


<210>  15
<211>  51
<212>  DNA
<213>  Adeno-Associated Virus


<220>
<221>  primer_bind
<222>  (1)..(51)

<400>  15
gcgaatccgt cgaccacctt cagtgcggca aagtttgctg ccttcatcac a               51


<210>  16
<211>  42
<212>  DNA
<213>  Adeno-Associated Virus


<220>
<221>  primer_bind
<222>  (1)..(42)

<400>  16
acccgaacct gggccctgcc ggcctacaac aaccacctct ac                         42


<210>  17
<211>  51
<212>  DNA
<213>  Adeno-Associated Virus


<220>
<221>  primer_bind
<222>  (1)..(51)

<400>  17
tattacttaa gcagaacaaa cactccaagt ggagccacca cgcagtcaag g               51

<210>  18
<211>  51
<212>  DNA
<213>  Adeno-Associated Virus

<220>
<221>  primer_bind
<222>  (1)..(51)

<400>  18
acatctgcag ataacaacaa cagtgaatac tcgtgggctg gagctaccaa g                51

<210>  19
<211>  48
<212>  DNA
<213>  Adeno-Associated Virus

<220>
<221>  primer_bind
<222>  (1)..(48)

<400>  19
ttcagtgcgg ccaagtttgc ttccttcatc gcacagtact ccacggga                48

<210>  20
<211>  48
<212>  DNA
<213>  Adeno-Associated Virus

<220>
<221>  primer_bind
<222>  (1)..(48)

<400>  20
cgctggaatc ccgaaattca gtacgcttcc aactacaaca agtctgtt                48

<210>  21
<211>  45
<212>  DNA
<213>  Adeno-Associated Virus

<220>
<221>  primer_bind
<222>  (1)..(45)

<400>  21
gttaatgtgg actttgctgt cgacactaat ggcgtgtatt cagag                45

<210>  22
<211>  48
<212>  DNA
<213>  Adeno-Associated Virus

<220>
<221>  primer_bind
<222>  (1)..(48)

<400>  22
aagtctgtta atgtagactt tactgtggac gctaatggcg tgtattca                    48


<210>  23
<211>  45
<212>  DNA
<213>  Adeno-Associated Virus


<220>
<221>  primer_bind
<222>  (1)..(45)

<400>  23
ccaaagcccg cagaacggca tagggacgac agcaggggtc ttgtg                       45


<210>  24
<211>  45
<212>  DNA
<213>  Adeno-Associated Virus


<220>
<221>  primer_bind
<222>  (1)..(45)

<400>  24
ggcctggttg aggaacctgt taggacggcc ccgggaaaaa agagg                       45


<210>  25
<211>  42
<212>  DNA
<213>  Adeno-Associated Virus


<220>
<221>  primer_bind
<222>  (1)..(42)

<400>  25
cctgttaaga cggccccggg aaaaaggagg ccggtagagc ac                          42


<210>  26
<211>  54
<212>  DNA
<213>  Adeno-Associated Virus


<220>
<221>  primer_bind
<222>  (1)..(54)

<400>  26
gactcctcct cgggaaccgg aagggccggc cagcagcctg caagaaaaag attg             54

<210>  27
<211>  39
<212>  DNA
<213>  Adeno-Associated Virus

<220>
<221>  primer_bind
<222>  (1)..(39)

<400>  27
cagcagcgag tatcaaggac atctgcagat aacaacaac                              39

<210>  28
<211>  39
<212>  DNA
<213>  Adeno-Associated Virus

<220>
<221>  primer_bind
<222>  (1)..(39)

<400>  28
tggactggag ctaccagata ccacctcaat ggcagagac                              39

<210>  29
<211>  42
<212>  DNA
<213>  Adeno-Associated Virus

<220>
<221>  primer_bind
<222>  (1)..(42)

<400>  29
ccgggcccgg caatggcaag ccacagggac gatgaagaaa ag                          42

<210>  30
<211>  45
<212>  DNA
<213>  Adeno-Associated Virus

<220>
<221>  primer_bind
<222>  (1)..(45)

<400>  30
cacaaggacg atgaagaaag gttttttcct caaagcgggg ttctc                       45

<210>  31
<211>  42
<212>  DNA
<213>  Adeno-Associated Virus

<220>
<221>  primer_bind
<222>  (1)..(42)

<400>  31
gttctcatct ttggtagaca aggctcagag aaaacaaatg tg                          42


<210>  32
<211>  30
<212>  DNA
<213>  Adeno-Associated Virus


<220>
<221>  primer_bind
<222>  (1)..(30)

<400>  32
cctgggccct gcccgcttac aacaaccatc                                        30


<210>  33
<211>  29
<212>  DNA
<213>  Adeno-Associated Virus


<220>
<221>  primer_bind
<222>  (1)..(29)

<400>  33
cctgggtctt gcccgcctac aacaaccac                                         29


<210>  34
<211>  30
<212>  DNA
<213>  Adeno-Associated Virus


<220>
<221>  primer_bind
<222>  (1)..(30)

<400>  34
gcctactttg gatacgccac cccctggggg                                        30


<210>  35
<211>  28
<212>  DNA
<213>  Adeno-Associated Virus


<220>
<221>  primer_bind
<222>  (1)..(28)

<400>  35
ggtcaaccgc gccgctgtca gcgccttc                                          28

<210>  36
<211>  29
<212>  DNA
<213>  Adeno-Associated Virus

<220>
<221>  primer_bind
<222>  (1)..(29)

<400>  36
atatcaccag cttcgcggac gtgcccgtc                                          29

<210>  37
<211>  32
<212>  DNA
<213>  Adeno-Associated Virus

<220>
<221>  primer_bind
<222>  (1)..(32)

<400>  37
gacgtgcccg tcagcgcctt catcacccag ta                                      32

<210>  38
<211>  29
<212>  DNA
<213>  Adeno-Associated Virus

<220>
<221>  primer_bind
<222>  (1)..(29)

<400>  38
cgggcccacc gaaaccaaga cccaatcag                                          29

<210>  39
<211>  25
<212>  DNA
<213>  Adeno-Associated Virus

<220>
<221>  primer_bind
<222>  (1)..(25)

<400>  39
ctcgccgacg acgcatcctt cgggg                                              25

<210>  40
<211>  27
<212>  DNA
<213>  Adeno-Associated Virus

<220>
<221>  primer_bind
<222>  (1)..(27)

<400>  40
cgccaacaac ctcgcctcca ccgtcca                                          27


<210>  41
<211>  28
<212>  DNA
<213>  Adeno-Associated Virus


<220>
<221>  primer_bind
<222>  (1)..(28)

<400>  41
catgggcctt gccggcctat aacaacca                                         28


<210>  42
<211>  27
<212>  DNA
<213>  Adeno-Associated Virus


<220>
<221>  primer_bind
<222>  (1)..(27)

<400>  42
ctgggccctg cccgcctaca acaacca                                          27


<210>  43
<211>  33
<212>  DNA
<213>  Adeno-Associated Virus


<220>
<221>  primer_bind
<222>  (1)..(33)

<400>  43
gagaccggta gagccagcac cccagcgttc tcc                                   33


<210>  44
<211>  35
<212>  DNA
<213>  Adeno-Associated Virus


<220>
<221>  primer_bind
<222>  (1)..(35)

<400>  44
cccagcgttc tccagacgcc tctacgggca tcggc                                 35

<210>  45
<211>  28
<212>  DNA
<213>  Adeno-Associated Virus

<220>
<221>  primer_bind
<222>  (1)..(28)

<400>  45
ctacttcggc tacgccaccc cctggggg                                          28

<210>  46
<211>  40
<212>  DNA
<213>  Adeno-Associated Virus

<220>
<221>  primer_bind
<222>  (1)..(40)

<400>  46
gacaaccggt caaaacaaca atgccaactt tgcctggact                             40

<210>  47
<211>  42
<212>  DNA
<213>  Adeno-Associated Virus

<220>
<221>  primer_bind
<222>  (1)..(42)

<400>  47
ccttcaacca gtcaaagcag aacgctttca tcacgcaata ca                          42

<210>  48
<211>  35
<212>  DNA
<213>  Adeno-Associated Virus

<220>
<221>  primer_bind
<222>  (1)..(35)

<400>  48
ttgaggaagg cgctaaggcg gctcctggaa agaag                                  35

<210>  49
<211>  42
<212>  DNA
<213>  Adeno-Associated Virus

<220>
<221>  primer_bind
<222>  (1)..(42)

<400>  49
acccgaacct gggccctgcc ggcctacaac aaccacctct ac                          42


<210>  50
<211>  40
<212>  DNA
<213>  Adeno-Associated Virus


<220>
<221>  primer_bind
<222>  (1)..(40)

<400>  50
agatcctgat caagaacgcg ccagtacctg cggatcctcc                             40


<210>  51
<211>  39
<212>  DNA
<213>  Adeno-Associated Virus


<220>
<221>  primer_bind
<222>  (1)..(39)

<400>  51
aaagctgaac tctttcatcg cgcaatacag caccggaca                              39


<210>  52
<211>  30
<212>  DNA
<213>  Adeno-Associated Virus


<220>
<221>  primer_bind
<222>  (1)..(30)

<400>  52
ggttgaggaa ggggctagga cggctcctgg                                        30


<210>  53
<211>  43
<212>  DNA
<213>  Adeno-Associated Virus


<220>
<221>  primer_bind
<222>  (1)..(43)

<400>  53
aggcgctaag acggctcctg gaaagagaag accggtagag cca                         43

<210>  54
<211>  56
<212>  DNA
<213>  Adeno-Associated Virus

<220>
<221>  primer_bind
<222>  (1)..(56)

<400>  54
tggcctgaaa catcctccgc ctcagatcct gattcgaaac acgcctgtac ctgcgg          56

<210>  55
<211>  41
<212>  DNA
<213>  Adeno-Associated Virus

<220>
<221>  primer_bind
<222>  (1)..(41)

<400>  55
caacgcctac ttcggctacg caaccccctg ggggtatttt g                          41

<210>  56
<211>  47
<212>  DNA
<213>  Adeno-Associated Virus

<220>
<221>  primer_bind
<222>  (1)..(47)

<400>  56
cagctaccga caacaacgtg tcgcgaccac tgtgactcaa aacaaca                    47

<210>  57
<211>  54
<212>  DNA
<213>  Adeno-Associated Virus

<220>
<221>  primer_bind
<222>  (1)..(54)

<400>  57
gccttcaaca aggacaagct gaacgccttt atcacccagt attctactgg ccaa            54

<210>  58
<211>  49
<212>  DNA
<213>  Adeno-Associated Virus

<220>
<221>  primer_bind
<222>  (1)..(49)

<400>  58
ccactgtgac tcaaaacaac aacgccgagt ttgcttggcc tggagcttc                49


<210>  59
<211>  40
<212>  DNA
<213>  Adeno-Associated Virus


<220>
<221>  primer_bind
<222>  (1)..(40)

<400>  59
cccgaacctg ggccctgcca gcctacaaca atcacctcta                          40


<210>  60
<211>  43
<212>  DNA
<213>  Adeno-Associated Virus


<220>
<221>  primer_bind
<222>  (1)..(43)

<400>  60
agcggctaag acggctcctg gaaagagaag gcctgtagag cag                      43


<210>  61
<211>  30
<212>  DNA
<213>  Adeno-Associated Virus


<220>
<221>  primer_bind
<222>  (1)..(30)

<400>  61
gcctacgacc agcagctcag agcgggtgac                                     30


<210>  62
<211>  28
<212>  DNA
<213>  Adeno-Associated Virus


<220>
<221>  primer_bind
<222>  (1)..(28)

<400>  62
ggttgaggaa ggcgctagga cggctcct                                       28

<210>  63
<211>  35
<212>  DNA
<213>  Adeno-Associated Virus

<220>
<221>  primer_bind
<222>  (1)..(35)

<400>  63
gcagaatgaa ggcaccagga ccatcgccaa taacc                                35

<210>  64
<211>  27
<212>  DNA
<213>  Adeno-Associated Virus

<220>
<221>  primer_bind
<222>  (1)..(27)

<400>  64
gcagcaacgc gtcgccacga cactgtc                                         27

<210>  65
<211>  38
<212>  DNA
<213>  Adeno-Associated Virus

<220>
<221>  primer_bind
<222>  (1)..(38)

<400>  65
ctgtcgcaaa ataacaacgc caactttgcc tggaccgg                             38

<210>  66
<211>  31
<212>  DNA
<213>  Adeno-Associated Virus

<220>
<221>  primer_bind
<222>  (1)..(31)

<400>  66
caagctaagc tggcggcgtt catcacgcag t                                    31

<210>  67
<211>  31
<212>  DNA
<213>  Adeno-Associated Virus

<220>
<221>  primer_bind
<222>  (1)..(31)

<400>  67
gcgtctgcaa gaagatgcgt cttttggggg c                                    31


<210>  68
<211>  27
<212>  DNA
<213>  Adeno-Associated Virus


<220>
<221>  primer_bind
<222>  (1)..(27)

<400>  68
ctgggccctc cccgcctaca acaacca                                         27


<210>  69
<211>  29
<212>  DNA
<213>  Adeno-Associated Virus


<220>
<221>  primer_bind
<222>  (1)..(29)

<400>  69
tggcgtcgtt catcgcgcag tacagcacc                                       29


<210>  70
<211>  41
<212>  DNA
<213>  Adeno-Associated Virus


<220>
<221>  primer_bind
<222>  (1)..(41)

<400>  70
caaccactac ttcggctaca gcaccccctg ggggtatttt g                        41


<210>  71
<211>  42
<212>  DNA
<213>  Adeno-Associated Virus


<220>
<221>  primer_bind
<222>  (1)..(42)

<400>  71
caaaaacaga caacaacaac agcaatttta cctggactgg tg                        42

<210>  72
<211>  29
<212>  DNA
<213>  Adeno-Associated Virus

<220>
<221>  primer_bind
<222>  (1)..(29)

<400>  72
gcactgctat ggcctcacac aaagacgac                                          29

<210>  73
<211>  40
<212>  DNA
<213>  Adeno-Associated Virus

<220>
<221>  primer_bind
<222>  (1)..(40)

<400>  73
cgaatcctcc ggcggagttt tcagctacaa agtttgcttc                              40

<210>  74
<211>  41
<212>  DNA
<213>  Adeno-Associated Virus

<220>
<221>  primer_bind
<222>  (1)..(41)

<400>  74
tttcagctac aaagtttgct tcattcatca cccaatactc c                            41

<210>  75
<211>  30
<212>  DNA
<213>  Adeno-Associated Virus

<220>
<221>  primer_bind
<222>  (1)..(30)

<400>  75
cctgggcctt gcccacctac aataaccacc                                         30

<210>  76
<211>  40
<212>  DNA
<213>  Adeno-Associated Virus

<220>
<221>  primer_bind
<222>  (1)..(40)

<400>  76
ctggttgagg aaggcgctaa gacggctcct ggaaagaaac                              40


<210>  77
<211>  42
<212>  DNA
<213>  Adeno-Associated Virus


<220>
<221>  primer_bind
<222>  (1)..(42)

<400>  77
cactactttg gctacagcac cccttggggg tattttgact tc                           42


<210>  78
<211>  34
<212>  DNA
<213>  Adeno-Associated Virus


<220>
<221>  primer_bind
<222>  (1)..(34)

<400>  78
taccgccagc agcgagtatc aaagacatct gcgg                                    34


<210>  79
<211>  42
<212>  DNA
<213>  Adeno-Associated Virus


<220>
<221>  primer_bind
<222>  (1)..(42)

<400>  79
aagacatctg cggataacaa caacagtgaa tactcgtgga ct                           42


<210>  80
<211>  31
<212>  DNA
<213>  Adeno-Associated Virus


<220>
<221>  primer_bind
<222>  (1)..(31)

<400>  80
ccgggcccgg ccatggcaag ccacaaggac g                                       31

<210>  81
<211>  40
<212>  DNA
<213>  Adeno-Associated Virus

<220>
<221>  primer_bind
<222>  (1)..(40)

<400>  81
agaaaagttt tttcctcaga gcggggttct catctttggg                              40

<210>  82
<211>  41
<212>  DNA
<213>  Adeno-Associated Virus

<220>
<221>  primer_bind
<222>  (1)..(41)

<400>  82
catctttggg aagcaaggct cagagaaaac aaatgtggac a                            41

<210>  83
<211>  38
<212>  DNA
<213>  Adeno-Associated Virus

<220>
<221>  primer_bind
<222>  (1)..(38)

<400>  83
aatccttcga ccaccttcag tgcggcaaag tttgcttc                                38

<210>  84
<211>  51
<212>  DNA
<213>  Adeno-Associated Virus

<220>
<221>  primer_bind
<222>  (1)..(51)

<400>  84
gcgaatcctt cgaccacctt cagtgcggca aagtttgctt ccttcatcac a                 51

<210>  85
<211>  42
<212>  DNA
<213>  Adeno-Associated Virus

<220>
<221>  primer_bind
<222>  (1)..(42)

<400>  85
acccgaacct gggccctgcc cacctacaac aaccacctct ac                          42


<210>  86
<211>  51
<212>  DNA
<213>  Adeno-Associated Virus


<220>
<221>  primer_bind
<222>  (1)..(51)

<400>  86
tattacttga gcagaacaaa cactccaagt ggaaccacca cgcagtcaag g                51


<210>  87
<211>  51
<212>  DNA
<213>  Adeno-Associated Virus


<220>
<221>  primer_bind
<222>  (1)..(51)

<400>  87
acatctgcgg ataacaacaa cagtgaatac tcgtggactg gagctaccaa g                51


<210>  88
<211>  48
<212>  DNA
<213>  Adeno-Associated Virus


<220>
<221>  primer_bind
<222>  (1)..(48)

<400>  88
ttcagtgcgg caaagtttgc ttccttcatc acacagtact ccacggga                    48


<210>  89
<211>  48
<212>  DNA
<213>  Adeno-Associated Virus


<220>
<221>  primer_bind
<222>  (1)..(48)

<400>  89
cgctggaatc ccgaaattca gtacacttcc aactacaaca agtctgtt                    48

<210>  90
<211>  45
<212>  DNA
<213>  Adeno-Associated Virus

<220>
<221>  primer_bind
<222>  (1)..(45)

<400>  90
gttaatgtgg actttactgt ggacactaat ggcgtgtatt cagag                    45

<210>  91
<211>  48
<212>  DNA
<213>  Adeno-Associated Virus

<220>
<221>  primer_bind
<222>  (1)..(48)

<400>  91
aagtctgtta atgtggactt tactgtggac actaatggcg tgtattca                 48

<210>  92
<211>  45
<212>  DNA
<213>  Adeno-Associated Virus

<220>
<221>  primer_bind
<222>  (1)..(45)

<400>  92
ccaaagcccg cagagcggca taaggacgac agcaggggtc ttgtg                    45

<210>  93
<211>  45
<212>  DNA
<213>  Adeno-Associated Virus

<220>
<221>  primer_bind
<222>  (1)..(45)

<400>  93
ggcctggttg aggaacctgt taagacggct ccgggaaaaa agagg                    45

<210>  94
<211>  42
<212>  DNA
<213>  Adeno-Associated Virus

<220>
<221>  primer_bind
<222>  (1)..(42)

<400>  94
cctgttaaga cggctccggg aaaaaagagg ccggtagagc ac                    42


<210>  95
<211>  54
<212>  DNA
<213>  Adeno-Associated Virus


<220>
<221>  primer_bind
<222>  (1)..(54)

<400>  95
gactcctcct cgggaaccgg aaaggcgggc cagcagcctg caagaaaaag attg        54


<210>  96
<211>  39
<212>  DNA
<213>  Adeno-Associated Virus


<220>
<221>  primer_bind
<222>  (1)..(39)

<400>  96
cagcagcgag tatcaaagac atctgcggat aacaacaac                         39


<210>  97
<211>  39
<212>  DNA
<213>  Adeno-Associated Virus


<220>
<221>  primer_bind
<222>  (1)..(39)

<400>  97
tggactggag ctaccaagta ccacctcaat ggcagagac                         39


<210>  98
<211>  42
<212>  DNA
<213>  Adeno-Associated Virus


<220>
<221>  primer_bind
<222>  (1)..(42)

<400>  98
ccgggcccgg ccatggcaag ccacaaggac gatgaagaaa ag                    42

<210>  99
<211>  45
<212>  DNA
<213>  Adeno-Associated Virus

<220>
<221>  primer_bind
<222>  (1)..(45)

<400>  99
cacaaggacg atgaagaaaa gttttttcct cagagcgggg ttctc                    45

<210>  100
<211>  42
<212>  DNA
<213>  Adeno-Associated Virus

<220>
<221>  primer_bind
<222>  (1)..(42)

<400>  100
gttctcatct ttgggaagca aggctcagag aaaacaaatg tg                      42

<210>  101
<211>  30
<212>  DNA
<213>  Adeno-Associated Virus

<220>
<221>  primer_bind
<222>  (1)..(30)

<400>  101
cctgggccct gcccacttac aacaaccatc                                    30

<210>  102
<211>  29
<212>  DNA
<213>  Adeno-Associated Virus

<220>
<221>  primer_bind
<222>  (1)..(29)

<400>  102
cctgggtctt gcccacctac aacaaccac                                     29

<210>  103
<211>  36
<212>  DNA
<213>  Adeno-Associated Virus

<220>
<221>  primer_bind
<222>  (1)..(36)

<400>  103
aacgcctact ttggatacag caccccctgg gggtac                                36


<210>  104
<211>  34
<212>  DNA
<213>  Adeno-Associated Virus


<220>
<221>  primer_bind
<222>  (1)..(34)

<400>  104
cggggtcaac cgcgccagtg tcagcgcctt cgcc                                   34


<210>  105
<211>  35
<212>  DNA
<213>  Adeno-Associated Virus


<220>
<221>  primer_bind
<222>  (1)..(35)

<400>  105
gaaatatcac cagcttctcg gacgtgcccg tcagc                                  35


<210>  106
<211>  38
<212>  DNA
<213>  Adeno-Associated Virus


<220>
<221>  primer_bind
<222>  (1)..(38)

<400>  106
tcggacgtgc ccgtcagcag cttcatcacc cagtacag                               38


<210>  107
<211>  35
<212>  DNA
<213>  Adeno-Associated Virus


<220>
<221>  primer_bind
<222>  (1)..(35)

<400>  107
aagcgggccc accgaaacca aaacccaatc agcag                                  35

<210>  108
<211>  30
<212>  DNA
<213>  Adeno-Associated Virus

<220>
<221>  primer_bind
<222>  (1)..(30)

<400>  108
aagctcgccg acgacacatc cttcggggaa                                        30

<210>  109
<211>  30
<212>  DNA
<213>  Adeno-Associated Virus

<220>
<221>  primer_bind
<222>  (1)..(30)

<400>  109
cgccaacaac ctcacctcca ccgtccaagt                                        30

<210>  110
<211>  29
<212>  DNA
<213>  Adeno-Associated Virus

<220>
<221>  primer_bind
<222>  (1)..(29)

<400>  110
catgggcctt gcccacctat aacaaccac                                         29

<210>  111
<211>  27
<212>  DNA
<213>  Adeno-Associated Virus

<220>
<221>  primer_bind
<222>  (1)..(27)

<400>  111
ctgggccctg cccacctaca acaacca                                           27

<210>  112
<211>  33
<212>  DNA
<213>  Adeno-Associated Virus

<220>
<221>  primer_bind
<222>  (1)..(33)

<400>  112
gagaccggta gagccatcac cccagcgttc tcc                                    33


<210>  113
<211>  35
<212>  DNA
<213>  Adeno-Associated Virus


<220>
<221>  primer_bind
<222>  (1)..(35)

<400>  113
cccagcgttc tccagactcc tctacgggca tcggc                                  35


<210>  114
<211>  28
<212>  DNA
<213>  Adeno-Associated Virus


<220>
<221>  primer_bind
<222>  (1)..(28)

<400>  114
ctacttcggc tacagcaccc cctggggg                                          28


<210>  115
<211>  40
<212>  DNA
<213>  Adeno-Associated Virus


<220>
<221>  primer_bind
<222>  (1)..(40)

<400>  115
gacaaccggg caaaacaaca atagcaactt tgcctggact                            40


<210>  116
<211>  42
<212>  DNA
<213>  Adeno-Associated Virus


<220>
<221>  primer_bind
<222>  (1)..(42)

<400>  116
ccttcaacca gtcaaagctg aactctttca tcacgcaata ca                          42

<210>  117
<211>  35
<212>  DNA
<213>  Adeno-Associated Virus

<220>
<221>  primer_bind
<222>  (1)..(35)

<400>  117
ttgaggaagg cgctaagacg gctcctggaa agaag                                35

<210>  118
<211>  42
<212>  DNA
<213>  Adeno-Associated Virus

<220>
<221>  primer_bind
<222>  (1)..(42)

<400>  118
acccgaacct gggccctgcc cacctacaac aaccacctct ac                        42

<210>  119
<211>  40
<212>  DNA
<213>  Adeno-Associated Virus

<220>
<221>  primer_bind
<222>  (1)..(40)

<400>  119
agatcctgat caagaacacg cctgtacctg cggatcctcc                           40

<210>  120
<211>  39
<212>  DNA
<213>  Adeno-Associated Virus

<220>
<221>  primer_bind
<222>  (1)..(39)

<400>  120
aaagctgaac tctttcatca cgcaatacag caccggaca                            39

<210>  121
<211>  30
<212>  DNA
<213>  Adeno-Associated Virus

<220>
<221>  primer_bind
<222>  (1)..(30)

<400>  121
ggttgaggaa ggcgctaaga cggctcctgg                                        30


<210>  122
<211>  43
<212>  DNA
<213>  Adeno-Associated Virus


<220>
<221>  primer_bind
<222>  (1)..(43)

<400>  122
aggcgctaag acggctcctg gaaagaagag accggtagag cca                         43


<210>  123
<211>  56
<212>  DNA
<213>  Adeno-Associated Virus


<220>
<221>  primer_bind
<222>  (1)..(56)

<400>  123
tggcctgaaa catcctccgc ctcagatcct gatcaagaac acgcctgtac ctgcgg           56


<210>  124
<211>  41
<212>  DNA
<213>  Adeno-Associated Virus


<220>
<221>  primer_bind
<222>  (1)..(41)

<400>  124
caacgcctac ttcggctaca gcaccccctg ggggtatttt g                          41


<210>  125
<211>  47
<212>  DNA
<213>  Adeno-Associated Virus


<220>
<221>  primer_bind
<222>  (1)..(47)

<400>  125
cagctaccga caacaacgtg tctcaaccac tgtgactcaa aacaaca                     47

<210>  126
<211>  54
<212>  DNA
<213>  Adeno-Associated Virus

<220>
<221>  primer_bind
<222>  (1)..(54)

<400>  126
gccttcaaca aggacaagct gaactctttc atcacccagt attctactgg ccaa          54

<210>  127
<211>  49
<212>  DNA
<213>  Adeno-Associated Virus

<220>
<221>  primer_bind
<222>  (1)..(49)

<400>  127
ccactgtgac tcaaaacaac aacagcgaat ttgcttggcc tggagcttc          49

<210>  128
<211>  40
<212>  DNA
<213>  Adeno-Associated Virus

<220>
<221>  primer_bind
<222>  (1)..(40)

<400>  128
cccgaacctg ggccctgccc acctacaaca atcacctcta          40

<210>  129
<211>  43
<212>  DNA
<213>  Adeno-Associated Virus

<220>
<221>  primer_bind
<222>  (1)..(43)

<400>  129
agcggctaag acggctcctg gaaagaagag gcctgtagag cag          43

<210>  130
<211>  30
<212>  DNA
<213>  Adeno-Associated Virus

<220>
<221>  primer_bind
<222>  (1)..(30)

<400>  130
gcctacgacc agcagctcaa agcgggtgac                                        30


<210>  131
<211>  29
<212>  DNA
<213>  Adeno-Associated Virus


<220>
<221>  primer_bind
<222>  (1)..(29)

<400>  131
tggttgagga aggcgctaag acggctcct                                         29


<210>  132
<211>  35
<212>  DNA
<213>  Adeno-Associated Virus


<220>
<221>  primer_bind
<222>  (1)..(35)

<400>  132
gcagaatgaa ggcaccaaga ccatcgccaa taacc                                  35


<210>  133
<211>  27
<212>  DNA
<213>  Adeno-Associated Virus


<220>
<221>  primer_bind
<222>  (1)..(27)

<400>  133
gcagcaacgc gtctccacga cactgtc                                           27


<210>  134
<211>  38
<212>  DNA
<213>  Adeno-Associated Virus


<220>
<221>  primer_bind
<222>  (1)..(38)

<400>  134
ctgtcgcaaa ataacaacag caactttgcc tggaccgg                               38

<210>  135
<211>  31
<212>  DNA
<213>  Adeno-Associated Virus


<220>
<221>  primer_bind
<222>  (1)..(31)

<400>  135
caagctaagc tggcgtcgtt catcacgcag t                                    31


<210>  136
<211>  31
<212>  DNA
<213>  Adeno-Associated Virus


<220>
<221>  primer_bind
<222>  (1)..(31)

<400>  136
gcgtctgcaa gaagatacgt cttttggggg c                                    31


<210>  137
<211>  27
<212>  DNA
<213>  Adeno-Associated Virus


<220>
<221>  primer_bind
<222>  (1)..(27)

<400>  137
ctgggccctc cccacctaca acaacca                                         27


<210>  138
<211>  29
<212>  DNA
<213>  Adeno-Associated Virus


<220>
<221>  primer_bind
<222>  (1)..(29)

<400>  138
tggcgtcgtt catcacgcag tacagcacc                                       29


<210>  139
<211>  8344
<212>  DNA
<213>  Adeno-Associated Virus

<220>
<221>  gene
<222>  (1)..(8344)

<400>  139
cacctaaatt gtaagcgtta atattttgtt aaaattcgcg ttaaattttt gttaaatcag      60

ctcatttttt aaccaatagg ccgaaatcgg caaaatccct tataaatcaa aagaatagac     120

cgagataggg ttgagtgttg ttccagtttg gaacaagagt ccactattaa agaacgtgga     180

ctccaacgtc aaagggcgaa aaaccgtcta tcagggcgat ggcccactac gtgaaccatc     240

accctaatca agttttttgg ggtcgaggtg ccgtaaagca ctaaatcgga accctaaagg     300

gagcccccga tttagagctt gacggggaaa gccggcgaac gtggcgagaa aggaagggaa     360

gaaagcgaaa ggagcgggcg ctagggcgct ggcaagtgta gcggtcacgc tgcgcgtaac     420

caccacaccc gccgcgctta atgcgccgct acagggcgcg tcccattcgc cattcaggct     480

gcgcaactgt tgggaagggc gatcggtgcg ggcctcttcg ctattacgcc agctggcgaa     540

agggggatgt gctgcaaggc gattaagttg ggtaacgcca gggttttccc agtcacgacg     600

ttgtaaaacg acggccagtg aattgtaata cgactcacta tagggcgaat tggagctcca     660

ccgcggtggc ggccgctcta gagtcctgta ttagaggtca cgtgagtgtt ttgcgacatt     720

ttgcgacacc atgtggtcac gctgggtatt taagcccgag tgagcacgca gggtctccat     780

tttgaagcgg gaggtttgaa cgcgcagcac ggcggggttt tacgagattg tgattaaggt     840

ccccagcgac cttgacgggc atctgcccgg catttctgac agctttgtga actgggtggc     900

cgagaaggaa tgggagttgc cgccagattc tgacatggat ctgaatctga ttgagcaggc     960

acccctgacc gtggccgaga agctgcagcg cgactttctg acggaatggc gccgtgtgag    1020

taaggccccg gaggcccttt tctttgtgca atttgagaag ggagagagct acttccacat    1080

gcacgtgctc gtggaaacca ccggggtgaa atccatggtt ttgggacgtt tcctgagtca    1140

gattcgcgaa aaactgattc agagaattta ccgcgggatc gagccgactt tgccaaactg    1200

gttcgcggtc acaaagacca gaaatggcgc cggaggcggg aacaaggtgg tggatgagtg    1260

ctacatcccc aattacttgc tccccaaaac ccagcctgag ctccagtggg cgtggactaa    1320

tatggaacag tatttaagcg cctgtttgaa tctcacggag cgtaaacggt tggtggcgca    1380

gcatctgacg cacgtgtcgc agacgcagga gcagaacaaa gagaatcaga atcccaattc    1440

tgatgcgccg gtgatcagat caaaaacttc agccaggtac atggagctgg tcgggtggct    1500

cgtggacaag gggattacct cggagaagca gtggatccag gaggaccagg cctcatacat    1560

ctccttcaat gcggcctcca actcgcggtc ccaaatcaag gctgccttgg acaatgcggg    1620

aaagattatg agcctgacta aaaccgcccc cgactacctg gtgggccagc agcccgtgga    1680

ggacatttcc agcaatcgga tttataaaat tttggaacta aacgggtacg atccccaata    1740

tgcggcttcc gtctttctgg gatgggccac gaaaaagttc ggcaagagga acaccatctg 1800

gctgtttggg cctgcaacta ccgggaagac caacatcgcg gaggccatag cccacactgt 1860

gcccttctac gggtgcgtaa actggaccaa tgagaacttt cccttcaacg actgtgtcga 1920

caagatggtg atctggtggg aggaggggaa gatgaccgcc aaggtcgtgg agtcggccaa 1980

agccattctc ggaggaagca aggtgcgcgt ggaccagaaa tgcaagtcct cggcccagat 2040

agacccgact cccgtgatcg tcacctccaa caccaacatg tgcgccgtga ttgacgggaa 2100

ctcaacgacc ttcgaacacc agcagccgtt gcaagaccgg atgttcaaat ttgaactcac 2160

ccgccgtctg gatcatgact ttgggaaggt caccaagcag gaagtcaaag actttttccg 2220

gtgggcaaag gatcacgtgg ttgaggtgga gcatgaattc tacgtcaaaa agggtggagc 2280

caagaaaaga cccgccccca gtgacgcaga tataagtgag cccaaacggg tgcgcgagtc 2340

agttgcgcag ccatcgacgt cagacgcgga agcttcgatc aactacgcag acaggtacca 2400

aaacaaatgt tctcgtcacg tgggcatgaa tctgatgctg tttccctgca gacaatgcga 2460

gagaatgaat cagaattcaa atatctgctt cactcacgga cagaaagact gtttagagtg 2520

ctttcccgtg tcagaatctc aacccgtttc tgtcgtcaaa aaggcgtatc agaaactgtg 2580

ctacattcat catatcatgg gaaaggtgcc agacgcttgc actgcctgcg atctggtcaa 2640

tgtggatttg gatgactgca tctttgaaca ataaatgatt taaaccaggt atggctgccg 2700

atggttatct tccagattgg ctcgaggaca acctctctga gggcattcgc gagtggtggg 2760

acttgaaacc tggagccccg aagcccaaag ccaaccagca aaagcaggac gacggccggg 2820

gtctggtgct tcctggctac aagtacctcg gacccttcaa cggactcgac aagggggagc 2880

ccgtcaacgc ggcggacgca gcggccctcg agcacgacaa ggcctacgac cagcagctca 2940

aagcgggtga caatccgtac ctgcggtata accacgccga cgccgagttt caggagcgtc 3000

tgcaagaaga tacgtctttt gggggcaacc tcgggcgagc agtcttccag gccaagaagc 3060

gggttctcga acctctcggt ctggttgagg aaggcgctaa gacggctcct ggaaagaaac 3120

gtccggtaga gcagtcgcca caagagccag actcctcctc gggcatcggc aagacaggcc 3180

agcagcccgc taaaaagaga ctcaattttg gtcagactgg cgactcagag tcagtccccg 3240

atccacaacc tctcggagaa cctccagcaa cccccgctgc tgtgggacct actacaatgg 3300

cttcaggcgg tggcgcacca atggcagaca ataacgaagg cgccgacgga gtgggtaatg 3360

cctcaggaaa ttggcattgc gattccacat ggctgggcga cagagtcatc accaccagca 3420

cccgcacctg ggccttgccc acctacaata accacctcta caagcaaatc tccagtgctt 3480

caacgggggc cagcaacgac aaccactact tcggctacag ccccctgggg gtattttgat 3540

ttcaacagat tccactgcca cttttcacca cgtgactggc agcgactcat caacaacaat 3600

tggggattcc ggcccaagag actcaacttc aaactcttca acatccaagt caaggaggtc 3660

acgacgaatg atggcgtcac aaccatcgct aataacctta ccagcacggt tcaagtcttc 3720

tcggactcgg agtaccagct tccgtacgtc ctcggctctg cgcaccaggg ctgcctccct 3780

ccgttcccgg cggacgtgtt catgattccg caatacggct acctgacgct caacaatggc 3840

agccaagccg tgggacgttc atccttttac tgcctggaat atttcccttc tcagatgctg 3900

agaacgggca acaactttac cttcagctac acctttgagg aagtgccttt ccacagcagc 3960

tacgcgcaca gccagagcct ggaccggctg atgaatcctc tcatcgacca atacctgtat 4020

tacctgaaca gaactcaaaa tcagtccgga agtgcccaaa acaaggactt gctgtttagc 4080

cgtgggtctc cagctggcat gtctgttcag cccaaaaact ggctacctgg accctgttat 4140

cggcagcagc gcgtttctaa aacaaaaaca gacaacaaca acagcaattt tacctggact 4200

ggtgcttcaa aatataacct caatgggcgt gaatccatca tcaaccctgg cactgctatg 4260

gcctcacaca aagacgacga agacaagttc tttcccatga gcggtgtcat gatttttgga 4320

aaagagagcg ccggagcttc aaacactgca ttggacaatg tcatgattac agacgaagag 4380

gaaattaaag ccactaaccc tgtggccacc gaaagatttg ggaccgtggc agtcaatttc 4440

cagagcagca gcacagaccc tgcgaccgga gatgtgcatg ctatgggagc attacctggc 4500

atggtgtggc aagatagaga cgtgtacctg cagggtccca tttgggccaa aattcctcac 4560

acagatggac actttcaccc gtctcctctt atgggcggct ttggactcaa gaacccgcct 4620

cctcagatcc tcatcaaaaa cacgcctgtt cctgcgaatc ctccggcgga gttttcagct 4680

acaaagtttg cttcattcat cacccaatac tccacaggac aagtgagtgt ggaaattgaa 4740

tgggagctgc agaaagaaaa cagcaagcgc tggaatcccg aagtgcagta cacatccaat 4800

tatgcaaaat ctgccaacgt tgattttact gtggacaaca atggacttta tactgagcct 4860

cgccccattg gcacccgtta ccttacccgt cccctgtaat ttacgtgtta atcaataaac 4920

cggttgattc gtttcagttg aactttggtc tcgcggccgc tcgataagct tttgttccct 4980

ttagtgaggg ttaattcgaa gcttggcgta atcatggtca tagctgtttc ctgtgtgaaa 5040

ttgttatccg ctcacaattc cacacaacat acgagccgga agcataaagt gtaaagcctg 5100

gggtgcctaa tgagtgagct aactcacatt aattgcgttg cgctcactgc ccgctttcca 5160

gtcgggaaac ctgtcgtgcc agctgcatta atgaatcggc caacgcgcgg ggagaggcgg 5220

tttgcgtatt gggcgctctt ccgcttcctc gctcactgac tcgctgcgct cggtcgttcg 5280

gctgcgcgag cggtatcagc tccctcaaag cgtaataccg gttatcccac agatcagggg 5340

atacgcagaa agacatgtga gtaaggcagc caaagtccag gaaccggtaa aaggccccgc 5400

gcgcttagtg ctttacggca cctcgacccc aaaaaacttg attagggtga tggttcacgt 5460

agtgggccat cgccctgata gacggttttt cgccctttga cgttggagtc cacgttcttt 5520

aatagtggac tcttgttcca aactggaaca acactcaacc ctatctcggt ctattctttt 5580

gatttataag ggattttgcc gatttcggcc tattggttaa aaaatgagct gatttaacaa 5640

aaatttaacg cgaattttaa caaaatatta acgtttacaa tttaaatatt tgcttataca 5700

atcttcctgt ttttggggct tttctgatta tcaaccgggg tacatatgat tgacatgcta 5760

gttttacgat taccgttcat cgattctctt gtttgctcca gactctcagg caatgacctg 5820

atagcctttg tagagacctc tcaaaaatag ctaccctctc cggcatgaat ttatcagcta 5880

gaacggttga atatcatatt gatggtgatt tgactgtctc cggcctttct cacccgtttg 5940

aatctttacc tacacattac tcaggcattg catttaaaat atatgagggt tctaaaaatt 6000

tttatccttg cgttgaaata aaggcttctc ccgcaaaagt attacagggt cataatgttt 6060

ttggtacaac cgatttagct ttatgctctg aggctttatt gcttaatttt gctaattctt 6120

tgccttgcct gtatgattta ttggatgttg gaattcctga tgcggtattt tctccttacg 6180

catctgtgcg gtatttcaca ccgcatatgg tgcactctca gtacaatctg ctctgatgcc 6240

gcatagttaa gccagccccg acacccgcca acacccgctg acgcgccctg acgggcttgt 6300

ctgctcccgg catccgctta cagacaagct gtgaccgtct ccgggagctg catgtgtcag 6360

aggttttcac cgtcatcacc gaaacgcgcg agacgaaagg gcctcgtgat acgcctattt 6420

ttataggtta atgtcatgat aataatggtt tcttagacgt caggtggcac ttttcgggga 6480

aatgtgcgcg gaacccctat ttgtttattt ttctaaatac attcaaatat gtatccgctc 6540

atgagacaat aaccctgata aatgcttcaa taatattgaa aaaggaagag tatgagtatt 6600

caacatttcc gtgtcgccct tattcccttt tttgcggcat tttgccttcc tgtttttgct 6660

cacccagaaa cgctggtgaa agtaaaagat gctgaagatc agttgggtgc acgagtgggt 6720

tacatcgaac tggatctcaa cagcggtaag atccttgaga gttttcgccc cgaagaacgt 6780

tttccaatga tgagcacttt taaagttctg ctatgtggcg cggtattatc ccgtattgac 6840

gccgggcaag agcaactcgg tcgccgcata cactattctc agaatgactt ggttgagtac 6900

tcaccagtca cagaaaagca tcttacggat ggcatgacag taagagaatt atgcagtgct 6960

gccataacca tgagtgataa cactgcggcc aacttacttc tgacaacgat cggaggaccg 7020

aaggagctaa ccgctttttt gcacaacatg ggggatcatg taactcgcct tgatcgttgg 7080

gaaccggagc tgaatgaagc cataccaaac gacgagcgtg acaccacgat gcctgtagca 7140

atggcaacaa cgttgcgcaa actattaact ggcgaactac ttactctagc ttcccggcaa 7200

caattaatag actggatgga ggcggataaa gttgcaggac cacttctgcg ctcggccctt 7260

ccggctggct ggtttattgc tgataaatct ggagccggtg agcgtgggtc tcgcggtatc 7320

attgcagcac tggggccaga tggtaagccc tcccgtatcg tagttatcta cacgacgggg 7380

agtcaggcaa ctatggatga acgaaataga cagatcgctg agataggtgc ctcactgatt 7440

aagcattggt aactgtcaga ccaagtttac tcatatatac tttagattga tttaaaactt 7500

catttttaat ttaaaaggat ctaggtgaag atcctcacct aaattgtaag cgttaatatt 7560

ttgttaaaat tcgcgttaaa tttttgttaa atcagctcat tttttaacca ataggccgaa 7620

atcggcaaaa tcccttataa atcaaaagaa tagaccgaga tagggttgag tgttgttcca 7680

gtttggaaca agagtccact attaaagaac gtggactcca acgtcaaagg gcgaaaaacc 7740

gtctatcagg gcgatggccc actacgtgaa ccatcaccct aatcaagttt tttggggtcg 7800

aggtgccgta aagcactaaa tcggaaccct aaagggagcc cccgatttag agcttgacgg 7860

ggaaagccgg cgaacgtggc gagaaaggaa gggaagaaag cgaaaggagc gggcgctagg 7920

gcgctggcaa gtgtagcggt cacgctgcgc gtaaccacca cacccgccgc gcttaatgcg 7980

ccgctacagg gcgcgtccca ttcgccattc aggctgcgca actgttggga agggcgatcg 8040

gtgcgggcct cttcgctatt acgccagctg gcgaaagggg gatgtgctgc aaggcgatta 8100

agttgggtaa cgccagggtt ttcccagtca cgacgttgta aaacgacggc cagtgaattg 8160

taatacgact cactataggg cgaattggag ctccaccgcg gtggcggccg ctctagagtc 8220

ctgtattaga ggtcacgtga gtgttttgcg acattttgcg acaccatgtg gtcacgctgg 8280

gtatttaagc ccgagtgagc acgcagggtc tccattttga agcgggaggt ttgaacgcgc 8340

agcc 8344

<210> 140
<211> 8267
<212> DNA
<213> Adeno-Associated Virus

<220>
<221> gene
<222> (1)..(8267)

<400> 140
aattcccatc atcaataata taccttattt tggattgaag ccaatatgat aatgaggggg 60

tggagtttgt gacgtggcgc ggggcgtggg aacggggcgg gtgacgtagt agtctctaga 120

gtcctgtatt agaggtcacg tgagtgtttt gcgacatttt gcgacaccat gtggtcacgc 180

tgggtattta agcccgagtg agcacgcagg gtctccattt tgaagcggga ggtttgaacg 240

cgcagccacc aacggcgggg ttttacgaga ttgtgattaa ggtccccagc gaccttgacg 300

ggcatctgcc cggcatttct gacagctttg tgaactgggt ggccgagaag gaatgggagt 360

tgccgccaga ttctgacatg gatctgaatc tgattgagca ggcacccctg accgtggccg 420

agaagctgca gcgcgacttt ctgacggaat ggcgccgtgt gagtaaggcc ccggaggccc 480

ttttctttgt gcaatttgag aagggagaga gctacttcca catgcacgtg ctcgtggaaa 540

ccaccggggt gaaatccatg gttttgggac gtttcctgag tcagattcgc gaaaaactga 600

ttcagagaat ttaccgcggg atcgagccga ctttgccaaa ctggttcgcg gtcacaaaga 660

ccagaaatgg cgccggaggc gggaacaagg tggtggatga gtgctacatc cccaattact 720

tgctccccaa aacccagcct gagctccagt gggcgtggac taatatggaa cagtatttaa 780

gcgcctgttt gaatctcacg gagcgtaaac ggttggtggc gcagcatctg acgcacgtgt 840

cgcagacgca ggagcagaac aaagagaatc agaatcccaa ttctgatgcg ccggtgatca 900

gatcaaaaac ttcagccagg tacatggagc tggtcgggtg gctcgtggac aaggggatta 960

cctcggagaa gcagtggatc caggaggacc aggcctcata catctccttc aatgcggcct 1020

ccaactcgcg gtcccaaatc aaggctgcct tggacaatgc gggaaagatt atgagcctga 1080

ctaaaaccgc ccccgactac ctggtgggcc agcagcccgt ggaggacatt tccagcaatc 1140

ggatttataa aattttggaa ctaaacgggt acgatcccca atatgcggct tccgtctttc 1200

tgggatgggc cacgaaaaag ttcggcaaga ggaacaccat ctggctgttt gggcctgcaa 1260

ctaccgggaa gaccaacatc gcggaggcca tagcccacac tgtgcccttc tacgggtgcg 1320

taaactggac caatgagaac tttcccttca acgactgtgt cgacaagatg gtgatctggt 1380

gggaggaggg gaagatgacc gccaaggtcg tggagtcggc caaagccatt ctcggaggaa 1440

gcaaggtgcg cgtggaccag aaatgcaagt cctcggccca gatagacccg actcccgtga 1500

tcgtcacctc caacaccaac atgtgcgccg tgattgacgg gaactcaacg accttcgaac 1560

accagcagcc gttgcaagac cggatgttca aatttgaact cacccgccgt ctggatcatg 1620

actttgggaa ggtcaccaag caggaagtca aagacttttt ccggtgggca aaggatcacg 1680

tggttgaggt ggagcatgaa ttctacgtca aaaagggtgg agccaagaaa agacccgccc 1740

ccagtgacgc agatataagt gagcccaaac gggtgcgcga gtcagttgcg cagccatcga 1800

cgtcagacgc ggaagcttcg atcaactacg cagacaggta ccaaaacaaa tgttctcgtc 1860

acgtgggcat gaatctgatg ctgtttccct gcagacaatg cgagagaatg aatcagaatt 1920

caaatatctg cttcactcac ggacagaaag actgtttaga gtgctttccc gtgtcagaat 1980

ctcaacccgt ttctgtcgtc aaaaaggcgt atcagaaact gtgctacatt catcatatca 2040

tgggaaaggt gccagacgct tgcactgcct gcgatctggt caatgtggat ttggatgact 2100

gcatctttga acaataaatg atttaaatca ggtatggctg ccgatggtta tcttccagat 2160

tggctcgagg acactctctc tgaaggaata agacagtggt ggaagctcaa acctggccca 2220

ccaccaccaa agcccgcaga gcggcataag gacgacagca ggggtcttgt gcttcctggg 2280

tacaagtacc tcggaccctt caacggactc gacaagggag agccggtcaa cgaggcagac 2340

gccgcggccc tcgagcacga caaagcctac gaccggcagc tcgacagcgg agacaacccg 2400

tacctcaagt acaaccacgc cgacgcggag tttcaggagc gccttaaaga agatacgtct 2460

tttgggggca acctcggacg agcagtcttc caggcgaaaa agagggttct tgaacctctg 2520

ggcctggttg aggaacctgt taagacggct ccgggaaaaa agaggccggt agagcactct 2580

cctgtggagc cagactcctc ctcgggaacc ggaaaggcgg gccagcagcc tgcaagaaaa 2640

agattgaatt ttggtcagac tggagacgca gactcagtac ctgaccccca gcctctcgga 2700

cagccaccag cagccccctc tggtctggga actaatacga tggctacagg cagtggcgca 2760

ccaatggcag acaataacga gggcgccgac ggagtgggta attcctccgg aaattggcat 2820

tgcgattcca catggatggg cgacagagtc atcaccacca gcacccgaac ctgggccctg 2880

cccacctaca acaaccacct ctacaaacaa atttccagcc aatcaggagc ctcgaacgac 2940

aatcactact ttggctacag caccccttgg gggtattttg acttcaacag attccactgc 3000

cacttttcac cacgtgactg gcaaagactc atcaacaaca actggggatt ccgacccaag 3060

agactcaact tcaagctctt taacattcaa gtcaaagagg tcacgcagaa tgacggtacg 3120

acgacgattg ccaataacct taccagcacg gttcaggtgt ttactgactc ggagtaccag 3180

ctcccgtacg tcctcggctc ggcgcatcaa ggatgcctcc cgccgttccc agcagacgtc 3240

ttcatggtgc cacagtatgg atacctcacc ctgaacaacg ggagtcaggc agtaggacgc 3300

tcttcatttt actgcctgga gtactttcct tctcagatgc tgcgtaccgg aaacaacttt 3360

accttcagct acacttttga ggacgttcct ttccacagca gctacgctca cagccagagt 3420

ctggaccgtc tcatgaatcc tctcatcgac cagtacctgt attacttgag cagaacaaac 3480

actccaagtg gaaccaccac gcagtcaagg cttcagtttt ctcaggccgg agcgagtgac 3540

attcgggacc agtctaggaa ctggcttcct ggaccctgtt accgccagca gcgagtatca 3600

aagacatctg cggataacaa caacagtgaa tactcgtgga ctggagctac caagtaccac 3660

ctcaatggca gagactctct ggtgaatccg ggcccggcca tggcaagcca caaggacgat 3720

gaagaaaagt ttttcctcag gagcggggtt ctcatctttg ggaagcaagg ctcagagaaa 3780

acaaatgtgg acattgaaaa ggtcatgatt acagacgaag aggaaatcag gacaaccaat 3840

cccgtggcta cggagcagta tggttctgta tctaccaacc tccagagagg caacagacaa 3900

gcagctaccg cagatgtcaa cacacaaggc gttcttccag gcatggtctg gcaggacaga 3960

gatgtgtacc ttcaggggcc catctgggca aagattccac acacggacgg acattttcac 4020

ccctctcccc tcatgggtgg attcggactt aaacaccctc ctccacagat tctcatcaag 4080

aacaccccgg tacctgcgaa tccttcgacc accttcagtg cggcaaagtt tgcttccttc 4140

atcacacagt actccacggg acaggtcagc gtggagatcg agtgggagct gcagaaggaa 4200

aacagcaaac gctggaatcc cgaaattcag tacacttcca actacaacaa gtctgttaat 4260

gtggacttta ctgtggacac taatggcgtg tattcagagc ctcgccccat tggcaccaga 4320

tacctgactc gtaatctgta attgcttgtt aatcaataaa ccgtttaatt cgtttcagtt 4380

gaactttggt ctctgcgtat ttctttctta tctagtttcc atgctctaga gaaacctacg 4440

tcacccgccc cgttcccacg ccccgcgcca cgtcacaaac tccaccccct cattatcata 4500

ttggcttcaa tccaaaataa ggtatattat tgatgatgca tcgctggcgt aatagcgaag 4560

aggcccgcac cgatcgccct tcccaacagt tgcgcagcct gaatggcgaa tggaattcca 4620

gacgattgag cgtcaaaatg taggtatttc catgagcgtt tttcctgttg caatggctgg 4680

cggtaatatt gttctggata ttaccagcaa ggccgatagt ttgagttctt ctactcaggc 4740

aagtgatgtt attactaatc aaagaagtat tgcgacaacg gttaatttgc gtgatggaca 4800

gactctttta ctcggtggcc tcactgatta taaaaacact tctcaggatt ctggcgtacc 4860

gttcctgtct aaaatccctt taatcggcct cctgtttagc tcccgctctg attctaacga 4920

ggaaagcacg ttatacgtgc tcgtcaaagc aaccatagta cgcgccctgt agcggcgcat 4980

taagcgcggc gggtgtggtg gttacgcgca gcgtgaccgc tacacttgcc agcgccctag 5040

cgcccgctcc tttcgctttc ttcccttcct ttctcgccac gttcgccggc tttccccgtc 5100

aagctctaaa tcgggggctc cctttagggt tccgatttag tgctttacgg cacctcgacc 5160

ccaaaaaact tgattagggt gatggttcac gtagtgggcc atcgccctga tagacggttt 5220

ttcgcccttt gacgttggag tccacgttct ttaatagtgg actcttgttc caaactggaa 5280

caacactcaa ccctatctcg gtctattctt ttgatttata agggattttg ccgatttcgg 5340

cctattggtt aaaaaatgag ctgatttaac aaaaatttaa cgcgaatttt aacaaaatat 5400

taacgcttac aatttaaata tttgcttata caatcttcct gtttttgggg cttttctgat 5460

tatcaaccgg ggtacatatg attgacatgc tagttttacg attaccgttc atcgattctc 5520

ttgtttgctc cagactctca ggcaatgacc tgatagcctt tgtagagacc tctcaaaaat 5580

agctaccctc tccggcatga atttatcagc tagaacggtt gaatatcata ttgatggtga 5640

tttgactgtc tccggccttt ctcacccgtt tgaatcttta cctacacatt actcaggcat 5700

tgcatttaaa atatatgagg gttctaaaaa tttttatcct tgcgttgaaa taaaggcttc 5760

tcccgcaaaa gtattacagg gtcataatgt ttttggtaca accgatttag ctttatgctc 5820

tgaggcttta ttgcttaatt ttgctaattc tttgccttgc ctgtatgatt tattggatgt 5880

tggaatcgcc tgatgcggta ttttctcctt acgcatctgt gcggtatttc acaccgcata 5940

tggtgcactc tcagtacaat ctgctctgat gccgcatagt taagccagcc ccgacacccg 6000

ccaacacccg ctgacgcgcc ctgacgggct tgtctgctcc cggcatccgc ttacagacaa 6060

gctgtgaccg tctccgggag ctgcatgtgt cagaggtttt caccgtcatc accgaaacgc 6120

gcgagacgaa agggcctcgt gatacgccta tttttatagg ttaatgtcat gataataatg 6180

gtttcttaga cgtcaggtgg cacttttcgg ggaaatgtgc gcggaacccc tatttgttta 6240

tttttctaaa tacattcaaa tatgtatccg ctcatgagac aataaccctg ataaatgctt 6300

caataatatt gaaaaaggaa gagtatgagt attcaacatt tccgtgtcgc ccttattccc 6360

ttttttgcgg cattttgcct tcctgttttt gctcacccag aaacgctggt gaaagtaaaa 6420

gatgctgaag atcagttggg tgcacgagtg ggttacatcg aactggatct caacagcggt 6480

aagatccttg agagttttcg ccccgaagaa cgttttccaa tgatgagcac ttttaaagtt 6540

ctgctatgtg gcgcggtatt atcccgtatt gacgccgggc aagagcaact cggtcgccgc 6600

atacactatt ctcagaatga cttggttgag tactcaccag ttcccgtatt gacgccgggc 6660

aagagcaact cggtcgccgc atacactatt ctcagaatga cttggttgag tactcaccag 6720

tcacagaaaa gcatcttacg gatggcatga cagtaagaga attatgcagt gctgccataa 6780

ccatgagtga taacactgcg gccaacttac ttctgacaac gatcggagga ccgaaggagc 6840

taaccgcttt tttgcacaac atgggggatc atgtaactcg ccttgatcgt tgggaaccgg 6900

agctgaatga agccatacca aacgacgagc gtgacaccac gatgcctgta gcaatggcaa 6960

caacgttgcg caaactatta actggcgaac tacttactct agcttcccgg caacaattaa 7020

tagactggat ggaggcggat aaagttgcag gaccacttct gcgctcggcc cttccggctg 7080

gctggtttat tgctgataaa tctggagccg gtgagcgtgg gtctcgcggt atcattgcag 7140

cactggggcc agatggtaag ccctcccgta tcgtagttat ctacacgacg gggagtcagg 7200

caactatgga tgaacgaaat agacagatcg ctgagatagg tgcctcactg attaagcatt 7260

ggtaactgtc agaccaagtt tactcatata tactttagat tgatttaaaa cttcattttt 7320

aatttaaaag gatctaggtg aagatccttt ttgataatct catgaccaaa atcccttaac 7380

gtgagttttc gttccactga gcgtcagacc ccgtagaaaa gatcaaagga tcttcttgag 7440

atcctttttt tctgcgcgta atctgctgct tgcaaacaaa aaaaccaccg ctaccagcgg 7500

tggtttgttt gccggatcaa gagctaccaa ctctttttcc gaaggtaact ggcttcagca 7560

gagcgcagat accaaatact gttcctttct tctagtgtag ccgtagttag gccaccactt 7620

caagaactct gtagcaccgc ctacatacct cgctctgcta atcctgttac cagtggctgc 7680

tgccagtggc gataagtcgt gtcttaccgg gttggactca agacgatagt taccggataa 7740

ggcgcagcgg tcgggctgaa cggggggttc gtgcacacag cccagcttgg agcgaacgac 7800

ctacaccgaa ctgatatttt ttttttcgtg agctatgaga aagcgccacg cttcccgaag 7860

ggagaaaggc ggacaggtat ccggtaagcg gcagggtcgg aacaggagag cgcacgaggg 7920

agcttccagg gggaaacgcc tggtatcttt atagtcctgt cgggtttcgc cacctctgac 7980

ttgagcgtcg atttttgtga tgctcgtcag gggggcggag cctatggaaa aacgccagca 8040

acgcggcctt tttacggttc ctggcctttt gctggccttt tgctcacatg ttctttcctg 8100

cgttatcccc tgattctgtg gataaccgta ttaccgcctt tgagtgagct gataccgctc 8160

gccgcagccg aacgaccgag cgcagcgagt cagtgagcga ggaagcggaa gagcgcccaa 8220

tacgcaaacc gcctctcccc cgcgcgttgg ccgattcatt aatgcag 8267

<210> 141
<211> 8179
<212> DNA
<213> Adeno-Associated Virus

<220>
<221> gene
<222> (1)..(8179)

<400> 141
aattcccatc atcaataata taccttattt aggattgaag ccaatatgat aatgagccgg 60

tggagtttgt gacgtggcgc ggggcgtggg aacggggcgg gtgacgtagt agtctctaga 120

gtcctgtatt agaggtcacg tgagtgtttt gcgacttttt gcgacaccat gtggtcacgc 180

tgggtattta agcccgagtg agcacgcagg gtctccattt tgaagcggga ggtttgaacg 240

cgcagccacc acgggggggt tttacgagat tgtgattaag gtccccagcg accttgacgg 300

gcatctgccc ggcatttctg acagctttgt gaactgggtg gccgagaagg aatgggagtt 360

gccgccagat tctgacatgg atctgaatct gattgagcag gcacccctga ccgtggccga 420

gaagctgcag cgcgactttc tgacggaatg gcgccgtgtg agtaaggccc cggaggccct 480

tttctttgtg caatttgaga agggagagag ctacttccac atgcacgtgc tcgtggaaac 540

caccggggtg aaatccatgg ttttgggacg tttcctgagt cagattcgcg aaaaactgat 600

tcagagaatt taccgcggga tcgagccgac tttgccaaac tggttcgcgg tcacaaagac 660

cagaaatggc gccggaggcg ggaacaaggt ggtggatgag tgctacatcc ccaattactt 720

gctccccaaa acccagcctg agctccagtg ggcgtggact aatatggaac agtatttaag 780

cgcctgtttg aatctcacgg agcgtaaacg gttggtggcg cagcatctga cgcacgtgtc 840

gcagacgcag gagcagaaca aagagaatca gaatcccaat tctgatgcgc cggtgatcag 900

atcaaaaact tcagccaggt acatggagct ggtcgggtgg ctcgtggaca aggggattac 960

ctcggagaag cagtggatcc aggaggacca ggcctcatac atctccttca atgcggcctc 1020

caactcgcgg tcccaaatca aggctgcctt ggacaatgcg ggaaagatta tgagcctgac 1080

taaaaccgcc cccgactacc tggtgggcca gcagcccgtg gaggacattt ccagcaatcg 1140

gatttataaa attttggaac taaacgggta cgatccccaa tatgcggctt ccgtctttct 1200

gggatgggcc acgaaaaagt tcggcaagag gaacaccatc tggctgtttg ggcctgcaac 1260

taccgggaag accaacatcg cggaggccat agcccacact gtgcccttct acgggtgcgt 1320

aaactggacc aatgagaact ttcccttcaa cgactgtgtc gacaagatgg tgatctggtg 1380

ggaggagggg aagatgaccg ccaaggtcgt ggagtcggcc aaagccattc tcggaggaag 1440

caaggtgcgc gtggaccaga aatgcaagtc ctcggcccag atagacccga ctcccgtgat 1500

cgtcacctcc aacaccaaca tgtgcgccgt gattgacggg aactcaacga ccttcgaaca 1560

ccagcagccg ttgcaagacc ggatgttcaa atttgaactc acccgccgtc tggatcatga 1620

ctttgggaag gtcaccaagc aggaagtcaa agactttttc cggtgggcaa aggatcacgt 1680

ggttgaggtg gagcatgaat tctacgtcaa aaagggtgga gccaagaaaa gacccgcccc 1740

cagtgacgca gatataagtg agcccaaacg ggtgcgcgag tcagttgcgc agccatcgac 1800

gtcagacgcg gaagcttcga tcaactacgc agacaggtac caaaacaaat gttctcgtca 1860

cgtgggcatg aatctgatgc tgtttccctg cagacaatgc gagagaatga atcagaattc 1920

aaatatctgc ttcactcacg gacagaaaga ctgtttagag tgctttcccg tgtcagaatc 1980

tcaacccgtt tctgtcgtca aaaaggcgta tcagaaactg tgctacattc atcatatcat 2040

gggaaaggtg ccagacgctt gcactgcctg cgatctggtc aatgtggatt tggatgactg 2100

catctttgaa caataaatga tttaaatcag gtatggctgc tgacggttat cttccagatt 2160

ggctcgagga caacctttct gaaggcattc gtgagtggtg ggctctgaaa cctggagtcc 2220

ctcaacccaa agcgaaccaa caacaccagg acaaccgtcg gggtcttgtg cttccgggtt 2280

acaaatacct cggacccggt aacggactcg acaaaggaga gccggtcaac gaggcggacg 2340

cggcagccct cgaacacgac aaagcttacg accagcagct caaggccggt gacaacccgt 2400

acctcaagta caaccacgcc gacgccgagt ttcaggagcg tcttcaagaa gatacgtctt 2460

ttgggggcaa ccttggcaga gcagtcttcc aggccaaaaa gaggatcctt gagcctcttg 2520

gtctggttga ggaagcagct aaaacggctc ctggaaagaa gggggctgta gatcagtctc 2580

ctcaggaacc ggactcatca tctggtgttg gcaaatcggg caaacagcct gccagaaaaa 2640

gactaaattt cggtcagact ggagactcag agtcagtccc agaccctcaa cctctcggag 2700

aaccaccagc agcccccaca agtttgggat ctaatacaat ggcttcaggc ggtggcgcac 2760

caatggcaga caataacgag ggtgccgatg gagtgggtaa ttcctcagga aattggcatt 2820

gcgattccca atggctgggc gacagagtca tcaccaccag caccagaacc tgggccctgc 2880

ccacttacaa caaccatctc tacaagcaaa tctccagcca atcaggagct tcaaacgaca 2940

accactactt tggctacagc accccttggg ggtattttga ctttaacaga ttccactgcc 3000

acttctcacc acgtgactgg cagcgactca ttaacaacaa ctggggattc cggcccaaga 3060

aactcagctt caagctcttc aacatccaag ttagaggggt cacgcagaac gatggcacga 3120

cgactattgc caataacctt accagcacgg ttcaagtgtt tacggactcg gagtatcagc 3180

tcccgtacgt gctcgggtcg gcgcaccaag gctgtctccc gccgtttcca gcggacgtct 3240

tcatggtccc tcagtatgga tacctcaccc tgaacaacgg aagtcaagcg gtgggacgct 3300

catcctttta ctgcctggag tacttccctt cgcagatgct aaggactgga aataacttcc 3360

aattcagcta taccttcgag gatgtacctt ttcacagcag ctacgctcac agccagagtt 3420

tggatcgctt gatgaatcct cttattgatc agtatctgta ctacctgaac agaacgcaag 3480

gaacaacctc tggaacaacc aaccaatcac ggctgctttt tagccaggct gggcctcagt 3540

ctatgtcttt gcaggccaga aattggctac ctgggccctg ctaccggcaa cagagacttt 3600

caaagactgc taacgacaac aacaacagta actttccttg gacagcggcc agcaaatatc 3660

atctcaatgg ccgcgactcg ctggtgaatc caggaccagc tatggccagt cacaaggacg 3720

atgaagaaaa atttttccct atgcacggca atctaatatt tggcaaagaa gggacaacgg 3780

caagtaacgc agaattagat aatgtaatga ttacggatga agaagagatt cgtaccacca 3840

atcctgtggc aacagagcag tatggaactg tggcaaataa cttgcagagc tcaaatacag 3900

ctcccacgac tggaactgtc aatcatcagg gggccttacc tggcatggtg tggcaagatc 3960

gtgacgtgta ccttcaagga cctatctggg caaagattcc tcacacggat ggacactttc 4020

atccttctcc tctgatggga ggctttggac tgaaacatcc gcctcctcaa atcatgatca 4080

aaaatactcc ggtaccggca aatcctccga cgactttcag cccggccaag tttgcttcat 4140

ttatcactca gtactccact ggacaggtca gcgtggaaat tgagtgggag ctacagaaag 4200

aaaacagcaa acgttggaat ccagagattc agtacacttc caactacaac aagtctgtta 4260

atgtggactt tactgtagac actaatggtg tttatagtga acctcgccct attggaaccc 4320

ggtatctcac acgaaacttg tgattgcttg ttaatcaata aaccgtttaa ttcgtttcag 4380

ttgaactttg gtctctgcgt atttctttct tatctagttt ccatgctcta gactactacg 4440

tcacccgccc cgttcccacg ccccgcgcca cgtcacaaac tccaccccct cattatcata 4500

ttggcttcaa tccaaaataa ggtatattat tgatgatgca tcgctggcgt aatagcgaag 4560

aggcccgcac cgatcgccct tcccaacagt tgcgcagcct gaatggcgaa tggaattcca 4620

gacgattgag cgtcaaaatg taggtatttc catgagcgtt tttcctgttg caatggctgg 4680

cggtaatatt gttctggata ttaccagcaa ggccgatagt ttgagttctt ctactcaggc 4740

aagtgatgtt attactaatc aaagaagtat tgcgacaacg gttaatttgc gtgatggaca 4800

gactctttta ctcggtggcc tcactgatta taaaaacact tctcaggatt ctggcgtacc 4860

gttcctgtct aaaatccctt taatcggcct cctgtttagc tcccgctctg attctaacga 4920

ggaaagcacg ttatacgtgc tcgtcaaagc aaccatagta cgcgccctgt agcggcgcat 4980

taagcgcggc gggtgtggtg gttacgcgca gcgtgaccgc tacacttgcc agcgccctag 5040

cgcccgctcc tttcgctttc ttcccttcct ttctcgccac gttcgccggc tttccccgtc 5100

aagctctaaa tcgggggctc cctttagggt tccgatttag tgctttacgg cacctcgacc 5160

ccaaaaaact tgattagggt gatggttcac gtagtgggcc atcgccctga tagacggttt 5220

ttcgcccttt gacgttggag tccacgttct ttaatagtgg actcttgttc caaactggaa 5280

caacactcaa ccctatctcg gtctattctt ttgatttata agggattttg ccgatttcgg 5340

cctattggtt aaaaaatgag ctgatttaac aaaaatttaa cgcgaatttt aacaaaatat 5400

taacgtttac aatttaaata tttgcttata caatcttcct gtttttgggg cttttctgat 5460

tatcaaccgg ggtacatatg attgacatgc tagttttacg attaccgttc atcgattctc 5520

ttgtttgctc cagactctca ggcaatgacc tgatagcctt tgtagagacc tctcaaaaat 5580

agctaccctc tccggcatga atttatcagc tagaacggtt gaatatcata ttgatggtga 5640

tttgactgtc tccggccttt ctcacccgtt tgaatcttta cctacacatt actcaggcat 5700

tgcatttaaa atatatgagg gttctaaaaa tttttatcct tgcgttgaaa taaaggcttc 5760

tcccgcaaaa gtattacagg gtcataatgt ttttggtaca accgatttag ctttatgctc 5820

tgaggcttta ttgcttaatt ttgctaattc tttgccttgc ctgtatgatt tattggatgt 5880

tggaattcct gatgcggtat tttctcctta cgcatctgtg cggtatttca caccgcatat 5940

ggtgcactct cagtacaatc tgctctgatg ccgcatagtt aagccagccc cgacacccgc 6000

caacacccgc tgacgcgccc tgacgggctt gtctgctccc ggcatccgct tacagacaag 6060

ctgtgaccgt ctccgggagc tgcatgtgtc agaggttttc accgtcatca ccgaaacgcg 6120

cgagacgaaa gggcctcgtg atacgcctat ttttataggt taatgtcatg ataataatgg 6180

tttcttagac gtcaggtggc acttttcggg gaaatgtgcg cggaacccct atttgtttat 6240

ttttctaaat acattcaaat atgtatccgc tcatgagaca ataaccctga taaatgcttc 6300

aataatattg aaaaaggaag agtatgagta ttcaacattt ccgtgtcgcc cttattccct 6360

tttttgcggc attttgcctt cctgtttttg ctcacccaga aacgctggtg aaagtaaaag 6420

atgctgaaga tcagttgggt gcacgagtgg gttacatcga actggatctc aacagcggta 6480

agatccttga gagttttcgc cccgaagaac gttttccaat gatgagcact tttaaagttc 6540

tgctatgtgg cgcggtatta tcccgtattg acgccgggca agagcaactc ggtcgccgca 6600

tacactattc tcagaatgac ttggttgagt actcaccagt cacagaaaag catcttacgg 6660

atggcatgac agtaagagaa ttatgcagtg ctgccataac catgagtgat aacactgcgg 6720

ccaacttact tctgacaacg atcggaggac cgaaggagct aaccgctttt ttgcacaaca 6780

tgggggatca tgtaactcgc cttgatcgtt gggaaccgga gctgaatgaa gccataccaa 6840

acgacgagcg tgacaccacg atgcctgtag caatggcaac aacgttgcgc aaactattaa 6900

ctggcgaact acttactcta gcttcccggc aacaattaat agactggatg gaggcggata 6960

aagttgcagg accacttctg cgctcggccc ttccggctgg ctggtttatt gctgataaat 7020

ctggagccgg tgagcgtggg tctcgcggta tcattgcagc actggggcca gatggtaagc 7080

cctcccgtat cgtagttatc tacacgacgg ggagtcaggc aactatggat gaacgaaata 7140

gacagatcgc tgagataggt gcctcactga ttaagcattg gtaactgtca gaccaagttt 7200

actcatatat actttagatt gatttaaaac ttcattttta atttaaaagg atctaggtga 7260

agatcctttt tgataatctc atgaccaaaa tcccttaacg tgagttttcg ttccactgag 7320

cgtcagaccc cgtagaaaag atcaaaggat cttcttgaga tccttttttt ctgcgcgtaa 7380

tctgctgctt gcaaacaaaa aaaccaccgc taccagcggt ggtttgtttg ccggatcaag 7440

agctaccaac tctttttccg aaggtaactg gcttcagcag agcgcagata ccaaatactg 7500

tccttctagt gtagccgtag ttaggccacc acttcaagaa ctctgtagca ccgcctacat 7560

acctcgctct gctaatcctg ttaccagtgg ctgctgccag tggcgataag tcgtgtctta 7620

ccgggttgga ctcaagacga tagttaccgg ataaggcgca gcggtcgggc tgaacggggg 7680

gttcgtgcac acagcccagc ttggagcgaa cgacctacac cgaactgaga tacctacagc 7740

gtgagctatg agaaagcgcc acgcttcccg aagggagaaa ggcggacagg tatccggtaa 7800

gcggcagggt cggaacagga gagcgcacga gggagcttcc agggggaaac gcctggtatc 7860

tttatagtcc tgtcgggttt cgccacctct gacttgagcg tcgatttttg tgatgctcgt 7920

caggggggcg gagcctatgg aaaaacgcca gcaacgcggc ctttttacgg ttcctggcct 7980

tttgctggcc ttttgctcac atgttctttc ctgcgttatc ccctgattct gtggataacc 8040

gtattaccgc ctttgagtga gctgataccg ctcgccgcag ccgaacgacc gagcgcagcg 8100

agtcagtgag cgaggaagcg gaagagcgcc caatacgcaa accgcctctc cccgcgcgtt 8160

ggccgattca ttaatgcag 8179

<210> 142
<211> 8173
<212> DNA
<213> Adeno-Associated Virus


<220>
<221> gene
<222> (1)..(8173)

<400> 142
aattcccatc atcaataata taccttattt aggattgaag ccaatatgat aatgagccgg 60

tggagtttgt gacgtggcgc ggggcgtggg aacggggcgg gtgacgtagt agtctctaga 120

gtcctgtatt agaggtcacg tgagtgtttt gcgacttttt gcgacaccat gtggtcacgc 180

tgggtattta agcccgagtg agcacgcagg gtctccattt tgaagcggga ggtttgaacg 240

cgcagccacc acgggggggt tttacgagat tgtgattaag gtccccagcg accttgacgg 300

gcatctgccc ggcatttctg acagctttgt gaactgggtg gccgagaagg aatgggagtt 360

gccgccagat tctgacatgg atctgaatct gattgagcag gcacccctga ccgtggccga 420

gaagctgcag cgcgactttc tgacggaatg gcgccgtgtg agtaaggccc cggaggccct 480

tttctttgtg caatttgaga agggagagag ctacttccac atgcacgtgc tcgtggaaac 540

caccggggtg aaatccatgg ttttgggacg tttcctgagt cagattcgcg aaaaactgat 600

tcagagaatt taccgcggga tcgagccgac tttgccaaac tggttcgcgg tcacaaagac 660

cagaaatggc gccggaggcg ggaacaaggt ggtggatgag tgctacatcc ccaattactt 720

gctccccaaa acccagcctg agctccagtg ggcgtggact aatatggaac agtatttaag 780

cgcctgtttg aatctcacgg agcgtaaacg gttggtggcg cagcatctga cgcacgtgtc 840

gcagacgcag gagcagaaca aagagaatca gaatcccaat tctgatgcgc cggtgatcag 900

atcaaaaact tcagccaggt acatggagct ggtcgggtgg ctcgtggaca aggggattac 960

ctcggagaag cagtggatcc aggaggacca ggcctcatac atctccttca atgcggcctc 1020

caactcgcgg tcccaaatca aggctgcctt ggacaatgcg ggaaagatta tgagcctgac 1080

taaaaccgcc cccgactacc tggtgggcca gcagcccgtg gaggacattt ccagcaatcg 1140

gatttataaa attttggaac taaacgggta cgatccccaa tatgcggctt ccgtctttct 1200

gggatgggcc acgaaaaagt tcggcaagag gaacaccatc tggctgtttg ggcctgcaac 1260

taccgggaag accaacatcg cggaggccat agcccacact gtgcccttct acgggtgcgt 1320

aaactggacc aatgagaact ttcccttcaa cgactgtgtc gacaagatgg tgatctggtg 1380

ggaggagggg aagatgaccg ccaaggtcgt ggagtcggcc aaagccattc tcggaggaag 1440

caaggtgcgc gtggaccaga aatgcaagtc ctcggcccag atagacccga ctcccgtgat 1500

cgtcacctcc aacaccaaca tgtgcgccgt gattgacggg aactcaacga ccttcgaaca 1560

ccagcagccg ttgcaagacc ggatgttcaa atttgaactc acccgccgtc tggatcatga 1620

ctttgggaag gtcaccaagc aggaagtcaa agactttttc cggtgggcaa aggatcacgt 1680

ggttgaggtg gagcatgaat tctacgtcaa aaagggtgga gccaagaaaa gacccgcccc 1740

cagtgacgca gatataagtg agcccaaacg ggtgcgcgag tcagttgcgc agccatcgac 1800

gtcagacgcg gaagcttcga tcaactacgc agacaggtac caaaacaaat gttctcgtca 1860

cgtgggcatg aatctgatgc tgtttccctg cagacaatgc gagagaatga atcagaattc 1920

aaatatctgc ttcactcacg gacagaaaga ctgtttagag tgctttcccg tgtcagaatc 1980

tcaacccgtt tctgtcgtca aaaaggcgta tcagaaactg tgctacattc atcatatcat 2040

gggaaaggtg ccagacgctt gcactgcctg cgatctggtc aatgtggatt tggatgactg 2100

catctttgaa caataaatga tttaaatcag gtatgactga cggttacctt ccagattggc 2160

tagaggacaa cctctctgaa ggcgttcgag agtggtgggc gctgcaacct ggagccccta 2220

aacccaaggc aaatcaacaa catcaggaca acgctcgggg tcttgtgctt ccgggttaca 2280

aatacctcgg acccggcaac ggactcgaca aggggaaccc cgtcaacgca gcggacgcgg 2340

cagccctcga gcacgacaag gcctacgacc agcagctcaa ggccggtgac aacccctacc 2400

tcaagtacaa ccacgccgac gcggagttcc agcagcggct tcagggcgac acatcgtttg 2460

ggggcaacct cggcagagca gtcttccagg ccaaaaagag ggttcttgaa cctcttggtc 2520

tggttgagca agcgggtgag acggctcctg gaaagaagag accgttgatt gaatcccccc 2580

agcagcccga ctcctccacg ggtatcggca aaaaaggcaa gcagccggct aaaaagaagc 2640

tcgttttcga agacgaaact ggagcaggcg acggaccccc tgagggatca acttccggag 2700

ccatgtctga tgacagtgag atgcgtgcag cagctggcgg agctgcagtc gagggcggac 2760

aaggtgccga tggagtgggt aatgcctcgg gtgattggca ttgcgattcc acctggtctg 2820

agggccacgt cacgaccacc agcaccagaa cctgggtctt gcccacctac aacaaccacc 2880

tctacaagcg actcggagag agcctgcagt ccaacaccta caacggattc tccaccccct 2940

ggggatactt tgacttcaac cgcttccact gccacttctc accacgtgac tggcagcgac 3000

tcatcaacaa caactggggc atgcgaccca aagccatgcg ggtcaaaatc ttcaacatcc 3060

aggtcaagga ggtcacgacg tcgaacggcg agacaacggt ggctaataac cttaccagca 3120

cggttcagat ctttgcggac tcgtcgtacg aactgccgta cgtgatggat gcgggtcaag 3180

agggcagcct gcctcctttt cccaacgacg tctttatggt gccccagtac ggctactgtg 3240

gactggtgac cggcaacact tcgcagcaac agactgacag aaatgccttc tactgcctgg 3300

agtactttcc ttcgcagatg ctgcggactg gcaacaactt tgaaattacg tacagttttg 3360

agaaggtgcc tttccactcg atgtacgcgc acagccagag cctggaccgg ctgatgaacc 3420

ctctcatcga ccagtacctg tggggactgc aatcgaccac caccggaacc accctgaatg 3480

ccgggactgc caccaccaac tttaccaagc tgcggcctac caacttttcc aactttaaaa 3540

agaactggct gcccgggcct tcaatcaagc agcagggctt ctcaaagact gccaatcaaa 3600

actacaagat ccctgccacc gggtcagaca gtctcatcaa atacgagacg cacagcactc 3660

tggacggaag atggagtgcc ctgacccccg gacctccaat ggccacggct ggacctgcgg 3720

acagcaagtt cagcaacagc cagctcatct ttgcggggcc taaacagaac ggcaacacgg 3780

ccaccgtacc cgggactctg atcttcacct ctgaggagga gctggcagcc accaacgcca 3840

ccgatacgga catgtggggc aacctacctg gcggtgacca gagcaacagc aacctgccga 3900

ccgtggacag actgacagcc ttgggagccg tgcctggaat ggtctggcaa aacagagaca 3960

tttactacca gggtcccatt tgggccaaga ttcctcatac cgatggacac tttcacccct 4020

caccgctgat tggtgggttt gggctgaaac acccgcctcc tcaaattttt atcaagaaca 4080

ccccggtacc tgcgaatcct gcaacgacct tcagctctac tccggtaaac tccttcatta 4140

ctcagtacag cactggccag gtgtcggtgc agattgactg ggagatccag aaggagcggt 4200

ccaaacgctg gaaccccgag gtccagttta cctccaacta cggacagcaa aactctctgt 4260

tgtgggctcc cgatgcggct gggaaataca ctgagcctag ggctatcggt acccgctacc 4320

tcacccacca cctgtaattg cttgttaatc aataaaccgt ttaattcgtt tcagttgaac 4380

tttggtctct gcgtatttct ttcttatcta gtttccatgc tctagactac tacgtcaccc 4440

gccccgttcc cacgccccgc gccacgtcac aaactccacc ccctcattat catattggct 4500

tcaatccaaa ataaggtata ttattgatga tgcatcgctg gcgtaatagc gaagaggccc 4560

gcaccgatcg cccttcccaa cagttgcgca gcctgaatgg cgaatggaat tccagacgat 4620

tgagcgtcaa aatgtaggta tttccatgag cgtttttcct gttgcaatgg ctggcggtaa 4680

tattgttctg gatattacca gcaaggccga tagtttgagt tcttctactc aggcaagtga 4740

tgttattact aatcaaagaa gtattgcgac aacggttaat ttgcgtgatg gacagactct 4800

tttactcggt ggcctcactg attataaaaa cacttctcag gattctggcg taccgttcct 4860

gtctaaaatc cctttaatcg gcctcctgtt tagctcccgc tctgattcta acgaggaaag 4920

cacgttatac gtgctcgtca aagcaaccat agtacgcgcc ctgtagcggc gcattaagcg 4980

cggcgggtgt ggtggttacg cgcagcgtga ccgctacact tgccagcgcc ctagcgcccg 5040

ctcctttcgc tttcttccct tcctttctcg ccacgttcgc cggctttccc cgtcaagctc 5100

taaatcgggg gctcccttta gggttccgat ttagtgcttt acggcacctc gaccccaaaa 5160

aacttgatta gggtgatggt tcacgtagtg ggccatcgcc ctgatagacg gtttttcgcc 5220

ctttgacgtt ggagtccacg ttctttaata gtggactctt gttccaaact ggaacaacac 5280

tcaaccctat ctcggtctat tcttttgatt tataagggat tttgccgatt tcggcctatt 5340

ggttaaaaaa tgagctgatt taacaaaaat ttaacgcgaa ttttaacaaa atattaacgt 5400

ttacaattta aatatttgct tatacaatct tcctgttttt ggggcttttc tgattatcaa 5460

ccggggtaca tatgattgac atgctagttt tacgattacc gttcatcgat tctcttgttt 5520

gctccagact ctcaggcaat gacctgatag cctttgtaga gacctctcaa aaatagctac 5580

cctctccggc atgaatttat cagctagaac ggttgaatat catattgatg gtgatttgac 5640

tgtctccggc ctttctcacc cgtttgaatc tttacctaca cattactcag gcattgcatt 5700

taaaatatat gagggttcta aaaattttta tccttgcgtt gaaataaagg cttctcccgc 5760

aaaagtatta cagggtcata atgtttttgg tacaaccgat ttagctttat gctctgaggc 5820

tttattgctt aattttgcta attctttgcc ttgcctgtat gatttattgg atgttggaat 5880

tcctgatgcg gtattttctc cttacgcatc tgtgcggtat ttcacaccgc atatggtgca 5940

ctctcagtac aatctgctct gatgccgcat agttaagcca gccccgacac ccgccaacac 6000

ccgctgacgc gccctgacgg gcttgtctgc tcccggcatc cgcttacaga caagctgtga 6060

ccgtctccgg gagctgcatg tgtcagaggt tttcaccgtc atcaccgaaa cgcgcgagac 6120

gaaagggcct cgtgatacgc ctatttttat aggttaatgt catgataata atggtttctt 6180

agacgtcagg tggcactttt cggggaaatg tgcgcggaac ccctatttgt ttatttttct 6240

aaatacattc aaatatgtat ccgctcatga gacaataacc ctgataaatg cttcaataat 6300

attgaaaaag gaagagtatg agtattcaac atttccgtgt cgcccttatt cccttttttg 6360

cggcattttg ccttcctgtt tttgctcacc cagaaacgct ggtgaaagta aaagatgctg 6420

aagatcagtt gggtgcacga gtgggttaca tcgaactgga tctcaacagc ggtaagatcc 6480

ttgagagttt tcgccccgaa gaacgttttc caatgatgag cacttttaaa gttctgctat 6540

gtggcgcggt attatcccgt attgacgccg ggcaagagca actcggtcgc cgcatacact 6600

attctcagaa tgacttggtt gagtactcac cagtcacaga aaagcatctt acggatggca 6660

tgacagtaag agaattatgc agtgctgcca taaccatgag tgataacact gcggccaact 6720

tacttctgac aacgatcgga ggaccgaagg agctaaccgc ttttttgcac aacatggggg 6780

atcatgtaac tcgccttgat cgttgggaac cggagctgaa tgaagccata ccaaacgacg 6840

agcgtgacac cacgatgcct gtagcaatgg caacaacgtt gcgcaaacta ttaactggcg 6900

aactacttac tctagcttcc cggcaacaat taatagactg gatggaggcg gataaagttg 6960

caggaccact tctgcgctcg gcccttccgg ctggctggtt tattgctgat aaatctggag 7020

ccggtgagcg tgggtctcgc ggtatcattg cagcactggg gccagatggt aagccctccc 7080

gtatcgtagt tatctacacg acggggagtc aggcaactat ggatgaacga aatagacaga 7140

tcgctgagat aggtgcctca ctgattaagc attggtaact gtcagaccaa gtttactcat 7200

atatacttta gattgattta aaacttcatt tttaatttaa aaggatctag gtgaagatcc 7260

tttttgataa tctcatgacc aaaatccctt aacgtgagtt ttcgttccac tgagcgtcag 7320

accccgtaga aaagatcaaa ggatcttctt gagatccttt ttttctgcgc gtaatctgct 7380

gcttgcaaac aaaaaaacca ccgctaccag cggtggtttg tttgccggat caagagctac 7440

caactctttt tccgaaggta actggcttca gcagagcgca gataccaaat actgtccttc 7500

tagtgtagcc gtagttaggc caccacttca agaactctgt agcaccgcct acatacctcg 7560

ctctgctaat cctgttacca gtggctgctg ccagtggcga taagtcgtgt cttaccgggt 7620

tggactcaag acgatagtta ccggataagg cgcagcggtc gggctgaacg gggggttcgt 7680

gcacacagcc cagcttggag cgaacgacct acaccgaact gagataccta cagcgtgagc 7740

tatgagaaag cgccacgctt cccgaaggga gaaaggcgga caggtatccg gtaagcggca 7800

gggtcggaac aggagagcgc acgagggagc ttccaggggg aaacgcctgg tatctttata 7860

gtcctgtcgg gtttcgccac ctctgacttg agcgtcgatt tttgtgatgc tcgtcagggg 7920

ggcggagcct atggaaaaac gccagcaacg cggccttttt acggttcctg gccttttgct 7980

ggccttttgc tcacatgttc tttcctgcgt tatcccctga ttctgtggat aaccgtatta 8040

ccgcctttga gtgagctgat accgctcgcc gcagccgaac gaccgagcgc agcgagtcag 8100

tgagcgagga agcggaagag cgcccaatac gcaaaccgcc tctccccgcg cgttggccga 8160

ttcattaatg cag 8173

<210> 143
<211> 7226
<212> DNA
<213> Adeno-Associated Virus

<220>
<221> gene
<222> (1)..(7226)

<400> 143
cacctaaatt gtaagcgtta atattttgtt aaaattcgcg ttaaattttt gttaaatcag 60

ctcatttttt aaccaatagg ccgaaatcgg caaaatccct tataaatcaa aagaatagac 120

cgagataggg ttgagtgttg ttccagtttg gaacaagagt ccactattaa agaacgtgga 180

ctccaacgtc aaagggcgaa aaaccgtcta tcagggcgat ggcccactac gtgaaccatc 240

accctaatca agttttttgg ggtcgaggtg ccgtaaagca ctaaatcgga accctaaagg 300

gagcccccga tttagagctt gacggggaaa gccggcgaac gtggcgagaa aggaagggaa 360

gaaagcgaaa ggagcgggcg ctagggcgct ggcaagtgta gcggtcacgc tgcgcgtaac 420

caccacaccc gccgcgctta atgcgccgct acagggcgcg tcccattcgc cattcaggct 480

gcgcaactgt tgggaagggc gatcggtgcg ggcctcttcg ctattacgcc agctggcgaa 540

agggggatgt gctgcaaggc gattaagttg ggtaacgcca gggttttccc agtcacgacg 600

ttgtaaaacg acggccagtg aattgtaata cgactcacta tagggcgaat tggagctcca 660

ccgcggtggc ggccgctcta gagtcctgta ttagaggtca cgtgagtgtt ttgcgacatt 720

ttgcgacacc atgtggtcac gctgggtatt taagcccgag tgagcacgca gggtctccat 780

tttgaagcgg gaggtttgaa cgcgcagcca ccacggcggg gttttacgag attgtgatta 840

aggtccccag cgaccttgac gggcatctgc ccggcatttc tgacagcttt gtgaactggg 900

tggccgagaa ggaatgggag ttgccgccag attctgacat ggatctgaat ctgattgagc 960

aggcacccct gaccgtggcc gagaagctgc agcgcgactt tctgacggaa tggcgccgtg 1020

tgagtaaggc cccggaggcc cttttctttg tgcaatttga gaagggagag agctacttcc 1080

acatgcacgt gctcgtggaa accaccgggg tgaaatccat ggttttggga cgtttcctga 1140

gtcagattcg cgaaaaactg attcagagaa tttaccgcgg gatcgagccg actttgccaa 1200

actggttcgc ggtcacaaag accagaaatg gcgccggagg cgggaacaag gtggtggatg 1260

agtgctacat ccccaattac ttgctcccca aaacccagcc tgagctccag tgggcgtgga 1320

ctaatatgga acagtattta agcgcctgtt tgaatctcac ggagcgtaaa cggttggtgg 1380

cgcagcatct gacgcacgtg tcgcagacgc aggagcagaa caaagagaat cagaatccca 1440

attctgatgc gccggtgatc agatcaaaaa cttcagccag gtacatggag ctggtcgggt 1500

ggctcgtgga caaggggatt acctcggaga agcagtggat ccaggaggac caggcctcat 1560

acatctcctt caatgcggcc tccaactcgc ggtcccaaat caaggctgcc ttggacaatg 1620

cgggaaagat tatgagcctg actaaaaccg cccccgacta cctggtgggc cagcagcccg 1680

tggaggacat ttccagcaat cggatttata aaattttgga actaaacggg tacgatcccc 1740

aatatgcggc ttccgtcttt ctgggatggg ccacgaaaaa gttcggcaag aggaacacca 1800

tctggctgtt tgggcctgca actaccggga agaccaacat cgcggaggcc atagcccaca 1860

ctgtgccctt ctacgggtgc gtaaactgga ccaatgagaa ctttcccttc aacgactgtg 1920

tcgacaagat ggtgatctgg tgggaggagg ggaagatgac cgccaaggtc gtggagtcgg 1980

ccaaagccat tctcggagga agcaaggtgc gcgtggacca gaaatgcaag tcctcggccc 2040

agatagaccc gactcccgtg atcgtcacct ccaacaccaa catgtgcgcc gtgattgacg 2100

ggaactcaac gaccttcgaa caccagcagc cgttgcaaga ccggatgttc aaatttgaac 2160

tcacccgccg tctggatcat gactttggga aggtcaccaa gcaggaagtc aaagactttt 2220

tccggtgggc aaaggatcac gtggttgagg tggagcatga attctacgtc aaaaagggtg 2280

gagccaagaa aagacccgcc cccagtgacg cagatataag tgagcccaaa cgggtgcgcg 2340

agtcagttgc gcagccatcg acgtcagacg cggaagcttc gatcaactac gcagacaggt 2400

accaaaacaa atgttctcgt cacgtgggca tgaatctgat gctgtttccc tgcagacaat 2460

gcgagagaat gaatcagaat tcaaatatct gcttcactca cggacagaaa gactgtttag 2520

agtgctttcc cgtgtcagaa tctcaacccg tttctgtcgt caaaaaggcg tatcagaaac 2580

tgtgctacat tcatcatatc atgggaaagg tgccagacgc ttgcactgcc tgcgatctgg 2640

tcaatgtgga tttggatgac tgcatctttg aacaataaat gatttaaata gtcatgtctt 2700

ttgttgatca ccctccagat tggttggaag aagttggtga aggtcttcgc gagtttttgg 2760

gccttgaagc gggcccaccg aaaccaaaac ccaatcagca gcatcaagat caagcccgtg 2820

gtcttgtgct gcctggttat aactatctcg gacccggaaa cggtctcgat cgaggagagc 2880

ctgtcaacag ggcagacgag gtcgcgcgag agcacgacat ctcgtacaac gagcagcttg 2940

aggcgggaga caacccctac ctcaagtaca accacgcgga cgccgagttt caggagaagc 3000

tcgccgacga cacatccttc gggggaaacc tcggaaaggc agtctttcag gccaagaaaa 3060

gggttctcga accttttggc ctggttgaag agggtgctaa gacggcccct accggaaagc 3120

ggatagacga ccactttcca aaaagaaaga aggctcggac cgaagaggac tccaagcctt 3180

ccacctcgtc agacgccgaa gctggaccca gcggatccca gcagctgcaa atcccagccc 3240

aaccagcctc aagtttggga gctgatacaa tgtctgcggg aggtggcggc ccattgggcg 3300

acaataacca aggtgccgat ggagtgggca atgcctcggg agattggcat tgcgattcca 3360

cgtggatggg ggacagagtc gtcaccaagt ccacccgaac ctgggtgctg cccagctaca 3420

acaaccacca gtaccgagag atcaaaagcg gctccgtcga cggaagcaac gccaacgcct 3480

actttggata cagcaccccc tgggggtact ttgactttaa ccgcttccac agccactgga 3540

gcccccgaga ctggcaaaga ctcatcaaca actactgggg cttcagaccc cggtccctca 3600

gagtcaaaat cttcaacatt caagtcaaag aggtcacggt gcaggactcc accaccacca 3660

tcgccaacaa cctcacctcc accgtccaag tgtttacgga cgacgactac cagctgccct 3720

acgtcgtcgg caacgggacc gagggatgcc tgccggcctt ccctccgcag gtctttacgc 3780

tgccgcagta cggttacgcg acgctgaacc gcgacaacac agaaaatccc accgagagga 3840

gcagcttctt ctgcctagag tactttccca gcaagatgct gagaacgggc aacaactttg 3900

agtttaccta caactttgag gaggtgccct tccactccag cttcgctccc agtcagaacc 3960

tgttcaagct ggccaacccg ctggtggacc agtacttgta ccgcttcgtg agcacaaata 4020

acactggcgg agtccagttc aacaagaacc tggccgggag atacgccaac acctacaaaa 4080

actggttccc ggggcccatg ggccgaaccc agggctggaa cctgggctcc ggggtcaacc 4140

gcgccagtgt cagcgccttc gccacgacca ataggatgga gctcgagggc gcgagttacc 4200

aggtgccccc gcagccgaac ggcatgacca acaacctcca gggcagcaac acctatgccc 4260

tggagaacac tatgatcttc aacagccagc cggcgaaccc gggcaccacc gccacgtacc 4320

tcgagggcaa catgctcatc accagcgaga gcgagacgca gccggtgaac cgcgtggcgt 4380

acaacgtcgg cgggcagatg gccaccaaca accagagctc caccactgcc cccgcgaccg 4440

gcacgtacaa cctccaggaa atcgtgcccg gcagcgtgtg gatggagagg gacgtgtacc 4500

tccaaggacc catctgggcc aagatcccag agacgggggc gcactttcac ccctctccgg 4560

ccatgggcgg attcggactc aaacacccac cgcccatgat gctcatcaag aacacgcctg 4620

tgcccggaaa tatcaccagc ttctcggacg tgcccgtcag cagcttcatc acccagtaca 4680

gcaccgggca ggtcaccgtg gagatggagt gggagctcaa gaaggaaaac tccaagaggt 4740

ggaacccaga gatccagtac acaaacaact acaacgaccc ccagtttgtg gactttgccc 4800

cggacagcac cggggaatac agaaccacca gacctatcgg aacccgatac cttacccgac 4860

ccctttaacc cattcatgtc gcataccctc aataaaccgt gtattcgtgt cagtaaaata 4920

ctgcctcttg tggtcatttc tagaactaga actagtggat cccccgggct gcaggaattc 4980

gatatcaagc ttatcgatac cgtcgacctc gagggggggc ccggtaccca gcttttgttc 5040

cctttagtga gggttaattt cgagcttggc gtaatcatgg tcatagctgt ttcctgtgtg 5100

aaattgttat ccgctcacaa ttccacacaa catacgagcc ggaagcataa agtgtaaagc 5160

ctggggtgcc taatgagtga gctaactcac attaattgcg ttgcgctcac tgcccgcttt 5220

ccagtcggga aacctgtcgt gccagctgca ttaatgaatc ggccaacgcg cggggagagg 5280

cggtttgcgt attgggcgct cttccgcttc ctcgctcact gactcgctgc gctcggtcgt 5340

tcggctgcgg cgagcggtat cagctcactc aaaggcggta atacggttat ccacagaatc 5400

aggggataac gcaggaaaga acatgtgagc aaaaggccag caaaaggcca ggaaccgtaa 5460

aaaggccgcg ttgctggcgt ttttccatag gctccgcccc cctgacgagc atcacaaaaa 5520

tcgacgctca agtcagaggt ggcgaaaccc gacaggacta taaagatacc aggcgtttcc 5580

ccctggaagc tccctcgtgc gctctcctgt tccgaccctg ccgcttaccg gatacctgtc 5640

cgcctttctc ccttcgggaa gcgtggcgct ttctcatagc tcacgctgta ggtatctcag 5700

ttcggtgtag gtcgttcgct ccaagctggg ctgtgtgcac gaaccccccg ttcagcccga 5760

ccgctgcgcc ttatccggta actatcgtct tgagtccaac ccggtaagac acgacttatc 5820

gccactggca gcagccactg gtaacaggat tagcagagcg aggtatgtag gcggtgctac 5880

agagttcttg aagtggtggc ctaactacgg ctacactaga aggacagtat ttggtatctg 5940

cgctctgctg aagccagtta ccttcggaaa aagagttggt agctcttgat ccggcaaaca 6000

aaccaccgct ggtagcggtg gtttttttgt ttgcaagcag cagattacgc gcagaaaaaa 6060

aggatctcaa gaagatcctt tgatcttttc tacggggtct gacgctcagt ggaacgaaaa 6120

ctcacgttaa gggattttgg tcatgagatt atcaaaaagg atcttcacct agatcctttt 6180

aaattaaaaa tgaagtttta aatcaatcta aagtatatat gagtaaactt ggtctgacag 6240

ttaccaatgc ttaatcagtg aggcacctat ctcagcgatc tgtctatttc gttcatccat 6300

agttgcctga ctccccgtcg tgtagataac tacgatacgg gagggcttac catctggccc 6360

cagtgctgca atgataccgc gagacccacg ctcaccggct ccagatttat cagcaataaa 6420

ccagccagcc ggaagggccg agcgcagaag tggtcctgca actttatccg cctccatcca 6480

gtctattaat tgttgccggg aagctagagt aagtagttcg ccagttaata gtttgcgcaa 6540

cgttgttgcc attgctacag gcatcgtggt gtcacgctcg tcgtttggta tggcttcatt 6600

cagctccggt tcccaacgat caaggcgagt tacatgatcc cccatgttgt gcaaaaaagc 6660

ggttagctcc ttcggtcctc cgatcgttgt cagaagtaag ttggccgcag tgttatcact 6720

catggttatg gcagcactgc ataattctct tactgtcatg ccatccgtaa gatgcttttc 6780

tgtgactggt gagtactcaa ccaagtcatt ctgagaatag tgtatgcggc gaccgagttg 6840

ctcttgcccg gcgtcaatac gggataatac cgcgccacat agcagaactt taaaagtgct 6900

catcattgga aaacgttctt cggggcgaaa actctcaagg atcttaccgc tgttgagatc 6960

cagttcgatg taacccactc gtgcacccaa ctgatcttca gcatctttta ctttcaccag 7020

cgtttctggg tgagcaaaaa caggaaggca aaatgccgca aaaaagggaa taagggcgac 7080

acggaaatgt tgaatactca tactcttcct ttttcaatat tattgaagca tttatcaggg 7140

ttattgtctc atgagcggat acatatttga atgtatttag aaaaataaac aaataggggt 7200

tccgcgcaca tttccccgaa aagtgc 7226

<210> 144
<211> 7226
<212> DNA
<213> Adeno-Associated Virus

<220>
<221> gene
<222> (1)..(7226)

<400> 144
cacctaaatt gtaagcgtta atattttgtt aaaattcgcg ttaaattttt gttaaatcag 60

ctcatttttt aaccaatagg ccgaaatcgg caaaatccct tataaatcaa aagaatagac 120

cgagataggg ttgagtgttg ttccagtttg gaacaagagt ccactattaa agaacgtgga 180

ctccaacgtc aaagggcgaa aaaccgtcta tcagggcgat ggcccactac gtgaaccatc 240

accctaatca agttttttgg ggtcgaggtg ccgtaaagca ctaaatcgga accctaaagg 300

gagcccccga tttagagctt gacggggaaa gccggcgaac gtggcgagaa aggaagggaa 360

gaaagcgaaa ggagcgggcg ctagggcgct ggcaagtgta gcggtcacgc tgcgcgtaac 420

caccacaccc gccgcgctta atgcgccgct acagggcgcg tcccattcgc cattcaggct 480

gcgcaactgt tgggaagggc gatcggtgcg ggcctcttcg ctattacgcc agctggcgaa 540

agggggatgt gctgcaaggc gattaagttg ggtaacgcca gggttttccc agtcacgacg 600

ttgtaaaacg acggccagtg aattgtaata cgactcacta tagggcgaat tggagctcca 660

ccgcggtggc ggccgctcta gagtcctgta ttagaggtca cgtgagtgtt ttgcgacatt 720

ttgcgacacc atgtggtcac gctgggtatt taagcccgag tgagcacgca gggtctccat 780

tttgaagcgg gaggtttgaa cgcgcagcca ccacggcggg gttttacgag attgtgatta 840

aggtccccag cgaccttgac gggcatctgc ccggcatttc tgacagcttt gtgaactggg 900

tggccgagaa ggaatgggag ttgccgccag attctgacat ggatctgaat ctgattgagc 960

aggcacccct gaccgtggcc gagaagctgc agcgcgactt tctgacggaa tggcgccgtg 1020

tgagtaaggc cccggaggcc cttttctttg tgcaatttga gaagggagag agctacttcc 1080

acatgcacgt gctcgtggaa accaccgggg tgaaatccat ggttttggga cgtttcctga 1140

gtcagattcg cgaaaaactg attcagagaa tttaccgcgg gatcgagccg actttgccaa 1200

actggttcgc ggtcacaaag accagaaatg gcgccggagg cgggaacaag gtggtggatg 1260

agtgctacat ccccaattac ttgctcccca aaacccagcc tgagctccag tgggcgtgga 1320

ctaatatgga acagtattta agcgcctgtt tgaatctcac ggagcgtaaa cggttggtgg 1380

cgcagcatct gacgcacgtg tcgcagacgc aggagcagaa caaagagaat cagaatccca 1440

attctgatgc gccggtgatc agatcaaaaa cttcagccag gtacatggag ctggtcgggt 1500

ggctcgtgga caaggggatt acctcggaga agcagtggat ccaggaggac caggcctcat 1560

acatctcctt caatgcggcc tccaactcgc ggtcccaaat caaggctgcc ttggacaatg 1620

cgggaaagat tatgagcctg actaaaaccg cccccgacta cctggtgggc cagcagcccg 1680

tggaggacat ttccagcaat cggatttata aaattttgga actaaacggg tacgatcccc 1740

aatatgcggc ttccgtcttt ctgggatggg ccacgaaaaa gttcggcaag aggaacacca 1800

tctggctgtt tgggcctgca actaccggga agaccaacat cgcggaggcc atagcccaca 1860

ctgtgccctt ctacgggtgc gtaaactgga ccaatgagaa ctttcccttc aacgactgtg 1920

tcgacaagat ggtgatctgg tgggaggagg ggaagatgac cgccaaggtc gtggagtcgg 1980

ccaaagccat tctcggagga agcaaggtgc gcgtggacca gaaatgcaag tcctcggccc 2040

agatagaccc gactcccgtg atcgtcacct ccaacaccaa catgtgcgcc gtgattgacg 2100

ggaactcaac gaccttcgaa caccagcagc cgttgcaaga ccggatgttc aaatttgaac 2160

tcacccgccg tctggatcat gactttggga aggtcaccaa gcaggaagtc aaagactttt 2220

tccggtgggc aaaggatcac gtggttgagg tggagcatga attctacgtc aaaaagggtg 2280

gagccaagaa aagacccgcc cccagtgacg cagatataag tgagcccaaa cgggtgcgcg 2340

agtcagttgc gcagccatcg acgtcagacg cggaagcttc gatcaactac gcagacaggt 2400

accaaaacaa atgttctcgt cacgtgggca tgaatctgat gctgtttccc tgcagacaat 2460

gcgagagaat gaatcagaat tcaaatatct gcttcactca cggacagaaa gactgtttag 2520

agtgctttcc cgtgtcagaa tctcaacccg tttctgtcgt caaaaaggcg tatcagaaac 2580

tgtgctacat tcatcatatc atgggaaagg tgccagacgc ttgcactgcc tgcgatctgg 2640

tcaatgtgga tttggatgac tgcatctttg aacaataaat gatttaaata gtcatgtctt 2700

ttgttgatca ccctccagat tggttggaag aagttggtga aggtcttcgc gagttttggg 2760

gccttgaagc gggcccaccg aaaccaaaac ccaatcagca gcatcaagat caagcccgtg 2820

gtcttgtgct gcctggttat aactatctcg gacccggaaa cggtctcgat cgaggagagc 2880

ctgtcaacag ggcagacgag gtcgcgcgag agcacgacat ctcgtacaac gagcagcttg 2940

aggcgggaga caacccctac ctcaagtaca accacgcgga cgccgagttt caggagaagc 3000

tcgccgacga cacatccttc gggggaaacc tcggaaaggc agtctttcag gccaagaaaa 3060

gggttctcga accttttggc ctggttgaag agggtgctaa gacggcccct accggaaagc 3120

ggatagacga ccactttcca aaaagaaaga aggctcggac cgaagaggac tccaagcctt 3180

ccacctcgtc agacgccgaa gctggaccca gcggatccca gcagctgcaa atcccagccc 3240

aaccagcctc aagtttggga gctgatacaa tgtctgcggg aggtggcggc ccattgggcg 3300

acaataacca aggtgccgat ggagtgggca atgcctcggg agattggcat tgcgattcca 3360

cgtggatggg ggacagagtc gtcaccaagt ccacccgaac ctgggtgctg cccagctaca 3420

acaaccacca gtaccgagag atcaaaagcg gctccgtcga cggaagcaac gccaacgcct 3480

actttggata cagcaccccc tgggggtact ttgactttaa ccgcttccac agccactgga 3540

gcccccgaga ctggcaaaga ctcatcaaca actactgggg cttcagaccc cggtccctca 3600

gagtcaaaat cttcaacatt caagtcaaag aggtcacggt gcaggactcc accaccacca 3660

tcgccaacaa cctcacctcc accgtccaag tgtttacgga cgacgactac cagctgccct 3720

acgtcgtcgg caacgggacc gagggatgcc tgccggcctt ccctccgcag gtctttacgc 3780

tgccgcagta cggttacgcg acgctgaacc gcgacaacac agaaaatccc accgagagga 3840

gcagcttctt ctgcctagag tactttccca gcaagatgct gagaacgggc aacaactttg 3900

agtttaccta caactttgag gaggtgccct tccactccag cttcgctccc agtcagaacc 3960

tgttcaagct ggccaacccg ctggtggacc agtacttgta ccgcttcgtg agcacaaata 4020

acactggcgg agtccagttc aacaagaacc tggccgggag atacgccaac acctacaaaa 4080

actggttccc ggggcccatg ggccgaaccc agggctggaa cctgggctcc ggggtcaacc 4140

gcgccagtgt cagcgccttc gccacgacca ataggatgga gctcgagggc gcgagttacc 4200

aggtgccccc gcagccgaac ggcatgacca acaacctcca gggcagcaac acctatgccc 4260

tggagaacac tatgatcttc aacagccagc cggcgaaccc gggcaccacc gccacgtacc 4320

tcgagggcaa catgctcatc accagcgaga gcgagacgca gccggtgaac cgcgtggcgt 4380

acaacgtcgg cgggcagatg gccaccaaca accagagctc caccactgcc cccgcgaccg 4440

gcacgtacaa cctccaggaa atcgtgcccg gcagcgtgtg gatggagagg gacgtgtacc 4500

tccaaggacc catctgggcc aagatcccag agacgggggc gcactttcac ccctctccgg 4560

ccatgggcgg attcggactc aaacacccac cgcccatgat gctcatcaag aacacgcctg 4620

tgcccggaaa tatcaccagc ttctcggacg tgcccgtcag cagcttcatc acccagtaca 4680

gcaccgggca ggtcaccgtg gagatggagt gggagctcaa gaaggaaaac tccaagaggt 4740

ggaacccaga gatccagtac acaaacaact acaacgaccc ccagtttgtg gactttgccc 4800

cggacagcac cggggaatac agaaccacca gacctatcgg aacccgatac cttacccgac 4860

ccctttaacc cattcatgtc gcataccctc aataaaccgt gtattcgtgt cagtaaaata 4920

ctgcctcttg tggtcatttc tagaactaga actagtggat cccccgggct gcaggaattc 4980

gatatcaagc ttatcgatac cgtcgacctc gagggggggc ccggtaccca gcttttgttc 5040

cctttagtga gggttaattt cgagcttggc gtaatcatgg tcatagctgt ttcctgtgtg 5100

aaattgttat ccgctcacaa ttccacacaa catacgagcc ggaagcataa agtgtaaagc 5160

ctggggtgcc taatgagtga gctaactcac attaattgcg ttgcgctcac tgcccgcttt 5220

ccagtcggga aacctgtcgt gccagctgca ttaatgaatc ggccaacgcg cggggagagg 5280

cggtttgcgt attgggcgct cttccgcttc ctcgctcact gactcgctgc gctcggtcgt 5340

tcggctgcgg cgagcggtat cagctcactc aaaggcggta atacggttat ccacagaatc 5400

aggggataac gcaggaaaga acatgtgagc aaaaggccag caaaaggcca ggaaccgtaa 5460

aaaggccgcg ttgctggcgt ttttccatag gctccgcccc cctgacgagc atcacaaaaa 5520

tcgacgctca agtcagaggt ggcgaaaccc gacaggacta taaagatacc aggcgtttcc 5580

ccctggaagc tccctcgtgc gctctcctgt tccgaccctg ccgcttaccg gatacctgtc 5640

cgcctttctc ccttcgggaa gcgtggcgct ttctcatagc tcacgctgta ggtatctcag 5700

ttcggtgtag gtcgttcgct ccaagctggg ctgtgtgcac gaaccccccg ttcagcccga 5760

ccgctgcgcc ttatccggta actatcgtct tgagtccaac ccggtaagac acgacttatc 5820

gccactggca gcagccactg gtaacaggat tagcagagcg aggtatgtag gcggtgctac 5880

agagttcttg aagtggtggc ctaactacgg ctacactaga aggacagtat ttggtatctg 5940

cgctctgctg aagccagtta ccttcggaaa aagagttggt agctcttgat ccggcaaaca 6000

aaccaccgct ggtagcggtg gtttttttgt ttgcaagcag cagattacgc gcagaaaaaa 6060

aggatctcaa gaagatcctt tgatcttttc tacggggtct gacgctcagt ggaacgaaaa 6120

ctcacgttaa gggattttgg tcatgagatt atcaaaaagg atcttcacct agatcctttt 6180

aaattaaaaa tgaagtttta aatcaatcta aagtatatat gagtaaactt ggtctgacag 6240

ttaccaatgc ttaatcagtg aggcacctat ctcagcgatc tgtctatttc gttcatccat 6300

agttgcctga ctccccgtcg tgtagataac tacgatacgg gagggcttac catctggccc 6360

cagtgctgca atgataccgc gagacccacg ctcaccggct ccagatttat cagcaataaa 6420

ccagccagcc ggaagggccg agcgcagaag tggtcctgca actttatccg cctccatcca 6480

gtctattaat tgttgccggg aagctagagt aagtagttcg ccagttaata gtttgcgcaa 6540

cgttgttgcc attgctacag gcatcgtggt gtcacgctcg tcgtttggta tggcttcatt 6600

cagctccggt tcccaacgat caaggcgagt tacatgatcc cccatgttgt gcaaaaaagc 6660

ggttagctcc ttcggtcctc cgatcgttgt cagaagtaag ttggccgcag tgttatcact 6720

catggttatg gcagcactgc ataattctct tactgtcatg ccatccgtaa gatgcttttc 6780

tgtgactggt gagtactcaa ccaagtcatt ctgagaatag tgtatgcggc gaccgagttg 6840

ctcttgcccg gcgtcaatac gggataatac cgcgccacat agcagaactt taaaagtgct 6900

catcattgga aaacgttctt cggggcgaaa actctcaagg atcttaccgc tgttgagatc 6960

cagttcgatg taacccactc gtgcacccaa ctgatcttca gcatctttta ctttcaccag 7020

cgtttctggg tgagcaaaaa caggaaggca aaatgccgca aaaaagggaa taagggcgac 7080

acggaaatgt tgaatactca tactcttcct ttttcaatat tattgaagca tttatcaggg 7140

ttattgtctc atgagcggat acatatttga atgtatttag aaaaataaac aaataggggt 7200

tccgcgcaca tttccccgaa aagtgc 7226

<210> 145
<211> 8182
<212> DNA
<213> Adeno-Associated Virus

<220>
<221> gene
<222> (1)..(8182)

<400> 145
aattcccatc atcaataata taccttattt aggattgaag ccaatatgat aatgagccgg 60

tggagtttgt gacgtggcgc ggggcgtggg aacggggcgg gtgacgtagt agtctctaga 120

gtcctgtatt agaggtcacg tgagtgtttt gcgacttttt gcgacaccat gtggtcacgc 180

tgggtattta agcccgagtg agcacgcagg gtctccattt tgaagcggga ggtttgaacg 240

cgcagccacc acgggggggt tttacgagat tgtgattaag gtccccagcg accttgacgg 300

gcatctgccc ggcatttctg acagctttgt gaactgggtg gccgagaagg aatgggagtt 360

gccgccagat tctgacatgg atctgaatct gattgagcag gcacccctga ccgtggccga 420

gaagctgcag cgcgactttc tgacggaatg gcgccgtgtg agtaaggccc cggaggccct 480

tttctttgtg caatttgaga agggagagag ctacttccac atgcacgtgc tcgtggaaac 540

caccggggtg aaatccatgg ttttgggacg tttcctgagt cagattcgcg aaaaactgat 600

tcagagaatt taccgcggga tcgagccgac tttgccaaac tggttcgcgg tcacaaagac 660

cagaaatggc gccggaggcg ggaacaaggt ggtggatgag tgctacatcc ccaattactt 720

gctccccaaa acccagcctg agctccagtg ggcgtggact aatatggaac agtatttaag 780

cgcctgtttg aatctcacgg agcgtaaacg gttggtggcg cagcatctga cgcacgtgtc 840

gcagacgcag gagcagaaca aagagaatca gaatcccaat tctgatgcgc cggtgatcag 900

atcaaaaact tcagccaggt acatggagct ggtcgggtgg ctcgtggaca aggggattac 960

ctcggagaag cagtggatcc aggaggacca ggcctcatac atctccttca atgcggcctc 1020

caactcgcgg tcccaaatca aggctgcctt ggacaatgcg ggaaagatta tgagcctgac 1080

taaaaccgcc cccgactacc tggtgggcca gcagcccgtg gaggacattt ccagcaatcg 1140

gatttataaa attttggaac taaacgggta cgatccccaa tatgcggctt ccgtctttct 1200

gggatgggcc acgaaaaagt tcggcaagag gaacaccatc tggctgtttg ggcctgcaac 1260

taccgggaag accaacatcg cggaggccat agcccacact gtgcccttct acgggtgcgt 1320

aaactggacc aatgagaact ttcccttcaa cgactgtgtc gacaagatgg tgatctggtg 1380

ggaggagggg aagatgaccg ccaaggtcgt ggagtcggcc aaagccattc tcggaggaag 1440

caaggtgcgc gtggaccaga aatgcaagtc ctcggcccag atagacccga ctcccgtgat 1500

cgtcacctcc aacaccaaca tgtgcgccgt gattgacggg aactcaacga ccttcgaaca 1560

ccagcagccg ttgcaagacc ggatgttcaa atttgaactc acccgccgtc tggatcatga 1620

ctttgggaag gtcaccaagc aggaagtcaa agactttttc cggtgggcaa aggatcacgt 1680

ggttgaggtg gagcatgaat tctacgtcaa aaagggtgga gccaagaaaa gacccgcccc 1740

cagtgacgca gatataagtg agcccaaacg ggtgcgcgag tcagttgcgc agccatcgac 1800

gtcagacgcg gaagcttcga tcaactacgc agacaggtac caaaacaaat gttctcgtca 1860

cgtgggcatg aatctgatgc tgtttccctg cagacaatgc gagagaatga atcagaattc 1920

aaatatctgc ttcactcacg gacagaaaga ctgtttagag tgctttcccg tgtcagaatc 1980

tcaacccgtt tctgtcgtca aaaaggcgta tcagaaactg tgctacattc atcatatcat 2040

gggaaaggtg ccagacgctt gcactgcctg cgatctggtc aatgtggatt tggatgactg 2100

catctttgaa caataaatga tttaaatcag gtatggctgc cgatggttat cttccagatt 2160

ggctcgagga caacctctct gagggcattc gcgagtggtg ggacctgaaa cctggagccc 2220

cgaaacccaa agccaaccag caaaagcagg acaacggccg gggtctggtg cttcctggct 2280

acaagtacct cggacccttc aacggactcg acaaggggga gcccgtcaac gcggcggacg 2340

cagcggccct cgagcacgac aaggcctacg accagcagct caaagcgggt gacaatccgt 2400

acctgcggta taaccacgcc gacgccgagt ttcaggagcg tctgcaagaa gatacgtcat 2460

ttgggggcaa cctcggcgga gcagtcttcc aggccaagaa gcgggttctc gaacctctcg 2520

gtctggttga ggaaggcgct aagacggctc ctgcaaagaa gagaccggta gagccgtcac 2580

ctcagcgttc ccccgactcc tccacgggca tcggcaagaa aggccagcag cccgccagaa 2640

agagactcaa tttcggtcag actggcgact cagagtcagt ccccgaccct caacctctcg 2700

gagaacctcc agcagcgccc tctagtgtgg gatctggtac agtggctgca ggcggtggcg 2760

caccaatggc agacaataac gaaggtgccg acggagtggg taatgcctca ggaaattggc 2820

attgcgattc cacatggctg ggcgacagag tcattaccac cagcacccga acctgggccc 2880

tgcccaccta caacaaccac ctctacaagc aaatctccag tgaaactgca ggtagtacca 2940

acgacaacac ctacttcggc tacagcaccc cctgggggta ttttgacttt aacagattcc 3000

actgccactt ctcaccacgt gactggcagc gactcatcaa caacaactgg ggattccggc 3060

ccaagaagct gcggttcaag ctcttcaaca tccaggtcaa ggaggtcacg acgaatgacg 3120

gcgttacgac catcgctaat aaccttacca gcacgattca ggtattctcg gactcggaat 3180

accagctgcc gtacgtcctc ggctctgcgc accagggctg cctgcctccg ttcccggcgg 3240

acgtcttcat gattcctcag tacggctacc tgactctcaa caatggcagt cagtctgtgg 3300

gacgttcctc cttctactgc ctggagtact tcccctctca gatgctgaga acgggcaaca 3360

actttgagtt cagctacagc ttcgaggacg tgcctttcca cagcagctac gcacacagcc 3420

agagcctgga ccggctgatg aatcccctca tcgaccagta cttgtactac ctggccagaa 3480

cacagagtaa cccaggaggc acagctggca atcgggaact gcagttttac cagggcgggc 3540

cttcaactat ggccgaacaa gccaagaatt ggttacctgg accttgcttc cggcaacaaa 3600

gagtctccaa aacgctggat caaaacaaca acagcaactt tgcttggact ggtgccacca 3660

aatatcacct gaacggcaga aactcgttgg ttaatcccgg cgtcgccatg gcaactcaca 3720

aggacgacga ggaccgcttt ttcccatcca gcggagtcct gatttttgga aaaactggag 3780

caactaacaa aactacattg gaaaatgtgt taatgacaaa tgaagaagaa attcgtccta 3840

ctaatcctgt agccacggaa gaatacggga tagtcagcag caacttacaa gcggctaata 3900

ctgcagccca gacacaagtt gtcaacaacc agggagcctt acctggcatg gtctggcaga 3960

accgggacgt gtacctgcag ggtcccatct gggccaagat tcctcacacg gatggcaact 4020

ttcacccgtc tcctttgatg ggcggctttg gacttaaaca tccgcctcct cagatcctga 4080

tcaagaacac tcccgttccc gctaatcctc cggaggtgtt tactcctgcc aagtttgctt 4140

cgttcatcac acagtacagc accggacaag tcagcgtgga aatcgagtgg gagctgcaga 4200

aggaaaacag caagcgctgg aacccggaga ttcagtacac ctccaacttt gaaaagcaga 4260

ctggtgtgga ctttgccgtt gacagccagg gtgtttactc tgagcctcgc cctattggca 4320

ctcgttacct cacccgtaat ctgtaattgc ttgttaatca ataaaccgtt taattcgttt 4380

cagttgaact ttggtctctg cgtatttctt tcttatctag tttccatgct ctagactact 4440

acgtcacccg ccccgttccc acgccccgcg ccacgtcaca aactccaccc cctcattatc 4500

atattggctt caatccaaaa taaggtatat tattgatgat gcatcgctgg cgtaatagcg 4560

aagaggcccg caccgatcgc ccttcccaac agttgcgcag cctgaatggc gaatggaatt 4620

ccagacgatt gagcgtcaaa atgtaggtat ttccatgagc gtttttcctg ttgcaatggc 4680

tggcggtaat attgttctgg atattaccag caaggccgat agtttgagtt cttctactca 4740

ggcaagtgat gttattacta atcaaagaag tattgcgaca acggttaatt tgcgtgatgg 4800

acagactctt ttactcggtg gcctcactga ttataaaaac acttctcagg attctggcgt 4860

accgttcctg tctaaaatcc ctttaatcgg cctcctgttt agctcccgct ctgattctaa 4920

cgaggaaagc acgttatacg tgctcgtcaa agcaaccata gtacgcgccc tgtagcggcg 4980

cattaagcgc ggcgggtgtg gtggttacgc gcagcgtgac cgctacactt gccagcgccc 5040

tagcgcccgc tcctttcgct ttcttccctt cctttctcgc cacgttcgcc ggctttcccc 5100

gtcaagctct aaatcggggg ctccctttag ggttccgatt tagtgcttta cggcacctcg 5160

accccaaaaa acttgattag ggtgatggtt cacgtagtgg gccatcgccc tgatagacgg 5220

tttttcgccc tttgacgttg gagtccacgt tctttaatag tggactcttg ttccaaactg 5280

gaacaacact caaccctatc tcggtctatt cttttgattt ataagggatt ttgccgattt 5340

cggcctattg gttaaaaaat gagctgattt aacaaaaatt taacgcgaat tttaacaaaa 5400

tattaacgtt tacaatttaa atatttgctt atacaatctt cctgtttttg gggcttttct 5460

gattatcaac cggggtacat atgattgaca tgctagtttt acgattaccg ttcatcgatt 5520

ctcttgtttg ctccagactc tcaggcaatg acctgatagc ctttgtagag acctctcaaa 5580

aatagctacc ctctccggca tgaatttatc agctagaacg gttgaatatc atattgatgg 5640

tgatttgact gtctccggcc tttctcaccc gtttgaatct ttacctacac attactcagg 5700

cattgcattt aaaatatatg agggttctaa aaatttttat ccttgcgttg aaataaaggc 5760

ttctcccgca aaagtattac agggtcataa tgtttttggt acaaccgatt tagctttatg 5820

ctctgaggct ttattgctta attttgctaa ttctttgcct tgcctgtatg atttattgga 5880

tgttggaatt cctgatgcgg tattttctcc ttacgcatct gtgcggtatt tcacaccgca 5940

tatggtgcac tctcagtaca atctgctctg atgccgcata gttaagccag ccccgacacc 6000

cgccaacacc cgctgacgcg ccctgacggg cttgtctgct cccggcatcc gcttacagac 6060

aagctgtgac cgtctccggg agctgcatgt gtcagaggtt ttcaccgtca tcaccgaaac 6120

gcgcgagacg aaagggcctc gtgatacgcc tatttttata ggttaatgtc atgataataa 6180

tggtttctta gacgtcaggt ggcacttttc ggggaaatgt gcgcggaacc cctatttgtt 6240

tatttttcta aatacattca aatatgtatc cgctcatgag acaataaccc tgataaatgc 6300

ttcaataata ttgaaaaagg aagagtatga gtattcaaca tttccgtgtc gcccttattc 6360

ccttttttgc ggcattttgc cttcctgttt ttgctcaccc agaaacgctg gtgaaagtaa 6420

aagatgctga agatcagttg ggtgcacgag tgggttacat cgaactggat ctcaacagcg 6480

gtaagatcct tgagagtttt cgccccgaag aacgttttcc aatgatgagc acttttaaag 6540

ttctgctatg tggcgcggta ttatcccgta ttgacgccgg gcaagagcaa ctcggtcgcc 6600

gcatacacta ttctcagaat gacttggttg agtactcacc agtcacagaa aagcatctta 6660

cggatggcat gacagtaaga gaattatgca gtgctgccat aaccatgagt gataacactg 6720

cggccaactt acttctgaca acgatcggag gaccgaagga gctaaccgct tttttgcaca 6780

acatggggga tcatgtaact cgccttgatc gttgggaacc ggagctgaat gaagccatac 6840

caaacgacga gcgtgacacc acgatgcctg tagcaatggc aacaacgttg cgcaaactat 6900

taactggcga actacttact ctagcttccc ggcaacaatt aatagactgg atggaggcgg 6960

ataaagttgc aggaccactt ctgcgctcgg cccttccggc tggctggttt attgctgata 7020

aatctggagc cggtgagcgt gggtctcgcg gtatcattgc agcactgggg ccagatggta 7080

agccctcccg tatcgtagtt atctacacga cggggagtca ggcaactatg gatgaacgaa 7140

atagacagat cgctgagata ggtgcctcac tgattaagca ttggtaactg tcagaccaag 7200

tttactcata tatactttag attgatttaa aacttcattt ttaatttaaa aggatctagg 7260

tgaagatcct ttttgataat ctcatgacca aaatccctta acgtgagttt tcgttccact 7320

gagcgtcaga ccccgtagaa aagatcaaag gatcttcttg agatcctttt tttctgcgcg 7380

taatctgctg cttgcaaaca aaaaaaccac cgctaccagc ggtggtttgt ttgccggatc 7440

aagagctacc aactcttttt ccgaaggtaa ctggcttcag cagagcgcag ataccaaata 7500

ctgtccttct agtgtagccg tagttaggcc accacttcaa gaactctgta gcaccgccta 7560

catacctcgc tctgctaatc ctgttaccag tggctgctgc cagtggcgat aagtcgtgtc 7620

ttaccgggtt ggactcaaga cgatagttac cggataaggc gcagcggtcg ggctgaacgg 7680

ggggttcgtg cacacagccc agcttggagc gaacgaccta caccgaactg agatacctac 7740

agcgtgagct atgagaaagc gccacgcttc ccgaagggag aaaggcggac aggtatccgg 7800

taagcggcag ggtcggaaca ggagagcgca cgagggagct tccaggggga aacgcctggt 7860

atctttatag tcctgtcggg tttcgccacc tctgacttga gcgtcgattt ttgtgatgct 7920

cgtcaggggg gcggagccta tggaaaaacg ccagcaacgc ggccttttta cggttcctgg 7980

ccttttgctg gccttttgct cacatgttct ttcctgcgtt atcccctgat tctgtggata 8040

accgtattac cgcctttgag tgagctgata ccgctcgccg cagccgaacg accgagcgca 8100

gcgagtcagt gagcgaggaa gcggaagagc gcccaatacg caaaccgcct ctccccgcgc 8160

gttggccgat tcattaatgc ag 8182

<210> 146
<211> 8185
<212> DNA
<213> Adeno-Associated Virus

<220>
<221> gene
<222> (1)..(8185)

<400> 146
aattcccatc atcaataata taccttattt aggattgaag ccaatatgat aatgagccgg 60

tggagtttgt gacgtggcgc ggggcgtggg aacggggcgg gtgacgtagt agtctctaga 120

gtcctgtatt agaggtcacg tgagtgtttt gcgacttttt gcgacaccat gtggtcacgc 180

tgggtattta agcccgagtg agcacgcagg gtctccattt tgaagcggga ggtttgaacg 240

cgcagccacc acgggggggt tttacgagat tgtgattaag gtccccagcg accttgacgg 300

gcatctgccc ggcatttctg acagctttgt gaactgggtg gccgagaagg aatgggagtt 360

gccgccagat tctgacatgg atctgaatct gattgagcag gcacccctga ccgtggccga 420

gaagctgcag cgcgactttc tgacggaatg gcgccgtgtg agtaaggccc cggaggccct 480

tttctttgtg caatttgaga agggagagag ctacttccac atgcacgtgc tcgtggaaac 540

caccggggtg aaatccatgg ttttgggacg tttcctgagt cagattcgcg aaaaactgat 600

tcagagaatt taccgcggga tcgagccgac tttgccaaac tggttcgcgg tcacaaagac 660

cagaaatggc gccggaggcg ggaacaaggt ggtggatgag tgctacatcc ccaattactt 720

gctccccaaa acccagcctg agctccagtg ggcgtggact aatatggaac agtatttaag 780

cgcctgtttg aatctcacgg agcgtaaacg gttggtggcg cagcatctga cgcacgtgtc 840

gcagacgcag gagcagaaca aagagaatca gaatcccaat tctgatgcgc cggtgatcag 900

atcaaaaact tcagccaggt acatggagct ggtcgggtgg ctcgtggaca aggggattac 960

ctcggagaag cagtggatcc aggaggacca ggcctcatac atctccttca atgcggcctc 1020

caactcgcgg tcccaaatca aggctgcctt ggacaatgcg ggaaagatta tgagcctgac 1080

taaaaccgcc cccgactacc tggtgggcca gcagcccgtg gaggacattt ccagcaatcg 1140

gatttataaa attttggaac taaacgggta cgatccccaa tatgcggctt ccgtctttct 1200

gggatgggcc acgaaaaagt tcggcaagag gaacaccatc tggctgtttg ggcctgcaac 1260

taccgggaag accaacatcg cggaggccat agcccacact gtgcccttct acgggtgcgt 1320

aaactggacc aatgagaact ttcccttcaa cgactgtgtc gacaagatgg tgatctggtg 1380

ggaggagggg aagatgaccg ccaaggtcgt ggagtcggcc aaagccattc tcggaggaag 1440

caaggtgcgc gtggaccaga aatgcaagtc ctcggcccag atagacccga ctcccgtgat 1500

cgtcacctcc aacaccaaca tgtgcgccgt gattgacggg aactcaacga ccttcgaaca 1560

ccagcagccg ttgcaagacc ggatgttcaa atttgaactc acccgccgtc tggatcatga 1620

ctttgggaag gtcaccaagc aggaagtcaa agactttttc cggtgggcaa aggatcacgt 1680

ggttgaggtg gagcatgaat tctacgtcaa aaagggtgga gccaagaaaa gacccgcccc 1740

cagtgacgca gatataagtg agcccaaacg ggtgcgcgag tcagttgcgc agccatcgac 1800

gtcagacgcg gaagcttcga tcaactacgc agacaggtac caaaacaaat gttctcgtca 1860

cgtgggcatg aatctgatgc tgtttccctg cagacaatgc gagagaatga atcagaattc 1920

aaatatctgc ttcactcacg gacagaaaga ctgtttagag tgctttcccg tgtcagaatc 1980

tcaacccgtt tctgtcgtca aaaaggcgta tcagaaactg tgctacattc atcatatcat 2040

gggaaaggtg ccagacgctt gcactgcctg cgatctggtc aatgtggatt tggatgactg 2100

catctttgaa caataaatga tttaaatcag gtatggctgc cgatggttat cttccagatt 2160

ggctcgagga caacctctct gagggcattc gcgagtggtg ggcgctgaaa cctggagccc 2220

cgaagcccaa agccaaccag caaaagcagg acgacggccg gggtctggtg cttcctggct 2280

acaagtacct cggacccttc aacggactcg acaaggggga gcccgtcaac gcggcggacg 2340

cagcggccct ggagcacgac aaggcctacg accagcagct gcaggcgggt gacaatccgt 2400

acctgcggta taaccacgcc gacgccgagt ttcaggagcg tctgcaagaa gatacgtctt 2460

ttgggggcaa cctcgggcga gcagtcttcc aggccaagaa gcgggttctc gaacctctcg 2520

gtctggttga ggaaggcgct aagacggctc ctggaaagaa gagaccggta gagccatcac 2580

cccagcgttc tccagactcc tctacgggca tcggcaagaa aggccaacag cccgccagaa 2640

aaagactcaa ttttggtcag actggcgact cagagtcagt tccagaccct caacctctcg 2700

gagaacctcc agcagcgccc tctggtgtgg gacctaatac aatggctgca ggcggtggcg 2760

caccaatggc agacaataac gaaggcgccg acggagtggg tagttcctcg ggaaattggc 2820

attgcgattc cacatggctg ggcgacagag tcatcaccac cagcacccga acctgggccc 2880

tgcccaccta caacaaccac ctctacaagc aaatctccaa cgggacatcg ggaggagcca 2940

ccaacgacaa cacctacttc ggctacagca ccccctgggg gtattttgac tttaacagat 3000

tccactgcca cttttcacca cgtgactggc agcgactcat caacaacaac tggggattcc 3060

ggcccaagag actcagcttc aagctcttca acatccaggt caaggaggtc acgcagaatg 3120

aaggcaccaa gaccatcgcc aataacctca ccagcaccat ccaggtgttt acggactcgg 3180

agtaccagct gccgtacgtt ctcggctctg cccaccaggg ctgcctgcct ccgttcccgg 3240

cggacgtgtt catgattccc cagtacggct acctaacact caacaacggt agtcaggccg 3300

tgggacgctc ctccttctac tgcctggaat actttccttc gcagatgctg agaaccggca 3360

acaacttcca gtttacttac accttcgagg acgtgccttt ccacagcagc tacgcccaca 3420

gccagagctt ggaccggctg atgaatcctc tgattgacca gtacctgtac tacttgtctc 3480

ggactcaaac aacaggaggc acggcaaata cgcagactct gggcttcagc caaggtgggc 3540

ctaatacaat ggccaatcag gcaaagaact ggctgccagg accctgttac cgccaacaac 3600

gcgtctcaac gacaaccggg caaaacaaca atagcaactt tgcctggact gctgggacca 3660

aataccatct gaatggaaga aattcattgg ctaatcctgg catcgctatg gcaacacaca 3720

aagacgacga ggagcgtttt tttcccagta acgggatcct gatttttggc aaacaaaatg 3780

ctgccagaga caatgcggat tacagcgatg tcatgctcac cagcgaggaa gaaatcaaaa 3840

ccactaaccc tgtggctaca gaggaatacg gtatcgtggc agataacttg cagcagcaaa 3900

acacggctcc tcaaattgga actgtcaaca gccagggggc cttacccggt atggtctggc 3960

agaaccggga cgtgtacctg cagggtccca tctgggccaa gattcctcac acggacggca 4020

acttccaccc gtctccgctg atgggcggct ttggcctgaa acatcctccg cctcagatcc 4080

tgatcaagaa cacgcctgta cctgcggatc ctccgaccac cttcaaccag tcaaagctga 4140

actctttcat cacgcaatac agcaccggac aggtcagcgt ggaaattgaa tgggagctgc 4200

agaaggaaaa cagcaagcgc tggaaccccg agatccagta cacctccaac tactacaaat 4260

ctacaagtgt ggactttgct gttaatacag aaggcgtgta ctctgaaccc cgccccattg 4320

gcacccgtta cctcacccgt aatctgtaat tgcttgttaa tcaataaacc gtttaattcg 4380

tttcagttga actttggtct ctgcgtattt ctttcttatc tagtttccat gctctagact 4440

actacgtcac ccgccccgtt cccacgcccc gcgccacgtc acaaactcca ccccctcatt 4500

atcatattgg cttcaatcca aaataaggta tattattgat gatgcatcgc tggcgtaata 4560

gcgaagaggc ccgcaccgat cgcccttccc aacagttgcg cagcctgaat ggcgaatgga 4620

attccagacg attgagcgtc aaaatgtagg tatttccatg agcgtttttc ctgttgcaat 4680

ggctggcggt aatattgttc tggatattac cagcaaggcc gatagtttga gttcttctac 4740

tcaggcaagt gatgttatta ctaatcaaag aagtattgcg acaacggtta atttgcgtga 4800

tggacagact cttttactcg gtggcctcac tgattataaa aacacttctc aggattctgg 4860

cgtaccgttc ctgtctaaaa tccctttaat cggcctcctg tttagctccc gctctgattc 4920

taacgaggaa agcacgttat acgtgctcgt caaagcaacc atagtacgcg ccctgtagcg 4980

gcgcattaag cgcggcgggt gtggtggtta cgcgcagcgt gaccgctaca cttgccagcg 5040

ccctagcgcc cgctcctttc gctttcttcc cttcctttct cgccacgttc gccggctttc 5100

cccgtcaagc tctaaatcgg gggctccctt tagggttccg atttagtgct ttacggcacc 5160

tcgaccccaa aaaacttgat tagggtgatg gttcacgtag tgggccatcg ccctgataga 5220

cggtttttcg ccctttgacg ttggagtcca cgttctttaa tagtggactc ttgttccaaa 5280

ctggaacaac actcaaccct atctcggtct attcttttga tttataaggg attttgccga 5340

tttcggccta ttggttaaaa aatgagctga tttaacaaaa atttaacgcg aattttaaca 5400

aaatattaac gtttacaatt taaatatttg cttatacaat cttcctgttt ttggggcttt 5460

tctgattatc aaccggggta catatgattg acatgctagt tttacgatta ccgttcatcg 5520

attctcttgt ttgctccaga ctctcaggca atgacctgat agcctttgta gagacctctc 5580

aaaaatagct accctctccg gcatgaattt atcagctaga acggttgaat atcatattga 5640

tggtgatttg actgtctccg gcctttctca cccgtttgaa tctttaccta cacattactc 5700

aggcattgca tttaaaatat atgagggttc taaaaatttt tatccttgcg ttgaaataaa 5760

ggcttctccc gcaaaagtat tacagggtca taatgttttt ggtacaaccg atttagcttt 5820

atgctctgag gctttattgc ttaattttgc taattctttg ccttgcctgt atgatttatt 5880

ggatgttgga attcctgatg cggtattttc tccttacgca tctgtgcggt atttcacacc 5940

gcatatggtg cactctcagt acaatctgct ctgatgccgc atagttaagc cagccccgac 6000

acccgccaac acccgctgac gcgccctgac gggcttgtct gctcccggca tccgcttaca 6060

gacaagctgt gaccgtctcc gggagctgca tgtgtcagag gttttcaccg tcatcaccga 6120

aacgcgcgag acgaaagggc ctcgtgatac gcctattttt ataggttaat gtcatgataa 6180

taatggtttc ttagacgtca ggtggcactt ttcggggaaa tgtgcgcgga acccctattt 6240

gtttattttt ctaaatacat tcaaatatgt atccgctcat gagacaataa ccctgataaa 6300

tgcttcaata atattgaaaa aggaagagta tgagtattca acatttccgt gtcgccctta 6360

ttcccttttt tgcggcattt tgccttcctg tttttgctca cccagaaacg ctggtgaaag 6420

taaaagatgc tgaagatcag ttgggtgcac gagtgggtta catcgaactg gatctcaaca 6480

gcggtaagat ccttgagagt tttcgccccg aagaacgttt tccaatgatg agcactttta 6540

aagttctgct atgtggcgcg gtattatccc gtattgacgc cgggcaagag caactcggtc 6600

gccgcataca ctattctcag aatgacttgg ttgagtactc accagtcaca gaaaagcatc 6660

ttacggatgg catgacagta agagaattat gcagtgctgc cataaccatg agtgataaca 6720

ctgcggccaa cttacttctg acaacgatcg gaggaccgaa ggagctaacc gcttttttgc 6780

acaacatggg ggatcatgta actcgccttg atcgttggga accggagctg aatgaagcca 6840

taccaaacga cgagcgtgac accacgatgc ctgtagcaat ggcaacaacg ttgcgcaaac 6900

tattaactgg cgaactactt actctagctt cccggcaaca attaatagac tggatggagg 6960

cggataaagt tgcaggacca cttctgcgct cggcccttcc ggctggctgg tttattgctg 7020

ataaatctgg agccggtgag cgtgggtctc gcggtatcat tgcagcactg gggccagatg 7080

gtaagccctc ccgtatcgta gttatctaca cgacggggag tcaggcaact atggatgaac 7140

gaaatagaca gatcgctgag ataggtgcct cactgattaa gcattggtaa ctgtcagacc 7200

aagtttactc atatatactt tagattgatt taaaacttca tttttaattt aaaaggatct 7260

aggtgaagat cctttttgat aatctcatga ccaaaatccc ttaacgtgag ttttcgttcc 7320

actgagcgtc agaccccgta gaaaagatca aaggatcttc ttgagatcct ttttttctgc 7380

gcgtaatctg ctgcttgcaa acaaaaaaac caccgctacc agcggtggtt tgtttgccgg 7440

atcaagagct accaactctt tttccgaagg taactggctt cagcagagcg cagataccaa 7500

atactgtcct tctagtgtag ccgtagttag gccaccactt caagaactct gtagcaccgc 7560

ctacatacct cgctctgcta atcctgttac cagtggctgc tgccagtggc gataagtcgt 7620

gtcttaccgg gttggactca agacgatagt taccggataa ggcgcagcgg tcgggctgaa 7680

cggggggttc gtgcacacag cccagcttgg agcgaacgac ctacaccgaa ctgagatacc 7740

tacagcgtga gctatgagaa agcgccacgc ttcccgaagg gagaaaggcg gacaggtatc 7800

cggtaagcgg cagggtcgga acaggagagc gcacgaggga gcttccaggg ggaaacgcct 7860

ggtatcttta tagtcctgtc gggtttcgcc acctctgact tgagcgtcga tttttgtgat 7920

gctcgtcagg ggggcggagc ctatggaaaa acgccagcaa cgcggccttt ttacggttcc 7980

tggccttttg ctggcctttt gctcacatgt tctttcctgc gttatcccct gattctgtgg 8040

ataaccgtat taccgccttt gagtgagctg ataccgctcg ccgcagccga acgaccgagc 8100

gcagcgagtc agtgagcgag gaagcggaag agcgcccaat acgcaaaccg cctctccccg 8160

cgcgttggcc gattcattaa tgcag 8185

<210> 147
<211> 8261
<212> DNA
<213> Adeno-Associated Virus

<220>
<221> gene
<222> (1)..(8261)

<400> 147
acggcggggt tttacgagat tgtgattaag gtccccagcg accttgacgg gcatctgccc 60

ggcatttctg acagctttgt gaactgggtg gccgagaagg aatgggagtt gccgccagat 120

tctgacatgg atctgaatct gattgagcag gcacccctga ccgtggccga gaagctgcag 180

cgcgactttc tgacggaatg gcgccgtgtg agtaaggccc cggaggccct tttctttgtg 240

caatttgaga agggagagag ctacttccac atgcacgtgc tcgtggaaac caccggggtg 300

aaatccatgg ttttgggacg tttcctgagt cagattcgcg aaaaactgat tcagagaatt 360

taccgcggga tcgagccgac tttgccaaac tggttcgcgg tcacaaagac cagaaatggc 420

gccggaggcg ggaacaaggt ggtggatgag tgctacatcc ccaattactt gctccccaaa 480

acccagcctg agctccagtg ggcgtggact aatatggaac agtatttaag cgcctgtttg 540

aatctcacgg agcgtaaacg gttggtggcg cagcatctga cgcacgtgtc gcagacgcag 600

gagcagaaca aagagaatca gaatcccaat tctgatgcgc cggtgatcag atcaaaaact 660

tcagccaggt acatggagct ggtcgggtgg ctcgtggaca aggggattac ctcggagaag 720

cagtggatcc aggaggacca ggcctcatac atctccttca atgcggcctc caactcgcgg 780

tcccaaatca aggctgcctt ggacaatgcg ggaaagatta tgagcctgac taaaaccgcc 840

cccgactacc tggtgggcca gcagcccgtg gaggacattt ccagcaatcg gatttataaa 900

attttggaac taaacgggta cgatccccaa tatgcggctt ccgtctttct gggatgggcc 960
acgaaaaagt tcggcaagag gaacaccatc tggctgtttg ggcctgcaac taccgggaag 1020
accaacatcg cggaggccat agcccacact gtgcccttct acgggtgcgt aaactggacc 1080
aatgagaact ttcccttcaa cgactgtgtc gacaagatgg tgatctggtg ggaggagggg 1140
aagatgaccg ccaaggtcgt ggagtcggcc aaagccattc tcggaggaag caaggtgcgc 1200
gtggaccaga aatgcaagtc ctcggcccag atagacccga ctcccgtgat cgtcacctcc 1260
aacaccaaca tgtgcgccgt gattgacggg aactcaacga ccttcgaaca ccagcagccg 1320
ttgcaagacc ggatgttcaa atttgaactc acccgccgtc tggatcatga ctttgggaag 1380
gtcaccaagc aggaagtcaa agactttttc cggtgggcaa aggatcacgt ggttgaggtg 1440
gagcatgaat tctacgtcaa aaagggtgga gccaagaaaa gacccgcccc cagtgacgca 1500
gatataagtg agcccaaacg ggtgcgcgag tcagttgcgc agccatcgac gtcagacgcg 1560
gaagcttcga tcaactacgc agacaggtac caaaacaaat gttctcgtca cgtgggcatg 1620
aatctgatgc tgtttccctg cagacaatgc gagagaatga atcagaattc aaatatctgc 1680
ttcactcacg gacagaaaga ctgtttagag tgctttcccg tgtcagaatc tcaacccgtt 1740
tctgtcgtca aaaaggcgta tcagaaactg tgctacattc atcatatcat gggaaaggtg 1800
ccagacgctt gcactgcctg cgatctggtc aatgtggatt tggatgactg catctttgaa 1860
caataaatga tttaaatcag gtatggctgc cgatggttat cttccagatt ggctcgagga 1920
caaccttagt gaaggaattc gcgagtggtg ggctttgaaa cctggagccc ctcaacccaa 1980
ggcaaatcaa caacatcaag acaacgctcg aggtcttgtg cttccgggtt acaaatacct 2040
tggacccggc aacggactcg acaaggggga gccggtcaac gcagcagacg cggcggccct 2100
cgagcacgac aaggcctacg accagcagct caaggccgga gacaacccgt acctcaagta 2160
caaccacgcc gacgccgagt tccaggagcg gctcaaagaa gatacgtctt ttgggggcaa 2220
cctcgggcga gcagtcttcc aggccaaaaa gaggcttctt gaacctcttg gtctggttga 2280
ggaagcggct aagacggctc ctggaaagaa gaggcctgta gagcagtctc ctcaggaacc 2340
ggactcctcc gcgggtattg gcaaatcggg tgcacagccc gctaaaaaga gactcaattt 2400
cggtcagact ggcgacacag agtcagtccc agaccctcaa ccaatcggag aacctcccgc 2460
agccccctca ggtgtgggat ctcttacaat ggcttcaggt ggtggcgcac cagtggcaga 2520
caataacgaa ggtgccgatg gagtgggtag ttcctcggga aattggcatt gcgattccca 2580
atggctgggg gacagagtca tcaccaccag cacccgaacc tgggccctgc ccacctacaa 2640
caatcacctc tacaagcaaa tctccaacag cacatctgga ggatcttcaa atgacaacgc 2700
ctacttcggc tacagcaccc cctgggggta ttttgacttc aacagattcc actgccactt 2760
ctcaccacgt gactggcagc gactcatcaa caacaactgg ggattccggc ctaagcgact 2820

caacttcaag ctcttcaaca ttcaggtcaa agaggttacg gacaacaatg gagtcaagac 2880

catcgccaat aaccttacca gcacggtcca ggtcttcacg gactcagact atcagctccc 2940

gtacgtgctc gggtcggctc acgagggctg cctcccgccg ttcccagcgg acgttttcat 3000

gattcctcag tacgggtatc tgacgcttaa tgatggaagc caggccgtgg gtcgttcgtc 3060

cttttactgc ctggaatatt tcccgtcgca aatgctaaga acgggtaaca acttccagtt 3120

cagctacgag tttgagaacg tacctttcca tagcagctac gctcacagcc aaagcctgga 3180

ccgactaatg aatccactca tcgaccaata cttgtactat ctctcaaaga ctattaacgg 3240

ttctggacag aatcaacaaa cgctaaaatt cagtgtggcc ggacccagca acatggctgt 3300

ccagggaaga aactacatac ctggacccag ctaccgacaa caacgtgtct caaccactgt 3360

gactcaaaac aacaacagcg aatttgcttg gcctggagct tcttcttggg ctctcaatgg 3420

acgtaatagc ttgatgaatc ctggacctgc tatggccagc cacaaagaag gagaggaccg 3480

tttctttcct ttgtctggat ctttaatttt tggcaaacaa ggaactggaa gagacaacgt 3540

ggatgcggac aaagtcatga taaccaacga agaagaaatt aaaactacta acccggtagc 3600

aacggagtcc tatggacaag tggccacaaa ccaccagagt gcccaagcac aggcgcagac 3660

cggctgggtt caaaaccaag gaatacttcc gggtatggtt tggcaggaca gagatgtgta 3720

cctgcaagga cccatttggg ccaaaattcc tcacacggac ggcaactttc acccttctcc 3780

gctgatggga gggtttggaa tgaagcaccc gcctcctcag atcctcatca aaaacacacc 3840

tgtacctgcg gatcctccaa cggccttcaa caaggacaag ctgaactctt tcatcaccca 3900

gtattctact ggccaagtca gcgtggagat cgagtgggag ctgcagaagg aaaacagcaa 3960

gcgctggaac ccggagatcc agtacacttc caactattac aagtctaata atgttgaatt 4020

tgctgttaat actgaaggtg tatatagtga accccgcccc attggcacca gatacctgac 4080

tcgtaatctg taattgcttg tttcggatct caacggcctt cacaggtaca agtgaacgcc 4140

tttatcaccc agtattctac tggaaggttt ttcctcgagt gggagctgca gaaggaaaaa 4200

agggcgctcc gaacccggag atccagtaca cttccaacta ttacaagtct aataatgttg 4260

aatttgttgt taatactgaa ggtgtatata gtgaaccccg ccccattggc accagatacc 4320

tgactcgtaa tctgtaattg cttgttaatc aataaaccgt ttaattcgtt tcagttgaac 4380

tttggtctct gcgaagggcg aattcgttta aacctgcagg actagaggtc ctgtattaga 4440

ggtcacgtga gtgttttgcg acattttgcg acaccaagag gtcacgctgg gtatttaagc 4500

ccgagtgagc acgcagggtc tccattttga agcgggaggt ttgaacgcgc agccgccaag 4560

ccgaattctg cagatatcca tcacactggc ggaaatgggg aagtaaagag ctgagatcag 4620

ttgggtgcac gagtgggtta catcgaactg gatctcaaca gcggtaagat ccttgagagt 4680

tttcgccccg aagaacgttt tccaatgatg agcactttta aagttctgct atgtggcgcg 4740

gtattatccc gtattgacgc cgggcaagag caactcggtc gccgcataca ctattctcag 4800

aatgacttgg ttgagtactc accagtcaca gaaaagcatc ttacggatgg catgacagta 4860

agagaattat gcagtgctgc cataaccatg agtgataaca ctgcggccaa cttacttctg 4920

acaacgatcg gaggaccgaa ggagctaacc gcttttttgc acaacatggg ggatcatgta 4980

actcgccttg atcgttggga accggagctg aatgaagcca taccaaacga cgagcgtgac 5040

accacgatgc ctgtagcaat ggcaacaacg ttgcgcaaac tattaaaagt ccggcaacta 5100

tggatgacga aatagaacga tcgctgaaat agtgcctcac tgatagcatg gtaactgtcg 5160

accagtttac ctcatatata cttagatgat taactcattt atcaagattc tgtaagtgaa 5220

gaatccgctt ttgatatctc atgacaattc cttaacgtga tttcgtccaa ctgaacgctc 5280

gcatctcgta agatcaggac tctctgaatc tttcagcgat tcgcgttgca ccgtacagac 5340

gggtagtcag tgcatggcac acgttgcgca actattactg gcgaactact tactctagct 5400

tcccggcaac aattaataga ctggatggag gcggataaag ttgcaggacc acttctgcgc 5460

tcggcccttc cggctggctg gtttattgct gataaatctg gagccggtga gcgtgggtct 5520

cgcggtatca ttgcagcact ggggccagat ggtaagcccc tcccgtatcg tagttatcta 5580

cacgacgggg gagtcagggc actatggatg gaccaaatag acagatcccc gaataggtgc 5640

ctcctgatta gcttggtaat ggccgaccag ttactctata tacttttatt gggcgtctca 5700

cgtcagaccc gtagaagatc aaggatcttc ttgagatcct ttttttctgc gcgtaatctg 5760

ctgcttgcaa acaaaaaaac caccgctacc agcggtggtt tgtttgccgg atcaagagct 5820

accaactctt tttccgaagg taactggctt cagcagagcg cagataccaa atactgttct 5880

tctagtgtag ccgtagttag gccaccactt caagaactct gtagcaccgc ctacatacct 5940

cgctctgcta atcctgttac cagtggctgc tgccagtggc gataagtcgt gtcttaccgg 6000

gttggactca agacgatagt taccggataa ggcgcagcgg tcgggctgaa cggggggttc 6060

gtgcacacag cccagcttgg agcgaacgt cctacaccg aatctgagat acctacagc 6120

gtgagttaca cgtgacttga tagcgccacg cttccgaggg agaaaggcgg acaggtatcc 6180

ggtaagcggc agggtcggaa caggagagcg cacgagggag cttccagggg gaaacgcctg 6240

gtatctttat agtcctgtcg ggtttcgcca cctctgactt gagcgtcgat ttttgtgatg 6300

ctcgtcaggg gggcggagcc tatggaaaaa cgccagcaac gcggcctttt tacggttcct 6360

ggcctttttg atggccttgg gctacgattc cgactggaag cgggcagtga gcgcaacgca 6420

attaatgtga gttagctcac tcattaggca ccccaggctt tacactttat gcttccggct 6480

cgtatgttgt gtggaattgt gagcggataa caatttcaca caggaaacag ctatgaccat 6540

gattacgcca agcgcgcaat taaccctcac taaagggaac aaaagctgga gctccaccgc 6600

ggtggaaacc ttataacaaa aagaagggcg ggggtggcct cactgattaa aataacactt 6660

tctcagacat tcaggcgtac cgttcctgtc taaaatctct ttaatcggcc tcctgtttaa 6720

ctcccgctct gattctaacg aggaaaccct tccgatttac gactggtagc gggcagtgag 6780

cgcaacgcaa ttaatgtgag ttagctcact cattaggcac cccaggcttt acactttatg 6840

cttccggctc gtatgttgtg tggaattgtg agcggataac aatttcacac aggaaacagc 6900

tatgaccatg attacgccaa gcgcgcaatt aaccctcact aaagggaaca aaagctggag 6960

ctccaccgcg gtggcgattt tttaggatcg atttagtgct ttacggcacc tcgaccccaa 7020

aaacttgatt agggtgatgg ttcacgtatt gggccatcgc cctgatagac ggtttttcgc 7080

cctttgacgt tggagtccac gttctttaat agtggactct tgttccaaac tggaacaaca 7140

ctcaacccta tctcggtcta ttcttttgat ttataaggga ttttgccgat ttcggcctat 7200

tggttaaaaa atgagctgat ttaacaaaaa tttaacgcga attttaacaa aatattaacg 7260

cttacaattt aggtggcact tttcggggaa atgtgcgcgg aacccctatt tgtttatttt 7320

tctaaataca ttcaaatatg tatccgctca tgagacaata accctgataa atgcttcaat 7380

aatattgaaa aaggaagagt gagtatgagt attcaacatt tccgtgtcgc ccttattccc 7440

ttttttgcgg cattttgcct tcctgttttt gctcacccag aaacgctggt gaaagtaaaa 7500

gatgctgaag atcagttggg tgcacgagtg ggttacatcg aactggatct caacagcggt 7560

aagatccttg agagttttcg ccccgaagaa cgttttccaa tgatgagcac ttttaaagtt 7620

ctgctatgtg gcgcggtatt atcccgtatt gacgccgggc aagagcaact cggtcgccgc 7680

atacactatt ctcagaatga cttggttgag tactcaccag tcacagaaaa gcatcttacg 7740

gatggcatga cagtaagaga attatgcagt gctgccataa ccatgagtga taacactgcg 7800

gccaacttac ttctgacaac gatcggagga ccgaaggagc taaccgcttt tttgcacaac 7860

atgggggatc atgtaactcg ccttgatcgt tgggaaccgg agctgaatga agccatacca 7920

aacgacgagc gtgacaccac gatgcctgta gcaatggcaa caacgttgcg caaactatta 7980

actggcgaac tacttactct agcttcccgg caacaattaa tagactggat ggaggcggat 8040

aaagttgcag gaccacttct gcgctcggcc cttccggctg gctggtttat tgctgataaa 8100

tctggagccg gtgagcgtgg gtctcgcggt atcattgcag cactggggcc agatggtaag 8160

ccctcccgta tcgtagttac tacacgacgg ggagtcaggc aactatggat gaacgaaata 8220

gacagatcgc tgagataggt gcctcactga ttaagcattg g 8261

<210> 148
<211> 7775
<212> DNA
<213> Adeno-Associated Virus

<220>
<221>  gene
<222>  (1)..(7775)

<400>  148
ggcatttctg acagctttgt gaactgggtg gccgagaagg aatgggagtt gccgccagat      60

tctgacatgg atctgaatct gattgagcag gcacccctga ccgtggccga gaagctgcag     120

cgcgactttc tgacggaatg gcgccgtgtg agtaaggccc cggaggctct tttctttgtg     180

caatttgaga agggagagag ctacttccac atgcacgtgc tcgtggaaac caccggggtg     240

aaatccatgg ttttgggacg tttcctgagt cagattcgcg aaaaactgat tcagagaatt     300

taccgcggga tcgagccgac tttgccaaac tggttcgcgg tcacaaagac cagaaatggc     360

gccggaggcg ggaacaaggt ggtggatgag tgctacatcc ccaattactt gctccccaaa     420

acccagcctg agctccagtg ggcgtggact aatatggaac agtatttaag cgcctgtttg     480

aatctcacgg agcgtaacgg gtggtggcgc agcatctgac gcacgtgtcg cagacgcaaa     540

accgcccccg actacctggt gggccagcag cccgtggagg acatttccag caatcggatt     600

tataaaattt tggaactaaa cgggtacgat ccccaatatg cggcttccgt ctttctggga     660

tgggccacga aaaagttcgg caagaggaac accatctggc tgtttgggcc tgcaactacc     720

gggaagacca acatcgcgga ggccatagcc cacactgtgc ccttctacgg gtgcgtaaac     780

tggaccaatg agaactttcc cttcaacgac tgtgtcgaca agatggtgat ctggtgggag     840

gaggggaaga tgaccgccaa ggtcgtggag tcggccaaag ccatttctcg gaggaaagca     900

aaggtgcgcg tggaccagaa atgcaagtcc tctcactcac ggacagaaag actgtttaga     960

gtgctttccc gtgtcagaat ctcaacccgt ttctgtcgtc aaaaaggcgt atcaaaaaac    1020

tgtgctacat tcatcatatc atgggaaagg tgccagacgc ttgcactgcc tgcgatctgg    1080

tcaatgtgga tttggatgac tgcatctttg aacaataaat gatttaaatc aggtatggct    1140

gccgatggtt atcgcactgc ctgcgatctg gtcaatgtgg atttggatga ctgcatcttt    1200

gaacaataaa tgatttaaat caggtatggc tgccgatggt tatcttccag attggctcga    1260

ggacaacctc tctgagggca ttcgcgagtg gtgggacttg aaacctggag ccccgaaacc    1320

caaagccaac cagcaaaagc aggacgacgg ccggggtctg gtgcttcctg gctacaagta    1380

cctcggaccc ttcaacggac tcgacaaggg ggagcccgtc aacgcggcgg acgcagcggc    1440

cctcgagcac gacaaggcct acgaccagca gctcaaagcg ggtgacaatc cgtacctgcg    1500

gtataaccac gccgacgccg agtttcagga gcgtctgcaa gaagatacgt cttttggggg    1560

caacctcggg cgagcagtct tccaggccaa gaagcgggtt ctcgaacctc tcggtctggt    1620

tgaggaaggc gctaagacgg ctcctggaaa gaagagaccg gtagagccat caccccagcg    1680

ttctccagac tcctcaatgt ggatttggat gactgcatct ttgaacaata tatgatttaa    1740

atcaggtatg gctgccgatg gttatcttcc agattggctc gaggacaacc tctctgaggg 1800

cattcgcgag tggtgggact tgaaacctgg agccccgaaa cccaaagcca accagcaaaa 1860

gcaggacgac ggccggggtc tggtgcttcc tggctacaag tacctcggac ccttcaacgg 1920

actcgacaag ggggagcccg tcaacgcggc ggacgcagcg gccctcgagc acgacaaggc 1980

ctacgaccag cagctcaaag cgggtgacaa tccgtacctg cggtataacc acgccgacgc 2040

cgagtttcag gagcgtctgc aagaagatac gtcttttggg ggcaacctcg ggcgagcagt 2100

cttccaggcc aagaagcggg ttctcgaacc tctcggtctg gttgaggaag gcgctaagac 2160

ggctcctgga aagaagagac cggtagagcc atcaccccag cgttctccag actcctctac 2220

gggcatcggc aagaaaggcc agcagcccgc gaaaaagaga ctcaactttg ggcagactgg 2280

cgactcagag tcagtgcccg accctcaacc aatcggagaa ccccccgcag cccctctggt 2340

ctgggatctg gtacaatggc tgcaggcggt ggcgctccaa tggcagacaa tccccaaccc 2400

caattttcag aacctccgga aaaaaaatcc tcttttgggg gccacccccg gccaaccatc 2460

cttccagccc aaaaaccggt tccccaaccc tttcgtcttg gttgagaaag gccctaaaac 2520

gctcccggaa aaaaaaaaac cggaaaaccc tcccccccac cttcccccaa atcctccaac 2580

ggcctccgca aaaaaaggcc accacccccc aaaaaaaaaa tccactttgg gcaaaacggc 2640

caatccaaat ccgtggccca acctccaccc atccgaaaac ccccccccag cccctctggg 2700

ccgggatttg gtaacatggc tggcgggggg ggccccccca tgggaaccct ccccacctac 2760

aacaaccacc aaaataaaca aagcccccaa cggaggggtt attcccccag aaattgggga 2820

agcaccaacg acaacaccta cttcggctac agcaccccct gggggtattt tgactttaac 2880

agattccact gccacttctc accacgtgac tggcagcgac tcatcaacaa caactgggga 2940

ttccggccca aaaaactcaa cttcaagctc ttcaacatcc acgtcaagga ggtcacgcaa 3000

aatgaaggca ccaagaccat cgccaataac cttaccagca cgaatcaggt ctttaccgac 3060

tcggaatacc agctcccgta cctcctcggc tctgcgcacc agggctgcct gcctgatgaa 3120

ccccctcatc gaccagtacc tgtactacct gtctcggact cagtccacgg gaggtaccgc 3180

aggaactcag cagttgctat tttctcaggc cgggcctaat aacatgtcgg ctcaggccaa 3240

aaactggcta cccgggccct gctaccggca gcaacgcgtc tccacgacac tgtcgcaaaa 3300

taacaacagc aactttgcct ggaccggtgc caccaagtat catctgaatg gcagaaactc 3360

tctggtaaat cccggtgtcg ctatggcaac ccacaaggac gacgaagagc gattttttcc 3420

gtccagcgga gtcttaatgt ttgggaaaca gggagctgga aaagacaacg tggactatag 3480

catcgttatg ctaaccagtg aggaagaaat taaaaccacc aacccagtgg ccacagaaca 3540

gtacggcgtg gtggccgata acctgcaaca gcaaaacgcc gctcctattg taggggccgt 3600

caacagtcaa ggagccttac ctgtcatggt ctggcagaac cgggacgtgt acctgcaggg 3660

tcctatctgg gccaagattc ctcacacgga cggaaacttt catccctcgc cgctgatggg 3720

aggctttgga ctgaaacacc cgcctcctcc atcctgatta aaaatacacc tgttcccgtg 3780

gatcctccaa ctaccttcag tccagctaag ctggcgtcgt tcatcacgca gtacagcacc 3840

ggacaggtca gcgtggaaat tgaatgggag ctgcaaaaaa aaaacagcaa acgctggaac 3900

ccaaaaattc aatactcttc caactactac aaatctacaa atgtggactt tgctgttaac 3960

acagatggca cttattggta cgatcagact ggagcgggca gtgagcgcac gcaattaatg 4020

tgagttagct cactcattag gcaccccagg ctttacactt tatgcttccg gctcgtatgt 4080

tgtgtggaat tgtgagcgga taacaatttc acacaggaaa cagctatgac catgattacg 4140

ccaagcgcgc aattaaccct cactaaaggg aacaaaagct ggagctccac cgcggtggtg 4200

ctttacggca cctcgacccc aaaaaacttg attagggtga tggttcacga aaagggccat 4260

cgccctgata gacggttttt cgccctttga cgttggagtc cacgttcttt aatagtggac 4320

tcttgttcca aactggaaca acactcaacc ctatctcggt ctattctttt gatttataag 4380

ggattttgcc gatttcggcc tattggttaa aaaatgagct gatttaacaa aaatttaacg 4440

cgaattttaa caaaatatta acgcttacaa tttaggtggc acttttcggg gaaatgtgcg 4500

cggaacccct atttgtttat ttttctaaat acattcaaat atgtatccgc tcatgagaca 4560

ataaccctga taaatgcttc aataatattg aaaaaggaag agtatgagta ttcaacattt 4620

ccgtgtcgcc cttattccct tttttgcggc attttgcctt cctgtttttg ctcacccaga 4680

aacgctggtg aaagtaaaag atgctgaaga tcagttgggt gcacgagtgg gttacatcga 4740

actggatctc aacagcggta agatccttga gagttttcgc cccgaagaac gttttccaat 4800

gatgagcact tttaaagttc tgctatgtgg cgcggtatta tcccgtattg acgcccgggc 4860

aagagcaact cggtcgccgc atacactatt ctcagatgac ttggttgagt actccccagt 4920

cacagaaaag catcttacga tggcatgaca gtaaggagat tatgcatgct gccataccat 4980

gatgaataac actgcgcact tacttctgac acgatcggag gaccgaagga gctcggcgct 5040

tctggggctt tttttgaagg ctaaaaaagg gcccaagggg gggggggggg ggcaagcaca 5100

accccccgta cgtcgggggg gggagaagga gattttcctt tagcaaagag aacggtgccc 5160

cccccggcga gggaacgggg cccctggttt cccccgccca acggagaaac catacaccca 5220

ctactatgca caacatctat atgtaatcat aaaaccatag atcatcgcta tagcgcgacg 5280

cgcccctctc tgcatactgt gaggtaagac gatctgtggc tccgattctc gacatattga 5340

cgctagactg cgttgctcag tcttgcacga gtgggttaca tcgaactgga tctcaacagc 5400

ggtaagatcc ttgagagttt tcgccccgaa gaacgttttc caatgatgag cacttttaaa 5460

gttctgctat gtggcgcggt attatcccgt attgacgccg ggcaagagca actcggtcgc 5520

cgcatacact attctcagaa tgacttggtt gagtactcac cagtcacaga aaagcatctt 5580

acggatggca tgacagtaag agaattatgc agtgctgcca taaccatgag tgataacact 5640

gcggccaact tacttctgac aacgatcgga ggaccgaagg agctaaccgc ttttttgcac 5700

aacatggggg atcatgtaac tcgccttgat cgttgggaac cggagctgaa tgaagccata 5760

ccaaacgacg agcgtgacac cacgatgcct gtagcaatgg caacaacgtt gcgcaaacta 5820

ttaactggcg aactacttac tctagcttcc cggcaacaat taatagactg gatggaggcg 5880

gataaagttg caggaccact tctgcgctcg gcccttccgg ctggctggtt tattgctgat 5940

aaatctggag ccggtgagcg tgggtctcgc ggtatcattg cagcactggg gccagatggt 6000

aagccctccc gtatcgtagt tatctacacg acggggagtc aggcaactat ggatcaacaa 6060

ttaatagact ggatggaggc ggataaagtt gcaggaccac ttctgcgctc ggcccttccg 6120

gctggctggt ttattgctga taaatctgga gccggtgagc gtgggtctcg cggtatcatt 6180

gcagcactgg ggccagatgg taagccctcc cgtatcgtag ttatctacac gacggggagt 6240

caggcaacta tggatgaacg aaatagacag atcgctgaga taggtgcctc actgattaag 6300

cattggtaac tgtcagacca agtttactca tatatacttt agattgattt aaaacttcat 6360

ttttaattta aaaggatcta ggtgaagatc ctttttgata atctcatgac caaaatccct 6420

taacgtgagt tttcgttcca ctgagcgtca gaccccgtag aaaagatcaa aggatcttct 6480

tgagatcctt tttttctgcg cgtaatctgc tgcttgcaaa caaaaaaacc accgctacca 6540

gcggtggttt gtttgccgga tcaagagcta ccaactcttt ttccgaaggt aactggcttc 6600

agcagagcgc agataccaaa tactgttctt ctagtgtagc cgtagttagg ccaccacttc 6660

aagaactctg tagcaccgcc tacatccgaa ggtaactggc ttcagcagag cgcagatacc 6720

aaatactgtt cttctagtgt agccgtagtt aggccaccac ttcaagaact ctgtagcacc 6780

gcctacatac ctcgctctgc taatcctgtt accagtggct gctgccagtg gcgataagtc 6840

gtgtcttacc gggttggact caagacgata gttaccggat aaggcgcagc ggtcgggctg 6900

aacggggggt tcgtgcacac agcccagctt ggagcgaacg acctacaccg aactgagata 6960

cctacagcgt gagctatgag aaagcgccac gcttcccgaa gggagaaagg cggacaggta 7020

tccggtaagc ggcagggtcg gaacaggaga gcgcacgagg gagcttccag ggggaaacgc 7080

ctggtatctt tatagtcctg tcgggtttcg ccacctctga cttgagcgtc gatttttgtg 7140

atgctcgtca gggggggcgg agcctatgga aaaacgccag caacgcggcc tttttacggt 7200

tgaacaggag agcgcacgag ggagcttcca gggggaaacg cctggtatct ttatagtcct 7260

gtcgggtttc gccacctctg acttgagcgt cgatttttgt gatgctcgtc aggggggcgg 7320

agcctatgga aaaacgccag caacgcggcc tttttacggt tcctggcctt ttgctggcct 7380

tttgctggcc ttttgctcac atgttctttc ctgcgttatc ccctgattct gtggataacc 7440

gtattaccgc ctttgagtga gctgataccg ctcgccgcag ccgaacgacc gagcgcagcg 7500

agtcagtgag cgaggaagcg gaagagcgcc caatacgcaa accgcctctc cccgcgcgtt 7560

acagactgga gcgggcagtg agcgcaacgc aattaatgtg agttagctca ctcattaggc 7620

accccaggct ttacacttta tgcttccggc tcgtatgttg tgtggaattg tgagcggata 7680

acaatttcac acaggaaaca gctatgacca tgattacgcc aagcgcgcaa ttaaccctca 7740

ctaaagggaa caaaagctgg agctccaccg cggtg 7775

<210> 149
<211> 8344
<212> DNA
<213> Adeno-Associated Virus

<220>
<221> gene
<222> (1)..(8344)

<400> 149
cacctaaatt gtaagcgtta atattttgtt aaaattcgcg ttaaattttt gttaaatcag 60

ctcatttttt aaccaatagg ccgaaatcgg caaaatccct tataaatcaa aagaatagac 120

cgagataggg ttgagtgttg ttccagtttg gaacaagagt ccactattaa agaacgtgga 180

ctccaacgtc aaagggcgaa aaaccgtcta tcagggcgat ggcccactac gtgaaccatc 240

accctaatca agttttttgg ggtcgaggtg ccgtaaagca ctaaatcgga accctaaagg 300

gagcccccga tttagagctt gacggggaaa gccggcgaac gtggcgagaa aggaagggaa 360

gaaagcgaaa ggagcgggcg ctagggcgct ggcaagtgta gcggtcacgc tgcgcgtaac 420

caccacaccc gccgcgctta atgcgccgct acagggcgcg tcccattcgc cattcaggct 480

gcgcaactgt tgggaagggc gatcggtgcg ggcctcttcg ctattacgcc agctggcgaa 540

agggggatgt gctgcaaggc gattaagttg ggtaacgcca gggttttccc agtcacgacg 600

ttgtaaaacg acggccagtg aattgtaata cgactcacta tagggcgaat tggagctcca 660

ccgcggtggc ggccgctcta gagtcctgta ttagaggtca cgtgagtgtt ttgcgacatt 720

ttgcgacacc atgtggtcac gctgggtatt taagcccgag tgagcacgca gggtctccat 780

tttgaagcgg gaggtttgaa cgcgcagcac ggcggggttt tacgagattg tgattaaggt 840

ccccagcgac cttgacgggc atctgcccgg catttctgac agctttgtga actgggtggc 900

cgagaaggaa tgggagttgc cgccagattc tgacatggat ctgaatctga ttgagcaggc 960

acccctgacc gtggccgaga agctgcagcg cgactttctg acggaatggc gccgtgtgag 1020

taaggccccg gaggcccttt tctttgtgca atttgagaag ggagagagct acttccacat 1080

gcacgtgctc gtggaaacca ccggggtgaa atccatggtt ttgggacgtt tcctgagtca 1140

gattcgcgaa aaactgattc agagaattta ccgcgggatc gagccgactt tgccaaactg 1200

gttcgcggtc acaaagacca gaaatggcgc cggaggcggg aacaaggtgg tggatgagtg 1260

ctacatcccc aattacttgc tccccaaaac ccagcctgag ctccagtggg cgtggactaa 1320

tatggaacag tatttaagcg cctgtttgaa tctcacggag cgtaaacggt tggtggcgca 1380

gcatctgacg cacgtgtcgc agacgcagga gcagaacaaa gagaatcaga atcccaattc 1440

tgatgcgccg gtgatcagat caaaaacttc agccaggtac atggagctgg tcgggtggct 1500

cgtggacaag gggattacct cggagaagca gtggatccag gaggaccagg cctcatacat 1560

ctccttcaat gcggcctcca actcgcggtc ccaaatcaag gctgccttgg acaatgcggg 1620

aaagattatg agcctgacta aaaccgcccc cgactacctg gtgggccagc agcccgtgga 1680

ggacatttcc agcaatcgga tttataaaat tttggaacta aacgggtacg atccccaata 1740

tgcggcttcc gtctttctgg gatgggccac gaaaaagttc ggcaagagga acaccatctg 1800

gctgtttggg cctgcaacta ccgggaagac caacatcgcg gaggccatag cccacactgt 1860

gcccttctac gggtgcgtaa actggaccaa tgagaacttt cccttcaacg actgtgtcga 1920

caagatggtg atctggtggg aggaggggaa gatgaccgcc aaggtcgtgg agtcggccaa 1980

agccattctc ggaggaagca aggtgcgcgt ggaccagaaa tgcaagtcct cggcccagat 2040

agacccgact cccgtgatcg tcacctccaa caccaacatg tgcgccgtga ttgacgggaa 2100

ctcaacgacc ttcgaacacc agcagccgtt gcaagaccgg atgttcaaat ttgaactcac 2160

ccgccgtctg gatcatgact ttgggaaggt caccaagcag gaagtcaaag actttttccg 2220

gtgggcaaag gatcacgtgg ttgaggtgga gcatgaattc tacgtcaaaa agggtggagc 2280

caagaaaaga cccgccccca gtgacgcaga tataagtgag cccaaacggg tgcgcgagtc 2340

agttgcgcag ccatcgacgt cagacgcgga agcttcgatc aactacgcag acaggtacca 2400

aaacaaatgt tctcgtcacg tgggcatgaa tctgatgctg tttccctgca gacaatgcga 2460

gagaatgaat cagaattcaa atatctgctt cactcacgga cagaaagact gtttagagtg 2520

ctttcccgtg tcagaatctc aacccgtttc tgtcgtcaaa aaggcgtatc agaaactgtg 2580

ctacattcat catatcatgg gaaaggtgcc agacgcttgc actgcctgcg atctggtcaa 2640

tgtggatttg gatgactgca tctttgaaca ataaatgatt taaaccaggt atggctgccg 2700

atggttatct tccagattgg ctcgaggaca acctctctga gggcattcgc gagtggtggg 2760

acttgaaacc tggagccccg aagcccaaag ccaaccagca aaagcaggac gacggccggg 2820

gtctggtgct tcctggctac aagtacctcg gacccttcaa cggactcgac aagggggagc 2880

ccgtcaacgc ggcggacgca gcggccctcg agcacgacaa ggcctacgac cagcagctca 2940

aagcgggtga caatccgtac ctgcggtata accacgccga cgccgagttt caggagcgtc 3000

tgcaagaaga tacgtctttt gggggcaacc tcgggcgagc agtcttccag gccaagaagc 3060
gggttctcga acctctcggt ctggttgagg aaggcgctag aacggctcct ggaaagaaac 3120
gtccggtaga gcagtcgcca caagagccag actcctcctc gggcatcggc aagacaggcc 3180
agcagcccgc taaaaagaga ctcaattttg gtcagactgg cgactcagag tcagtccccg 3240
atccacaacc tctcggagaa cctccagcaa cccccgctgc tgtgggacct actacaatgg 3300
cttcaggcgg tggcgcacca atggcagaca ataacgaagg cgccgacgga gtgggtaatg 3360
cctcaggaaa ttggcattgc gattccacat ggctgggcga cagagtcatc accaccagca 3420
cccgcacctg ggccttgccc gcctacaata accacctcta caagcaaatc tccagtgctt 3480
caacgggggc cagcaacgac aaccactact tcggctacgc ccccctgggg gtattttgat 3540
ttcaacagat tccactgcca cttttcacca cgtgactggc agcgactcat caacaacaat 3600
tggggattcc ggcccaagag actcaacttc aaactcttca acatccaagt caaggaggtc 3660
acgacgaatg atggcgtcac aaccatcgct aataacctta ccagcacggt tcaagtcttc 3720
tcggactcgg agtaccagct tccgtacgtc ctcggctctg cgcaccaggg ctgcctccct 3780
ccgttcccgg cggacgtgtt catgattccg caatacggct acctgacgct caacaatggc 3840
agccaagccg tgggacgttc atccttttac tgcctggaat atttcccttc tcagatgctg 3900
agaacgggca acaactttac cttcagctac acctttgagg aagtgccttt ccacagcagc 3960
tacgcgcaca gccagagcct ggaccggctg atgaatcctc tcatcgacca atacctgtat 4020
tacctgaaca gaactcaaaa tcagtccgga agtgcccaaa acaaggactt gctgtttagc 4080
cgtgggtctc cagctggcat gtctgttcag cccaaaaact ggctacctgg accctgttat 4140
cggcagcagc gcgtttctaa aacaaaaaca gacaacaaca acgccaattt tacctggact 4200
ggtgcttcaa aatataacct caatgggcgt gaatccatca tcaaccctgg cactgctatg 4260
gccgcacaca aagacgacga agacaagttc tttcccatga gcggtgtcat gatttttgga 4320
aaagagagcg ccggagcttc aaacactgca ttggacaatg tcatgattac agacgaagag 4380
gaaattaaag ccactaaccc tgtggccacc gaaagatttg ggaccgtggc agtcaatttc 4440
cagagcagca gcacagaccc tgcgaccgga gatgtgcatg ctatgggagc attacctggc 4500
atggtgtggc aagatagaga cgtgtacctg cagggtccca tttgggccaa aattcctcac 4560
acagatggac actttcaccc gtctcctctt atgggcggct ttggactcaa gaacccgcct 4620
cctcagatcc tcatcaaaaa cacgcctgtt cctgcgaatc ctccggcgga gtttgcagct 4680
acaaagtttg ctgcattcat cacccaatac tccacaggac aagtgagtgt ggaaattgaa 4740
tgggagctgc agaaagaaaa cagcaagcgc tggaatcccg aagtgcagta cacatccaat 4800
tatgcaaaat ctgccaacgt tgattttact gtggacaaca atggacttta tactgagcct 4860
cgccccattg gcacccgtta ccttacccgt cccctgtaat ttacgtgtta atcaataaac 4920

cggttgattc gtttcagttg aactttggtc tcgcggccgc tcgataagct tttgttccct 4980

ttagtgaggg ttaatttcga gcttggcgta atcatggtca tagctgtttc ctgtgtgaaa 5040

ttgttatccg ctcacaattc cacacaacat acgagccgga agcataaagt gtaaagcctg 5100

gggtgcctaa tgagtgagct aactcacatt aattgcgttg cgctcactgc ccgctttcca 5160

gtcgggaaac ctgtcgtgcc agctgcatta atgaatcggc caacgcgcgg ggagaggcgg 5220

tttgcgtatt gggcgctctt ccgcttcctc gctcactgac tcgctgcgct cggtcgttcg 5280

gctgcgcgag cggtatcagc tccctcaaag cgtaataccg gttatcccac agatcagggg 5340

atacgcagaa agacatgtga gtaaggcagc caaagtccag gaaccggtaa aaggccccgc 5400

gcgcttagtg ctttacggca cctcgacccc aaaaaacttg attagggtga tggttcacgt 5460

agtgggccat cgccctgata gacggttttt cgccctttga cgttggagtc cacgttcttt 5520

aatagtggac tcttgttcca aactggaaca acactcaacc ctatctcggt ctattctttt 5580

gatttataag ggattttgcc gatttcggcc tattggttaa aaaatgagct gatttaacaa 5640

aaatttaacg cgaattttaa caaaatatta acgtttacaa tttaaatatt tgcttataca 5700

atcttcctgt ttttggggct tttctgatta tcaaccgggg tacatatgat tgacatgcta 5760

gttttacgat taccgttcat cgattctctt gtttgctcca gactctcagg caatgacctg 5820

atagcctttg tagagacctc tcaaaaatag ctaccctctc cggcatgaat ttatcagcta 5880

gaacggttga atatcatatt gatggtgatt tgactgtctc cggcctttct cacccgtttg 5940

aatctttacc tacacattac tcaggcattg catttaaaat atatgagggt tctaaaaatt 6000

tttatccttg cgttgaaata aaggcttctc ccgcaaaagt attacagggt cataatgttt 6060

ttggtacaac cgatttagct ttatgctctg aggctttatt gcttaatttt gctaattctt 6120

tgccttgcct gtatgattta ttggatgttg gaattcctga tgcggtattt tctccttacg 6180

catctgtgcg gtatttcaca ccgcatatgg tgcactctca gtacaatctg ctctgatgcc 6240

gcatagttaa gccagccccg acacccgcca acacccgctg acgcgccctg acgggcttgt 6300

ctgctcccgg catccgctta cagacaagct gtgaccgtct ccgggagctg catgtgtcag 6360

aggttttcac cgtcatcacc gaaacgcgcg agacgaaagg gcctcgtgat acgcctattt 6420

ttataggtta atgtcatgat aataatggtt tcttagacgt caggtggcac ttttcgggga 6480

aatgtgcgcg gaacccctat ttgtttattt ttctaaatac attcaaatat gtatccgctc 6540

atgagacaat aaccctgata aatgcttcaa taatattgaa aaaggaagag tatgagtatt 6600

caacatttcc gtgtcgccct tattcccttt tttgcggcat tttgccttcc tgtttttgct 6660

cacccagaaa cgctggtgaa agtaaaagat gctgaagatc agttgggtgc acgagtgggt 6720

tacatcgaac tggatctcaa cagcggtaag atccttgaga gttttcgccc cgaagaacgt 6780

tttccaatga tgagcacttt taaagttctg ctatgtggcg cggtattatc ccgtattgac 6840

gccgggcaag agcaactcgg tcgccgcata cactattctc agaatgactt ggttgagtac 6900

tcaccagtca cagaaaagca tcttacggat ggcatgacag taagagaatt atgcagtgct 6960

gccataacca tgagtgataa cactgcggcc aacttacttc tgacaacgat cggaggaccg 7020

aaggagctaa ccgctttttt gcacaacatg ggggatcatg taactcgcct tgatcgttgg 7080

gaaccggagc tgaatgaagc cataccaaac gacgagcgtg acaccacgat gcctgtagca 7140

atggcaacaa cgttgcgcaa actattaact ggcgaactac ttactctagc ttcccggcaa 7200

caattaatag actggatgga ggcggataaa gttgcaggac cacttctgcg ctcggccctt 7260

ccggctggct ggtttattgc tgataaatct ggagccggtg agcgtgggtc tcgcggtatc 7320

attgcagcac tggggccaga tggtaagccc tcccgtatcg tagttatcta cacgacgggg 7380

agtcaggcaa ctatggatga acgaaataga cagatcgctg agataggtgc ctcactgatt 7440

aagcattggt aactgtcaga ccaagtttac tcatatatac tttagattga tttaaaactt 7500

catttttaat ttaaaaggat ctaggtgaag atcctcacct aaattgtaag cgttaatatt 7560

ttgttaaaat tcgcgttaaa tttttgttaa atcagctcat tttttaacca ataggccgaa 7620

atcggcaaaa tcccttataa atcaaaagaa tagaccgaga tagggttgag tgttgttcca 7680

gtttggaaca agagtccact attaaagaac gtggactcca acgtcaaagg gcgaaaaacc 7740

gtctatcagg gcgatggccc actacgtgaa ccatcaccct aatcaagttt tttggggtcg 7800

aggtgccgta aagcactaaa tcggaaccct aaagggagcc cccgatttag agcttgacgg 7860

ggaaagccgg cgaacgtggc gagaaaggaa gggaagaaag cgaaaggagc gggcgctagg 7920

gcgctggcaa gtgtagcggt cacgctgcgc gtaaccacca cacccgccgc gcttaatgcg 7980

ccgctacagg gcgcgtccca ttcgccattc aggctgcgca actgttggga agggcgatcg 8040

gtgcgggcct cttcgctatt acgccagctg gcgaaagggg gatgtgctgc aaggcgatta 8100

agttgggtaa cgccagggtt ttcccagtca cgacgttgta aaacgacggc cagtgaattg 8160

taatacgact cactataggg cgaattggag ctccaccgcg gtggcggccg ctctagagtc 8220

ctgtattaga ggtcacgtga gtgttttgcg acattttgcg acaccatgtg gtcacgctgg 8280

gtatttaagc ccgagtgagc acgcagggtc tccattttga agcgggaggt ttgaacgcgc 8340

agcc 8344

<210> 150
<211> 8267
<212> DNA
<213> Adeno-Associated Virus

<220>
<221> gene

<222>   (1)..(8267)

<400>   150
aattcccatc atcaataata taccttattt tggattgaag ccaatatgat aatgaggggg      60

tggagtttgt gacgtggcgc ggggcgtggg aacggggcgg gtgacgtagt agtctctaga     120

gtcctgtatt agaggtcacg tgagtgtttt gcgacatttt gcgacaccat gtggtcacgc     180

tgggtattta agcccgagtg agcacgcagg gtctccattt tgaagcggga ggtttgaacg     240

cgcagccacc aacggcgggg ttttacgaga ttgtgattaa ggtccccagc gaccttgacg     300

ggcatctgcc cggcatttct gacagctttg tgaactgggt ggccgagaag gaatgggagt     360

tgccgccaga ttctgacatg gatctgaatc tgattgagca ggcacccctg accgtggccg     420

agaagctgca gcgcgacttt ctgacggaat ggcgccgtgt gagtaaggcc ccggaggccc     480

ttttctttgt gcaatttgag aagggagaga gctacttcca catgcacgtg ctcgtggaaa     540

ccaccggggt gaaatccatg gttttgggac gtttcctgag tcagattcgc gaaaaactga     600

ttcagagaat ttaccgcggg atcgagccga ctttgccaaa ctggttcgcg gtcacaaaga     660

ccagaaatgg cgccggaggc gggaacaagg tggtggatga gtgctacatc cccaattact     720

tgctccccaa aacccagcct gagctccagt gggcgtggac taatatggaa cagtatttaa     780

gcgcctgttt gaatctcacg gagcgtaaac ggttggtggc gcagcatctg acgcacgtgt     840

cgcagacgca ggagcagaac aaagagaatc agaatcccaa ttctgatgcg ccggtgatca     900

gatcaaaaac ttcagccagg tacatggagc tggtcgggtg gctcgtggac aaggggatta     960

cctcggagaa gcagtggatc caggaggacc aggcctcata catctccttc aatgcggcct    1020

ccaactcgcg gtcccaaatc aaggctgcct tggacaatgc gggaaagatt atgagcctga    1080

ctaaaaccgc ccccgactac ctggtgggcc agcagcccgt ggaggacatt tccagcaatc    1140

ggatttataa aattttggaa ctaaacgggt acgatcccca atatgcggct tccgtctttc    1200

tgggatgggc cacgaaaaag ttcggcaaga ggaacaccat ctggctgttt gggcctgcaa    1260

ctaccgggaa gaccaacatc gcggaggcca tagcccacac tgtgcccttc tacgggtgcg    1320

taaactggac caatgagaac tttcccttca acgactgtgt cgacaagatg gtgatctggt    1380

gggaggaggg gaagatgacc gccaaggtcg tggagtcggc caaagccatt ctcggaggaa    1440

gcaaggtgcg cgtggaccag aaatgcaagt cctcggccca gatagacccg actcccgtga    1500

tcgtcacctc caacaccaac atgtgcgccg tgattgacgg gaactcaacg accttcgaac    1560

accagcagcc gttgcaagac cggatgttca aatttgaact cacccgccgt ctggatcatg    1620

actttgggaa ggtcaccaag caggaagtca aagacttttt ccggtgggca aaggatcacg    1680

tggttgaggt ggagcatgaa ttctacgtca aaaagggtgg agccaagaaa agacccgccc    1740

ccagtgacgc agatataagt gagcccaaac gggtgcgcga gtcagttgcg cagccatcga    1800

cgtcagacgc ggaagcttcg atcaactacg cagacaggta ccaaaacaaa tgttctcgtc 1860

acgtgggcat gaatctgatg ctgtttccct gcagacaatg cgagagaatg aatcagaatt 1920

caaatatctg cttcactcac ggacagaaag actgtttaga gtgctttccc gtgtcagaat 1980

ctcaacccgt ttctgtcgtc aaaaaggcgt atcagaaact gtgctacatt catcatatca 2040

tgggaaaggt gccagacgct tgcactgcct gcgatctggt caatgtggat ttggatgact 2100

gcatctttga acaataaatg atttaaatca ggtatggctg ccgatggtta tcttccagat 2160

tggctcgagg acactctctc tgaaggaata agacagtggt ggaagctcaa acctggccca 2220

ccaccaccaa agcccgcaga acggcatagg gacgacagca ggggtcttgt gcttcctggg 2280

tacaagtacc tcggaccctt caacggactc gacaagggag agccggtcaa cgaggcagac 2340

gccgcggccc tcgagcacga caaagcctac gaccggcagc tcgacagcgg agacaacccg 2400

tacctcaagt acaaccacgc cgacgcggag tttcaggagc gccttaaaga agatacgtct 2460

tttgggggca acctcggacg agcagtcttc caggcgaaaa agagggttct tgaacctctg 2520

ggcctggttg aggaacctgt taggacggcc ccgggaaaaa ggaggccggt agagcactct 2580

cctgtggagc cagactcctc ctcgggaacc ggaagggccg gccagcagcc tgcaagaaaa 2640

agattgaatt ttggtcagac tggagacgca gactcagtac ctgaccccca gcctctcgga 2700

cagccaccag cagccccctc tggtctggga actaatacga tggctacagg cagtggcgca 2760

ccaatggcag acaataacga gggcgccgac ggagtgggta attcctccgg aaattggcat 2820

tgcgattcca catggatggg cgacagagtc atcaccacca gcacccgaac ctgggccctg 2880

ccggcctaca acaaccacct ctacaaacaa atttccagcc aatcaggagc ctcgaacgac 2940

aatcactact ttggctacgc caccccttgg gggtattttg acttcaacag attccactgc 3000

cacttttcac cacgtgactg gcaaagactc atcaacaaca actggggatt ccgacccaag 3060

agactcaact tcaagctctt taacattcaa gtcaaagagg tcacgcagaa tgacggtacg 3120

acgacgattg ccaataacct taccagcacg gttcaggtgt ttactgactc ggagtaccag 3180

ctcccgtacg tcctcggctc ggcgcatcaa ggatgcctcc cgccgttccc agcagacgtc 3240

ttcatggtgc cacagtatgg atacctcacc ctgaacaacg ggagtcaggc agtaggacgc 3300

tcttcatttt actgcctgga gtactttcct tctcagatgc tgcgtaccgg aaacaacttt 3360

accttcagct acacttttga ggacgttcct ttccacagca gctacgctca cagccagagt 3420

ctggaccgtc tcatgaatcc tctcatcgac cagtacctgt attacttaag cagaacaaac 3480

actccaagtg gagccaccac gcagtcaagg cttcagtttt ctcaggccgg agcgagtgac 3540

attcgggacc agtctaggaa ctggcttcct ggaccctgtt accgccagca gcgagtagct 3600

aagacatctg cagataacaa caacgctgaa tactcgtggg ctggagctac cagataccac 3660

ctcaatggca gagactctct ggtgaatccg ggcccggcga tggcagccca cagggacgat 3720

gaagaaaggt tttttcctca ggccggggtt ctcatctttg gtagacaagg cgccgagaaa 3780

acaaatgtgg acattgaaaa ggtcatgatt acagacgaag aggaaatcag gacaaccaat 3840

cccgtggcta cggagcagta tggttctgta tctaccaacc tccagagagg caacagacaa 3900

gcagctaccg cagatgtcaa cacacaaggc gttcttccag gcatggtctg gcaggacaga 3960

gatgtgtacc ttcaggggcc catctgggca aagattccac acacggacgg acattttcac 4020

ccctctcccc tcatgggtgg attcggactt aaacaccctc ctccacagat tctcatcaag 4080

aacaccccgg tacctgcgaa tccgtcgacc accttcagtg cggccaagtt tgctgccttc 4140

atcgcacagt actccacggg acaggtcagc gtggagatcg agtgggagct gcagaaggaa 4200

aacagcaaac gctggaatcc cgaaattcag tacgcttcca actacaacaa gtctgttaat 4260

gtagactttg ctgtggacgc taatggcgtg tattcagagc ctcgccccat tggcaccaga 4320

tacctgactc gtaatctgta attgcttgtt aatcaataaa ccgtttaatt cgtttcagtt 4380

gaactttggt ctctgcgtat ttctttctta tctagtttcc atgctctaga gaaacctacg 4440

tcacccgccc cgttcccacg ccccgcgcca cgtcacaaac tccaccccct cattatcata 4500

ttggcttcaa tccaaaataa ggtatattat tgatgatgca tcgctggcgt aatagcgaag 4560

aggcccgcac cgatcgccct tcccaacagt tgcgcagcct gaatggcgaa tggaattcca 4620

gacgattgag cgtcaaaatg taggtatttc catgagcgtt tttcctgttg caatggctgg 4680

cggtaatatt gttctggata ttaccagcaa ggccgatagt ttgagttctt ctactcaggc 4740

aagtgatgtt attactaatc aaagaagtat tgcgacaacg gttaatttgc gtgatggaca 4800

gactctttta ctcggtggcc tcactgatta taaaaacact tctcaggatt ctggcgtacc 4860

gttcctgtct aaaatccctt taatcggcct cctgtttagc tcccgctctg attctaacga 4920

ggaaagcacg ttatacgtgc tcgtcaaagc aaccatagta cgcgccctgt agcggcgcat 4980

taagcgcggc gggtgtggtg gttacgcgca gcgtgaccgc tacacttgcc agcgccctag 5040

cgcccgctcc tttcgctttc ttcccttcct ttctcgccac gttcgccggc tttccccgtc 5100

aagctctaaa tcgggggctc cctttagggt tccgatttag tgctttacgg cacctcgacc 5160

ccaaaaaact tgattagggt gatggttcac gtagtgggcc atcgccctga tagacggttt 5220

ttcgcccttt gacgttggag tccacgttct ttaatagtgg actcttgttc caaactggaa 5280

caacactcaa ccctatctcg gtctattctt ttgatttata agggattttg ccgatttcgg 5340

cctattggtt aaaaaatgag ctgatttaac aaaaatttaa cgcgaatttt aacaaaatat 5400

taacgcttac aatttaaata tttgcttata caatcttcct gtttttgggg cttttctgat 5460

tatcaaccgg ggtacatatg attgacatgc tagttttacg attaccgttc atcgattctc 5520

ttgtttgctc cagactctca ggcaatgacc tgatagcctt tgtagagacc tctcaaaaat 5580

agctaccctc tccggcatga atttatcagc tagaacggtt gaatatcata ttgatggtga 5640

tttgactgtc tccggccttt ctcacccgtt tgaatcttta cctacacatt actcaggcat 5700

tgcatttaaa atatatgagg gttctaaaaa tttttatcct tgcgttgaaa taaaggcttc 5760

tcccgcaaaa gtattacagg gtcataatgt ttttggtaca accgatttag ctttatgctc 5820

tgaggcttta ttgcttaatt ttgctaattc tttgccttgc ctgtatgatt tattggatgt 5880

tggaatcgcc tgatgcggta ttttctcctt acgcatctgt gcggtatttc acaccgcata 5940

tggtgcactc tcagtacaat ctgctctgat gccgcatagt taagccagcc ccgacacccg 6000

ccaacacccg ctgacgcgcc ctgacgggct tgtctgctcc cggcatccgc ttacagacaa 6060

gctgtgaccg tctccgggag ctgcatgtgt cagaggtttt caccgtcatc accgaaacgc 6120

gcgagacgaa agggcctcgt gatacgccta tttttatagg ttaatgtcat gataataatg 6180

gtttcttaga cgtcaggtgg cacttttcgg ggaaatgtgc gcggaacccc tatttgttta 6240

tttttctaaa tacattcaaa tatgtatccg ctcatgagac aataaccctg ataaatgctt 6300

caataatatt gaaaaaggaa gagtatgagt attcaacatt tccgtgtcgc ccttattccc 6360

ttttttgcgg cattttgcct tcctgttttt gctcacccag aaacgctggt gaaagtaaaa 6420

gatgctgaag atcagttggg tgcacgagtg ggttacatcg aactggatct caacagcggt 6480

aagatccttg agagttttcg ccccgaagaa cgttttccaa tgatgagcac ttttaaagtt 6540

ctgctatgtg gcgcggtatt atcccgtatt gacgccgggc aagagcaact cggtcgccgc 6600

atacactatt ctcagaatga cttggttgag tactcaccag ttcccgtatt gacgccgggc 6660

aagagcaact cggtcgccgc atacactatt ctcagaatga cttggttgag tactcaccag 6720

tcacagaaaa gcatcttacg gatggcatga cagtaagaga attatgcagt gctgccataa 6780

ccatgagtga taacactgcg gccaacttac ttctgacaac gatcggagga ccgaaggagc 6840

taaccgcttt tttgcacaac atgggggatc atgtaactcg ccttgatcgt tgggaaccgg 6900

agctgaatga agccatacca aacgacgagc gtgacaccac gatgcctgta gcaatggcaa 6960

caacgttgcg caaactatta actggcgaac tacttactct agcttcccgg caacaattaa 7020

tagactggat ggaggcggat aaagttgcag gaccacttct gcgctcggcc cttccggctg 7080

gctggtttat tgctgataaa tctggagccg gtgagcgtgg gtctcgcggt atcattgcag 7140

cactggggcc agatggtaag ccctcccgta tcgtagttat ctacacgacg gggagtcagg 7200

caactatgga tgaacgaaat agacagatcg ctgagatagg tgcctcactg attaagcatt 7260

ggtaactgtc agaccaagtt tactcatata tactttagat tgatttaaaa cttcattttt 7320

aatttaaaag gatctaggtg aagatccttt ttgataatct catgaccaaa atcccttaac 7380

gtgagttttc gttccactga gcgtcagacc ccgtagaaaa gatcaaagga tcttcttgag 7440

atcctttttt tctgcgcgta atctgctgct tgcaaacaaa aaaaccaccg ctaccagcgg 7500

tggtttgttt gccggatcaa gagctaccaa ctctttttcc gaaggtaact ggcttcagca 7560

gagcgcagat accaaatact gttcctttct tctagtgtag ccgtagttag gccaccactt 7620

caagaactct gtagcaccgc ctacatacct cgctctgcta atcctgttac cagtggctgc 7680

tgccagtggc gataagtcgt gtcttaccgg gttggactca agacgatagt taccggataa 7740

ggcgcagcgg tcgggctgaa cggggggttc gtgcacacag cccagcttgg agcgaacgac 7800

ctacaccgaa ctgatatttt ttttttcgtg agctatgaga aagcgccacg cttcccgaag 7860

ggagaaaggc ggacaggtat ccggtaagcg gcagggtcgg aacaggagag cgcacgaggg 7920

agcttccagg gggaaacgcc tggtatcttt atagtcctgt cgggtttcgc cacctctgac 7980

ttgagcgtcg atttttgtga tgctcgtcag gggggcggag cctatggaaa aacgccagca 8040

acgcggcctt tttacggttc ctggcctttt gctggccttt tgctcacatg ttctttcctg 8100

cgttatcccc tgattctgtg gataaccgta ttaccgcctt tgagtgagct gataccgctc 8160

gccgcagccg aacgaccgag cgcagcgagt cagtgagcga ggaagcggaa gagcgcccaa 8220

tacgcaaacc gcctctcccc cgcgcgttgg ccgattcatt aatgcag 8267

<210> 151
<211> 8179
<212> DNA
<213> Adeno-Associated Virus

<220>
<221> gene
<222> (1)..(8179)

<400> 151
aattcccatc atcaataata taccttattt aggattgaag ccaatatgat aatgagccgg 60

tggagtttgt gacgtggcgc ggggcgtggg aacggggcgg gtgacgtagt agtctctaga 120

gtcctgtatt agaggtcacg tgagtgtttt gcgacttttt gcgacaccat gtggtcacgc 180

tgggtattta agcccgagtg agcacgcagg gtctccattt tgaagcggga ggtttgaacg 240

cgcagccacc acgggggggt tttacgagat tgtgattaag gtccccagcg accttgacgg 300

gcatctgccc ggcatttctg acagctttgt gaactgggtg gccgagaagg aatgggagtt 360

gccgccagat tctgacatgg atctgaatct gattgagcag gcacccctga ccgtggccga 420

gaagctgcag cgcgactttc tgacggaatg gcgccgtgtg agtaaggccc cggaggccct 480

tttctttgtg caatttgaga agggagagag ctacttccac atgcacgtgc tcgtggaaac 540

caccggggtg aaatccatgg ttttgggacg tttcctgagt cagattcgcg aaaaactgat 600

tcagagaatt taccgcggga tcgagccgac tttgccaaac tggttcgcgg tcacaaagac 660

cagaaatggc gccggaggcg ggaacaaggt ggtggatgag tgctacatcc ccaattactt 720

gctccccaaa acccagcctg agctccagtg ggcgtggact aatatggaac agtatttaag 780
cgcctgtttg aatctcacgg agcgtaaacg gttggtggcg cagcatctga cgcacgtgtc 840
gcagacgcag gagcagaaca aagagaatca gaatcccaat tctgatgcgc cggtgatcag 900
atcaaaaact tcagccaggt acatggagct ggtcgggtgg ctcgtggaca aggggattac 960
ctcggagaag cagtggatcc aggaggacca ggcctcatac atctccttca atgcggcctc 1020
caactcgcgg tcccaaatca aggctgcctt ggacaatgcg ggaaagatta tgagcctgac 1080
taaaaccgcc cccgactacc tggtgggcca gcagcccgtg gaggacattt ccagcaatcg 1140
gatttataaa attttggaac taaacgggta cgatccccaa tatgcggctt ccgtctttct 1200
gggatgggcc acgaaaaagt tcggcaagag gaacaccatc tggctgtttg ggcctgcaac 1260
taccgggaag accaacatcg cggaggccat agcccacact gtgcccttct acgggtgcgt 1320
aaactggacc aatgagaact ttcccttcaa cgactgtgtc gacaagatgg tgatctggtg 1380
ggaggagggg aagatgaccg ccaaggtcgt ggagtcggcc aaagccattc tcggaggaag 1440
caaggtgcgc gtggaccaga aatgcaagtc ctcggcccag atagacccga ctcccgtgat 1500
cgtcacctcc aacaccaaca tgtgcgccgt gattgacggg aactcaacga ccttcgaaca 1560
ccagcagccg ttgcaagacc ggatgttcaa atttgaactc acccgccgtc tggatcatga 1620
ctttgggaag gtcaccaagc aggaagtcaa agactttttc cggtgggcaa aggatcacgt 1680
ggttgaggtg gagcatgaat tctacgtcaa aaagggtgga gccaagaaaa gacccgcccc 1740
cagtgacgca gatataagtg agcccaaacg ggtgcgcgag tcagttgcgc agccatcgac 1800
gtcagacgcg gaagcttcga tcaactacgc agacaggtac caaaacaaat gttctcgtca 1860
cgtgggcatg aatctgatgc tgtttccctg cagacaatgc gagagaatga atcagaattc 1920
aaatatctgc ttcactcacg gacagaaaga ctgtttagag tgctttcccg tgtcagaatc 1980
tcaacccgtt tctgtcgtca aaaaggcgta tcagaaactg tgctacattc atcatatcat 2040
gggaaaggtg ccagacgctt gcactgcctg cgatctggtc aatgtggatt tggatgactg 2100
catctttgaa caataaatga tttaaatcag gtatggctgc tgacggttat cttccagatt 2160
ggctcgagga caacctttct gaaggcattc gtgagtggtg ggctctgaaa cctggagtcc 2220
ctcaacccaa agcgaaccaa caacaccagg acaaccgtcg gggtcttgtg cttccgggtt 2280
acaaatacct cggacccggt aacggactcg acaaaggaga gccggtcaac gaggcggacg 2340
cggcagccct cgaacacgac aaagcttacg accagcagct caaggccggt gacaacccgt 2400
acctcaagta caaccacgcc gacgccgagt ttcaggagcg tcttcaagaa gatacgtctt 2460
ttgggggcaa ccttggcaga gcagtcttcc aggccaaaaa gaggatcctt gagcctcttg 2520
gtctggttga ggaagcagct agaacggctc ctggaaagaa gggggctgta gatcagtctc 2580
ctcaggaacc ggactcatca tctggtgttg gcaaatcggg caaacagcct gccagaaaaa 2640

gactaaattt cggtcagact ggagactcag agtcagtccc agaccctcaa cctctcggag 2700

aaccaccagc agcccccaca agtttgggat ctaatacaat ggcttcaggc ggtggcgcac 2760

caatggcaga caataacgag ggtgccgatg gagtgggtaa ttcctcagga aattggcatt 2820

gcgattccca atggctgggc gacagagtca tcaccaccag caccagaacc tgggccctgc 2880

ccgcttacaa caaccatctc tacaagcaaa tctccagcca atcaggagct tcaaacgaca 2940

accactactt tggctacagc accccttggg ggtattttga ctttaacaga ttccactgcc 3000

acttctcacc acgtgactgg cagcgactca ttaacaacaa ctggggattc cggcccaaga 3060

aactcagctt caagctcttc aacatccaag ttagaggggt cacgcagaac gatggcacga 3120

cgactattgc caataacctt accagcacgg ttcaagtgtt tacggactcg gagtatcagc 3180

tcccgtacgt gctcgggtcg gcgcaccaag gctgtctccc gccgtttcca gcggacgtct 3240

tcatggtccc tcagtatgga tacctcaccc tgaacaacgg aagtcaagcg gtgggacgct 3300

catcctttta ctgcctggag tacttccctt cgcagatgct aaggactgga aataacttcc 3360

aattcagcta taccttcgag gatgtacctt ttcacagcag ctacgctcac agccagagtt 3420

tggatcgctt gatgaatcct cttattgatc agtatctgta ctacctgaac agaacgcaag 3480

gaacaacctc tggaacaacc aaccaatcac ggctgctttt tagccaggct gggcctcagt 3540

ctatgtcttt gcaggccaga aattggctac ctgggccctg ctaccggcaa cagagacttg 3600

caaagactgc taacgacaac aacaacagta actttccttg gacagcggcc agcaaatatc 3660

atctcaatgg ccgcgactcg ctggtgaatc caggaccagc tatggccagt cacaaggacg 3720

atgaagaaaa atttttccct atgcacggca atctaatatt tggcaaagaa gggacaacgg 3780

caagtaacgc agaattagat aatgtaatga ttacggatga agaagagatt cgtaccacca 3840

atcctgtggc aacagagcag tatggaactg tggcaaataa cttgcagagc tcaaatacag 3900

ctcccacgac tggaactgtc aatcatcagg gggccttacc tggcatggtg tggcaagatc 3960

gtgacgtgta ccttcaagga cctatctggg caaagattcc tcacacggat ggacactttc 4020

atccttctcc tctgatggga ggctttggac tgaaacatcc gcctcctcaa atcatgatca 4080

aaaatactcc ggtaccggca aatcctccga cgactttcag cccggccaag tttgctgcat 4140

ttatcactca gtactccact ggacaggtca gcgtggaaat tgagtgggag ctacagaaag 4200

aaaacagcaa acgttggaat ccagagattc agtacacttc caactacaac aagtctgtta 4260

atgtggactt tactgtagac actaatggtg tttatagtga acctcgccct attggaaccc 4320

ggtatctcac acgaaacttg tgattgcttg ttaatcaata aaccgtttaa ttcgtttcag 4380

ttgaactttg gtctctgcgt atttctttct tatctagttt ccatgctcta gactactacg 4440

tcacccgccc cgttcccacg ccccgcgcca cgtcacaaac tccaccccct cattatcata 4500

ttggcttcaa tccaaaataa ggtatattat tgatgatgca tcgctggcgt aatagcgaag 4560

aggcccgcac cgatcgccct tcccaacagt tgcgcagcct gaatggcgaa tggaattcca 4620

gacgattgag cgtcaaaatg taggtatttc catgagcgtt tttcctgttg caatggctgg 4680

cggtaatatt gttctggata ttaccagcaa ggccgatagt ttgagttctt ctactcaggc 4740

aagtgatgtt attactaatc aaagaagtat tgcgacaacg gttaatttgc gtgatggaca 4800

gactctttta ctcggtggcc tcactgatta taaaaacact tctcaggatt ctggcgtacc 4860

gttcctgtct aaaatccctt taatcggcct cctgtttagc tcccgctctg attctaacga 4920

ggaaagcacg ttatacgtgc tcgtcaaagc aaccatagta cgcgccctgt agcggcgcat 4980

taagcgcggc gggtgtggtg gttacgcgca gcgtgaccgc tacacttgcc agcgccctag 5040

cgcccgctcc tttcgctttc ttcccttcct ttctcgccac gttcgccggc tttccccgtc 5100

aagctctaaa tcgggggctc cctttagggt tccgatttag tgctttacgg cacctcgacc 5160

ccaaaaaact tgattagggt gatggttcac gtagtgggcc atcgccctga tagacggttt 5220

ttcgcccttt gacgttggag tccacgttct ttaatagtgg actcttgttc caaactggaa 5280

caacactcaa ccctatctcg gtctattctt ttgatttata agggattttg ccgatttcgg 5340

cctattggtt aaaaaatgag ctgatttaac aaaaatttaa cgcgaatttt aacaaaatat 5400

taacgtttac aatttaaata tttgcttata caatcttcct gtttttgggg cttttctgat 5460

tatcaaccgg ggtacatatg attgacatgc tagttttacg attaccgttc atcgattctc 5520

ttgtttgctc cagactctca ggcaatgacc tgatagcctt tgtagagacc tctcaaaaat 5580

agctaccctc tccggcatga atttatcagc tagaacggtt gaatatcata ttgatggtga 5640

tttgactgtc tccggccttt ctcacccgtt tgaatcttta cctacacatt actcaggcat 5700

tgcatttaaa atatatgagg gttctaaaaa tttttatcct tgcgttgaaa taaaggcttc 5760

tcccgcaaaa gtattacagg gtcataatgt ttttggtaca accgatttag ctttatgctc 5820

tgaggcttta ttgcttaatt ttgctaattc tttgccttgc ctgtatgatt tattggatgt 5880

tggaattcct gatgcggtat tttctcctta cgcatctgtg cggtatttca caccgcatat 5940

ggtgcactct cagtacaatc tgctctgatg ccgcatagtt aagccagccc cgacacccgc 6000

caacacccgc tgacgcgccc tgacgggctt gtctgctccc ggcatccgct tacagacaag 6060

ctgtgaccgt ctccgggagc tgcatgtgtc agaggttttc accgtcatca ccgaaacgcg 6120

cgagacgaaa gggcctcgtg atacgcctat ttttataggt taatgtcatg ataataatgg 6180

tttcttagac gtcaggtggc acttttcggg gaaatgtgcg cggaacccct atttgtttat 6240

ttttctaaat acattcaaat atgtatccgc tcatgagaca ataaccctga taaatgcttc 6300

aataatattg aaaaaggaag agtatgagta ttcaacattt ccgtgtcgcc cttattccct 6360

tttttgcggc attttgcctt cctgtttttg ctcacccaga aacgctggtg aaagtaaaag 6420

atgctgaaga tcagttgggt gcacgagtgg gttacatcga actggatctc aacagcggta 6480

agatccttga gagttttcgc cccgaagaac gttttccaat gatgagcact tttaaagttc 6540

tgctatgtgg cgcggtatta tcccgtattg acgccgggca agagcaactc ggtcgccgca 6600

tacactattc tcagaatgac ttggttgagt actcaccagt cacagaaaag catcttacgg 6660

atggcatgac agtaagagaa ttatgcagtg ctgccataac catgagtgat aacactgcgg 6720

ccaacttact tctgacaacg atcggaggac cgaaggagct aaccgctttt ttgcacaaca 6780

tgggggatca tgtaactcgc cttgatcgtt gggaaccgga gctgaatgaa gccataccaa 6840

acgacgagcg tgacaccacg atgcctgtag caatggcaac aacgttgcgc aaactattaa 6900

ctggcgaact acttactcta gcttcccggc aacaattaat agactggatg gaggcggata 6960

aagttgcagg accacttctg cgctcggccc ttccggctgg ctggtttatt gctgataaat 7020

ctggagccgg tgagcgtggg tctcgcggta tcattgcagc actggggcca gatggtaagc 7080

cctcccgtat cgtagttatc tacacgacgg ggagtcaggc aactatggat gaacgaaata 7140

gacagatcgc tgagataggt gcctcactga ttaagcattg gtaactgtca gaccaagttt 7200

actcatatat actttagatt gatttaaaac ttcattttta atttaaaagg atctaggtga 7260

agatcctttt tgataatctc atgaccaaaa tcccttaacg tgagttttcg ttccactgag 7320

cgtcagaccc cgtagaaaag atcaaaggat cttcttgaga tccttttttt ctgcgcgtaa 7380

tctgctgctt gcaaacaaaa aaaccaccgc taccagcggt ggtttgtttg ccggatcaag 7440

agctaccaac tctttttccg aaggtaactg gcttcagcag agcgcagata ccaaatactg 7500

tccttctagt gtagccgtag ttaggccacc acttcaagaa ctctgtagca ccgcctacat 7560

acctcgctct gctaatcctg ttaccagtgg ctgctgccag tggcgataag tcgtgtctta 7620

ccgggttgga ctcaagacga tagttaccgg ataaggcgca gcggtcgggc tgaacggggg 7680

gttcgtgcac acagcccagc ttggagcgaa cgacctacac cgaactgaga tacctacagc 7740

gtgagctatg agaaagcgcc acgcttcccg aagggagaaa ggcggacagg tatccggtaa 7800

gcggcagggt cggaacagga gagcgcacga gggagcttcc agggggaaac gcctggtatc 7860

tttatagtcc tgtcgggttt cgccacctct gacttgagcg tcgatttttg tgatgctcgt 7920

caggggggcg gagcctatgg aaaaacgcca gcaacgcggc ctttttacgg ttcctggcct 7980

tttgctggcc ttttgctcac atgttctttc ctgcgttatc ccctgattct gtggataacc 8040

gtattaccgc ctttgagtga gctgataccg ctcgccgcag ccgaacgacc gagcgcagcg 8100

agtcagtgag cgaggaagcg gaagagcgcc caatacgcaa accgcctctc cccgcgcgtt 8160

ggccgattca ttaatgcag 8179

<210> 152

<211>  8172
<212>  DNA
<213>  Adeno-Associated Virus


<220>
<221>  gene
<222>  (1)..(8172)

<400>  152
aattcccatc atcaataata taccttattt aggattgaag ccaatatgat aatgagccgg      60

tggagtttgt gacgtggcgc ggggcgtggg aacggggcgg gtgacgtagt agtctctaga     120

gtcctgtatt agaggtcacg tgagtgtttt gcgacttttt gcgacaccat gtggtcacgc     180

tgggtattta agcccgagtg agcacgcagg gtctccattt tgaagcggga ggtttgaacg     240

cgcagccacc acgggggggt tttacgagat tgtgattaag gtccccagcg accttgacgg     300

gcatctgccc ggcatttctg acagctttgt gaactgggtg gccgagaagg aatgggagtt     360

gccgccagat tctgacatgg atctgaatct gattgagcag gcacccctga ccgtggccga     420

gaagctgcag cgcgactttc tgacggaatg gcgccgtgtg agtaaggccc cggaggccct     480

tttctttgtg caatttgaga agggagagag ctacttccac atgcacgtgc tcgtggaaac     540

caccggggtg aaatccatgg ttttgggacg tttcctgagt cagattcgcg aaaaactgat     600

tcagagaatt taccgcggga tcgagccgac tttgccaaac tggttcgcgg tcacaaagac     660

cagaaatggc gccggaggcg ggaacaaggt ggtggatgag tgctacatcc ccaattactt     720

gctccccaaa acccagcctg agctccagtg ggcgtggact aatatggaac agtatttaag     780

cgcctgtttg aatctcacgg agcgtaaacg gttggtggcg cagcatctga cgcacgtgtc     840

gcagacgcag gagcagaaca aagagaatca gaatcccaat tctgatgcgc cggtgatcag     900

atcaaaaact tcagccaggt acatggagct ggtcgggtgg ctcgtggaca aggggattac     960

ctcggagaag cagtggatcc aggaggacca ggcctcatac atctccttca atgcggcctc    1020

caactcgcgg tcccaaatca aggctgcctt ggacaatgcg ggaaagatta tgagcctgac    1080

taaaaccgcc cccgactacc tggtgggcca gcagcccgtg gaggacattt ccagcaatcg    1140

gatttataaa attttggaac taaacgggta cgatccccaa tatgcggctt ccgtctttct    1200

gggatgggcc acgaaaaagt tcggcaagag gaacaccatc tggctgtttg ggcctgcaac    1260

taccgggaag accaacatcg cggaggccat agcccacact gtgcccttct acgggtgcgt    1320

aaactggacc aatgagaact ttcccttcaa cgactgtgtc gacaagatgg tgatctggtg    1380

ggaggagggg aagatgaccg ccaaggtcgt ggagtcggcc aaagccattc tcggaggaag    1440

caaggtgcgc gtggaccaga aatgcaagtc ctcggcccag atagacccga ctcccgtgat    1500

cgtcacctcc aacaccaaca tgtgcgccgt gattgacggg aactcaacga ccttcgaaca    1560

ccagcagccg ttgcaagacc ggatgttcaa atttgaactc acccgccgtc tggatcatga    1620

ctttgggaag gtcaccaagc aggaagtcaa agactttttc cggtgggcaa aggatcacgt 1680

ggttgaggtg gagcatgaat tctacgtcaa aaagggtgga gccaagaaaa gacccgcccc 1740

cagtgacgca gatataagtg agcccaaacg ggtgcgcgag tcagttgcgc agccatcgac 1800

gtcagacgcg gaagcttcga tcaactacgc agacaggtac caaaacaaat gttctcgtca 1860

cgtgggcatg aatctgatgc tgtttccctg cagacaatgc gagagaatga atcagaattc 1920

aaatatctgc ttcactcacg gacagaaaga ctgtttagag tgctttcccg tgtcagaatc 1980

tcaacccgtt tctgtcgtca aaaaggcgta tcagaaactg tgctacattc atcatatcat 2040

gggaaaggtg ccagacgctt gcactgcctg cgatctggtc aatgtggatt tggatgactg 2100

catctttgaa caataaatga tttaaatcag gtatgactga cggttacctt ccagattggc 2160

tagaggacaa cctctctgaa ggcgttcgag agtggtgggc gctgcaacct ggagccccta 2220

aacccaaggc aaatcaacaa catcaggaca acgctcgggg tcttgtgctt ccgggttaca 2280

aatacctcgg acccggcaac ggactcgaca agggggaacc cgtcaacgca gcggacgcgg 2340

cagccctcga gcacgacaag gcctacgacc agcagctcaa ggccggtgac aacccctacc 2400

tcaagtacaa ccacgccgac gcggagttcc agcagcggct tcagggcgac acatcgtttg 2460

ggggcaacct cggcagagca gtcttccagg ccaaaaagag ggttcttgaa cctcttggtc 2520

tggttgagca agcgggtgag acggctcctg gaaagaggag accgttgatt gaatcccccc 2580

agcagcccga ctcctccacg ggtatcggca aaaaaggcaa gcagccggct aaaaagaagc 2640

tcgttttcga agacgaaact ggagcaggcg acggaccccc tgagggatca acttccggag 2700

ccatgtctga tgacagtgag atgcgtgcag cagctggcgg agctgcagtc gagggcggac 2760

aaggtgccga tggagtgggt aatgcctcgg gtgattggca ttgcgattcc acctggtctg 2820

agggccacgt cacgaccacc agcaccagaa cctgggtctt gcccgcctac aacaaccacc 2880

tctacaagcg actcggagag agcctgcagt ccaacaccta caacggattc tccaccccct 2940

ggggatactt tgacttcaac cgcttccact gccacttctc accacgtgac tggcagcgac 3000

tcatcaacaa caactggggc atgcgaccca aagccatgcg ggtcaaaatc ttcaacatcc 3060

aggtcaagga ggtcacgacg tcgaacggcg agacaacggt ggctaataac cttaccagca 3120

cggttcagat ctttgcggac tcgtcgtacg aactgccgta cgtgatggat gcgggtcaag 3180

agggcagcct gcctcctttt cccaacgacg tctttatggt gccccagtac ggctactgtg 3240

gactggtgac cggcaacact tcgcagcaac agactgacag aaatgccttc tactgcctgg 3300

agtactttcc ttcgcagatg ctgcggactg gcaacaactt tgaaattacg tacagttttg 3360

agaaggtgcc tttccactcg atgtacgcgc acagccagag cctggaccgg ctgatgaacc 3420

ctctcatcga ccagtacctg tggggactgc aatcgaccac caccggaacc accctgaatg 3480

ccgggactgc caccaccaac tttaccaagc tgcggcctac caacttttcc aactttaaaa 3540

agaactggct gcccgggcct tcaatcaagc agcagggctt cgcaaagact gccaatcaaa 3600

actacaagat ccctgccacc gggtcagaca gtctcatcaa atacgagacg cacagcactc 3660

tggacggaag atggagtgcc ctgacccccg gacctccaat ggccacggct ggacctgcgg 3720

acagcaagtt cagcaacagc cagctcatct ttgcggggcc taaacagaac ggcaacacgg 3780

ccaccgtacc cgggactctg atcttcacct ctgaggagga gctggcagcc accaacgcca 3840

ccgatacgga catgtggggc aacctacctg gcggtgacca gagcaacagc aacctgccga 3900

ccgtggacag actgacagcc ttgggagccg tgcctggaat ggtctggcaa aacagagaca 3960

tttactacca gggtcccatt tgggccaaga ttcctcatac cgatggacac tttcacccct 4020

caccgctgat tggtgggttt gggctgaaac acccgcctcc tcaaattttt atcaagaaca 4080

ccccggtacc tgcgaatcct gcaacgacct tcagctctac tccggtaaac gccttcatta 4140

ctcagtacag cactggccag gtgtcggtgc agattgactg ggagatccag aaggagcggt 4200

ccaaacgctg gaaccccgag gtccagttta cctccaacta cggacagcaa aactctctgt 4260

tgtgggctcc cgatgcggct gggaaataca ctgagcctag ggctatcggt acccgctacc 4320

tcacccacca cctgtaattg cttgttaatc aataaaccgt ttaattcgtt tcagttgaac 4380

tttggtctct gcgtatttct ttcttatcta gtttccatgc tctagactac tacgtcaccc 4440

gccccgttcc cacgccccgc gccacgtcac aaactccacc ccctcattat catattggct 4500

tcaatccaaa ataaggtata ttattgatga tgcatcgctg gcgtaatagc gaagaggccc 4560

gcaccgatcg cccttcccaa cagttgcgca gcctgaatgg cgaatggaat tccagacgat 4620

tgagcgtcaa aatgtaggta tttccatgag cgtttttcct gttgcaatgg ctggcggtaa 4680

tattgttctg gatattacca gcaaggccga tagtttgagt tcttctactc aggcaagtga 4740

tgttattact aatcaaagaa gtattgcgac aacggttaat ttgcgtgatg gacagactct 4800

tttactcggt ggcctcactg attataaaaa cacttctcag gattctggcg taccgttcct 4860

gtctaaaatc cctttaatcg gcctcctgtt tagctcccgc tctgattcta acgaggaaag 4920

cacgttatac gtgctcgtca aagcaaccat agtacgcgcc ctgtagcggc gcattaagcg 4980

cggcgggtgt ggtggttacg cgcagcgtga ccgctacact tgccagcgcc ctagcgcccg 5040

ctcctttcgc tttcttccct tcctttctcg ccacgttcgc cggctttccc cgtcaagctc 5100

taaatcgggg gctcccttta gggttccgat ttagtgcttt acggcacctc gaccccaaaa 5160

aacttgatta gggtgatggt tcacgtagtg ggccatcgcc ctgatagacg gtttttcgcc 5220

ctttgacgtt ggagtccacg ttctttaata gtggactctt gttccaaact ggaacaacac 5280

tcaaccctat ctcggtctat tcttttgatt tataagggat tttgccgatt tcggcctatt 5340

ggttaaaaaa tgagctgatt taacaaaaat ttaacgcgaa ttttaacaaa atattaacgt 5400

ttacaattta aatatttgct tatacaatct tcctgttttt ggggcttttc tgattatcaa 5460

ccggggtaca tatgattgac atgctagttt tacgattacc gttcatcgat tctcttgttt 5520

gctccagact ctcaggcaat gacctgatag cctttgtaga gacctctcaa aaatagctac 5580

cctctccggc atgaatttat cagctagaac ggttgaatat catattgatg gtgatttgac 5640

tgtctccggc ctttctcacc cgtttgaatc tttacctaca cattactcag gcattgcatt 5700

taaaatatat gagggttcta aaaattttta tccttgcgtt gaaataaagg cttctcccgc 5760

aaaagtatta cagggtcata atgtttttgg tacaaccgat ttagctttat gctctgaggc 5820

tttattgctt aattttgcta attctttgcc ttgcctgtat gatttattgg atgttggaat 5880

tcctgatgcg gtattttctc cttacgcatc tgtgcggtat ttcacaccgc atatggtgca 5940

ctctcagtac aatctgctct gatgccgcat agttaagcca gccccgacac ccgccaacac 6000

ccgctgacgc gccctgacgg gcttgtctgc tcccggcatc cgcttacaga caagctgtga 6060

ccgtctccgg gagctgcatg tgtcagaggt tttcaccgtc atcaccgaaa cgcgcgagac 6120

gaaagggcct cgtgatacgc ctatttttat aggttaatgt catgataata atggtttctt 6180

agacgtcagg tggcactttt cggggaaatg tgcgcggaac ccctatttgt ttatttttct 6240

aaatacattc aaatatgtat ccgctcatga gacaataacc ctgataaatg cttcaataat 6300

attgaaaaag gaagagtatg agtattcaac atttccgtgt cgcccttatt cccttttttg 6360

cggcattttg ccttcctgtt tttgctcacc cagaaacgct ggtgaaagta aaagatgctg 6420

aagatcagtt gggtgcacga gtgggttaca tcgaactgga tctcaacagc ggtaagatcc 6480

ttgagagttt tcgccccgaa gaacgttttc caatgatgag cacttttaaa gttctgctat 6540

gtggcgcggt attatcccgt attgacgccg ggcaagagca actcggtcgc cgcatacact 6600

attctcagaa tgacttggtt gagtactcac cagtcacaga aaagcatctt acggatggca 6660

tgacagtaag agaattatgc agtgctgcca taaccatgag tgataacact gcggccaact 6720

tacttctgac aacgatcgga ggaccgaagg agctaaccgc ttttttgcac aacatggggg 6780

atcatgtaac tcgccttgat cgttgggaac cggagctgaa tgaagccata ccaaacgacg 6840

agcgtgacac cacgatgcct gtagcaatgg caacaacgtt gcgcaaacta ttaactggcg 6900

aactacttac tctagcttcc cggcaacaat taatagactg gatggaggcg gataaagttg 6960

caggaccact tctgcgctcg gcccttccgg ctggctggtt tattgctgat aaatctggag 7020

ccggtgagcg tgggtctcgc ggtatcattg cagcactggg gccagatggt aagccctccc 7080

gtatcgtagt tatctacacg acggggagtc aggcaactat ggatgaacga aatagacaga 7140

tcgctgagat aggtgcctca ctgattaagc attggtaact gtcagaccaa gtttactcat 7200

atatacttta gattgattta aaacttcatt tttaatttaa aaggatctag gtgaagatcc 7260

tttttgataa tctcatgacc aaaatccctt aacgtgagtt ttcgttccac tgagcgtcag 7320

accccgtaga aaagatcaaa ggatcttctt gagatccttt ttttctgcgc gtaatctgct 7380

gcttgcaaac aaaaaaacca ccgctaccag cggtggtttg tttgccggat caagagctac 7440

caactctttt tccgaaggta actggcttca gcagagcgca gataccaaat actgtccttc 7500

tagtgtagcc gtagttaggc caccacttca agaactctgt agcaccgcct acatacctcg 7560

ctctgctaat cctgttacca gtggctgctg ccagtggcga taagtcgtgt cttaccgggt 7620

tggactcaag acgatagtta ccggataagg cgcagcggtc gggctgaacg gggggttcgt 7680

gcacacagcc cagcttggag cgaacgacct acaccgaact gagataccta cagcgtgagc 7740

tatgagaaag cgccacgctt cccgaaggga gaaaggcgga caggtatccg gtaagcggca 7800

gggtcggaac aggagagcgc acgagggagc ttccaggggg aaacgcctgg tatctttata 7860

gtcctgtcgg gtttcgccac ctctgacttg agcgtcgatt tttgtgatgc tcgtcagggg 7920

ggcggagcct atggaaaaac gccagcaacg cggccttttt acggttcctg gccttttgct 7980

ggccttttgc tcacatgttc tttcctgcgt tatcccctga ttctgtggat aaccgtatta 8040

ccgcctttga gtgagctgat accgctcgcc gcagccgaac gaccgagcgc agcgagtcag 8100

tgagcgagga agcggaagag cgcccaatac gcaaaccgcc tctccccgcg cgttggccga 8160

ttcattaatg ca 8172

<210> 153
<211> 7226
<212> DNA
<213> Adeno-Associated Virus

<220>
<221> gene
<222> (1)..(7226)

<400> 153
cacctaaatt gtaagcgtta atattttgtt aaaattcgcg ttaaattttt gttaaatcag 60

ctcatttttt aaccaatagg ccgaaatcgg caaaatccct tataaatcaa aagaatagac 120

cgagataggg ttgagtgttg ttccagtttg gaacaagagt ccactattaa agaacgtgga 180

ctccaacgtc aaagggcgaa aaaccgtcta tcagggcgat ggcccactac gtgaaccatc 240

accctaatca agttttttgg ggtcgaggtg ccgtaaagca ctaaatcgga accctaaagg 300

gagcccccga tttagagctt gacggggaaa gccggcgaac gtggcgagaa aggaagggaa 360

gaaagcgaaa ggagcgggcg ctagggcgct ggcaagtgta gcggtcacgc tgcgcgtaac 420

caccacaccc gccgcgctta atgcgccgct acagggcgcg tcccattcgc cattcaggct 480

gcgcaactgt tgggaagggc gatcggtgcg ggcctcttcg ctattacgcc agctggcgaa 540

agggggatgt gctgcaaggc gattaagttg ggtaacgcca gggttttccc agtcacgacg 600

ttgtaaaacg acggccagtg aattgtaata cgactcacta tagggcgaat tggagctcca 660

ccgcggtggc ggccgctcta gagtcctgta ttagaggtca cgtgagtgtt ttgcgacatt 720

ttgcgacacc atgtggtcac gctgggtatt taagcccgag tgagcacgca gggtctccat 780

tttgaagcgg gaggtttgaa cgcgcagcca ccacggcggg gttttacgag attgtgatta 840

aggtccccag cgaccttgac gggcatctgc ccggcatttc tgacagcttt gtgaactggg 900

tggccgagaa ggaatgggag ttgccgccag attctgacat ggatctgaat ctgattgagc 960

aggcacccct gaccgtggcc gagaagctgc agcgcgactt tctgacggaa tggcgccgtg 1020

tgagtaaggc cccggaggcc cttttctttg tgcaatttga gaagggagag agctacttcc 1080

acatgcacgt gctcgtggaa accaccgggg tgaaatccat ggttttggga cgtttcctga 1140

gtcagattcg cgaaaaactg attcagagaa tttaccgcgg gatcgagccg actttgccaa 1200

actggttcgc ggtcacaaag accagaaatg gcgccggagg cgggaacaag gtggtggatg 1260

agtgctacat ccccaattac ttgctcccca aaacccagcc tgagctccag tgggcgtgga 1320

ctaatatgga acagtattta agcgcctgtt tgaatctcac ggagcgtaaa cggttggtgg 1380

cgcagcatct gacgcacgtg tcgcagacgc aggagcagaa caaagagaat cagaatccca 1440

attctgatgc gccggtgatc agatcaaaaa cttcagccag gtacatggag ctggtcgggt 1500

ggctcgtgga caaggggatt acctcggaga agcagtggat ccaggaggac caggcctcat 1560

acatctcctt caatgcggcc tccaactcgc ggtcccaaat caaggctgcc ttggacaatg 1620

cgggaaagat tatgagcctg actaaaaccg cccccgacta cctggtgggc cagcagcccg 1680

tggaggacat ttccagcaat cggatttata aaattttgga actaaacggg tacgatcccc 1740

aatatgcggc ttccgtcttt ctgggatggg ccacgaaaaa gttcggcaag aggaacacca 1800

tctggctgtt tgggcctgca actaccggga agaccaacat cgcggaggcc atagcccaca 1860

ctgtgccctt ctacgggtgc gtaaactgga ccaatgagaa ctttcccttc aacgactgtg 1920

tcgacaagat ggtgatctgg tgggaggagg ggaagatgac cgccaaggtc gtggagtcgg 1980

ccaaagccat tctcggagga agcaaggtgc gcgtggacca gaaatgcaag tcctcggccc 2040

agatagaccc gactcccgtg atcgtcacct ccaacaccaa catgtgcgcc gtgattgacg 2100

ggaactcaac gaccttcgaa caccagcagc cgttgcaaga ccggatgttc aaatttgaac 2160

tcacccgccg tctggatcat gactttggga aggtcaccaa gcaggaagtc aaagactttt 2220

tccggtgggc aaaggatcac gtggttgagg tggagcatga attctacgtc aaaaagggtg 2280

gagccaagaa aagacccgcc cccagtgacg cagatataag tgagcccaaa cgggtgcgcg 2340

agtcagttgc gcagccatcg acgtcagacg cggaagcttc gatcaactac gcagacaggt 2400

accaaaacaa atgttctcgt cacgtgggca tgaatctgat gctgtttccc tgcagacaat 2460

gcgagagaat gaatcagaat tcaaatatct gcttcactca cggacagaaa gactgtttag 2520

agtgctttcc cgtgtcagaa tctcaacccg tttctgtcgt caaaaaggcg tatcagaaac 2580

tgtgctacat tcatcatatc atgggaaagg tgccagacgc ttgcactgcc tgcgatctgg 2640

tcaatgtgga tttggatgac tgcatctttg aacaataaat gatttaaata gtcatgtctt 2700

ttgttgatca ccctccagat tggttggaag aagttggtga aggtcttcgc gagtttttgg 2760

gccttgaagc gggcccaccg aaaccagaac ccaatcagca gcatcaagat caagcccgtg 2820

gtcttgtgct gcctggttat aactatctcg gacccggaaa cggtctcgat cgaggagagc 2880

ctgtcaacag ggcagacgag gtcgcgcgag agcacgacat ctcgtacaac gagcagcttg 2940

aggcgggaga caacccctac ctcaagtaca accacgcgga cgccgagttt caggagaagc 3000

tcgccgacga cgcatccttc gggggaaacc tcggaaaggc agtctttcag gccaagaaaa 3060

gggttctcga accttttggc ctggttgaag agggtgctaa gacggcccct accggaaagc 3120

ggatagacga ccactttcca aaaagaaaga aggctcggac cgaagaggac tccaagcctt 3180

ccacctcgtc agacgccgaa gctggaccca gcggatccca gcagctgcaa atcccagccc 3240

aaccagcctc aagtttggga gctgatacaa tgtctgcggg aggtggcggc ccattgggcg 3300

acaataacca aggtgccgat ggagtgggca atgcctcggg agattggcat tgcgattcca 3360

cgtggatggg ggacagagtc gtcaccaagt ccacccgaac ctgggtgctg cccagctaca 3420

acaaccacca gtaccgagag atcaaaagcg gctccgtcga cggaagcaac gccaacgcct 3480

actttggata cgccaccccc tgggggtact ttgactttaa ccgcttccac agccactgga 3540

gcccccgaga ctggcaaaga ctcatcaaca actactgggg cttcagaccc cggtccctca 3600

gagtcaaaat cttcaacatt caagtcaaag aggtcacggt gcaggactcc accaccacca 3660

tcgccaacaa cctcgcctcc accgtccaag tgtttacgga cgacgactac cagctgccct 3720

acgtcgtcgg caacggaccc gagggatgcc tgccggcctt ccctccgcag gtctttacgc 3780

tgccgcagta cggttacgcg acgctgaacc gcgacaacac agaaaatccc accgagagga 3840

gcagcttctt ctgcctagag tactttccca gcaagatgct gagaacgggc aacaactttg 3900

agtttaccta caactttgag gaggtgccct tccactccag cttcgctccc agtcagaacc 3960

tgttcaagct ggccaacccg ctggtggacc agtacttgta ccgcttcgtg agcacaaata 4020

acactggcgg agtccagttc aacaagaacc tggccgggag atacgccaac acctacaaaa 4080

actggttccc ggggcccatg ggccgaaccc agggctggaa cctgggctcc ggggtcaacc 4140

gcgccgctgt cagcgccttc gccacgacca ataggatgga gctcgagggc gcgagttacc 4200

aggtgccccc gcagccgaac ggcatgacca acaacctcca gggcagcaac acctatgccc 4260

tggagaacac tatgatcttc aacagccagc cggcgaaccc gggcaccacc gccacgtacc 4320

tcgagggcaa catgctcatc accagcgaga gcgagacgca gccggtgaac cgcgtggcgt 4380

acaacgtcgg cgggcagatg gccaccaaca accagagctc caccactgcc cccgcgaccg 4440

gcacgtacaa cctccaggaa atcgtgcccg gcagcgtgtg gatggagagg gacgtgtacc 4500

tccaaggacc catctgggcc aagatcccag agacgggggc gcactttcac ccctctccgg 4560

ccatgggcgg attcggactc aaacacccac cgcccatgat gctcatcaag aacacgcctg 4620

tgcccggaaa tatcaccagc ttcgcggacg tgcccgtcag cgccttcatc acccagtaca 4680

gcaccgggca ggtcaccgtg gagatggagt gggagctcaa gaaggaaaac tccaagaggt 4740

ggaacccaga gatccagtac acaaacaact acaacgaccc ccagtttgtg gactttgccc 4800

cggacagcac cggggaatac agaaccacca gacctatcgg aacccgatac cttacccgac 4860

ccctttaacc cattcatgtc gcataccctc aataaaccgt gtattcgtgt cagtaaaata 4920

ctgcctcttg tggtcatttc tagaactaga actagtggat cccccgggct gcaggaattc 4980

gatatcaagc ttatcgatac cgtcgacctc gagggggggc ccggtaccca gcttttgttc 5040

cctttagtga gggttaattt cgagcttggc gtaatcatgg tcatagctgt ttcctgtgtg 5100

aaattgttat ccgctcacaa ttccacacaa catacgagcc ggaagcataa agtgtaaagc 5160

ctggggtgcc taatgagtga gctaactcac attaattgcg ttgcgctcac tgcccgcttt 5220

ccagtcggga aacctgtcgt gccagctgca ttaatgaatc ggccaacgcg cggggagagg 5280

cggtttgcgt attgggcgct cttccgcttc ctcgctcact gactcgctgc gctcggtcgt 5340

tcggctgcgg cgagcggtat cagctcactc aaaggcggta atacggttat ccacagaatc 5400

aggggataac gcaggaaaga acatgtgagc aaaaggccag caaaaggcca ggaaccgtaa 5460

aaaggccgcg ttgctggcgt ttttccatag gctccgcccc cctgacgagc atcacaaaaa 5520

tcgacgctca agtcagaggt ggcgaaaccc gacaggacta taaagatacc aggcgtttcc 5580

ccctggaagc tccctcgtgc gctctcctgt tccgaccctg ccgcttaccg gatacctgtc 5640

cgcctttctc ccttcgggaa gcgtggcgct ttctcatagc tcacgctgta ggtatctcag 5700

ttcggtgtag gtcgttcgct ccaagctggg ctgtgtgcac gaaccccccg ttcagcccga 5760

ccgctgcgcc ttatccggta actatcgtct tgagtccaac ccggtaagac acgacttatc 5820

gccactggca gcagccactg gtaacaggat tagcagagcg aggtatgtag gcggtgctac 5880

agagttcttg aagtggtggc ctaactacgg ctacactaga aggacagtat ttggtatctg 5940

cgctctgctg aagccagtta ccttcggaaa aagagttggt agctcttgat ccggcaaaca 6000

aaccaccgct ggtagcggtg gtttttttgt ttgcaagcag cagattacgc gcagaaaaaa 6060

aggatctcaa gaagatcctt tgatcttttc tacggggtct gacgctcagt ggaacgaaaa 6120

ctcacgttaa gggattttgg tcatgagatt atcaaaaagg atcttcacct agatcctttt 6180

aaattaaaaa tgaagtttta aatcaatcta aagtatatat gagtaaactt ggtctgacag 6240

ttaccaatgc ttaatcagtg aggcacctat ctcagcgatc tgtctatttc gttcatccat 6300

agttgcctga ctccccgtcg tgtagataac tacgatacgg gagggcttac catctggccc 6360

cagtgctgca atgataccgc gagacccacg ctcaccggct ccagatttat cagcaataaa 6420

ccagccagcc ggaagggccg agcgcagaag tggtcctgca actttatccg cctccatcca 6480

gtctattaat tgttgccggg aagctagagt aagtagttcg ccagttaata gtttgcgcaa 6540

cgttgttgcc attgctacag gcatcgtggt gtcacgctcg tcgtttggta tggcttcatt 6600

cagctccggt tcccaacgat caaggcgagt tacatgatcc cccatgttgt gcaaaaaagc 6660

ggttagctcc ttcggtcctc cgatcgttgt cagaagtaag ttggccgcag tgttatcact 6720

catggttatg gcagcactgc ataattctct tactgtcatg ccatccgtaa gatgcttttc 6780

tgtgactggt gagtactcaa ccaagtcatt ctgagaatag tgtatgcggc gaccgagttg 6840

ctcttgcccg gcgtcaatac gggataatac cgcgccacat agcagaactt taaaagtgct 6900

catcattgga aaacgttctt cggggcgaaa actctcaagg atcttaccgc tgttgagatc 6960

cagttcgatg taacccactc gtgcacccaa ctgatcttca gcatctttta ctttcaccag 7020

cgtttctggg tgagcaaaaa caggaaggca aaatgccgca aaaaagggaa taagggcgac 7080

acggaaatgt tgaatactca tactcttcct ttttcaatat tattgaagca tttatcaggg 7140

ttattgtctc atgagcggat acatatttga atgtatttag aaaaataaac aaataggggt 7200

tccgcgcaca tttccccgaa aagtgc 7226

<210> 154
<211> 8179
<212> DNA
<213> Adeno-Associated Virus

<220>
<221> gene
<222> (1)..(8179)

<400> 154
aattcccatc atcaataata taccttattt aggattgaag ccaatatgat aatgagccgg 60

tggagtttgt gacgtggcgc ggggcgtggg aacggggcgg gtgacgtagt agtctctaga 120

gtcctgtatt agaggtcacg tgagtgtttt gcgacttttt gcgacaccat gtggtcacgc 180

tgggtattta agcccgagtg agcacgcagg gtctccattt tgaagcggga ggtttgaacg 240

cgcagccacc acgggggggt tttacgagat tgtgattaag gtccccagcg accttgacgg 300

gcatctgccc ggcatttctg acagctttgt gaactgggtg gccgagaagg aatgggagtt 360

gccgccagat tctgacatgg atctgaatct gattgagcag gcacccctga ccgtggccga 420

gaagctgcag cgcgactttc tgacggaatg gcgccgtgtg agtaaggccc cggaggccct 480

tttctttgtg caatttgaga agggagagag ctacttccac atgcacgtgc tcgtggaaac 540

caccggggtg aaatccatgg ttttgggacg tttcctgagt cagattcgcg aaaaactgat 600

tcagagaatt taccgcggga tcgagccgac tttgccaaac tggttcgcgg tcacaaagac 660

cagaaatggc gccggaggcg ggaacaaggt ggtggatgag tgctacatcc ccaattactt 720

gctccccaaa acccagcctg agctccagtg ggcgtggact aatatggaac agtatttaag 780

cgcctgtttg aatctcacgg agcgtaaacg gttggtggcg cagcatctga cgcacgtgtc 840

gcagacgcag gagcagaaca aagagaatca gaatcccaat tctgatgcgc cggtgatcag 900

atcaaaaact tcagccaggt acatggagct ggtcgggtgg ctcgtggaca aggggattac 960

ctcggagaag cagtggatcc aggaggacca ggcctcatac atctccttca atgcggcctc 1020

caactcgcgg tcccaaatca aggctgcctt ggacaatgcg ggaaagatta tgagcctgac 1080

taaaaccgcc cccgactacc tggtgggcca gcagcccgtg gaggacattt ccagcaatcg 1140

gatttataaa attttggaac taaacgggta cgatccccaa tatgcggctt ccgtctttct 1200

gggatgggcc acgaaaaagt tcggcaagag gaacaccatc tggctgtttg ggcctgcaac 1260

taccgggaag accaacatcg cggaggccat agcccacact gtgcccttct acgggtgcgt 1320

aaactggacc aatgagaact ttcccttcaa cgactgtgtc gacaagatgg tgatctggtg 1380

ggaggagggg aagatgaccg ccaaggtcgt ggagtcggcc aaagccattc tcggaggaag 1440

caaggtgcgc gtggaccaga aatgcaagtc ctcggcccag atagacccga ctcccgtgat 1500

cgtcacctcc aacaccaaca tgtgcgccgt gattgacggg aactcaacga ccttcgaaca 1560

ccagcagccg ttgcaagacc ggatgttcaa atttgaactc acccgccgtc tggatcatga 1620

ctttgggaag gtcaccaagc aggaagtcaa agactttttc cggtgggcaa aggatcacgt 1680

ggttgaggtg gagcatgaat tctacgtcaa aaagggtgga gccaagaaaa gacccgcccc 1740

cagtgacgca gatataagtg agcccaaacg ggtgcgcgag tcagttgcgc agccatcgac 1800

gtcagacgcg gaagcttcga tcaactacgc agacaggtac caaaacaaat gttctcgtca 1860

cgtgggcatg aatctgatgc tgtttccctg cagacaatgc gagagaatga atcagaattc 1920

aaatatctgc ttcactcacg gacagaaaga ctgtttagag tgctttcccg tgtcagaatc 1980

tcaacccgtt tctgtcgtca aaaaggcgta tcagaaactg tgctacattc atcatatcat 2040

gggaaaggtg ccagacgctt gcactgcctg cgatctggtc aatgtggatt tggatgactg 2100

catctttgaa caataaatga tttaaatcag gtatggctgc cgatggttat cttccagatt 2160

ggctcgagga caacctctct gagggcattc gcgagtggtg ggacttgaaa cctggagccc 2220

cgaaacccaa agccaaccag caaaagcagg acgacggccg gggtctggtg cttcctggct 2280

acaagtacct cggacccttc aacggactcg acaaggggga gcccgtcaac gcggcggatg 2340

cagcggccct cgagcacgac aaggcctacg accagcagct caaagcgggt gacaatccgt 2400

acctgcggta taaccacgcc gacgccgagt ttcaggagcg tctgcaagaa gatacgtctt 2460

ttgggggcaa cctcgggcga gcagtcttcc aggccaagaa gagggttctc gaaccttttg 2520

gtctggttga ggaaggtgct aggacggctc ctggaaagaa acgtccggta gagcagtcgc 2580

cacaagagcc agactcctcc tcgggcattg gcaagacagg ccagcagccc gctaaaaaga 2640

gactcaattt tggtcagact ggcgactcag agtcagtccc cgacccacaa cctctcggag 2700

aacctccagc aacccccgct gctgtgggac ctactacaat ggcttcaggc ggtggcgcac 2760

caatggcaga caataacgaa ggcgccgacg gagtgggtaa tgcctcagga aattggcatt 2820

gcgattccac atggctgggc gacagagtca tcaccaccag cacccgaaca tgggccttgc 2880

ccgcctataa caaccacctc tacaagcaaa tctccagtgc ttcaacgggg gccagcaacg 2940

acaaccacta cttcggctac agcaccccct gggggtattt tgatttcaac agattccact 3000

gccatttctc accacgtgac tggcagcgac tcatcaacaa caattgggga ttccggccca 3060

agagactcaa cttcaagctc ttcaacatcc aagtcaagga ggtcacgacg aatgatggcg 3120

tcacgaccat cgctaataac cttaccagca cggttcaagt cttctcggac tcggagtacc 3180

agttgccgta cgtcctcggc tctgcgcacc agggctgcct ccctccgttc ccggcggacg 3240

tgttcatgat tccgcagtac ggctacctaa cgctcaacaa tggcagccag gcagtgggac 3300

ggtcatcctt ttactgcctg gaatatttcc catcgcagat gctgagaacg ggcaataact 3360

ttaccttcag ctacaccttc gaggacgtgc ctttccacag cagctacgcg cacagccaga 3420

gcctggaccg gctgatgaat cctctcatcg accagtacct gtattacctg aacagaactc 3480

agaatcagtc cggaagtgcc caaaacaagg acttgctgtt tagccggggg tctccagctg 3540

gcatgtctgt tcagcccaaa aactggctac ctggaccctg ttaccggcag cagcgcgttg 3600

ctaaaacaaa aacagacaac aacaacagca actttacctg gactggtgct tcaaaatata 3660

accttaatgg gcgtgaatct ataatcaacc ctggcactgc tatggcctca cacaaagacg 3720

acaaagacaa gttctttccc atgagcggtg tcatgatttt tggaaaggag agcgccggag 3780

cttcaaacac tgcattggac aatgtcatga tcacagacga agaggaaatc aaagccacta 3840

accccgtggc caccgaaaga tttgggactg tggcagtcaa tctccagagc agcagcacag 3900

accctgcgac cggagatgtg catgttatgg gagccttacc tggaatggtg tggcaagaca 3960

gagacgtata cctgcagggt cctatttggg ccaaaattcc tcacacggat ggacactttc 4020

acccgtctcc tctcatgggc ggctttggac ttaagcaccc gcctcctcag atcctcatca 4080

aaaacacgcc tgttcctgcg aatcctccgg cagagttttc ggctacaaag tttgctgcat 4140

tcatcaccca gtattccaca ggacaagtga gcgtggagat tgaatgggag ctgcagaaag 4200

aaaacagcaa acgctggaat cccgaagtgc agtatacatc taactatgca aaatctgcca 4260

acgttgattt cactgtggac aacaatggac tttatactga gcctcgcccc attggcaccc 4320

gttacctcac ccgtcccctg taattgcttg ttaatcaata aaccgtttaa ttcgtttcag 4380

ttgaactttg gtctctgcgt atttctttct tatctagttt ccatgctcta gactactacg 4440

tcacccgccc cgttcccacg ccccgcgcca cgtcacaaac tccaccccct cattatcata 4500

ttggcttcaa tccaaaataa ggtatattat tgatgatgca tcgctggcgt aatagcgaag 4560

aggcccgcac cgatcgccct tcccaacagt tgcgcagcct gaatggcgaa tggaattcca 4620

gacgattgag cgtcaaaatg taggtatttc catgagcgtt tttcctgttg caatggctgg 4680

cggtaatatt gttctggata ttaccagcaa ggccgatagt ttgagttctt ctactcaggc 4740

aagtgatgtt attactaatc aaagaagtat tgcgacaacg gttaatttgc gtgatggaca 4800

gactctttta ctcggtggcc tcactgatta taaaaacact tctcaggatt ctggcgtacc 4860

gttcctgtct aaaatccctt taatcggcct cctgtttagc tcccgctctg attctaacga 4920

ggaaagcacg ttatacgtgc tcgtcaaagc aaccatagta cgcgccctgt agcggcgcat 4980

taagcgcggc gggtgtggtg gttacgcgca gcgtgaccgc tacacttgcc agcgccctag 5040

cgcccgctcc tttcgctttc ttcccttcct ttctcgccac gttcgccggc tttccccgtc 5100

aagctctaaa tcgggggctc cctttagggt tccgatttag tgctttacgg cacctcgacc 5160

ccaaaaaact tgattagggt gatggttcac gtagtgggcc atcgccctga tagacggttt 5220

ttcgcccttt gacgttggag tccacgttct ttaatagtgg actcttgttc caaactggaa 5280

caacactcaa ccctatctcg gtctattctt ttgatttata agggattttg ccgatttcgg 5340

cctattggtt aaaaaatgag ctgatttaac aaaaatttaa cgcgaatttt aacaaaatat 5400

taacgtttac aatttaaata tttgcttata caatcttcct gtttttgggg cttttctgat 5460

tatcaaccgg ggtacatatg attgacatgc tagttttacg attaccgttc atcgattctc 5520

ttgtttgctc cagactctca ggcaatgacc tgatagcctt tgtagagacc tctcaaaaat 5580

agctaccctc tccggcatga atttatcagc tagaacggtt gaatatcata ttgatggtga 5640

tttgactgtc tccggccttt ctcacccgtt tgaatcttta cctacacatt actcaggcat 5700

tgcatttaaa atatatgagg gttctaaaaa tttttatcct tgcgttgaaa taaaggcttc 5760

tcccgcaaaa gtattacagg gtcataatgt ttttggtaca accgatttag ctttatgctc 5820

tgaggcttta ttgcttaatt ttgctaattc tttgccttgc ctgtatgatt tattggatgt 5880

tggaattcct gatgcggtat tttctcctta cgcatctgtg cggtatttca caccgcatat 5940

ggtgcactct cagtacaatc tgctctgatg ccgcatagtt aagccagccc cgacacccgc 6000

caacacccgc tgacgcgccc tgacgggctt gtctgctccc ggcatccgct tacagacaag 6060

ctgtgaccgt ctccgggagc tgcatgtgtc agaggttttc accgtcatca ccgaaacgcg 6120

cgagacgaaa gggcctcgtg atacgcctat ttttataggt taatgtcatg ataataatgg 6180

tttcttagac gtcaggtggc acttttcggg gaaatgtgcg cggaacccct atttgtttat 6240
ttttctaaat acattcaaat atgtatccgc tcatgagaca ataaccctga taaatgcttc 6300
aataatattg aaaaaggaag agtatgagta ttcaacattt ccgtgtcgcc cttattccct 6360
tttttgcggc attttgcctt cctgtttttg ctcacccaga aacgctggtg aaagtaaaag 6420
atgctgaaga tcagttgggt gcacgagtgg gttacatcga actggatctc aacagcggta 6480
agatccttga gagttttcgc cccgaagaac gttttccaat gatgagcact tttaaagttc 6540
tgctatgtgg cgcggtatta tcccgtattg acgccgggca agagcaactc ggtcgccgca 6600
tacactattc tcagaatgac ttggttgagt actcaccagt cacagaaaag catcttacgg 6660
atggcatgac agtaagagaa ttatgcagtg ctgccataac catgagtgat aacactgcgg 6720
ccaacttact tctgacaacg atcggaggac cgaaggagct aaccgctttt ttgcacaaca 6780
tgggggatca tgtaactcgc cttgatcgtt gggaaccgga gctgaatgaa gccataccaa 6840
acgacgagcg tgacaccacg atgcctgtag caatggcaac aacgttgcgc aaactattaa 6900
ctggcgaact acttactcta gcttcccggc aacaattaat agactggatg gaggcggata 6960
aagttgcagg accacttctg cgctcggccc ttccggctgg ctggtttatt gctgataaat 7020
ctggagccgg tgagcgtggg tctcgcggta tcattgcagc actggggcca gatggtaagc 7080
cctcccgtat cgtagttatc tacacgacgg ggagtcaggc aactatggat gaacgaaata 7140
gacagatcgc tgagataggt gcctcactga ttaagcattg gtaactgtca gaccaagttt 7200
actcatatat actttagatt gatttaaaac ttcattttta atttaaaagg atctaggtga 7260
agatcctttt tgataatctc atgaccaaaa tcccttaacg tgagttttcg ttccactgag 7320
cgtcagaccc cgtagaaaag atcaaaggat cttcttgaga tccttttttt ctgcgcgtaa 7380
tctgctgctt gcaaacaaaa aaaccaccgc taccagcggt ggtttgtttg ccggatcaag 7440
agctaccaac tctttttccg aaggtaactg gcttcagcag agcgcagata ccaaatactg 7500
tccttctagt gtagccgtag ttaggccacc acttcaagaa ctctgtagca ccgcctacat 7560
acctcgctct gctaatcctg ttaccagtgg ctgctgccag tggcgataag tcgtgtctta 7620
ccgggttgga ctcaagacga tagttaccgg ataaggcgca gcggtcgggc tgaacggggg 7680
gttcgtgcac acagcccagc ttggagcgaa cgacctacac cgaactgaga tacctacagc 7740
gtgagctatg agaaagcgcc acgcttcccg aagggagaaa ggcggacagg tatccggtaa 7800
gcggcagggt cggaacagga gagcgcacga gggagcttcc agggggaaac gcctggtatc 7860
tttatagtcc tgtcgggttt cgccacctct gacttgagcg tcgatttttg tgatgctcgt 7920
caggggggcg gagcctatgg aaaaacgcca gcaacgcggc ctttttacgg ttcctggcct 7980
tttgctggcc ttttgctcac atgttctttc ctgcgttatc ccctgattct gtggataacc 8040
gtattaccgc ctttgagtga gctgataccg ctcgccgcag ccgaacgacc gagcgcagcg 8100

agtcagtgag cgaggaagcg gaagagcgcc caatacgcaa accgcctctc cccgcgcgtt 8160

ggccgattca ttaatgcag 8179

<210> 155
<211> 8182
<212> DNA
<213> Adeno-Associated Virus

<220>
<221> gene
<222> (1)..(8182)

<400> 155
aattcccatc atcaataata taccttattt aggattgaag ccaatatgat aatgagccgg 60

tggagtttgt gacgtggcgc ggggcgtggg aacggggcgg gtgacgtagt agtctctaga 120

gtcctgtatt agaggtcacg tgagtgtttt gcgacttttt gcgacaccat gtggtcacgc 180

tgggtattta agcccgagtg agcacgcagg gtctccattt tgaagcggga ggtttgaacg 240

cgcagccacc acgggggggt tttacgagat tgtgattaag gtccccagcg accttgacgg 300

gcatctgccc ggcatttctg acagctttgt gaactgggtg gccgagaagg aatgggagtt 360

gccgccagat tctgacatgg atctgaatct gattgagcag gcacccctga ccgtggccga 420

gaagctgcag cgcgactttc tgacggaatg gcgccgtgtg agtaaggccc cggaggccct 480

tttctttgtg caatttgaga agggagagag ctacttccac atgcacgtgc tcgtggaaac 540

caccggggtg aaatccatgg ttttgggacg tttcctgagt cagattcgcg aaaaactgat 600

tcagagaatt taccgcggga tcgagccgac tttgccaaac tggttcgcgg tcacaaagac 660

cagaaatggc gccggaggcg ggaacaaggt ggtggatgag tgctacatcc ccaattactt 720

gctccccaaa acccagcctg agctccagtg ggcgtggact aatatggaac agtatttaag 780

cgcctgtttg aatctcacgg agcgtaaacg gttggtggcg cagcatctga cgcacgtgtc 840

gcagacgcag gagcagaaca aagagaatca gaatcccaat tctgatgcgc cggtgatcag 900

atcaaaaact tcagccaggt acatggagct ggtcgggtgg ctcgtggaca aggggattac 960

ctcggagaag cagtggatcc aggaggacca ggcctcatac atctccttca atgcggcctc 1020

caactcgcgg tcccaaatca aggctgcctt ggacaatgcg ggaaagatta tgagcctgac 1080

taaaaccgcc cccgactacc tggtgggcca gcagcccgtg gaggacattt ccagcaatcg 1140

gatttataaa attttggaac taaacgggta cgatccccaa tatgcggctt ccgtctttct 1200

gggatgggcc acgaaaaagt tcggcaagag gaacaccatc tggctgtttg ggcctgcaac 1260

taccgggaag accaacatcg cggaggccat agcccacact gtgcccttct acgggtgcgt 1320

aaactggacc aatgagaact ttcccttcaa cgactgtgtc gacaagatgg tgatctggtg 1380

ggaggagggg aagatgaccg ccaaggtcgt ggagtcggcc aaagccattc tcggaggaag 1440

caaggtgcgc gtggaccaga aatgcaagtc ctcggcccag atagacccga ctcccgtgat 1500

cgtcacctcc aacaccaaca tgtgcgccgt gattgacggg aactcaacga ccttcgaaca 1560

ccagcagccg ttgcaagacc ggatgttcaa atttgaactc acccgccgtc tggatcatga 1620

ctttgggaag gtcaccaagc aggaagtcaa agactttttc cggtgggcaa aggatcacgt 1680

ggttgaggtg gagcatgaat tctacgtcaa aaagggtgga gccaagaaaa gacccgcccc 1740

cagtgacgca gatataagtg agcccaaacg ggtgcgcgag tcagttgcgc agccatcgac 1800

gtcagacgcg gaagcttcga tcaactacgc agacaggtac caaaacaaat gttctcgtca 1860

cgtgggcatg aatctgatgc tgtttccctg cagacaatgc gagagaatga atcagaattc 1920

aaatatctgc ttcactcacg gacagaaaga ctgtttagag tgctttcccg tgtcagaatc 1980

tcaacccgtt tctgtcgtca aaaaggcgta tcagaaactg tgctacattc atcatatcat 2040

gggaaaggtg ccagacgctt gcactgcctg cgatctggtc aatgtggatt tggatgactg 2100

catctttgaa caataaatga tttaaatcag gtatggctgc cgatggttat cttccagatt 2160

ggctcgagga caacctctct gagggcattc gcgagtggtg ggacctgaaa cctggagccc 2220

cgaaacccaa agccaaccag caaaagcagg acaacggccg gggtctggtg cttcctggct 2280

acaagtacct cggacccttc aacggactcg acaaggggga gcccgtcaac gcggcggacg 2340

cagcggccct cgagcacgac aaggcctacg accagcagct caaagcgggt gacaatccgt 2400

acctgcggta taaccacgcc gacgccgagt ttcaggagcg tctgcaagaa gatacgtcat 2460

ttgggggcaa cctcgggcga gcagtcttcc aggccaagaa gcgggttctc gaacctctcg 2520

gtctggttga ggaaggcgct aggacggctc ctgcaaagaa gagaccggta gagccgtcac 2580

ctcagcgttc ccccgactcc tccacgggca tcggcaagaa aggccagcag cccgccagaa 2640

agagactcaa tttcggtcag actggcgact cagagtcagt ccccgaccct caacctctcg 2700

gagaacctcc agcagcgccc tctagtgtgg gatctggtac agtggctgca ggcggtggcg 2760

caccaatggc agacaataac gaaggtgccg acggagtggg taatgcctca ggaaattggc 2820

attgcgattc cacatggctg ggcgacagag tcattaccac cagcacccga acctgggccc 2880

tgcccgccta caacaaccac ctctacaagc aaatctccag tgaaactgca ggtagtacca 2940

acgacaacac ctacttcggc tacagcaccc cctgggggta ttttgacttt aacagattcc 3000

actgccactt ctcaccacgt gactggcagc gactcatcaa caacaactgg ggattccggc 3060

ccaagaagct gcggttcaag ctcttcaaca tccaggtcaa ggaggtcacg acgaatgacg 3120

gcgttacgac catcgctaat aaccttacca gcacgattca ggtattctcg gactcggaat 3180

accagctgcc gtacgtcctc ggctctgcgc accagggctg cctgcctccg ttcccggcgg 3240

acgtcttcat gattcctcag tacggctacc tgactctcaa caatggcagt cagtctgtgg 3300

gacgttcctc cttctactgc ctggagtact tcccctctca gatgctgaga acgggcaaca 3360

actttgagtt cagctacagc ttcgaggacg tgcctttcca cagcagctac gcacacagcc 3420

agagcctgga ccggctgatg aatcccctca tcgaccagta cttgtactac ctggccagaa 3480

cacagagtaa cccaggaggc acagctggca atcgggaact gcagttttac cagggcgggc 3540

cttcaactat ggccgaacaa gccaagaatt ggttacctgg accttgcttc cggcaacaaa 3600

gagtcgccaa aacgctggat caaaacaaca acagcaactt tgcttggact ggtgccacca 3660

aatatcacct gaacggcaga aactcgttgg ttaatcccgg cgtcgccatg gcaactcaca 3720

aggacgacga ggaccgcttt ttcccatcca gcggagtcct gatttttgga aaaactggag 3780

caactaacaa aactacattg gaaaatgtgt taatgacaaa tgaagaagaa attcgtccta 3840

ctaatcctgt agccacggaa gaatacggga tagtcagcag caacttacaa gcggctaata 3900

ctgcagccca gacacaagtt gtcaacaacc agggagcctt acctggcatg gtctggcaga 3960

accgggacgt gtacctgcag ggtcccatct gggccaagat tcctcacacg gatggcaact 4020

ttcacccgtc tcctttgatg ggcggctttg gacttaaaca tccgcctcct cagatcctga 4080

tcaagaacac tcccgttccc gctaatcctc cggaggtgtt tactcctgcc aagtttgctg 4140

cgttcatcac acagtacagc accggacaag tcagcgtgga aatcgagtgg gagctgcaga 4200

aggaaaacag caagcgctgg aacccggaga ttcagtacac ctccaacttt gaaaagcaga 4260

ctggtgtgga ctttgccgtt gacagccagg gtgtttactc tgagcctcgc cctattggca 4320

ctcgttacct cacccgtaat ctgtaattgc ttgttaatca ataaaccgtt taattcgttt 4380

cagttgaact ttggtctctg cgtatttctt tcttatctag tttccatgct ctagactact 4440

acgtcacccg ccccgttccc acgccccgcg ccacgtcaca aactccaccc cctcattatc 4500

atattggctt caatccaaaa taaggtatat tattgatgat gcatcgctgg cgtaatagcg 4560

aagaggcccg caccgatcgc ccttcccaac agttgcgcag cctgaatggc gaatggaatt 4620

ccagacgatt gagcgtcaaa atgtaggtat ttccatgagc gtttttcctg ttgcaatggc 4680

tggcggtaat attgttctgg atattaccag caaggccgat agtttgagtt cttctactca 4740

ggcaagtgat gttattacta atcaaagaag tattgcgaca acggttaatt tgcgtgatgg 4800

acagactctt ttactcggtg gcctcactga ttataaaaac acttctcagg attctggcgt 4860

accgttcctg tctaaaatcc ctttaatcgg cctcctgttt agctcccgct ctgattctaa 4920

cgaggaaagc acgttatacg tgctcgtcaa agcaaccata gtacgcgccc tgtagcggcg 4980

cattaagcgc ggcgggtgtg gtggttacgc gcagcgtgac cgctacactt gccagcgccc 5040

tagcgcccgc tcctttcgct ttcttccctt cctttctcgc cacgttcgcc ggctttcccc 5100

gtcaagctct aaatcggggg ctccctttag ggttccgatt tagtgcttta cggcacctcg 5160

accccaaaaa acttgattag ggtgatggtt cacgtagtgg gccatcgccc tgatagacgg 5220

tttttcgccc tttgacgttg gagtccacgt tctttaatag tggactcttg ttccaaactg 5280

gaacaacact caaccctatc tcggtctatt cttttgattt ataagggatt ttgccgattt 5340

cggcctattg gttaaaaaat gagctgattt aacaaaaatt taacgcgaat tttaacaaaa 5400

tattaacgtt tacaatttaa atatttgctt atacaatctt cctgtttttg gggcttttct 5460

gattatcaac cggggtacat atgattgaca tgctagtttt acgattaccg ttcatcgatt 5520

ctcttgtttg ctccagactc tcaggcaatg acctgatagc ctttgtagag acctctcaaa 5580

aatagctacc ctctccggca tgaatttatc agctagaacg gttgaatatc atattgatgg 5640

tgatttgact gtctccggcc tttctcaccc gtttgaatct ttacctacac attactcagg 5700

cattgcattt aaaatatatg agggttctaa aaatttttat ccttgcgttg aaataaaggc 5760

ttctcccgca aaagtattac agggtcataa tgtttttggt acaaccgatt tagctttatg 5820

ctctgaggct ttattgctta attttgctaa ttctttgcct tgcctgtatg atttattgga 5880

tgttggaatt cctgatgcgg tattttctcc ttacgcatct gtgcggtatt tcacaccgca 5940

tatggtgcac tctcagtaca atctgctctg atgccgcata gttaagccag ccccgacacc 6000

cgccaacacc cgctgacgcg ccctgacggg cttgtctgct cccggcatcc gcttacagac 6060

aagctgtgac cgtctccggg agctgcatgt gtcagaggtt ttcaccgtca tcaccgaaac 6120

gcgcgagacg aaagggcctc gtgatacgcc tatttttata ggttaatgtc atgataataa 6180

tggtttctta gacgtcaggt ggcacttttc ggggaaatgt gcgcggaacc cctatttgtt 6240

tatttttcta aatacattca aatatgtatc cgctcatgag acaataaccc tgataaatgc 6300

ttcaataata ttgaaaaagg aagagtatga gtattcaaca tttccgtgtc gcccttattc 6360

ccttttttgc ggcattttgc cttcctgttt ttgctcaccc agaaacgctg gtgaaagtaa 6420

aagatgctga agatcagttg ggtgcacgag tgggttacat cgaactggat ctcaacagcg 6480

gtaagatcct tgagagtttt cgccccgaag aacgttttcc aatgatgagc acttttaaag 6540

ttctgctatg tggcgcggta ttatcccgta ttgacgccgg gcaagagcaa ctcggtcgcc 6600

gcatacacta ttctcagaat gacttggttg agtactcacc agtcacagaa aagcatctta 6660

cggatggcat gacagtaaga gaattatgca gtgctgccat aaccatgagt gataacactg 6720

cggccaactt acttctgaca acgatcggag gaccgaagga gctaaccgct tttttgcaca 6780

acatggggga tcatgtaact cgccttgatc gttgggaacc ggagctgaat gaagccatac 6840

caaacgacga gcgtgacacc acgatgcctg tagcaatggc aacaacgttg cgcaaactat 6900

taactggcga actacttact ctagcttccc ggcaacaatt aatagactgg atggaggcgg 6960

ataaagttgc aggaccactt ctgcgctcgg cccttccggc tggctggttt attgctgata 7020

aatctggagc cggtgagcgt gggtctcgcg gtatcattgc agcactgggg ccagatggta 7080

agccctcccg tatcgtagtt atctacacga cggggagtca ggcaactatg gatgaacgaa 7140

atagacagat cgctgagata ggtgcctcac tgattaagca ttggtaactg tcagaccaag 7200

tttactcata tatactttag attgatttaa aacttcattt ttaatttaaa aggatctagg 7260

tgaagatcct ttttgataat ctcatgacca aaatccctta acgtgagttt tcgttccact 7320

gagcgtcaga ccccgtagaa aagatcaaag gatcttcttg agatcctttt tttctgcgcg 7380

taatctgctg cttgcaaaca aaaaaaccac cgctaccagc ggtggtttgt ttgccggatc 7440

aagagctacc aactcttttt ccgaaggtaa ctggcttcag cagagcgcag ataccaaata 7500

ctgtccttct agtgtagccg tagttaggcc accacttcaa gaactctgta gcaccgccta 7560

catacctcgc tctgctaatc ctgttaccag tggctgctgc cagtggcgat aagtcgtgtc 7620

ttaccgggtt ggactcaaga cgatagttac cggataaggc gcagcggtcg ggctgaacgg 7680

ggggttcgtg cacacagccc agcttggagc gaacgaccta caccgaactg agatacctac 7740

agcgtgagct atgagaaagc gccacgcttc ccgaagggag aaaggcggac aggtatccgg 7800

taagcggcag ggtcggaaca ggagagcgca cgagggagct tccaggggga aacgcctggt 7860

atctttatag tcctgtcggg tttcgccacc tctgacttga gcgtcgattt ttgtgatgct 7920

cgtcaggggg gcggagccta tggaaaaacg ccagcaacgc ggccttttta cggttcctgg 7980

ccttttgctg gccttttgct cacatgttct ttcctgcgtt atcccctgat tctgtggata 8040

accgtattac cgcctttgag tgagctgata ccgctcgccg cagccgaacg accgagcgca 8100

gcgagtcagt gagcgaggaa gcggaagagc gcccaatacg caaaccgcct ctccccgcgc 8160

gttggccgat tcattaatgc ag 8182

<210> 156
<211> 8185
<212> DNA
<213> Adeno-Associated Virus

<220>
<221> gene
<222> (1)..(8185)

<400> 156
aattcccatc atcaataata taccttattt aggattgaag ccaatatgat aatgagccgg 60

tggagtttgt gacgtggcgc ggggcgtggg aacggggcgg gtgacgtagt agtctctaga 120

gtcctgtatt agaggtcacg tgagtgtttt gcgacttttt gcgacaccat gtggtcacgc 180

tgggtattta agcccgagtg agcacgcagg gtctccattt tgaagcggga ggtttgaacg 240

cgcagccacc acgggggggt tttacgagat tgtgattaag gtccccagcg accttgacgg 300

gcatctgccc ggcatttctg acagctttgt gaactgggtg gccgagaagg aatgggagtt 360

gccgccagat tctgacatgg atctgaatct gattgagcag gcacccctga ccgtggccga 420

gaagctgcag cgcgactttc tgacggaatg gcgccgtgtg agtaaggccc cggaggccct 480

tttctttgtg caatttgaga agggagagag ctacttccac atgcacgtgc tcgtggaaac 540

caccggggtg aaatccatgg ttttgggacg tttcctgagt cagattcgcg aaaaactgat 600

tcagagaatt taccgcggga tcgagccgac tttgccaaac tggttcgcgg tcacaaagac 660

cagaaatggc gccggaggcg ggaacaaggt ggtggatgag tgctacatcc ccaattactt 720

gctccccaaa acccagcctg agctccagtg ggcgtggact aatatggaac agtatttaag 780

cgcctgtttg aatctcacgg agcgtaaacg gttggtggcg cagcatctga cgcacgtgtc 840

gcagacgcag gagcagaaca aagagaatca gaatcccaat tctgatgcgc cggtgatcag 900

atcaaaaact tcagccaggt acatggagct ggtcgggtgg ctcgtggaca aggggattac 960

ctcggagaag cagtggatcc aggaggacca ggcctcatac atctccttca atgcggcctc 1020

caactcgcgg tcccaaatca aggctgcctt ggacaatgcg ggaaagatta tgagcctgac 1080

taaaaccgcc cccgactacc tggtgggcca gcagcccgtg gaggacattt ccagcaatcg 1140

gatttataaa attttggaac taaacgggta cgatccccaa tatgcggctt ccgtctttct 1200

gggatgggcc acgaaaaagt tcggcaagag gaacaccatc tggctgtttg ggcctgcaac 1260

taccgggaag accaacatcg cggaggccat agcccacact gtgcccttct acgggtgcgt 1320

aaactggacc aatgagaact ttcccttcaa cgactgtgtc gacaagatgg tgatctggtg 1380

ggaggagggg aagatgaccg ccaaggtcgt ggagtcggcc aaagccattc tcggaggaag 1440

caaggtgcgc gtggaccaga aatgcaagtc ctcggcccag atagacccga ctcccgtgat 1500

cgtcacctcc aacaccaaca tgtgcgccgt gattgacggg aactcaacga ccttcgaaca 1560

ccagcagccg ttgcaagacc ggatgttcaa atttgaactc acccgccgtc tggatcatga 1620

ctttgggaag gtcaccaagc aggaagtcaa agactttttc cggtgggcaa aggatcacgt 1680

ggttgaggtg gagcatgaat tctacgtcaa aaagggtgga gccaagaaaa gacccgcccc 1740

cagtgacgca gatataagtg agcccaaacg ggtgcgcgag tcagttgcgc agccatcgac 1800

gtcagacgcg gaagcttcga tcaactacgc agacaggtac caaaacaaat gttctcgtca 1860

cgtgggcatg aatctgatgc tgtttccctg cagacaatgc gagagaatga atcagaattc 1920

aaatatctgc ttcactcacg gacagaaaga ctgtttagag tgctttcccg tgtcagaatc 1980

tcaacccgtt tctgtcgtca aaaaggcgta tcagaaactg tgctacattc atcatatcat 2040

gggaaaggtg ccagacgctt gcactgcctg cgatctggtc aatgtggatt tggatgactg 2100

catctttgaa caataaatga tttaaatcag gtatggctgc cgatggttat cttccagatt 2160

ggctcgagga caacctctct gagggcattc gcgagtggtg ggcgctgaaa cctggagccc 2220

cgaagcccaa agccaaccag caaaagcagg acgacggccg gggtctggtg cttcctggct 2280

acaagtacct cggacccttc aacggactcg acaaggggga gcccgtcaac gcggcggacg 2340

cagcggccct ggagcacgac aaggcctacg accagcagct gcaggcgggt gacaatccgt 2400

acctgcggta taaccacgcc gacgccgagt ttcaggagcg tctgcaagaa gatacgtctt 2460

ttgggggcaa cctcgggcga gcagtcttcc aggccaagaa gcgggttctc gaacctctcg 2520

gtctggttga ggaaggggct agggcggctc ctggaaggag aagaccggta gagccagcac 2580

cccagcgtgc tccagacgcc tctacgggca tcggcaagaa aggccaacag cccgccagaa 2640

aaagactcaa ttttggtcag actggcgact cagagtcagt tccagaccct caacctctcg 2700

gagaacctcc agcagcgccc tctggtgtgg gacctaatac aatggctgca ggcggtggcg 2760

caccaatggc agacaataac gaaggcgccg acggagtggg tagttcctcg ggaaattggc 2820

attgcgattc cacatggctg ggcgacagag tcatcaccac cagcacccga acctgggccc 2880

tgccggccta caacaaccac ctctacaagc aaatctccaa cgggacatcg ggaggagcca 2940

ccaacgacaa cacctacttc ggctacgcca ccccctgggg gtattttgac tttaacagat 3000

tccactgcca cttttcacca cgtgactggc agcgactcat caacaacaac tggggattcc 3060

ggcccaagag actcagcttc aagctcttca acatccaggt caaggaggtc acgcagaatg 3120

aaggcaccaa gaccatcgcc aataacctca ccagcaccat ccaggtgttt acggactcgg 3180

agtaccagct gccgtacgtt ctcggctctg cccaccaggg ctgcctgcct ccgttcccgg 3240

cggacgtgtt catgattccc cagtacggct acctaacact caacaacggt agtcaggccg 3300

tgggacgctc ctccttctac tgcctggaat actttccttc gcagatgctg agaaccggca 3360

acaacttcca gtttacttac accttcgagg acgtgccttt ccacagcagc tacgcccaca 3420

gccagagctt ggaccggctg atgaatcctc tgattgacca gtacctgtac tacttgtctc 3480

ggactcaaac aacaggaggc acggcaaata cgcagactct gggcttcagc caaggtgggc 3540

ctaatacaat ggccaatcag gcaaagaact ggctgccagg accctgttac cgccaacaac 3600

gcgtctcaac gacaaccggg caaaacaaca atgccaactt tgcctggact gctgggacca 3660

aataccatct gaatggaaga aattcattgg ctaatcctgg catcgctatg gcaacacaca 3720

aagacgacga ggagcgtttt tttcccagta acgggatcct gatttttggc aaacaaaatg 3780

ctgccagaga caatgcggat tacagcgatg tcatgctcac cagcgaggaa gaaatcaaaa 3840

ccactaaccc tgtggctaca gaggaatacg gtatcgtggc agataacttg cagcagcaaa 3900

acacggctcc tcaaattgga actgtcaaca gccagggggc cttacccggt atggtctggc 3960

agaaccggga cgtgtacctg cagggtccca tctgggccaa gattcctcac acggacggca 4020

acttccaccc gtctccgctg atgggcggct ttggcctgaa acatcctccg cctcagatcc 4080

tgatccgaaa cgcgcctgta cctgcggatc ctccggctac cttcaaccag gcaaagctga 4140

acgctttcat cgcgcaatac agcaccggac aggtcagcgt ggaaattgaa tgggagctgc 4200

agaaggaaaa cagcaagcgc tggaaccccg agatccagta cacctccaac tactacaaat 4260

ctacaagtgt ggactttgct gttaatacag aaggcgtgta ctctgaaccc cgccccattg 4320

gcacccgtta cctcacccgt aatctgtaat tgcttgttaa tcaataaacc gtttaattcg 4380

tttcagttga actttggtct ctgcgtattt ctttcttatc tagtttccat gctctagact 4440

actacgtcac ccgccccgtt cccacgcccc gcgccacgtc acaaactcca ccccctcatt 4500

atcatattgg cttcaatcca aaataaggta tattattgat gatgcatcgc tggcgtaata 4560

gcgaagaggc ccgcaccgat cgcccttccc aacagttgcg cagcctgaat ggcgaatgga 4620

attccagacg attgagcgtc aaaatgtagg tatttccatg agcgtttttc ctgttgcaat 4680

ggctggcggt aatattgttc tggatattac cagcaaggcc gatagtttga gttcttctac 4740

tcaggcaagt gatgttatta ctaatcaaag aagtattgcg acaacggtta atttgcgtga 4800

tggacagact cttttactcg gtggcctcac tgattataaa aacacttctc aggattctgg 4860

cgtaccgttc ctgtctaaaa tccctttaat cggcctcctg tttagctccc gctctgattc 4920

taacgaggaa agcacgttat acgtgctcgt caaagcaacc atagtacgcg ccctgtagcg 4980

gcgcattaag cgcggcgggt gtggtggtta cgcgcagcgt gaccgctaca cttgccagcg 5040

ccctagcgcc cgctcctttc gctttcttcc cttcctttct cgccacgttc gccggctttc 5100

cccgtcaagc tctaaatcgg gggctccctt tagggttccg atttagtgct ttacggcacc 5160

tcgaccccaa aaaacttgat tagggtgatg gttcacgtag tgggccatcg ccctgataga 5220

cggtttttcg ccctttgacg ttggagtcca cgttctttaa tagtggactc ttgttccaaa 5280

ctggaacaac actcaaccct atctcggtct attcttttga tttataaggg attttgccga 5340

tttcggccta ttggttaaaa aatgagctga tttaacaaaa atttaacgcg aattttaaca 5400

aaatattaac gtttacaatt taaatatttg cttatacaat cttcctgttt ttggggcttt 5460

tctgattatc aaccggggta catatgattg acatgctagt tttacgatta ccgttcatcg 5520

attctcttgt ttgctccaga ctctcaggca atgacctgat agcctttgta gagacctctc 5580

aaaaatagct accctctccg gcatgaattt atcagctaga acggttgaat atcatattga 5640

tggtgatttg actgtctccg gcctttctca cccgtttgaa tctttaccta cacattactc 5700

aggcattgca tttaaaatat atgagggttc taaaaatttt tatccttgcg ttgaaataaa 5760

ggcttctccc gcaaaagtat tacagggtca taatgttttt ggtacaaccg atttagcttt 5820

atgctctgag gctttattgc ttaattttgc taattctttg ccttgcctgt atgatttatt 5880

ggatgttgga attcctgatg cggtattttc tccttacgca tctgtgcggt atttcacacc 5940

gcatatggtg cactctcagt acaatctgct ctgatgccgc atagttaagc cagccccgac 6000

acccgccaac acccgctgac gcgccctgac gggcttgtct gctcccggca tccgcttaca 6060

gacaagctgt gaccgtctcc gggagctgca tgtgtcagag gttttcaccg tcatcaccga 6120

aacgcgcgag acgaaagggc ctcgtgatac gcctattttt ataggttaat gtcatgataa 6180

taatggtttc ttagacgtca ggtggcactt ttcggggaaa tgtgcgcgga acccctattt 6240

gtttattttt ctaaatacat tcaaatatgt atccgctcat gagacaataa ccctgataaa 6300

tgcttcaata atattgaaaa aggaagagta tgagtattca acatttccgt gtcgccctta 6360

ttcccttttt tgcggcattt tgccttcctg tttttgctca cccagaaacg ctggtgaaag 6420

taaaagatgc tgaagatcag ttgggtgcac gagtgggtta catcgaactg gatctcaaca 6480

gcggtaagat ccttgagagt tttcgccccg aagaacgttt tccaatgatg agcactttta 6540

aagttctgct atgtggcgcg gtattatccc gtattgacgc cgggcaagag caactcggtc 6600

gccgcataca ctattctcag aatgacttgg ttgagtactc accagtcaca gaaaagcatc 6660

ttacggatgg catgacagta agagaattat gcagtgctgc cataaccatg agtgataaca 6720

ctgcggccaa cttacttctg acaacgatcg gaggaccgaa ggagctaacc gcttttttgc 6780

acaacatggg ggatcatgta actcgccttg atcgttggga accggagctg aatgaagcca 6840

taccaaacga cgagcgtgac accacgatgc ctgtagcaat ggcaacaacg ttgcgcaaac 6900

tattaactgg cgaactactt actctagctt cccggcaaca attaatagac tggatggagg 6960

cggataaagt tgcaggacca cttctgcgct cggcccttcc ggctggctgg tttattgctg 7020

ataaatctgg agccggtgag cgtgggtctc gcggtatcat tgcagcactg gggccagatg 7080

gtaagccctc ccgtatcgta gttatctaca cgacggggag tcaggcaact atggatgaac 7140

gaaatagaca gatcgctgag ataggtgcct cactgattaa gcattggtaa ctgtcagacc 7200

aagtttactc atatatactt tagattgatt taaaacttca tttttaattt aaaaggatct 7260

aggtgaagat cctttttgat aatctcatga ccaaaatccc ttaacgtgag ttttcgttcc 7320

actgagcgtc agaccccgta gaaaagatca aaggatcttc ttgagatcct ttttttctgc 7380

gcgtaatctg ctgcttgcaa acaaaaaaac caccgctacc agcggtggtt tgtttgccgg 7440

atcaagagct accaactctt tttccgaagg taactggctt cagcagagcg cagataccaa 7500

atactgtcct tctagtgtag ccgtagttag gccaccactt caagaactct gtagcaccgc 7560

ctacatacct cgctctgcta atcctgttac cagtggctgc tgccagtggc gataagtcgt 7620

gtcttaccgg gttggactca agacgatagt taccggataa ggcgcagcgg tcgggctgaa 7680

cggggggttc gtgcacacag cccagcttgg agcgaacgac ctacaccgaa ctgagatacc 7740

tacagcgtga gctatgagaa agcgccacgc ttcccgaagg gagaaaggcg gacaggtatc 7800

cggtaagcgg cagggtcgga acaggagagc gcacgaggga gcttccaggg ggaaacgcct 7860

ggtatcttta tagtcctgtc gggtttcgcc acctctgact tgagcgtcga tttttgtgat 7920

gctcgtcagg ggggcggagc ctatggaaaa acgccagcaa cgcggccttt ttacggttcc 7980

tggccttttg ctggcctttt gctcacatgt tctttcctgc gttatcccct gattctgtgg 8040

ataaccgtat taccgccttt gagtgagctg ataccgctcg ccgcagccga acgaccgagc 8100

gcagcgagtc agtgagcgag gaagcggaag agcgcccaat acgcaaaccg cctctccccg 8160

cgcgttggcc gattcattaa tgcag 8185

<210> 157
<211> 8262
<212> DNA
<213> Adeno-Associated Virus

<220>
<221> gene
<222> (1)..(8262)

<400> 157
acggcggggt tttacgagat tgtgattaag gtccccagcg accttgacgg gcatctgccc 60

ggcatttctg acagctttgt gaactgggtg gccgagaagg aatgggagtt gccgccagat 120

tctgacatgg atctgaatct gattgagcag gcacccctga ccgtggccga gaagctgcag 180

cgcgactttc tgacggaatg gcgccgtgtg agtaaggccc cggaggccct tttctttgtg 240

caatttgaga agggagagag ctacttccac atgcacgtgc tcgtggaaac caccggggtg 300

aaatccatgg ttttgggacg tttcctgagt cagattcgcg aaaaactgat tcagagaatt 360

taccgcggga tcgagccgac tttgccaaac tggttcgcgg tcacaaagac cagaaatggc 420

gccggaggcg ggaacaaggt ggtggatgag tgctacatcc ccaattactt gctccccaaa 480

acccagcctg agctccagtg ggcgtggact aatatggaac agtatttaag cgcctgtttg 540

aatctcacgg agcgtaaacg gttggtggcg cagcatctga cgcacgtgtc gcagacgcag 600

gagcagaaca aagagaatca gaatcccaat tctgatgcgc cggtgatcag atcaaaaact 660

tcagccaggt acatggagct ggtcgggtgg ctcgtggaca aggggattac ctcggagaag 720

cagtggatcc aggaggacca ggcctcatac atctccttca atgcggcctc caactcgcgg 780

tcccaaatca aggctgcctt ggacaatgcg ggaaagatta tgagcctgac taaaaccgcc 840

cccgactacc tggtgggcca gcagcccgtg gaggacattt ccagcaatcg gatttataaa 900

attttggaac taaacgggta cgatccccaa tatgcggctt ccgtctttct gggatgggcc 960

acgaaaaagt tcggcaagag gaacaccatc tggctgtttg ggcctgcaac taccgggaag 1020

accaacatcg cggaggccat agcccacact gtgcccttct acgggtgcgt aaactggacc 1080

aatgagaact ttcccttcaa cgactgtgtc gacaagatgg tgatctggtg ggaggagggg 1140

aagatgaccg ccaaggtcgt ggagtcggcc aaagccattc tcggaggaag caaggtgcgc 1200

gtggaccaga aatgcaagtc ctcggcccag atagacccga ctcccgtgat cgtcacctcc 1260

aacaccaaca tgtgcgccgt gattgacggg aactcaacga ccttcgaaca ccagcagccg 1320

ttgcaagacc ggatgttcaa atttgaactc acccgccgtc tggatcatga ctttgggaag 1380

gtcaccaagc aggaagtcaa agactttttc cggtgggcaa aggatcacgt ggttgaggtg 1440

gagcatgaat tctacgtcaa aaagggtgga gccaagaaaa gacccgcccc cagtgacgca 1500

gatataagtg agcccaaacg ggtgcgcgag tcagttgcgc agccatcgac gtcagacgcg 1560

gaagcttcga tcaactacgc agacaggtac caaaacaaat gttctcgtca cgtgggcatg 1620

aatctgatgc tgtttccctg cagacaatgc gagagaatga atcagaattc aaatatctgc 1680

ttcactcacg gacagaaaga ctgtttagag tgctttcccg tgtcagaatc tcaacccgtt 1740

tctgtcgtca aaaaggcgta tcagaaactg tgctacattc atcatatcat gggaaaggtg 1800

ccagacgctt gcactgcctg cgatctggtc aatgtggatt tggatgactg catctttgaa 1860

caataaatga tttaaatcag gtatggctgc cgatggttat cttccagatt ggctcgagga 1920

caaccttagt gaaggaattc gcgagtggtg ggctttgaaa cctggagccc ctcaacccaa 1980

ggcaaatcaa caacatcaag acaacgctcg aggtcttgtg cttccgggtt acaaatacct 2040

tggacccggc aacggactcg acaaggggga gccggtcaac gcagcagacg cggcggccct 2100

cgagcacgac aaggcctacg accagcagct caaggccgga gacaacccgt acctcaagta 2160

caaccacgcc gacgccgagt tccaggagcg gctcaaagaa gatacgtctt ttgggggcaa 2220

cctcgggcga gcagtcttcc aggccaaaaa gaggcttctt gaacctcttg gtctggttga 2280

ggaagcggct aagacggctc ctggaaagag aaggcctgta gagcagtctc ctcaggaacc 2340

ggactcctcc gcgggtattg gcaaatcggg tgcacagccc gctaaaaaga gactcaattt 2400

cggtcagact ggcgacacag agtcagtccc agaccctcaa ccaatcggag aacctcccgc 2460

agccccctca ggtgtgggat ctcttacaat ggcttcaggt ggtggcgcac cagtggcaga 2520

caataacgaa ggtgccgatg gagtgggtag ttcctcggga aattggcatt gcgattccca 2580

atggctgggg gacagagtca tcaccaccag cacccgaacc tgggccctgc ccgcctacaa 2640

caatcacctc tacaagcaaa tctccaacag cacatctgga ggatcttcaa atgacaacgc 2700

ctacttcggc tacgcaaccc cctgggggta ttttgacttc aacagattcc actgccactt 2760

ctcaccacgt gactggcagc gactcatcaa caacaactgg ggattccggc ctaagcgact 2820

caacttcaag ctcttcaaca ttcaggtcaa agaggttacg gacaacaatg gagtcaagac 2880

catcgccaat aaccttacca gcacggtcca ggtcttcacg gactcagact atcagctccc 2940

gtacgtgctc gggtcggctc acgagggctg cctcccgccg ttcccagcgg acgttttcat 3000

gattcctcag tacgggtatc tgacgcttaa tgatggaagc caggccgtgg gtcgttcgtc 3060

cttttactgc ctggaatatt tcccgtcgca aatgctaaga acgggtaaca acttccagtt 3120

cagctacgag tttgagaacg tacctttcca tagcagctac gctcacagcc aaagcctgga 3180

ccgactaatg aatccactca tcgaccaata cttgtactat ctctcaaaga ctattaacgg 3240

ttctggacag aatcaacaaa cgctaaaatt cagtgtggcc ggacccagca acatggctgt 3300

ccagggaaga aactacatac ctggacccag ctaccgacaa caacgtgtcg cgaccactgt 3360

gactcaaaac aacaacgccg aatttgcttg gcctggagct tcttcttggg ctctcaatgg 3420

acgtaatagc ttgatgaatc ctggacctgc tatggccagc cacaaagaag gagaggaccg 3480

tttctttcct ttgtctggat ctttaatttt tggcaaacaa ggaactggaa gagacaacgt 3540

ggatgcggac aaagtcatga taaccaacga agaagaaatt aaaactacta acccggtagc 3600

aacggagtcc tatggacaag tggccacaaa ccaccagagt gcccaagcac aggcgcagac 3660

cggctgggtt caaaaccaag gaatacttcc gggtatggtt tggcaggaca gagatgtgta 3720

cctgcaagga cccatttggg ccaaaattcc tcacacggac ggcaactttc acccttctcc 3780

gctgatggga gggtttggaa tgaagcaccc gcctcctcag atcctcatca aaaacacacc 3840

tgtacctgcg gatcctccaa cggccttcaa caaggacaag ctgaacgcct tcatcaccca 3900

gtattctact ggccaagtca gcgtggagat cgagtgggag ctgcagaagg aaaacagcaa 3960

gcgctggaac ccggagatcc agtacacttc caactattac aagtctaata atgttgaatt 4020

tgctgttaat actgaaggtg tatatagtga accccgcccc attggcacca gatacctgac 4080

tcgtaatctg taattgcttg tttcggatct caacggcctt cacaggtaca agtgaacgcc 4140

tttatcaccc agtattctac tggaaggttt ttcctcgagt gggagctgca gaaggaaaaa 4200

agggcgctcc gaacccggag atccagtaca cttccaacta ttacaagtct aataatgttg 4260

aatttgttgt taatactgaa ggtgtatata gtgaaccccg ccccattggc accagatacc 4320

tgactcgtaa tctgtaattg cttgttaatc aataaaccgt ttaattcgtt tcagttgaac 4380

tttggtctct gcgaagggcg aattcgttta aacctgcagg actagaggtc ctgtattaga 4440

ggtcacgtga gtgttttgcg acattttgcg acaccaagag gtcacgctgg gtatttaagc 4500

ccgagtgagc acgcagggtc tccattttga agcgggaggt ttgaacgcgc agccgccaag 4560

ccgaattctg cagatatcca tcacactggc ggaaatgggg aagtaaagag ctgagatcag 4620

ttgggtgcac gagtgggtta catcgaactg gatctcaaca gcggtaagat ccttgagagt 4680

tttcgccccg aagaacgttt tccaatgatg agcactttta aagttctgct atgtggcgcg 4740

gtattatccc gtattgacgc cgggcaagag caactcggtc gccgcataca ctattctcag 4800

aatgacttgg ttgagtactc accagtcaca gaaaagcatc ttacggatgg catgacagta 4860

agagaattat gcagtgctgc cataaccatg agtgataaca ctgcggccaa cttacttctg 4920

acaacgatcg gaggaccgaa ggagctaacc gcttttttgc acaacatggg ggatcatgta 4980

actcgccttg atcgttggga accggagctg aatgaagcca taccaaacga cgagcgtgac 5040

accacgatgc ctgtagcaat ggcaacaacg ttgcgcaaac tattaaaagt ccggcaacta 5100

tggatgacga aatagaacga tcgctgaaat agtgcctcac tgatagcatg gtaactgtcg 5160

accagtttac ctcatatata cttagatgat taactcattt atcaagattc tgtaagtgaa 5220

gaatccgctt ttgatatctc atgacaattc cttaacgtga tttcgtccaa ctgaacgctc 5280

gcatctcgta agatcaggac tctctgaatc tttcagcgat tcgcgttgca ccgtacagac 5340

gggtagtcag tgcatggcac acgttgcgca actattactg gcgaactact tactctagct 5400

tcccggcaac aattaataga ctggatggag gcggataaag ttgcaggacc acttctgcgc 5460

tcggcccttc cggctggctg gtttattgct gataaatctg gagccggtga gcgtgggtct 5520

cgcggtatca ttgcagcact ggggccagat ggtaagcccc tcccgtatcg tagttatcta 5580

cacgacgggg gagtcagggc actatggatg gaccaaatag acagatcccc gaataggtgc 5640

ctcctgatta gcttggtaat ggccgaccag ttactctata tacttttatt gggcgtctca 5700

cgtcagaccc gtagaagatc aaggatcttc ttgagatcct ttttttctgc gcgtaatctg 5760

ctgcttgcaa acaaaaaaac caccgctacc agcggtggtt tgtttgccgg atcaagagct 5820

accaactctt tttccgaagg taactggctt cagcagagcg cagataccaa atactgttct 5880

tctagtgtag ccgtagttag gccaccactt caagaactct gtagcaccgc ctacatacct 5940

cgctctgcta atcctgttac cagtggctgc tgccagtggc gataagtcgt gtcttaccgg 6000

gttggactca agacgatagt taccggataa ggcgcagcgg tcgggctgaa cggggggttc 6060

gtgcacacag ccccagcttg gagcgaacgt cctaacaccg aatctgagat tacctacagc 6120

gtgagttaca cgtgacttga tagcgccacg cttccgaggg agaaaggcgg acaggtatcc 6180

ggtaagcggc agggtcggaa caggagagcg cacgagggag cttccagggg gaaacgcctg 6240

gtatctttat agtcctgtcg ggtttcgcca cctctgactt gagcgtcgat ttttgtgatg 6300

ctcgtcaggg gggcggagcc tatggaaaaa cgccagcaac gcggcctttt tacggttcct 6360

ggcctttttg atggccttgg gctacgattc cgactggaag cgggcagtga gcgcaacgca 6420

attaatgtga gttagctcac tcattaggca ccccaggctt tacactttat gcttccggct 6480

cgtatgttgt gtggaattgt gagcggataa caatttcaca caggaaacag ctatgaccat 6540

gattacgcca agcgcgcaat taaccctcac taaagggaac aaaagctgga gctccaccgc 6600

ggtggaaacc ttataacaaa aagaagggcg ggggtggcct cactgattaa aataacactt 6660

tctcagacat tcaggcgtac cgttcctgtc taaaatctct ttaatcggcc tcctgtttaa 6720

ctcccgctct gattctaacg aggaaaccct tccgatttac gactggtagc gggcagtgag 6780

cgcaacgcaa ttaatgtgag ttagctcact cattaggcac cccaggcttt acactttatg 6840

cttccggctc gtatgttgtg tggaattgtg agcggataac aatttcacac aggaaacagc 6900

tatgaccatg attacgccaa gcgcgcaatt aaccctcact aaagggaaca aaagctggag 6960

ctccaccgcg gtggcgattt tttaggatcg atttagtgct ttacggcacc tcgaccccaa 7020

aaacttgatt agggtgatgg ttcacgtatt gggccatcgc cctgatagac ggtttttcgc 7080

cctttgacgt tggagtccac gttctttaat agtggactct tgttccaaac tggaacaaca 7140

ctcaacccta tctcggtcta ttcttttgat ttataaggga ttttgccgat ttcggcctat 7200

tggttaaaaa atgagctgat ttaacaaaaa tttaacgcga attttaacaa aatattaacg 7260

cttacaattt aggtggcact tttcggggaa atgtgcgcgg aacccctatt tgtttatttt 7320

tctaaataca ttcaaatatg tatccgctca tgagacaata accctgataa atgcttcaat 7380

aatattgaaa aaggaagagt gagtatgagt attcaacatt tccgtgtcgc ccttattccc 7440

ttttttgcgg cattttgcct tcctgttttt gctcacccag aaacgctggt gaaagtaaaa 7500

gatgctgaag atcagttggg tgcacgagtg ggttacatcg aactggatct caacagcggt 7560

aagatccttg agagttttcg ccccgaagaa cgttttccaa tgatgagcac ttttaaagtt 7620

ctgctatgtg gcgcggtatt atcccgtatt gacgccgggc aagagcaact cggtcgccgc 7680

atacactatt ctcagaatga cttggttgag tactcaccag tcacagaaaa gcatcttacg 7740

gatggcatga cagtaagaga attatgcagt gctgccataa ccatgagtga taacactgcg 7800

gccaacttac ttctgacaac gatcggagga ccgaaggagc taaccgcttt tttgcacaac 7860

atgggggatc atgtaactcg ccttgatcgt tgggaaccgg agctgaatga agccatacca 7920

aacgacgagc gtgacaccac gatgcctgta gcaatggcaa caacgttgcg caaactatta 7980

actggcgaac tacttactct agcttcccgg caacaattaa tagactggat ggaggcggat 8040

aaagttgcag gaccacttct gcgctcggcc cttccggctg gctggtttat tgctgataaa 8100

tctggagccg gtgagcgtgg gtctcgcggt atcattgcag cactggggcc agatggtaag 8160

ccctcccgta tcgtagttat ctacacgacg gggagtcagg caactatgga tgaacgaaat 8220

agacagatcg ctgagatagg tgcctcactg attaagcatt gg 8262

<210> 158
<211> 7716
<212> DNA
<213> Adeno-Associated Virus

<220>
<221> gene
<222> (1)..(7716)

<400> 158
   ggcatttctg acagctttgt gaactgggtg gccgagaagg aatgggagtt gccgccagat 60
   tctgacatgg atctgaatct gattgagcag gcacccctga ccgtggccga gaagctgcag 120
   cgcgactttc tgacggaatg gcgccgtgtg agtaaggccc cggaggctct tttctttgtg 180
   caatttgaga agggagagag ctacttccac atgcacgtgc tcgtggaaac caccggggtg 240
   aaatccatgg ttttgggacg tttcctgagt cagattcgcg aaaaactgat tcagagaatt 300
   taccgcggga tcgagccgac tttgccaaac tggttcgcgg tcacaaagac cagaaatggc 360
   gccggaggcg ggaacaaggt ggtggatgag tgctacatcc ccaattactt gctccccaaa 420
   acccagcctg agctccagtg ggcgtggact aatatggaac agtatttaag cgcctgtttg 480
   aatctcacgg agcgtaacgg gtggtggcgc agcatctgac gcacgtgtcg cagacgcaaa 540
   accgcccccg actacctggt gggccagcag cccgtggagg acatttccag caatcggatt 600
   tataaaattt tggaactaaa cgggtacgat ccccaatatg cggcttccgt ctttctggga 660
   tgggccacga aaaagttcgg caagaggaac accatctggc tgtttgggcc tgcaactacc 720
   gggaagacca acatcgcgga ggccatagcc cacactgtgc ccttctacgg gtgcgtaaac 780
   tggaccaatg agaactttcc cttcaacgac tgtgtcgaca agatggtgat ctggtgggag 840
   gaggggaaga tgaccgccaa ggtcgtggag tcggccaaag ccatttctcg gaggaaagca 900
   aaggtgcgcg tggaccagaa atgcaagtcc tctcactcac ggacagaaag actgtttaga 960
   gtgctttccc gtgtcagaat ctcaacccgt ttctgtcgtc aaaaaggcgt atcaaaaaac 1020
   tgtgctacat tcatcatatc atgggaaagg tgccagacgc ttgcactgcc tgcgatctgg 1080
   tcaatgtgga tttggatgac tgcatctttg aacaataaat gatttaaatc aggtatggct 1140
   gccgatggtt atcgcactgc ctgcgatctg gtcaatgtgg atttggatga ctgcatcttt 1200
   gaacaataaa tgatttaaat caggtatggc tgccgatggt tatcttccag attggctcga 1260
   ggacaacctc tctgagggca ttcgcgagtg gtgggacttg aaacctggag ccccgaaacc 1320
   caaagccaac cagcaaaagc aggacgacgg ccggggtctg gtgcttcctg gctacaagta 1380
   cctcggaccc ttcaacggac tcgacaaggg ggagcccgtc aacgcggcgg acgcagcggc 1440
   cctcgagcac gacaaggcct acgaccagca gctcaaagcg ggtgacaatc cgtacctgcg 1500
   gtataaccac gccgacgccg agtttcagga gcgtctgcaa gaagatacgt cttttggggg 1560
   caacctcggg cgagcagtct tccaggccaa gaagcgggtt ctcgaacctc tcggtctggt 1620
   tgaggaaggc gctaagacgg ctcctggaaa gaagagaccg gtagagccat caccccagcg 1680
   ttctccagac tcctcaatgt ggatttggat gactgcatct ttgaacaata tatgatttaa 1740
   atcaggtatg gctgccgatg gttatcttcc agattggctc gaggacaacc tctctgaggg 1800
   cattcgcgag tggtgggact tgaaacctgg agccccgaaa cccaaagcca accagcaaaa 1860
   gcaggacgac ggccggggtc tggtgcttcc tggctacaag tacctcggac ccttcaacgg 1920
   actcgacaag ggggagcccg tcaacgcggc ggacgcagcg gccctcgagc acgacaaggc 1980
   ctacgaccag cagctcagag cgggtgacaa tccgtacctg cggtataacc acgccgacgc 2040
   cgagtttcag gagcgtctgc aagaagatgc gtctttttct ggttgaggaa ggcgctagga 2100
   cggctcctgg aaagaagaga ccggtagagc catcacccca gcgttctcca gactcctcta 2160
   cgggcatcgg caagaaaggc cagcagcccg cgaaaaagag actcaacttt gggcagactg 2220
   gcgactcaga gtcagtgccc gaccctcaac caatcggaga accccccgca gcccctctgg 2280
   tctgggatct ggtacaatgg ctgcaggcgg tggcgctcca atggcagaca atccccaacc 2340
   ccaattttca gaacctccgg aaaaaaaatc ctcttttggg ggccaccccc ggccaaccat 2400
   ccttccagcc caaaaaccgg ttccccaacc ctttcgtctt ggttgagaaa ggccctaaaa 2460
   cgctcccgga aaaaaaaaaa ccggaaaacc ctccccccca ccttccccca aatcctccaa 2520
   cggcctccgc aaaaaaaggc caccaccccc caaaaaaaaa atccactttg ggcaaaacgg 2580
   ccaatccaaa tccgtggccc aacctccacc catccgaaaa ccccccccca gcccctctgg 2640
   gccgggattt ggtaacatgg ctggcggggg gggccccccc atgggaaccc tccccgccta 2700
   caacaaccac caaaataaac aaagccccca acggaggggt tattccccca gaaattgggg 2760
   aagcaccaac gacaacacct acttcggcta cagcaccccc tgggggtatt ttgactttaa 2820
   cagattccac tgccacttct caccacgtga ctggcagcga ctcatcaaca acaactgggg 2880
   attccggccc aaaaaactca acttcaagct cttcaacatc cacgtcaagg aggtcacgca 2940
   aaatgaaggc accaggacca tcgccaataa ccttaccagc acgaatcagg tctttaccga 3000
   ctcggaatac cagctcccgt acctcctcgg ctctgcgcac cagggctgcc tgcctgatga 3060
   accccctcat cgaccagtac ctgtactacc tgtctcggac tcagtccacg ggaggtaccg 3120
   caggaactca gcagttgcta ttttctcagg ccgggcctaa taacatgtcg gctcaggcca 3180
   aaaactggct acccgggccc tgctaccggc agcaacgcgt cgccacgaca ctgtcgcaaa 3240
   ataacaacgc caactttgcc tggaccggtg ccaccaagta tcatctgaat ggcagaaact 3300
   ctctggtaaa tcccggtgtc gctatggcaa cccacaagga cgacgaagag cgattttttc 3360
   cgtccagcgg agtcttaatg tttgggaaac agggagctgg aaaagacaac gtggactata 3420
   gcatcgttat gctaaccagt gaggaagaaa ttaaaaccac caacccagtg gccacagaac 3480
   agtacggcgt ggtggccgat aacctgcaac agcaaaacgc cgctcctatt gtaggggccg 3540
   tcaacagtca aggagcctta cctgtcatgg tctggcagaa ccgggacgtg tacctgcagg 3600
   gtcctatctg ggccaagatt cctcacacgg acggaaactt tcatccctcg ccgctgatgg 3660
   gaggctttgg actgaaacac ccgcctcctc catcctgatt aaaaatacac ctgttcccgt 3720
   ggatcctcca actaccttca gtccagctaa gctggcggcg ttcatcgcgc agtacagcac 3780
   cggacaggtc agcgtggaaa ttgaatggga gctgcaaaaa aaaaacagca aacgctggaa 3840
   cccaaaaatt caatactctt ccaactacta caaatctaca aatgtggact ttgctgttaa 3900
   cacagatggc acttattggt acgatcagac tggagcgggc agtgagcgca cgcaattaat 3960
   gtgagttagc tcactcatta ggcaccccag gctttacact ttatgcttcc ggctcgtatg 4020
   ttgtgtggaa ttgtgagcgg ataacaattt cacacaggaa acagctatga ccatgattac 4080
   gccaagcgcg caattaaccc tcactaaagg gaacaaaagc tggagctcca ccgcggtggt 4140
   gctttacggc acctcgaccc caaaaaactt gattagggtg atggttcacg aaaagggcca 4200
   tcgccctgat agacggtttt tcgccctttg acgttggagt ccacgttctt taatagtgga 4260
   ctcttgttcc aaactggaac aacactcaac cctatctcgg tctattcttt tgatttataa 4320
   gggattttgc cgatttcggc ctattggtta aaaaatgagc tgatttaaca aaaatttaac 4380
   gcgaatttta acaaaatatt aacgcttaca atttaggtgg cacttttcgg ggaaatgtgc 4440
   gcggaacccc tatttgttta tttttctaaa tacattcaaa tatgtatccg ctcatgagac 4500
   aataaccctg ataaatgctt caataatatt gaaaaaggaa gagtatgagt attcaacatt 4560
   tccgtgtcgc ccttattccc ttttttgcgg cattttgcct tcctgttttt gctcacccag 4620
   aaacgctggt gaaagtaaaa gatgctgaag atcagttggg tgcacgagtg ggttacatcg 4680
   aactggatct caacagcggt aagatccttg agagttttcg ccccgaagaa cgttttccaa 4740
   tgatgagcac ttttaaagtt ctgctatgtg gcgcggtatt atcccgtatt gacgcccggg 4800
   caagagcaac tcggtcgccg catacactat tctcagatga cttggttgag tactccccag 4860
   tcacagaaaa gcatcttacg atggcatgac agtaaggaga ttatgcatgc tgccatacca 4920
   tgatgaataa cactgcgcac ttacttctga cacgatcgga ggaccgaagg agctcggcgc 4980
   ttctggggct ttttttgaag gctaaaaaag ggcccaaggg gggggggggg gggcaagcac 5040
   aaccccccgt acgtcggggg ggggagaagg agattttcct ttagcaaaga gaacggtgcc 5100
   ccccccggcg agggaacggg gcccctggtt tcccccgccc aacggagaaa ccatacaccc 5160
   actactatgc acaacatcta tatgtaatca taaaaccata gatcatcgct atagcgcgac 5220
   gcgcccctct ctgcatactg tgaggtaaga cgatctgtgg ctccgattct cgacatattg 5280
   acgctagact gcgttgctca gtcttgcacg agtgggttac atcgaactgg atctcaacag 5340
   cggtaagatc cttgagagtt ttcgccccga agaacgtttt ccaatgatga gcacttttaa 5400
   agttctgcta tgtggcgcgg tattatcccg tattgacgcc gggcaagagc aactcggtcg 5460
   ccgcatacac tattctcaga atgacttggt tgagtactca ccagtcacag aaaagcatct 5520
   tacggatggc atgacagtaa gagaattatg cagtgctgcc ataaccatga gtgataacac 5580
   tgcggccaac ttacttctga caacgatcgg aggaccgaag gagctaaccg cttttttgca 5640
   caacatgggg gatcatgtaa ctcgccttga tcgttgggaa ccggagctga atgaagccat 5700
   accaaacgac gagcgtgaca ccacgatgcc tgtagcaatg gcaacaacgt tgcgcaaact 5760
   attaactggc gaactactta ctctagcttc ccggcaacaa ttaatagact ggatggaggc 5820
   ggataaagtt gcaggaccac ttctgcgctc ggcccttccg gctggctggt ttattgctga 5880
   taaatctgga gccggtgagc gtgggtctcg cggtatcatt gcagcactgg ggccagatgg 5940
   taagccctcc cgtatcgtag ttatctacac gacggggagt caggcaacta tggatcaaca 6000
   attaatagac tggatggagg cggataaagt tgcaggacca cttctgcgct cggcccttcc 6060
   ggctggctgg tttattgctg ataaatctgg agccggtgag cgtgggtctc gcggtatcat 6120
   tgcagcactg gggccagatg gtaagccctc ccgtatcgta gttatctaca cgacggggag 6180
   tcaggcaact atggatgaac gaaatagaca gatcgctgag ataggtgcct cactgattaa 6240
   gcattggtaa ctgtcagacc aagtttactc atatatactt tagattgatt taaaacttca 6300
   tttttaattt aaaaggatct aggtgaagat cctttttgat aatctcatga ccaaaatccc 6360
   ttaacgtgag ttttcgttcc actgagcgtc agaccccgta gaaaagatca aaggatcttc 6420
   ttgagatcct ttttttctgc gcgtaatctg ctgcttgcaa acaaaaaaac caccgctacc 6480
   agcggtggtt tgtttgccgg atcaagagct accaactctt tttccgaagg taactggctt 6540
   cagcagagcg cagataccaa atactgttct tctagtgtag ccgtagttag gccaccactt 6600
   caagaactct gtagcaccgc ctacatccga aggtaactgg cttcagcaga gcgcagatac 6660
   caaatactgt tcttctagtg tagccgtagt taggccacca cttcaagaac tctgtagcac 6720
   cgcctacata cctcgctctg ctaatcctgt taccagtggc tgctgccagt ggcgataagt 6780
   cgtgtcttac cgggttggac tcaagacgat agttaccgga taaggcgcag cggtcgggct 6840
   gaacgggggg ttcgtgcaca cagcccagct tggagcgaac gacctacacc gaactgagat 6900
   acctacagcg tgagctatga gaaagcgcca cgcttcccga agggagaaag gcggacaggt 6960
   atccggtaag cggcagggtc ggaacaggag agcgcacgag ggagcttcca gggggaaacg 7020
   cctggtatct ttatagtcct gtcgggtttc gccacctctg acttgagcgt cgatttttgt 7080
   gatgctcgtc aggggggcgg agcctatgga aaaacgccag caacgcggcc ctttttacgg 7140
   ttgaacagga gagcgcacga gggagcttcc agggggaaac gcctggtatc tttatagtcc 7200
   tgtcgggttt cgccacctct gacttgagcg tcgatttttg tgatgctcgt caggggggcg 7260
   gagcctatgg aaaaacgcca gcaacgcggc ctttttacgg ttcctggcct tttgctggcc 7320
   ttttgctggc cttttgctca catgttcttt cctgcgttat cccctgattc tgtggataac 7380
   cgtattaccg cctttgagtg agctgatacc gctcgccgca gccgaacgac cgagcgcagc 7440
   gagtcagtga gcgaggaagc ggaagagcgc ccaatacgca aaccgcctct ccccgcaaga 7500
   tacagactgg agcgggcagt gagcgcaacg caattaatgt gagttagctc actcattagg 7560
   caccccaggc tttacacttt atgcttccgg ctcgtatgtt gtgtggaatt gtgagcggat 7620
   aacaatttca cacaggaaac agctatgacc atgattacgc caagcgcgca attaaccctc 7680
   actaaaggga acaaaagctg gagctccacc gcggtg 7716

<210>  159
<211>  19
<212>  DNA
<213>  Mus musculus

<220>
<221>  primer_bind
<222>  (1)..(19)

<400>  159
tttccacagc cacgaagct                                                    19

<210>  160
<211>  20
<212>  DNA
<213>  Mus musculus

<220>
<221>  primer_bind
<222>  (1)..(20)

<400>  160
tgatgaaagg gctcccaagt                                                   20

<210>  161
<211>  21
<212>  DNA
<213>  Mus musculus

<220>
<221>  primer_bind
<222>  (1)..(21)

<400>  161
tgttctctgg gaaatcgtgg a                                                 21

<210>  162
<211>  21
<212>  DNA
<213>  Mus musculus

<220>
<221>  primer_bind
<222>  (1)..(21)

<400>  162
tcagaattgc cattcgacaa c                                                 21

<210>  163
<211>  17
<212>  DNA
<213>  Mus musculus

<220>
<221>  primer_bind
<222>  (1)..(17)

<400>  163
ccagaactcc aggcggt                                                    17


<210>  164
<211>  21
<212>  DNA
<213>  Mus musculus


<220>
<221>  primer_bind
<222>  (1)..(21)

<400>  164
tgggaacttc tcatcccttt g                                               21


<210>  165
<211>  19
<212>  DNA
<213>  Mus musculus


<220>
<221>  primer_bind
<222>  (1)..(19)

<400>  165
gcctatcgcc aatgagctg                                                  19


<210>  166
<211>  18
<212>  DNA
<213>  Mus musculus


<220>
<221>  primer_bind
<222>  (1)..(18)

<400>  166
ttcggtttgg gtgcagtg                                                   18


<210>  167
<211>  20
<212>  DNA
<213>  Mus musculus


<220>
<221>  primer_bind
<222>  (1)..(20)

<400>  167
ctttgcctac ctctccctcg                                                 20

<210>  168
<211>  19
<212>  DNA
<213>  Mus musculus

<220>
<221>  primer_bind
<222>  (1)..(19)

<400>  168
cacacttggc ggttccttc                                                    19

<210>  169
<211>  20
<212>  DNA
<213>  Mus musculus

<220>
<221>  primer_bind
<222>  (1)..(20)

<400>  169
tgccacctac tcccttggat                                                   20

<210>  170
<211>  18
<212>  DNA
<213>  Mus musculus

<220>
<221>  primer_bind
<222>  (1)..(18)

<400>  170
ggctgttgga acgctgac                                                     18

<210>  171
<211>  22
<212>  DNA
<213>  Mus musculus

<220>
<221>  primer_bind
<222>  (1)..(22)

<400>  171
cagcttaaag ggcgggtcag ag                                                22

<210>  172
<211>  22
<212>  DNA
<213>  Mus musculus

<220>
<221>  primer_bind
<222>  (1)..(22)

<400>  172
tggagacgcc agctctggct ca                                                22


<210>  173
<211>  20
<212>  DNA
<213>  Mus musculus


<220>
<221>  primer_bind
<222>  (1)..(20)

<400>  173
ccagacgctc ttcgagaacc                                                   20


<210>  174
<211>  20
<212>  DNA
<213>  Mus musculus


<220>
<221>  primer_bind
<222>  (1)..(20)

<400>  174
gttatagaag tggcggttgt                                                   20